(19) Europäisches Patentamt
European Patent Office
Office européen des brevets

(11) EP 4 556 466 A1

(12) EUROPEAN PATENT APPLICATION
published in accordance with Art. 153(4) EPC

(43) Date of publication:
**21.05.2025 Bulletin 2025/21**

(21) Application number: **23851805.4**

(22) Date of filing: **08.08.2023**

(51) International Patent Classification (IPC):
***C07D 231/56*** (2006.01) ***C07D 401/04*** (2006.01)
***C07D 401/12*** (2006.01) ***C07D 401/14*** (2006.01)
***C07D 403/12*** (2006.01) ***C07D 403/14*** (2006.01)
***C07D 405/12*** (2006.01) ***C07D 405/14*** (2006.01)
***C07D 413/12*** (2006.01) ***C07D 413/14*** (2006.01)
***C07D 417/12*** (2006.01) ***C07D 419/12*** (2006.01)
***C07D 417/14*** (2006.01) ***C07D 419/14*** (2006.01)
***A61K 31/416*** (2006.01) ***A61P 27/06*** (2006.01)
***A61P 27/02*** (2006.01) ***A61P 7/00*** (2006.01)

(52) Cooperative Patent Classification (CPC):
**A61K 31/416; A61P 7/00; A61P 27/02; A61P 27/06; C07D 231/56; C07D 401/04; C07D 401/12; C07D 401/14; C07D 403/12; C07D 403/14; C07D 405/12; C07D 405/14; C07D 413/12; C07D 413/14; C07D 417/12;** (Cont.)

(86) International application number:
**PCT/CN2023/111668**

(87) International publication number:
**WO 2024/032584 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.08.2022 CN 202210942067**

(71) Applicant: **Beyang Therapeutics Co., Ltd.**
**Suzhou, Jiangsu 215127 (CN)**

(72) Inventors:
- **HU, Qiyue**
  **Suzhou, Jiangsu 215127 (CN)**
- **XI, Zhiguo**
  **Suzhou, Jiangsu 215127 (CN)**
- **LIU, Suxing**
  **Suzhou, Jiangsu 215127 (CN)**

(74) Representative: **HGF**
**HGF Limited**
**1 City Walk**
**Leeds LS11 9DX (GB)**

# (54) PROTEIN TYROSINE KINASE INHIBITOR AND MEDICAL USE THEREOF

(57) The present invention relates to a protein tyrosine kinase inhibitor for the treatment of protein tyrosine kinase-mediated proliferative diseases or symptoms, for example ocular diseases including pathological neovascularization, retinal ischemia, retinal edema, and diabetic retinopathy, as well as malignant tumors. More particularly, the present invention relates to a compound with protein tyrosine kinase inhibitory activity and/or improved solubility and ocular bioavailability, and its uses in the treatment of ocular diseases and malignant tumors.

(52) Cooperative Patent Classification (CPC): (Cont.)
**C07D 417/14; C07D 419/12; C07D 419/14**

**Description**

FIELD OF THE INVENTION

The present invention relates to the field of medicine, in particular, to a protein tyrosine kinase inhibitor and its use in the treatment of protein tyrosine kinase-mediated proliferative diseases or symptoms.

BACKGROUND OF THE INVENTION

Age-related macular degeneration (AMD) and diabetic retinopathy (DR) are the most common ocular diseases and the leading causes of blindness in the elderly and working-age populations. These lesions are associated with neovascularization and extravasation in the posterior segment of the eye. Specifically, wet AMD is marked by neovascularization from the choroidal microvascular beds and invasion into the subretinal space, while DR is preferentially characterized by changes in extravasation and neovascularization at the retinal level. Both AMD and DR are characterized by the proliferation and migration of endothelial cells (ECs), increased vascular permeability, and inflammation, in which vascular endothelial growth factor-A (VEGF-A) and its corresponding receptors (VEGFRs) play critical roles. In addition, tumor growth and metastasis depend on the tumor vascular network to provide adequate oxygen and nutrients. Tumor angiogenesis relies on a highly complex procedure involving growth factor signaling, endothelial cell proliferation, extracellular matrix (ECM) remodeling, and interactions with stromal cells. Among them, the most important pro-angiogenic driving factor is vascular endothelial growth factor (VEGF). It has been shown that many proliferative disorders, such as ocular diseases, tumors, and cancers, involve the overexpression or upregulation of receptor tyrosine kinase (RTK) activity. Receptor tyrosine kinases are a class of kinase enzymes that modify proteins by chemically adding phosphate groups (phosphorylation). Phosphorylation typically leads to functional changes in target proteins by altering enzyme activity, cellular localization, or binding to other proteins. Kinases are known to regulate most cellular pathways, especially those involved in signal transduction. To date, one of the approaches to inhibit the VEGF pathway is to inhibit the RTK activity. In the treatment of AMD and DR, the goal of protein tyrosine kinase inhibitor therapy is to counteract pathological neovascularization and disease progression, thereby preventing vision impairment. At the same time, the importance of VEGFR as a pro-angiogenic inducer in tumor growth, invasion, and extravasation makes it an excellent therapeutic target for various cancers. To date, the FDA has not approved small molecule tyrosine kinase inhibitors for the treatment of neovascular AMD and DR.

SUMMARY OF THE INVENTION

The present invention provides a compound targeted for the treatment of protein tyrosine kinase-mediated proliferative diseases or symptoms, such as ocular diseases including pathological neovascularization, retinal ischaemia, retinal edema, and diabetic retinopathy (DR), as well as malignant tumors. The clinical success of VEGF antibody drugs (such as pegaptanib, ranibizumab, aflibercept, and brolucizumab) has validated this concept. For instance, the U.S. Food and Drug Administration (FDA) approved aflibercept (VEGF Trap-Eye) for the treatment of neovascular AMD. Ziv-aflibercept, with the same molecular structure, has also been approved by the FDA for systemic use in combination therapy for patients with metastatic colorectal cancer. However, these VEGF antibody drugs require multiple intravitreal injections administered by retinal specialists. Frequent intravitreal injections are less convenient for both clinicians and patients and carry rare but serious injection-related risks (endophthalmitis, retinal detachment, cataracts, intraocular inflammation, etc.), leading to poor efficacy in many patients who are not administered on time. The favorable clinical outcomes of VEGF antibody biologics, along with the challenges associated with intravitreal injections, have prompted efforts to develop small molecule anti-angiogenic agents as therapies for neovascular AMD, particularly VEGFR tyrosine kinase inhibitors for ocular administration. Small molecule targeted VEGFR tyrosine kinase inhibitors have many advantages over recombinant proteins. They can be formulated as eye drops, thus avoiding intravitreal injections. Small molecule eye drops are capable of crossing cell membranes and directly interacting with the cytoplasmic domains of RTK, and also, economically inexpensive. So far, however, no small molecule tyrosine kinase inhibitors have been approved by the FDA for the treatment of neovascular AMD and DR.

Vascular endothelial growth factor (VEGF) and its receptors (VEGFRs) are known as the most potent vascular permeabilizers and endothelial cell-specific mitogens, playing critical roles in the proliferation, migration, and angiogenesis of endothelial cells. Angiogenesis is an important mechanism in many physiological and pathological processes and is involved in the proliferation, migration, and survival of endothelial cells, which in turn leads to further tubular formation and ultimately promotes vascularization. VEGF and its receptors (VEGFRs) play significant roles in pathological angiogenesis associated with conditions such as tumor development and ocular neovascular diseases. For example, the expression level of VEGF is significantly positively correlated with the degree of vascularization in tumor tissues. VEGF acts on the VEGFR2 receptors, activating the phosphorylation of the VEGFR receptor tyrosine kinase and leading to the

signal transduction of abnormal cells, thereby promoting the proliferation of endothelial cells and neovascularization. VEGF is a key participant in many different cancers and ocular diseases associated with pathological neovascularization. The compound of the present invention (e.g., the compound prepared in Embodiment 1) is able to inhibit anti-angiogenic tyrosine kinases, effectively antagonizing the tyrosine kinase activity of all VEGFRs (VEGFR1, VEGFR2, VEGFR3) while avoiding high selectivity for inhibiting EGFR (Embodiment 2).

VEGFR2 is the primary receptor for VEGF-induced endothelial cell signaling. During development and/or after tissue damage, the ligand VEGF binds to the receptor, leading to the autophosphorylation and activation of VEGFR2, which induces angiogenesis and bypasses blocked vessels. The compound of the present invention has a significant inhibitory effect on VEGF-induced VEGFR2 autophosphorylation (pVEGFR2) in human endothelial cells, thereby blocking the signal transduction of abnormal cells and inhibiting neovascularization (Embodiment 3). The main function of VEGFR receptor signal transduction is to promote the proliferation of endothelial cells and neovascularization. The compound of the present invention shows the ability to inhibit the proliferation of human endothelial cells induced by VEGF at a nanomolar concentration level. In summary, the compound of the present invention is a novel tyrosine kinase inhibitor. In addition to being applicable for the treatment of neovascular AMD and DR, this novel tyrosine kinase inhibitor can also be used in therapeutic indications for tumors, by blocking neovascularization in tumors and thus disrupting the blood and nutrient supply necessary for tumor growth, leading to tumor cell death.

One of the goals in medicinal chemistry is to enhance the bioavailability and stability of compounds to improve their efficacy. Bioavailability refers to the rate and extent to which a therapeutic agent is absorbed from its dosage form and becomes available at the site of action. Existing VEGF tyrosine kinase inhibitors often have issues like low solubility and/or kinase inhibitory activity, which significantly affect their bioavailability and thus reduce their efficacy. This is particularly challenging for compounds with low solubility to be formulated as eye drops. For example, although Axitinib exhibits good inhibition for VEGF tyrosine kinases (VEGFR1, VEGFR2, and VEGFR3), it has low solubility (<2 micrograms/milliliter). The present invention provides a compound with significant solubility, enhanced benefits and/or excellent kinase inhibitory activity (Embodiment 4), such as a thermodynamic solubility of 10 micrograms per milliliter or above, or 100 micrograms per milliliter or above, or even 1,000 micrograms per milliliter or above. For example, the $IC_{50}$ for VEGFR2 may be 100 nM or below, or 10 nM or below, or even 1 nM or below.

Melanin-containing cells in the eye are located in the retinal pigment epithelium and choroid of the posterior segment, and the ciliary body and iris of the anterior segment. The binding of compounds to melanin may affect ocular pharmacokinetics after topical administration. The present invention provides a compound-containing eye drop formulation aimed at treating AMD and DR. Both AMD and DR are diseases of the posterior segment of the eye, and the compound-containing eye drop formulation targets effective delivery to the tissues at the posterior segment of the eye. In such cases, some drugs may tightly bind to melanin tissues in the posterior segment (retinal pigment epithelium, choroid) or anterior segment (ciliary body, iris) of the eye. Many clinical drugs binds to melanin, thereby affecting their ocular pharmacokinetics. The binding rate between the compound and melanin is an important factor in ocular pharmacokinetics and pharmacodynamics, mainly related to the tissue distribution of the compound within the eyeball, which must be considered during drug discovery and development. The present invention provides insights into the impact of melanin on compounds (Embodiment 5).

One of the key challenges in developing small molecule tyrosine kinase inhibitors for clinical use in AMD and DR is overcoming the risk of "target toxicity". In a healthy vascular system, inhibiting VEGFR may potentially lead to serious adverse events, such as hypertension, haemorrhage, and thrombus. Despite a number of clinical successes in oncological indications, the safety profile of oral VEGFR-2 inhibitors may be the main reason for limited clinical use and/or development in patients with AMD and DR. Theoretically, topical ophthalmic drops can offer an effective therapy that limits systemic exposure and avoids problems of targeted toxicity.

While topical ocular administration has proven to be a successful strategy for treating diseases related to the anterior segment of the eye (such as glaucoma), there are currently no FDA-approved topical therapies for ocular diseases associated with posterior ocular tissues (such as retina and choroid), including neovascular AMD and DR. This is largely due to the anatomical and physiological barriers that the human eye has evolved to protect itself from exogenous substances. The tear film is one of the first barriers to overcome. Compounds in the anterior segment of the eye can be quickly washed away by the tear film, leading to nasolacrimal duct drainage, which means that compounds need to be rapidly absorbed after local instillation. However, absorption/permeation into ocular tissues can also be challenging. One pathway for absorption involves the permeability of the cornea, which consists of an epithelium with tight junctions and alternating lipophilic and hydrophilic layers. The other pathway for absorption is penetration into the conjunctiva and subsequent diffusion into the sclera. The sclera is relatively more permeable compared to other ocular tissues; however, drugs entering the conjunctiva tend to be "lost" to the systemic circulation due to the highly vascularized nature of this tissue. Compounds exposed in the sclera may potentially diffuse into the choroid, which is the primary target tissue for neovascular AMD. The diffusion from the choroid to the retina (the secondary target tissue for neovascular AMD) is further diminished by the blood-retinal barrier (BRB). The BRB functions similarly to the blood-brain barrier and may pose a significant obstacle to compound diffusion. Due to these anatomical and physiological barriers, it is estimated that only less

than 5 % of the locally administered dose reaches the posterior ocular tissues. Despite these challenges associated with local administration, this present invention focuses on developing structure-activity relationships (SAR) related to ocular and blood exposure. Through the compound-containing eye drop formulation, effective delivery to the posterior ocular tissues (choroid and retina) can be achieved (Embodiment 6). Observations indicate that the compound degrades rapidly in plasma, which is beneficial for avoiding systemic targeted toxicity. In contrast, Axitinib exhibits low exposure in posterior ocular tissues, with levels in the retina below the lower limit of quantitation. The present invention provides a novel compound-containing eye drop formulation that achieves sufficient drug concentration in the posterior segment of the eye (such as the choroid and retina) to bind to relevant receptors in the target eye, thereby increasing the bioavailability in the posterior segment and addressing the issues encountered with existing topical therapeutic agents in ocular delivery.

Because of the limited permeability of many ocular drops to the corneal and conjunctival barriers, a major limitation of ocular drops may be the need for high concentrations of compounds in ocular formulations in order to achieve therapeutically effective doses in the posterior ocular tissues. Depending on the compound (the molecule itself or its high concentration), ocular formulations may have side effects on anterior ocular tissues (including the conjunctiva, cornea, and/or lens), leading to various ocular surface injuries, such as corneal epithelial defects and erosions. In particular, it has been clinically observed that treatment with EGFR antibody drugs may cause ocular side effects, such as epithelial degeneration and defects, ulcers, corneal epithelial thinning, erosion, and/or corneal edema and keratitis. EGFR is a major factor in the wound healing of human corneal epithelial cells. Therefore, it is essential to select compounds for topical ocular formulations that avoid inhibiting EGFR activity. The compound of the present invention exhibits high selectivity for inhibiting EGFR tyrosine kinase activity in addition to effectively antagonizing VEGFR1, VEGFR2, and VEGFR3 tyrosine kinase activities (Embodiment 2).

AMD is an ocular disease that leads to vision acuity loss due to maculopathy. The macula is a region of the retina responsible for clear and detailed vision. In wet AMD, choroidal blood vessels (choroidal neovascularization, CNV) germinate and expand into the submacular space, leading to fluid and blood leakage. This results in retinal edema, scar tissue formation, and irreversible damage to the macula. DR is a common complication of diabetes, and many structural and functional abnormalities of the retina are associated with the progression of diabetes. Almost all patients diagnosed with diabetes for more than 15 years develop DR. DR is divided into two stages based on the duration of its progression: the non-proliferative stage, also known as the early stage, characterized by vascular leakage; and the proliferative stage, also known as late stage, characterized by retinal vascular proliferation induced by various growth factors. Based on the properties of the compound of the present invention as an effective VEGF receptor inhibitor, several animal models of choroidal and retinal neovascular diseases are used to evaluate the in vivo efficacy of the compound. Upregulation of VEGF leads to abnormal growth and leakage of retinal blood vessels, resulting in vision impairment. In Dutch belted rabbits, intravitreal injection of VEGF causes transient retinal leakage, similar to human wet AMD and DR. The compound of the present invention demonstrates efficacy in inhibiting retinal leakage in this model. The oxygen-induced retinopathy (OIR) mouse model exhibits reproducible and quantifiable proliferative retinal neovascularization, with human vascular pathology (vitreous neovascularization) being very similar to the clusters formed in the mouse OIR. The compound of the present invention is evaluated for its in vivo efficacy in retinal vascular lesions using the OIR model. In laser-induced choroidal neovascularization (CNV) rat model, green laser photocoagulation pulses are applied to the rat's eye to induce localized rupture of Bruch's membrane (the extracellular matrix between the retina and choroid). The rupture of Bruch's membrane induces the production of local inflammatory factors and VEGF, leading to CNV. This model has been validated in humans, monkeys, pigs, and rodents for the formation of CNV following laser injury and represents a VEGF-dependent pathology. Streptozotocin (STZ)-induced diabetic rats are considered a useful preclinical animal model for investigating the pathogenesis and treatment of human DR. Significant increases in retinal vascular permeability, such as intraretinal hemorrhages and fluorescein leakage, are detected in all diabetic rat groups, indicating pre-proliferative changes in DR. However, some of these rats exhibit vitreous neovascularization attached to the retina and retinal folds, which is a prominent manifestation of severe and proliferative DR, marking the proliferative stage of DR. During the progression of DR, there is an increased expression of angiogenesis-related growth factors VEGF and its receptors VEGFR1 and VEGFR2 in the retinas of STZ-induced diabetic mice. The compound-containing ocular formulation of the present invention is evaluated for the in vivo efficacy against DR using a STZ-induced diabetic mouse retinopathy rat model, particularly the inhibition of vascular leakage and angiogenesis involved in the progression of DR in diabetic mice.

Based on the properties of the compound of the present invention as an effective VEGF receptor inhibitor, the present invention relates to a compound that can be used to treat pathological conditions caused or exacerbated by ocular angiogenesis, neovascularization, and/or vascular leakage, such as in AMD, including neovascular (wet/exudative) AMD, dry AMD, and geographic atrophy; DR (including proliferative DR, non-proliferative DR, and diabetic macular edema); pathological CNV and vascular leakage caused by any mechanism, such as sickle cell disease, high myopia, trauma; traumatic choroidal rupture, ocular histoplasmosis, optic nerve drusen, angioid streaks, or some retinal dystrophies; pathological retinal neovascularization and vascular leakage caused by any mechanism, such as sickle cell retinopathy, carotid-cavernous fistula, Eales' disease, ocular ischemic syndrome, hyperviscosity syndrome, familial exudative vitreoretinopathy, idiopathic occlusive small arteritis, retinal vasculitis, birdshot retinochoroidopathy, sarcoidosis, or

toxoplasmosis; uveitis; retinal vein occlusion (central or branch); ocular trauma; surgery-induced neovascularization; surgery-induced edema; ocular ischemia; cystoid macular edema; retinopathy of prematurity; sickle cell retinopathy; Coats disease; and/or neovascular glaucoma.

Additionally, the occurrence, progression, and metastasis of many tumors, as well as the formation of tumor neovascularization, are closely related to the abnormal expression of tyrosine kinases. In particular, some tyrosine kinase receptors are abnormally expressed in solid tumor cells, with vascular endothelial growth factor receptors (VEGFRs) being highly expressed in many tumor cells and tumor vascular endothelial cells. The VEGFR family is directly related to the occurrence, progression, and formation of tumor angiogenesis. In addition to ocular diseases associated with pathological neovascularization, the clinical success of VEGF antibody drugs in treating malignant tumors validates this concept. The present invention relates to the treatment of solid tumors and leukemia, for example: breast cancer, lung cancer (especially non-small cell lung cancer), adenocarcinoma, colorectal cancer, renal cancer, liver cancer, pancreatic cancer, ovarian cancer, prostate cancer, glioma, glioblastoma, myeloma, acute myeloid leukemia, agnogenic myeloid metaplasia, mesothelioma, myelodysplastic syndrome. The invention also relates to the prevention of tumor metastatic spread and micrometastasis. According to the embodiments of the present invention, the compound is an anti-angiogenic kinase inhibitor, and examples of protein tyrosine kinase inhibitors that can yield beneficial therapeutic outcomes include receptor tyrosine kinase inhibitors such as but not limited to VEGFR, Tie-2, and FGFR. In one aspect, the present invention provides a compound of formula (I),

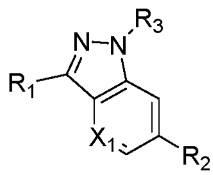 formula (I),

wherein, said $R_1$ contains a substituted cyclic structure;
$R_2$ contains an optionally substituted substituent;
$R_3$ contains an optionally substituted substituent;
$X_1$ is an optionally substituted atom;
or a pharmaceutically acceptable prodrug, a pharmaceutically active metabolite, and/or a pharmaceutically acceptable salt of said compound.

In one aspect, the present invention provides a compound disclosed herein, or a pharmaceutically acceptable prodrug, a pharmaceutically active metabolite, and/or a pharmaceutically acceptable salt of said compound.

In one aspect, the present invention provides a pharmaceutical composition, comprising the compound disclosed herein or a pharmaceutically acceptable prodrug, a pharmaceutically active metabolite, and/or a pharmaceutically acceptable salt of said compound, as well as an optional carrier.

In one aspect, the present invention provides use of the compound disclosed herein or a pharmaceutically acceptable prodrug, a pharmaceutically active metabolite, and/or a pharmaceutically acceptable salt of said compound, and/or the pharmaceutical composition disclosed herein in the preparation of a medicament for the treatment of protein tyrosine kinase-mediated diseases.

In one aspect, the present invention provides a compound disclosed herein or a pharmaceutically acceptable prodrug, a pharmaceutically active metabolite, and/or a pharmaceutically acceptable salt of said compound, and/or a pharmaceutical composition disclosed herein for the treatment of protein tyrosine kinase-mediated diseases.

In one aspect, the present invention provides a treatment method, comprising administrating the compound disclosed herein or a pharmaceutically acceptable prodrug, a pharmaceutically active metabolite, and/or a pharmaceutically acceptable salt of said compound, and/or the pharmaceutical composition disclosed herein.

In one aspect, the present invention provides a method for modulating kinase receptor activity, comprising administrating the compound disclosed herein or a pharmaceutically acceptable prodrug, a pharmaceutically active metabolite, and/or a pharmaceutically acceptable salt of said compound, and/or the pharmaceutical composition disclosed herein.

Those skilled in the art can easily comprehend various aspects and advantages of the present invention from the detailed description below. The detailed description below showcases and describes exemplary embodiments of the present invention. As those skilled in the art will recognize, the content of the present invention allows for modifications to the disclosed specific embodiments without departing from the spirit and scope of the invention involved. Accordingly, the descriptions in the specification of the present invention are merely exemplary, not restrictive.

DETAILED DESCRIPTION OF THE INVENTION

The following specific embodiments illustrate the implementation of the present invention. Those skilled in the art can easily understand other advantages and effects of the present invention based on the content disclosed herein.

DEFINITIONS OF TERMS

In the present invention, the term "basic nitrogen atom" generally refers to nitrogen atoms that exhibit basicity. For example, basic nitrogen atoms may be able to provide non-shared electron pairs. Basic nitrogen atoms are well-known in the art, for example, they have conjugate acids with a pKa greater than 3 or 5. For example, non-basic nitrogen atoms may include nitrogen atoms directly bonded to oxygen, directly bonded to aromatic rings, or directly bonded to carbonyl.

In the present invention, the term "methyl" generally refers to the residue derived from the removal of hydrogen atoms from a one-carbon atom group. Methyl may be substituted or unsubstituted. The term "alkyl" generally refers to saturated, linear or branched aliphatic hydrocarbyl with residues derived from the removal of hydrogen atoms from the same or different carbon atoms of the parent alkane, which may be a linear or branched group compromising 1 to 20 carbon atoms, preferably 1 to 12 carbon atoms, and more preferably 1 to 6 carbon atoms. Non-limiting examples of alkyl include, but are not limited to, methyl ($-CH_2-$), 1,1-ethyl ($-CH(CH_3)-$), 1,2-ethyl ($-CH_2CH_2)-$, 1,1-propyl ($-CH(CH_2CH_3)-$), 1,2-propyl ($-CH_2CH(CH_3)-$), 1,3-propyl ($-CH_2CH_2CH_2-$), 1,4-butyl ($-CH_2CH_2CH_2CH_2-$), and 1,5-butyl ($-CH_2CH_2CH_2CH_2CH_2-$). Alkyl may be substituted or unsubstituted. For example, when substituted, substituents can be substituted at any available points of attachment, preferably, one or more substituents are independently and optionally selected from the group consisting of: alkyl, alkenyl, alkynyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthio, heterocycloalkylthio, and oxo group. For instance, the substituent can be hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, $-N(H)SO_2H$, or $C_{1-6}$ aliphatic group.

In the present invention, the term "aryl" generally refers to the residue derived from the removal of hydrogen atoms from the same or different carbon atoms of an aromatic ring. The term "aromatic ring" can refer to a six- to fourteen-membered or six- to ten-membered (such as benzene and naphthalene) all-carbon monocyclic or fused polycyclic ring (i.e., a ring sharing pairs of adjacent carbon atoms) with a conjugated $\pi$-electron system. The aromatic ring may be fused to heteroaryl, heterocyclyl, or cycloalkyl ring, wherein the ring connected to the parent structure is an aryl ring. Aryl may be substituted or unsubstituted; when substituted, the substituents are preferably one or more of the following groups, independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

In the present invention, the term "heteroaryl" generally refers to the residue derived from the removal of hydrogen atoms from the same or different carbon atoms of a heteroaryl ring. The term "heteroaryl ring" refers to a heteroaromatic system containing one to four heteroatoms and five to fourteen ring atoms, wherein the heteroatoms may be selected from the group consisting of: oxygen, sulfur, and nitrogen. The heteroaryl may be five- to ten-membered, or five- or six-membered heteroaryl, such as furanyl, thiophenyl, pyridyl, pyrrolyl, N-alkylpyrrolyl, pyrimidinyl, pyrazinyl, imidazolyl and tetrazolyl. The heteroaryl ring may be fused to aryl, heterocyclyl, or cycloalkyl ring, wherein the ring connected to the parent structure is a heteroaryl ring. Heteroaryl may be optionally substituted or unsubstituted; when substituted, the substituents are preferably one or more of the following groups, independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

In the present invention, the term "aliphatic heterocyclyl" generally refers to stable non-aromatic three- to seven-membered monocyclic structures, fused seven- to ten-membered bicyclic heterocyclic structures, or bridged six- to ten-membered bicyclic heterocyclic structures. These cyclic structures may be fully saturated or partially saturated, and contain one or more heteroatoms aside from carbon atoms, wherein the heteroatoms may be selected from the group consisting of: oxygen, sulfur, and nitrogen. For example, these structures may contain 1-4 aforementioned defined heteroatoms. When used to denote an atom in a heterocyclic structure, the term "nitrogen" may encompass nitrogen that has undergone substitution reactions. The aliphatic heterocyclyl may be substituted or unsubstituted. In the present invention, the term "alcyl" generally refers to the residue derived from the removal of hydrogen atoms from the same or different carbon atoms of a carbon ring. The term "alicylic ring" usually refers to saturated or partially unsaturated monocyclic or polycyclic hydrocarbyl, with 3 to 20 carbon atoms, preferably 3 to 12 carbon atoms, more preferably 3 to 10 carbon atoms, still more preferably 3 to 8 carbon atoms. Non-limiting examples of monocyclic carbon rings include cyclopropane, cyclobutane, cyclopentane, cyclopentene, cyclohexane, cyclohexene, cyclohexadiene, cycloheptane, cycloheptatriene, cyclooctane, etc.; polycyclic carbon rings may include spirocyclic, fused, and bridged carbon rings. The alcyl may be substituted or unsubstituted.

In the present invention, the term "partially unsaturated" typically refers to cyclic structures that contain at least one double bond or triple bond between ring molecules. The term "partially unsaturated" encompasses cyclic structures with multiple unsaturated bonds, but is not intended to include aryl or heteroaryl ring as defined in this invention. The term "unsaturated" refers to cyclic structures with one or more unsaturated bonds.

In the present invention, the term "halogen" generally refers to fluorine, chlorine, bromine, iodine, for example, it may be fluorine or chlorine.

In the present invention, the term "aliphatic group" typically refers to hydrocarbons with linear, branched, or cyclic structures having 1 to 12 carbon atoms, which may be fully saturated hydrocarbons or hydrocarbons containing one or more unsaturated units, provided that the unsaturated units are not aromatic groups. For example, suitable aliphatic groups may include alkyl, alkenyl, alkynyl, and mixtures of these groups with substituted or unsubstituted linear, branched, or cyclic structures; for instance, the groups can be cycloalkyl alkyl, cycloalkenyl alkyl, or cycloalkyl alkenyl. For example, the aliphatic group may contain 1-12, 1-8, 1-6, 1-4, or 1-3 carbon atoms.

In the present invention, the terms "optional" or "optionally" generally imply that the event or situation described subsequently may or may not occur, and this description includes scenarios where the event or situation occurs or does not occur. For example, "heterocyclyl optionally substituted by alkyl" implies that alkyl may, but does not have to be present; this description may include situations where heterocyclyl is substituted by alkyl and where heterocyclyl is not substituted by alkyl.

In the present invention, the term "substituted" generally refers to one or more hydrogen atoms in a group, for example up to 5, or 1 to 3 hydrogen atoms independently of each other, being substituted by the corresponding number of substituents. The substituents are only at their possible chemical locations, which can be determined by those skilled in the art without undue effort (by experimental or theoretical means) whether the substitution is possible. For instance, an amino or hydroxyl group with a free hydrogen may be unstable when bonded to a carbon atom with an unsaturated bond (such as alkenyl).

The one or more hydrogen atoms in a group, for example, up to 5, or 1 to 3 hydrogen atoms independently of each other, are replaced by the corresponding number of substituents. The substituents are only at their possible chemical locations, which can be determined by those skilled in the art without undue effort (by experimental or theoretical means) whether the substitution is possible. For instance, an amino or hydroxyl group with a free hydrogen may be unstable when bonded to a carbon atom with an unsaturated bond (such as alkenyl).

In the present invention, the term "compound" generally refers to substances that contain two or more different elements. For example, the compound of the present invention may be an organic compound; specifically, the compound may include those with a molecular weight of less than 500, those with a molecular weight of less than 1,000, those with a molecular weight greater than 1,000, as well as those with a molecular weight greater than 10,000 or greater than 100,000. The compound of the present invention may also refer to a compound that is connected by chemical bonds, for example it may be a compound in which one or more molecules with a molecular weight of less than 1,000 that are linked to a biomolecule by chemical bonds. The above-mentioned biomolecule may be polysaccharides, proteins, nucleic acids, peptides, etc.. For instance, the compound of the present invention may include those comprising proteins connected to one or more molecules with a molecular weight of less than 1,000, those comprising proteins connected to one or more molecules with a molecular weight of less than 10,000, or those comprising proteins connected to one or more molecules with a molecular weight of less than 100,000.

In some embodiments, the compound of the present invention may be in a "mixture form", wherein the "mixture form" of the compound refers to a composition that includes more than one of the compound of the present invention, its tautomer, mesomer, racemate, enantiomer, and diastereomer.

Unless otherwise specified, the structures described in the present invention may also include compounds that differ only in the presence or absence of one or more isotope-enriched atoms. For example, all compounds that are identical to the structures described in this invention except for the substitution of hydrogen atoms with deuterium or tritium, or the substitution of carbon atoms with carbon-13 or carbon-14, are within the scope of this invention. In the present invention, the term "pharmaceutical composition" generally refers to a mixture containing one or more compounds as described in the present invention, or their physiologically/medicinally acceptable salts or prodrugs, along with other chemical components, such as physiologically/medicinally acceptable carriers and excipients. Pharmaceutical compositions may facilitate the administration to organisms and promote the absorption of active ingredients, thus exerting biological activity. Conventional methods for preparing pharmaceutical compositions can be found in the *Chinese Pharmacopoeia.*

In the present invention, the term "pharmaceutically acceptable salt" or "medicinally acceptable salt" generally refers to the salts of the compound of the present invention or the ligands-drug conjugates described herein, or the salts of the compound described in the present invention. Such salts, when used in mammals, can be safe and/or effective and possess the desired biological activity. The antibody-drug conjugates of the present invention can form salts with acids, and non-limiting examples of pharmaceutically acceptable salts include hydrochloride, hydrobromide, hydroiodide, sulfate, bisulfate, citrate, acetate, succinate, ascorbate, oxalate, nitrate, sorbate, hydrophosphate, dihydric phosphate,

salicylate, hydrogen citrate, tartrate, maleate, fumarate, formate, benzoate, methanesulfonate, ethanesulfonate, benzenesulfonate, and p-toluenesulfonate.

In the present invention, the term "solvate" or "solvated compound" generally refers to the medicinally acceptable solvate formed by the ligands-drug conjugates of the present invention with one or more solvent molecules. Non-limiting examples of solvent molecules include water, ethanol, acetonitrile, isopropanol, DMSO, and ethyl acetate. The term "pharmaceutically acceptable prodrug" refers to compounds that can be converted under physiological conditions or through solvent hydrolysis into specific compounds or pharmaceutically acceptable salts thereof. The term "pharmaceutically active metabolite" is intended to denote pharmacologically active products generated by the metabolism of specific compounds or their salts in vivo. The metabolites of compounds can be identified using conventional techniques known in the art, and their activity can be determined using tests as described herein.

Pharmaceutical compositions can be in the form of sterile injectable aqueous or oily suspensions for intramuscular and subcutaneous administration. These suspensions can be formulated with suitable dispersing or wetting agents and suspending agents above according to known techniques. Sterile injectable formulations can also be sterile injectable solutions or suspensions prepared in non-toxic, parenterally acceptable diluents or solvents, such as solutions prepared in 1,3-butanediol. In addition, sterile fixed oils can be readily used as solvents or suspending media. For example, any blended fixed oil, including synthetic glycerol mono- or di-esters, can be used. Furthermore, fatty acids such as oleic acid can also be used to prepare injectable formulations. For ocular administration, the compound of the present invention is delivered in a pharmaceutically acceptable ophthalmic carrier such that the compound remains in contact with the ocular surface for a sufficient period of time to allow the compound to penetrate the cornea and internal regions of the eye, including, for example, the anterior chamber, posterior chamber, vitreous body, aqueous humor, vitreous humor, cornea, iris/ciliary body, lens, choroid/retina, and veins. Pharmaceutically acceptable ophthalmic carriers include ointments, vegetable oils, or encapsulating materials. The compound of the present invention may also be injected directly into the vitreous body and aqueous humor.

In the present invention, the term "comprise" generally refers to including the explicitly specified features, but does not exclude other elements. The terms "greater than" and "less than" generally refer to cases that include the specified value itself.

In the present invention, the term "about" generally refers to a variation of 0.5 % to 10 % greater than or less than the specified value, such as a variation of 0.5 %, 1 %, 1.5 %, 2 %, 2.5 %, 3 %, 3.5 %, 4 %, 4.5 %, 5 %, 5.5 %, 6 %, 6.5 %, 7 %, 7.5 %, 8 %, 8.5 %, 9 %, 9.5 %, or 10 % greater than or less than the specified value.

DETAILED DESCRIPTION OF THE INVENTION

In one aspect, the present invention provides a compound of formula (I),

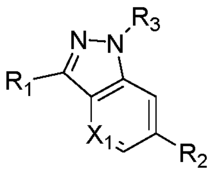
formula (I),

wherein, said $R_1$ contains a substituted cyclic structure;
$R_2$ contains an optionally substituted substituent;
$R_3$ contains an optionally substituted substituent;
$X_1$ is an optionally substituted atom;
or a pharmaceutically acceptable prodrug, a pharmaceutically active metabolite, and/or a pharmaceutically acceptable salt of said compound.

For example, the cyclic structure of said $R_1$ is substituted by a substituent containing basic nitrogen atoms.

For example, the cyclic structure of said $R_1$ is substituted by a substituent selected from the group consisting of: optionally substituted amino, and optionally substituted aliphatic heterocyclyl containing nitrogen atoms.

In one aspect, the present invention provides a compound of formula (I),

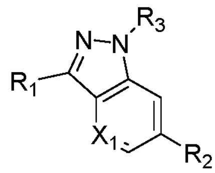 formula (I),

wherein, $R_1$ contains $(R_{1-0})_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$; $R_{1-0}$, $R_{1-1}$, $R_{1-2}$, $R_{1-3}$, $R_{1-4}$, and $R_{1-5}$ each independently contain an optionally substituted substituent; nr0, nr1, nr2, nr3, nr4 and nr5 are each independently selected from 0 or a larger number; the cyclic structure of the $R_1$ is substituted by a substituent containing basic nitrogen atoms;
$R_2$ contains an optionally substituted substituent;
$R_3$ contains an optionally substituted substituent;
$X_1$ is an optionally substituted atom;
or a pharmaceutically acceptable prodrug, a pharmaceutically active metabolite, and/or a pharmaceutically acceptable salt of said compound.

In one aspect, the present invention provides a compound of formula (I),

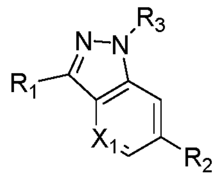 formula (I),

wherein, $R_1$ contains $(C{=}C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$; $R_{1-1}$, $R_{1-2}$, $R_{1-3}$, $R_{1-4}$, $R_{1-5}$ each independently contain an optionally substituted substituent; nr0, nr1, nr2, nr3, nr4 and nr5 are each independently selected from 0 or a larger number; the cyclic structure of the $R_1$ is substituted by a substituent containing basic nitrogen atoms;
$R_2$ contains an optionally substituted substituent;
$R_3$ contains an optionally substituted substituent;
$X_1$ is an optionally substituted atom;
or a pharmaceutically acceptable prodrug, a pharmaceutically active metabolite, and/or a pharmaceutically acceptable salt of said compound.

In one aspect, the present invention provides a compound of formula (I),

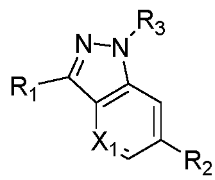 formula (I),

wherein, $R_1$ contains $(C{=}C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$; nr0, nr1, nr2, nr3, nr4 and nr5 are each independently selected from 0 or a larger number;
each $R_{1-1}$ is selected from the group consisting of: optionally substituted aryl and optionally substituted heteroaryl; for example, each $R_{1-1}$ is selected from the group consisting of: optionally substituted aryl, optionally substituted five-membered heteroaryl and optionally substituted six-membered heteroaryl; for example, each $R_{1-1}$ is selected from the group consisting of: optionally substituted aryl, optionally substituted five-membered heteroaryl containing a N atom, five-membered heteroaryl containing two N atoms, six-membered heteroaryl containing a N atom, and optionally substituted six-membered heteroaryl containing two N atoms; for example, each $R_{1-1}$ is selected from the group consisting of: optionally substituted aryl, optionally substituted pyridyl and optionally substituted pyrazolyl;
each $R_{1-2}$ is selected from the group consisting of: chemical bond and hydroxyl; for example, each $R_{1-2}$ may be - or -O-;
each $R_{1-3}$ is selected from optionally substituted alkyl; for example, each $R_{1-3}$ may be optionally substituted methyl, optionally substituted ethyl, and optionally substituted propyl;

each $R_{1-4}$ is selected from optionally substituted aliphatic heterocyclyl; for example, each $R_{1-4}$ may be optionally substituted five-membered aliphatic heterocyclyl and optionally substituted six-membered aliphatic heterocyclyl; for example, each $R_{1-4}$ is selected from the group consisting of: optionally substituted five-membered aliphatic heterocyclyl containing a N atom, five-membered aliphatic heterocyclyl containing two N atoms, six-membered aliphatic heterocyclyl containing a N atom, and six-membered aliphatic heterocyclyl containing two N atoms; for example, each $R_{1-4}$ is selected from the group consisting of: optionally substituted pyrrolyl, optionally substituted piperidyl, and optionally substituted piperazinyl;

each $R_{1-5}$ is selected from optionally substituted alkyl; for example, each $R_{1-5}$ may be optionally substituted methyl; for example, two $R_{1-5}$ may be included;

$R_2$ contains an optionally substituted substituent;

$R_3$ contains an optionally substituted substituent;

$X_1$ is an optionally substituted atom;

or a pharmaceutically acceptable prodrug, a pharmaceutically active metabolite, and/or a pharmaceutically acceptable salt of said compound.

In one aspect, the present invention provides a compound of formula (I),

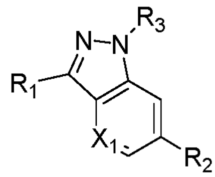
formula (I),

wherein, $R_1$ contains $(C{=}C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$; nr0, nr1, nr2, nr3, nr4 and nr5 are each independently selected from 0 or a larger number;

each $R_{1-1}$ is selected from the group consisting of: optionally substituted aryl and optionally substituted heteroaryl; for example, each $R_{1-1}$ is selected from the group consisting of: optionally substituted aryl, optionally substituted five-membered heteroaryl and optionally substituted six-membered heteroaryl; for example, each $R_{1-1}$ is selected from the group consisting of: optionally substituted aryl, optionally substituted five-membered heteroaryl containing a N atom, five-membered heteroaryl containing two N atoms, six-membered heteroaryl containing a N atom, and optionally substituted six-membered heteroaryl containing two N atoms; for example, each $R_{1-1}$ is selected from the group consisting of: optionally substituted aryl, optionally substituted pyridyl and optionally substituted pyrazolyl;

each $R_{1-2}$ is selected from the group consisting of: chemical bond and hydroxyl; for example, each $R_{1-2}$ may be - or -O-;

each $R_{1-3}$ is selected from optionally substituted alkyl; for example, each $R_{1-3}$ may be optionally substituted methyl, optionally substituted ethyl, and optionally substituted propyl;

each $R_{1-4}$ is selected from optionally substituted aliphatic heterocyclyl; for example, each $R_{1-4}$ may be optionally substituted five-membered aliphatic heterocyclyl and optionally substituted six-membered aliphatic heterocyclyl; for example, each $R_{1-4}$ is selected from the group consisting of: optionally substituted five-membered aliphatic heterocyclyl containing a N atom, five-membered aliphatic heterocyclyl containing two N atoms, six-membered aliphatic heterocyclyl containing a N atom, and six-membered aliphatic heterocyclyl containing two N atoms; for example, each $R_{1-4}$ is selected from the group consisting of: optionally substituted pyrrolyl, optionally substituted piperidyl, and optionally substituted piperazinyl;

each $R_{1-5}$ is selected from optionally substituted alkyl; for example, each $R_{1-5}$ may be optionally substituted methyl; for example, two $R_{1-5}$ may be included;

$R_2$ contains optionally substituted -S-optionally substituted aryl-optionally substituted acylamino-optionally substituted alkyl, or -S-optionally substituted aryl-optionally substituted acylamino-optionally substituted alcyl; for example, $R_2$ contains optionally substituted -S-optionally halo-substituted aryl-optionally substituted acylamino-optionally substituted methyl, or -S-optionally substituted aryl-optionally substituted acylamino-optionally substituted cyclopropyl;

$R_3$ contains hydrogen or an optional isotope of hydrogen;

$X_1$ is optionally substituted CH;

or a pharmaceutically acceptable prodrug, a pharmaceutically active metabolite, and/or a pharmaceutically acceptable salt of said compound.

For example, $R_1$ contains $(R_{1-0})_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein $R_{1-0}$, $R_{1-1}$, $R_{1-2}$, $R_{1-3}$, $R_{1-4}$, and $R_{1-5}$ each independently contain an optionally substituted substituent, and nr0, nr1, nr2, nr3, nr4 and nr5 are each independently selected from 0 or a larger number.

For example, $R_{1-0}$ is a chemical bond or selected from the group consisting of: optionally substituted alkenyl, optionally substituted alkynyl, and optionally substituted amino. For example, $R_{1-0}$ is a chemical bond or selected from the group consisting of: optionally substituted vinyl, optionally substituted ethynyl, and optionally substituted amino. For example, nr0 is 0 or 1. For example, nr0 is 0, 1, 2, or 3.

For example, $R_{1-1}$ is a chemical bond or selected from the group consisting of: optionally substituted acylamino, optionally substituted aryl, and optionally substituted heteroaryl. For example, $R_{1-1}$ is a chemical bond or selected from the group consisting of: optionally substituted acylamino, optionally substituted phenyl, and optionally substituted heteroaryl. For example, each $R_{1-1}$ is selected from the group consisting of: optionally substituted aryl, optionally substituted five-membered heteroaryl and optionally substituted six-membered heteroaryl; for example, each $R_{1-1}$ is selected from the group consisting of: optionally substituted aryl, optionally substituted five-membered heteroaryl containing a N atom, five-membered heteroaryl containing two N atoms, six-membered heteroaryl containing a N atom, and optionally substituted six-membered heteroaryl containing two N atoms; for example, each $R_{1-1}$ is selected from the group consisting of: optionally substituted aryl, optionally substituted pyridyl and optionally substituted pyrazolyl. For example, $R_{1-1}$ is a chemical bond or selected from the group consisting of: optionally substituted acylamino, optionally substituted phenyl, optionally substituted pyridyl and optionally substituted pyrazolyl. For example, nr1 is 0 or 1. For example, nr1 is 0, 1, 2, or 3.

For example, $R_{1-2}$ is a chemical bond or selected from the group consisting of: hydrogen, protium, deuterium, tritium, and optionally substituted hydroxyl. For example, each $R_{1-2}$ may be - or -O-. For example, nr2 is 0 or 1. For example, nr2 is 0, 1, 2, or 3.

For example, $R_{1-3}$ is a chemical bond or selected from the group consisting of: hydrogen, protium, deuterium, tritium, and optionally substituted alkyl. For example, $R_{1-3}$ is a chemical bond or selected from the group consisting of: hydrogen, protium, deuterium, tritium, optionally substituted methyl, optionally substituted ethyl, and optionally substituted propyl. For example, each $R_{1-3}$ may be optionally substituted methyl, optionally substituted ethyl, and optionally substituted propyl. For example, nr3 is 0 or 1. For example, nr3 is 0, 1, 2, or 3.

For example, $R_{1-4}$ is a chemical bond or selected from the group consisting of: hydrogen, protium, deuterium, tritium, optionally substituted amino, and optionally substituted aliphatic heterocyclyl. For example, $R_{1-4}$ is a chemical bond or selected from the group consisting of: hydrogen, protium, deuterium, tritium, optionally substituted amino, optionally substituted pyrrolyl, optionally substituted piperidyl, optionally substituted piperazinyl, and optionally substituted morpholinyl. For example, each $R_{1-4}$ may be optionally substituted five-membered aliphatic heterocyclyl and optionally substituted six-membered aliphatic heterocyclyl; for example, each $R_{1-4}$ is selected from the group consisting of: optionally substituted five-membered aliphatic heterocyclyl containing a N atom, five-membered aliphatic heterocyclyl containing two N atoms, six-membered aliphatic heterocyclyl containing a N atom, and optionally substituted six-membered aliphatic heterocyclyl containing two N atoms; for example, each $R_{1-4}$ is selected from the group consisting of: optionally substituted pyrrolyl, optionally substituted piperidyl, and optionally substituted piperazinyl. For example, nr4 is 0 or 1. For example, nr4 is 0, 1, 2, or 3.

For example, $R_{1-5}$ is a chemical bond or selected from the group consisting of: hydrogen, protium, deuterium, tritium, and optionally substituted alkyl. For example, $R_{1-5}$ is a chemical bond or selected from the group consisting of: hydrogen, protium, deuterium, tritium, optionally substituted methyl, and optionally substituted ethyl. For example, each $R_{1-5}$ may be optionally substituted methyl; for example, two $R_{1-5}$ may be included. For example, nr5 is 0, 1, or 2. For example, nr5 is 0, 1, 2, or 3.

For example, $R_2$ is optionally substituted sulfydryl; For example, $R_2$ is substituted by one or more $R_{2-1}$, and each said $R_{2-1}$ is independently an optionally substituted substituent. For example, $R_{2-1}$ is optionally substituted aryl; For example, $R_{2-1}$ is optionally substituted phenyl;

For example, $R_{2-1}$ is substituted by one or more $R_{2-2}$, and each said $R_{2-2}$ is independently an optionally substituted substituent. For example, $R_{2-2}$ is selected from the group consisting of: hydrogen, protium, deuterium, tritium, halogen, optionally substituted carbonyl, optionally substituted carboxyl, optionally substituted acylamino, and optionally substituted alkyl. For example, $R_{2-2}$ is selected from the group consisting of: hydrogen, protium, deuterium, tritium, halogen, optionally substituted carbonyl, optionally substituted carboxyl, optionally substituted acylamino, and optionally substituted methyl.

For example, $R_{2-2}$ is substituted by one or more $R_{2-3}$, and each said $R_{2-3}$ is independently an optionally substituted substituent. For example, $R_{2-3}$ is selected from the group consisting of: hydrogen, protium, deuterium, tritium, halogen, optionally substituted alkyl, optionally substituted alcyl, and optionally substituted amino. For example, $R_{2-3}$ is selected from the group consisting of: hydrogen, protium, deuterium, tritium, halogen, optionally substituted methyl, optionally substituted ethyl, optionally substituted cyclopropyl, and optionally substituted amino.

For example, $R_{2-3}$ is substituted by one or more $R_{2-4}$, and each said $R_{2-4}$ is independently an optionally substituted substituent. For example, $R_{2-4}$ is selected from the group consisting of: hydrogen, protium, deuterium, tritium, halogen, optionally substituted alkyl, and optionally substituted alcyl. For example, $R_{2-4}$ is selected from the group consisting of: hydrogen, protium, deuterium, tritium, halogen, optionally substituted methyl, optionally substituted ethyl, and optionally substituted cyclopropyl.

For example, $R_2$ contains optionally substituted -S-optionally substituted aryl-optionally substituted acylamino-optionally substituted alkyl, or -S-optionally substituted aryl-optionally substituted acylamino-optionally substituted alcyl; for example, $R_2$ contains optionally substituted -S-optionally halo-substituted aryl-optionally substituted acylamino-optionally substituted methyl, or -S-optionally substituted aryl-optionally substituted acylamino-optionally substituted cyclopropyl; for example, $R_2$ contains optionally substituted -S-F substituted or unsubstituted aryl-acylamino-methyl, or -S-F substituted or unsubstituted aryl-acylamino-cyclopropyl.

For example, $R_3$ is selected from the group consisting of: hydrogen, protium, deuterium, and tritium.

For example, $X_1$ is selected from the group consisting of: optionally substituted CH and N. For example, $X_1$ is optionally substituted CH.

In some embodiments of the present invention, the compound has the following structure:

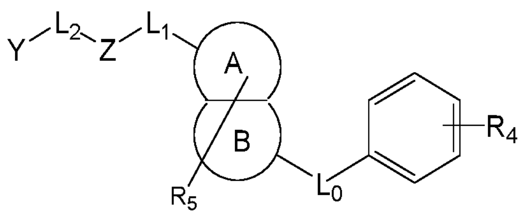

(II)

wherein,

Ring A is five- to seven-membered nitrogen-containing heterocyclyl;
Ring B is five- to seven-membered aryl or heterocyclyl;
Z is -C(O)NH-, -NHC(O)- or

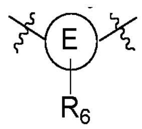

;

Ring E is aryl or heteroaryl;
$L_0$ is selected from: O, S, N($R_{La}$), $C_1$-$C_6$ alkylene, C(O); $R_{La}$ is selected from: H, $C_1$-$C_6$ alkyl;
$L_1$ is selected from: single bond, $C_1$-$C_6$ alkylene, $C_2$-$C_6$ alkenylene, $C_2$-$C_6$ alkinylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, -N($R_{Lb}$)C(O)O-, -N($R_{Lb}$)C(O)N($R_{Lb}$)-, -N($R_{Lb}$)-, -S(O)$_2$-, -S(O)$_2$N($R_{Lb}$)-, -N($R_{Lb}$)S(O)$_2$-, -S(O)-, -S(O)N($R_{Lb}$)-, -N($R_{Lb}$)S(O)-; H atom(s) in the alkylene may be optionally substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxyl, $C_1$-$C_{10}$ alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, four- to ten-membered heterocyclyl, four- to ten-membered heterocyclic alkyl;
$L_2$ is selected from: single bond, $C_1$-$C_6$ alkylene, -($C_0$-$C_6$ alkylene)-$Q_2$-($C_0$-$C_6$ alkylene)-, $Q_2$ is selected from: -O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, - S(O)$_2$-, -S(O)$_2$N($R_{Lc}$)-, -N($R_{rc}$)S(O)$_2$-, -S(O)-, -S(O)N($R_{Lc}$)-, -N($R_{Lc}$)S(O)-; H atom(s) in the alkylene may be optionally substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxyl, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, 4-10 membered heterocyclyl, 4-10 membered heterocyclic alkyl;
Y is selected from: H, -$NR_7R_8$, nitrogen-containing heterocyclyl, wherein, the nitrogen-containing heterocyclyl may be optionally substituted by one or more $R_9$ groups; $R_9$ is selected from: halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), - ($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);
$R_7$, $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, four- to ten-membered heterocyclyl, four- to ten-membered heterocyclic alkyl; wherein, said

$C_1$-$C_{10}$ alkyl, $C_2$-$C_{10}$ alkenyl, $C_2$-$C_{10}$ alkynyl, $C_3$-$C_{10}$ cycloalkyl, four- to ten-membered heterocyclyl may be optionally substituted by one or more groups selected from the group consisting of: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, four- to ten-membered heterocyclyl, four- to ten-membered heterocyclic alkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)$OR_{401}$, -$NR_{402}$C(O)$OR_{401}$, -OC(O)$R_{401}$, -$NR_{402}SO_2R_{401}$, -$SO_2NR_{401}R_{402}$, -$NR_{402}$C(O)$R_{401}$, -C(O)$NR_{401}R_{402}$, -$NR_{401}R_{402}$, -($C_0$-$C_6$ alkylene)-$NR_{401}R_{402}$, -$SR_{401}$, -S(O)$R_{401}$, -S$(O)_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein, said $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, four- to ten-membered heterocyclyl may be optionally substituted by one or more groups selected from the group consisting of: halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_{401}$ and $R_{402}$ are each independently selected from: H, $C_1$-$C_{10}$ alkyl, $C_3$-$C_{10}$ cycloalkyl, $C_3$-$C_{10}$ cycloalkyl alkyl, $C_6$-$C_{10}$ aryl, $C_6$-$C_{10}$ aralkyl, four- to ten-membered heterocyclyl, four- to ten-membered heterocyclic alkyl;

$R_5$ is one or more independent substituents on the

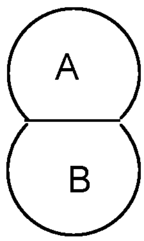

ring, each $R_5$ being independently selected from: H, halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), - $SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_6$ is one or more independent substituents on the E ring, each $R_6$ being independently selected from: H, halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl).

**In** some embodiments of the present invention, Ring A is five-membered nitrogen-containing heteroaryl, such as

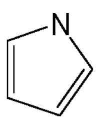, 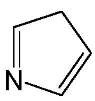, 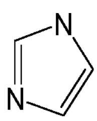, 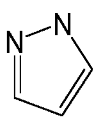, 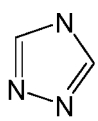.

In some embodiments of the present invention, Ring B is a benzene ring or six-membered nitrogen-containing heterocyclyl, such as

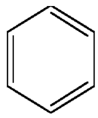, 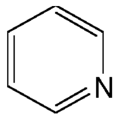, 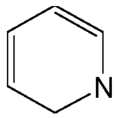, 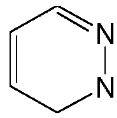.

In some embodiments of the present invention, part

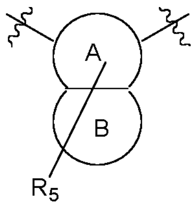

has one of the following structures:

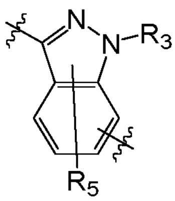, 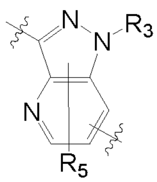, 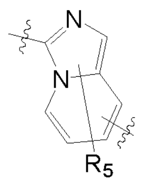, 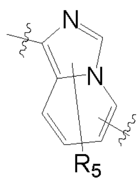, 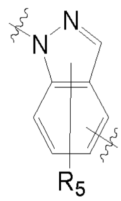, 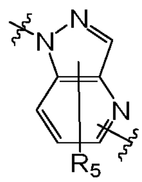,

in particular:

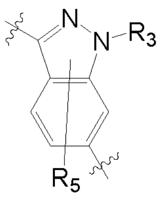,

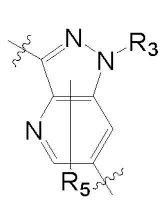, 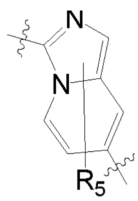, 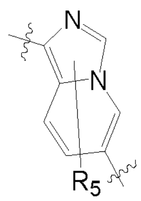, 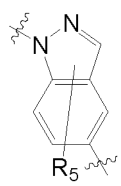。

In some embodiments of the present invention, part

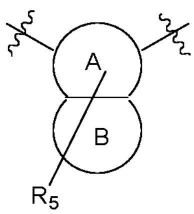

is

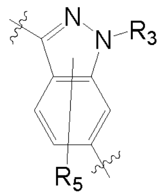

,

$L_2$ is $C_1$-$C_6$ alkylene or - ($C_0$-$C_6$ alkylene)-$Q_2$-($C_0$-$C_6$ alkylene)-, Y is -$NR_7R_8$ or nitrogen-containing heterocyclyl, wherein, the nitrogen-containing heterocyclyl may be optionally substituted by one or more $R_9$ groups.

In some embodiments of the present invention, part

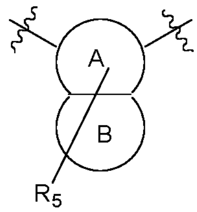

is

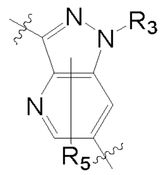

,

Y is selected from: H, - $NR_7R_8$, nitrogen-containing heterocyclyl, wherein, the nitrogen-containing heterocyclyl may be optionally substituted by one or more $R_9$ groups.

In some embodiments of the present invention, part

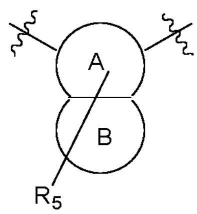

is

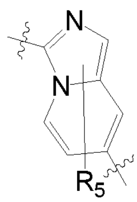

,

Y is selected from: H, - $NR_7R_8$, nitrogen-containing heterocyclyl, wherein, the nitrogen-containing heterocyclyl may be optionally substituted by one or more $R_9$ groups.

In some embodiments of the present invention, part

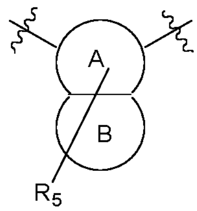

is

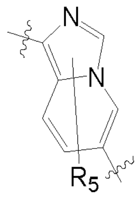

,

Y is selected from: H, - $NR_7R_8$, nitrogen-containing heterocyclyl, wherein, the nitrogen-containing heterocyclyl may be optionally substituted by one or more $R_9$ groups.

In some embodiments of the present invention, part

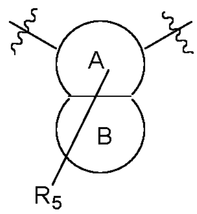

is

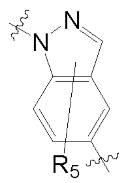

,

Y is selected from: H, $-NR_7R_8$, nitrogen-containing heterocyclyl, wherein, the nitrogen-containing heterocyclyl may be optionally substituted by one or more $R_9$ groups.

In some embodiments of the present invention, $R_5$ is selected from: H, halogen, cyano, amino, hydroxyl, $C_1$-$C_6$ alkyl, $C_1$-$C_6$ haloalkyl.

In some embodiments of the present invention, $R_5$ is H.

In some embodiments of the present invention, $R_3$ is selected from: H, $C_1$-$C_6$ alkyl.

In some embodiments of the present invention, $R_3$ is H.

In some embodiments of the present invention, $L_0$ is S.

In some embodiments of the present invention, $L_0$ is O.

In some embodiments of the present invention, $L_0$ is NH.

In some embodiments of the present invention, $L_0$ is methylene.

In some embodiments of the present invention, $L_0$ is NH, Z is -NHC(O)-, the compound has the following structure:

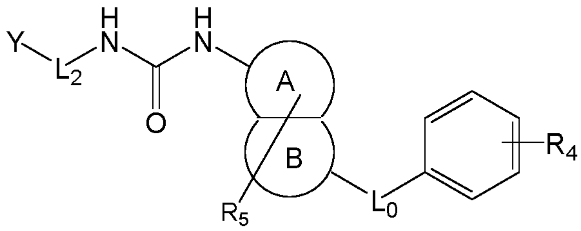

(III-1)

wherein, $L_2$ is selected from: $C_1$-$C_6$ alkylene, -($C_1$-$C_6$ alkylene)-$Q_2$-($C_1$-$C_6$ alkylene)-; Y is -$NR_7R_8$ or optionally substituted nitrogen-containing heterocyclyl, wherein, the nitrogen-containing heterocyclyl may be optionally substituted by one or more $R_9$ groups.

In some embodiments of the present invention, Z is

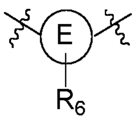

,

the compound has the following structure:

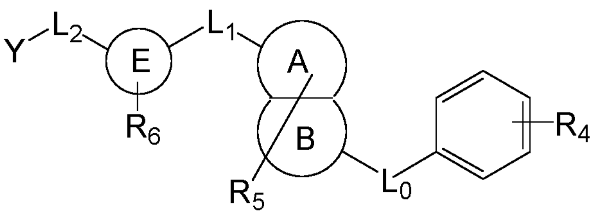

(III-2).

In some embodiments of the present invention, the compound has the following structure:

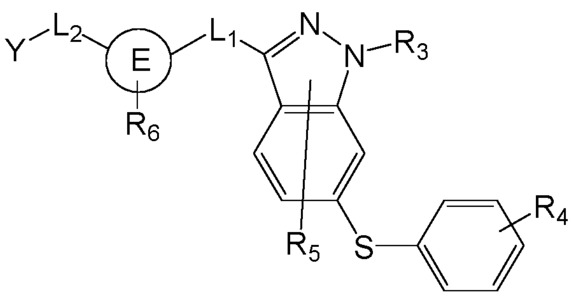

(IV).

In some embodiments of the present invention, $L_1$ is $C_2$-$C_6$ alkenylene, such as

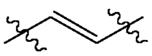

,

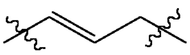

,

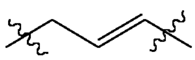

,

in particular

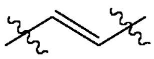

.

In some embodiments of the present invention, $L_1$ is $C_2$-$C_6$ alkinylene, such as

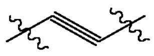, 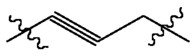,

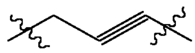,

in particular

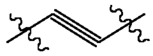.

In some embodiments of the present invention, $L_1$ is -$Q_1$-($C_0$-$C_6$ alkylene)-.

In some embodiments of the present invention, $L_1$ is -($C_0$-$C_6$ alkylene)-$Q_1$-.

In some embodiments of the present invention, $Q_1$ is -N($R_{Lb}$)-, and $R_{Lb}$ is selected from: H, $C_1$-$C_3$ alkyl, in particular H;

In some embodiments of the present invention, $L_1$ is -NH-.

In some embodiments of the present invention, Ring E is a benzene ring or five- or six-membered nitrogen-containing heteroaryl, such as

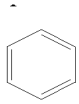, 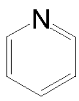, 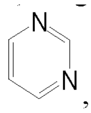, 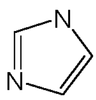, 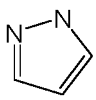

. Specifically, when Ring E is a benzene ring, Y is -$NR_7R_8$ or optionally substituted nitrogen-containing heterocyclyl, wherein, the nitrogen-containing heterocyclyl may be optionally substituted by one or more $R_9$ groups.

In some embodiments of the present invention, part

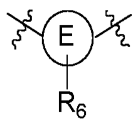

has the following structure:

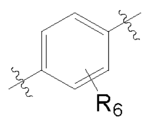,

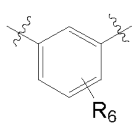, 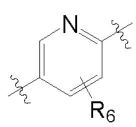, 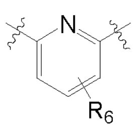, 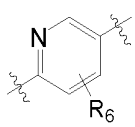, 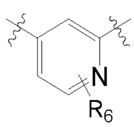, 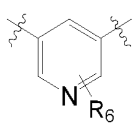, 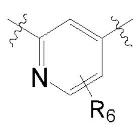, 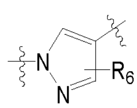,

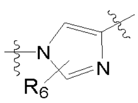, 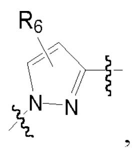, 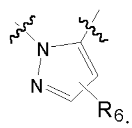.

In some embodiments of the present invention, $R_6$ is selected from: H, halogen, cyano, amino, hydroxyl, $C_1$-$C_6$ alkyl, $C_1$-$C_6$ haloalkyl.

In some embodiments of the present invention, $R_6$ is H.

In some embodiments of the present invention, $L_2$ is a single bond.

In some embodiments of the present invention, $L_2$ is $C_1$-$C_6$ alkylene, such as

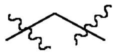, 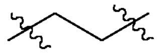,

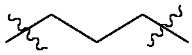.

In some embodiments of the present invention, $L_2$ is -$Q_2$-($C_0$-$C_6$ alkylene)-.

In some embodiments of the present invention, $Q_2$ is selected from: -O-, -S-, -N($R_{Lc}$)-, and $R_{Lc}$ is selected from: H, $C_1$-$C_3$ alkyl, in particular H.

In some embodiments of the present invention, $L_2$ is -O-($C_0$-$C_6$ alkylene)-, such as

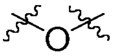, 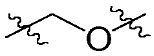,

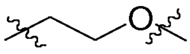, 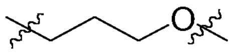.

In some embodiments of the present invention, Ring E is five- or six-membered nitrogen-containing heteroaryl, $L_2$ is a single bond, Y is H.

In some embodiments of the present invention, Y is -$NR_7R_8$, and $R_7$, $R_8$ are each independently selected from: H, $C_1$-$C_6$ alkyl, -($C_0$-$C_6$ alkylene)-($C_3$-$C_6$ cycloalkyl), such as

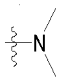, 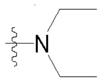.

In some embodiments of the present invention, Y is nitrogen-containing heterocyclyl and may be optionally substituted by one or more $R_9$, specifically, the nitrogen-containing heterocyclyl is four- to eight-membered saturated heterocyclyl, such as,

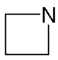, 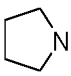, 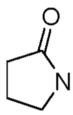, 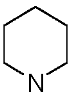, 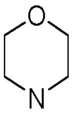, 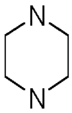, 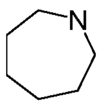, 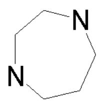.

In some embodiments of the present invention, Y is selected from:

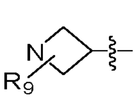, 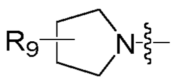, 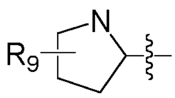,

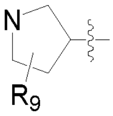, 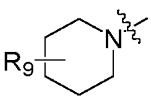, 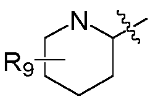, 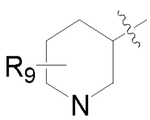, 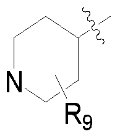, 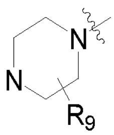, 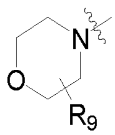, 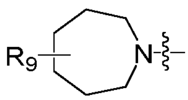,

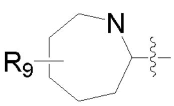, 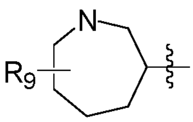, 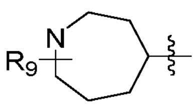, 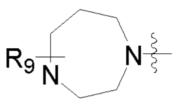

(the substituent position of $R_9$ can be at any suitable carbon atom or nitrogen atom), more specifically, Y is selected from:

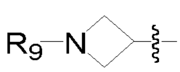, 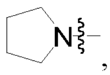, 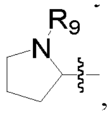,

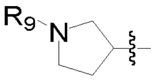, 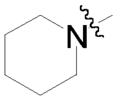, 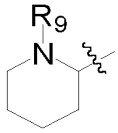, 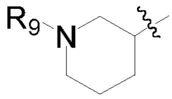, 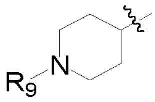, 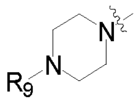, 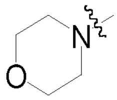, 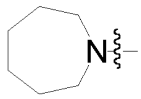,

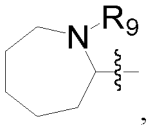, 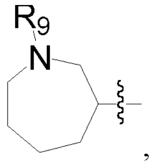, 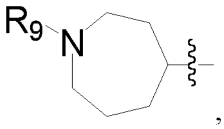, 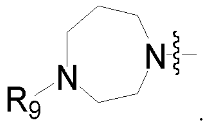.

In some embodiments of the present invention, $R_9$ is selected from: H, $C_1$-$C_6$ alkyl (for example -$CH_3$,

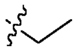,

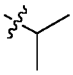, 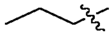),

$C_1$-$C_6$ hydroxyl substituted alkyl (for example

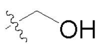, 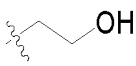),

$C_1$-$C_6$ amino substituted alkyl (for example

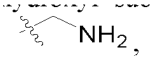, 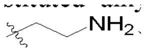

), $C_1$-$C_6$ alkoxy substituted alkyl (for example

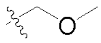,

,

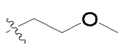),

$C_1$-$C_6$ alkylamino substituted alkyl (for example

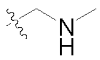, 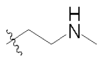, 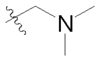, 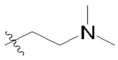),

-($C_0$-$C_6$ alkylene)-($C_3$-$C_6$ cycloalkyl) (for example

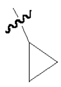, 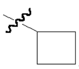, 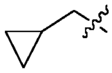, 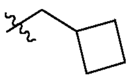).

In some embodiments of the present invention, Y is selected from:

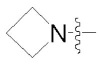, 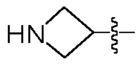, 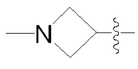,

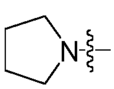, 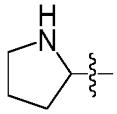, 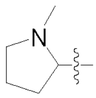, 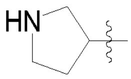, 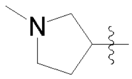, 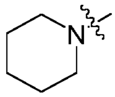, 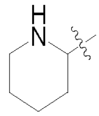, 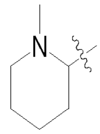, 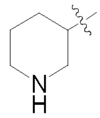,

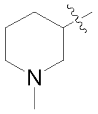, 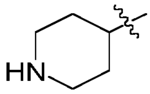, 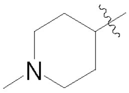, 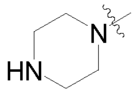, 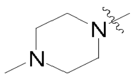, 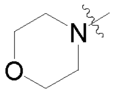, 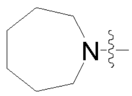, 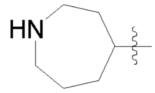,

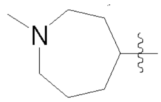, 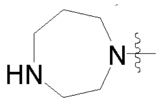, 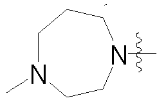.

In some embodiments of the present invention, part

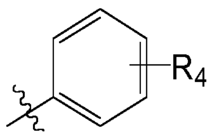

is

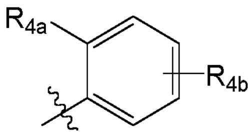,

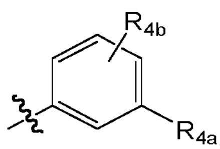, or 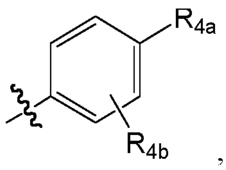,

wherein,

$R_{4a}$ is selected from: $-C(O)NR_{401}R_{402}$, $-(C_0-C_6$ alkylene)-$NR_{401}R_{402}$, $-C(O)R_{401}$, $-C(O)OR_{401}$, $R_{401}$ and $R_{402}$ are independently selected from: H, $C_1-C_6$ alkyl, $-(C_0-C_6$ alkylene)-$(C_3-C_6$ cycloalkyl); wherein, said $C_0-C_6$ alkylene, $C_1-C_6$ alkyl, $C_3-C_6$ cycloalkyl may be optionally substituted by one or more groups selected from the group consisting of: halogen, cyano, amino, hydroxyl, $C_1-C_6$ alkyl, $C_1-C_6$ haloalkyl;

$R_{4b}$ is one or more independent substituents on the benzene ring, which are selected from: H, halogen, cyano, amino, hydroxyl, $C_1-C_6$ alkyl, $C_1-C_6$ haloalkyl.

In some embodiments of the present invention, $R_{4a}$ is selected from:

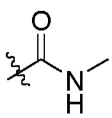, 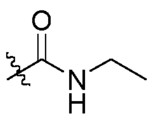, 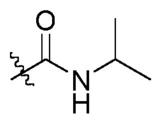,

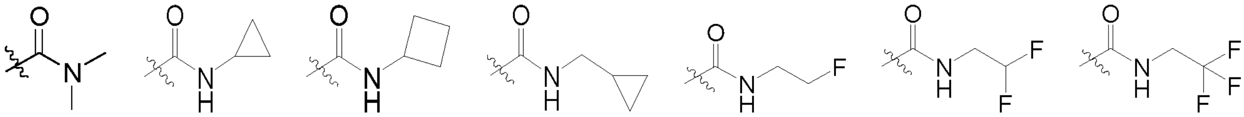, 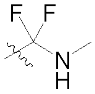, 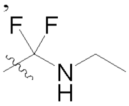, 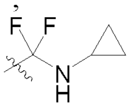, 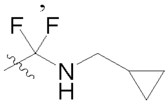, 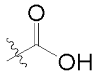, 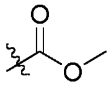, 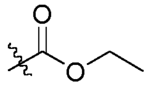, 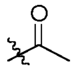, 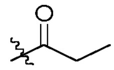,

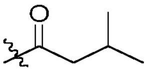, 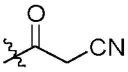, 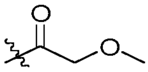, 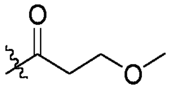, 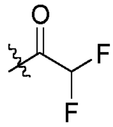, 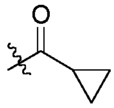, 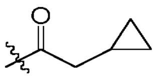.

In some embodiments of the present invention, $R_{4b}$ is H.

In some embodiments of the present invention, $R_{4b}$ is halogen, such as F.

In some embodiments of the present invention, part

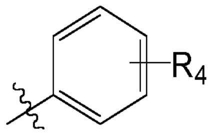

is

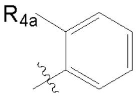, 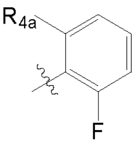,

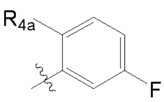, 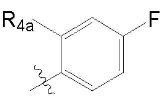, 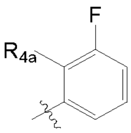, 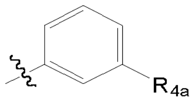, 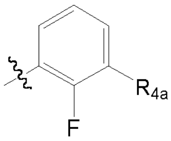, 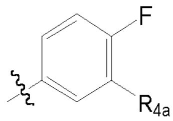, 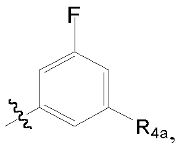, 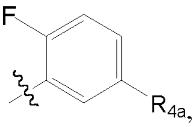, 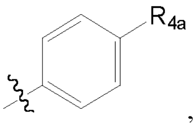, 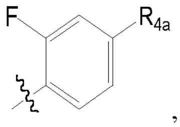, 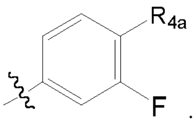.

In one aspect, the present invention provides a compound having a structure selected from the group consisting of:

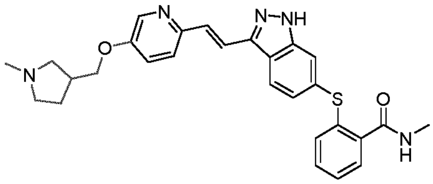 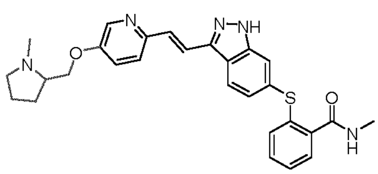 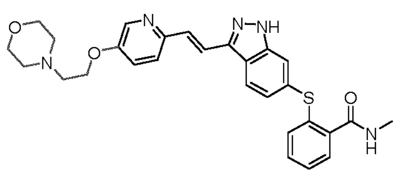

, , ,

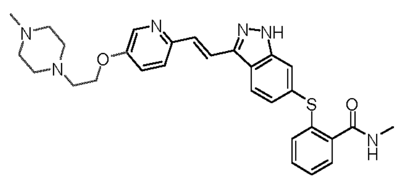
,
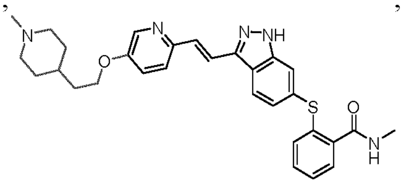
,
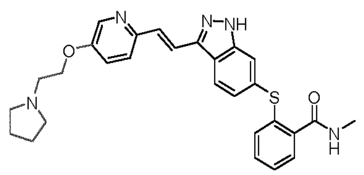
,
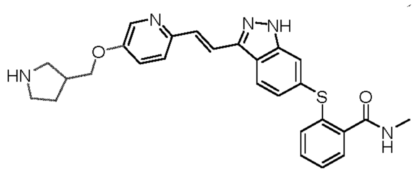
,
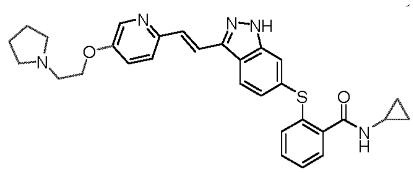
,
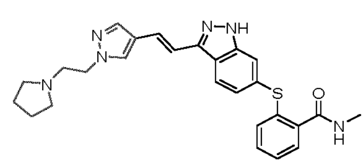
,
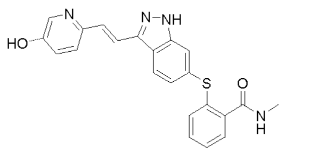
,
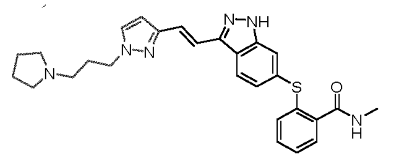
,
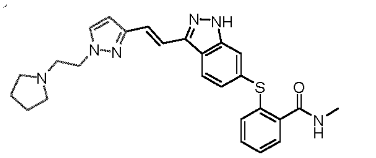
,
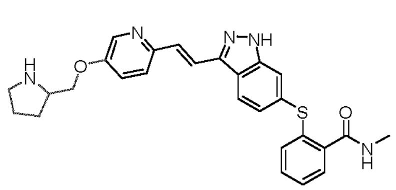
,
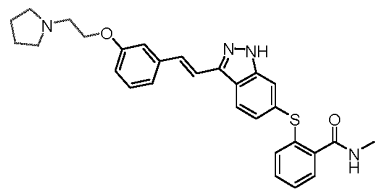
,
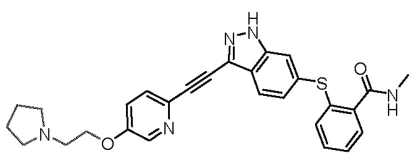
,
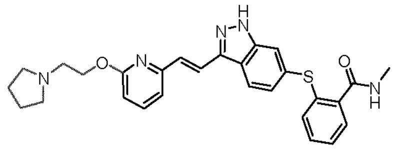
,
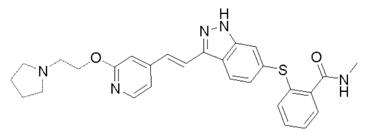
,
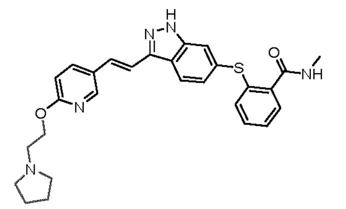
,
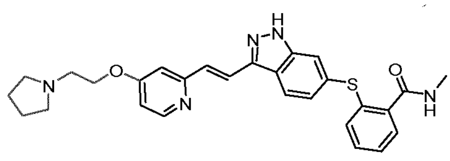
,
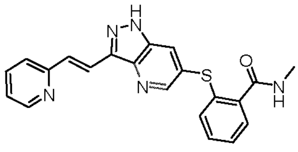
,
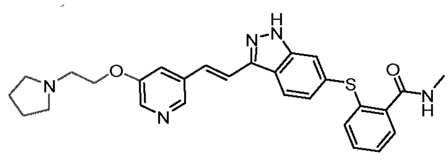
,
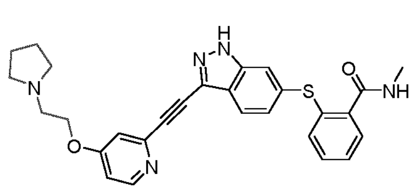
,
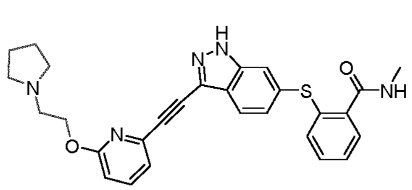
,
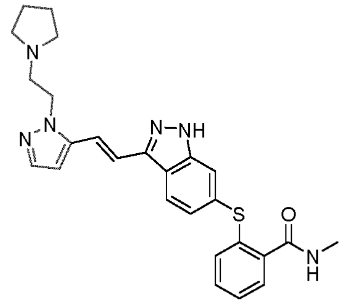
,

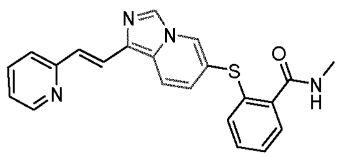 , 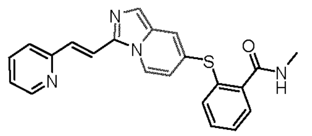 , 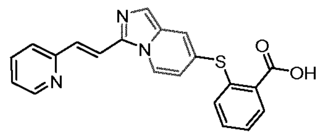 ,

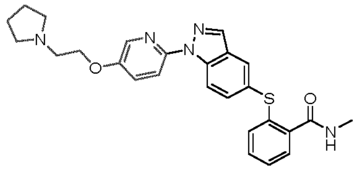 , 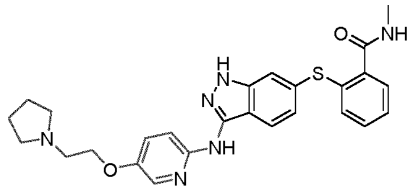 , 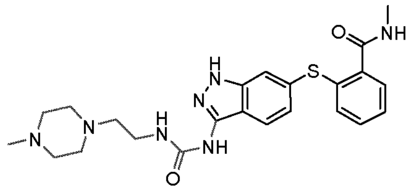 ,

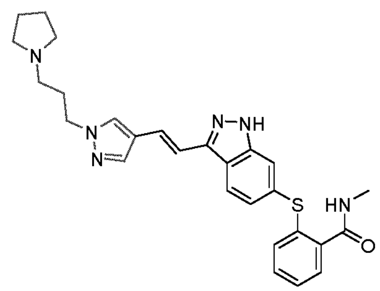 , 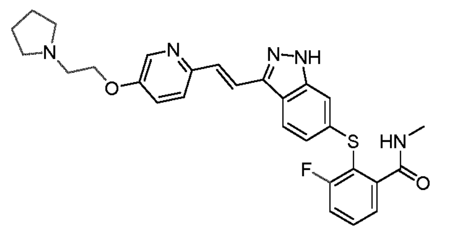 ,

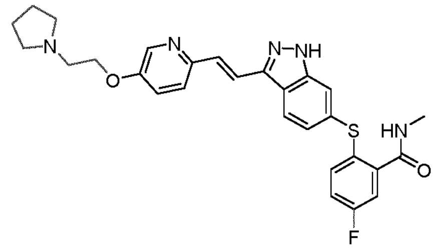 , 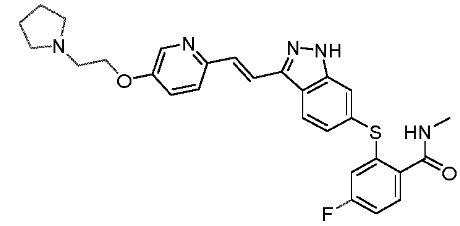 ,

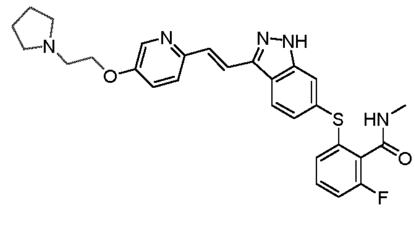 , 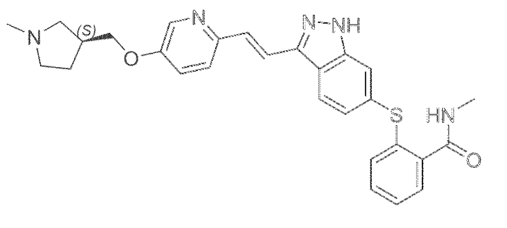 ,

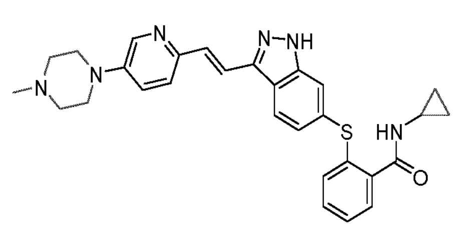 , 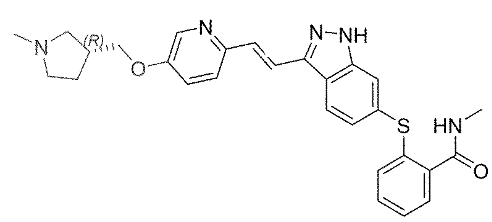 ,

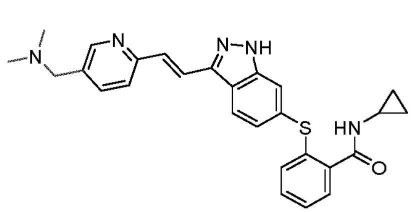 , 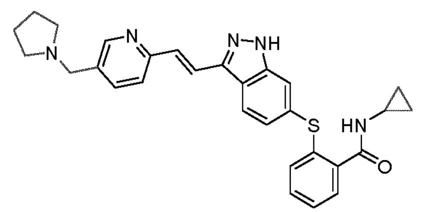 ,

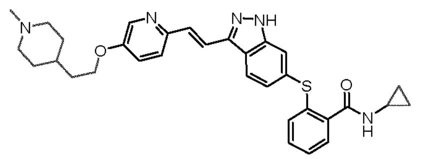,
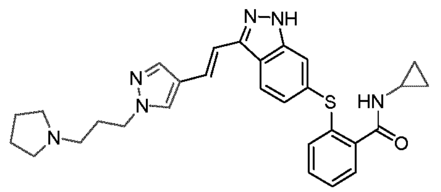,

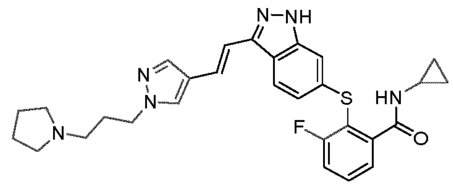,
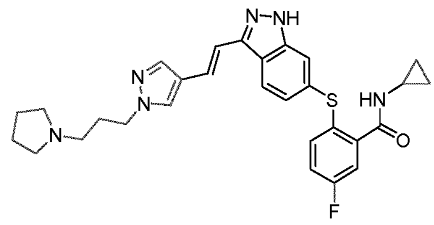,

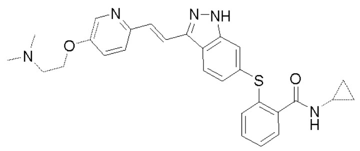,
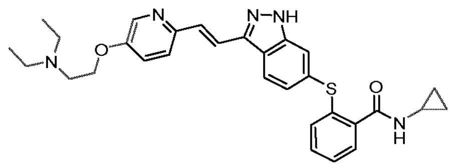,
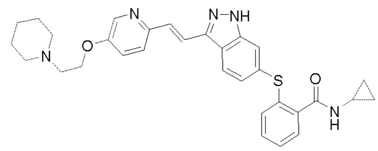,

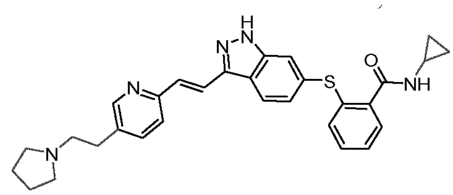,
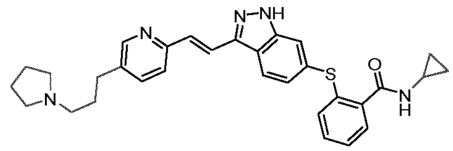,

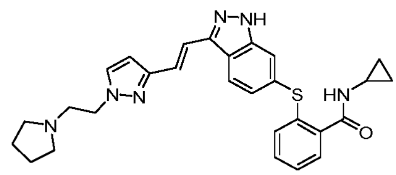,
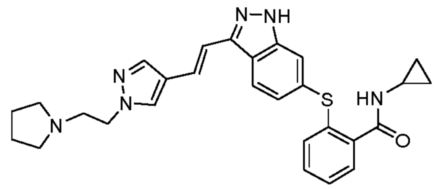,
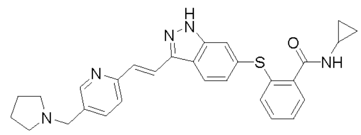,

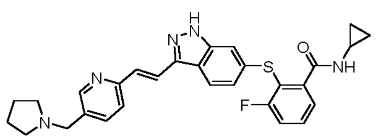,
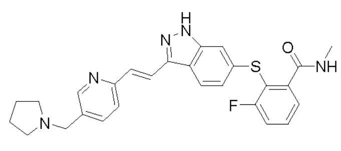,
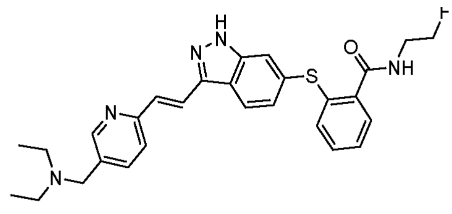,

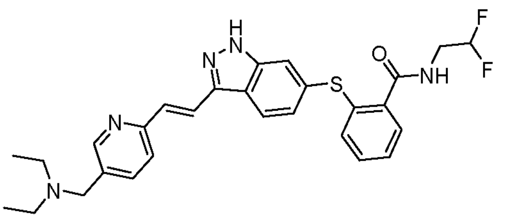 and 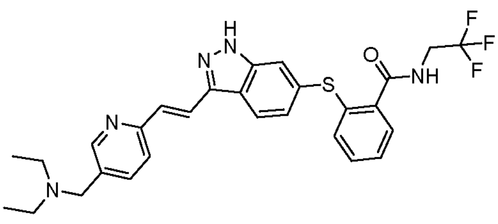,

or a pharmaceutically acceptable prodrug, a pharmaceutically active metabolite, and/or a pharmaceutically acceptable salt of said compound.

In one aspect, the present invention provides a compound having a structure selected from the group consisting

of:

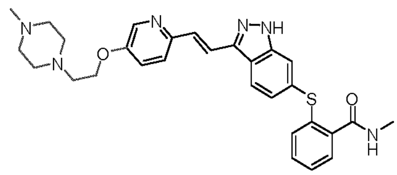,
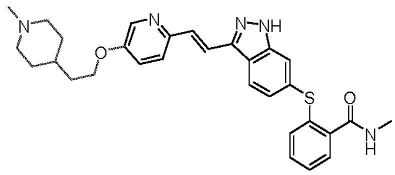,
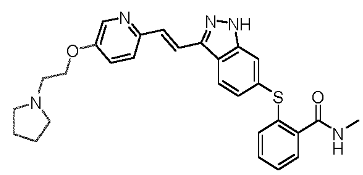,

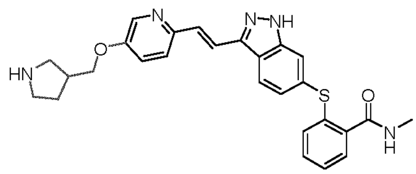,
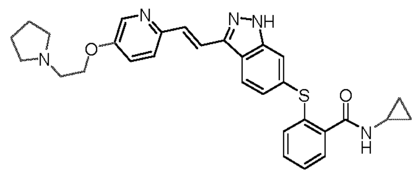,
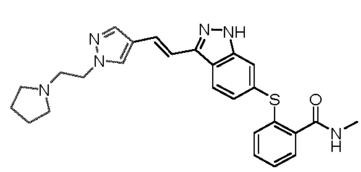,

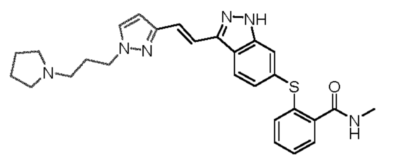,
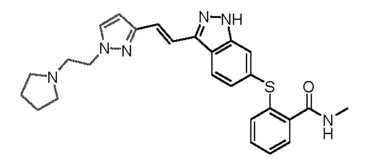,
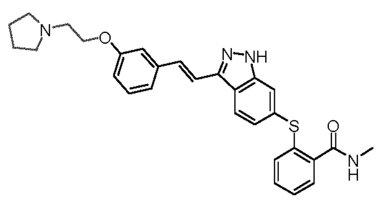,

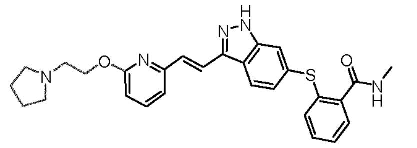,
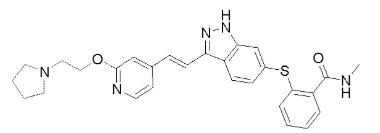,
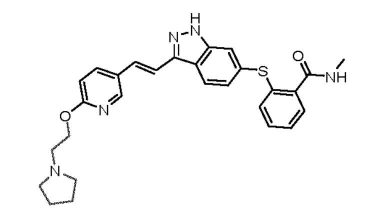,

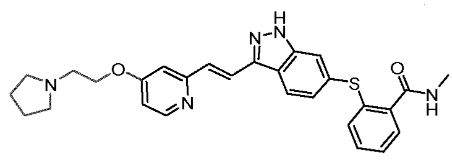,
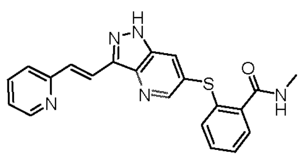,
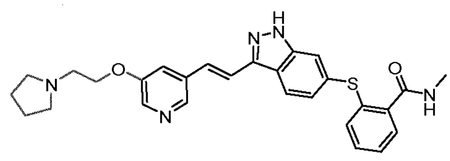,

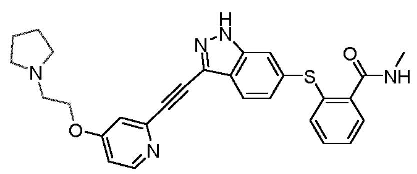,
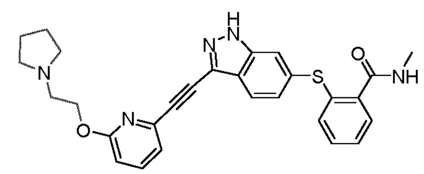,
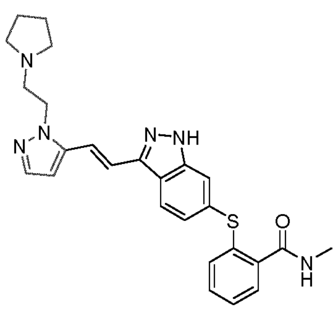,

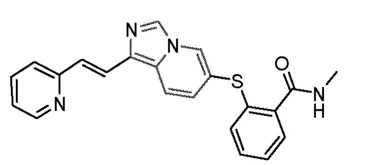,
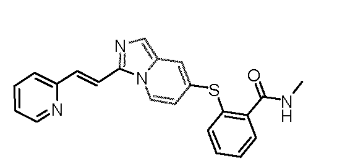,
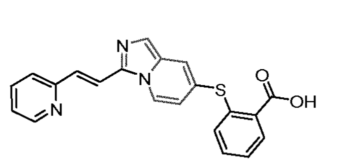,

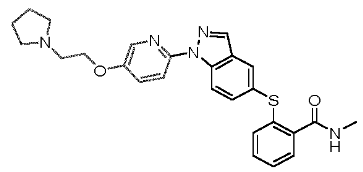,
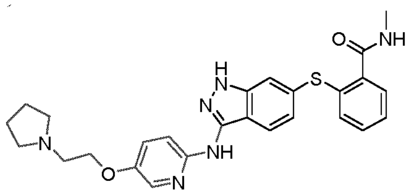,
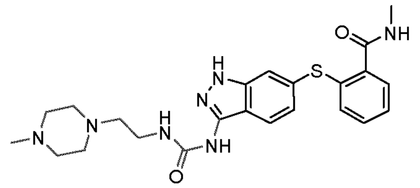,

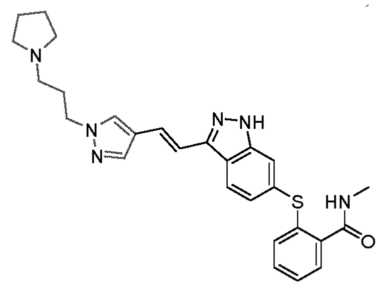,
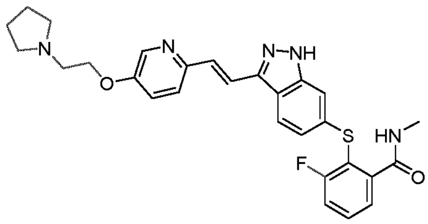,

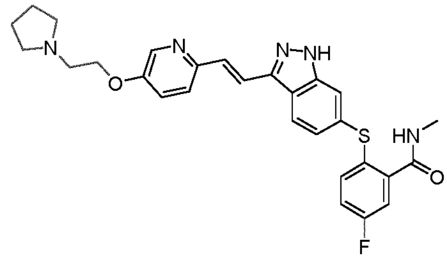,
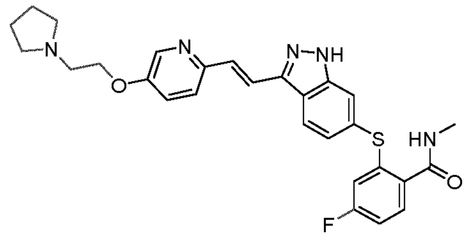,

and

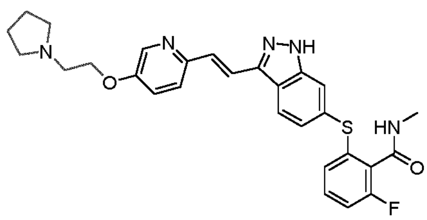,

or a pharmaceutically acceptable prodrug, a pharmaceutically active metabolite, and/or a pharmaceutically acceptable salt of said compound.

In one aspect, the present invention provides a compound having a structure selected from the group consisting of:

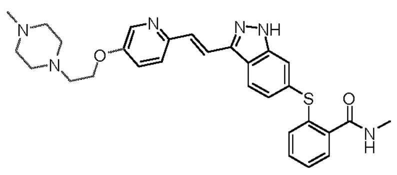,
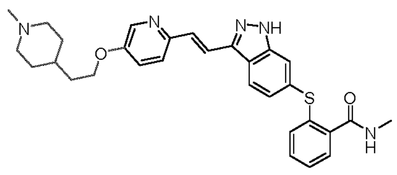,
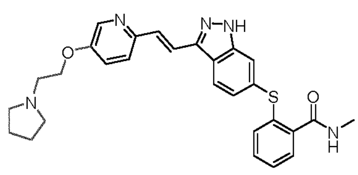,

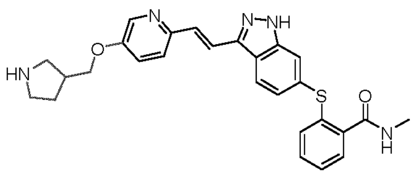,
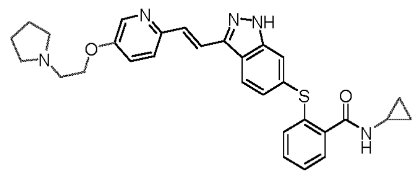,
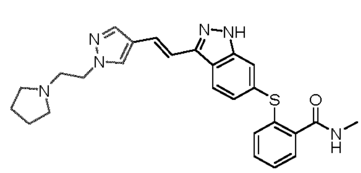,

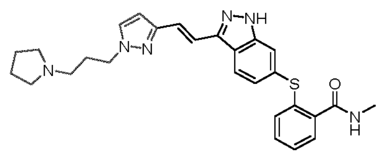,
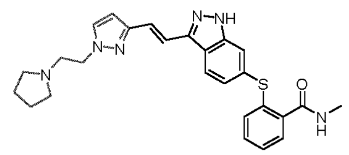,
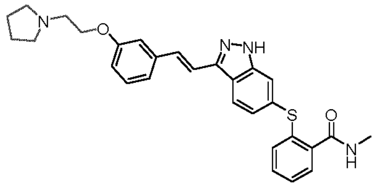,

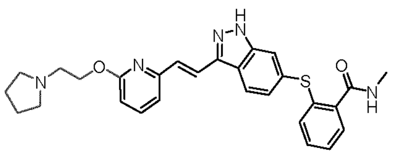,
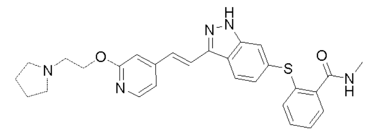,
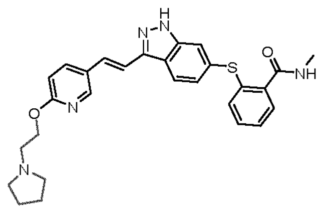,

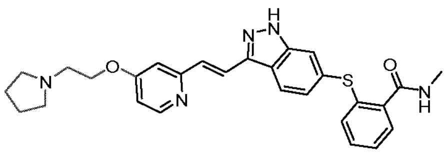,
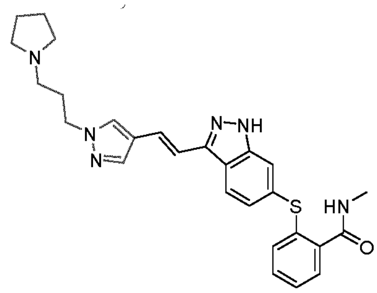,

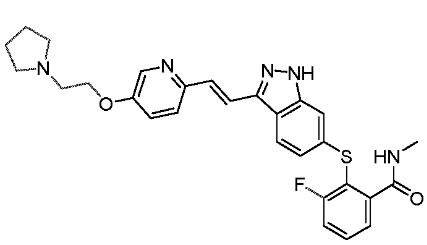,
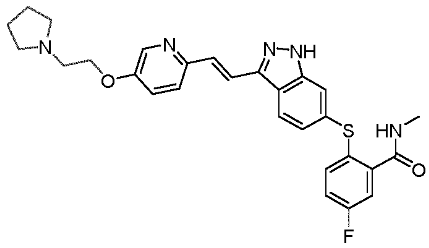,

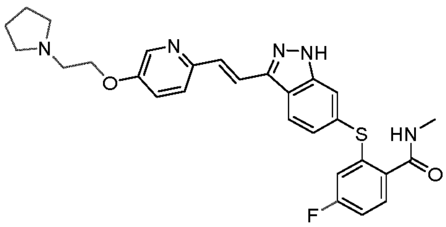, and
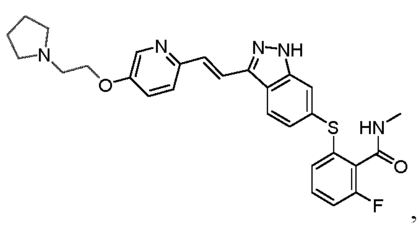,

or a pharmaceutically acceptable prodrug, a pharmaceutically active metabolite, and/or a pharmaceutically acceptable salt of said compound.

In one aspect, the present invention provides a compound having a structure selected from the group consisting of:

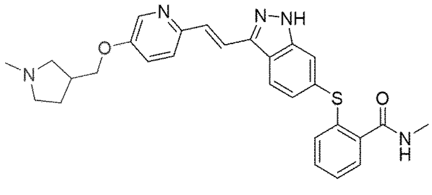
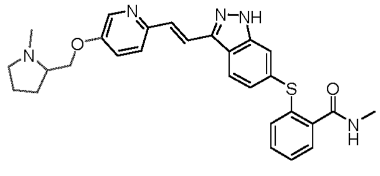
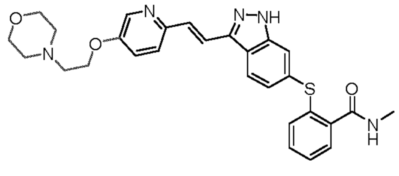

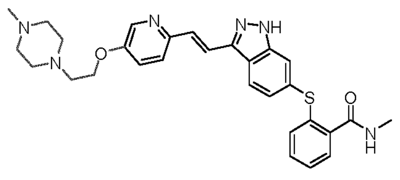,
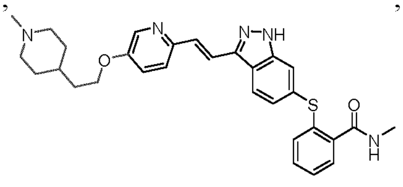,
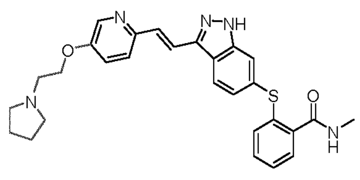,

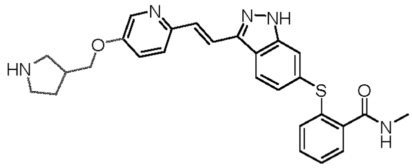,
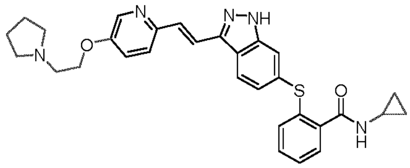,
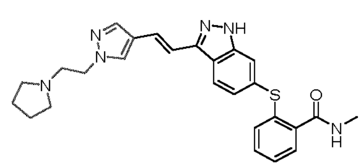,

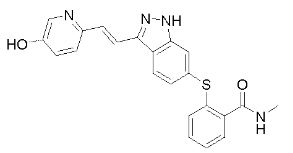,
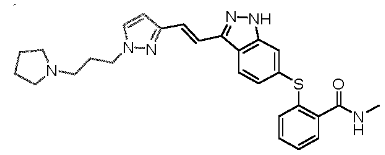,
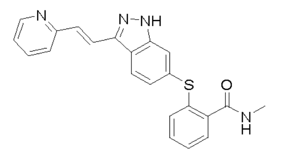,

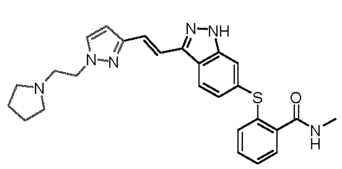,
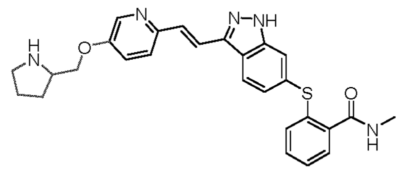,
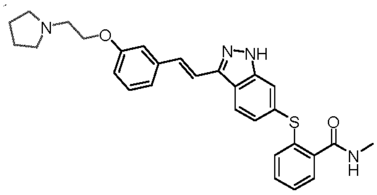,

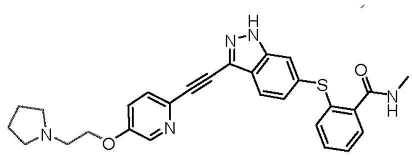,
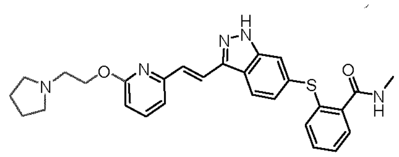,
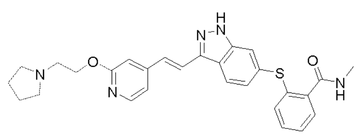,

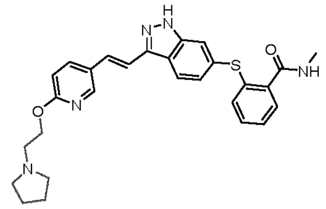,
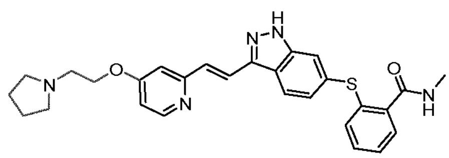,
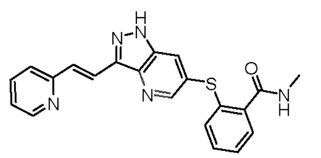,

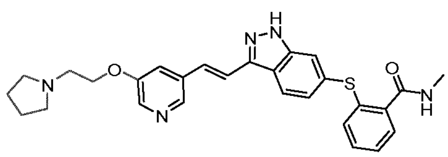,
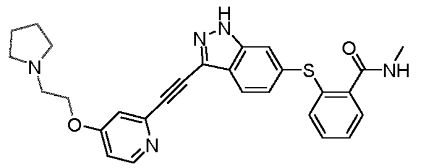,

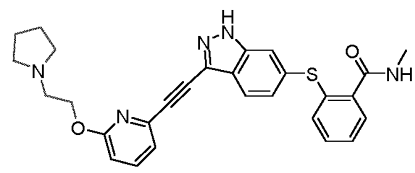,
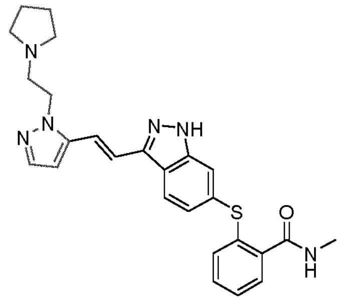,
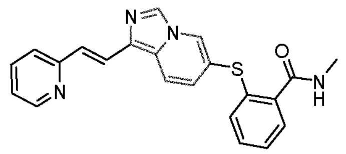,

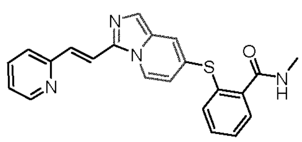,
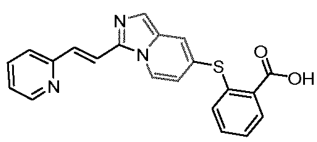,
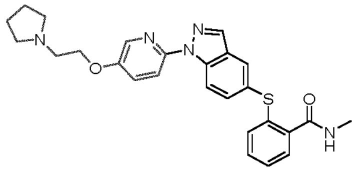,

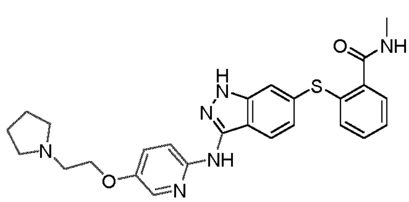,
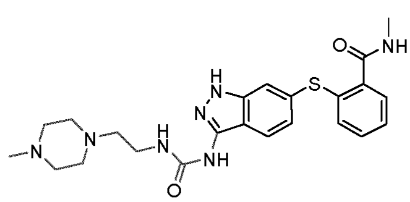,
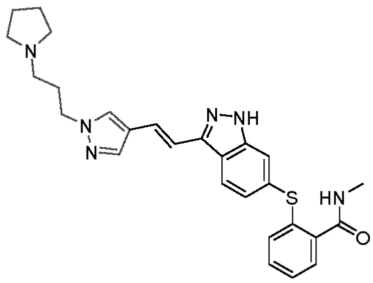,

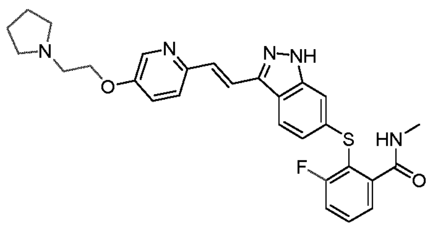,
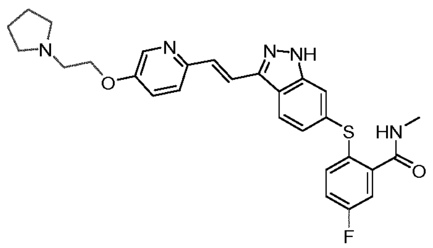,

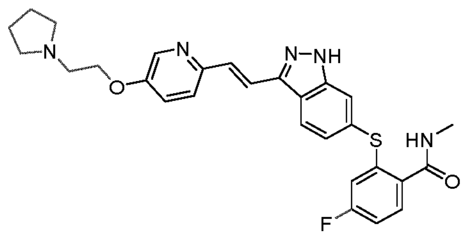,
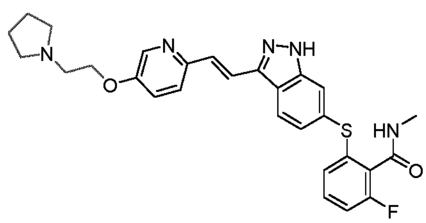,

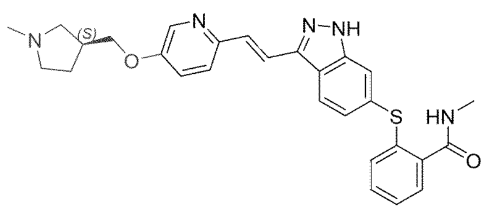.
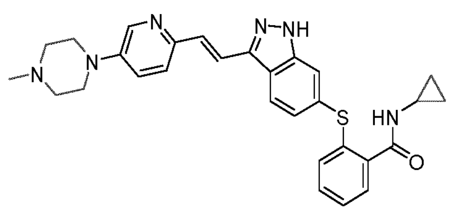.

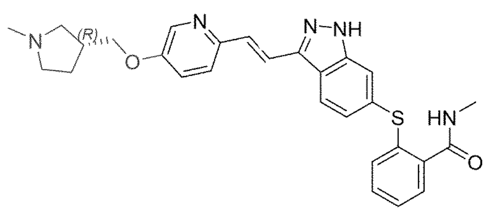

,

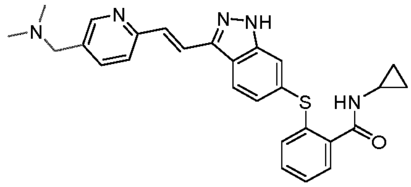

,

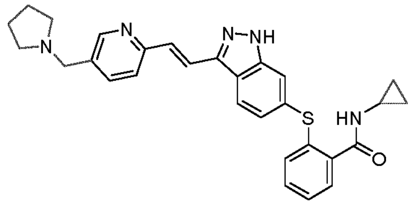

,

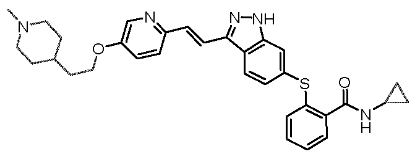

,

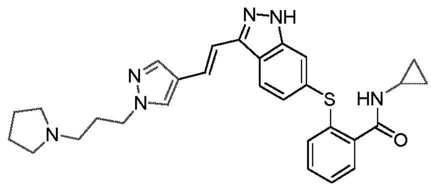

,

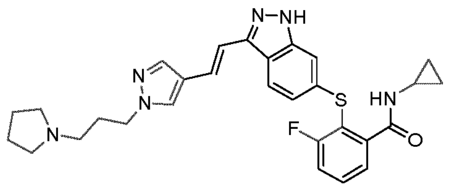

,

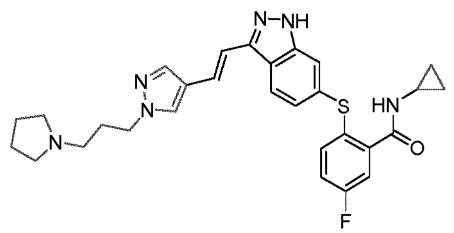

,

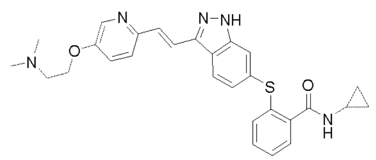

,

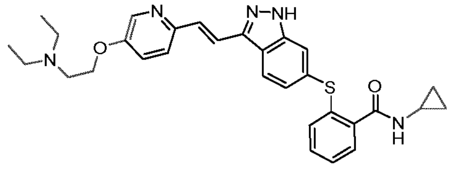

,

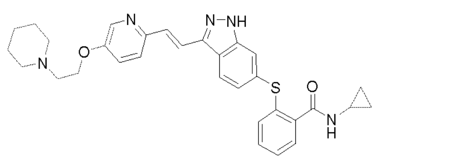

,

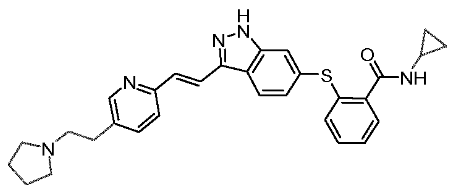

,

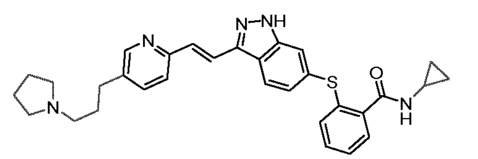

,

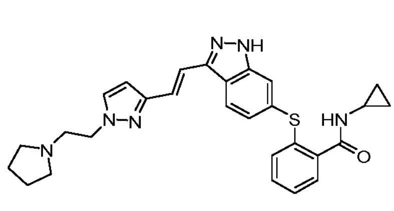

,

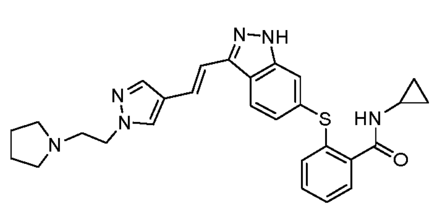

,

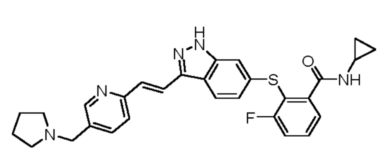

,

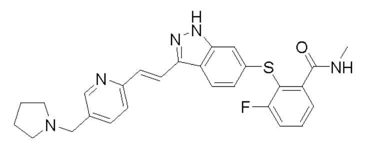
,
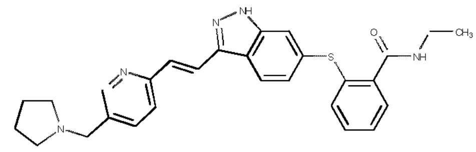
,
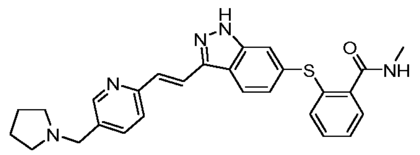
,
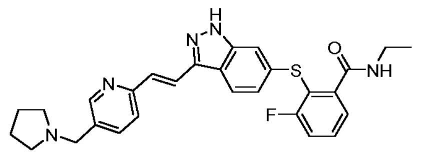
,
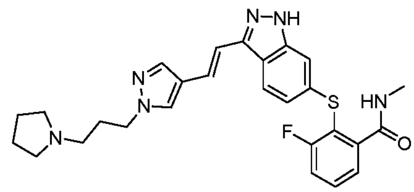
,
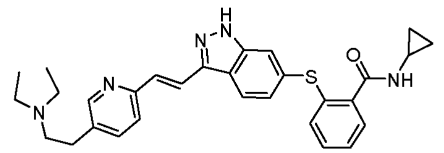
,
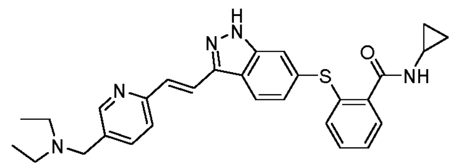
,
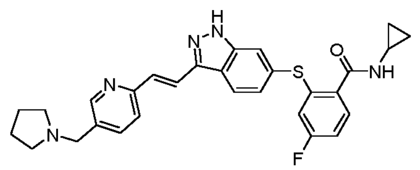
,
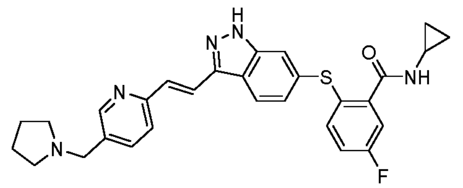
,
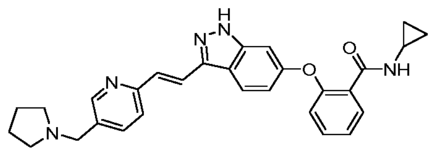
,
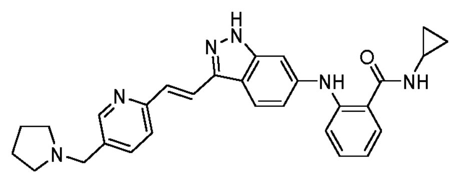
,
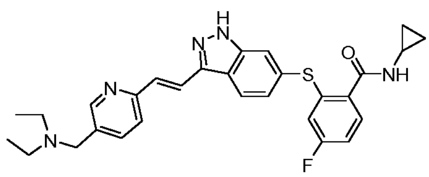
,
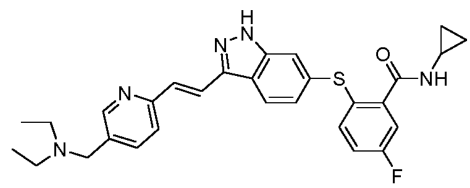
,
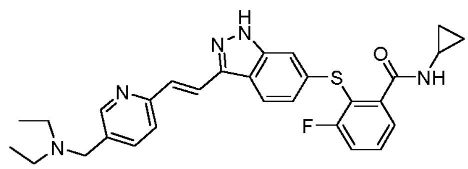
,
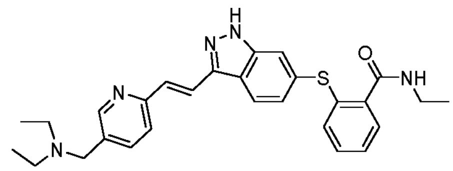
,
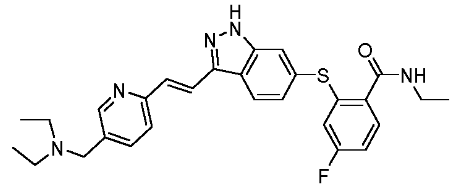
,

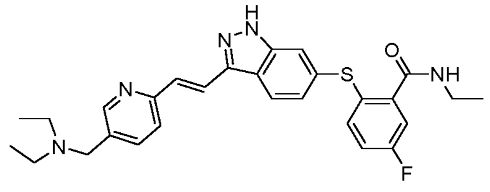,

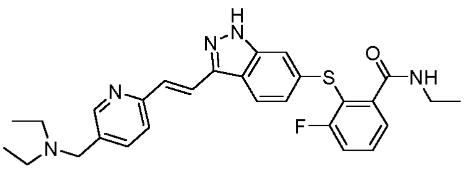,

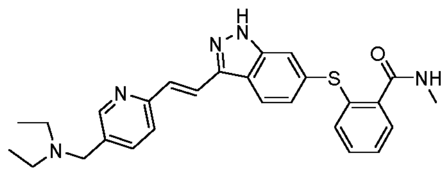,

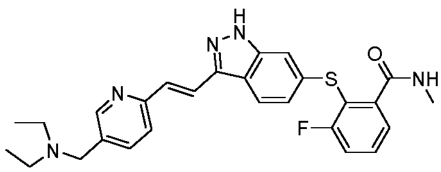,

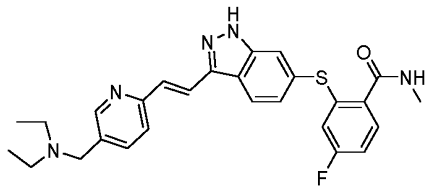,

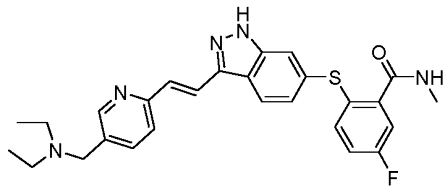,

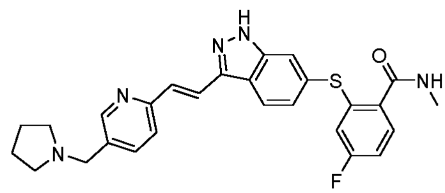,

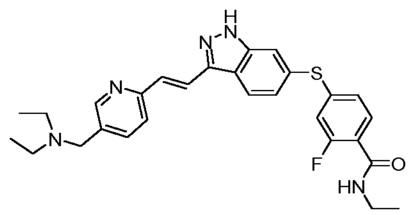,

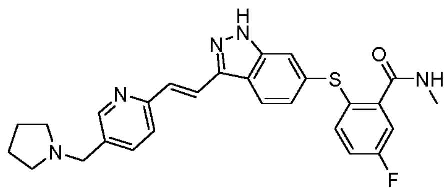,

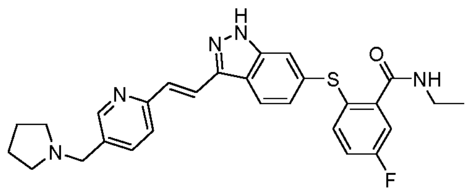,

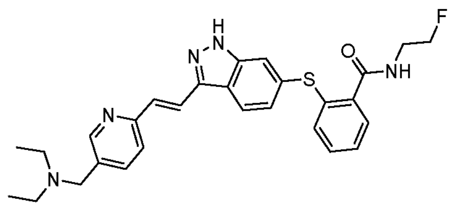,

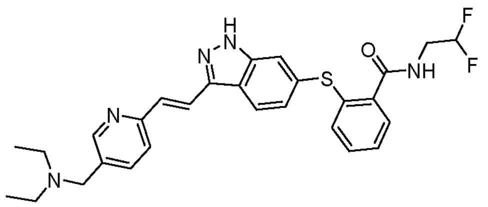,

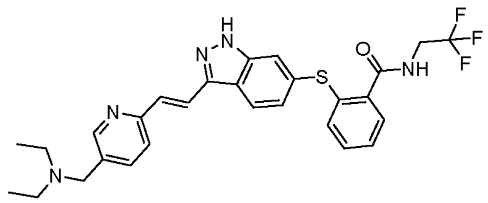,

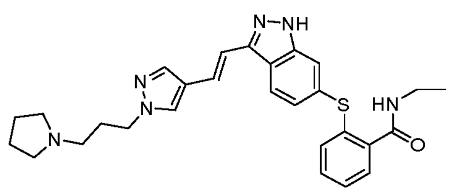,

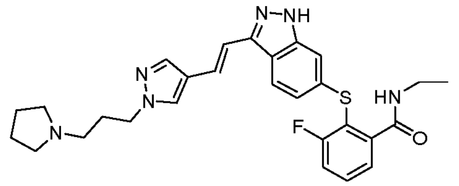,
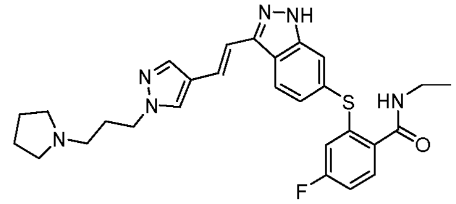,

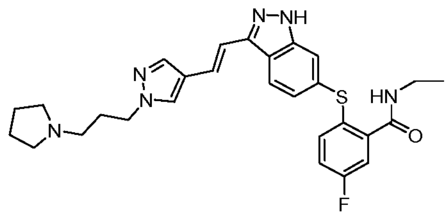, and
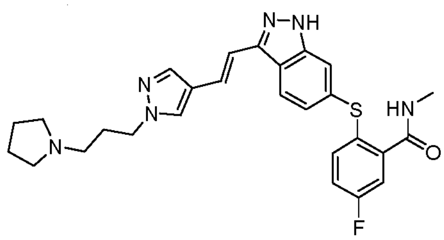,

or a pharmaceutically acceptable prodrug, a pharmaceutically active metabolite, and/or a pharmaceutically acceptable salt of said compound.

In one aspect, the present invention provides a compound having a structure selected from the group consisting of:

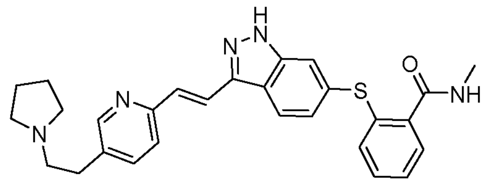,
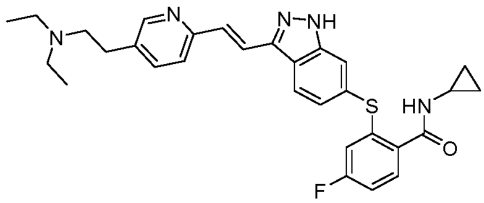,
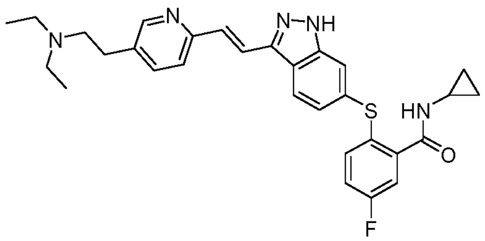,
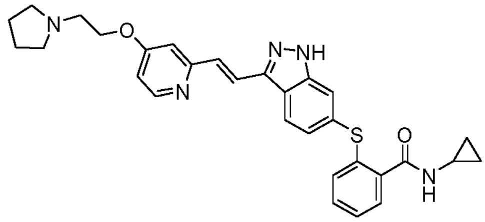,
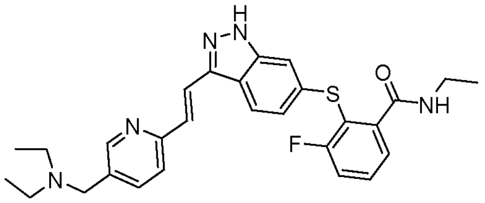,
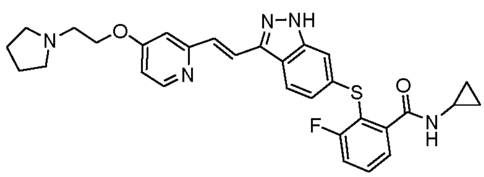,
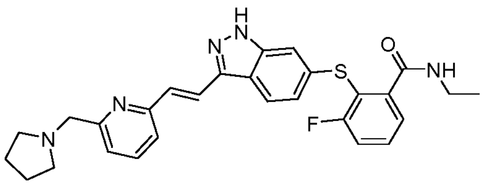,
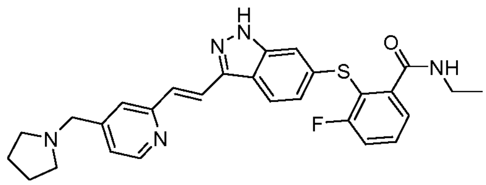,

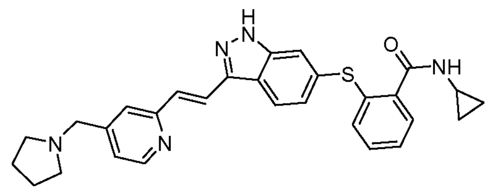
,

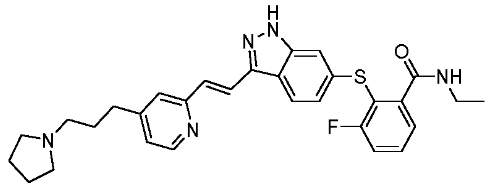
,

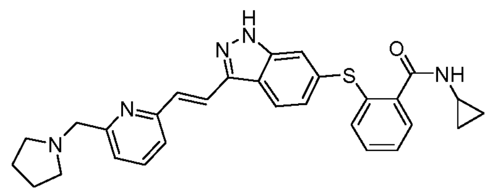
,

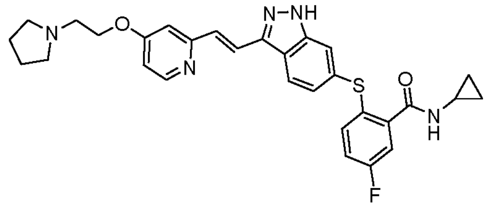
,

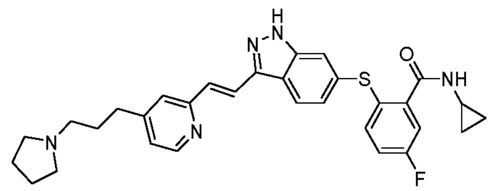
,

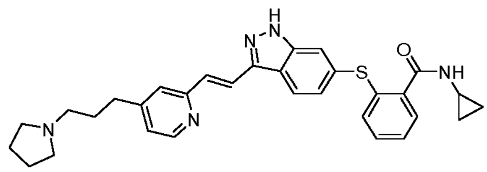
,

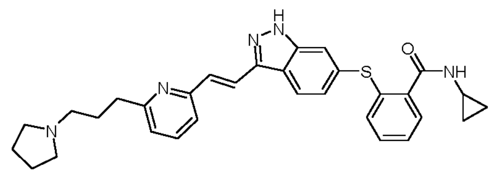
,

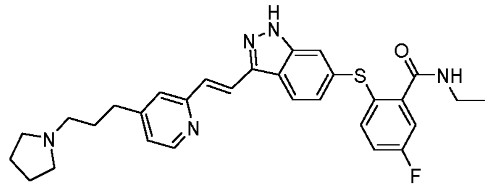
,

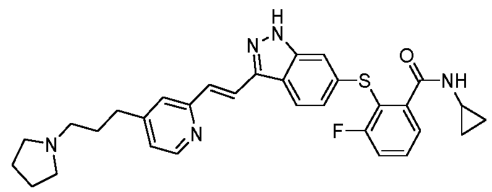
,

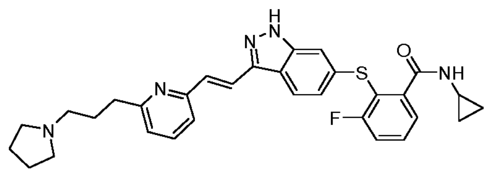
,

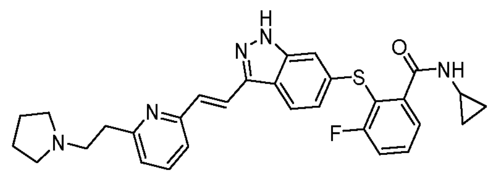
,

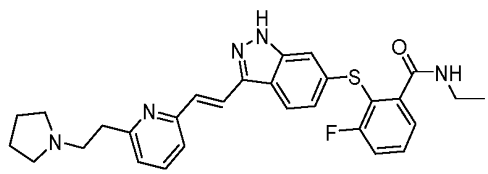
,

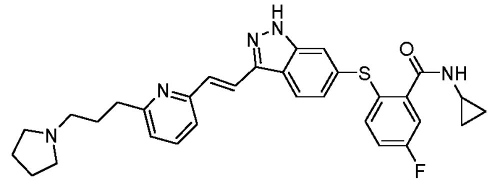
,

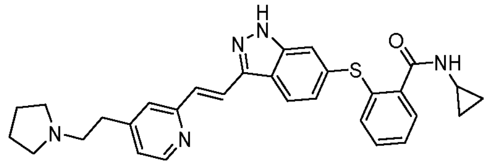
,

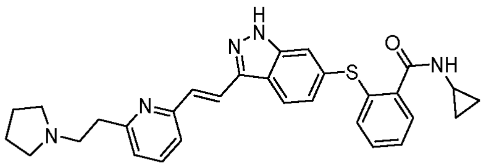,
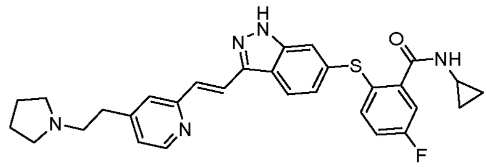,
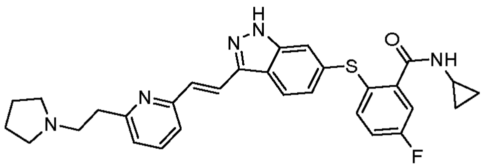,
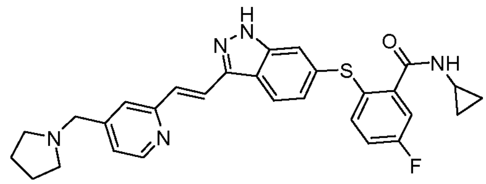,
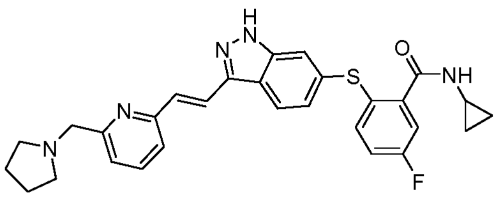,
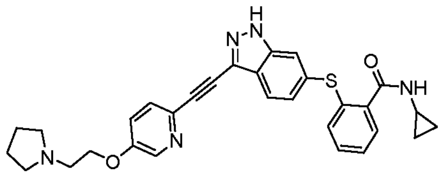

or a pharmaceutically acceptable prodrug, a pharmaceutically active metabolite, and/or a pharmaceutically acceptable salt of said compound.

Pharmaceutical Composition

In one aspect, the present invention provides a pharmaceutical composition, comprising the compound disclosed herein or a pharmaceutically acceptable prodrug, a pharmaceutically active metabolite, and/or a pharmaceutically acceptable salt of said compound, as well as an optional carrier.

The pharmaceutical composition described in the present invention may contain one or more excipients in addition to the active compound. The excipients may be selected from the group consisting of: fillers (diluents), binders, wetting agents, disintegrants, and excipients. Depending on the route of administration, the composition may contain 0.1 to 99 weight percent of the active compound.

The pharmaceutical composition containing the active ingredient can be in a form suitable for ocular administration, such as aqueous or oily suspensions, dispersible powders or granules, emulsions, or slurries. The composition for ocular administration can be prepared according to any known methods for preparing pharmaceutical compositions in the field. The composition may contain binders, fillers, lubricants, disintegrants, or pharmaceutically acceptable wetting agents, etc. It may also contain one or more ingredients selected from the group consisting of: sweeteners, flavoring agents, coloring agents, and preservatives.

Aqueous suspensions may contain the active substance and excipients for mixing with the suitably prepared aqueous suspensions. Aqueous suspensions may also contain one or more preservatives, such as one or more coloring agents, one or more flavoring agents, and one or more sweeteners. Oily suspensions can be prepared by suspending the active ingredients in vegetable oils. Oily suspensions may contain thickeners.

Specifically, the pharmaceutical composition may be delivered via any suitable route of administration, such as gastrointestinal administration (e.g., oral, sublingual, rectal) or non-gastrointestinal administration (e.g., intravenous, intramuscular, intranasal, intraocular, intracranial, vaginal, intraperitoneal, transdermal, subcutaneous, intradermal, or respiratory administration). In some embodiments of the present invention, the pharmaceutical composition is administered via intraocular routes (e.g., eye drops, ophthalmic ointments, subconjunctival injections, intraocular injections).

Specifically, the pharmaceutical composition can take any suitable dosage form, such as gastrointestinal dosage forms, including but not limited to tablets, pills, powders, granules, capsules, troches, syrups, liquids, emulsions, suspensions, etc.; non-gastrointestinal dosage forms, such as injectable dosage forms (e.g., for subcutaneous, intravenous, intramuscular, intraperitoneal injection), respiratory dosage forms (e.g., sprays, aerosols, powder aerosols), dermal dosage forms (e.g., topical solutions, lotions, ointments, plasters, pastes, patches), mucosal dosage forms (e.g., eye drops, ophthalmic ointments, nasal drops, gargles, sublingual tablets), and cavity dosage forms (e.g., suppositories, aerosols, effervescent tablets, drops, pills) for the rectum, vagina, urethra, nose, ear canal, etc.

In some embodiments of the present invention, the above-mentioned pharmaceutical composition is an ophthalmic formulation, such as eye drops or ophthalmic ointments.

In one aspect, the present invention provides use of the compound disclosed herein or a pharmaceutically acceptable prodrug, a pharmaceutically active metabolite, and/or a pharmaceutically acceptable salt of the compound, and/or the pharmaceutical composition disclosed herein in the preparation of a medicament for the treatment of diseases. In one aspect, the present invention provides a compound disclosed herein or a pharmaceutically acceptable prodrug, a pharmaceutically active metabolite, and/or a pharmaceutically acceptable salt of the compound, and/or a pharmaceutical composition disclosed herein for the treatment of diseases.

In some embodiments of the present invention, the disease is a protein tyrosine kinase-mediated proliferative disease. In some embodiments of the present invention, the disease is an ocular disease, including but not limited to pathological neovascularization, retinal ischemia, retinal edema, and DR.

In some embodiments of the present invention, the disease is an ocular disease, including but not limited to DR (including simple (background) DR, proliferative DR, and diabetic macular edema); AMD (including neovascular (wet/exudative) AMD, dry AMD, and geographic atrophy); pathological CNV originating from any pathological mechanism (i.e., high myopia, trauma, sickle cell anemia; ocular histoplasmosis, angioid streaks, traumatic choroidal rupture, optic nerve drusen, and certain retinal dystrophies); pathological retinal neovascularization originating from any pathological mechanism (i.e., sickle cell retinopathy, Eales disease (retinal periphlebitis), ocular ischemic syndrome, carotid cavernous sinus fistula, familial exudative vitreoretinopathy, hyperviscosity syndrome, idiopathic occlusive arteritis, birdshot retinochoroidopathy, retinal vasculitis, sarcoidosis, or toxoplasmosis); uveitis; retinal vein occlusion (central or branch); ocular trauma; postsurgical edema; postsurgical neovascularization; cystoid macular edema; ocular ischemia; retinopathy of prematurity; Coat's disease; sickle cell retinopathy and/or neovascular glaucoma;

In some embodiments of the present invention, the ocular disease is a posterior segment disease, which occurs in the vitreous body, retina, choroid, sclera, or optic nerve.

In some embodiments of the present invention, the disease is DR, including simple (background) DR (non-proliferative DR), proliferative DR, and diabetic macular edema, particularly non-proliferative DR.

In other embodiments of the present invention, the disease is AMD, including neovascular (wet/exudative) AMD, dry AMD, and geographic atrophy.

In some embodiments of the present invention, the disease is a tumor, particularly a malignant tumor (cancer), including but not limited to solid tumors and leukemia, such as breast cancer, lung cancer (especially non-small cell lung cancer), adenocarcinoma, colorectal cancer, renal cancer, liver cancer, pancreatic cancer, ovarian cancer, prostate cancer, glioma, glioblastoma, myeloma, acute myeloid leukemia, agnogenic myeloid metaplasia, mesothelioma, myelodysplastic syndrome.

In some embodiments of the present invention, the disease is a hematologic malignancy, including: leukemia, lymphoma, and multiple myeloma (MM).

Specifically, leukemia may include chronic lymphocytic leukemia (CLL), chronic myeloid leukemia (CML), acute lymphoblastic leukemia (ALL), acute myeloid leukemia (AML), and acute monocytic leukemia.

Specifically, lymphoma may include Hodgkin lymphoma (HL) and non-Hodgkin lymphoma (NHL), for example, diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphoma, primary mediastinal B-cell lymphoma, Burkitt's lymphoma, lymphoplasmacytic lymphoma, hairy cell leukemia (HCL), immunoblastic large cell lymphoma, precursor B lymphoblastic lymphoma and primary central nervous system (CNS) lymphoma and T-cell non-Hodgkin lymphoma (NHL), such as precursor T lymphoblastic lymphoma/leukemia, peripheral T-cell lymphoma (PTCL), angioimmunoblastic T-cell lymphoma, extranodal natural killer (NK)/T-cell lymphoma, enteropathy-type T-cell lymphoma, subcutaneous panniculitis-like T-cell lymphoma, and anaplastic large cell lymphoma, NK/T-cell lymphoma, particularly diffuse large B-cell lymphoma (DLBCL). Specifically, in these applications, the treatment of tumors includes the destruction of tumors, and the prevention of tumor metastatic spread and micrometastasis.

In one aspect, the present invention provides a treatment method, comprising administrating the compound disclosed herein or a pharmaceutically acceptable prodrug, a pharmaceutically active metabolite, and/or a pharmaceutically acceptable salt of said compound, and/or the pharmaceutical composition disclosed herein.

As is well known to those skilled in the art, the dosage of a drug depends on various factors, including but not limited to the following: the activity of the specific compound used, the age of the patient, the weight of the patient, the health status of the patient, the patient's activity level, the patient's diet, the time of administration, the route of administration, the rate of excretion, the combination of drugs, etc.; furthermore, the optimal treatment regimen, such as the mode of treatment, the compound of the present invention or tautomer, mesomer, racemate, enantiomer, and diastereomer, or mixtures thereof, or its medicinally acceptable salt, and/or the daily dosage or the type of the compound or its tautomer, mesomer, racemate, enantiomer, and diastereomer, or mixture thereof, or its medicinally acceptable salt can be verified according to conventional treatment regimens.

In one aspect, the present invention provides a method for modulating kinase receptor activity, comprising administrating the compound disclosed herein or a pharmaceutically acceptable prodrug, a pharmaceutically active metabolite, and/or a pharmaceutically acceptable salt of said compound, and/or the pharmaceutical composition

disclosed herein.

For instance, the method inhibits kinase receptor activity. The compound described in the invention may exhibit inhibitory activity against the proliferation of kinase receptors in vitro. The inhibitory activity may be defined as a decrease in the activity of the kinase receptors compared to a negative control or a control drug after the addition of the compound of the present invention, such as a decrease by more than 1 %, more than 2 %, more than 4 %, more than 5 %, more than 8 %, more than 10 %, more than 15 %, more than 18 %, more than 20 %, more than 25 %, more than 40 %, more than 50 %, more than 60 %, more than 70 %, more than 80 %, more than 90 %, or more than 95 %. For example, the inhibitory activity can be expressed in terms of the $IC_{50}$ value (nM) for kinase receptor activity, such as below 10,000, below 5,000, below 4,000, below 3,000, below 2,000, below 1,000, below 500, below 400, below 300, below 200, below 150, below 120, below 110, below 100, below 99, below 98, below 97, below 95, below 90, below 80, below 75, below 70, below 65, below 62, below 60, below 50, below 40, below 30, below 25, below 23, below 22, below 20, below 19, below 18, below 18.5, below 17, below 15, below 12, below 10, below 9, below 8.5, below 7, below 6.7, below 6, below 5.9, below 5.5, below 5.0, below 4.8, below 4.5, below 4.4, below 4, below 3.5, below 3, below 2.5, below 2, below 1.5, below 1.0, below 0.5, below 0.3, below 0.29, below 0.25, below 0.21, below 0.20, below 0.18, below 0.17, below 0.15, below 0.12, below 0.10, below 0.09, below 0.08, below 0.07, below 0.06, below 0.05, below 0.04, below 0.03, below 0.02, or below 0.01. For example, the inhibition of kinase receptor activity can be detected by mobility shift assay.

For example, the kinase receptors include VEGFR (e.g., VEGFR1, VEGFR2, VEGFR3), EGFR, or their functionally active fragments, particularly VEGFR. For example, the methods are for non-therapeutic and/or non-diagnostic purposes. For example, the methods are in vitro and/or ex vivo methods.

In some embodiments of the present invention, the methods selectively inhibit VEGFR (e.g., VEGFR1, VEGFR2, VEGFR3, particularly VEGFR2) compared to EGFR.

In one aspect, the present invention provides a method for inhibiting ocular neovascularization and retinal vascular leakage, comprising administration of the compound disclosed herein or a pharmaceutically acceptable prodrug, a pharmaceutically active metabolite, and/or a pharmaceutically acceptable salt of the compound, and/or the pharmaceutical composition disclosed herein.

Specifically, the administration can be done through any suitable route of administration, particularly intraocular administration, such as administration via eye drops, ophthalmic ointments, subconjunctival injection, intraocular injection, especially eye drops.

Without intending to be limited by any theory, the following embodiments are merely provided to illustrate the compound the present invention, preparation method, and use thereof, and are not intended to limit the scope of this invention.

EMBODIMENTS

Table 1. Information about the compound of the present invention

| No. | Structural formula |
|---|---|
| I-1 | 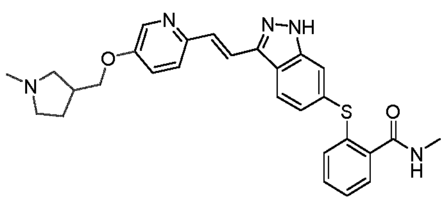 |
| I-2 | 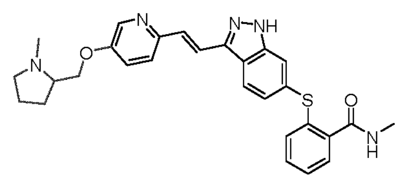 |
| I-3 | 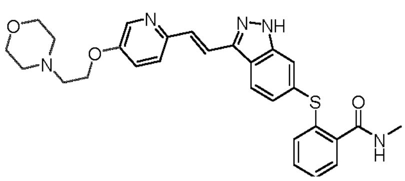 |

(continued)

| No. | Structural formula |
|---|---|
| I-4 | 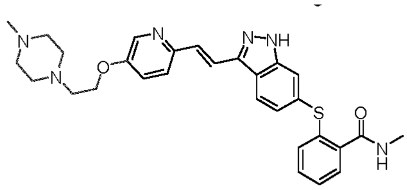 |
| I-5 | 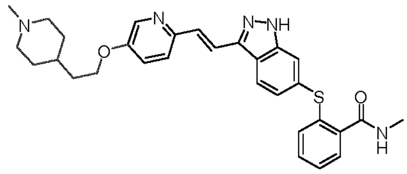 |
| I-6 | 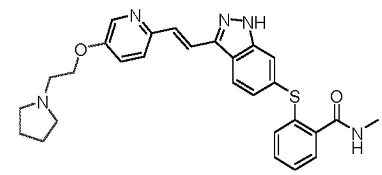 |
| I-7 | 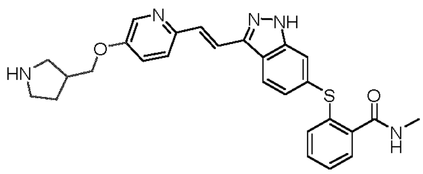 |
| I-8 | 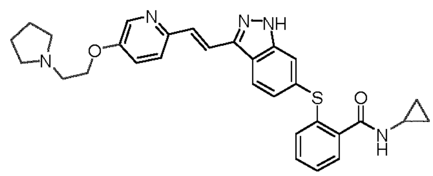 |
| I-9 | 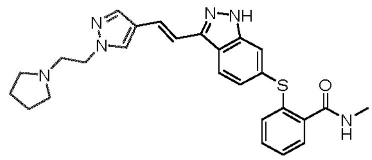 |
| I-10 | 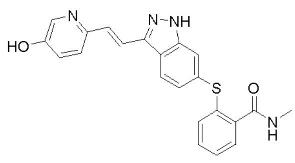 |
| I-11 | 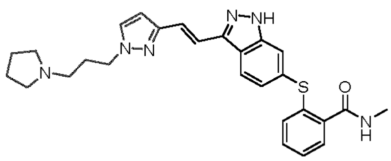 |
| I-12 | 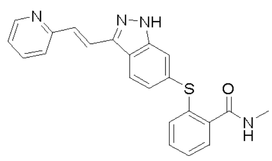 |

(continued)

| No. | Structural formula |
|---|---|
| I-13 | 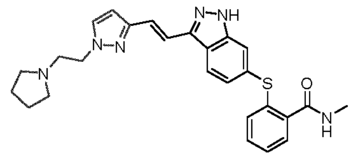 |
| I-14 | 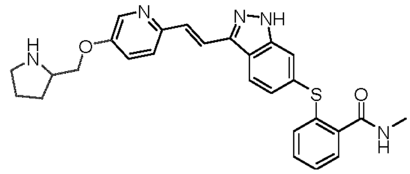 |
| I-15 | 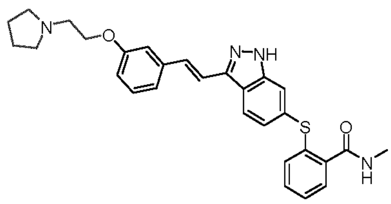 |
| I-16 | 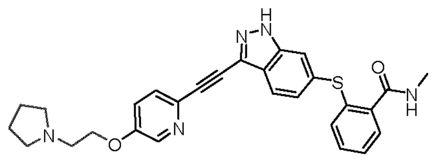 |
| I-17 | 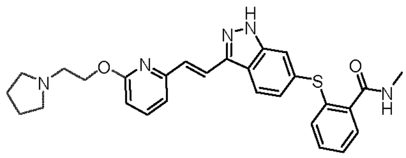 |
| I-18 | 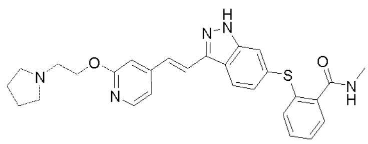 |
| I-19 | 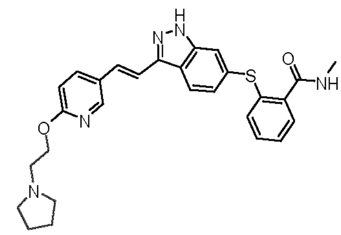 |
| I-20 | 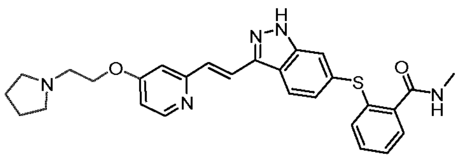 |
| I-21 | 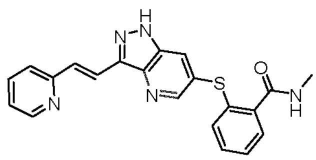 |

(continued)

| No. | Structural formula |
| --- | --- |
| I-22 | 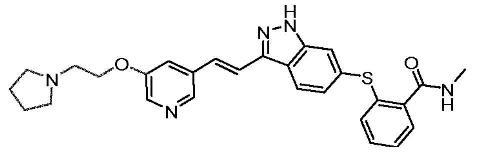 |
| I-23 | 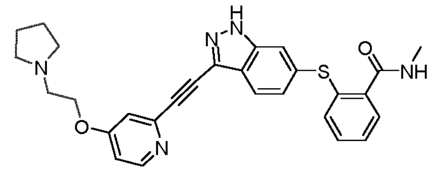 |
| I-24 | 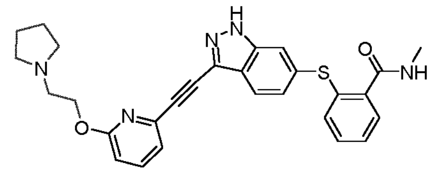 |
| I-25 | 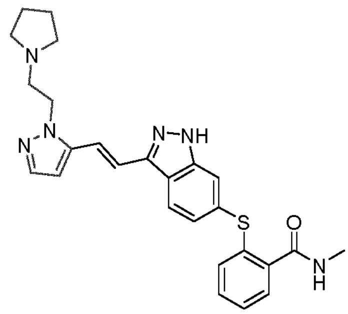 |
| I-26 | 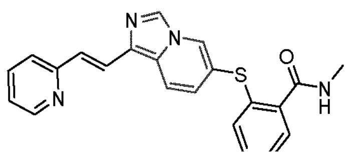 |
| I-27 | 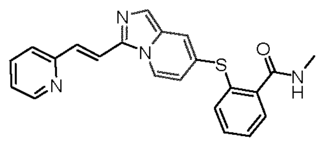 |
| I-28 | 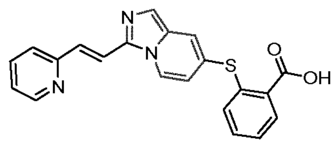 |
| I-29 | 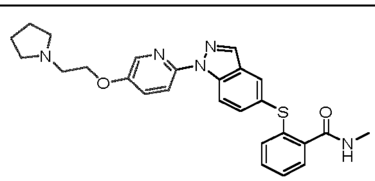 |

(continued)

| No. | Structural formula |
| --- | --- |
| I-30 | 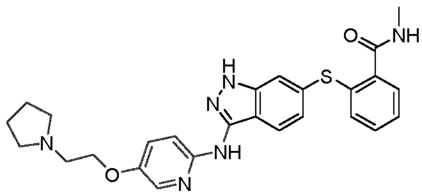 |
| I-31 | 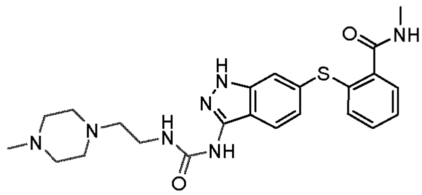 |
| I-32 | 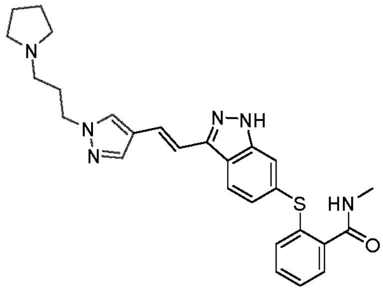 |
| I-33 | 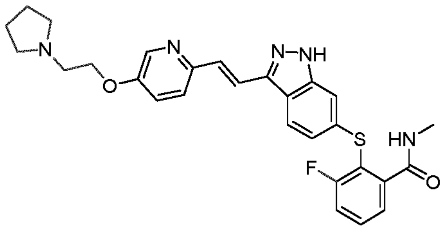 |
| I-34 | 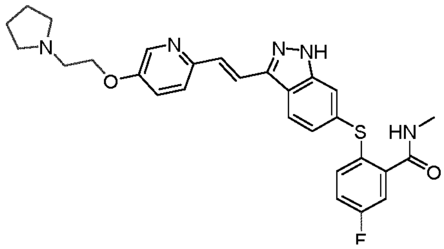 |
| I-35 | 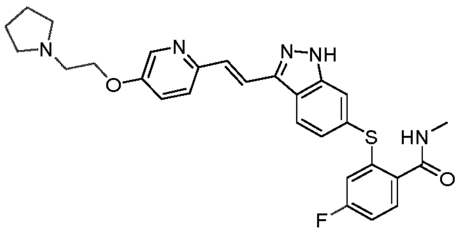 |

(continued)

| No. | Structural formula |
|---|---|
| I-36 | 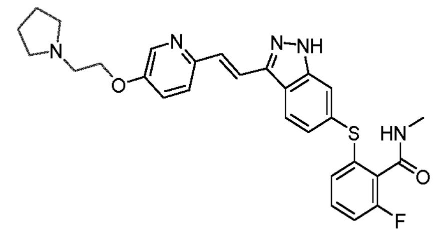 |
| I-37 | |
| I-38 | 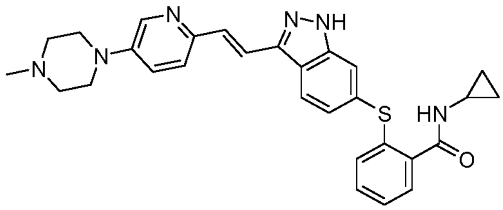 |
| I-39 | 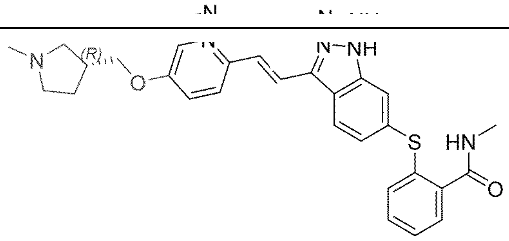 |
| I-40 | 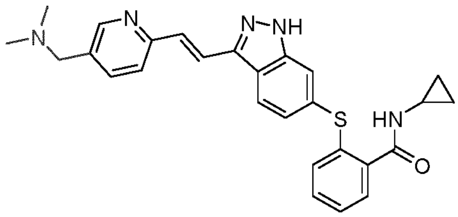 |
| I-41 | 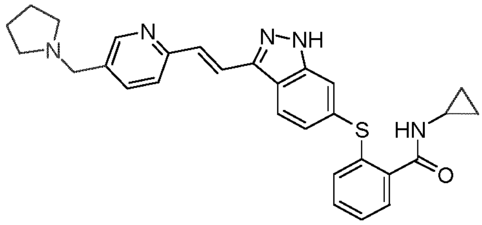 |
| I-42 | 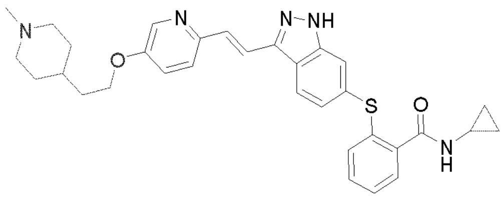 |
| I-43 | 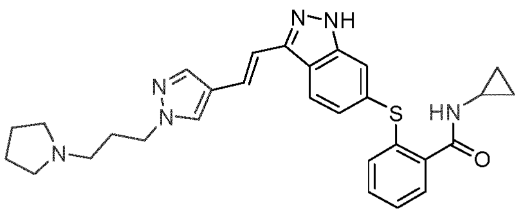 |

(continued)

| No. | Structural formula |
|---|---|
| I-44 | |
| I-46 | 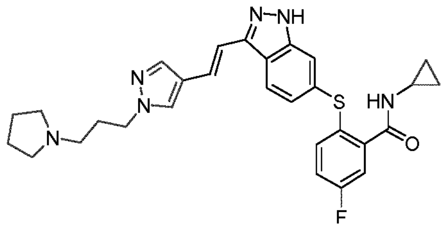 |
| I-51 | 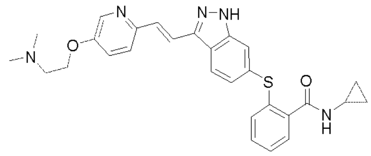 |
| I-52 | 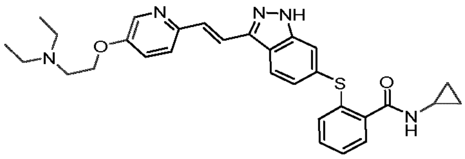 |
| I-53 | 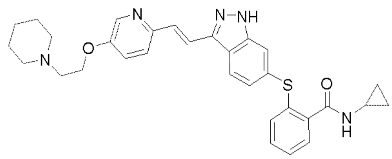 |
| I-55 | 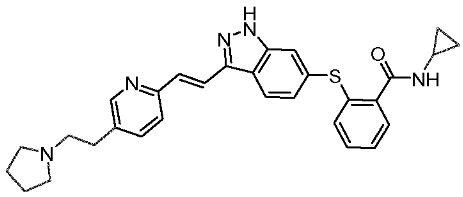 |
| I-59 | 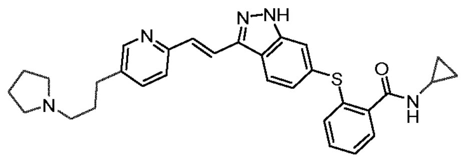 |
| I-60 | 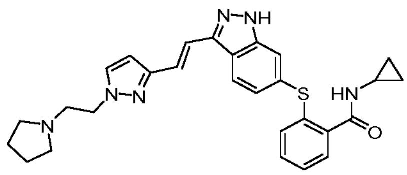 |
| I-61 | 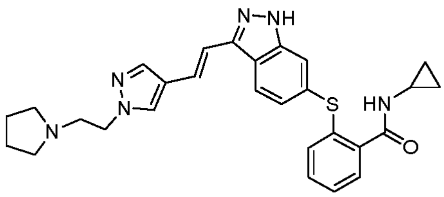 |

(continued)

| No. | Structural formula |
|---|---|
| I-67 | 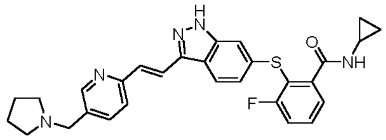 |
| I-68 | 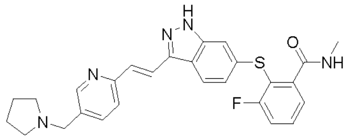 |
| I-69 | 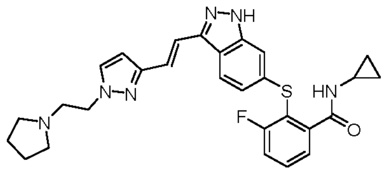 |
| I-73 | 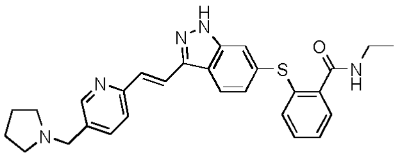 |
| I-74 | 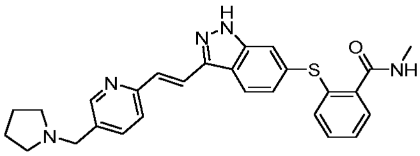 |
| I-75 | 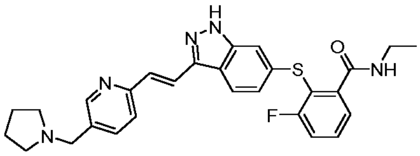 |
| I-76 | 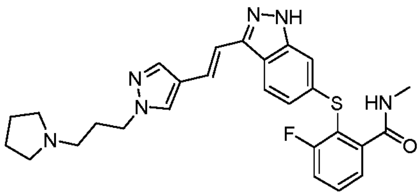 |
| I-77 | 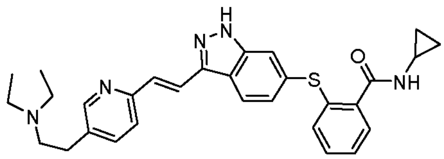 |
| I-78 | 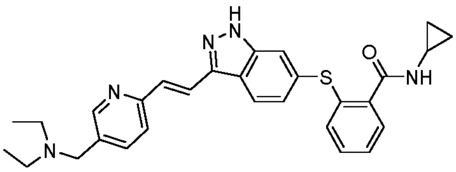 |

(continued)

| No. | Structural formula |
|---|---|
| I-79 | 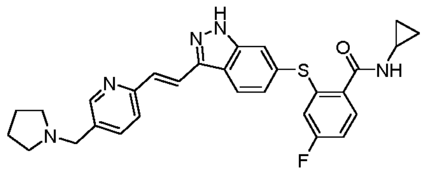 |
| I-80 | 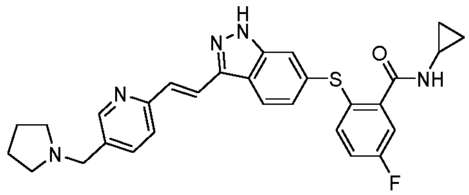 |
| I-81 | 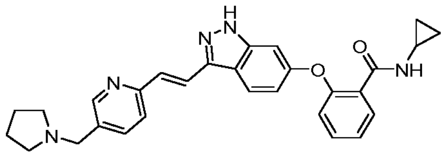 |
| I-82 | 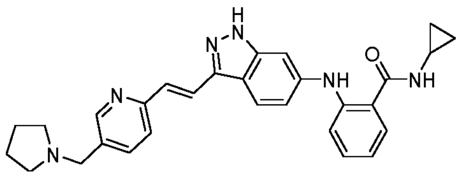 |
| I-83 | 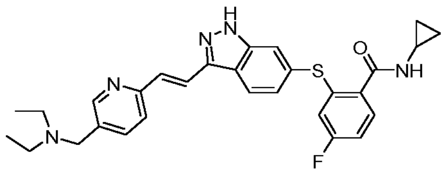 |
| I-84 | 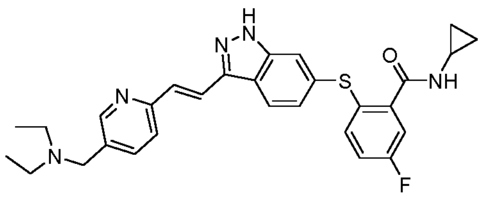 |
| I-85 | 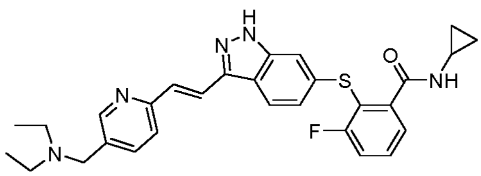 |
| I-86 | 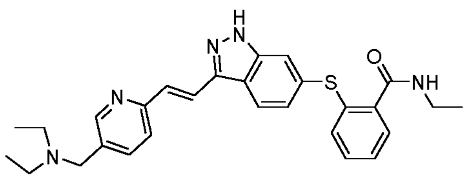 |

(continued)

| No. | Structural formula |
| --- | --- |
| I-87 | 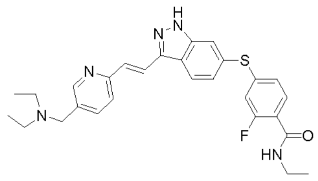 |
| I-88 | 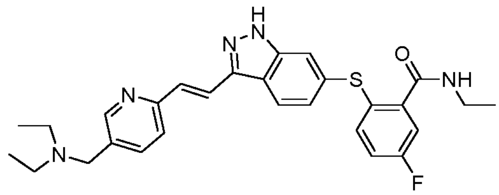 |
| I-90 | 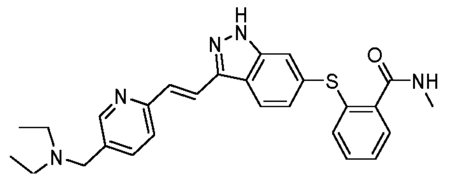 |
| I-91 | 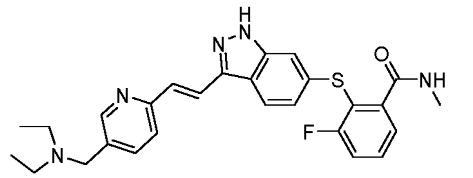 |
| I-92 | 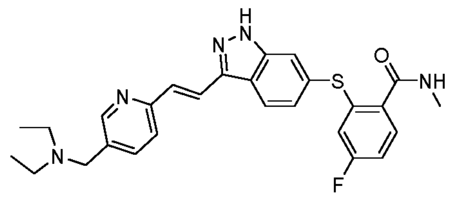 |
| I-93 | 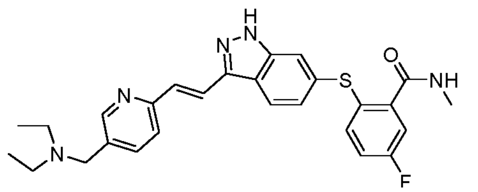 |
| I-94 | 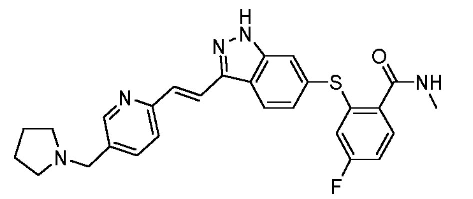 |
| I-95 | 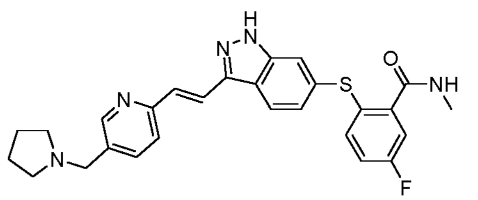 |

(continued)

| No. | Structural formula |
|---|---|
| I-97 | 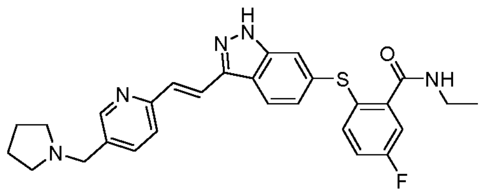 |
| I-98 | 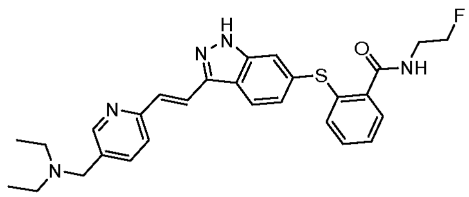 |
| I-99 | 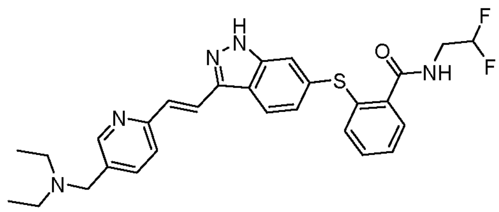 |
| I-100 | 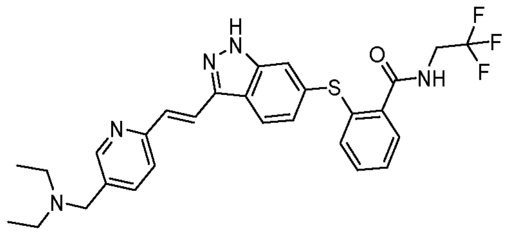 |
| I-101 | 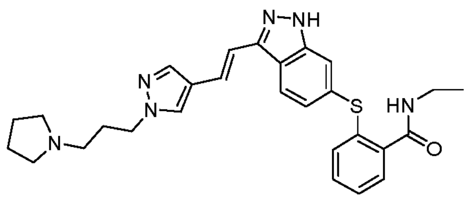 |
| I-102 | 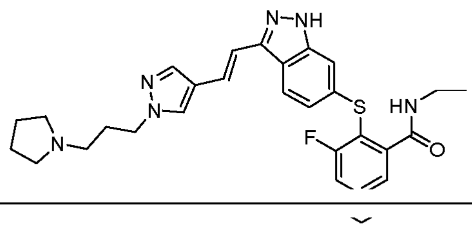 |
| I-103 | 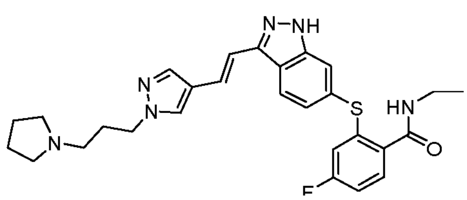 |

(continued)

| No. | Structural formula |
|---|---|
| I-104 | 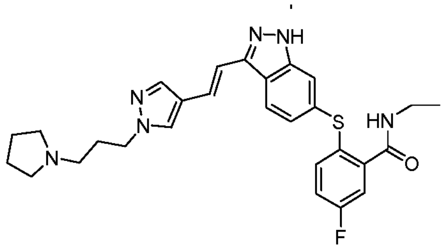 |
| I-106 | 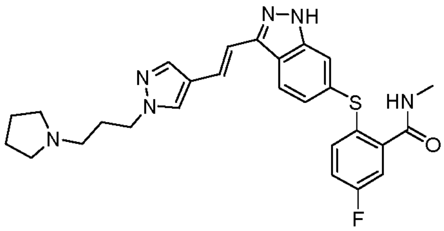 |
| I-107 | 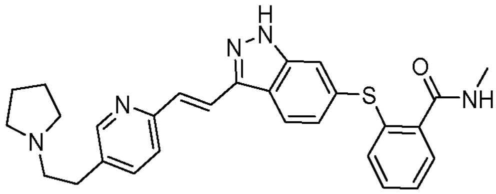 |
| I-108 | 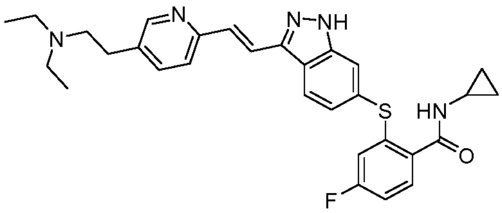 |
| I-109 | 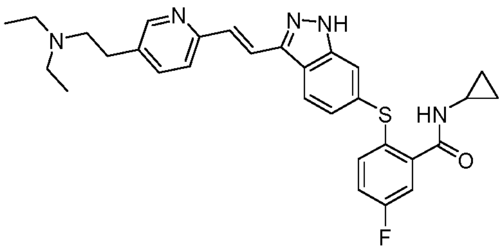 |
| I-110 | 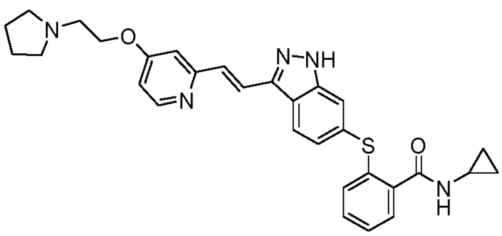 |
| I-111 | 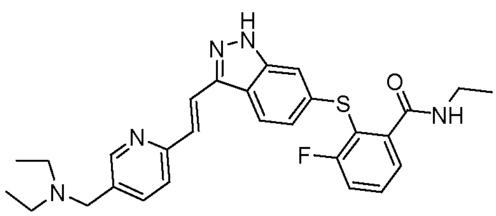 |

(continued)

| No. | Structural formula |
|---|---|
| I-112 | 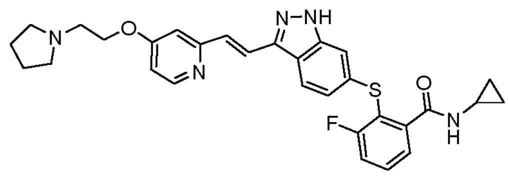 |
| I-113 | 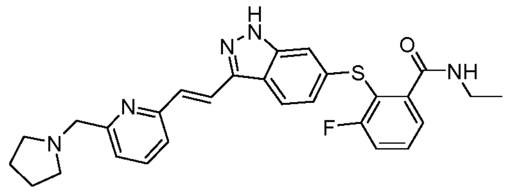 |
| I-114 | 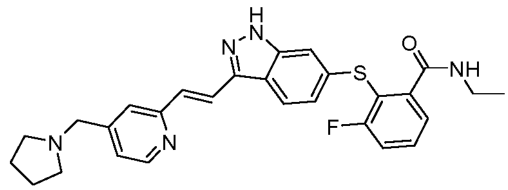 |
| I-115 | 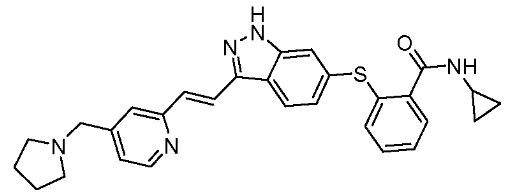 |
| I-116 | 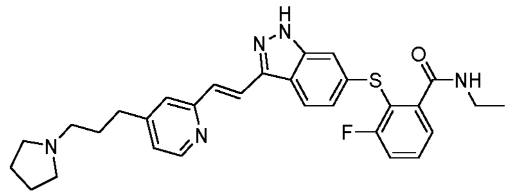 |
| I-117 | 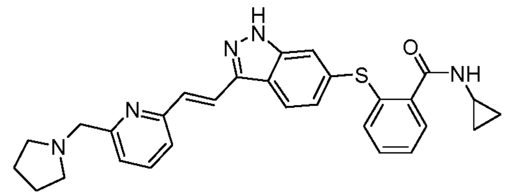 |
| I-118 | 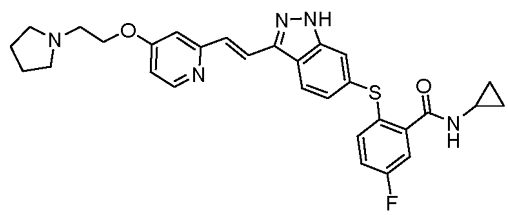 |
| I-119 | 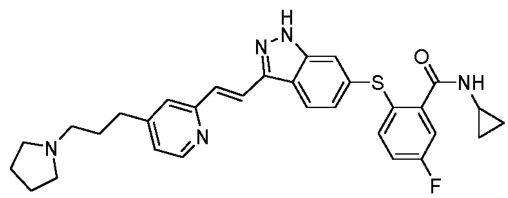 |

(continued)

| No. | Structural formula |
|---|---|
| I-120 | 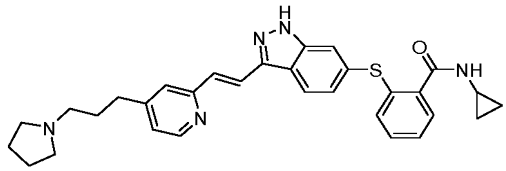 |
| I-121 | 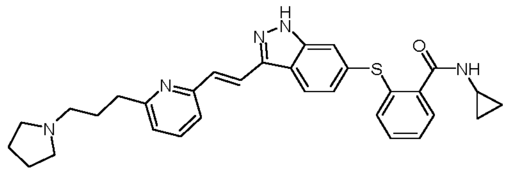 |
| I-122 | 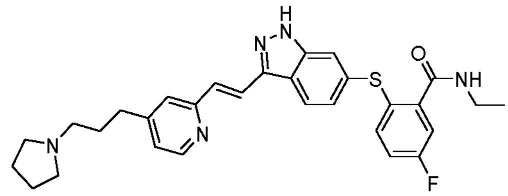 |
| I-123 | 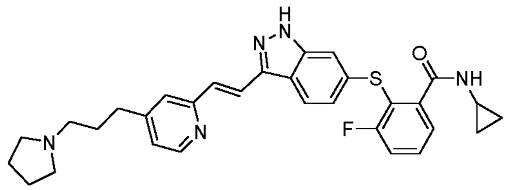 |
| I-124 | 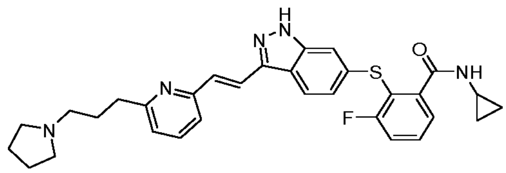 |
| I-128 | 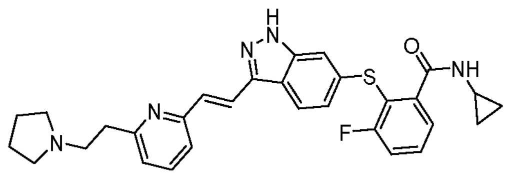 |
| I-129 | 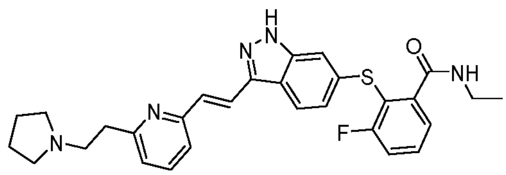 |
| I-132 | 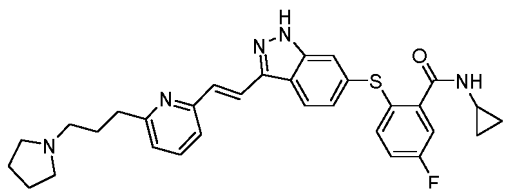 |

(continued)

| No. | Structural formula |
|---|---|
| I-133 | 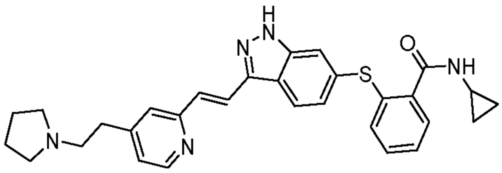 |
| I-134 | 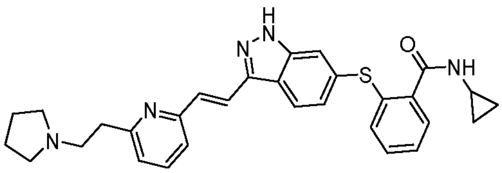 |
| I-135 | 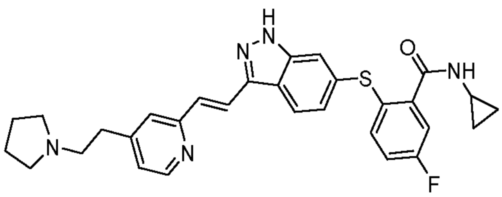 |
| I-136 | 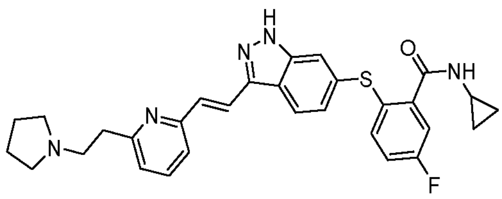 |
| I-137 | 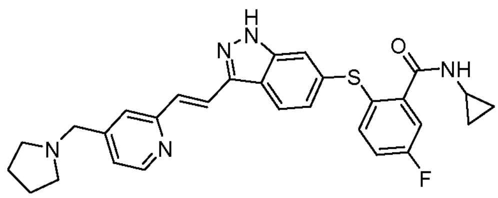 |
| I-138 | 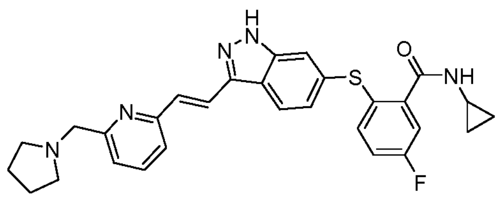 |
| I-139 | 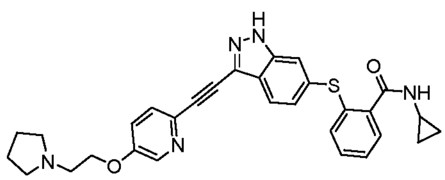 |

EMBODIMENT 1

I-10

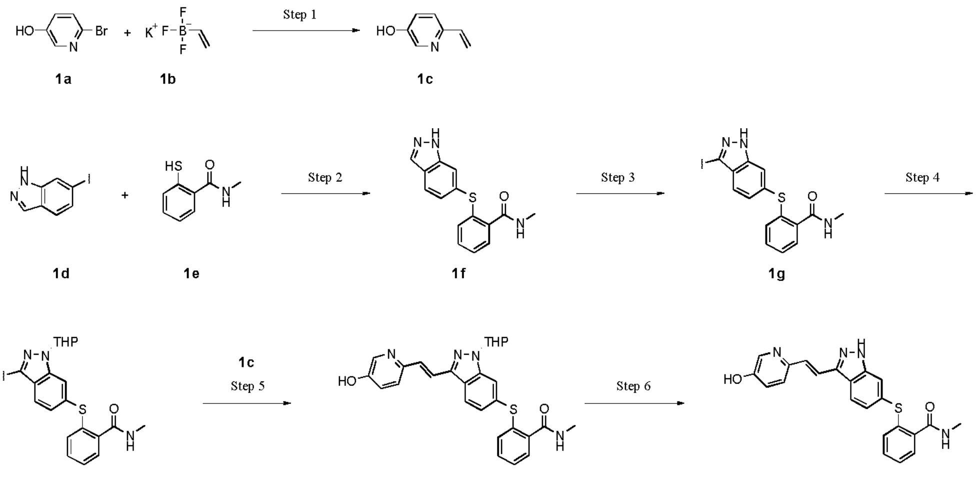


1a 1b 1c

1d 1e 1f 1g

1h 1i I-10

Step 1

Under the protection of nitrogen at room temperature, potassium vinyltrifluoroborate 1b (11.6 g, 86.21 mmol), Pd(dppf)$Cl_2$ (1.3 g, 1.72 mmol) and triethylamine (8.7 g, 86.21 mmol) were added to the solution of 6-bromopyridin-3-ol 1a (10 g, 57.47 mmol) in N,N-dimethylformamide (150 mL); the solution was heated to 85 °C and stirred to react for about 16 h. After the reaction of the starting materials completed, as indicated by thin-layer chromatography (petroleum ether/ethyl acetate =1/1), the mixture was poured into water (100 mL) and extracted with ethyl acetate (50 mL × 3); and the combined organic layers were washed with saturated brine (100 mL), dried over anhydrous sodium sulfate and concentrated to obtain a crude product. The crude product was purified by silica gel column chromatography (petroleum ether/ethyl acetate = 10/1-1/1) to obtain a white solid, 6-vinylpyridin-3-ol 1c (4.3 g, 35.50 mmol, yield: 61.76 %).
This product was verified by LCMS and HNMR.
For MS-ESI, the calculated value was $[M+H]^+$122.1, and the measurement was 122.1. $^1$H NMR (400 MHz, $CDCl_3$): δ (ppm) 8.17-8.18 (m, 1H), 7.40 (d, J = 8.4 Hz, 1H), 7.25-7.28 (m, 1H), 6.80 (dd, J = 11.2 Hz, 11.2 Hz, 1H), 5.93 (d, J = 17.6 Hz, 1H), 5.38 (d, J = 11.2 Hz, 1H).

Step 2

At room temperature, 6-iodo-1H-indazole 1d (13.3 g, 77.74 mmol), cesium carbonate (50.66 g, 155 mmol) and Pd(dppf)$Cl_2$ (11.38 g, 15.55 mmol) were added to the solution of *N*-methyl-2-mercaptobenzamide 1e (13 g, 77.74 mmol) in N,N-dimethylformamide (250 mL); the solution was heated to 80 °C and stirred to react for about 2 h; after the reaction of the starting materials completed, as indicated by thin-layer chromatography (dichloromethane/methanol =15/1), the mixture was poured into water (1000 mL) and extracted with ethyl acetate (100 mL × 3); the combined organic layers were washed with saturated brine (200 mL), dried over anhydrous sodium sulfate and concentrated to obtain a crude product; and the crude product was purified by silica gel column chromatography (petroleum ether/ethyl acetate =2/1~0/1) to obtain a yellow solid, 2-(1H-indazol-6-ylthio)-N-methylbenzamide 1f (8.3 g, yield: 37.7 %).
This product was verified by LCMS and HNMR.
For MS-ESI, the calculated value was $[M+H]^+$ 285.1, and the measurement was 285.0.
$^1$H NMR (400 MHz, $CDCl_3$) δ: 8.07 (s, 1H), 7.72 (d, 1H, J=8.4Hz), 7.62-7.65 (m, 2H), 7.26-7.30 (m, 2H), 7.15-7.21 (m, 2H), 6.39 (s, 1H), 2.98 (d, 3H, J=4.8Hz).

Step 3

At 0°C, $K_2CO_3$ (6.83 g, 49.4 mmol) and $I_2$ (10.66 g, 42.0 mmol) were added to the solution of 2-(1H-indazol-6-ylthio)-N-methylbenzamide 1f in N,N-dimethylformamide;, the solution was heated to room temperature to react for about 3.5 h; after the reaction of the starting materials completed, as indicated by thin-layer chromatography (dichloromethane/methanol =15/1), the mixture was poured into water (1000 mL) and extracted with ethyl acetate (100 mL × 3); and the combined organic layers were washed with saturated brine (100 mL), dried over anhydrous sodium sulfate and concentrated to obtain a crude product, 2-[(3-iodo-1H-indazol-6-yl)thio]-N-methylbenzamide 1 g (8.8 g, 87.0 %).
This product was verified by LCMS and HNMR.

For MS-ESI, the calculated value was $[M+H]^+$ 411.0, and the measurement was 410.8.

$^1H$ NMR (400 MHz, DMSO-$d_6$) δ: 13.59 (s, 1H), 8.40 (d, 1H, J=4.8Hz), 7.58 (s, 1H), 7.44-7.50 (m, 2H), 7.28-7.32 (m, 2H), 7.22-7.27 (m, 1H), 7.14 (dd, 1H, J=8.4, 2.0Hz), 7.02 (dd, 1H, J = 8.0, 2.0Hz), 2.76 (d, 3H, J=4.4Hz).

Step 4

At room temperature, 3,4-dihydro-2H-pyrane (DHP) (1.03 g, 12.22 mmol) and TsOH (63.12 mg, 366.53 μmol) were added to the solution of 2-[(3-iodo-1H-indazol-6-yl)thio]-N-methylbenzamide 1 g (1 g, 2.44 mmol) in THF (20 mL), and the mixture was stirred and refluxed for 16 h. After the thin-layer chromatography (petroleum ether/ethyl acetate = 1/2) showed the complete reaction of the starting materials, the mixture was poured into water (100 mL) and extracted with ethyl acetate (50 mL × 3). The organic layers were combined, washed with saturated brine (100 mL), dried over anhydrous sodium sulfate and concentrated to obtain a crude product. The crude product was purified by silica gel column chromatography (petroleum ether/ethyl acetate = 10/1-/5/1-3/1-1/1) to obtain a white solid, 2-(3-iodo-1-tetrahydropyran-2-yl-indazol-6-yl)thio-N-methyl-benzamide 1h (650 mg, 1.32 mmol, yield: 53.92 %).

This product was verified by LCMS and HNMR.

For MS-ESI, the calculated value was $[M+H]^+$ 494.0, and the measurement was 493.9.

$^1H$ NMR (400MHz, $CDCl_3$): δ (ppm) 7.66 (s, 1H), 7.58-7.65 (m, 1H), 7.42 (d, 1H, J = 8.4 Hz), 7.29-7.35 (m, 2H), 7.14-7.22 (m, 2H), 6.27 (s, 1H), 5.64 (dd, 1H, J = 9.4, 2.6 Hz), 4.13 -4.15 (m, 1H), 3.64-3.76 (m, 1H), 2.97 (d, 3H, J = 4.8 Hz), 2.46-2.54 (m, 1H), 2.02-2.18 (m, 2H), 1.66-1.84 (m, 3H).

Step 5

Under the protection of nitrogen at room temperature, 6-vinylpyridin-3-ol 1c (147.3 mg, 1.22 mmol), triethylamine (307.7 mg, 3.04 mmol), $Pd_2(dba)_3$ (464 mg, 506.73 μmol) and $P(o\text{-}tol)_3$ (308.5 mg, 1.01 mmol) were added to the solution of 2-(3-iodo-1-tetrahydropyran-2-yl-indazol-6-yl)thio-N-methyl-benzamide 1h (500 mg, 1.01 mmol) in 1,4-dioxane (10 mL), and the solution was stirred at room temperature to allow for reaction. After the reaction of the starting materials completed, as indicated by thin-layer chromatography (dichloromethane/methanol = 20 /1), the mixture was poured into water (20 mL) and extracted with ethyl acetate (20 mL × 3); the combined organic layers were washed with saturated brine (30 mL), dried over anhydrous sodium sulfate and concentrated to obtain a crude product; and the crude product was purified by silica gel column chromatography (petroleum ether/ethyl acetate = 30/1-1/1) to obtain a yellow solid, 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol, yield: 81.11 %); the product purity, as determined by LCMS, was approximately 30 %.

This product was verified by LCMS.

For MS-ESI, the calculated value was $[M+H]^+$ 487.2, and the measurement was 487.4.

Step 6

At room temperature, trifluoroacetic acid (35.2 mg, 308.27 μmol, 23.75μL) was added to the solution of 2-[3-[(E)-2-(5-hydroxy-2-pyridyl)vinyl]-1-tetrahydropyran-2-yl-indazol-6-yl] thio-N-methyl-benzamide 1i (150 mg, 308.27 μmol) in dichloromethane (3 mL); the mixture was stirred at 35°C to react for 16 h. After the reaction of the starting materials completed, as indicated by thin-layer chromatography (dichloromethane/methanol = 10/1), the mixture was poured into water (40 mL), adjusted with solid sodium carbonate to pH=8-9, and extracted with DCM (30 mL × 3). The organic layers were combined, washed with saturated brine (20 mL), dried over anhydrous sodium sulfate and concentrated to obtain a crude product. The crude product was purified by preparative thin-layer chromatography to obtain a white solid, 2-({3-[(E)-2-(5-hydroxypyridin-2-yl)vinyl]-1H-indazol-6-yl}thio)-N-methylbenzamide I-10 (6.1 mg, 15.16 μmol, yield: 4.92 %).

This product was verified by LCMS and HNMR.

For MS-ESI, the calculated value was $[M+H]^+$ 403.1, and the measurement was 403.1.

$^1H$ NMR (400MHz, $CDCl_3$&$CD_3OD$): δ (ppm) 8.05-8.04 (m, 1H), 7.90 (d, J=8.4Hz, 1H), 7.51-7.42 (m, 3H), 7.39-7.35 (m, 2H), 7.21-7.18 (m, 2H), 7.14-7.07 (m, 3H), 2.82 (s, 3H).

I-14

Step 1

Step 2

Step 1

Under the protection of nitrogen at room temperature, 2-(hydroxymethyl)pyrrolidin-1-tert-butyl carboxylate 2a (198.5 mg, 986.47 μmol), $Ph_3P$ (323.4 mg, 1.23 mmol) and DIAD (249.3 mg, 1.23 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol) in tetrahydrofuran (10 mL); the solution was heated to 40 °C and stirred to react for about 16 h. After the reaction of the starting materials completed, as indicated by thin-layer chromatography (dichloromethane/methanol = 10/1), the mixture was poured into water (30 mL) and extracted with ethyl acetate (30 mL × 3); and the combined organic layers were washed with saturated brine (40 mL), dried over anhydrous sodium sulfate and concentrated to obtain a crude product. The crude product was purified by preparative thin-layer chromatography (dichloromethane/methanol = 10 /1) to obtain a white solid, 2-[[6-[(E)-2-[6-[2-(methylcarbamoyl)phenyl] thio-1-tetrahydropyran-2-yl-indazol-3-yl]vinyl]-3-pyridyl]oxymethyl]pyrrolidin-1-tert-butyl carboxylate 2b (400 mg, 597.17 μmol, yield: 72.64 %); the product purity, as determined by LCMS, was approximately 29 %.

This product was verified by LCMS.

For MS-ESI, the calculated value was $[M+H]^+$670.3, and the measurement was 670.2.

Step 2

At room temperature, trifluoroacetic acid (340.4 mg, 2.99 mmol) was added to the solution of 2-[[6-[(E) -2-[6-[2-(methylcarbamoyl)phenyl] thio-1-tetrahydropyran-2-yl-indazol-3-yl]vinyl]-3-pyridyl]oxymethyl]pyrrolidin-1-tert-butyl carboxylate 2b (0.4 g, 597.17 μmol) in dichloromethane (5 mL), heated to 35°C and stirred to react for about 16 h. After the reaction of the starting materials completed, as indicated by thin-layer chromatography (dichloromethane/-methanol = 10/1), the mixture was poured into water (40 mL), adjusted with solid sodium carbonate to pH=8-9, and extracted with dichloromethane (30 mL × 3); and the combined organic layers were washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[(pyrrolidin-2-yl)methoxy]pyridin-2-yl}vinyl]-1H-indazol-6-yl}thio)benzamide I-14 (13.9 mg, 28.62 μmol, yield: 4.79 %).

This product was verified by LCMS, HNMR and CNMR.

For MS-ESI, the calculated value was $[M+H]^+$486.2, and the measurement was 487.0.

$^1H$ NMR (400MHz, $CDCl_3$&$CD_3OD$): δ (ppm) 8.24-8.23 (m, 1H), 7.98 (d, J=8.8Hz, 1H), 7.65-7.61 (m, 1H), 7.55-7.52 (m, 1H), 7.50-7.48 (m, 2H), 7.44-7.39 (m, 1H), 7.29-7.22 (m, 3H), 7.19-7.15 (m, 2H), 4.05-4.02 (m, 1H), 3.94 (t, J=8.0Hz, 1H), 3.53-3.50 (m, 1H), 3.04-2.91 (m, 2H), 2.88 (s, 1H), 2.02-1.95 (m, 1H), 1.89-1.79 (m, 2H), 1.64-1.57 (m, 1H).

$^{13}C$ NMR (100 MHz, $CDCl_3$&$CD_3OD$): δ (ppm) 169.83, 154.32, 148.42, 142.19, 137.25, 136.56, 135.24, 133.46, 131.10, 130.65, 139.35, 128.04, 126.65, 125.39, 122.26, 122.03, 121.83, 121.47, 120.46, 114.42, 70.93, 57.08, 46.10, 46.06, 29.59, 27.64, 26.33, 25.07.

I-2

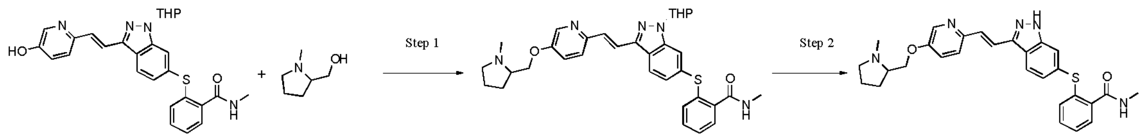


1i 3a 3b I-2

Step 1

Under the protection of nitrogen at room temperature, (1-methylpyrrolidin-2-yl)methanol 3a (113.6 mg, 986.47 μmol), $Ph_3P$ (215.6 mg, 822.06 μmol) and DIAD (249.3 mg, 1.23 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol) in tetrahydrofuran (5 mL); the solution was stirred at 30°C to react for about 16 h. After the thin-layer chromatography (dichloromethane/methanol = 10/1) showed the complete reaction of the starting materials, the mixture was poured into water (30 mL) and extracted with ethyl acetate (20 mL × 3), and the organic layers were combined, washed with saturated brine (30 mL), dried over anhydrous sodium sulfate and concentrated to obtain a crude product. The crude product was purified by silica gel column chromatography (petroleum ether/ethyl acetate = 10/1-dichloromethane/methanol = 10/1) to obtain a yellow solid, N-methyl-2-[3-[(E)-2-[5-[(1-methylpyrrolidin-2-yl)methoxy]-2-pyridyl]vinyl]-1-tetrahydropyran-2-ylindol-6-yl] thiobenzamide 3b (240 mg, 411.14 μmol, yield: 50.01 %).

This product was verified by LCMS.

For MS-ESI, the calculated value was 584.3, and the measurement was 584.3.

Step 2

At room temperature, trifluoroacetic acid (46.9 mg, 411.14 μmol) was added to the solution of N-methyl-2-[3-[(E)-2-[5-[(1-methylpyrrolidin-2-yl)methoxy]-2-pyridyl]vinyl]-1-tetrahydropyran-2-ylindol-6-yl] thiobenzamide 3b (240 mg, 411.14 μmol) in dichloromethane (5 mL). The mixture was stirred at 30°C for 16 hours. After the reaction of the starting materials completed, as indicated by thin-layer chromatography (dichloromethane/methanol = 10/1), the mixture was poured into water (10 mL), adjusted with solid sodium carbonate to pH=8-9, and extracted with DCM (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[(1-methylpyrrolidin-2-yl)methoxy]pyridin-2-yl}vinyl]-IH-indazol-6-yl}thio)benzamide I-2 (31.6 mg, 63.25 μmol, yield: 7.97 %).

This product was verified by LCMS, HNMR and CNMR.

For MS-ESI, the calculated value was $[M+H]^+$500.2, and the measurement was 500.1.

$^1H$ NMR (400MHz, $CDCl_3$&$CD_3OD$): δ (ppm) 8.23 (s, 1H), 7.94 (d, J = 8.0 Hz, 1H), 7.63 (s, 1H), 7.47 (dd, J = 39.8, 29.7 Hz, 4H), 7.24 (s, 2H), 4.02 (d, J = 6.4 Hz,2H), 3.08 (s, 3H), 2.85 (s, 1H), 2.70 (s, 3H), 2.28 - 2.32 (m, 1H), 2.63 (s, 1H), 1.98-2.02 (m, 1H), 1.80 (s, 1H), 0.82 (s, 1H).

$^{13}C$ NMR (100MHz, $CDCl_3$&$CD_3OD$): δ (ppm) 169.43, 154.32, 148.32, 137.43, 136.68, 134.67, 133.45, 131.85, 130.74, 129.50, 128.34, 126.93, 125.01, 122.20, 121.86, 121.79, 121.64, 121.49, 120.47, 113.93, 70.84, 64.29, 57.63, 41.61, 29.62, 28.32, 26.44, 22.71, 22.63.

I-5

Step 1

Step 2

Step 1

Under the protection of nitrogen at room temperature, 2-(1-methyl-4-piperidyl)ethanol 4a (141.3 mg, 986.47 μmol), $Ph_3P$ (323. 4 mg, 1.23 mmol) and DIAD (249.3 mg, 1.23 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol) in tetrahydrofuran (5 mL); the solution was stirred at 30°C to react for about 16 h. After the reaction of the starting materials completed, as indicated by thin-layer chromatography (dichloromethane/methanol = 10/1), the mixture was poured into water (20 mL) and extracted with ethyl acetate (20 mL × 3); and the combined organic layers were washed with saturated brine (20 mL), dried over anhydrous sodium sulfate and concentrated to obtain a crude product. The crude product was purified by preparative thin-layer chromatography (dichloromethane/methanol = 10/1) to obtain a white solid, N-methyl-2-[3-[(E)-2-[5-[2-(1-methyl-4-piperidyl)ethoxy]-2-pyridyl]vinyl]-1-tetrahydropyran-2-yl-indazol-6-yl]thiobenzamide 4b (120 mg, 196.14 μmol, yield: 23.86 %).

This product was verified by LCMS.

For MS-ESI, the calculated value was $[M+H]^+$612.3, and the measurement was 612.2.

Step 2

At room temperature, trifluoroacetic acid (22.36 mg, 196.14 μmol) was added to the solution of N-methyl-2-[3-[(E)-2-[5-[2-(1-methyl-4-piperidyl)ethoxy]-2-pyridyl]vinyl]-1-tetrahydropyran-2-yl-indazol-6-yl]thiobenzamide 4b (120 mg, 196.14 μmol) in dichloromethane (5 mL). The mixture was stirred at 40°C for 16 hours. After the reaction of the starting materials completed, as indicated by thin-layer chromatography (dichloromethane/methanol = 10/1), the mixture was poured into water (10 mL), adjusted with solid sodium carbonate to pH=8-9, and extracted with DCM (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[2-(1-methylpiperidin-4-yl)ethoxy]pyridin-2-yl}vinyl]-1H-indazol-6-yl}thio)benzamide I-5 (17 mg, 32.22 μmol, yield: 16.42 %).

This product was verified by LCMS, HNMR and CNMR.

For MS-ESI, the calculated value was $[M+H]^+$582.2, and the measurement was 528.2.

$^1H$ NMR (400MHz, $CDCl_3$&$CD_3OD$):δ 8.24 (d, J = 2.4 Hz, 1H), 8.06 (d, J = 8.6 Hz, 1H), 7.56-7.80 (m, 3H), 7.48-7.55 (m, 2H), 7.41-7.43 (m, 1H),7.32 (dd, J = 13.0, 6.4 Hz, 2H), 7.21 (d, J = 8.6 Hz, 2H), 4.17 (t, J = 6.2 Hz, 2H), 2.95 (d, J = 11.2 Hz, 2H), 2.88 (s, 3H), 2.34 (s, 3H), 2.16 (dd, J = 26.8, 13.4 Hz, 3H), 1.75-1.99 (m, 4H), 1.64 (s, 1H).

$^{13}C$ NMR (100 MHz, $CDCl_3$&$CD_3OD$) δ 154.34, 148.14, 137.65, 132.13, 130.84, 129.77, 128.68, 127.18, 124.68, 122.24, 121.58, 121.51, 121.37, 66.12, 55.66, 46.22, 35.50, 31.94, 31.81, 29.66, 26.59.

I-1

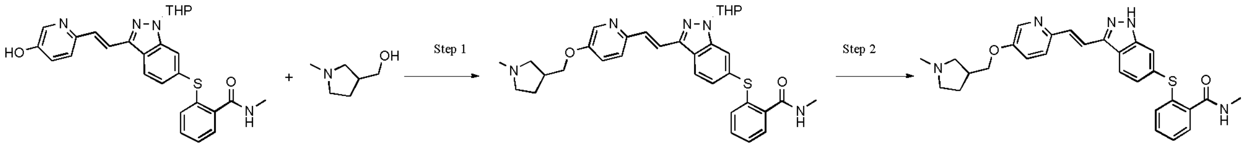


1i 5a 5b I-1

Step 1

Under the protection of nitrogen at room temperature, (1-methylpyrrolidin-3-yl)methanol 5a (113.6 mg, 986.47 μmol), $Ph_3P$ (323. 4 mg, 1.23 mmol) and DIAD (249.3 mg, 1.23 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol) in tetrahydrofuran (5 mL); the solution was stirred at 30°C to react for about 16 h. After the reaction of the starting materials completed, as indicated by thin-layer chromatography (dichloromethane/methanol = 10/1), the mixture was poured into water (20 mL) and extracted with ethyl acetate (20 mL × 3); and the combined organic layers were washed with saturated brine (20 mL), dried over anhydrous sodium sulfate and concentrated to obtain a crude product. The crude product was purified by preparative thin-layer chromatography (dichloromethane/methanol = 10/1) to obtain a white solid, N-methyl-2-[3-[(E)-2-[5-[(1-methylpyrrolidin-3-yl)methoxy]-2-pyridyl]vinyl]-1-tetrahydropyran-2-ylindol-6-yl]thiobenzamide 5b (170 mg, 291.22 μmol, yield: 35.43 %).

This product was verified by LCMS.

For MS-ESI, the calculated value was $[M+H]^+$584.3, and the measurement was 584.2.

Step 2

At room temperature, trifluoroacetic acid (33.2 mg, 291.22 μmol) was added to the solution of N-methyl-2-[3-[(E)-2-[5-[(1-methylpyrrolidin-3-yl)methoxy]-2-pyridyl]vinyl]-1-tetrahydropyran-2-ylindol-6-yl]thiobenzamide 5b (170 mg, 291.22 μmol) in dichloromethane (5 mL). The mixture was stirred at 40°C for 16 hours. After the reaction of the starting materials completed, as indicated by thin-layer chromatography (dichloromethane/methanol = 10/1), the mixture was poured into water (10 mL), adjusted with solid sodium carbonate to pH=8-9, and extracted with DCM (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[(1-methylpyrrolidin-3-yl)methoxy]pyridin-2-yl}vinyl]-1H-indazol-6-yl}thio) benzamide I-1 (24.3 mg, 48.64 μmol, yield: 16.70 %).

This product was verified by LCMS, HNMR and CNMR.

For MS-ESI, the calculated value was $[M+H]^+$500.2, and the measurement was 500.1.

$^1H$ NMR (400 MHz, $CD_3OD$&$CDCl_3$) δ 8.27 (d, J = 2.8 Hz, 1H), 8.07 (d, J = 4.8 Hz, 1H), 7.65-7.77 (m, 2H), 7.43 - 7.50 (m, 4H), 7.26-7.38 (m, 2H), 7.21-7.26 (m, 2H), 4.03-4.11 (m, 2H), 2.92-2.97 (m, 1H), 2.88 (s, 3H), 2.74-2.86 (m, 3H), 2.63-2.69 (m, 1H), 2.50 (s, 3H), 2.14 - 2.23 (m, 1H), 1.72 - 1.80 (m, 1H).

$^{13}C$ NMR (100 MHz, $CD_3OD$&$CDCl_3$) δ: 170.33, 154.69, 148.16, 142.79, 142.73, 142.21, 137.09, 135.42, 133.78, 131.17, 130.33, 129.15, 127.67, 126.43, 125.44, 122.39, 122.02, 121.65, 121.27, 120.29, 114.32, 71.13, 71.08, 58.73, 55.53, 41.15, 37.35, 27.40, 25.66.

I-4

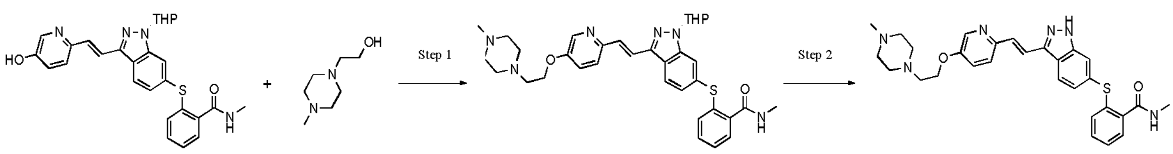


1i 6a 6b I-4

Step 1

Under the protection of nitrogen at 0°C, 2-(4-methylpiperazin-1-yl)ethanol 6a (177.8 mg, 1.23 mmol), $Ph_3P$ (404.3 mg, 1.54 mmol) and DIAD (311.7 mg, 1.54 mmol) were added to the solution of 2-[3-[(E)-2-(5-hydroxy-2-pyridyl) vinyl]-1-tetrahydropyran-2-yl-indazol-6-yl] thio-N-methyl-benzamide 1i (500 mg, 1.03 mmol) in tetrahydrofuran (5 mL); the solution was stirred at 30°C to react for about 16 h. After the reaction of the starting materials completed, as indicated by thin-layer chromatography (dichloromethane/methanol = 10/1), the mixture was poured into water (30 mL) and extracted with ethyl acetate (30 mL × 3); and the combined organic layers were washed with saturated brine (40 mL), dried over anhydrous sodium sulfate and concentrated to obtain a crude product. The crude product was purified by preparative thin-layer chromatography (dichloromethane/methanol = 10/1) to obtain a yellow solid, N-methyl-2-[3-[(E)-2-[5-[2-(4-methylpiperazin-1-yl)ethoxy]-2-pyridyl]vinyl]-1-tetrahydropyran-2-yl-indazol-6-yl]thiobenzamide 6b (400 mg, 652.76 μmol, yield: 63.52 %).

This product was verified by LCMS.

For MS-ESI, the calculated value was $[M+H]^+$613.3, and the measurement was 613.2.

Step 2

At room temperature, trifluoroacetic acid (74.4 mg, 652.76 μmol) was added to the solution of N-methyl-2-[3-[(E)-2-[5-[2-(4-methylpiperazin-1-yl)ethoxy]-2-pyridyl]vinyl]-1-tetrahydropyran-2-yl-indazol-6-yl]thiobenzamide 6b (400 mg, 652.76 μmol) in dichloromethane (10 mL). The mixture was stirred at 30°C for 16 hours. After the reaction of the starting materials completed, as indicated by thin-layer chromatography (dichloromethane/methanol = 10/1), the mixture was poured into water (10 mL), adjusted with solid sodium carbonate to pH=8-9, and extracted with DCM (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[2-(4-methylpiperazin-1-yl)ethoxy]pyridin-2-yl}vinyl]-IH-indazol-6-yl}thio)benzamide I-4 (27.5 mg, 52.02 μmol, yield: 7.97 %).

This product was verified by LCMS, HNMR and CNMR.

For MS-ESI, the calculated value was $[M+H]^+$529.2, and the measurement was 529.2.

$^1H$ NMR (400 MHz, $CD_3OD$&$CDCl_3$) δ 8.27 (d, J = 2.4 Hz, 1H), 8.05 (d, J = 11.2 Hz, 1H), 7.81 (s, 1H), 7.61-7.71 (m, 3H), 7.41-7.50 (m, 3H), 7.27-7.32 (m, 2H), 7.21 (d, J = 1.6 Hz, 2H),4.12 (s, 2H), 2.87 (d, J = 15.2 Hz, 4H), 2.50-2.73 (m, 8H), 2.33 (s, 3H).

$^{13}C$ NMR (100 MHz, $CD_3OD$&$CDCl_3$) δ: 170.27, 154.35, 148.36, 137.19, 136.77, 135.58, 133.62, 131.08, 130.43, 129.19, 127.72, 126.39, 125.57, 122.34, 122.18, 121.34, 65.97, 56.72, 54.32, 52.80, 45.07, 25.92.

I-7

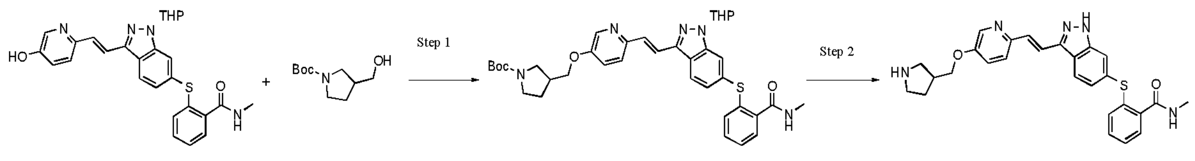


1i 7a 7b I-7

Step 1

Under the protection of nitrogen at 0°C, 3-(hydroxymethyl)pyrrolidin-1-tert-butyl carboxylate 7a (900 mg, 4.47 mmol), $Ph_3P$ (972 mg, 3.71 mmol) and DIAD (750 mg, 3.71 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (750 mg, 1.54 mmol) in tetrahydrofuran (5 mL); the solution was stirred at 40°C to react for about 16 h. After the reaction of the starting materials completed, as indicated by thin-layer chromatography (dichloromethane/methanol = 10/1), the mixture was poured into water (30 mL) and extracted with ethyl acetate (30 mL × 3); and the combined organic layers were washed with saturated brine (40 mL), dried over anhydrous sodium sulfate and concentrated to obtain a crude product. The crude product was purified by silica gel column chromatography (petroleum ether/ethyl acetate = 10/1~0/1) to obtain a white solid, 3-[[6-[(E)-2-[6-[2-(methylcarbamoyl)phenyl]thio-1-tetrahydropyran-2-yl-indazol-3-yl]vinyl]-3-pyridyl]oxymethyl]pyrrolidin-1-tert-butyl carboxylate 7b (200 mg, 298.58 μmol, yield: 19.37 %).

This product was verified by LCMS and HNMR.

For MS-ESI, the calculated value was $[M+H]^+$670.3, and the measurement was 670.2.

$^1H$ NMR (400 MHz, $CDCl_3$) δ 8.34 (s, 2H), 8.00 (d, J = 8.4 Hz, 1H), 7.75 (s, 1H), 7.72 - 7.62 (m, 3H), 7.56 (dd, J =

7.2, 1.6 Hz, 1H), 7.52 - 7.43 (m, 2H), 7.36 - 7.30 (m, 1H), 7.25 - 7.17 (m, 2H), 5.81 - 5.66 (m, 1H), 4.02 (d, J = 11.2 Hz, 3H), 3.70 (m, 3H), 3.42 (s, 1H), 3.23 (s, 1H), 2.98 (dd, J = 4.8, 1.2 Hz, 3H), 2.53 (d, J = 13.2 Hz, 1H), 2.11 (s, 4H), 1.93 - 1.66 (m, 4H), 1.65 - 1.42 (m, 9H).

Step 2

At room temperature, trifluoroacetic acid (74.4 mg, 652.76 μmol) was added to the solution of 3-[[6-[(E)-2-[6-[2-(methylcarbamoyl)phenyl]thio-1-tetrahydropyran-2-yl-indazol-3-yl]vinyl]-3-pyridyl]oxymethyl]pyrrolidin-1-tert-butyl carboxylate 7b (100 mg, 149.29 μmol) in dichloromethane (4 mL). The mixture was stirred at room temperature to react for about 2 h. After the reaction of the starting materials completed, as indicated by thin-layer chromatography (dichloromethane/methanol = 10/1), the mixture was poured into water (10 mL), adjusted with solid sodium carbonate to pH=8-9, and extracted with ethyl acetate (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-(3-[(E)-2-{5-[(pyrrolidin-3-yl)methoxy]pyridin-2-yl}vinyl]-1H-indazol-6-yl}thio)benzamide I-7 (18 mg, 37.07 μmol, yield: 24.83 %).
This product was verified by LCMS, HNMR and CNMR.
For MS-ESI, the calculated value was $[M+H]^+$486.2, and the measurement was 486.1.
$^1$H NMR (400 MHz, $CD_3OD$) δ 8.41 (s, 1H), 8.05 (m, 1H), 7.69 - 7.45 (m, 3H), 7.44 - 6.96 (m, 4H), 5.35 (s, 1H), 4.40 - 3.97 (m, 2H), 3.68 - 3.34 (m, 3H), 3.32 - 3.24 (m, 1H), 3.22 - 2.92 (m, 1H), 2.87 (d, J = 12.0 Hz, 3H), 2.42 - 2.18 (m, 1H), 2.08 - 1.84 (m, 1H).
$^{13}$C NMR (100MHz, $CD_3OD$): δ (ppm) 170.31, 155.28, 142.86, 142.18, 137.76, 134.65, 134.50, 131.60, 130.32, 130.14, 127.72, 126.80, 125.61, 123.74, 120.81, 120.36, 113.83, 69.79, 45.24, 37.89, 26.64, 25.34.

I-37

Step 1

Step 2

Step 1

Under the protection of nitrogen at room temperature, [(3S)-1-methylpyrrolidin-3-yl]methanol 8a (114 mg, 986.47 μmol), $Ph_3P$ (501 mg, 1.91 mmol) and DIAD (249 mg, 1.23 mmol) were added to the solution of 2-[3-[(E)-2-(5-hydroxy-2-pyridyl)vinyl]-1-tetrahydropyran-2-yl-indazol-6-yl] thio-N-methyl-benzamide 1i (400 mg, 822.06 μmol) in tetrahydrofuran (10 mL); the solution was stirred at 40°C to react for about 16 h. After the reaction of the starting materials completed, as indicated by thin-layer chromatography (dichloromethane/methanol = 10/1), the mixture was poured into water (50 mL) and extracted with ethyl acetate (30 mL × 3); and the combined organic layers were washed with saturated brine (50 mL), dried over anhydrous sodium sulfate and concentrated to obtain a crude product. The crude product was purified by preparative thin-layer chromatography to obtain a white solid, N-methyl-2-[1-methyl-3-[(E)-2-[5-[(3S)-1-methylpyrrolidin-3-yl]methoxy]-2-pyridyl]vinyl]indazol-6-yl]thiobenzamide 8b (300 mg, 513.93 μmol, yield: 62.52 %).
This product was verified by LCMS.
For MS-ESI, the calculated value was $[M+H]^+$584.3, and the measurement was 584.8.

Step 2

At room temperature, trifluoroacetic acid (3 g, 26.31 mmol) was added to the solution of N-methyl-2-[1-methyl-3-[(E)-2-[5-[(3S)-1-methylpyrrolidin-3-yl]methoxy]-2-pyridyl]vinyl]indazol-6-yl]thiobenzamide 8b (200 mg, 342.62 μmol) in dichloromethane (4 mL). The mixture was stirred at room temperature to react for about 16 h. After the reaction of the starting materials completed, as indicated by thin-layer chromatography (dichloromethane/methanol = 10/1), the mixture was poured into water (20 mL), adjusted with the aqueous solution of sodium carbonate (10 mL) to pH=8-9, and extracted with ethyl acetate (20 mL × 3). The organic layers were combined, washed with saturated brine (30 mL), dried over anhydrous sodium sulfate and concentrated to obtain a crude product. The crude product was purified by preparative thin-layer chromatography to obtain a white solid, N-methyl-2-({3-[(E)-2-(5-{[(3S)-1-methylpyrrolidin-3-yl] methoxy}pyridin-2-yl)vinyl]-1H-indazol-6-yl}thio)benzamide I-37 (43 mg, 86.06 μmol, yield: 25.12 %).
This product was verified by LCMS, HNMR and CNMR.
For MS-ESI, the calculated value was $[M+H]^+$500.2, and the measurement was 500.1.
$^1$H NMR (400 MHz, $CD_3OD$) δ 8.27 (d, J = 2.8 Hz, 1H), 8.04 (d, J = 8.4 Hz, 1H), 7.78 (s, 1H), 7.69 (d, J = 16.4 Hz, 1H), 7.65 - 7.60 (m, 2H), 7.49 (s, 1H), 7.42 (dd, J = 8.8, 2.8 Hz, 1H), 7.29 (dd, J = 13.2, 6.8 Hz, 2H), 7.23 - 7.18 (m, 2H), 4.11 (d, J = 5.2 Hz, 2H), 3.13 (dd, J = 9.2, 4.8 Hz, 1H), 2.88 (s, 3H), 2.82 (s, 1H), 2.54 (s, 3H), 2.45 - 2.38 (m, 1H), 2.16 - 2.08 (m,

1H), 1.90 - 1.82 (m, 2H), 1.77 (dd, J = 13.2, 6.0 Hz, 1H).

$^{13}$C NMR (100 MHz, $CDCl_3$&$CD_3OD$) δ 168.68, 153.46, 147.44, 135.78, 133.95, 132.59, 130.85, 129.82, 128.58, 127.35, 125.96, 124.24, 121.34, 121.02, 120.60, 69.94, 63.40, 56.74, 40.70, 27.41, 25.51, 21.78.

I-39

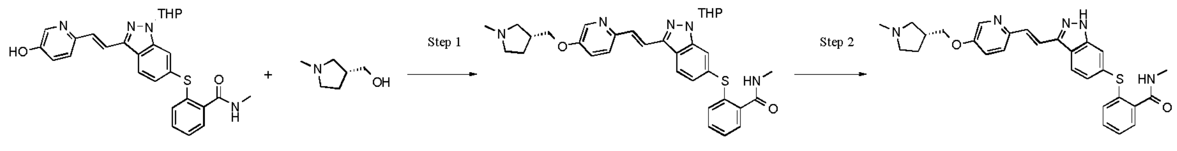


1i 9a 9b I-39

Step 1

Under the protection of nitrogen at 0°C, [(3R)-1-methylpyrrolidin-3-yl]methanol 9a (142 mg, 1.23 mmol), $Ph_3P$ (501 mg, 1.91 mmol) and DIAD (386.5 mg, 1.91 mmol) were added to the solution of 2-[3-[(E)-2-(5-hydroxy-2-pyridyl) vinyl]-1-tetrahydropyran-2-yl-indazol-6-yl] thio-N-methyl-benzamide 1i (500 mg, 1.03 mmol) in tetrahydrofuran (10 mL); the solution was stirred at 40 °C to react for about 16 h. After the reaction of the starting materials completed, as indicated by thin-layer chromatography (petroleum ether/ethyl acetate =1/1), the mixture was poured into water (20 mL) and extracted with ethyl acetate (20 mL × 3); and the combined organic layers were washed with saturated brine (20 mL), dried over anhydrous sodium sulfate and concentrated to obtain a crude product. The crude product was purified by silica gel column chromatography (petroleum ether/ethyl acetate = 10/1) to obtain a yellow solid, N-methyl-2-[1-methyl-3-[(E)-2-[5-[(3R)-1-methylpyrrolidin-3-yl]methoxy]-2-pyridyl]vinyl]indazol-6-yl]thiobenzamide 9b (350 mg, 599.58 μmol, yield: 58.35 %).

This product was verified by LCMS.

For MS-ESI, the calculated value was $[M+H]^+$584.3, and the measurement was 584.2.

Step 2

At room temperature, trifluoroacetic acid (5.3 g, 46.04 mmol) was added to the solution of N-methyl-2-[1-methyl-3-[(E)-2-[5-[(3R)-1-methylpyrrolidin-3-yl]methoxy]-2-pyridyl]vinyl]indazol-6-yl]thiobenzamide 9b (350 mg, 599.58 μmol) in dichloromethane (6 mL). The mixture was stirred at room temperature to react for about 16 h. After the reaction of the starting materials completed, as indicated by thin-layer chromatography (dichloromethane/methanol = 10/1), the mixture was poured into water (5 mL), adjusted with solid sodium carbonate to pH=8-9, and extracted with dichloromethane (5 mL × 3). The organic layers were combined, washed with water (5 mL × 2), dried over anhydrous sodium sulfate and concentrated to obtain a crude product. The crude product was purified by preparative thin-layer chromatography to obtain a yellow solid, N-methyl-2-({3-[(E)-2-(5-{ [(3R)-1-methylpyrrolidin-3-yl]methoxy}pyridin-2-yl)vinyl]-1H-indazol-6-yl}thio)benzamide I-39 (45 mg, 90.07 μmol, yield: 15.02 %).

This product was verified by LCMS, HNMR and CNMR.

For MS-ESI, the calculated value was $[M+H]^+$500.2, and the measurement was 500.1.

$^1$H NMR (400 MHz, $CD_3OD$&$CDCl_3$) δ 8.27 (s, 1H), 8.04 (d, J = 8.4 Hz, 1H), 7.76 (s, 1H), 7.71-7.67 (m, 1H), 7.64-7.61 (m, 2H), 7.49-7.47 (m, 1H), 7.43-7.41 (m, 1H), 7.33-7.27 (m, 2H), 7.21 (t, J = 8.0 Hz, 2H), 4.11 (d, J = 5.2 Hz, 2H), 3.15 - 3.13 (m, 1H), 2.89 (s, 3H), 2.82 (s, 1H), 2.54 (s, 3H), 2.43-2.39(m, 1H), 2.16-2.11 (m, 1H), 1.88-1.85 (m, 2H), 1.80-1.75 (m, 2H).

$^{13}$C NMR (100 MHz, $CDCl_3$&$CD_3OD$) δ 169.88, 154.17, 148.04, 142.25, 141.19, 136.90, 136.34, 135.28, 133.24, 130.79, 130.20, 128.97, 127.50, 125.29, 122.05, 121.78, 121.54, 121.10, 120.12, 114.30, 70.29, 64.04, 57.23, 41.01, 27.74, 25.77, 22.21.

I-3

Step 1 Step 2 Step 3

Step 1

At low temperature, sodium hydride (1.5 g, 37.15 mmol, purity: 60 %) was added stepwisely to the solution of 6-vinylpyridin-3-ol 1c (1.5 g, 12.38 mmol) in dried N,N-dimethylformamide (20 mL). Then, the mixture was stirred at 0°C for 1 h. Then, 4-(2-chloroethyl)morpholinehydrochloride 10a (2.8 g, 14.86 mmol) was slowly added. The mixture was stirred at 60°C for about 4 h. After the thin-layer chromatography (petroleum ether/ethyl acetate = 3/1) showed the complete reaction of the starting materials, the mixture was poured into water (100 mL) and extracted with ethyl acetate (60 mL × 3). The organic layers were combined, washed with saturated brine (100 mL), dried over anhydrous sodium sulfate and concentrated to obtain a crude product. The crude product was purified by preparative thin-layer chromatography to obtain a yellow oily liquid, 4-[2-[(6-vinyl-3-pyridyl)oxy]ethyl]morpholine 10b (1.5 g, 6.40 mmol, yield: 51.70 %).

This product was verified by LCMS and HNMR.

For MS-ESI, the calculated value was $[M+H]^+$235.1, and the measurement was 235.1. $^1$H NMR (400 MHz, $CDCl_3$): δ (ppm) 8.29 (d, J = 2.8 Hz, 1H), 7.32-7.30 (m, 1H), 7.20-7.17 (m, 1H), 6.82-6.75 (m, 1H), 6.07-6.02 (m, 1H), 5.39-5.36 (m, 1H), 4.19-4.13 (m, 2H), 3.77-3.75 (m, 4H), 2.85-2.82 (m, 2H), 2.61-2.59 (m, 4H).

Step 2

Under the protection of nitrogen at room temperature, 4-[2-[(6-vinyl-3-pyridyl)oxy]ethyl]morpholine 10b (57 mg, 0.24 mmol), triethylamine (61.5 mg, 0.61 mmol), $Pd_2(dba)_3$ (45.5 mg, 0.20 mmol) and P(o-tol)3 (61.7 mg, 0.20 mmol) were added to the solution of 2-(3-iodo-1-tetrahydropyran-2-yl-indazol-6-yl)thio-N-methyl-benzamide 1h (100 mg, 0.20 mmol) in 1,4-dioxane (5 mL); the solution was heated to 100°C and stirred to allow for reaction. After the reaction of the starting materials completed, as indicated by thin-layer chromatography (dichloromethane/methanol = 20 /1), the mixture was poured into water (10 mL) and extracted with ethyl acetate (10 mL × 3); the combined organic layers were washed with saturated brine (20 mL), dried over anhydrous sodium sulfate and concentrated to obtain a crude product; and the crude product was purified by preparative thin-layer chromatography (dichloromethane/methanol = 20/1) to obtain a brownish-yellow solid, N-methyl-2-[3-[(E)-2-[5-(2-morpholinoethoxy)-2-pyridyl]vinyl]-1-tetrahydropyran-2-yl-indazol-6-yl]thiobenzamide 10c (100 mg, 166.74 μmol, yield: 82.26 %).

This product was verified by LCMS.

For MS-ESI, the calculated value was $[M+H]^+$600.2, and the measurement was 600.1.

Step 3

At room temperature, trifluoroacetic acid (380.2 mg, 3.33 mmol) was added to the solution of N-methyl-2-[3-[(E)-2-[5-(2-morpholineethoxy)-2-pyridyl]vinyl]-1-tetrahydropyran-2-yl-indazol-6-yl]thiobenzamide 10c (400 mg, 0.67 mmol) in dichloromethane (2 mL). The mixture was stirred at 35°C for 16 hours. After the reaction of the starting materials completed, as indicated by thin-layer chromatography (dichloromethane/methanol = 10/1), the mixture was poured into water (40 mL), adjusted with solid sodium carbonate to pH=8-9, and extracted with dichloromethane (30 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[2-(morpholin-4-yl)ethoxy]pyridin-2-yl}vinyl]-IH-indazol-6-yl}thio)benzamide I-3 (53.7 mg, 104.15 μmol, yield: 15.62 %).

This product was verified by LCMS, HNMR and CNMR.

For MS-ESI, the calculated value was $[M+H]^+$516.2, and the measurement was 516.2

$^1$H NMR (400MHz, $CDCl_3$): δ (ppm) 10.28 (s, 1H), 8.38-8.37 (m, 1H), 8.02 (d, J = 8.4 Hz, 1H), 7.78-7.74 (m, 1H), 7.68-7.66 (m, 1H), 7.57-7.52 (m, 2H), 7.44 (d, J = 8.8 Hz, 1H), 7.34-7.32 (m, 2H), 7.27-7.22 (m, 3H), 6.37-6.34 (m, 1H), 4.22 (t, J = 5.6 Hz, 2H), 3.78 (t, J = 4.4 Hz, 4H), 2.98 (d, J = 5.2 Hz, 3H), 2.87 (t, J = 5.6 Hz, 2H), 2.63 (s, 4H). $^{13}$C NMR (100 MHz, $CDCl_3$&$CD_3OD$): δ (ppm) 170.16, 154.28, 148.42, 137.19, 136.59, 135.60, 133.54, 131.08, 130.51, 129.24, 127.79, 126.42, 125.60, 122.32, 122.21, 121.99, 121.40, 114.51, 66.52, 65.88, 57.34, 53.82, 26.09.

I-19

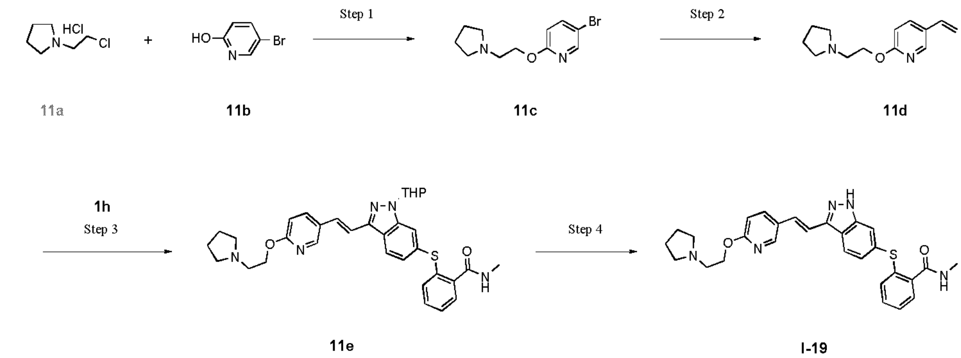


11a 11b 11c 11d

11e I-19

Step 1

Under the protection of nitrogen at 0°C, sodium hydride (689.67 mg, 17.24 mmol, purity: 60 %) was added to the solution of 5-bromopyridin-2-ol 11b (1 g, 5.75 mmol) in N,N-dimethylformamide (20 mL) in portions; the mixture was stirred at 0°C for 1 h; 1-(2-chloroethyl)pyrrolidine hydrochloride 11a (1.17 g, 6.90 mmol) was then added at 0°C; and the mixture was heated to 60°C and stirred for 16 h. After the thin-layer chromatography (petroleum ether/ethyl acetate = 1/1) showed the complete reaction of the starting materials, the mixture was poured into water (50 mL) and extracted with ethyl acetate (50 mL × 3), and the organic layers were combined, washed with saturated brine (100 mL), dried over anhydrous sodium sulfate and concentrated to obtain a crude product, which was a brownish-yellow oily liquid 5-bromo-2-(2-pyrrolidin-1-methoxy)pyridine 11c (1 g, 3.69 mmol, yield: 64.17 %). This product was verified by LCMS and HNMR.

For MS-ESI, the calculated value was $[M+H]^+$272.9, and the measurement was 272.9.

$^1$H NMR (400 MHz, DMSO-$d_6$) δ 8.18 - 8.02 (m, 1H), 7.50 (dt, J = 13.8, 6.9 Hz, 1H), 7.41 - 7.21 (m, 1H), 4.12 (dd, J = 5.5, 2.9 Hz, 2H), 2.76 (dd, J = 5.5, 2.8 Hz, 2H), 2.48 (d, J = 1.7 Hz, 4H), 1.65 (d, J = 3.1 Hz, 4H).

Step 2

At room temperature, potassium vinyltrifluoroborate 1b (750.0 mg, 5.60 mmol), Pd(dppf)$Cl_2$ (80.1 mg, 109.53 μmol) and triethylamine (560.0 mg, 5.53 mmol) were added to the solution of 5-bromo-2-(2-pyrrolidin-1-methoxy)pyridine 11c (1 g, 3.69 mmol) in dried N,N-dimethylformamide (20 mL), heated to 85°C and stirred to react for about 16 h. After the reaction of the starting materials completed, as indicated by thin-layer chromatography (dichloromethane/methanol = 10/1), the mixture was poured into water (50 mL) and extracted with ethyl acetate (50 mL × 3); and the combined organic layers were washed with saturated brine (200 mL), dried over anhydrous sodium sulfate and concentrated to obtain a crude product. The crude product was purified by preparative thin-layer chromatography (dichloromethane/methanol = 10/1) to obtain a yellow solid, 2-(2-pyrrolidin-1-ethoxy)-5-vinylpyridine 11d (340 mg, 1.56 mmol, yield: 42.23 %).

This product was verified by LCMS and HNMR.

For MS-ESI, the calculated value was $[M+H]^+$219.1, and the measurement was 219.1.

$^1$H NMR (400 MHz, $CDCl_3$) δ 7.89 - 7.53 (m, 1H), 7.52 - 7.32 (m, 1H), 6.84 - 6.54 (m, 1H), 6.51 - 6.27 (m, 1H), 5.73 - 5.42 (m, 1H), 5.16 (dt, J = 22.5, 11.2 Hz, 1H), 4.06 (d, J = 17.0 Hz, 2H), 2.83 (d, J = 10.8 Hz, 2H), 2.60 (d, J = 7.4 Hz, 4H), 1.84 - 1.76 (m, 4H).

Step 3

Under the protection of nitrogen at room temperature, 2-(2-pyrrolidin-1-ethoxy)-5-vinylpyridine 11d (280 mg, 1.28 mmol), triethylamine (300 mg, 2.96 mmol), $Pd_2(dba)_3$ (250 mg, 1.11 mmol) and P(o-tol)3 (250 mg, 821.40 μmol) were added to the solution of 2-(3-iodo-1-tetrahydropyran-2-yl-indazol-6-yl)thio-N-methyl-benzamide 1h (500 mg, 1.01 mmol) in 1,4-dioxane (20 mL); the solution was heated to 100 °C and stirred to react for about 2 h. After the reaction of the starting materials completed, as indicated by thin-layer chromatography (dichloromethane/methanol = 20 /1), the mixture was poured into water (30 mL) and extracted with ethyl acetate (30 mL × 3); the combined organic layers were washed with saturated brine (50 mL), dried over anhydrous sodium sulfate and concentrated to obtain a crude product; and the crude product was purified by preparative thin-layer chromatography (dichloromethane/methanol = 10/1) to obtain a yellow solid, N-methyl-2-[3-[(E)-2-[6-(2-pyrrolidin-1-ylethoxy)-3-pyridyl]vinyl]-1-tetrahydropyran-2-yl-indazol-6-yl]thiobenzamide 11e (200 mg, 342.62 μmol, yield: 33.81 %).

This product was verified by LCMS.

For MS-ESI, the calculated value was $[M+H]^+$584.3, and the measurement was 584.4.

Step 4

At room temperature, trifluoroacetic acid (1.20 g, 10.52 mmol) was added to the solution of N-methyl-2-[3-[(E)-2-[6-(2-pyrrolidin-1-ylethoxy)-3-pyridyl]vinyl]-1-tetrahydropyran-2-yl-indazol-6-yl]thiobenzamide 11e (200 mg, 342.62 μmol) in dichloromethane (8 mL). The mixture was stirred at 40°C for 16 hours. After the reaction of the starting materials completed, as indicated by thin-layer chromatography (dichloromethane/methanol = 10/1), the mixture was poured into water (10 mL), adjusted with the aqueous solution of sodium carbonate (10 mL) to pH=8-9, and extracted with ethyl acetate (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{6-[2-(pyrrolidin-1-yl)ethoxy]pyridin-3-yl}vinyl]-1H-indazol-6-yl}thio)benzamide I-19 (10.4 mg, 20.82 μmol, yield: 6.08 %).

This product was verified by LCMS, HNMR and CNMR.

For MS-ESI, the calculated value was $[M+H]^+$500.2, and the measurement was 500.0.

$^1H$ NMR (400 MHz, $CDCl_3$) δ 11.49 (s, 1H), 7.84 (d, J = 8.5 Hz, 1H), 7.65 (d, J = 5.0 Hz, 1H), 7.43 - 7.28 (m, 6H), 7.22 - 7.08 (m, 2H), 6.94 (dd, J = 41.7, 16.5 Hz, 2H), 6.19 (d, J = 9.4 Hz, 1H), 4.13 (s, 2H), 3.07 - 2.92 (m, 3H), 2.72 (s, 2H), 1.85 (s, 2H), 1.62 (s, 4H), 1.28 (d, J = 10.3 Hz, 2H).

$^{13}C$ NMR (100MHz, $CDCl_3$): δ (ppm) 170.29, 162.66, 137.38, 137.19, 136.91, 135.50, 133.68, 131.08, 130.36, 127.70, 126.39, 125.63, 125.28, 121.29, 119.95, 118.46, 54.22, 54.00, 25.76, 23.11.

I-17

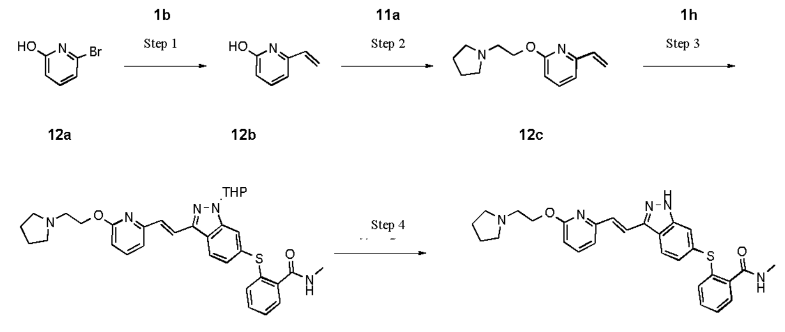


12a 12b 12c

12d I-17

Step 1

Under the protection of nitrogen at room temperature, potassium vinyltrifluoroborate 1b (577. 4 mg, 4.31 mmol), Pd(dppf)$Cl_2$ (100 mg, 137.81 μmol) and triethylamine (872.4 mg, 8.62 mmol) were added to the solution of 6-bromopyridin-2-ol 12a (500 mg, 2.87 mmol) in *N,N*-dimethylformamide (10 mL); the solution was heated to 80 °C and stirred to react for about 16 h. After the reaction of the starting materials completed, as indicated by thin-layer chromatography (dichloromethane/methanol = 10/1), the mixture was poured into water (100 mL) and extracted with ethyl acetate (40 mL × 3); and the combined organic layers were washed with saturated brine (100 mL), dried over anhydrous sodium sulfate and concentrated to obtain a crude product. The crude product was purified by silica gel column chromatography (petroleum ether/ethyl acetate = 10/1-1/10) to obtain a white solid, 6-vinylpyridin-2-ol 12b (140 mg, 1.16 mmol, yield: 40.22 %).

This product was verified by HNMR. $^1H$ NMR (400 MHz, $CDCl_3$): δ (ppm) 11.84 (s, 1H), 7.42 (s, 1H), 6.46 (m, 2H), 6.14 (m, 2H), 5.55 (d, J = 11.3 Hz, 1H).

Step 2

Under the protection of nitrogen at room temperature, 1-(2-chloroethyl)pyrrolidine hydrochloride 11a (185.3 mg, 1.09 mmol), NaI (19.1 mg, 127.13 μmol) and silver carbonate (751.2 mg, 2.72 mmol) were added to the solution of 6-vinylpyridin-2-ol 12b (110 mg, 908.07 μmol) in N,N-dimethylformamide (2 mL); the solution was heated to 100 °C and stirred to react for about 4 h. After the reaction of the starting materials completed, as indicated by thin-layer chromatography (dichloromethane/methanol = 10/1), the mixture was poured into water (10 mL) and extracted with ethyl acetate (10 mL × 3); and the combined organic layers were washed with saturated brine (20 mL), dried over anhydrous sodium sulfate and concentrated to obtain a crude product. The crude product was purified by preparative thin-layer chromatography (dichloromethane/methanol = 10 /1) to obtain a yellow solid, 2-(2-pyrrolidin-1-ethoxy)-6-vinylpyridine 12c (75 mg, 343.57 μmol, yield: 37.84 %).

This product was verified by LCMS and HNMR.

For MS-ESI, the calculated value was $[M+H]^+$219.1, and the measurement was 219.1.

$^1$H NMR (400MHz, $CDCl_3$): δ (ppm) 7.53 (t, J=8.0Hz, 1H), 6.84 (d, J=7.2 Hz, 1H), 6.75-6.67 (m, 2H), 6.30-6.25(m, 1H), 5.43-5.40 (m, 1H), 4.58 (t, J=6.0Hz, 2H), 3.01(s, 2H), 2.76 (s, 4H), 1.88 (s, 4H).

Step 3

Under the protection of nitrogen at room temperature, 2-(2-pyrrolidin-1-ethoxy)-6-vinylpyridine 12c (265.5 mg, 1.22 mmol), triethylamine (307.7 mg, 3.04 mmol), $Pd_2(dba)_3$ (464 mg, 506.73 μmol) and P(o-tol)3 (308.5 mg, 1.01 mmol) were added to the solution of 2-(3-iodo-1-tetrahydropyran-2-yl-indazol-6-yl)thio-N-methyl-benzamide 1h (500 mg, 1.01 mmol) in 1,4-dioxane (20 mL); the solution was heated to 100 °C and stirred to react for about 2 h. After the reaction of the starting materials completed, as indicated by thin-layer chromatography (dichloromethane/methanol = 10/1), the mixture was poured into water (30 mL) and extracted with ethyl acetate (30 mL × 3); the combined organic layers were washed with saturated brine (40 mL), dried over anhydrous sodium sulfate and concentrated to obtain a crude product; and the crude product was purified by preparative thin-layer chromatography (dichloromethane/methanol = 10/1) to obtain a yellow solid, N-methyl-2-[3-[(E)-2-[6-(2-pyrrolidin-1-ylethoxy)-2-pyridyl]vinyl]-1-tetrahydropyran-2-yl-indazol-6-yl]thiobenzamide 12d (370 mg, 633.84 μmol, yield: 62.54 %).

This product was verified by LCMS.

For MS-ESI, the calculated value was $[M+H]^+$584.3, and the measurement was 584.1.

Step 4

At room temperature, trifluoroacetic acid (78.1 mg, 685.23 μmol) was added to the solution of N-methyl-2-[3-[(E)-2-[6-(2-pyrrolidin-1-ylethoxy)-2-pyridyl]vinyl]-1-tetrahydropyran-2-yl-indazol-6-yl]thiobenzamide 12d (0.4 g, 685.23 μmol) in dichloromethane (10 mL). The mixture was stirred at 30°C for 16 hours. After the reaction of the starting materials completed, as indicated by thin-layer chromatography (dichloromethane/methanol = 10/1), the mixture was poured into water (40 mL), adjusted with the solid particles of sodium carbonate to pH=8-9, and extracted with dichloromethane (20 mL × 3). The organic layers were combined, washed with saturated brine (30 mL), dried over anhydrous sodium sulfate and concentrated to obtain a crude product. The crude product was purified by preparative thin-layer chromatography to obtain a yellow solid, N-methyl-2-({3-[(E)-2-{6-[2-(pyrrolidin-1-yl)ethoxy]pyridin-2-yl}vinyl]-1H-indazol-6-yl}thio)benzamide I-17 (27.5 mg, 55.04 μmol, yield: 8.03 %).

This product was verified by LCMS, HNMR and CNMR.

For MS-ESI, the calculated value was $[M+H]^+$500.2, and the measurement was 500.1.

$^1$H NMR (400MHz, $CDCl_3$): δ (ppm) 10.61-10.42 (m, 1H), 8.02-7.97 (m, 2H), 7.65 (s, 1H), 7.56 (t, J = 7.6 Hz, 1H), 7.43 (d, J = 16.1 Hz, 1H), 7.32 (d, J = 3.7 Hz, 1H), 7.24 (d, J = 8.3 Hz, 1H), 6.95 (d, J = 7.1 Hz, 1H), 6.70 (d, J = 8.3 Hz, 1H), 6.36 (s, 1H), 4.62 (t, J = 5.9 Hz, 2H), 2.98 (t, J = 6.1 Hz, 3H), 2.70 (s, 2H), 1.85 (s, 2H).

$^{13}$C NMR (100 MHz, $CDCl_3$): δ (ppm) 168.95, 163.13, 152.66, 143.44, 142.25, 138.96, 136.19, 135.01, 132.77, 130.85, 129.90, 128.59, 126.84, 125.06, 123.37, 121.68, 120.89, 115.98, 114.33, 110.36, 64.19, 55.05, 54.61, 26.88, 23.47.

I-15

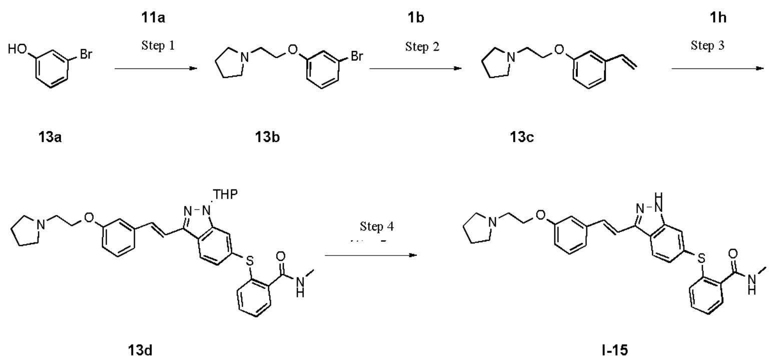


Step 1

Under the protection of nitrogen at room temperature, 1-(2-chloroethyl)pyrrolidine hydrochloride 11a (1.47 g, 8.67 mmol) and cesium carbonate (5.65 g, 17.34 mmol) were added to the solution of 3-bromophenol 13a (1.00 g, 5.78

mmol) in DMSO (10 mL); the solution was heated to 50°C and stirred to react for about 16 h. After the reaction of the starting materials completed, as indicated by thin-layer chromatography (petroleum ether/ethyl acetate =1/1), the mixture was poured into water (40 mL) and extracted with ethyl acetate (20 mL × 3); and the combined organic layers were washed with saturated brine (20 mL), dried over anhydrous sodium sulfate and concentrated to obtain a crude product. The crude product was purified by silica gel column chromatography (petroleum ether/ethyl acetate = 5/1~3/1) to obtain a white solid, 1-[2-(3-bromophenoxy)ethyl]pyrrolidine 13b (620 mg, 2.29 mmol, yield: 39.70 %),

This product was verified by LCMS and HNMR.

For MS-ESI, the calculated value was $[M+H]^+$270.0, and the measurement was 270.0.

$^1$H NMR (400 MHz, $CDCl_3$) δ 7.13-7.17 (m, 1H), 7.08-7.10 (m, 2H), 6.86-6.89 (m, 1H), 4.10 (t, J = 6.0 Hz, 2H), 2.91 (t, J = 6.0 Hz, 1H), 2.63-2.66 (m, 4H), 1.81-1.85 (m, 4H).

Step 2

Under the protection of nitrogen at room temperature, potassium vinyltrifluoroborate 1b (577.4 mg, 4.31 mmol), Pd(dppf)$Cl_2$ (65.00 mg, 88.83 μmol) and triethylamine (898.92 mg, 8.88 mmol) were added to the solution of 1-[2-(3-bromophenoxy)ethyl]pyrrolidine 13b (800 mg, 2.96 mmol) in N,N-dimethylformamide (8 mL); the solution was heated to 85 °C and stirred to react for about 16 h. After the reaction of the starting materials completed, as indicated by thin-layer chromatography (dichloromethane/methanol = 10/1), the mixture was poured into water (100 mL) and extracted with ethyl acetate (50 mL × 3); and the combined organic layers were washed with saturated brine (100 mL), dried over anhydrous sodium sulfate and concentrated to obtain a crude product. The crude product was purified by silica gel column chromatography (petroleum ether/ethyl acetate = 10/1-dichloromethane/methanol = 10/1) to obtain a white solid, 1-[2-(3-vinylphenoxy)ethyl]pyrrolidine 13c (300 mg, 1.38 mmol, yield: 46.62 %).

This product was verified by LCMS and HNMR.

For MS-ESI, the calculated value was $[M+H]^+$218.1, and the measurement was 218.1.

$^1$H NMR (400MHz, $CDCl_3$): δ (ppm)7.29-7.15 (m, 1H), 6.98 (m, 2H), 6.83 (m, 1H), 6.68 (dd, J = 17.6, 10.9 Hz, 1H), 5.84-5.60 (m, 1H), 5.24 (d, J = 10.9 Hz, 1H), 4.20-4.03 (m, 2H), 2.90 (t, J = 6.0 Hz, 1H), 2.70-2.54 (m, 4H), 1.87-1.75 (m, 4H).

Step 3

Under the protection of nitrogen at room temperature, 1-[2-(3-vinylphenoxy)ethyl]pyrrolidine 13c (132.14 mg, 608.08 μmol), triethylamine (123.06 mg, 1.22 mmol), $Pd_2(dba)_3$ (371.22 mg, 405.38 μmol) and P(o-tol)3 (123.38 mg, 405.38 μmol) were added to the solution of 2-(3-iodo-1-tetrahydropyran-2-yl-indazol-6-yl)thio-N-methyl-benzamide 1h (200 mg, 405.38 μmol) in 1,4-dioxane (5 mL); the solution was heated to 100 °C and stirred to react for about 4 h. After the reaction of the starting materials completed, as indicated by thin-layer chromatography (dichloromethane/methanol = 10 /1), the mixture was poured into water (20 mL) and extracted with ethyl acetate (10 mL × 3); the combined organic layers were washed with saturated brine (20 mL), dried over anhydrous sodium sulfate and concentrated to obtain a crude product; and the crude product was purified by preparative thin-layer chromatography (dichloromethane/methanol = 10/1) to obtain a yellow solid, N-methyl-2-[3-[(E)-2-[3-(2-pyrrolidin-1-ethoxy)phenyl]vinyl]-1-tetrahydropyran-2-yl-indazol-6-yl] thiobenzamide 13d (370 mg, 633.84 μmol, yield: 62.54 %).

This product was verified by LCMS.

For MS-ESI, the calculated value was $[M+H]^+$583.3, and the measurement was 583.1.

Step 4

At room temperature, trifluoroacetic acid (0.5 mL) was added to the solution of N-methyl-2-[3-[(E)-2-[3-(2-pyrrolidin-1-ethoxy)phenyl]vinyl]-1-tetrahydropyran-2-yl-indazol-6-yl]thiobenzamide 13d (110 mg, 188.76 μmol) in dichloromethane (3 mL). The mixture was stirred at 40°C to react for about 16 h. After the reaction of the starting materials completed, as indicated by thin-layer chromatography (dichloromethane/methanol = 10/1), the mixture was poured into water (30 mL), and extracted with ethyl acetate (20 mL × 3). The organic layers were combined, washed with saturated brine (30 mL), dried over anhydrous sodium sulfate and concentrated to obtain a crude product. The crude product was purified by preparative thin-layer chromatography to obtain a white solid, N-methyl-2-({3-[(E)-2-{3-[2-(pyrrolidin-1-yl) ethoxy]phenyl}vinyl]-1H-indazol-6-yl}thio)benzamide I-15 (11.8 mg, 23.66 μmol, yield: 12.54 %).

This product was verified by LCMS, HNMR and CNMR.

For MS-ESI, the calculated value was $[M+H]^+$499.2, and the measurement was 499.1.

$^1$H NMR (400MHz, CD3OD): δ (ppm) 8.07 (d, J = 8.5 Hz, 1H), 7.59 (s, 1H), 7.51-7.49 (m, 3H), 7.35-7.32 (m, 5H), 7.26-7.24 (m, 2H), 6.99 (d, J = 8.0 Hz, 1H), 4.44-4.41 (t, J=9.6 Hz,2H), 3.82-3.74 (m, 2H), 3.72-3.70 (m, 2H), 3.30-3.22 (m, 2H), 2.87 (s, 3H), 2.25-2.17 (m, 2H), 2.11-2.00 (m, 2H).

$^{13}$C NMR (100 MHz, CD3OD) δ 170.38, 158.23,143.23, 141.95, 139.35, 137.29, 134.97, 134.12, 131.44, 130.58,

130.26, 129.68, 127.67, 126.62, 125.08, 121.26, 120.13,119.19, 119.86, 114.05, 113.80, 111.92, 62.91, 54.34, 53.80, 36.94, 25.32, 22.51, 16.29.

I-22

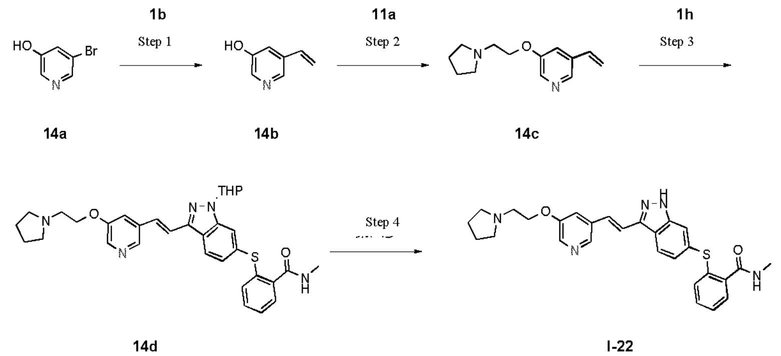


14a 14b 14c

14d I-22

Step 1

Under the protection of nitrogen at room temperature, potassium vinyltrifluoroborate 1b (2.31 g, 17.24 mmol), Pd(dppf)$Cl_2$ (1 g, 1.37 mmol) and triethylamine (3.00 g, 29.65 mmol) were added to the solution of 5-bromopyridin-3-ol 14a (2 g, 11.49 mmol) in N,N-dimethylformamide (20 mL); the solution was heated to 85 °C and stirred to react for about 16 h. After the reaction of the starting materials completed, as indicated by thin-layer chromatography (dichloromethane/-methanol = 10/1), the mixture was poured into water (100 mL) and extracted with ethyl acetate (50 mL × 3); and the combined organic layers were washed with saturated brine (200 mL), dried over anhydrous sodium sulfate and concentrated to obtain a crude product. The crude product was purified by alkaline alumina column chromatography (petroleum ether/ethyl acetate = 10/1~0/1) to obtain a white solid, 5-vinylpyridin-3-ol 14b (600 mg, 4.95 mmol, yield:43.09 %).

This product was verified by LCMS and HNMR.

For MS-ESI, the calculated value was $[M+H]^+$122.1, and the measurement was 122.1.

$^1$H NMR (400 MHz, $CDCl_3$) δ 8.12 (t, J = 27.6 Hz, 2H), 7.38 (s, 1H), 6.67 (dd, J = 17.6, 11.0 Hz, 1H), 5.83 (d, J = 17.6 Hz, 1H), 5.41 (d, J = 10.8 Hz, 1H).

Step 2

Under the protection of nitrogen at 0°C, sodium hydride (300.00 mg, 7.50 mmol, purity: 60 %) was added to the solution of 5-vinylpyridin-3-ol 14b (300 mg, 2.48 mmol) in N,N-dimethylformamide (4 mL) in portions; the mixture was stirred at 0°C for 1 h; 1-(2-chloroethyl)pyrrolidine hydrochloride 11a (600 mg, 3.53 mmol) was then added; and the mixture was heated to room temperature and stirred to react for about 2 h. After the reaction of the starting materials completed, as indicated by thin-layer chromatography (dichloromethane/methanol = 10/1), the mixture was poured into water (10 mL) and extracted with ethyl acetate (10 mL × 3); and the combined organic layers were washed with saturated brine (20 mL), dried over anhydrous sodium sulfate and concentrated to obtain a crude product. The crude product was purified by preparative thin-layer chromatography (petroleum ether/ethyl acetate = 5/1~3/1) to obtain a yellow solid 3-(2-pyrrolidin-1-ethoxy)-5-vinylpyridine 14c (190 mg, 870.39 μmol, yield: 35.15 %).

This product was verified by LCMS and HNMR.

For MS-ESI, the calculated value was $[M+H]^+$219.1, and the measurement was 219.1.

$^1$H NMR (400 MHz, $CDCl_3$) δ 8.33 - 8.06 (m, 2H), 7.61 (dd, J = 12.4, 7.6 Hz, 1H), 6.71 (dd, J = 17.6, 11.2 Hz, 1H), 5.83 (d, J = 17.6 Hz, 1H), 5.41 (d, J = 10.8 Hz, 1H), 4.22 (dd, J = 20.8, 15.2 Hz, 2H), 2.95 (dd, J = 17.2, 11.6 Hz, 2H), 2.54 (d, J = 105.6 Hz, 4H), 1.85 (s, 4H).

Step 3

Under the protection of nitrogen at room temperature, 3-(2-pyrrolidin-1-ethoxy)-3-vinylpyridine 14c (200 mg, 916.20 μmol), triethylamine (300.00 mg, 2.96 mmol), $Pd_2(dba)_3$ (320 mg, 1.43 mmol) and P(o-tol)3 P(o-tol)3 (220 mg, 722.83 μmol) were added to the solution of 2-(3-iodo-1-tetrahydropyran-2-yl-indazol-6-yl)thio-N-methyl-benzamide 1h (400 mg, 810.77 μmol) in 1,4-dioxane (5 mL); the solution was heated to 100 °C and stirred to react for about 2 h. After the reaction of the starting materials completed, as indicated by thin-layer chromatography (dichloromethane/methanol = 10 /1), the mixture was poured into water (10 mL) and extracted with ethyl acetate (10 mL × 3); the combined organic layers

were washed with saturated brine (20 mL), dried over anhydrous sodium sulfate and concentrated to obtain a crude product; and the crude product was purified by preparative thin-layer chromatography (dichloromethane/methanol = 10/1) to obtain a yellow oily liquid, N-methyl-2-[3-[(E)-2-[5-(2-pyrrolidin-1-ylethoxy)-3-pyridyl]vinyl]-1-tetrahydropyran-2-yl-indazol-6-yl]thiobenzamide 14d (100 mg, 171.31 μmol, yield: 21.13 %).

This product was verified by LCMS.

For MS-ESI, the calculated value was $[M+H]^+$584.3, and the measurement was 584.1.

Step 4

At room temperature, trifluoroacetic acid TFA (1.5 g, 13.16 mmol) was added to the solution of N-methyl-2-[3-[(E)-2-[5-(2-pyrrolidin-1-ylethoxy)-3-pyridyl]vinyl]-1-tetrahydropyran-2-yl-indazol-6-yl]thiobenzamide 14d (100 mg, 171.31 μmol) in dichloromethane (3 mL). The mixture was stirred at room temperature to react for about 2 h. After the reaction of the starting materials completed, as indicated by thin-layer chromatography (dichloromethane/-methanol = 10/1), the mixture was poured into water (10 mL), and extracted with ethyl acetate (10 mL × 3). The organic layers were combined, washed with saturated brine (20 mL), dried over anhydrous sodium sulfate and concentrated to obtain a crude product. The crude product was purified by preparative thin-layer chromatography to obtain a white solid, N-methyl-2-({3-[(E)-2-{5-[2-(pyrrolidin-1-yl)ethoxy]pyridin-3-yl}vinyl]-1H-indazol-6-yl}thio)benzamide I-22 (16.3 mg, 32.62 μmol, yield: 19.04 %).

This product was verified by LCMS, HNMR and CNMR.

For MS-ESI, the calculated value was $[M+H]^+$500.2, and the measurement was 500.9.

$^1H$ NMR (400 MHz, $CDCl_3$) δ 8.39 (s, 1H), 8.25 (d, J = 2.6 Hz, 1H), 7.97 (d, J = 8.5 Hz, 1H), 7.70 - 7.65 (m, 1H), 7.52 (s, 1H), 7.43 (d, J = 10.4 Hz, 3H), 7.37 - 7.32 (m, 2H), 7.25 (d, J = 9.7 Hz, 2H), 6.39 (s, 1H), 4.25 (t, J = 5.8 Hz, 2H), 3.00 (dd, J = 10.3, 5.3 Hz, 5H), 2.72 (s, 4H), 1.87 (s, 4H).

$^{13}C$ NMR (100MHz, $CDCl_3$): δ (ppm) 168.85, 155.10, 143.18, 142.19, 141.19, 137.13, 136.55, 134.73, 133.23, 131.65, 130.84, 128.62, 126.96, 125.05, 122.39, 121.41, 120.56, 117.33, 113.97, 67.38, 55.00, 54.72, 53.40, 26.87, 23.52.

I-9

Step 1

Step 2

Step 3

Step 4

Step 1

Under the protection of nitrogen at room temperature, 1-(2-chloroethyl)pyrrolidine hydrochloride 11a (2.8 g, 16.33 mmol) and cesium carbonate (13.3 g, 40.82 mmol) were added to the solution of 4-bromo-1*H*-pyrazole 15a (2 g, 13.61 mmol) in DMSO (20 mL); the solution was heated to 100 °C and stirred to react for about 2.5 h. After the thin-layer chromatography (dichloromethane/methanol = 10/1) showed the complete reaction of the starting materials, the mixture was poured into water (100 mL) and extracted with dichloromethane (40 mL × 3), and the organic layers were combined, washed with saturated brine (100 mL), dried over anhydrous sodium sulfate and concentrated to obtain a crude product. The crude product was purified by silica gel column chromatography (dichloromethane/methanol = 20/1) to obtain a yellow oily liquid, 4-bromo-1-(2-pyrrolidin-1-ethyl)pyrazole 15b (2.5 g, 10.24 mmol, yield: 75.25 %),

This product was verified by HNMR. $^1H$ NMR (400 MHz, $CDCl_3$): δ (ppm) 7.38 (d, J=2.4Hz, 1H), 6.25 (d, J=2.4Hz, 1H), 4.23 (t, J=6.8Hz, 2H), 2.94-2.91 (m, 2H), 2.57-2.52 (m, 4H), 1.79-1.77 (m, 4H).

Step 2

Under the protection of nitrogen at room temperature, potassium vinyltrifluoroborate 1b (440.8 mg, 3.29 mmol), $Pd(dppf)Cl_2$ (542.9 mg, 742.01 μmol) and triethylamine (668.1 mg, 6.60 mmol) were added to the solution of 4-bromo-1-(2-pyrrolidin-1-ethyl)pyrazole 15b (501.4 mg, 2.05 mmol) in N,N-dimethylformamide (10 mL); the solution was heated to 85 °C and stirred to react for about 16 h. After the thin-layer chromatography (dichloromethane/methanol = 10/1) showed the complete reaction of the starting materials, the mixture was poured into water (50 mL) and extracted with dichloromethane

(50 mL × 3), and the organic layers were combined, washed with saturated brine (50 mL), dried over anhydrous sodium sulfate and concentrated to obtain a crude product. The crude product was purified by silica gel column chromatography (dichloromethane/methanol = 20/1) to obtain a red oily liquid, 1-(2-pyrrolidin-1-ethyl)-4-vinylpyrazole 15c (66 mg, 345.06 μmol, yield: 16.80 %).

This product was verified by LCMS and HNMR.

For MS-ESI, the calculated value was $[M+H]^+$192.1, and the measurement was 192.2.

$^1$H NMR (400MHz, $CDCl_3$): δ (ppm) 7.60(d, J=2.4Hz, 1H), 7.47 (d, J=2.4Hz, 1H), 6.5 (t, J=6.8Hz, 1H), 5.5-5.0(m, 2H), 4.27(m, 2H), 2.98(m, 2H),2.575(m, 4H),1.81(m, 5H).

Step 3

Under the protection of nitrogen at room temperature, 1-(2-pyrrolidin-1-ethyl)-4-vinylpyrazole 15c (213.2 mg, 1.11 mmol), triethylamine (307.7 mg, 3.04 mmol), $Pd_2(dba)_3$ (500 mg, 546.04 μmol) and P(o-tol)3 (500.5 mg, 1.64 mmol) were added to the solution of 2-(3-iodo-1-tetrahydropyran-2-yl-indazol-6-yl)thio-N-methyl-benzamide 1h (500 mg, 1.01 mmol) in 1,4-dioxane (5 mL); the solution was heated to 100 °C and stirred to react for about 2 h. After the thin-layer chromatography (dichloromethane/methanol = 10/1) showed the complete reaction of the starting materials, the mixture was poured into water (15 mL) and filtered; the filtrate was extracted with dichloromethane (15 mL × 3); the organic layers were combined, washed with saturated brine (30 mL), dried over anhydrous sodium sulfate and concentrated to obtain a crude product, which was a red oily liquid N-methyl-2-[3-[(E)-2-[1-(2-pyrrolidin-1-ylethyl)pyrazol-4-yl]vinyl]-1-tetrahydropyran-2-yl-indazol-6-yl]thiobenzamide 15d (370 mg, 633.84 μmol, yield: 62.54 %).

This product was verified by LCMS.

For MS-ESI, the calculated value was $[M+H]^+$557.3, and the measurement was 557.3.

Step 4

At room temperature, trifluoroacetic acid (527.7 mg, 4.63 mmol) was added to the solution of N-methyl-2-[3-[(E)-2-[1-(2-pyrrolidin-1-ylethyl)pyrazol-4-yl]vinyl]-1-tetrahydropyran-2-yl-indazol-6-yl]thiobenzamide 15d (360.3 mg, 647.18 μmol) in dichloromethane (10 mL). The mixture was stirred at room temperature to react for about 16 h. After the reaction of the starting materials completed, as indicated by thin-layer chromatography (dichloromethane/methanol = 10/1), the mixture was poured into water (40 mL), adjusted with sodium carbonate to pH=8~9, and extracted with dichloromethane (10 mL × 3); and the combined organic layers were washed with saturated brine (20 mL), dried over anhydrous sodium sulfate and concentrated to obtain a crude product. The crude product was purified by preparative thin-layer chromatography to obtain a yellow solid, N-methyl-2-({3-[(E)-2-{1-[2-(pyrrolidin-1-yl)ethyl]-1H-pyrazole -4-yl}vinyl]-1H-indazol-6-yl}thio)benzamide I-9 (33.8 mg, 71.52 μmol, yield: 11.05 %).

This product was verified by LCMS, HNMR and CNMR.

For MS-ESI, the calculated value was $[M+H]^+$473.2, and the measurement was 473.1.

$^1$H NMR (400MHz, CD3OD): δ (ppm) 10.61-10.42 (m, 1H), 8.02-7.97 (m, 2H), 7.65 (s, 1H), 7.56 (t, J = 7.6 Hz, 1H), 7.43 (d, J = 16.1 Hz, 1H), 7.32 (d, J = 3.7 Hz, 1H), 7.24 (d, J = 8.3 Hz, 1H), 6.95 (d, J = 7.1 Hz, 1H), 6.70 (d, J = 8.3 Hz, 1H), 6.36 (s, 1H), 4.62 (t, J = 5.9 Hz, 2H), 2.98 (t, J = 6.1 Hz, 3H), 2.70 (s, 2H), 1.85 (s, 2H).

$^{13}$C NMR (100 MHz, $CDCl_3$): δ (ppm) 168.73, 144.24, 142.13, 137.42, 136.63, 134.57, 133.25, 130.85, 128.73, 127.88, 127.08, 124.55, 121.63, 120.57, 120.42, 118.04, 113.50, 55.77, 54.23, 51.40, 31.94, 29.70, 26.84, 23.53, 22.70,14.13.

I-32

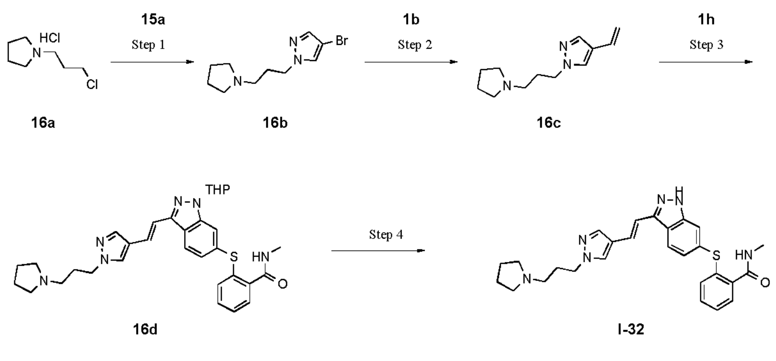

16a 16b 16c 16d I-32

Step 1

**Under** the protection of nitrogen at room temperature, 1-(3-chloropropyl)pyrrolidine hydrochloride 16a (2.4 g, 16.33 mmol) and cesium carbonate (13.3 g, 40.82 mmol) were added to the solution of 4-bromo-1*H*-pyrazole 15a (2 g, 13.61 mmol) in DMSO (20 mL); the solution was heated to 100 °C and stirred to react for about 4 h. After the reaction of the starting materials completed, as indicated by thin-layer chromatography (dichloromethane/methanol = 10/1), the mixture was poured into water (100 mL) and extracted with ethyl acetate (50 mL × 3); and the combined organic layers were washed with saturated brine (120 mL), dried over anhydrous sodium sulfate and concentrated to obtain a crude product. The crude product was purified by silica gel column chromatography (petroleum ether/ethyl acetate = 20/1-0/1) to obtain a yellow solid 4-bromo-1-(3-pyrrolidin-1-ylpropyl)pyrazole 16b (3 g, 11.62 mmol, yield: 85.40 %).

This product was verified by LCMS and HNMR.

For MS-ESI, the calculated value was $[M+H]^+$259.9, and the measurement was 260.1.

$^1$H NMR (400MHz, $CDCl_3$): δ (ppm) 7.43 (d, J = 10.8 Hz, 2H), 4.17 (t, J = 6.8 Hz, 2H), 2.46-2.39 (m, 6H), 2.06-1.99 (m, 2H), 1.82-1.74 (m, 4H).

Step 2

Under the protection of nitrogen at room temperature, potassium vinyltrifluoroborate 1b (778.3 mg, 5.81 mmol), Pd(dppf)$Cl_2$ (85 mg, 116.21 μmol) and triethylamine (587.9 mg, 5.81 mmol) were added to the solution of 4-bromo-1-(3-pyrrolidin-1-ylpropyl)pyrazole 16b (1 g, 3.87 mmol) in N,N-dimethylformamide (10 mL); the solution was heated to 100 °C and stirred to react for about 4 h. After the reaction of the starting materials completed, as indicated by thin-layer chromatography (dichloromethane/methanol = 10/1), the mixture was poured into water (50 mL) and extracted with ethyl acetate (50 mL × 3); and the combined organic layers were washed with saturated brine (120 mL), dried over anhydrous sodium sulfate and concentrated to obtain a crude product. The crude product was purified by silica gel column chromatography (petroleum ether/ethyl acetate = 10/1-0/1 and then dichloromethane/methanol = 20/1-10/1) to obtain a yellow oily liquid, 1-(3-pyrrolidin-1-ylpropyl)-4-vinylpyrazole 16c (300 mg, 1.46 mmol, yield: 37.72 %).

This product was verified by HNMR. $^1$H NMR (400 MHz, $CDCl_3$): δ (ppm) 7.45-7.41 (m, 2H), 6.52 (dd, J = 9.8, 11.2 Hz, 1H), 5.44 (d, J = 16.4 Hz, 1H), 5.05 (d, J = 11.2 Hz, 1H), 4.17 (d, J = 6.8 Hz, 4H), 2.53 (s, 4H), 2.11-1.99 (m, 4H), 1.79 (d, J = 3.2 Hz, 4H).

Step 3

Under the protection of nitrogen at room temperature, 1-(3-pyrrolidin-1-ylpropyl)-4-vinylpyrazole 16c (124.8 mg, 608.08 μmol), triethylamine (92.3 mg, 912.11 μmol), $Pd_2(dba)_3$ (185.1 mg, 608.08 μmol) and P(o-tol)3 (835.2 mg, 912.11 μmol) were added to the solution of 2-(3-iodo-1-tetrahydropyran-2-yl-indazol-6-yl)thio-N-methyl-benzamide 1h (300 mg, 608.08 μmol) in 1,4-dioxane (10 mL); the solution was heated to 100 °C and stirred to react for about 2 h. After the thin-layer chromatography (dichloromethane/methanol = 10/1) showed the complete reaction of the starting materials, the mixture was poured into water (30 mL), extracted with ethyl acetate (30 mL × 3), and the organic layers were combined, washed with saturated brine (50 mL), dried over anhydrous sodium sulfate and concentrated to obtain a crude product, which was a yellow solid N-methyl-2-[3-[(E)-2-[1-(3-pyrrolidin-1-ylpropyl)pyrazol-4-yl]vinyl]-1-tetrahydropyran-2-yl-indazol-6-yl]thiobenzamide 16d (300 mg, 525.63 μmol, yield: 86.44 %).

This product was verified by LCMS.

For MS-ESI, the calculated value was $[M+H]^+$571.3, and the measurement was 571.3.

Step 4

At room temperature, trifluoroacetic acid (100 mg, 876.05 μmol) was added to the solution of N-methyl-2-[3-[(E)-2-[1-(3-pyrrolidin-1-ylpropyl)pyrazol-4-yl]vinyl]-1-tetrahydropyran-2-yl-indazol-6-yl]thiobenzamide 16d (500 mg, 876.05 μmol) in dichloromethane (10 mL), and stirred at room temperature to react for about 16 h. After the reaction of the starting materials completed, as indicated by thin-layer chromatography (dichloromethane/methanol = 10/1), the mixture was poured into water (40 mL), adjusted with sodium carbonate to pH=8~9, and extracted with dichloromethane (30 mL × 3); and the combined organic layers were washed with saturated brine (40 mL), dried over anhydrous sodium sulfate and concentrated to obtain a crude product. The crude product was purified by preparative thin-layer chromatography to obtain a yellow solid, N-methyl-2-({3-[(E)-2-{1-[3-(pyrrolidin-1-yl)propyl]-1H-pyrazole -4-yl} vinyl]-1H-indazol-6-yl}thio)benzamide I-32 (15.4 mg, 31.65 μmol, yield: 3.61 %).

This product was verified by LCMS, HNMR and CNMR.

For MS-ESI, the calculated value was $[M+H]^+$487.2, and the measurement was 487.1.

$^1$H NMR (400 MHz, $CDCl_3$) δ (ppm) 7.91 (d, J = 8.0 Hz, 1H), 7.74 (s, 1H), 7.66-7.62 (m, 1H), 7.58 (s, 1H), 7.47 (s,

1H), 7.34-7.29 (m, 3H), 7.25-7.21 (m, 2H), 7.20-7.17 (m, 1H), 7.11 (d, J = 16.8 Hz, 1H), 6.33 (s, 1H), 4.22 (t, J = 6.8 Hz, 2H), 2.96 (d, J = 4.8 Hz, 3H), 2.59 (d, J = 19.2 Hz, 5H), 2.18-2.11 (m, 2H), 1.84 (s, 5H).

$^{13}$C NMR (100 MHz, $CDCl_3$) $\delta$ (ppm) 168.62, 144.36, 142.08, 137.46, 134.46, 131.96, 130.88, 128.81, 127.58, 127.17, 124.62, 121.80, 121.63, 120.49, 117.88, 113.31, 54.06, 53.00, 50.20, 29.71, 29.07, 26.82, 23.46.

I-13 and I-25

Step 1 Step 2 Step 3

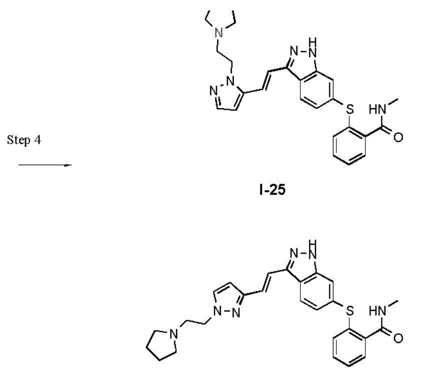


I-25

I-13

Step 1

Under the protection of nitrogen at room temperature, 1-(2-chloroethyl)pyrrolidine hydrochloride 11a (2.8 g, 16.33 mmol) and cesium carbonate (13.3 g, 40.82 mmol) were added to the solution of 3-bromo-1H-pyrazole 17a (2 g, 13.61 mmol) in DMSO (20 mL); the solution was heated to 100 °C and stirred to react for about 4 h. After the thin-layer chromatography (dichloromethane/methanol = 10/1) showed the complete reaction of the starting materials, the mixture was poured into water (30 mL) and extracted with dichloromethane (40 mL × 3), and the organic layers were combined, washed with saturated brine (40 mL), dried over anhydrous sodium sulfate and concentrated to obtain a crude product. The crude product was purified by silica gel column chromatography (petroleum ether/ethyl acetate = 1/0~3/1) to obtain a yellow oily liquid mixture (1.2 g, 4.92 mmol, yield: 36.12 %) of 3-bromo-1-(2-pyrrolidin-1-ethyl)pyrazole 17b (major) and 5-bromo-1-(2-pyrrolidin-1-ethyl)pyrazole 17b'.

3-bromo-1-(2-pyrrolidin-1-ethyl)pyrazole 17b was verified by LCMS and HNMR.

For MS-ESI, the calculated value was $[M+H]^+$245.9, and the measurement was 246.0.

$^1$H NMR (400 MHz, $CDCl_3$): $\delta$ (ppm) 7.38 (d, J=2.4Hz, 1H), 6.25 (d, J=2.4Hz, 1H), 4.23 (t, J=6.8Hz, 2H), 2.94-2.91 (m, 2H), 2.57-2.52 (m, 4H), 1.79-1.77 (m, 4H).

5-bromo-1-(2-pyrrolidin-1-ethyl)pyrazole 17b' was verified by LCMS.

For MS-ESI, the calculated value was $[M+H]^+$245.9, and the measurement was 246.0.

Step 2

Under the protection of nitrogen at room temperature, potassium vinyltrifluoroborate 1b (493.8 mg, 3.69 mmol), $Pd(dppf)Cl_2$ (54 mg, 73.73 $\mu$mol) and triethylamine (373 mg, 3.69 mmol) were added to the solution of the mixture (600 mg, 2.46 mmol) of 3-bromo-1-(2-pyrrolidin-1-ethyl)pyrazole 17b (major) and 5-bromo-1-(2-pyrrolidin-1-ethyl)pyrazole 17b' in N,N-dimethylformamide (5 mL); the solution was heated to 85 °C and stirred to react for about 16 h. After the thin-layer chromatography (dichloromethane/methanol = 10/1) showed the complete reaction of the starting materials, the mixture was poured into water (30 mL) and extracted with dichloromethane (30 mL × 3), and the organic layers were combined, washed with saturated brine (30 mL), dried over anhydrous sodium sulfate and concentrated to obtain a crude product. The crude product was purified by preparative thin-layer chromatography (dichloromethane/methanol = 10/1) to obtain a

red oily liquid mixture (0.4 g, 2.09 mmol, yield: 85.09 %) of 1-(2-pyrrolidin-1-ethyl)-3-vinylpyrazole 17c and 1-(2-pyrrolidin-1-ethyl)-5-vinylpyrazole 17c'.

This product mixture was verified by LCMS.

For MS-ESI, the calculated value was $[M+H]^+$192.1, and the measurement was 192.1.

Step 3

Under the protection of nitrogen at room temperature, the mixture (232.6 mg, 1.22 mmol) of 1-(2-pyrrolidin-1-ethyl)-3-vinylpyrazole 17c and 1-(2-pyrrolidin-1-ethyl)-5-vinylpyrazole 17c', triethylamine (307.7 mg, 3.04 mmol), $Pd_2(dba)_3$ (464 mg, 506.73 μmol) and P(o-tol)3 (308.5 mg, 1.01 mmol) were added to the solution of 2-(3-iodo-1-tetrahydropyran-2-yl-indazol-6-yl)thio-N-methyl-benzamide 1h (500 mg, 1.01 mmol) in 1,4-dioxane (10 mL); the solution was heated to 100 °C and stirred to react for about 4 h. After the thin-layer chromatography (dichloromethane/methanol = 10/1) showed the complete reaction of the starting materials, the mixture was poured into water (30 mL) and filtered; the filtrate was extracted with ethyl acetate (20 mL × 3); and the organic layers were combined, washed with saturated brine (30 mL), dried over anhydrous sodium sulfate and concentrated to obtain a crude product. The crude product was purified by preparative thin-layer chromatography (dichloromethane/methanol = 10/1) to obtain a yellow solid mixture (250 mg, 449.06 μmol, yield: 44.31 %) of N-methyl-2-[3-[(E)-2-[1-(2-pyrrolidin-1-ylethyl)pyrazole -3-yl]vinyl]-1-tetrahydropyran-2-yl-indazol-6-yl]thiobenzamide 17d and N-methyl-2-[3-[(E)-2-[2-(2-pyrrolidin-1-ylethyl)pyrazole -3-yl]vinyl]-1-tetrahydropyran-2-yl-indazol-6-yl]thiobenzamide 17d'.

This product was verified by LCMS.

Step 4

At room temperature, trifluoroacetic acid (256 mg, 2.25 mmol) was added to the solution of the mixture (250 mg, 449.06 μmol) of N-methyl-2-[3-[(E)-2-[1-(2-pyrrolidin-1-ylethyl)pyrazol-3-yl]vinyl]-1-tetrahydropyran-2-yl-indazol-6-yl]thiobenzamide 17d and N-methyl-2-[3-[(E)-2-[2-(2-pyrrolidin-1-ylethyl)pyrazol-3-yl]vinyl]-1-tetrahydropyran-2-yl-indazol-6-yl]thiobenzamide 17d' in dichloromethane (5 mL). The mixture was stirred at 30°C to react for about 16 h. After the reaction of the starting materials completed, as indicated by thin-layer chromatography (dichloromethane/methanol = 10/1), the mixture was poured into water (40 mL), adjusted with sodium carbonate to pH=8~9, and extracted with dichloromethane (20 mL × 3); and the combined organic layers were washed with saturated brine (30 mL), dried over anhydrous sodium sulfate and concentrated to obtain a crude product. The crude product was purified by preparative thin-layer chromatography to obtain a yellow solid, N-methyl-2-({3-[(E)-2-{1-[2-(pyrrolidin-1-yl)ethyl]-1H-pyrazole -3-yl}vinyl]-1H-indazol-6-yl}thio)benzamide I-13 (4 mg, 8.47 μmol, yield: 1.88 %) and a yellow solid, N-methyl-2-({3-[(E)-2-{1-[2-(pyrrolidin-1-yl)ethyl]-1H-pyrazole -5-yl}vinyl]-1H-indazol-6-yl}thio)benzamide I-25 (7.4 mg, 15.68 μmol, yield: 3.48 %), respectively.

The obtained two products were each verified by LCMS, HNMR and CNMR.

N-methyl-2-({3-[(E)-2-{1-[2-(pyrrolidin-1-yl)ethyl]-1H-pyrazole -3-yl}vinyl]-1H-indazol-6-yl}thio)benzamide I-13: for MS-ESI, the calculated value was $[M+H]^+$473.2, and the measurement was 473.2.

$^1$H NMR (400MHz, CD3OD): δ (ppm) δ 8.02 (d, J = 8.8 Hz, 1H), 7.75 (d, J = 2.0 Hz, 1H), 7.59 (s, 1H), 7.50 - 7.46 (m, 3H), 7.37 - 7.31 (m, 2H), 7.25-7.20 (m, 2H), 6.73 (s, 1H), 4.59 (t, J = 5.6 Hz, 2H), 3.79-3.73 (m, 4H), 3.15 (s, 2H), 2.88 (s, 3H), 2.17 (s, 2H), 2.06 (s, 2H).

$^{13}$C NMR (100 MHz, CD3OD): δ (ppm) 170.33, 151.68, 142.74, 142.14, 137.64, 134.90, 134.17, 132.05, 131.46, 130.26, 127.68, 126.65, 125.06, 122.45, 121.15, 121.05, 119.93, 113.79, 103.49, 54.34, 54.29, 25.33, 22.57. N-methyl-2-({3-[(E)-2-{1-[2-(pyrrolidin-1-yl)ethyl]-1H-pyrazole -5-yl}vinyl]-1H-indazol-6-yl}thio)benzamide I-25: for MS-ESI, the calculated value was $[M+H]^+$473.2, and the measurement was 473.1.

$^1$H NMR (400MHz, CD3OD): δ (ppm) δ 8.07 (d, J = 8.4 Hz, 1H), 7.61 - 7.59 (m, 2H), 7.51 - 7.49 (m, 3H), 7.37-7.34 (m, 2H), 7.25-7.21 (m, 2H), 6.81 (d, J = 2.0 Hz, 1H), 4.72 (t, J = 5.6 Hz, 2H), 3.78 (t, J = 5.6 Hz, 4H), 3.15 (s, 2H), 2.88 (s, 3H), 2.17 (s, 1H), 2.05 (s, 1H)

$^{13}$C NMR (100 MHz, CD3OD): δ (ppm) 168.95, 163.13, 152.66, 138.96, 135.01, 131.37, 129.90, 126.84, 123.37, 120.89, 115.89, 114.33, 110.36, 64.19, 55.05, 54.61, 26.88, 23.47.

I-18

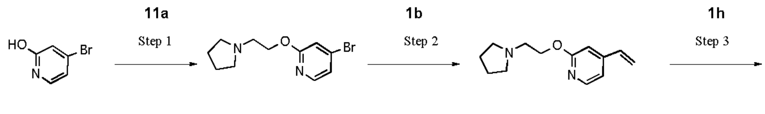


18a 18b 18c

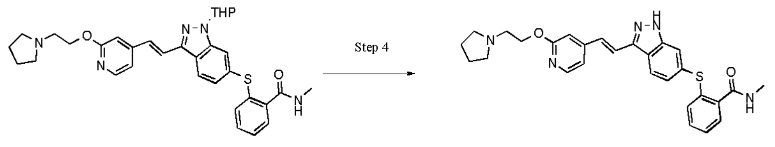


18d I-18

Step 1

Under the protection of nitrogen at room temperature, 1-(2-chloroethyl)pyrrolidine hydrochloride 11a (3.1 g, 22.99 mmol), silver carbonate (6.3 g, 22.99 mmol) and NaI(241.2 mg, 1.61 mmol) were added to the solution of 4-bromopyridin-2-ol 18a (2 g, 13.61 mmol) in DMSO (20 mL); the solution was heated to 85 °C and stirred to react for about 16 h. After the thin-layer chromatography (dichloromethane/methanol = 10/1) showed the complete reaction of the starting materials, the mixture was poured into water (100 mL) and extracted with dichloromethane (40 mL × 3), and the organic layers were combined, washed with saturated brine (100 mL), dried over anhydrous sodium sulfate and concentrated to obtain a crude product. The crude product was purified by preparative thin-layer chromatography (dichloromethane/-methanol = 10/1) to obtain a yellow solid, 4-bromo-2-(2-pyrrolidin-1-methoxy)pyridine 18b (765 mg, 2.82 mmol, yield: 24.54 %).

This product was verified by LCMS and HNMR.

For MS-ESI, the calculated value was $[M+H]^+$272.9, and the measurement was 273.1.

$^1$H NMR (400 MHz, $CDCl_3$) δ 7.95 (d, J = 5.2 Hz, 1H), 7.00 (d, J = 5.6 Hz, 1H), 6.81 (s, 1H), 4.42 (t, J = 6.0 Hz, 1H), 2.86 (t, J = 6.0 Hz, 2H), 2.59 (d, J = 6.0 Hz, 4H), 1.77-1.81 (m, 4H).

Step 2

Under the protection of nitrogen at room temperature, potassium vinyltrifluoroborate 1b (148.2 mg, 1.11 mmol), $Pd(dppf)Cl_2$ (16.2 mg, 22.13 μmol) and triethylamine (112 mg, 1.11 mmol) were added to the solution of 4-bromo-2-(2-pyrrolidin-1-methoxy)pyridine 18b (200 mg, 737.59 μmol) in N,N-dimethylformamide (3 mL); the solution was heated to 85 °C and stirred to react for about 4 h. After the thin-layer chromatography (dichloromethane/methanol = 10/1) showed the complete reaction of the starting materials, the mixture was poured into water (50 mL) and extracted with dichloromethane (50 mL × 3), and the organic layers were combined, washed with saturated brine (50 mL), dried over anhydrous sodium sulfate and concentrated to obtain a crude product. The crude product was purified by preparative thin-layer chromatography (dichloromethane/methanol = 10/1) to obtain a yellow solid, 2-(2-pyrrolidin-1-ethoxy)-4-vinylpyridine 18c (10 mg, 45.81 μmol, yield: 6.21 %).

This product was verified by LCMS.

For MS-ESI, the calculated value was $[M+H]^+$219.1, and the measurement was 219.1.

Step 3

Under the protection of nitrogen at room temperature, 2-(2-pyrrolidin-1-ethoxy)-4-vinylpyridine 18c (58.4 mg, 267.55 μmol), triethylamine (33.8 mg, 334.44 μmol), $Pd_2(dba)_3$ (67.9 mg, 222.96 μmol) and P(o-tol)3 (306.3 mg, 334.44 μmol) were added to the solution of 2-(3-iodo-1-tetrahydropyran-2-yl-indazol-6-yl)thio-N-methyl-benzamide 1h (110 mg, 222.96 μmol) in 1,4-dioxane (2 mL); the solution was heated to 100 °C and stirred to react for about 2 h. After the thin-layer chromatography (dichloromethane/methanol = 10/1) showed the complete reaction of the starting materials, the mixture was poured into water (30 mL) and extracted with ethyl acetate (15 mL × 3), and the organic layers were combined, washed with saturated brine (40 mL), dried over anhydrous sodium sulfate and concentrated to obtain a crude product. The crude product was purified by preparative thin-layer chromatography (dichloromethane/methanol = 10/1) to obtain a yellow solid, N-methyl-2-[3-[(E)-2-[2-(2-pyrrolidin-1-methoxy)-4-pyridyl]vinyl]-1-tetrahydropyran-2-yl-indazol-6-yl]thiobenzamide 18d (37 mg, 63.38 μmol, yield: 28.43 %).

This product was verified by LCMS and HNMR.

For MS-ESI, the calculated value was $[M+H]^+$584.3, and the measurement was 584.3.

$^1$H NMR (400 MHz, $CDCl_3$) δ (ppm) δ 8.48 (d, J = 8.4 Hz, 0H), 7.62 - 7.77 (m, 3H), 7.41 (s, 1H), 7.27-7.28 (m, 3H), 7.20 - 7.23 (m, 3H), 6.29 (s, 1H), 5.75 (d, J = 6.8 Hz, 1H), 4.00 (d, J = 12.0 Hz, 1H), 3.73 (d, J = 8.8 Hz, 1H), 3.21 (s, 1H), 2.96 (d, J = 4.8 Hz, 3H), 2.66 - 2.70 (m, 2H), 2.20-2.49 (m, 4H), 1.58 - 1.73 (m, 8H), 1.22 (d, J = 24.4 Hz, 4H).

Step 4

At room temperature, trifluoroacetic acid (78.1 mg, 685.23 μmol) was added to the solution of N-methyl-2-[3-[(E)-2-[2-(2-pyrrolidin-1-methoxy)-4-pyridyl]vinyl]-1-tetrahydropyran-2-yl-indazol-6-yl]thiobenzamide 18d (400 mg, 685.23 μmol) in dichloromethane (10 mL). The mixture was stirred at 40°C to react for about 16 h. After the reaction of the starting materials completed, as indicated by thin-layer chromatography (dichloromethane/methanol = 10/1), the mixture was poured into water (40 mL), adjusted with sodium carbonate to pH=8~9, and extracted with dichloromethane (20 mL × 3); and the combined organic layers were washed with saturated brine (30 mL), dried over anhydrous sodium sulfate and concentrated to obtain a crude product. The crude product was purified by preparative thin-layer chromatography to obtain a yellow solid, N-methyl-2-({3-[(E)-2-{2-[2-(pyrrolidin-1-yl)ethoxy]pyridin-4-yl}vinyl]-1H-indazol-6-yl}thio)benzamide I-18 (8 mg, 16.01 μmol, yield: 2.34 %).

This product was verified by LCMS, HNMR and CNMR.

For MS-ESI, the calculated value was $[M+H]^+$500.2, and the measurement was 500.2.

$^1$H NMR (400 MHz, $CD_3OD$&$CDCl_3$) δ 8.01 (d, J = 8.6 Hz, 1H), 7.58 (dd, J = 15.6, 8.4 Hz, 3H), 7.46-7.50 (m, 1H), 7.26-7.33 (m, 3H), 7.23 (d, J = 8.6 Hz, 1H), 7.18 (d, J = 8.0 Hz, 1H), 6.74 (d, J = 7.2 Hz, 1H), 6.62 (s, 1H), 4.13 (t, J = 6.8 Hz, 2H), 2.82 - 2.91 (m, 5H), 2.69 (s, 4H), 1.84 (s, 4H).

$^{13}$C NMR (100 MHz, $CD_3OD$&$CDCl_3$) δ 170.27, 163.68, 149.42, 138.06, 136.98, 135.33, 133.98, 131.20, 130.40, 127.73, 127.12, 126.49, 125.82, 120.77, 116.77, 114.49,114.29, 104.30, 99.99, 54.57,54.02, 54.02,53.85, 29.43, 25.81, 23.29,22.96.

I-30

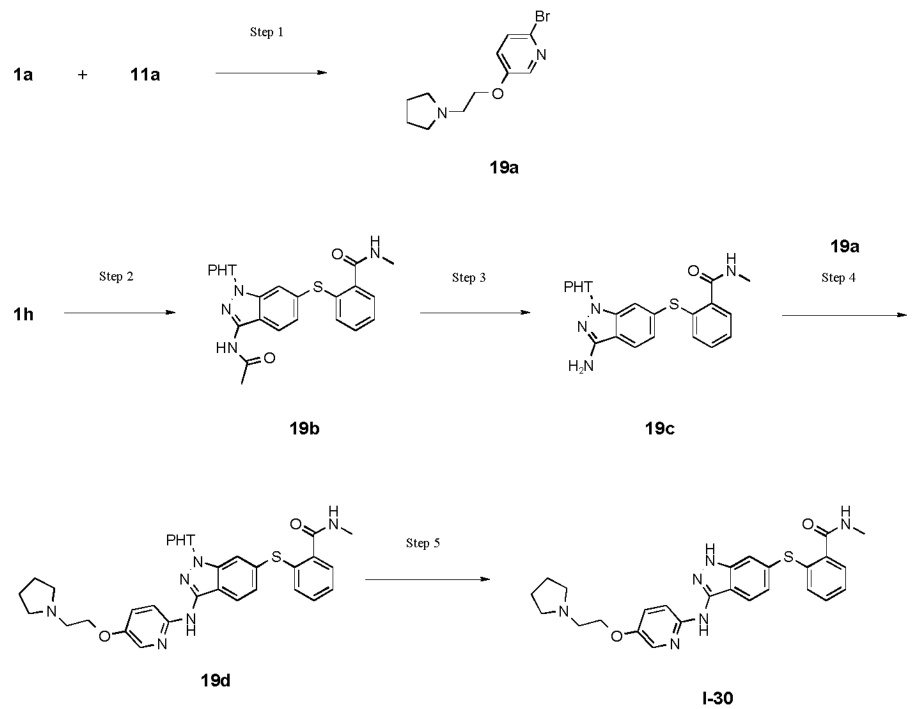


Step 1

Under the protection of nitrogen at 0°C, sodium hydride (253 mg, 6.32 mmol, purity: 60 %) was added to the solution of 6-bromopyridin-3-ol 1a (1 g, 5.75 mmol) in *N,N*-dimethylformamide (20 mL) in portions and stirred to react for about 1 h; 1-(2-chloroethyl)pyrrolidine hydrochloride 11a (1.08 g, 6.32 mmol) was slowly added at low temperature; and the mixture was stirred at low temperature to react for 1 h, heated to 50°C, and then stirred to react for about 3 h. After the thin-layer chromatography (petroleum ether/ethyl acetate = 2/1) showed the complete reaction of the starting materials, the reaction mixture was cooled to 0°C, and quenched with water (about 10 mL); then, the mixture was poured into water (100

mL) and extracted with ethyl acetate (50 mL × 3); and the organic layers were combined, washed with saturated brine (100 mL), dried over anhydrous sodium sulfate and concentrated to obtain a crude product, which was a yellow oily liquid 2-bromo-5-(2-pyrrolidin-1-methoxy)pyridine 19a (1.5 g, yield: 96.3 %).

This product was verified by LCMS and HNMR.

For MS-ESI, the calculated value was $[M+H]^+$272.9, and the measurement was 273.1.

$^1$H NMR (400MHz, $CDCl_3$) δ (ppm) 8.09 (d, 1H, J = 3.2Hz), 7.38 (d, 1H, J = 8.8Hz), 7.16 (dd, 1H, J = 8.8, 2.8Hz), 4.21 (t, 2H, J = 5.6Hz), 3.01 (t, 2H, J = 5.6Hz), 2.75 (s, 4H), 1.87-1.90 (m, 4H).

Step 2

Under the protection of nitrogen at room temperature, acetamide (71.83 mg, 1.22 mmol), (1R,2R)-N1,N2-dimethylcyclohexan-1,2-diamine (86.49 mg, 608.08 μmol), cuprous iodide (115.81 mg, 608.08 μmol) and sodium tert-butoxide (116.88 mg, 1.22 mmol) were added to the solution of 2-(3-iodo-1-tetrahydropyran-2-yl-indazol-6-yl)thio-N-methyl-benzamide 1h (300 mg, 608.08 μmol) in 1,4-dioxane (9 mL); the solution was heated to 110 °C and stirred to react for about 6 h. After the thin-layer chromatography (ethyl acetate) showed the complete reaction of the starting materials, the mixture was poured into water (30 mL) and extracted with ethyl acetate (20 mL × 3), and the organic layers were combined, washed with saturated brine (30 mL), dried over anhydrous sodium sulfate and concentrated to obtain a crude product. The crude product was purified by preparative thin-layer chromatography (ethyl acetate) to obtain a white solid, 2-(3-acetamido-1-tetrahydropyran-2-yl-indazol-6-yl)thio-*N*-methyl-benzamide 19b (0.17 g, 400.46 μmol, yield: 65.86 %).

This product was verified by LCMS.

For MS-ESI, the calculated value was $[M+H]^+$425.2, and the measurement was 425.0.

Step 3

At room temperature, NaOH (376.90 mg, 9.42 mmol) and $H_2O$ (2 mL) were added to the solution of 2-(3-acetamido-1-tetrahydropyran-2-yl-indazol-6-yl)thio-N-methyl-benzamide 19b (0.4 g, 942.25 μmol) in 1,4-dioxane (10 mL); the solution was heated to 100 °C and stirred to react for about 16 h. After the reaction of the starting materials completed, as indicated by thin-layer chromatography (dichloromethane/methanol = 20/1), the mixture was poured into water (40 mL) and extracted with ethyl acetate (20 mL × 3); and the combined organic layers were washed with saturated brine (20 mL), dried over anhydrous sodium sulfate and concentrated to obtain a crude product. The crude product was purified by preparative thin-layer chromatography (dichloromethane/methanol = 20/1, 0.1 % $NH_3 \cdot H_2O$) to obtain a white solid, 2-(3-amino-1-tetrahydropyran-2-yl-indazol-6-yl)thio-N-methyl-benzamide 19c (0.152 g, 397.41 μmol, yield: 42.18 %).

This product was verified by LCMS.

For MS-ESI, the calculated value was $[M+H]^+$383.1, and the measurement was 383.1.

Step 4

Under the protection of nitrogen at room temperature, XantPhos (378.20 mg, 653.63 μmol), $Pd_2(dba)_3$ (97.72 mg, 169.94 μmol), 2-bromo-5-(2-pyrrolidin-1-methoxy)pyridine 19a (124.8 mg, 608.08 μmol), cesium carbonate (1.60 g, 4.92 mmol) were added to the solution of 2-(3-amino-1-tetrahydropyran-2-yl-indazol-6-yl)thio-N-methyl-benzamide 19c (0.1 g, 261.45 μmol) in 1,4-dioxane (5 mL); the solution was heated to 100 °C and stirred to react for about 6 h. After the thin-layer chromatography (dichloromethane/methanol = 10/1) showed the complete reaction of the starting materials, the mixture was poured into water (30 mL) and extracted with ethyl acetate (10 mL × 3), and the organic layers were combined, washed with saturated brine (20 mL), dried over anhydrous sodium sulfate and concentrated to obtain a crude product, which was a yellow oily liquid. The crude product was purified by preparative thin-layer chromatography (dichloromethane/methanol = 10/1) to obtain a yellow solid, N-methyl-2-[3-[[5-(2-pyrrolidin-1-methoxy)-2-pyridyl]atnino]-1-tetrahydropyran-2-yl-indazol-6-yl]thiobenzamide 19d (0.1 g, 174.61 μmol, yield: 66.78 %).

This product was verified by LCMS.

For MS-ESI, the calculated value was $[M+H]^+$573.3, and the measurement was 573.1.

Step 5

At low temperature, HCl/MeOH (10 mL) was added to N-methyl-2-[3-[[5-(2-pyrrolidin-1-methoxy)-2-pyridyl] amino]-1-tetrahydropyran-2-yl-indazol-6-yl]thiobenzamide 19d (86 mg, 150.16 μmol), and stirred at room temperature to react for about 4 h. After the thin-layer chromatography (dichloromethane/methanol = 10/1) showed the complete reaction of the starting materials, the mixture was concentrated, and then poured into water (10 mL); the aqueous phase was adjusted with sodium bicarbonate to neutral pH and extracted with ethyl acetate (10 mL × 3); and the organic layers were

combined, washed with saturated brine (20 mL), dried over anhydrous sodium sulfate and concentrated to obtain a crude product. The crude product was purified by preparative thin-layer chromatography (dichloromethane/methanol = 10/1, 0.1 % $NH_3 \cdot H_2O$) to obtain a pale-yellow solid, N-methyl-2-{[3-({5-[2-(pyrrolidin-1-yl)ethoxy]pyridin-2-yl}amino)-1H-indazol-6-yl] thio}benzamide I-30 (20.3 mg, 41.55 μmol, yield: 27.67 %).

This product was verified by LCMS, HNMR and CNMR.

For MS-ESI, the calculated value was $[M+H]^+$489.2, and the measurement was 489.1.

$^1$H NMR (400MHz, DMSO-d6): δ 12.18 (s,1H), 9.46 (s,1H), 8.37-8.38 (m,1H), 8.07 (d, 1H, J=8.4Hz), 7.91-7.95 (m, 2H), 7.49 (d, 1H, J=7.6Hz), 7.38-7.42 (m, 2H), 7.24-7.32 (m, 2H), 6.96-7.02 (m, 2H), 4.07-4.09 (m, 2H), 2.77-2.79 (m, 6H), 2.51-2.52 (m, 3H), 1.68 (s, 4H).

$^{13}$C NMR (100MHz, DMSO-d6): δ 168.36, 149.76, 149.30, 144.53, 141.58, 137.35, 136.32, 134.90, 133.12, 130.69, 130.35, 128.19, 126.48, 125.33, 123.37, 122.35, 114.54, 114.31, 110.61, 68.14, 54.88, 54.47, 26.56, 26.43, 23.62.

I-31

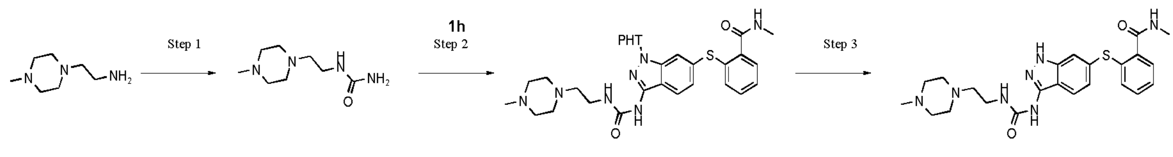


20a 20b 20c I-31

Step 1

At room temperature, acetic acid (55 mg, 915.88 μmol) and KOCN (2.08 g, 24.44 mmol) were added to the mixture of 2-(4-methylpiperazin-1-yl)ethylamine 20a (5 g, 34.91 mmol) and water (50 mL), and stirred at 25 °C to react for about 2 h. After the thin-layer chromatography (dichloromethane/methanol = 10/ 1) showed the complete reaction of the starting materials, these materials were directly lyophilized to obtain a sufficiently pure white solid crude product 2-(4-methylpiperazin-1-yl)ethyl urea 20b (6 g, 32.21 mmol, yield: 92.28 %).

This product was verified by LCMS and HNMR.

For MS-ESI, the calculated value was $[M+H]^+$187.1, and the measurement was 187.1.

$^1$H NMR (400MHz, $CDCl_3$): δ (ppm) 5.44 (s, 1H), 4.70 (d, J = 78.3 Hz, 2H), 3.29 (m, J = 10.5, 5.1 Hz, 2H), 2.82 (t, J = 6.1 Hz, 2H), 2.31 (d, J = 1.4 Hz, 8H), 2.01 (s, 3H).

Step 2

Under the protection of nitrogen at room temperature, 2-(3-iodo-1-tetrahydropyran-2-yl-indazol-6-yl)thio-N-methyl-benzamide 1h (200 mg, 405.38 μmol), potassium tert-butoxide (136.47 mg, 1.22 mmol) and cuprous iodide (15.44 mg, 81.08 μmol) were added to the solution of 2-(4-methylpiperazin-1-yl)ethylurea 20b (60.40 mg, 324.31 μmol) in 1,4-dioxane (5 mL); the solution was heated to 105 °C and stirred to react for about 16 h. After the reaction of the starting materials completed, as indicated by thin-layer chromatography (dichloromethane/methanol = 10 /1), the mixture was poured into water (20 mL) and extracted with dichloromethane (20 mL × 3); the combined organic layers were washed with saturated brine (30 mL), dried over anhydrous sodium sulfate and concentrated to obtain a crude product; and the crude product was purified by preparative thin-layer chromatography (dichloromethane/methanol = 10/1) to obtain a green oily liquid, N-methyl-2-[3-[2-(4-methylpiperazin-1-yl)ethylcarbamoylamino]-1-tetrahydropyran-2-yl-indazol-6-yl]thiobenzamide 20c (30 mg, 54.38 μmol, yield: 13.41 %).

This product was verified by LCMS.

For MS-ESI, the calculated value was $[M+H]^+$552.3, and the measurement was 552.1.

Step 3

At 0 °C, trifluoroacetic acid (18.60 mg, 163.13 μmol) was added to the solution of N-methyl-2-[3-[2-(4-methylpiperazin-1-yl)ethylcarbamoylamino]-1-tetrahydropyran-2-yl-indazol-6-yl]thiobenzamide 20c (30 mg, 54.38 μmol) in DCM (5 mL), and stirred at room temperature to react for about 16 h. After the reaction of the starting materials completed, as indicated by thin-layer chromatography (dichloromethane/methanol = 10/1), the mixture was poured into water (20 mL), adjusted with solid sodium carbonate to pH=8-9, and extracted with DCM (20 mL × 3); and the combined organic layers were washed with saturated brine (20 mL), dried over anhydrous sodium sulfate and concentrated to obtain a crude product. The crude product was purified by preparative thin-layer chromatography to obtain a white solid, N-methyl-2-{[3-{[2-(4-methylpiperazin-1-yl)ethyl]carbamoyl}amino)-1H-indazol-6-yl]thio}benzamide I-31 (17.4 mg, 37.21

μmol, yield: 68.43 %).

This product was verified by LCMS, HNMR and CNMR.

For MS-ESI, the calculated value was $[M+H]^+$468.2, and the measurement was 468.1.

$^1$H NMR (400MHz, $CDCl_3$&CD3OD): δ (ppm)7.74 (s, 1H), 7.46 (d, J = 19.2 Hz, 3H), 7.28 - 7.24 (m, 1H), 7.17 (s, 1H), 7.04 (d, J = 8.5 Hz, 1H), 3.47 (t, J = 6.2 Hz, 2H), 2.89 (s, 3H), 2.83 - 2.35 (m, 10H), 2.31 (s, 3H).

$^{13}$C NMR (100 MHz, $CDCl_3$&$CD_3OD$) δ 170.12, 156.23, 142.10, 141.37, 131.23, 130.49, 127.83, 126.49, 123.83, 120.50, 113.71, 113.40, 57.02, 54.54, 52.32, 45.20, 37.01, 26.10.

I-11

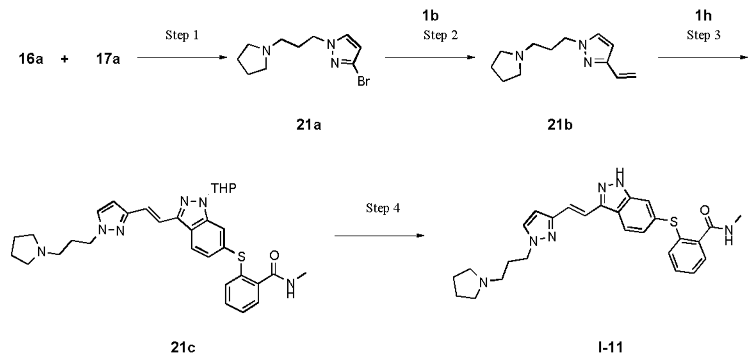


Step 1

Under the protection of nitrogen at room temperature, 16a (1 g, 6.80 mmol) and cesium carbonate (3.3 g, 10.21 mmol) were added to the solution of 3-bromo-1Hpyrazole 17a (1 g, 6.80 mmol) in DMSO (20 mL); the solution was heated to 100 °C and stirred to react for about 16 h. After the thin-layer chromatography (dichloromethane/methanol = 20/1) showed the complete reaction of the starting materials, the mixture was poured into water (40 mL) and extracted with ethyl acetate (20 mL × 3), and the organic layers were combined, washed with saturated brine (20 mL), dried over anhydrous sodium sulfate and concentrated to obtain a yellow solid 3-bromo-1-(3-pyrrolidin-1-ylpropyl)pyrazole 21a (1.6 g, 6.20 mmol, yield: 91.09 %).

This product was verified by LCMS and HNMR.

For MS-ESI, the calculated value was $[M+H]^+$259.9, and the measurement was 260.1.

$^1$H NMR (400 MHz, $CDCl_3$):δ (ppm) δ 7.31 (d, J = 2.4 Hz, 1H), 6.25 (d, J = 2.0 Hz, 1H), 4.18 (t, J = 7.2 Hz, 2H), 2.46-2.52 (m, 4H), 2.41 (t, J = 7.2 Hz, 2H), 2.03 (t, J = 7.2 Hz, 2H), 1.77-1.81 (m, 4H).

Step 2

Under the protection of nitrogen at room temperature, potassium vinyltrifluoroborate 1b (778.3 mg, 5.81 mmol), Pd(dppf)$Cl_2$ (85 mg, 116.21 μmol) and triethylamine (778.3 mg, 11.62 mmol) were added to the solution of 3-bromo-1-(3-pyrrolidin-1-ylpropyl)pyrazole 21a (1 g, 3.87 mmol) in *N,N*-dimethylformamide (10 mL); the solution was heated to 85 °C and stirred to react for about 16 h. After the thin-layer chromatography (dichloromethane/methanol = 10/1) showed the complete reaction of the starting materials, the mixture was poured into water (40 mL) and extracted with ethyl acetate (20 mL × 3); and the organic layers were combined, washed with water (40 mL) and brine (40 mL) in sequence, dried over anhydrous sodium sulfate and concentrated to obtain a crude product. The crude product was purified by preparative thin-layer chromatography to obtain a yellow oily liquid, 1-(3-pyrrolidin-1-ylpropyl)-3-vinylpyrazole 21b (290 mg, 1.41 mmol, yield: 36.47 %).

This product was verified by LCMS and HNMR.

For MS-ESI, the calculated value was $[M+H]^+$206.2, and the measurement was 206.1.

$^1$H NMR (400MHz, $CDCl_3$): δ (ppm) 7.34 (s, 1H), 6.78-6.66 (m, 1H), 6.39-6.37 (m, 1H), 5.74-5.70 (m, 1H), 5.36-5.26 (m, 1H), 4.25-4.16 (m, 2H), 2.50-2.46 (m, 4H), 2.44-2.42 (m, 2H), 2.10-1.99 (m, 2H), 1.83-1.79 (m, 4H).

Step 3

Under the protection of nitrogen at room temperature, 1-(3-pyrrolidin-1-ylpropyl)-3-vinylpyrazole 21b (187.3 mg, 912.11 μmol), triethylamine (184.6 mg, 1.82 mmol), $Pd_2(dba)_3$ (278.4 mg, 304.04 μmol) and P(o-tol)3 (185.08 mg, 608.08 μmol) were added to the solution of 2-(3-iodo-1-tetrahydropyran-2-yl-indazol-6-yl)thio-N-methyl-benzamide 1h (300 mg, 608.08 μmol) in 1,4-dioxane (10 mL); the solution was heated to 100 °C and stirred to react for about 1 h. After the thin-layer

chromatography (dichloromethane/methanol = 10/1) showed the complete reaction of the starting materials, the mixture was concentrated and filtered to obtain a crude product. The crude product was subjected to the preparative thin-layer chromatography to obtain a white solid N-methyl-2-[3-[(E)-2-[1-(3-pyrrolidin-1-ylpropyl)pyrazol-3-yl]vinyl]-1-tetrahydropyran-2-yl-indazol-6-yl]thiobenzamide 21c (10 mg, 17.52 μmol, yield: 2.88 %).

This product was verified by LCMS.

For MS-ESI, the calculated value was $[M+H]^+$571.3, and the measurement was 571.3.

Step 4

At room temperature, trifluoroacetic acid (599.3 mg, 5.26 mmol) was added to the solution of N-methyl-2-[3-[(E)-2-[1-(3-pyrrolidin-1-ylpropyl)pyrazol-3-yl]vinyl]-1-tetrahydropyran-2-yl-indazol-6-yl]thiobenzamide 21c (300 mg, 525.63 μmol) in dichloromethane (5 mL), and then stirred to react for about 16 h. After the reaction of the starting materials completed, as indicated by thin-layer chromatography (dichloromethane/methanol = 10/1), the mixture was poured into water (10 mL), and extracted with ethyl acetate (10 mL × 3); the combined organic layers were washed with saturated brine (40 mL), dried over anhydrous sodium sulfate and concentrated to obtain a crude product. The crude product was purified by preparative thin-layer chromatography to obtain a white solid, N-methyl-2-({3-[(E)-2-{1-[3-(pyrrolidin-1-yl)propyl]-1H-pyrazole -3-yl}vinyl]-1H-indazol-6-yl}thio)benzamide I-11 (16.4 mg, 33.70 μmol, yield: 6.41 %).

This product was verified by LCMS and HNMR.

For MS-ESI, the calculated value was $[M+H]^+$487.2, and the measurement was 487.2.

$^1$H NMR (400MHz, CD3OD): δ (ppm) δ 8.07-8.01 (m, 1H), 7.67-7.60 (m, 2H), 7.50 - 7.42 (m, 3H), 7.36-7.31(m, 2H), 7.22 (dd, J = 14.8, 8.4 Hz, 2H), 6.65 (d, J = 2.0, 1H), 4.24 (t, J = 6.8 Hz, 1H), 2.88 (s, 3H), 2.66-2.58 (m, 6H), 2.14-2.12 (m, 2H), 1.85-1.77 (m, 4H).

I-16

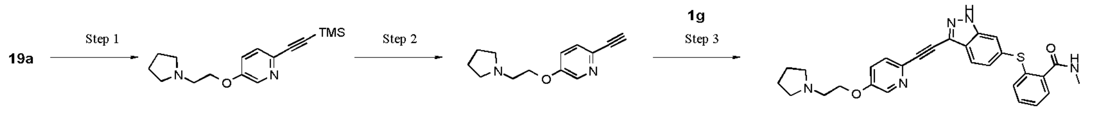


19a 22a 22b I-16

Step 1

Under the protection of nitrogen at room temperature, ethynyl(trimethyl)silane (2.17 g, 22.13 mmol, 3.13 mL), Pd(PPh3)2Cl2 (1.55 g, 2.21 mmol), cuprous iodide (421 mg, 2.21 mmol) and triethylamine (3.36 g, 33.19 mmol) were added to the solution of 2-bromo-5-(2-pyrrolidin-1-methoxy)pyridine 19a (3.0 g, 11.06 mmol) in *N,N*-dimethylformamide (60 mL); the solution was heated to 50 °C and stirred to react for 12 h. After the thin-layer chromatography (dichloromethane/methanol = 10/1) showed the complete reaction of the starting materials, the reaction mixture was poured into water (200 mL) and extracted with ethyl acetate (100 mL × 3), and the organic layers were combined, washed with water (100 mL × 3) and saturated brine (80 mL) in sequence, dried over anhydrous sodium sulfate and concentrated to obtain a crude product. The crude product was purified by silica gel column chromatography (dichloromethane/methanol = 100/1~20/1) to obtain a black oily liquid, trimethyl-[2-[5-(2-pyrrolidin-1-methoxy)-2-pyridyl]ethynyl]silane 22a (1.8 g, yield: 56.4 %).

This product was verified by LCMS and HNMR.

For MS-ESI, the calculated value was $[M+H]^+$ 289.2, and the measurement was 289.2.

$^1$H NMR (400 MHz, $CDCl_3$): δ (ppm) 7.28 (d, J = 3.2 Hz, 1H), 7.40 (d, J = 8.8 Hz,1H), 7.13-7.17 (m, 1H), 4.16 (t, J = 6.0 Hz, 2H), 2.89-2.97 (m, 2H), 2.62-2.66 (m, 4H), 1.81-1.84 (m, 4H), 0.26 (s, 9H).

Step 2

At room temperature, potassium carbonate (1.03 g, 7.49 mmol) was added to the solution of trimethyl-[2-[5-(2-pyrrolidin-1-methoxy)-2-pyridyl]ethynyl]silane 22a (1.8 g, 6.24 mmol) in methanol (30 mL), and then stirred to react for about 2 h. After the reaction of the starting materials completed, as indicated by thin-layer chromatography (dichloromethane/methanol = 10/1), the mixture was concentrated directly to obtain a crude product. The crude product was purified by preparative thin-layer chromatography (dichloromethane/methanol = 10/1) to obtain a brownish-yellow oily liquid, 2-ethynyl-5-(2-pyrrolidin-1-ethoxy)pyridine 22b (0.6 g, yield: 44.46 %).

This product was verified by LCMS and HNMR.

For MS-ESI, the calculated value was $[M+H]^+$217.1, and the measurement was 217.2.

$^1$H NMR (400 MHz, $CDCl_3$): δ (ppm) 8.29 (d, J = 2.8 Hz,1H), 7.42 (d, J = 8.4 Hz,1H), 7.15-7.18 (m, 1H), 4.17 (t, J =

5.6 Hz, 2H), 3.08 (s, 1H), 2.93 (t, J = 5.6 Hz, 2H), 2.62-2.65 (m, 4H), 1.80-1.84 (m, 4H).

Step 3

Under the protection of nitrogen at room temperature, 2-ethynyl-5-(2-pyrrolidin-1-ethoxy)pyridine 22b (159 mg, 0.73 mmol), dichlorobis(triphenylphosphin)palladium (10 mg, 0.01 mmol), cuprous iodide (28 mg, 0.15 mmol), triethylamine (184.6 mg, 1.82 mmol) were added to the solution of 2-[(3-iodo-1H-indazol-6-yl)thio]-N-methylbenzamide 1 g (300 mg, 0.73 mmol) in N,N-dimethylformamide (9 mL); the solution was heated to 100 °C and stirred to react for about 2 h. After the thin-layer chromatography (dichloromethane/methanol = 10/1) showed the complete reaction of the starting materials, the mixture was poured into water (50 mL) and extracted with ethyl acetate (20 mL × 3), and the organic layers were combined, washed with water (30 mL × 3) and saturated brine (30 mL) in sequence, dried over anhydrous sodium sulfate and concentrated to obtain a crude product. The crude product was purified by preparative thin-layer chromatography to obtain a white solid, N-methyl-2-{[3-(2-{5-[2-(pyrrolidin-1-yl)ethoxy]pyridin-2-yl}ethynyl)-1H-indazol-6-yl]thio}benzamide I-16 (15.8 mg, yield: 4.3 %).

This product was verified by LCMS, HNMR and CNMR.

For MS-ESI, the calculated value was $[M+H]^+$498.2, and the measurement was 498.1.

$^1$H NMR (400 MHz, $CDCl_3$) δ: 11.14 (br s, 1H), 8.39 (d, J=2.4Hz, 1H), 7.88 (d, J=8.4Hz, 1H), 7.59-7.64 (m, 3H), 7.30-7.31 (m, 2H), 7.19-7.27 (m, 3H), 6.37 (d, J=4.4Hz, 1H), 4.22 (t, J =6.0 Hz, 2H), 2.96-2.99 (m, 5H), 2.68 (s, 4H), 1.85 (s, 4H).

$^{13}$C NMR (100 MHz, DMSO-d6) δ: 168.30, 155.10, 141.06, 139.36, 137.86, 135.52, 134.19, 134.12, 130.92, 130.80, 128.78, 128.29, 128.02, 126.91, 126.36, 123.99, 121.72, 121.07, 115.10, 92.63, 79.46, 67.92, 54.57, 54.44, 26.56, 23.64.

I-20

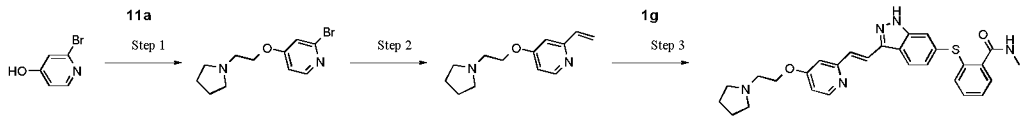


23a 23b 23c I-20

Step 1

Under the protection of nitrogen at low temperature, sodium hydride (92 mg, 2.30 mmol) was added to the solution of 2-bromopyridin-4-ol 23a (200 mg, 1.15 mmol) in N,N-dimethylformamide (4 mL) in portions, heated to 70 °C, stirred to react for about 0.5 h, and then cooled to room temperature, followed by the addition of 1-(2-chloroethyl)pyrrolidine hydrochloride 11a (195 mg, 1.15 mmol), and the mixture was heated to 70°C and stirred to react for about 16 h. After the thin-layer chromatography (dichloromethane/methanol = 20/1) showed the complete reaction of the starting materials, the mixture was poured into water (10 mL) and extracted with ethyl acetate (20 mL × 3), and the organic layers were combined, washed with saturated brine (20 mL), dried over anhydrous sodium sulfate and concentrated to obtain a yellow oily liquid 2-bromo-4-(2-pyrrolidin-1-methoxy)pyridine 23b (200 mg, yield: 64.2 %).

This product was verified by LCMS and HNMR.

For MS-ESI, the calculated value was $[M+H]^+$272.9, and the measurement was 272.9.

$^1$H NMR (400 MHz, $CDCl_3$) δ: 8.18 (d, 1H, J=6.0Hz), 7.04 (d, 1H, J=2.4Hz), 6.81-6.83 (m, 1H), 4.16-4.19 (m, 2H), 2.93-2.96 (m, 2H), 2.64-2.68 (m, 4H), 1.83-1.86 (m, 4H).

Step 2

Under the protection of nitrogen at room temperature, potassium vinyltrifluoroborate 1b (370.50 mg, 2.77 mmol), Pd(dppf)$Cl_2$ (50 mg, 0.068 mmol) and triethylamine (280 mg, 2.77 mmol) were added to the solution of 2-bromo-4-(2-pyrrolidin-1-methoxy)pyridine 23b (500 mg, 1.84 mmol) in N,N-dimethylformamide (5 mL); the solution was heated to 85 °C and stirred to react for about 16 h. After the reaction of the starting materials completed, as indicated by thin-layer chromatography (dichloromethane/methanol = 15/1), the mixture was poured into water (20 mL) and extracted with ethyl acetate (10 mL × 3); and the combined organic layers were washed with saturated brine (20 mL), dried over anhydrous sodium sulfate and concentrated to obtain a crude product. The crude product was purified by preparative thin-layer chromatography to obtain a white solid, 4-(2-pyrrolidin-1-ethoxy)-2-vinylpyridine 23c (50 mg, yield: 12.4 %).

This product was verified by LCMS and HNMR.

For MS-ESI, the calculated value was $[M+H]^+$219.1, and the measurement was 219.1.

$^1H$ NMR (400 MHz, $CDCl_3$) δ: 8.38 (d, 1H, J=5.6Hz), 6.88 (d, 1H, J=2.0Hz), 6.71-6.79 (m, 2H), 6.17 (d, 1H, J=17.2Hz), 5.47 (d, 1H, J=10.8Hz), 4.16-4.19 (m, 2H), 2.92-2.95 (m, 2H), 2.65 (s, 4H), 1.83 (s, 4H).

Step 3

Under the protection of nitrogen at room temperature, 4-(2-pyrrolidin-1-ethoxy)-2-vinylpyridine 23c (100 mg, 0.46 mmol), DIEA (592 mg, 4.58 mmol), Pd(OAc)2 (100 mg, 0.45 mmol) and P(o-tol)3 (139 mg, 0.46 mmol) were added to the solution of 2-[(3-iodo-1H-indazol-6-yl)thio]-N-methylbenzamide 1 g (562 mg, 1.37 mmol) in *N,N*-dimethylformamide (5 mL); the solution was heated to 100 °C and stirred to react for about 16 h. After the reaction of the starting materials completed, as indicated by thin-layer chromatography (dichloromethane/methanol = 10/1), the mixture was poured into water (10 mL), and extracted with ethyl acetate (10 mL × 3); the combined organic layers were washed with saturated brine (40 mL), dried over anhydrous sodium sulfate and concentrated to obtain a crude product. The crude product was first subjected to the preparative thin-layer chromatography and then purified by the preparative thin-layer chromatography (dichloromethane/methanol = 10/1) to obtain a white solid N-methyl-2-({3-[(E)-2-{4-[2-(pyrrolidin-1-yl)ethoxy]pyridin-2-yl} vinyl]-1H-indazol-6-yl}thio)benzamide I-20 (5 mg, yield: 2.18 %).
This product was verified by LCMS, HNMR and CNMR.
For MS-ESI, the calculated value was $[M+H]^+$500.2, and the measurement was 500.1.
$^1H$ NMR (400 MHz, CD3OD) δ: 8.38 (d, 1H, J= 6.0Hz), 8.09 (d, 1H, J=8.4Hz), 7.88 (d, 1H, J=16.4Hz), 7.62 (s, 1H), 7.49-7.56 (m, 2H), 7.31-7.36 (m, 3H), 7.23-7.26 (m, 2H), 6.94 (d, 1H, J= 4.0Hz), 4.34-4.37 (m, 2H), 3.13-3.14 (m, 2H), 2.86-2.88 (m, 7H), 1.92 (s, 4H).
$^{13}C$ NMR (100 MHz, CD3OD) δ: 170.38, 165.97, 157.09, 150.09, 142.29, 142.18, 137.59, 134.91, 134.16, 131.46, 130.31, 129.88, 129.25, 127.68, 127.45, 126.68, 125.36, 124.12, 121.14, 120.27, 113.92, 109.19, 108.12, 79.98, 66.15, 54.18, 54.11, 38.16, 29.37, 29.34, 27.39, 27.29, 25.38, 22.82, 20.23.

I-6

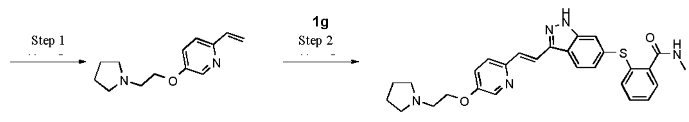


19a + 1b

24a

I-6

Step 1

Under the protection of nitrogen at room temperature, potassium trifluorovinyl borate 1b (371 mg, 2.77 mmol), $Pd(dppf)Cl_2$ (40 mg, 0.055 mmol) and triethylamine (280 mg, 2.77 mmol) were added to the solution of 2-bromo-5-(2-pyrrolidin-1-methoxy)pyridine 19a (0.5 g, 1.84 mmol) in N,N-dimethylformamide (10 mL); the solution was heated to 85 °C and stirred to react for about 16 h. After the thin-layer chromatography (dichloromethane/methanol = 15/1) showed the complete reaction of the starting materials, the mixture was poured into water (40 mL) and extracted with ethyl acetate (10 mL × 3), and the organic layers were combined, washed with water (30 mL) and saturated brine (30 mL) in sequence, dried over anhydrous sodium sulfate and concentrated to obtain a crude product. The crude product was purified by preparative thin-layer chromatography (dichloromethane/methanol = 10/1) to obtain a brownish-yellow oily liquid, 5-(2-pyrrolidin-1-ethoxy)-2-vinylpyridine 24a (220 mg, yield: 54.7 %).
This product was verified by LCMS and HNMR.
For MS-ESI, the calculated value was $[M+H]^+$219.1, and the measurement was 219.1.
$^1H$ NMR (400 MHz, $CDCl_3$) δ: 8.29 (d, 1H, J=2.8Hz), 7.29 (d, 1H, J=1.2Hz), 7.20 (dd, 1H, J=8.4, 2.8Hz), 6.77 (dd, 1H, J=17.6, 10.8Hz), 6.03 (dd, 1H, J=17.6, 0.8Hz), 5.37 (dd, 1H, J=10.8, 1.2Hz), 4.21 (t, 2H, J=6.0Hz), 2.99 (t, 2H, J=5.6Hz), 2.73 (s, 4H), 1.85-1.88 (m, 4H).

Step 2

Under the protection of nitrogen at room temperature, 5-(2-pyrrolidin-1-ethoxy)-2-vinylpyridine 24a (133 mg, 0.61 mmol), DIEA (157 mg, 1.22 mmol), Pd(OAc)2 (137 mg, 0.61 mmol) and P(o-tol)3 (123 mg, 0.41 mmol) were added to the solution of 2-[(3-iodo-1H-indazol-6-yl)thio]-N-methylbenzamide 1 g (166 mg, 0.41 mmol) in *N,N*-dimethylformamide (10 mL); the solution was heated to 100 °C and stirred to react for about 16 h. After the reaction of the starting materials completed, as indicated by thin-layer chromatography (dichloromethane/methanol = 20/1), the mixture was poured into water (40 mL), and extracted with ethyl acetate (20 mL × 3); the combined organic layers were washed with saturated brine (40 mL), dried over anhydrous sodium sulfate and concentrated to obtain a crude product. The crude product was first

subjected to the preparative thin-layer chromatography and then purified by the preparative thin-layer chromatography (dichloromethane/methanol = 10/1) three times to finally obtain a yellow solid N-methyl-2-({3-[(E)-2-{5-[2-(pyrrolidin-1-yl) ethoxy]pyridin-2-yl}vinyl]-1H-indazol-6-yl}thio)benzamide I-6 (4.5 mg, yield: 2.2 %).

This product was verified by LCMS, HNMR and CNMR.

For MS-ESI, the calculated value was $[M+H]^+$500.2, and the measurement was 500.2.

$^1$H NMR (400 MHz, CD3OD) δ: 8.28 (d, 1H, J=2.8Hz), 8.06 (d, 1H, J=8.4Hz), 7.71 (d, 1H, J=16.4Hz), 7.65 (d, 1H, J=8.8Hz), 7.60 (s, 1H), 7.48-7.55 (m, 2H), 7.45 (dd, 1H, J=8.8, 2.8Hz), 7.30-7.37 (m, 2H), 7.20-7.25 (m, 2H), 4.25 (t, 2H, J=5.6Hz), 3.00 (t, 2H, J=5.6Hz), 2.88 (s, 3H), 2.72-2.74 (m, 4H), 1.85-1.89 (m, 4H).

$^{13}$C NMR (100 MHz, CD3OD) δ: 170.33, 154.47, 148.22, 137.55, 137.16, 135.04, 134.01, 131.37, 130.28, 129.04, 127.66, 126.59, 125.23, 122.48, 121.94, 121.62, 121.20, 113.95, 66.75, 54.41, 54.16, 38.39, 38.17, 25.37, 22.84.

I-23

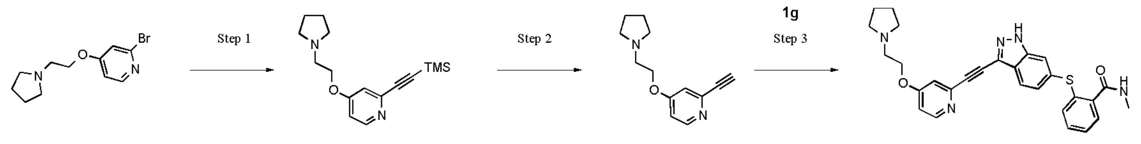


23b 25a 25b I-23

Step 1

Under the protection of nitrogen at room temperature, ethynyl(trimethyl)silane (283 mg, 2.88 mmol), Pd(PPh3) 2Cl2 (202 mg, 0.29 mmol), cuprous iodide (54.78 mg, 0.29 mmol) and triethylamine (436.63 mg, 4.31 mmol) were added to the solution of 2-bromo-4-(2-pyrrolidin-1-methoxy)pyridine 23b (390 mg, 1.44 mmol) in *N,N*-dimethylformamide (10 mL); the solution was heated to 50 °C and stirred to react for 12 h. After the thin-layer chromatography (dichloromethane/-methanol = 20/1) showed the complete reaction of the starting materials, the reaction mixture was poured into water (40 mL) and extracted with ethyl acetate (10 mL × 3), and the organic layers were combined, washed with saturated brine (20 mL), dried over anhydrous sodium sulfate and concentrated to obtain a crude product. The crude product was purified by preparative thin-layer chromatography (DCM /MeOH = 20:1) to obtain a black oily liquid, trimethyl-[2-[4-(2-pyrrolidin-1-methoxy)-2-pyridyl]ethynyl]silane25a (240 mg, yield: 57.85 %).

This product was verified by LCMS.

For MS-ESI, the calculated value was $[M+H]^+$289.2, and the measurement was 289.1.

Step 2

At room temperature, potassium carbonate (138 mg, 1.00 mmol) was added to the solution of trimethyl-[2-[4-(2-pyrrolidin-1-methoxy)-2-pyridyl]ethynyl]silane 25a (240 mg, 0.83 mmol) in methanol (10 mL), and then stirred to react for about 2 h. After the thin-layer chromatography (dichloromethane/methanol = 20/1) showed the complete reaction of the starting materials, the mixture was poured into water (30 mL) and extracted with ethyl acetate (10 mL × 3), and the organic layers were combined, washed with saturated brine (20 mL), dried over anhydrous sodium sulfate and concentrated to obtain a crude product. The crude product was purified by preparative thin-layer chromatography (dichloromethane/-methanol = 20/1) to obtain a red oily liquid, 2-ethynyl-4-(2-pyrrolidin-1-ethoxy)pyridine 25b (107 mg, yield: 59.46 %).

This product was verified by LCMS.

For MS-ESI, the calculated value was $[M+H]^+$217.1, and the measurement was 217.1.

Step 3

Under the protection of nitrogen at room temperature, 2-ethynyl-4-(2-pyrrolidin-1-ethoxy)pyridine 25b (107 mg, 0.49 mmol), dichlorobis(triphenylphosphin)palladium (69 mg, 0.099 mmol), cuprous iodide (19 mg, 0.099 mmol) , triethylamine (150 mg, 1.48 mmol) were added to the solution of 2-[(3-iodo-1H-indazol-6-yl)thio]-N-methylbenzamide 1 g (202 mg, 0.49 mmol) in N,N-dimethylformamide (9 mL); the solution was heated to 100 °C and stirred to react for about 2 h. After the reaction of the starting materials completed, as indicated by thin-layer chromatography (dichloromethane/-methanol = 10/1), the mixture was poured into water (40 mL), and extracted with ethyl acetate (10 mL × 3); the combined organic layers were washed with saturated brine (20 mL), dried over anhydrous sodium sulfate and concentrated to obtain a crude product. The crude product was purified by preparative thin-layer chromatography (dichloromethane/methanol /=10/1) to obtain a white solid, N-methyl-2-{[3-(2-{4-[2-(pyrrolidin-1-yl)ethoxy]pyridin-2-yl}ethynyl)-1H-indazol-6-yl]thio} benzamide I-23 (17.8 mg, yield: 7.23 %).

This product was verified by LCMS, HNMR and CNMR.

For MS-ESI, the calculated value was $[M+H]^+$498.2, and the measurement was 498.4.

$^1$H NMR (400MHz, CD3OD): δ (ppm) 8.39 (d, J=5.6Hz, 1H), 7.89 (d, J=8.4Hz, 1H), 7.62 (s, 1H), 7.49 (d, J=7.2Hz, 1H), 7.35 (s, 3H), 7.22-7.27 (m, 2H), 7.07 (d, J=4.8Hz, 1H), 4.31 (t, J=5.2Hz, 2H), 3.02 (t, J=5.2Hz, 2H), 2.74 (s, 4H), 1.87 (s, 4H).

$^{13}$C NMR (100 MHz, CD3OD) δ 170.30, 165.63, 150.62, 143.38, 140.86, 137.94, 135.11, 134.49, 131.79, 130.31, 127.81, 127.70, 126.86, 125.65, 123.81, 120.42, 113.83, 113.67, 110.49, 90.98, 80.53, 66.76, 54.14, 54.08, 25.31, 22.84.

I-24

Step 1 Step 2 Step 3 Step 4

Step 1

Under the protection of nitrogen at room temperature, 1-(2-chloroethyl)pyrrolidine hydrochloride 11a (1.54 g, 9.03 mmol) and $K_2CO_3$ (4.77 g, 34.48 mmol) were added to the solution of 6-bromopyridin-2-ol 12a (2 g, 11.49 mmol) in N,N-dimethylformamide (50 mL); the solution was heated to 70 °C and stirred to react for about 16 h. After the reaction of the starting materials completed, as indicated by thin-layer chromatography (dichloromethane/methanol = 15/1), the mixture was poured into water (200 mL) and extracted with ethyl acetate (50 mL × 3); and the combined organic layers were washed with saturated brine (50 mL), dried over anhydrous sodium sulfate and concentrated to obtain a crude product. The crude product was purified by preparative thin-layer chromatography to obtain a yellow oily liquid, 2-bromo-6-(2-pyrrolidin-1-methoxy)pyridine 26a (940 mg, 3.47 mmol, yield: 30.16 %).

This product was verified by LCMS and HNMR.

For MS-ESI, the calculated value was $[M+H]^+$272.9, and the measurement was 273.1.

$^1$H NMR (400MHz, $CDCl_3$): δ (ppm) 7.41 (t, J=8.0Hz, 1H), 7.05 (d, J=7.2Hz, 1H), 6.74 (d, J=8.4Hz, 1H), 4.45 (t, J=6.0Hz, 2H), 2.88 (t, J=6.0Hz, 2H), 2.61-2.64 (m, 4H), 1.80-1.84 (m, 4H).

Step 2

Under the protection of nitrogen at room temperature, ethynyl(trimethyl)silane (362.22 mg, 3.69 mmol), Pd(PPh3)2Cl2 (258.86 mg, 368.80 μmol), cuprous iodide (70.24 mg, 368.80 μmol) and triethylamine (559.78 mg, 5.53 mmo) were added to the solution of 2-bromo-6-(2-pyrrolidin-1-methoxy)pyridine 26a (0.5 g, 1.84 mmol) in N,N-dimethylformamide (20 mL); the solution was heated to 50 °C and stirred to react for 16 h. After LCMS showed the complete reaction of the starting materials, the reaction mixture was poured into water (80 mL) and extracted with ethyl acetate (20 mL × 3), and the organic layers were combined, washed with saturated brine (20 mL), dried over anhydrous sodium sulfate and concentrated to obtain a crude product. The crude product was purified by silica gel column chromatography (petroleum ether/ethyl acetate = 1/2) to obtain a red oily liquid, trimethyl-[2-[6-(2-pyrrolidin-1-methoxy)-2-pyridyl]ethynyl]silane26b (450 mg, 1.56 mmol, yield: 84.60 %).

This product was verified by LCMS and HNMR.

For MS-ESI, the calculated value was $[M+H]^+$289.2, and the measurement was 289.2.

$^1$H NMR (400MHz, $CDCl_3$): δ (ppm) 7.47-7.51 (m, 1H), 7.12 (d, J=7.2Hz, 1H), 6.76 (d, J=8.4Hz, 1H), 4.48 (t, J=5.6Hz, 2H), 2.89 (t, J=5.6Hz, 2H), 2.61-2.64 (m, 4H), 1.80-1.84 (m, 4H), 0.29 (s, 9H).

Step 3

At room temperature, potassium carbonate (229.98 mg, 1.66 mmol) was added to the solution of trimethyl-[2-[6-(2-pyrrolidin-1-methoxy)-2-pyridyl]ethynyl]silane 26b (400 mg, 1.39 mmol) in methanol (20 mL), and then stirred to react for about 2 h. After the thin-layer chromatography (dichloromethane/methanol = 20/1) showed the complete reaction of the starting materials, the mixture was poured into water (30 mL) and extracted with ethyl acetate (10 mL × 3), and the organic layers were combined, washed with saturated brine (20 mL), dried over anhydrous sodium sulfate and concentrated to obtain a crude product. The crude product was purified by preparative thin-layer chromatography (dichloromethane/methanol = 20/1) to obtain a red oily liquid, 2-ethynyl-6-(2-pyrrolidin-1-ethoxy)pyridine 26c (50 mg, 231.18 μmol, yield: 16.67 %).

This product was verified by LCMS and HNMR.

For MS-ESI, the calculated value was $[M+H]^+$217.1, and the measurement was 217.2.

$^1$H NMR (400MHz, $CDCl_3$): δ (ppm) 7.56 (t, J=8.0Hz, 1H), 7.11 (d, J=7.2Hz, 1H), 6.82 (d, J=8.4Hz, 1H), 4.51 (t, J=5.6Hz, 2H), 3.12 (s, 1H), 2.93 (t, J=5.6Hz, 2H), 2.67 (s, 1H), 1.84 (s, 1H).

Step 4

Under the protection of nitrogen at room temperature, 2-ethynyl-6-(2-pyrrolidin-1-ethoxy)pyridine 26c (25 mg, 115.59 μmol), dichlorobis(triphenylphosphin)palladium (16.23 mg, 23.12 μmol), cuprous iodide (4.40 mg, 23.12 μmol) and triethylamine (35.09 mg, 346.78 μmol) were added to the solution of 2-[(3-iodo-1*H*-indazol-6-yl)thio]-N-methylbenzamide 1 g (47.31 mg, 115.59 μmol) in N,N-dimethylformamide (10 mL); the solution was heated to 50°C and stirred to react for about 16 h. After the reaction of the starting materials completed, as indicated by thin-layer chromatography (dichloromethane/methanol = 10/1), the mixture was poured into water (40 mL), and extracted with ethyl acetate (10 mL × 3); the combined organic layers were washed with saturated brine (20 mL), dried over anhydrous sodium sulfate and concentrated to obtain a crude product. The crude product was purified by preparative thin-layer chromatography to obtain a white solid, N-methyl-2-{[3-(2-{6-[2-(pyrrolidin-1-yl)ethoxy]pyridin-2-yl}ethynyl)-1H-indazol-6-yl]thio}benzamide I-24 (17.8 mg, yield: 7.23 %).

This product was verified by LCMS, HNMR and CNMR.

For MS-ESI, the calculated value was $[M+H]^+$498.2, and the measurement was 498.0.

$^1$H NMR (400MHz, $CDCl_3$ + CD3OD): δ (ppm) 7.83 (d, J=8.4Hz, 1H), 7.79 (s, 1H), 7.73 (t, J=8.0Hz, 1H), 7.63 (s, 1H), 7.48-7.51 (m, 1H), 7.33-7.34 (m, 1H), 7.31-7.32 (m, 1H), 7.22-7.24 (m, 2H), 6.87 (d, J=8.4Hz, 1H), 4.59 (t, J=5.6Hz, 2H), 3.19 (t, J=5.2Hz, 2H), 2.96 (s, 4H), 2.88 (s, 3H), 1.95 (s, 4H).

I-8

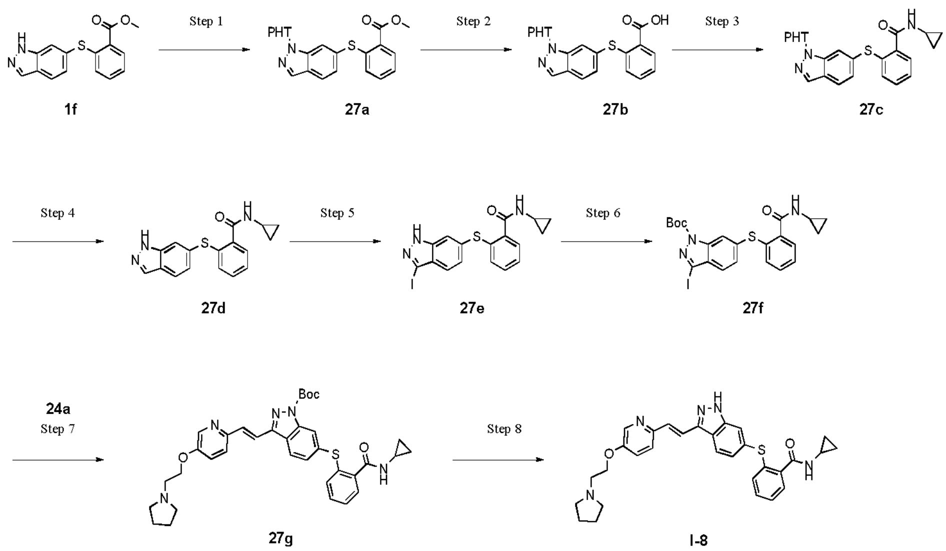

1f 27a 27b 27c
27d 27e 27f
27g I-8

Step 1

At room temperature, 3,4-dihydro-2H-pyrane (DHP) (1.8 g, 21.62 mmol) and TsOH (2.5 g, 14.78 mmol) were added to the solution of 2-(1H-indazol-6-ylthio)-N-methylbenzamide 1f (4 g, 14.07 mmol) in THF (20 mL), heated to 60°C and stirred to react for about 16 h. After the thin-layer chromatography (petroleum ether/ethyl acetate = 3/1) showed the complete reaction of the starting materials, the mixture was concentrated to obtain a crude product. The crude product was purified by preparative MPLC to obtain a yellow solid, 2-(1-tetrahydropyran-2-ylindazol-6-yl)thiomethyl benzoate 27a (2.5 g, 6.79 mmol, yield: 48.23 %).

This product was verified by LCMS and HNMR.

For MS-ESI, the calculated value was $[M+H]^+$369.1, and the measurement was 369.0.

$^1$H NMR (400 MHz, $CDCl_3$) δ 8.07 (d, J = 5.6 Hz, 1H), 8.02 (d, J = 7.6 Hz, 1H), 7.93 (d, J = 4.0 Hz, 1H), 7.77 (dd, J = 8.4, 4.0 Hz, 1H), 7.31 (d, J = 8.4 Hz, 1H), 7.25 (t, J = 7.6 Hz, 1H), 7.15 (t, J = 7.6 Hz, 1H), 6.85 (d, J = 8.0 Hz, 1H), 5.72 (dd, J = 9.6, 2.4 Hz, 1H), 4.13 - 4.03 (m, 1H), 3.99 (d, J = 2.0 Hz, 3H), 3.75 (dd, J = 11.2, 8.4 Hz, 1H), 2.59 - 2.39 (m, 1H), 2.13 (t, J = 10.4 Hz, 2H), 1.85 - 1.52 (m, 3H).

Step 2

At room temperature, 2-(1-tetrahydropyran-2-ylindazol-6-yl)thiomethyl benzoate 27a (2.5 g, 6.79 mmol) was dissolved in the mixed solution of $H_2O$ (8 mL) and THF (20 mL); lithium hydroxide (1.7 g, 40.71 mmol) was added; and the mixture was heated to 40 °C and stirred to react for about 16 h. After the thin-layer chromatography (petroleum ether/ethyl acetate = 1/1) showed the complete reaction of the starting materials, the reaction mixture was poured into water (20 mL), adjusted with 1N hydrochloric acid to pH = 6-7 and extracted with ethyl acetate (20 mL × 3), and the organic layers were combined, washed with saturated brine (20 mL), dried over anhydrous sodium sulfate and concentrated to obtain a white solid 2-(1-tetrahydropyran-2-ylindazol-6-yl)thiobenzoic acid 27b (2 g, 5.64 mmol, yield: 83.17 %).
This product was verified by LCMS and HNMR.
For MS-ESI, the calculated value was $[M+H]^+$355.1, and the measurement was 355.0.
$^1$H NMR (400 MHz, $CDCl_3$) δ 8.21 - 8.07 (m, 2H), 7.97 (s, 1H), 7.79 (dd, J = 8.4, 4.4 Hz, 1H), 7.40 - 7.30 (m, 1H), 7.28 - 7.25 (m, 1H), 7.18 (dd, J = 14.4, 7.2 Hz, 1H), 6.84 (dd, J = 8.0, 4.8 Hz, 1H), 5.74 (dd, J = 9.6, 2.4 Hz, 1H), 4.24 - 3.92 (m, 2H), 2.66 - 2.28 (m, 2H), 2.25 - 2.07 (m, 2H), 1.89 - 1.58 (m, 2H).

Step 3

At room temperature, cyclopropylamine (290 mg, 5.08 mmol), HATU (1.9 g, 5.08 mmol) and DIEA (980 mg, 7.60 mmol) were added to the solution of 2-(1-tetrahydropyran-2-ylindazol-6-yl)thiobenzoic acid 27b (900 mg, 2.54 mmol) in N,N-dimethylformamide (10 mL); and the solution was stirred to react for about 2 h. After the thin-layer chromatography (petroleum ether/ethyl acetate = 1/1) showed the complete reaction of the starting materials, the mixture was poured into water (50 mL) and extracted with ethyl acetate (20 mL × 3), and the organic layers were combined, washed with saturated brine (50 mL), dried over anhydrous sodium sulfate and concentrated to obtain a crude product. The crude product was purified by the silica gel column chromatography () to obtain a white solid N-cyclopropyl-2-(1-tetrahydropyran-2-ylindazol-6-yl)thiobenzamide 27c (700 mg, 1.78 mmol, yield: 70.05 %). This product was verified by LCMS and HNMR.
For MS-ESI, the calculated value was $[M+H]^+$394.2, and the measurement was 394.2.
$^1$H NMR (400 MHz, $CDCl_3$) δ 8.11 - 7.90 (m, 2H), 7.71 - 7.58 (m, 3H), 7.30 (dd, J = 4.8, 3.6 Hz, 2H), 7.25 - 7.18 (m, 1H), 7.12 (dd, J = 8.4, 1.2 Hz, 1H), 5.66 (dd, J = 9.6, 2.4 Hz, 1H), 4.11 - 3.89 (m, 2H), 2.38 (ddd, J = 17.6, 13.2, 7.6 Hz, 1H), 2.20 - 2.04 (m, 2H), 1.72 (ddd, J = 62.4, 40.0, 27.2 Hz, 4H), 0.82 (dd, J = 12.8, 7.2 Hz, 2H), 0.54 - 0.44 (m, 2H).

Step 4

At room temperature, trifluoroacetic acid (3 g, 52.62 mmol) was added to the solution of N-cyclopropyl-2-(1-tetrahydropyran-2-ylindazol-6-yl)thiobenzamide 27c (800 mg, 2.03 mmol) in DCM (10 mL), and the mixture was stirred at 40°C to react for about 16 h. After the reaction of the starting materials completed, as indicated by thin-layer chromatography (dichloromethane/methanol = 10/1), the mixture was poured into water (20 mL), adjusted with the sodium carbonate solution to pH=8-9, and extracted with dichloromethane (30 mL × 3); and the combined organic layers were washed with saturated brine (20 mL), dried over anhydrous sodium sulfate and concentrated to obtain a yellow solid, N-cyclopropyl-2-(1H-indazol-6-ylthio)benzamide 27d (500 mg, 1.62 mmol, yield: 79.49 %). This product was verified by LCMS and HNMR.
For MS-ESI, the calculated value was $[M+H]^+$310.1, and the measurement was 310.0.
$^1$H NMR (400 MHz, $CDCl_3$) δ 10.99 (s, 1H), 8.05 (s, 1H), 7.77 - 7.53 (m, 3H), 7.32 - 7.20 (m, 3H), 7.12 (d, J = 8.4 Hz, 1H), 6.60 (s, 1H), 2.90 - 2.80 (m, 1H), 0.81 (q, J = 6.4 Hz, 2H), 0.51 (d, J = 6.4 Hz, 2H).

Step 5

At room temperature, potassium carbonate (2.3 g, 16.81 mmol) and $I_2$ (2.1 g, 8.40 mmol) were added to the solution of N-cyclopropyl-2-(1H-indazol-6-ylthio)benzamide 27d (1.3 g, 4.20 mmol) in N,N-dimethylformamide (10 mL); the mixture was stirred at 40°C to react for about 2 h. After the reaction of the starting materials completed, as indicated by thin-layer chromatography (dichloromethane/methanol = 10/1), the mixture was poured into water (100 mL), and extracted with ethyl acetate (100 mL × 3); the combined organic layers were washed with saturated brine (200 mL), dried over anhydrous sodium sulfate and concentrated to obtain a crude product. The crude product was purified by the silica gel column chromatography (petroleum ether/ethyl acetate = 2/1~0/1) to obtain a yellow solid N-cyclopropyl-2-[(3-iodo-1H-indazol-6-yl)thio]benzamide 27e (1 g, 2.30 mmol, yield: 54.67 %).
This product was verified by LCMS and HNMR.
For MS-ESI, the calculated value was $[M+H]^+$436.0, and the measurement was 435.9.
$^1$H NMR (400 MHz, $CDCl_3$) δ 11.17 (s, 1H), 8.04 (s, 1H), 7.68 - 7.55 (m, 2H), 7.46 (d, J = 8.4 Hz, 1H), 7.32 (dd, J = 9.2, 5.6 Hz, 2H), 7.25 - 7.20 (m, 1H), 7.17 (d, J = 8.4 Hz, 1H), 2.88 - 2.80 (m, 1H), 0.85 (q, J = 6.8 Hz, 2H), 0.53 (q, J = 6.8 Hz,

2H).

Step 6

At room temperature, tert-butoxycarbonyl carbonate (250. mg, 1.15 mmol) and triethylamine (232 mg, 2.30 mmol) were added to the solution of N-cyclopropyl-2-[(3-iodo-1H-indazol-6-yl)thio]benzamide 27e (1.3 g, 4.20 mmol) in THF (10 mL); and the solution was stirred to react for about 2 h. After the reaction of the starting materials completed, as indicated by thin-layer chromatography (dichloromethane/methanol = 10/1), the mixture was poured into water (20 mL), and extracted with ethyl acetate (20 mL × 3); the combined organic layers were washed with saturated brine (30 mL), dried over anhydrous sodium sulfate and concentrated to obtain a crude product. The crude product was purified by preparative thin-layer chromatography (dichloromethane/methanol = 10/1) to obtain a white solid, 6-[2-(cyclopropylcarbamoyl) phenyl]thio-3-iodoindazol-1-tert-butyl carboxylate 27f (280 mg, 522.98 μmol, yield: 45.53 %).

This product was verified by LCMS and HNMR.

For MS-ESI, the calculated value was $[M\text{-}Boc+H]^+$436.0, and the measurement was 435.9.

$^1$H NMR (400 MHz, $CDCl_3$) δ 8.04 (s, 1H), 7.75 - 7.62 (m, 1H), 7.40 (dd, J = 13.6, 5.6 Hz, 4H), 7.31 - 7.19 (m, 2H), 2.81 (d, J = 4.4 Hz, 1H), 1.64 (s, 9H), 0.79 (t, J = 6.0 Hz, 2H), 0.47 (d, J = 2.4 Hz, 2H).

Step 7

Under the protection of nitrogen at room temperature, 5-(2-pyrrolidin-1-ethoxy)-2-vinylpyridine 24a (97 mg, 448.27 μmol), triethylamine (113 mg, 1.12 mmol), $Pd_2(dba)_3$ (171 mg, 186.78 μmol) and P(o-tol)3 (113 mg, 373.55 μmol) were added to the solution of 6-[2-(cyclopropylcarbamoyl)phenyl]thio-3-iodoindazol-1-tert-butyl carboxylate 27f (200 mg, 373.55 μmol) in 1,4-dioxane (5 mL); the solution was heated to 100 °C and stirred to react for about 2 h. After the reaction of the starting materials completed, as indicated by thin-layer chromatography (dichloromethane/methanol = 10/1), the mixture was poured into water (20 mL) and extracted with ethyl acetate (10 mL × 3); and the combined organic layers were washed with saturated brine (20 mL), dried over anhydrous sodium sulfate and concentrated to obtain a crude product. The crude product was purified by preparative thin-layer chromatography (dichloromethane/methanol = 10/1) to obtain a yellow oily liquid, 6-[2-(cyclopropylcarbamoyl)phenyl]thio-3-[(E)-2-[5-(2-pyrrolidin-1-ylethoxy)-2-pyridyl]vinyl]indazol-1-tert-butyl carboxylate 27 g (150 mg, 239.70 μmol, yield: 64.17 %).

This product was verified by LCMS.

For MS-ESI, the calculated value was $[M+H]^+$626.3, and the measurement was 626.2.

Step 8

At room temperature, trifluoroacetic acid (1 g, 8.77 mmol) was added to the solution of 6-[2-(cyclopropylcarbamoyl)phenyl]thio-3-[(E)-2-[5-(2-pyrrolidin-1-ylethoxy)-2-pyridyl]vinyl]indazol-1-tert-butyl carboxylate 27 g (150 mg, 239.70 μmol) in dichloromethane (5 mL), and then stirred to react for about 4 h. After the reaction of the starting materials completed, as indicated by thin-layer chromatography (dichloromethane/methanol = 10/1), the mixture was poured into water (20 mL), adjusted with the sodium carbonate solution to pH=8-9, and extracted with ethyl acetate (20 mL × 3); the combined organic layers were washed with saturated brine (30 mL), dried over anhydrous sodium sulfate and concentrated to obtain a crude product. The crude product was purified by preparative thin-layer chromatography to obtain a white solid, N-cyclopropyl-2-({3-[(E)-2-{5-[2-(pyrrolidin-1-yl)ethoxy]pyridin-2-yl}vinyl]-1H-indazol-6-yl}thio)benzamide I-8 (15.4 mg, 29.30 μmol, yield: 12.22 %).

This product was verified by LCMS and HNMR.

For MS-ESI, the calculated value was $[M+H]^+$526.2, and the measurement was 526.2.

$^1$H NMR (400 MHz, $CDCl_3$) δ 8.38 (d, J = 2.8 Hz, 1H), 8.01 (d, J = 8.4 Hz, 1H), 7.75 (d, J = 16.4 Hz, 1H), 7.67 (dd, J = 5.6, 3.2 Hz, 1H), 7.58 - 7.41 (m, 2H), 7.35 (dd, J = 24.0, 19.6 Hz, 2H), 7.25 (dd, J = 8.8, 2.8 Hz, 2H), 7.18 (d, J = 8.4 Hz, 1H), 6.54 (s, 1H), 4.24 (t, J = 5.6 Hz, 2H), 3.00 (t, J = 5.6 Hz, 2H), 2.89 - 2.80 (m, 1H), 2.72 (s, 4H), 1.87 (d, J = 3.2 Hz, 4H), 0.86 - 0.75 (m, 2H), 0.50 (t, J = 8.0 Hz, 2H).

I-38

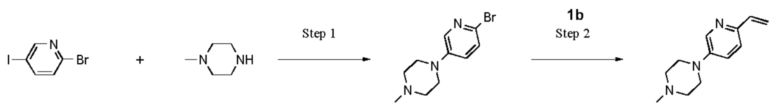


28a 28b 28c 28d

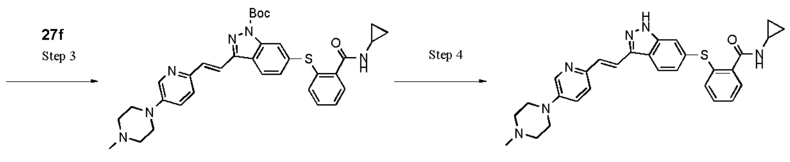


28e I-38

Step 1

Under the protection of nitrogen at room temperature, 1-methylpiperazine 28b (2.1 g, 21.13 mmol), BuONa (5.1 g, 52.84 mmol), $Pd_2(dba)_3$ (806.4 mg, 880.62 μmol) and Xantphos (254.8 mg, 440.31 μmol) were added to the solution of 2-bromo-5-iodopyridine 28a (5 g, 17.61 mmol) in 1,4-dioxane (50 mL); the solution was heated to 60 °C and stirred to react for about 3 h. After the reaction of the starting materials completed, as indicated by thin-layer chromatography (dichloromethane/methanol = 10/1), the mixture was poured into water (100 mL) and extracted with ethyl acetate (50 mL × 3); and the combined organic layers were washed with saturated brine (100 mL), dried over anhydrous sodium sulfate and concentrated to obtain a crude product. The crude product was purified by neutral alumina chromatographic columns (petroleum ether/ethyl acetate = 10/1) to obtain a yellow solid, 1-(6-bromo-3-pyridyl)-4-methylpiperazine 28c (2.1 g, 8.20 mmol, yield: 46.55 %).

This product was verified by LCMS and HNMR.

For MS-ESI, the calculated value was $[M+H]^+$257.9, and the measurement was 258.0.

$^1H$ NMR (400 MHz, $CDCl_3$) δ (ppm) 8.01 (d, J = 3.2 Hz, 1H), 7.30 (d, J = 8.8 Hz, 1H), 7.07 (dd, J = 8.8, 3.2 Hz, 1H), 3.21 (t, J = 4.8 Hz, 4H ), 2.57 (t, J = 5.2 Hz, 4H ), 2.35 (s, 3H).

Step 2

Under the protection of nitrogen at room temperature, potassium vinyltrifluoroborate 1b (1.3 g, 9.37 mmol), $Pd(dppf)Cl_2$ (571.3 mg, 780.82 μmol) and triethylamine (2.4 g, 23.42 mmol) were added to the solution of 1-(6-bromo-3-pyridyl)-4-methylpiperazine 28c (2 g, 7.81 mmol) in N,N-dimethylformamide (20 mL); the solution was heated to 85 °C and stirred to react for about 2 h. After the reaction of the starting materials completed, as indicated by thin-layer chromatography (dichloromethane/methanol = 10/1), the mixture was poured into water (100 mL) and extracted with ethyl acetate (50 mL × 3); and the combined organic layers were washed with saturated brine (100 mL), dried over anhydrous sodium sulfate and concentrated to obtain a crude product. The crude product was purified by neutral alumina chromatographic columns (petroleum ether/ethyl acetate = 10/1) to obtain a yellow oily liquid, 1-methyl-4-(6-vinyl-3-pyridyl)piperazine 28d (1.2 g, 5.90 mmol, yield: 75.60 %).

This product was verified by LCMS and HNMR.

For MS-ESI, the calculated value was $[M+H]^+$204.1, and the measurement was 204.1.

$^1H$ NMR (400 MHz, $CDCl_3$) δ (ppm) 8.27 (d, J = 3.2 Hz, 1H), 7.24 (s, 1H), 7.16-7.13 (m, 1H), 6.75 (dd, J = 17.5, 10.8 Hz, 1H), 5.98 (d, J = 17.6 Hz, 1H), 5.31 (d, J = 10.8 Hz, 1H), 3.28 - 3.20 (m, 4H), 2.62 - 2.55 (m, 4H), 2.36 (s, 3H).

Step 3

Under the protection of nitrogen at room temperature, 1-methyl-4-(6-vinyl-3-pyridyl)piperazine 28d (223.3 mg, 1.10 mmol), triethylamine (277.8 mg, 2.75 mmol), $Pd_2(dba)_3$ (502.8 mg, 549.13 μmol) and P(o-tol)3 (557.1 mg, 1.83 mmol) were added to the solution of 6-[2-(cyclopropylcarbamoyl)phenyl]thio-3-iodoindazol-1-tert-butyl carboxylate 27f (490 mg, 915.21 μmo) in 1,4-dioxane (10 mL); the solution was heated to 85 °C and stirred to react for about 2 h. After the reaction of the starting materials completed, as indicated by thin-layer chromatography (dichloromethane/methanol = 10/1), the mixture was poured into water (40 mL) and extracted with ethyl acetate (30 mL × 3); and the combined organic layers were washed with saturated brine (40 mL), dried over anhydrous sodium sulfate and concentrated to obtain a crude product. The crude product was purified by preparative MPLC to obtain a white solid, 6-[2-(cyclopropylcarbamoyl)phenyl]thio-3-[(E)-2-[5-(4-methylpiperazin-1-yl)-2-pyridyl]vinyl]indazol-1-tert-butyl carboxylate 28e (110 mg, 180.10 μmol, yield:

19.68 %).

This product was verified by LCMS.

For MS-ESI, the calculated value was $[M+H]^+$611.3, and the measurement was 611.2.

Step 4

At room temperature, trifluoroacetic acid (186.7 mg, 1.64 mmol) was added to the solution of 6-[2-(cyclopropylcarbamoyl)phenyl]thio-3-[(E)-2-[5-(4-methylpiperazin-1-yl)-2-pyridyl]vinyl]indazol-1-tert-butyl carboxylate 28e (100 mg, 163.73 μmol) in dichloromethane (2 mL), and then stirred to react for about 16 h. After the reaction of the starting materials completed, as indicated by thin-layer chromatography (dichloromethane/methanol = 10/1), the mixture was poured into water (20 mL), adjusted with the sodium carbonate solution to pH=8-9, and extracted with dichloromethane (20 mL × 3); and the combined organic layers were washed with saturated brine (20 mL), dried over anhydrous sodium sulfate and concentrated to obtain a crude product. The crude product was purified by preparative thin-layer chromatography to obtain a yellow solid, N-cyclopropyl-2-({3-[(E)-2-[5-(4-methylpiperazin-1-yl)pyridin-2-yl]vinyl]-1H-indazol-6-yl}thio)benzamide I-38 (50 mg, 97.91 μmol, yield: 59.80 %).

This product was verified by LCMS, HNMR and CNMR.

For MS-ESI, the calculated value was $[M+H]^+$511.2, and the measurement was 511.0.

$^1$H NMR (400MHz, CD3OD): δ (ppm) 8.22 (d, J = 2.8 Hz, 1H), 8.02 (d, J = 8.8 Hz, 1H), 7.64 - 7.54 (m, 3H), 7.49-7.43 (m, 2H), 7.40 - 7.29 (m, 3H), 7.25 - 7.23 (m, 1H), 7.17 (d, J = 8.4 Hz, 1H), 3.33 (s, 4H), 2.79-2.75 (m, 1H), 2.65-2.62 (m, 4H), 2.36 (s, 3H), 0.77-0.72 (m, 2H), 0.55 - 0.51 (m, 2H).

$^{13}$C NMR (100 MHz, $CDCl_3$& CD3OD) δ: 172.68, 147.59, 147.39, 138.66, 137.99, 135.33, 131.77, 130.85, 128.00, 126.48, 124.48, 123.29, 122.73, 122.17, 121.60, 55.71, 23.95, 6.80.

I-40

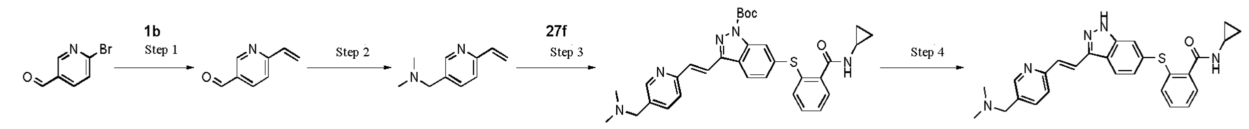


29a 29b 29c 29d I-40

Step 1

Under the protection of nitrogen at room temperature, potassium vinyltrifluoroborate 1b (5.4 g, 40.32 mmol), $Pd(dppf)Cl_2$ (975.3 mg, 1.34 mmol) and triethylamine (8.2 g, 80.64 mmol) were added to the solution of 6-bromopyridin-3-formaldehyde 29a (5 g, 26.88 mmol) in *N,N*-dimethylformamide (50 mL); the solution was heated to 85 °C and stirred to react for about 16 h. After the thin-layer chromatography (petroleum ether/ethyl acetate = 10/1) showed the complete reaction of the starting materials, the mixture was poured into water (250 mL) and extracted with ethyl acetate (100 mL × 3), and the organic layers were combined, washed with water (300 mL) and saturated brine (300 mL) in sequence, dried over anhydrous sodium sulfate and concentrated to obtain a crude product. The crude product was purified by the silica gel chromatographic column (petroleum ether/ethyl acetate = 10/1) to obtain a yellow oily liquid 6-vinylpyridin-3-formaldehyde 29b (1.7 g, 12.77 mmol, yield: 47.50 %).

This product was verified by LCMS and HNMR.

For MS-ESI, the calculated value was $[M+H]^+$134.1, and the measurement was 134.1.

$^1$H NMR (400 MHz, $CDCl_3$): δ 10.08 (s, 1H), 9.01 (s, 1H), 8.13 (d, J = 8.0 Hz, 1H), 7.48 (d, J = 8.4 Hz, 1H), 6.88 (dd, J=17.6 Hz, 10.8 Hz, 1H),6.41 (d, J=17.6 Hz, 1H), 5.68 (d, J=10.8 Hz, 1H).

Step 2

At room temperature, N-methylmethylamine hydrochloride (612.4 mg, 7.51 mmol) and NaBH(OAc)3 (2.4 g, 11.27 mmol) were added to the solution of 6-vinylpyridin-3-formaldehyde 29b (500 mg, 3.76 mmol) in THF (20 mL); and the solution was stirred to react for about 16 h. After the thin-layer chromatography (dichloromethane/methanol = 10/1) showed the complete reaction of the starting materials, the mixture was poured into water (20 mL), adjusted with sodium bicarbonate to pH = 8-9 and extracted with ethyl acetate (20 mL × 3), and the organic layers were combined, washed with saturated brine (30 mL), dried over anhydrous sodium sulfate and concentrated to obtain a crude product. The crude product was purified by silica gel column chromatography (DCM) to obtain a white solid, N,N-dimethyl-1-(6-vinyl-3-pyridyl) methylamine 29c (400 mg, 2.47 mmol, yield: 65.66 %).

This product was verified by LCMS and HNMR.

For MS-ESI, the calculated value was $[M+H]^+$163.1, and the measurement was 163.1.

$^1$H NMR (400MHz, $CDCl_3$ ): δ 8.45 (s, 1H), 7.62 (dd, J = 8.0 Hz, 1.6 Hz, 1H), 7.32 (d, J = 8.0 Hz, 1H), 6.81 (dd, J = 17.6 Hz, 10.8 Hz, 1H), 6.16 (d, J = 17.6 Hz, 1H), 5.46 (dd, J = 10.8 Hz, 4.4 Hz, 1H), 3.41 (s, 2H), 2.23 (s, 6H).

Step 3

Under the protection of nitrogen at room temperature, N,N-dimethyl-1-(6-vinyl-3-pyridyl)methylamine 29c (303 mg, 1.87 mmol), triethylamine (472.5 mg, 4.67 mmol), $Pd_2(dba)_3$ (427.6 mg, 466.94 μmol) and P(o-tol)3 (426.4 mg, 1.40 mmol) were added to the solution of were added to the solution of 6-[2-(cyclopropylcarbamoyl)phenyl]thio-3-iodoindazol-1-tert-butyl carboxylate 27f (500 mg, 933.89 μmol) in 1,4-dioxane (10 mL); the solution was heated to 85 °C and stirred to react for about 2 h. After the reaction of the starting materials completed, as indicated by thin-layer chromatography (petroleum ether/ethyl acetate =1/1), the mixture was poured into water (20 mL) and extracted with ethyl acetate (20 mL × 3); and the combined organic layers were washed with saturated brine (50 mL), dried over anhydrous sodium sulfate and concentrated to obtain a crude product. The crude product was purified by preparative MPLC to obtain a yellow oily liquid, 6-[2-(cyclopropylcarbamoyl)phenyl]thio-3-[(E)-2-[5-[(dimethylamino)methyl]-2-pyridyl]vinyl]indazol-1-tert-butyl carboxylate 29d (155 mg, 272.07 μmol, yield: 29.13 %).

This product was verified by LCMS.

For MS-ESI, the calculated value was $[M+H]^+$570.2, and the measurement was 570.2.

Step 4

At room temperature, trifluoroacetic acid (300.2 mg, 2.63 mmol) was added to the solution of 6-[2-**(cyclopropylcarbamoyl)phenyl]thio-3-[(E)-2-[5-[(dimethylamino)methyl]-2-pyridyl]vinyl]indazol-1-tert-butyl** carboxylate 29d (150 mg, 263.29 μmol) in dichloromethane (5 mL), and then stirred to react for about 16 h. After the reaction of the starting materials completed, as indicated by thin-layer chromatography (dichloromethane/methanol = 10/1), the mixture was poured into water (20 mL), adjusted with the sodium carbonate solution to pH=8-9, and extracted with dichloromethane (20 mL × 3); and the combined organic layers were washed with saturated brine (30 mL), dried over anhydrous sodium sulfate and concentrated to obtain a crude product. The crude product was purified by preparative thin-layer chromatography to obtain a white solid, N-cyclopropyl-2-({3-[(E)-2-{5-[(dimethylamino)methyl]pyridin-2-yl}vinyl]-1H-indazol-6-yl}thio)benzamide I-40 (29 mg, 61.75 μmol, yield: 23.46 %).

This product was verified by LCMS, HNMR and CNMR.

For MS-ESI, the calculated value was $[M+H]^+$470.2, and the measurement was 470.1.

$^1$H NMR (400MHz, CD3OD): δ 8.49 (s, 1H), 8.05 (d, J = 8.64 Hz, 1H), 7.86 (d, J = 16.8 Hz, 1H), 7.80 (dd, J = 8.0 Hz, 1.6 Hz, 1H), 7.68 (d, J = 8.0 Hz, 1H), 7.56 (d, J = 16.6 Hz, 2H), 7.49 - 7.44 (m, 1H), 7.37 - 7.27 (m, 2H), 7.24-7.2 (m, 2H), 3.57 (s, 2H), 2.63-2.8 (m,1H), 2.3 (s, 6H), 0.76 (q, J = 7.0 Hz, 2H), 0.60 - 0.51 (m, 2H).

13CNMR (100MHz, CD3OD&$CDCl_3$): δ 172.66, 156.23, 151.27, 139.82, 133.39, 132.97, 131.82, 130.51, 129.21, 128.05, 126.72, 122.94, 122.64, 121.78, 61.87, 23.98, 6.88.

I-41

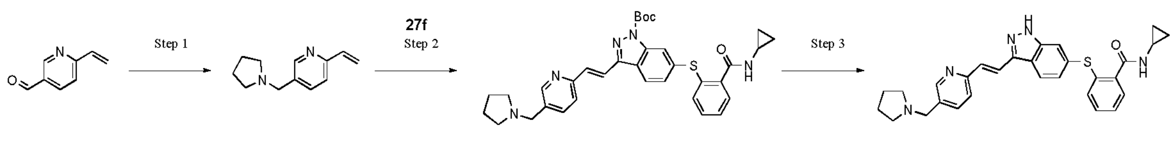


29b 30a 30b I-41

Step 1

At room temperature, pyrrolidine (320.5 mg, 4.51 mmol) and NaBH(OAc)3 (2.4 g, 11.27 mmol) were added to the solution of 6-vinylpyridin-3-formaldehyde 29b (500 mg, 3.76 mmol) in tetrahydrofuran (20 mL); and the solution was stirred to react for about 16 h. After the thin-layer chromatography (dichloromethane/methanol = 10/1) showed the complete reaction of the starting materials, the mixture was poured into water (20 mL), adjusted with sodium bicarbonate to pH = 8-9 and extracted with ethyl acetate (30 mL × 3), and the organic layers were combined, washed with saturated brine (50 mL), dried over anhydrous sodium sulfate and concentrated to obtain a crude product. The crude product was purified by silica gel column chromatography (DCM) to obtain a white solid, 5-(pyrrolidin-1-**ylmethyl)-2-vinylpyridine** 30a (420 mg, 2.23 mmol, yield: 59.41 %).

This product was verified by LCMS and HNMR.

For MS-ESI, the calculated value was $[M+H]^+$189.1, and the measurement was 189.1.

$^1H$ NMR (400MHz, $CDCl_3$ ): δ 8.49 (s, 1H), 7.64 (dd, J = 8.0 Hz, 1.6 Hz, 1H), 7.31 (d, J = 8.0 Hz, 1H), 6.81 (dd, J = 17.5 Hz, 10.8 Hz, 1H), 6.16 (d, J = 17.5 Hz, 1H), 5.45 (d, J = 10.8 Hz, 1H), 3.61 (s, 2H), 2.50 (s, 4H), 1.78 (s, 4H).

Step 2

Under the protection of nitrogen at room temperature, 5-(pyrrolidin-1-ylmethyl)-2-vinylpyridine 30a (351.6 mg, 1.87 mmol), triethylamine (472.5 mg, 4.67 mmol), $Pd_2(dba)_3$ (427.6 mg, 466.94 μmol) and P(o-tol)3 (426.4 mg, 1.40 mmol) were added to the solution of were added to the solution of 6-[2-(cyclopropylcarbamoyl)phenyl]thio-3-iodoindazol-1-tert-butyl carboxylate 27f (500 mg, 933.89 μmol) in 1,4-dioxane (10 mL); the solution was heated to 85 °C and stirred to react for about 2 h. After the reaction of the starting materials completed, as indicated by thin-layer chromatography (petroleum ether/ethyl acetate =1/1), the mixture was poured into water (20 mL) and extracted with ethyl acetate (20 mL × 3); and the combined organic layers were washed with saturated brine (50 mL), dried over anhydrous sodium sulfate and concentrated to obtain a crude product. The crude product was purified by preparative MPLC to obtain a yellow oily liquid, 6-[2-(cyclopropylcarbamoyl)phenyl]thio-3-[(E)-2-[5-(pyrrolidin-1-ylmethyl)-2-pyridyl]vinyl]indazol-1-tert-butyl carboxylate 30b (91 mg, 152.75 μmol, yield: 16.36 %).

This product was verified by LCMS.

For MS-ESI, the calculated value was $[M+H]^+$596.3, and the measurement was 596.3.

Step 3

At room temperature, trifluoroacetic acid (191.4 mg, 1.68 mmol) was added to the solution of 6-[2-(cyclopropylcarbamoyl)phenyl]thio-3-[(E)-2-[5-(pyrrolidin-1-ylmethyl)-2-pyridyl]vinyl]indazol-1-tert-butyl carboxylate 30b (100 mg, 167.85 μmol) in dichloromethane (5 mL), and then stirred to react for about 16 h. After the reaction of the starting materials completed, as indicated by thin-layer chromatography (dichloromethane/methanol = 10/1), the mixture was poured into water (30 mL), adjusted with the sodium carbonate solution to pH=8-9, and extracted with dichloromethane (20 mL × 3); and the combined organic layers were washed with saturated brine (30 mL), dried over anhydrous sodium sulfate and concentrated to obtain a crude product. The crude product was purified by preparative thin-layer chromatography to obtain a white solid, N-cyclopropyl-2-({3-[(E)-2-{5-[(pyrrolidin-1-yl)methyl]pyridin-2-yl}vinyl]-1H-indazol-6-yl}thio)benzamide I-41 (15 mg, 30.26 μmol, yield: 18.03 %).

This product was verified by LCMS, HNMR and CNMR.

For MS-ESI, the calculated value was $[M+H]^+$496.2, and the measurement was 496.1.

$^1H$ NMR (400MHz, CD3OD): δ 8.53 (d, J = 1.6 Hz, 1H), 8.06 (d, J = 8.5 Hz, 1H), 7.85 (dd, J = 16.7 Hz, 9.3 Hz, 2H), 7.69 (d, J = 8.1 Hz, 1H), 7.60 - 7.53 (m, 2H), 7.45 (dd, J = 7.1 Hz, 1.9 Hz, 1H), 7.34-7.32 (m, 2H), 7.28 - 7.24 (m, 1H), 7.20 (dd, J = 8.5 Hz, 1.4 Hz, 1H), 3.75 (s, 2H), 2.78-2.64 (m, 1H), 2.64 (s, 4H), 1.86 (s, 4H), 0.78 - 0.71 (m, 2H), 0.56 - 0.50 (m, 2H).

$^{13}C$ NMR (100MHz, CD3OD): δ 172.70, 155.96, 151.03, 143.79, 143.60, 139.59, 139.26, 135.90, 135.76, 133.23, 131.69, 130.39, 128.22, 126.42, 125.30, 122.93, 122.53, 121.60, 144.88, 58.17, 54.93, 24.19, 23.80, 6.49.

I-33

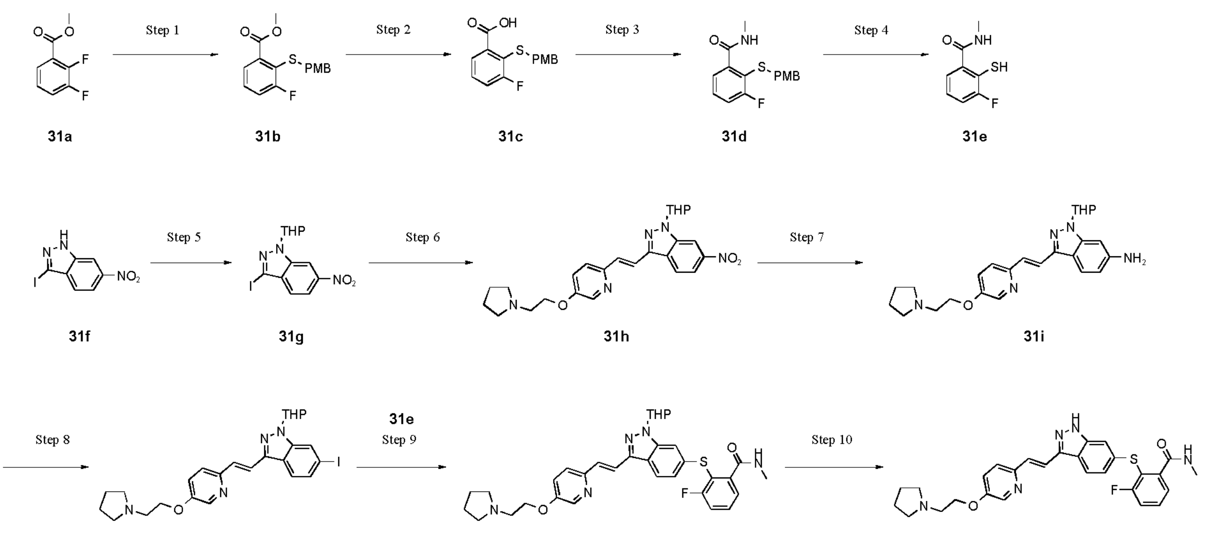


31a 31b 31c 31d 31e

31f 31g 31h 31i

31j 31k I-33

Step 1

In a 250 mL single-mouth flask, 2,3-difluoromethyl benzoate 31a (5.00 g, 29.1 mmol) was dissolved in N,N-dimethylformamide (50 mL), followed by the addition of (4-methoxyphenyl)methanethiol (4.564 g, 29.1 mmol) and cesium carbonate (18.94 g, 58.2 mmol); the mixture was stirred at room temperature to react for 2 h. The reaction system was filtered to collect the filtrate, which was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 3: 1), and concentrated to obtain a white solid 3-fluoro-2-[(4-methoxyphenyl)methylthio] methyl benzoate 31b (4.5 g, 14.7 mmol, yield: 50.5 %).
MS (ESI) M/Z: 329.1 [M+Na+]

Step 2

In a 250 mL single-mouth flask, 3-fluoro-2-[(4-methoxyphenyl)methylthio]methyl benzoate 31b (4.50 g, 14.7 mmol) was dissolved in tetrahydrofuran (45 mL), followed by the addition of lithium hydroxide (1.853 g, 44.1 mmol) and water (15 mL); the mixture was heated to 50 °C and stirred to react for 5 h. The reaction system was filtered to collect the filtrate, which was concentrated under reduced pressure; the dilute hydrochloric acid (1 mol/L) was added to the system to adjust the pH value to 5; and the reaction system was then filtered and dried to obtain a white solid 3-fluoro-2-[(4-methoxyphenyl)methylthio]benzoic acid 31c (3.80 g, 13.0 mmol, yield: 88.4 %).
MS (ESI) M/Z: 315.2 [M+Na+]

Step 3

In a 250 mL single-mouth flask,3-fluoro-2-[(4-methoxyphenyl)methylthio]benzoic acid 31c (3.80 g, 13.0 mmol) was dissolved in N,N-dimethylformamide (38 mL), followed by the addition of methylamine (506 mg, 19.5 mmol) and N-methylmorpholine (3.943 g, 39.0 mmol), and then 1-hydroxybenzotriazole (2.64 g, 19.5 mmol) and 1-(3-dimethylaminopropyl)-3-ethylcarbodiimide hydrochloride (3.72 g, 19.5 mmol); the mixture was maintained at room temperature and stirred to react for 3 h. The reaction system was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 4: 1), and concentrated to obtain a white solid 3-fluoro-2-[(4-methoxyphenyl) methylthio]-N-methylbenzamide 31d (2.95 g, 9.66 mmol, yield: 74.3 %).

Step 4

In a 250 mL three-mouth flask,, 3-fluoro-2-[(4-methoxyphenyl)methylthio]-N-methylbenzamide 31d (2.95 g, 9.66 mmol) was dissolved in trifluoroacetic acid (32 mL); the solution was heated to 70 °C, and stirred to react for 3 h. After concentration under reduced pressure, the solid residue obtained was separated by the silica gel column chromatography (eluent: dichloromethane/methanol = 30: 1), and concentrated to obtain 1.2 g of white solid 3-fluoro-N-methyl-2-thiobenzamide 31e (1.05 g, 5.68 mmol, yield: 58.8 %).

Step 5

At room temperature, 3,4-dyhydro-2H-pyran(2.2 g, 25.95 mmol) and p-toluenesulfonic acid(357.5 mg, 2.08 mmol) were added to the solution of 3-iodo-6-nitro-1H-indazole 31f (5 g, 17.30 mmol) in ethyl acetate (100 mL); the solution was heated to 75 °C and stirred to react for 16 h; after the reaction of the starting materials completed, as indicated by thin-layer chromatography (petroleum ether/ethyl acetate =10/1), the reaction liquid was concentrated to obtain a crude product; and the crude product was purified by column chromatography (petroleum ether/ethyl acetate = 20/1) to obtain a yellow solid compound, 3-iodo-6-nitro-1-tetrahydropyran-2-ylindazole 31 g (6.1 g, 16.35 mmol, yield: 94.50 %).
This product was verified by H-NMR.
$^1$H NMR (400 MHz, $CDCl_3$) δ 8.58 (s, 1H), 8.11 (dd, J = 8.8, 1.2 Hz, 1H), 7.63 (d, J = 8.8 Hz, 1H), 5.81 (dd, J = 9.2, 2.8 Hz, 1H), 4.06 (d, J = 10.4 Hz, 1H), 3.84-3.79 (m, 1H), 2.56-2.53 (m, 1H), 2.18-2.15 (m 2H), 1.81-1.73 (m, 3H).

Step 6

Under the atmosphere of nitrogen at room temperature, the compound 5-(2-pyrrolidin-1-ethoxy)-2-vinylpyridine 24a (3.5 g, 16.08 mmol), tris(dibenzalaceton)dipalladium (6.1 g, 6.70 mmol), triethylamine (4.1 g, 40.20 mmol) and tris(o-methylphenyl)phosphine (4.1 g, 13.40 mmol) were added to the solution of the compound 3-iodo-6-nitro-1-tetrahydropyran-2-ylindazole 31 g (5 g, 13.40 mmol) in dioxane (60 mL); the solution was heated to 100 °C and stirred to react for 2 h; after the reaction of the starting materials completed, as indicated by thin-layer chromatography (dichloromethane/-methanol =10/1), the mixture was poured into water (120 mL) and filtered; the filtrate was extracted with dichloromethane (120 mL × 3); the combined organic layers were dried over anhydrous sodium sulfate and concentrated to obtain a crude product; and the crude product was purified by column chromatography (dichloromethane/methanol =20/1) to obtain a yellow solid compound, 6-nitro-3-[(E)-2-[5-(2-pyrrolidin-1-ethoxy)-2-pyridyl]vinyl]-1-tetrahydropyran-2-ylindazole 31h (2.2 g, 4.64 mmol, yield: 34.62 %). This product was verified by LCMS and HNMR.

For MS-ESI, the calculated value was $[M+H]^+$464.2, and the measurement was 464.1.

$^1$H NMR (400 MHz, $CDCl_3$) δ 8.57 (s, 1H), 8.40 (d, J = 2.8 Hz, 1H), 8.15-8.10 (m, 2H), 7.79-7.74 (m, 1H), 7.62-7.58 (m, 1H), 7.27-7.24 (m, 1H), 7.14-7.10 (m, 1H), 5.84 (dd, J = 9.6, 2.8 Hz, 1H), 4.22 (t, J = 6.0 Hz, 2H), 4.10-4.08 (m, 1H), 3.86-3.81 (m, 1H), 2.98 (t, J = 5.6 Hz, 2H), 2.68-2.59 (m, 4H), 2.22-2.15 (m, 2H), 1.86-1.74 (m, 8H).

Step 7

Under the atmosphere of nitrogen at room temperature, sodium sulfide nonahydrate (4 g, 16.20 mmol, purity: 98.0 %) and water (20 mL) were added to the solution of the compound 6-nitro-3-[(*E*)-2-[5-(2-pyrrolidin-1-ethoxy)-2-pyridyl]vinyl]-1-tetrahydropyran-2-ylindazole 31h (2.2 g, 4.63 mmol) in methanol (20 mL) and tetrahydrofuran (20 mL); the solution was heated to 60 °C and stirred to react for 2 h; after the reaction of the starting materials completed, as indicated by thin-layer chromatography (dichloromethane/methanol =10/1), the mixture was poured into water (80 mL) and filtered; the filtrate was extracted with dichloromethane (80 mL × 3); the combined organic layers were washed with saturated brine (100 mL), dried over anhydrous sodium sulfate and concentrated to obtain a crude product; and the crude product was purified by column chromatography (DCM /MeOH=20/1 ~ 3:1) to obtain a brown oily compound, 3-[(*E*)-2-[5-(2-pyrrolidin-1-ethoxy)-2-pyridyl]vinyl]-1-tetrahydropyran-2-yl-indazol-6-amine 31i (0.9 g, 2.05 mmol, yield: 44.36 %).

This product was verified by LCMS and HNMR.

For MS-ESI, the calculated value was $[M+H]^+$434.2, and the measurement was 434.1.

$^1$H NMR (400 MHz, $CDCl_3$) δ 8.36 (d, J = 2.8 Hz, 1H), 7.83 (d, J = 8.4 Hz, 1H), 7.65 (d, J = 16.4 Hz, 1H), 7.52-7.45 (m, 2H), 7.24 (dd, J = 8.4, 2.8 Hz, 1H), 6.76 (d, J = 1.6 Hz, 1H), 6.66 (dd, J = 8.4, 1.6 Hz, 1H), 5.60 (dd, J = 9.6, 2.8 Hz, 1H), 4.20 (t, J = 6.0 Hz, 2H), 4.09-4.07 (m, 1H), 3.78-3.20 (m, 1H), 2.96 (t, J = 5.6 Hz, 2H), 2.66 (s, 4H), 2.20 (s, 1H), 2.09-2.05 (m, 1H), 1.86-1.84 (m, 4H), 1.78-1.63 (m, 4H).

Step 8

At 0~5°C, sodium nitrite (221.8 mg, 3.21 mmol) in water (6 mL) was added to the solution of the compound 3-[(*E*)-2-[5-(2-pyrrolidin-1-ethoxy)-2-pyridyl]vinyl]-1-tetrahydropyran-2-yl-indazol-6-amine 31i (1.3 g, 2.92 mmol) in acetic acid (12 mL) and acetonitrile (15 mL); the solution was heated to stirred at 0~5 °C to react for 1 h; sodium iodide (884.8 mg, 5.90 mmol) and iodine (370.9 mg, 1.46 mmol) in water (6 mL) were added; and the mxture was stirred at 0 °C to react for 3 h. After the reaction of the starting materials completed, as indicated by thin-layer chromatography (dichloromethane/-methanol = 10/1), the mixture was poured into water (50 mL); the mixture was adjusted with sodium carbonate to a pH value of 8~9, and extracted with dichloromethane (50 mL × 3); the combined organic layers were dried over anhydrous sodium sulfate and concentrated to obtain a crude product; and the crude product was purified by column chromatography (DCM /MeOH=20/1) to obtain a red solid compound, 6-iodo-3-[(*E*)-2-[5-(2-pyrrolidin-1-methoxy)-2-pyridyl]vinyl]-1-tetrahydropyran-2-ylindazole 31j (0.85 g, 1.56 mmol, yield: 53.30 %).

This product was verified by LCMS and HNMR.

For MS-ESI, the calculated value was $[M+H]^+$545.1, and the measurement was 546.0.

$^1$H NMR (400 MHz, $CDCl_3$) δ 8.35 (d, J = 3.6 Hz, 1H), 8.02 (s, 1H), 7.78 (d, J = 8.4 Hz, 1H), 7.73 (d, J = 16.4 Hz, 1H), 7.55 (d, J = 2.0 Hz, 1H), 7.53-7.51 (m, 1H), 7.46 (d, J = 8.4 Hz, 1H), 7.33-7.31 (m, 1H), 5.69 (d, J = 6.8 Hz, 1H), 4.06 (s, 2H), 3.78 (s, 2H), 2.57 (s, 2H), 2.16 (s, 4H), 1.80 (s, 2H), 1.60 (s, 8H).

Step 9

Under the atmosphere of nitrogen at room temperature, cesium carbonate (418.9 mg, 1.29 mmol), 3-fluoro-N-methyl-2-mercaptobenzamide 31e (142.9 mg, 771.45 μmol) and 1,1'-bis(diphenylphosphino)ferrocene]dichloropalladium (233.3 mg, 321.44 μmol) were added to the solution of the compound 6-iodo-3-[(*E*)-2-[5-(2-pyrrolidin-1-methoxy)-2-pyridyl]vinyl]-1-tetrahydropyran-2-ylindazole 31j (350 mg, 642.88 μmol) in N,N-dimethylformamide (5 mL); the solution was heated to 80 °C and stirred to react for 5 h; after the reaction of the starting materials completed, as indicated by thin-layer chromatography (dichloromethane/methanol =10/1), the mixture was poured into water (30 mL) and extracted with ethyl acetate (20 mL × 3); the combined organic layers were washed with saturated brine (30 mL), dried over anhydrous

sodium sulfate and concentrated to obtain a crude product; and the crude product was purified by neutral alumina column chromatography (petroleum ether/ethyl acetate =1/1) to obtain a yellow solid compound, 3-fluoro-N-methyl-2-[3-[(E)-2-[5-(2-pyrrolidin-1-ethoxy)-2-pyridyl]vinyl]-1-tetrahydropyran-2-yl-indazol-6-yl]thiobenzamide 31k (250 mg, 415.47 μmol, yield: 64.63 %).

This product was verified by LCMS.

For MS-ESI, the calculated value was $[M+H]^+$602.3, and the measurement was 602.2.

Step 10

At room temperature, trifluoroacetic acid (473.7 mg, 4.15 mmol) was added to the solution of the compound 3-fluoro-*N*-methyl-2-[3-[(*E*)-2-[5-(2-pyrrolidin-1-ethoxy)-2-pyridyl]vinyl]-1-tetrahydropyran-2-yl-indazol-6-yl]thiobenzamide 31k (250 mg, 415.47 μmol) in dichloromethane (5 mL); under the atmosphere of nitrogen, the solution was stirred at 30 °C to react for 16 h; after the reaction of the starting materials completed, as indicated by thin-layer chromatography (dichloromethane/methanol =10/1), the mixture was poured into water (40 mL), adjusted with sodium carbonate to a PH value of 8~9, and extracted with ethyl acetate (30 mL × 3); the combined organic layers were washed with saturated brine (40 mL), dried over anhydrous sodium sulfate and concentrated to obtain a crude product; and the crude product was purified by preparative high-performance liquid chromatography to obtain a white solid compound, 3-fluoro-N-methyl-2-({3-[(E)-2-{5-[2-(pyrrolidin-1-yl)ethoxy]pyridin-2-yl}vinyl]-1H-indazol-6-yl}thio)benzamide I-33 (78 mg, 150.69 μmol, yield: 36.27 %).

This product was verified by LCMS, HNMR and CNMR.

For MS-ESI, the calculated value was $[M+H]^+$518.2, and the measurement was 518.1.

$^1$H NMR (400MHz, $CDCl_3$&CD3OD): δ (ppm) 8.26 (d, J = 2.8 Hz, 1H), 8.01 (d, J = 8.4 Hz, 1H), 7.67 (d, J = 16.8 Hz, 1H), 7.61 (d, J = 8.8 Hz, 1H), 7.54 - 7.48 (m, 2H), 7.40 (dd, J = 8.8, 2.8 Hz, 1H), 7.29-7.23 (m, 2H), 7.17 (dd, J = 8.4, 1.2 Hz, 1H), 7.11-7.06 (m, 1H), 4.23 (t, J = 5.6 Hz, 2H), 2.98 (t, J = 5.2 Hz, 2H), 2.87 (s, 3H), 2.71 (s, 4H), 1.86 (t, J = 3.2 Hz, 4H).

$^{13}$C NMR (100 MHz, $CDCl_3$&CD3OD) δ (ppm) 168.80, 161.97, 160.22, 154.36, 148.28, 137.19, 134.26, 134.19, 130.02, 129.21, 124.79, 122.07, 120.25, 117.44, 115.31, 115.11, 114.88, 113.64, 66.98, 54.61, 54.41, 25.96, 223.06.

I-34

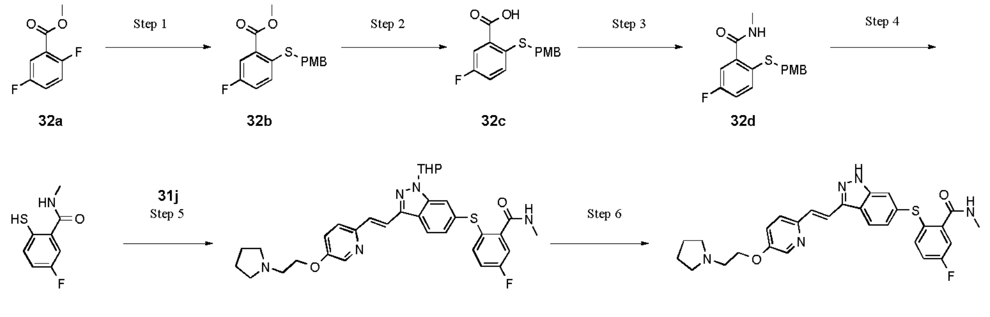


32a 32b 32c 32d 32e 32f I-34

Step 1

In a 250 mL single-mouth flask, 2,5-difluoromethyl benzoate 32a (5.00 g, 29.1 mmol) was dissolved in N,N-dimethylformamide (50 mL), followed by the addition of (4-methoxyphenyl)methanethiol (4.564 g, 29.1 mmol) and cesium carbonate (18.94 g, 58.2 mmol); the mixture was stirred at room temperature to react for 2 h. The reaction system was filtered to collect the filtrate, which was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 3: 1), and concentrated to obtain a white solid 5-fluoro-2-[(4-methoxyphenyl)methylthio] methyl benzoate 32b (4.9 g, 16 mmol, yield: 54.9 %).
MS (ESI) M/Z: 329.1 [M+Na+]

Step 2

In a 250 mL single-mouth flask, 5-fluoro-2-[(4-methoxyphenyl)methylthio]methyl benzoate 32b (4.9 g, 16 mmol) was dissolved in tetrahydrofuran (45 mL), followed by the addition of lithium hydroxide (2.017 g, 48 mmol) and water (15 mL); the mixture was heated to 50 °C and stirred to react for 5 h. The reaction system was filtered to collect the filtrate, which was concentrated under reduced pressure; the dilute hydrochloric acid (1 mol/L) was added to the system to adjust the pH

value to 5; and the reaction system was then filtered and dried to obtain a white solid 5-fluoro-2-[(4-methoxyphenyl) methylthio]benzoic acid 32c (3.94 g, 13.5 mmol, yield: 84.38 %).
MS (ESI) M/Z: 315.2 [M+Na+]

Step 3

In a 250 mL single-mouth flask, 5-fluoro-2-[(4-methoxyphenyl)methylthio]benzoic acid 32c (3.94 g, 13.5 mmol) was dissolved in N,N-dimethylformamide (38 mL), followed by the addition of methylamine (532 mg, 20.5 mmol) and N-methylmorpholine (4.095 g, 40.5 mmol), and then 1-hydroxybenzotriazole (2.78 g, 20.5 mmol) and 1-(3-dimethylaminopropyl)-3-ethylcarbodiimide hydrochloride (3.91 g, 20.5 mmol); the mixture was maintained at room temperature and stirred to react for 3 h. The reaction system was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 4: 1), and concentrated to obtain a white solid 5-fluoro-2-[(4-methoxyphenyl) methylthio]-N-methylbenzamide 32d (2.96 g, 9.7 mmol, yield: 71.9 %).

Step 4

In a 250 mL three-mouth flask,, 5-fluoro-2-[(4-methoxyphenyl)methylthio]-N-methylbenzamide 32d (2.96 g, 9.7 mmol) was dissolved in trifluoroacetic acid (32 mL); the solution was heated to 70 °C, and stirred to react for 3 h. After concentration under reduced pressure, the solid residue obtained was separated by the silica gel column chromatography (eluent: dichloromethane/methanol = 30: 1), and concentrated to obtain 1.2 g of white solid 5-fluoro-N-methyl-2-thiobenzamide 32e (1.1 g, 5.95 mmol, yield: 61.3 %).

MS (ESI) M/Z: 186.1[M+H+]
$^1$H NMR (400 MHz, MeOD) δ 7.40 (dd, J = 8.8, 5.2 Hz, 1H), 7.23 (dd, J = 8.8, 2.8 Hz, 1H), 7.08 (td, J = 8.4, 2.8 Hz, 1H), 2.89 (s, 3H).

Step 5

Under the atmosphere of nitrogen at room temperature, cesium carbonate (314.2 mg, 964.32 μmol), 5-fluoro-N-methyl-2-mercaptobenzamide 32e (148.8 mg, 803.60 μmol, it was synthesized according to the synthesis method for Compound 31e by taking 2,5-difluoromethyl benzoate as a starting material) and 1,1'-bis(diphenylphosphino)ferrocene] dichloropalladium (350 mg, 482.32 μmol) were added to the solution of the compound 6-iodo-3-[(*E*)-2-[5-(2-pyrrolidin-1-methoxy)-2-pyridyl]vinyl]-1-tetrahydropyran-2-ylindazole 31j (350 mg, 642.88 μmol) in N,N-dimethylformamide (5 mL); the solution was heated to 80 °C and stirred to react for 5 h; after the reaction of the starting materials completed, as indicated by thin-layer chromatography (dichloromethane/methanol =10/1), the mixture was poured into water (30 mL) and extracted with ethyl acetate (20 mL × 3); the combined organic layers were washed with saturated brine (30 mL), dried over anhydrous sodium sulfate and concentrated to obtain a crude product; and the crude product was purified by neutral alumina column chromatography (petroleum ether/ethyl acetate =1/1) to obtain a yellow solid compound, 5-fluoro-*N*-methyl-2-[3-[(*E*)-2-[5-(2-pyrrolidin-1-ethoxy)-2-pyridyl]vinyl]-1-tetrahydropyran-2-yl-indazol-6-yl]thiobenzamide 32f (250 mg, 415.47 μmol, yield: 64.63 %).
This product was verified by LCMS.
For MS-ESI, the calculated value was $[M+H]^+$602.3, and the measurement was 602.2.

Step 6

At room temperature, trifluoroacetic acid (473.7 mg, 4.15 mmol) was added to the solution of the compound 5-fluoro-*N*-methyl-2-[3-[(*E*)-2-[5-(2-pyrrolidin-1-ethoxy)-2-pyridyl]vinyl]-1-tetrahydropyran-2-yl-indazol-6-yl] thiobenzamide 32f (250 mg, 415.47 μmol) in dichloromethane (10 mL); under the atmosphere of nitrogen, the solution was stirred at 30 °C to react for 16 h; after the reaction of the starting materials completed, as indicated by thin-layer chromatography (dichloromethane/methanol =10/1), the mixture was poured into water (40 mL), adjusted with sodium carbonate to a PH value of 8~9, and extracted with ethyl acetate (30 mL × 3); the combined organic layers were washed with saturated brine (40 mL), dried over anhydrous sodium sulfate and concentrated to obtain a crude product; and the crude product was purified by preparative high-performance liquid chromatography to obtain a white solid compound, 5-fluoro-N-methyl-2-({3-[(E)-2-{5-[2-(pyrrolidin-1-yl)ethoxy]pyridin-2-yl}vinyl]-1H-indazol-6-yl}thio)benzamide I-34 (33 mg, 63.75 μmol, yield: 15.35 %).
This product was verified by LCMS, HNMR and CNMR.

For MS-ESI, the calculated value was $[M+H]^+$518.2, and the measurement was 518.0.

$^1$H NMR (400MHz, $CDCl_3$&CD3OD): δ (ppm) 8.28 (d, J = 2.4 Hz, 1H), 8.13 (d, J = 8.4 Hz, 1H), 7.74 - 7.70 (m, 2H), 7.68-7.63 (m, 1H), 7.57 - 7.50 (m, 2H), 7.44 (dd, J = 8.8, 2.8 Hz, 1H), 7.28 (d, J = 9.6 Hz, 1H), 6.97 - 6.93 (m, 1H), 6.70 (dd, J = 10.0, 2.4 Hz, 1H), 4.26 (t, J = 5.2 Hz, 2H), 3.03 (s, 2H), 2.91 (s, 3H), 2.76 (s, 4H), 1.89 (s, 4H).

$^{13}$C NMR (100 MHz, $CDCl_3$&CD3OD) δ (ppm) 169.22, 162.37, 154.39, 148.26, 142.24, 137.19, 131.46, 129.35, 126.55, 122.41, 122.07, 121.84, 120.94, 115.97, 115.73, 112.59, 112.37, 67.00, 54.61, 54.40, 26.00, 23.06.

I-35

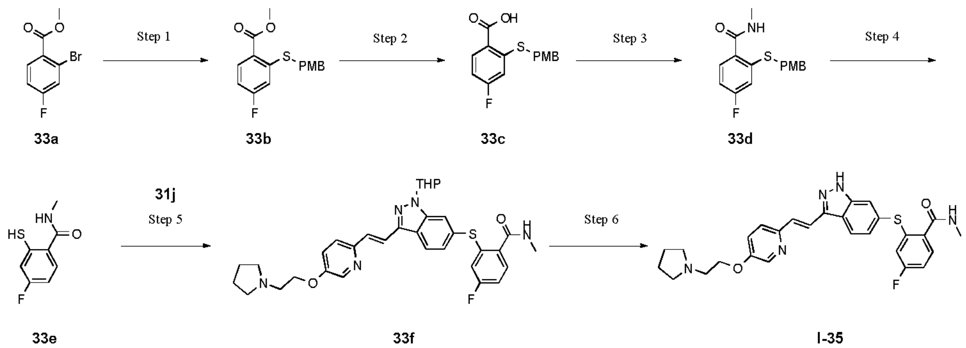

33a 33b 33c 33d
33e 33f I-35

Step 1

In a 250 mL single-mouth flask, 2-bromo-4-fluoromethyl benzoate 33a (5.00 g, 21.5 mmol) was dissolved in *N,N*-dimethylformamide (50 mL), followed by the addition of PMBSH (3.98 g, 25.8 mmol) and cesium carbonate (10.51 g, 32.3 mmol), and then Pd(dppf)$Cl_2$(0.79 g, 1.08 mmol); the mixture was stirred at 120 °C to react overnight. Aftertreatment: The reaction system was filtered to collect the filtrate, which was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 3: 1), and concentrated to obtain 5 g of white solid 4-fluoro-2-[(4-methoxyphenyl)methylmercapto]methyl benzoate 33b.
MS (ESI) M/Z: 329.1[M+Na+]

Step 2

In a 250 mL single-mouth flask, 4-fluoro-2-[(4-methoxyphenyl)methylthio]methyl benzoate 33b (4.50 g, 14.7 mmol) was dissolved in tetrahydrofuran (45 mL), followed by the addition of lithium hydroxide (1.853 g, 44.1 mmol) and water (15 mL); the mixture was heated to 50 °C and stirred to react for 5 h. The reaction system was filtered to collect the filtrate, which was concentrated under reduced pressure; the dilute hydrochloric acid (1 mol/L) was added to the system to adjust the pH value to 5; and the reaction system was then filtered and dried to obtain a white solid 4-fluoro-2-[(4-methoxyphenyl)methylthio]benzoic acid 33c (3.80 g, 13.0 mmol, yield: 88.4 %).
MS (ESI) M/Z: 315.2 [M+Na+]

Step 3

In a 250 mL single-mouth flask,4-fluoro-2-[(4-methoxyphenyl)methylthio]benzoic acid 33c (3.80 g, 13.0 mmol) was dissolved in N,N-dimethylformamide (38 mL), followed by the addition of methylamine (506 mg, 19.5 mmol) and N-methylmorpholine (3.943 g, 39.0 mmol), and then 1-hydroxybenzotriazole (2.64 g, 19.5 mmol) and 1-(3-dimethylaminopropyl)-3-ethylcarbodiimide hydrochloride (3.72 g, 19.5 mmol); the mixture was maintained at room temperature and stirred to react for 3 h. The reaction system was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 4: 1), and concentrated to obtain a white solid 4-fluoro-2-[(4-methoxyphenyl) methylthio]-N-methylbenzamide 33d (2.95 g, 9.66 mmol, yield: 74.3 %).

Step 4

In a 250 mL three-mouth flask,, 4-fluoro-2-[(4-methoxyphenyl)methylthio]-N-methylbenzamide 33d (2.95 g, 9.66

mmol) was dissolved in trifluoroacetic acid (32 mL); the solution was heated to 70 °C, and stirred to react for 3 h. After concentration under reduced pressure, the solid residue obtained was separated by the silica gel column chromatography (eluent: dichloromethane/methanol = 30: 1), and concentrated to obtain 1.2 g of white solid 4-fluoro-N-methyl-2-thiobenzamide 33e (1.05 g, 5.68 mmol, yield: 58.8 %).

MS (ESI) M/Z: 186.1[M+H+]
$^1$H NMR (400 MHz, CD3OD) δ 7.50 (dd, J = 8.6, 5.8 Hz, 1H), 7.18 (dd, J = 9.6, 2.5 Hz, 1H), 7.01 - 6.76 (m, 1H), 2.89 (s, 3H).

Step 5

Under the atmosphere of nitrogen at room temperature, cesium carbonate (359.1 mg, 1.10 mmol), 4-fluoro-N-methyl-2-thiobenzamide 33e (122.5 mg, 661.25 μmol) and 1,1'-bis(diphenylphosphino)ferrocene]dichloropalladium (199.9 mg, 275.52 μmol) were added to the solution of the compound 6-iodo-3-[(*E*)-2-[5-(2-pyrrolidin-1-methoxy)-2-pyridyl]vinyl]-1-tetrahydropyran-2-ylindazole 31j (300 mg, 551.04 μmol) in N,N-dimethylformamide (5 mL); the solution was heated to 80 °C and stirred to react for 5 h; after the reaction of the starting materials completed, as indicated by thin-layer chromatography (dichloromethane/methanol =10/1), the mixture was poured into water (30 mL) and extracted with ethyl acetate (20 mL × 3); the combined organic layers were washed with saturated brine (30 mL), dried over anhydrous sodium sulfate and concentrated to obtain a crude product; and the crude product was purified by neutral alumina column chromatography (petroleum ether/ethyl acetate =1/1) to obtain a yellow solid compound, 4-fluoro-*N*-methyl-2-[3-[(*E*)-2-[5-(2-pyrrolidin-1-ethoxy)-2-pyridyl]vinyl]-1-tetrahydropyran-2-yl-indazol-6-yl]thiobenzamide 33f (250 mg, 415.47 μmol, yield: 75.40 %).

This product was verified by LCMS.

For MS-ESI, the calculated value was $[M+H]^+$602.3, and the measurement was 602.1.

Step 6

At room temperature, trifluoroacetic acid (473.6 mg, 4.15 mmol) was added to the solution of the compound 4-fluoro-*N*-methyl-2-[3-[(*E*)-2-[5-(2-pyrrolidin-1-ethoxy)-2-pyridyl]vinyl]-1-tetrahydropyran-2-yl-indazol-6-yl]thiobenzamide 33f (250 mg, 415.47 μmol) in dichloromethane (5 mL); under the atmosphere of nitrogen, the solution was stirred at 30 °C to react for 16 h; after the reaction of the starting materials completed, as indicated by thin-layer chromatography (dichloromethane/methanol =10/1), the mixture was poured into water (40 mL), adjusted with sodium carbonate to a PH value of 9, and extracted with ethyl acetate (30 mL × 3); the combined organic layers were washed with saturated brine (40 mL), dried over anhydrous sodium sulfate and concentrated to obtain a crude product; and the crude product was purified by preparative high-performance liquid chromatography to obtain a yellow solid compound, 4-fluoro-N-methyl-2-(3-[(E)-2-{5-[2-(pyrrolidin-1-yl)ethoxy]pyridin-2-yl}vinyl]-1H-indazol-6-yl}thio)benzamide I-35 (20 mg, 38.64 μmol, yield: 9.30 %).

This product was verified by LCMS, HNMR and CNMR.

For MS-ESI, the calculated value was $[M+H]^+$518.2, and the measurement was 518.1.

$^1$H NMR (400MHz, $CDCl_3$&CD3OD): δ (ppm) δ 8.29 (d, J = 2.8 Hz, 1H), 8.13 (d, J = 8.4 Hz, 1H), 7.75 - 7.70 (m, 2H), 7.68-7.63 (m, 1H), 7.57-7.54 (m, 1H), 7.53 - 7.50 (m, 1H), 7.44 (dd, J = 8.8, 2.8 Hz, 1H), 7.28 (d, J = 10.0 Hz, 1H), 6.98-6.93 (m, 1H), 6.71 (dd, J = 9.6, 2.4 Hz, 1H), 4.27 (t, J = 5.6 Hz, 2H), 3.05 (t, J = 5.2 Hz, 2H), 2.91 (s, 3H), 2.78 (s, 4H), 1.89 (s, 4H).

$^{13}$C NMR (100 MHz, $CDCl_3$&CD3OD) δ (ppm) 170.59, 166.30, 163.79, 155.74, 149.73, 144.24, 143.69, 14.18, 142.10, 138.66, 136.12, 132.82, 132.34, 131.18, 131.09, 128.00, 125.86, 123.53, 123.14, 122.40, 117.94, 117.45, 117.21, 114.07, 113.85, 68.36, 56.02, 55.87, 30.93, 27.58, 24.52.

I-36

Step 1
Step 2
Step 3
Step 4

Step 5
Step 6

Step 1

In a 250 mL single-mouth flask, 2,6-difluoromethyl benzoate 34a (5.00 g, 29.1 mmol) was dissolved in N,N-dimethylformamide (50 mL), followed by the addition of (4-methoxyphenyl)methanethiol (4.564 g, 29.1 mmol) and cesium carbonate (18.94 g, 58.2 mmol); the mixture was stirred at room temperature to react for 2 h. The reaction system was filtered to collect the filtrate, which was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 3:1), and concentrated to obtain a white solid 2-fluoro-6-[(4-methoxyphenyl)methylthio] methyl benzoate 34b (4.5 g, 14.7 mmol, yield: 54.9 %).
MS (ESI) M/Z: 329.1 [M+Na+]

Step 2

In a 250 mL single-mouth flask, 2-fluoro-6-[(4-methoxyphenyl)methylthio]methyl benzoate 34b (4.50 g, 14.7 mmol) was dissolved in tetrahydrofuran (45 mL), followed by the addition of lithium hydroxide (1.853 g, 44.1 mmol) and water (15 mL); the mixture was heated to 50 °C and stirred to react for 5 h. The reaction system was filtered to collect the filtrate, which was concentrated under reduced pressure; the dilute hydrochloric acid (1 mol/L) was added to the system to adjust the pH value to 5; and the reaction system was then filtered and dried to obtain a white solid 2-fluoro-6-[(4-methoxyphenyl)methylthio]benzoic acid 34c (3.80 g, 13.0 mmol, yield: 88.4 %).
MS (ESI) M/Z: 315.2 [M+Na+]

Step 3

In a 250 mL single-mouth flask, 2-fluoro-6-[(4-methoxyphenyl)methylthio]benzoic acid 34c (3.80 g, 13.0 mmol) was dissolved in N,N-dimethylformamide (38 mL), followed by the addition of methylamine (506 mg, 19.5 mmol) and N-methylmorpholine (3.943 g, 39.0 mmol), and then 1-hydroxybenzotriazole (2.64 g, 19.5 mmol) and 1-(3-dimethylaminopropyl)-3-ethylcarbodiimide hydrochloride (3.72 g, 19.5 mmol); the mixture was maintained at room temperature and stirred to react for 3 h. The reaction system was diluted with ethyl acetate (200 mL), and washed with water (200 mL × 3). Liquid separation was conducted to collect an organic phase, which was dried over anhydrous sodium sulfate. After concentration under reduced pressure, the solid residue obtained was separated by the silica gel column chromatography (eluent: petroleum ether: ethyl acetate = 4: 1), and concentrated to obtain a white solid 2-fluoro-6-[(4-methoxyphenyl) methylthio]-N-methylbenzamide 34d (2.95 g, 9.66 mmol, yield: 74.3 %).

Step 4

In a 250 mL three-mouth flask,, 2-fluoro-6-[(4-methoxyphenyl)methylthio]-N-methylbenzamide 34d (2.95 g, 9.66 mmol) was dissolved in trifluoroacetic acid (32 mL); the solution was heated to 70 °C, and stirred to react for 3 h. After concentration under reduced pressure, the solid residue obtained was separated by the silica gel column chromatography (eluent: dichloromethane/methanol = 30: 1), and concentrated to obtain 1.2 g of white solid 2-fluoro-N-methyl-6-mercaptobenzamide 34e (1.05 g, 5.68 mmol, yield: 58.8 %).

MS (ESI) M/Z: 186.0[M+H+]
$^1$H NMR (400 MHz, CD3OD) δ 7.34 - 7.32 (m, 1H), 7.24 - 7.20 (m, 2H), 2.92 (s, 3H).

Step 5

Under the atmosphere of nitrogen at room temperature, cesium carbonate (314.2 mg, 964.32 μmol), 2-fluoro-N-methyl-6-mercaptobenzamide 34e (148.8 mg, 803.60 μmol, it was synthesized according to the synthesis method or Compound 31e by taking 2,6-difluoromethyl benzoate as a starting material) and 1,1'-bis(diphenylphosphino)ferrocene] dichloropalladium (350 mg, 482.32 μmol) were added to the solution of the compound 6-iodo-3-[(*E*)-2-[5-(2-pyrrolidin-1-methoxy)-2-pyridyl]vinyl]-1-tetrahydropyran-2-ylindazole 31j (350 mg, 642.88 μmol) in N,N-dimethylformamide (8 mL); the solution was heated to 80 °C and stirred to react for 5 h; after the reaction of the starting materials completed, as indicated by thin-layer chromatography (dichloromethane/methanol =10/1), the mixture was poured into water (25 mL) and extracted with dichloromethane (25 mL × 3); the combined organic layers were washed with water (20 mL × 2), dried over anhydrous sodium sulfate and concentrated to obtain a crude product; and the crude product was purified by preparative thin-layer chromatography (dichloromethane/methanol = 20/1) to obtain a red solid compound, 2-fluoro-*N*-methyl-6-[3-[(*E*)-2-[5-(2-pyrrolidin-1-ethoxy)-2-pyridyl]vinyl]-1-tetrahydropyran-2-yl-indazol-6-yl]thiobenzamide 34f

(197.9 mg, 328.88 μmol, yield: 51.16 %).

This product was verified by LCMS.

For MS-ESI, the calculated value was $[M+H]^+$602.3, and the measurement was 602.2.

Step 6

At room temperature, trifluoroacetic acid (1.2 g, 10.42 mmol) was added to the solution of the compound 2-fluoro-*N*-methyl-6-[3-[(*E*)-2-[5-(2-pyrrolidin-1-ethoxy)-2-pyridyl]vinyl]-1-tetrahydropyran-2-yl-indazol-6-yl]thiobenzamide 34f (197.9 mg, 328.88 μmol) in dichloromethane (5 mL); under the atmosphere of nitrogen, the solution was stirred at room temperature to react for 16 h; after the reaction of the starting materials completed, as indicated by thin-layer chromatography (dichloromethane/methanol =10/1), the mixture was poured into water (5 mL), adjusted with sodium carbonate to a PH value of 8~9, and extracted with dichloromethane (5 mL × 3); the combined organic layers were wached with water (5 mL × 2), dried over anhydrous sodium sulfate and concentrated to obtain a crude product; and the crude product was purified by preparative high-performance liquid chromatography to obtain a deep yellow solid compound, 2-fluoro-N-methyl-6-({3-[(E)-2-{5-[2-(pyrrolidin-1-yl)ethoxy]pyridin-2-yl}vinyl]-1H-indazol-6-yl}thio)benzamide I-36 (18 mg, 34.77 μmol, yield: 10.57 %).

This product was verified by LCMS, HNMR and CNMR.

For MS-ESI, the calculated value was $[M+H]^+$518.2, and the measurement was 518.1.

$^1$H NMR (400 MHz, CD3OD) δ 8.29 (d, J = 2.8 Hz, 1H), 8.09 (d, J = 8.4 Hz, 1H), 7.74 - 7.64 (m, 3H), 7.56-7.45 (m, 2H), 7.37-7.31 (m, 1H), 7.26 (d, J = 8.4 Hz, 1H), 7.12-7.05 (m, 2H), 4.27 (t, J = 5.2 Hz, 2H), 3.03 (t, J = 6.0 Hz, 2H), 2.89 (s, 3H), 2.77 (s, 4H), 1.89 (s, 4H).

$^{13}$C NMR (100 MHz, CD3OD&CDCl$_3$) δ: 165.56, 160.40, 154.46, 148.23, 142.74, 142.13, 137.16, 132.81,130.84, 130.75, 129.19, 126.78, 125.41, 122.41, 122.02, 121.64, 121.39, 120.47, 114.55, 113.84, 113.62, 66.96, 54.54, 54.28, 25.55, 22.96.

I-29

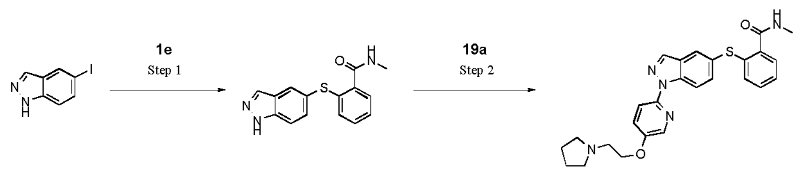


35a 35b I-29

Step 1

Under the atmosphere of nitrogen at room temperature, cesium carbonate (3.90 g, 11.96 mmol) and 1,1'-bis(diphenylphosphino)ferrocene]dichloropalladium (0.88 g, 1.20 mmol) were added to the solution of 5-iodo-1*H*-indazole 35a (1.02 g, 4.19 mmol) and N-methyl-2-mercaptobenzamide 1e (1 g, 5.98 mmol) in N,N-dimethylformamide (10 mL); the solution was heated to 80 °C and stirred to react for 2 h; after the reaction of the starting materials completed, as indicated by thin-layer chromatography (dichloromethane/methanol =20/1), the mixture was poured into water (30 mL) and extracted with ethyl acetate (10 mL × 3); the combined organic layers were washed with saturated brine (20 mL), dried over anhydrous sodium sulfate and concentrated to obtain a crude product; and the crude product was purified by column chromatography (petroleum ether/ethyl acetate =2/1-0/1) to obtain a yellow oily compound, 2-(1*H*-indazol-5-ylthio)-*N*-methylbenzamide 35b (0.8 g, yield: 47.2 %).

This product was verified by LCMS.

For MS-ESI, the calculated value was $[M+H]^+$284.1, and the measurement was 284.1.

Step 2

Under the atmosphere of nitrogen at room temperature, cesium carbonate (1.15 g, 3.53 mmol), 1,10-phenanthroline (95 mg, 0.53 mmol) and cuprous iodide (101 mg, 0.53 mmol) were added to the solution of the compound 2-(1H-indazol-5-ylthio)-*N*-methylbenzamide 35b(0.5 g, 1.76 mmol) and 2-bromo-5-(2-pyrrolidin-1-ylethoxy)pyridine 19a (478 mg, 1.76 mmol) in dimethyl sulfoxide (20 mL); the solution was heated to 100 °C and stirred to react for 16 h; and the product was indicated by thin-layer chromatography (dichloromethane/methanol =20/1) and LCMS. The mixture was poured into water (80 mL) and extracted with ethyl acetate (20 mL × 3); the combined organic layers were washed with saturated brine (30 mL), dried over anhydrous sodium sulfate and concentrated to obtain a crude product; and the crude product was purified by preparative high-performance liquid chromatography to obtain a white solid compound, N-methyl-2-[(1-{5-[2-(pyrrolidin-1-yl)ethoxy]pyridin-2-yl}-1H-indazol-5-yl)thio]benzamide I-29 (18 mg, yield: 2.2 %).
This product was verified by LCMS, HNMR and CNMR.
For MS-ESI, the calculated value was $[M+H]^+$474.2, and the measurement was 474.2.
$^1$H NMR (400 MHz, CD3OD) δ: 8.73 (d, 1H, J=8.8Hz), 8.23 (d, 1H, J=2.8Hz), 8.16 (d, 1H, J= 0.4Hz), 7.93-7.98 (m, 2H), 7.58-7.60 (m, 1H), 7.55 (dd, 1H, J=8.8, 1.6Hz), 7.46 (dd, 1H, J =8.8, 2.8Hz), 7.21-7.23 (m, 2H), 7.01-7.04 (m, 1H), 6.30 (s, 1H), 4.23 (t, 2H, J=6.0Hz), 3.04 (d, 3H, J=4.8Hz), 2.98 (t, 2H, J=6.0Hz), 2.66-2.68 (m, 4H), 1.84-1.88 (m, 4H).
$^{13}$C NMR (100 MHz, $CDCl_3$) δ: 168.68, 152.93, 147.72, 138.17, 137.07, 135.65, 135.04, 134.32, 133.06, 130.66, 129.81, 128.41, 126.67, 126.23, 125.95, 125.08, 115.85, 114.51, 68.05, 55.04, 54.77, 26.80, 23.52.

I-21

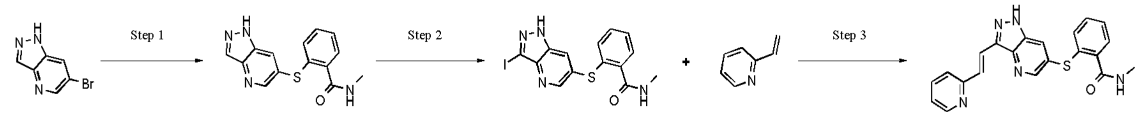


36a 36b 36c 36d I-21

Step 1

Under the atmosphere of nitrogen at room temperature, the compound *N*-methyl-2-sulfanylbenzamide 1e (2.03 g, 12.12 mmol), cesium carbonate (6.58 g, 20.20 mmol) and 1,1'-bis(diphenylphosphino)ferrocene]dichloropalladium (1.48 g, 2.02 mmol) were added to the solution of 6-bromo-1*H*-pyrazolo[4,3-b]pyridine 36a (2 g, 10.10 mmol) in N,N-dimethylformamide (20 mL); the solution was 100 °C and stirred to react for 1 h; after the reaction of the starting materials completed, as indicated by thin-layer chromatography (dichloromethane/methanol =20/1), the mixture was poured into water (40 mL) and extracted with ethyl acetate (20 mL × 3); the combined organic layers were washed with saturated brine (20 mL), dried over anhydrous sodium sulfate and concentrated to obtain a crude product; and the crude product was purified by column chromatography (dichloromethane/methanol =10/1) to obtain a brown solid compound, *N*-methyl-2-(1*H*-pyrazolo[4,3-b]pyridin-6-ylthio)benzamide 36b (1.59 g, 5.59 mmol, yield: 55.37 %).
This product was verified by LCMS and HNMR.
For MS-ESI, the calculated value was $[M+H]^+$285.1, and the measurement was 285.0.
$^1$H NMR (400MHz, DMSO-d6): δ (ppm) 13.43 (s, 1H), 8.44 (d, J = 2.0 Hz, 2H), 8.33 (s, 1H), 8.08 (s, 1H), 7.53-7.50 (m, 1H), 7.37-7.26 (m, 2H), 7.04-6.96 (m, 1H), 2.77 (d, J = 4.6 Hz, 3H).

Step 2

At room temperature, iodine (2.43 g, 9.57 mmol) and potassium carbonate (1.56 g, 11.25 mmol) were added to the solution of the compound *N*-methyl-2-(1*H*-pyrazolo[4,3-b]pyridin-6-ylthio)benzamide 36b (1.6 g, 5.63 mmol) in N,N-dimethylformamide (5 mL); the solution was stirred at 25 °C to react for 3.0 h. After the thin-layer chromatography (dichloromethane/methanol = 20/1) showed the complete reaction of the starting materials, the mixture was poured into water (10 mL) and extracted with ethyl acetate (10 mL × 3), and the organic layers were combined, washed with saturated brine (20 mL), dried over anhydrous sodium sulfate and concentrated to obtain a crude product, which was a brown solid 2-[(3-iodo-1H-pyrazolyl[4,3-b]pyridin-6-yl)thio]-N-methylbenzamide 36c (2.1 g, 5.12 mmol, yield: 90.97 %).
This product was verified by LCMS and HNMR.
For MS-ESI, the calculated value was $[M+H]^+$411.0, and the measurement was 410.9.
$^1$H NMR (400MHz, DMSO-d6): δ (ppm) 13.84 (s, 1H), 8.45 (d, J = 1.6 Hz, 2H), 8.06 (d, J = 1.6 Hz, 1H), 7.54-7.49 (m, 1H), 7.42-7.24 (m, 2H), 7.08-6.98 (m, 1H), 2.76 (d, J = 4.6 Hz, 3H).

Step 3

Under the protection of nitrogen at room temperature, 2-vinylpyridine 36d (38.44 mg, 365.65 μmol), diisopropylethylamine (63.03 mg, 487.53 μmol), palladium acetate (21.89 mg, 97.51 μmol) and 1,1'-bis(diphenylphosphine) ferrocene (16.56 mg, 195.01 μmol) were added to the solution of 2-[(3-iodo-1H-pyrazolyl[4,3-b]pyridin-6-yl)thio]-N-methylbenzamide 36c (100 mg, 243.76 μmol) in N,N-dimethylformamide (5 mL); the reaction liquid was heated to 100 °C and stirred to react for 16 h. After the reaction of the starting materials completed, as indicated by thin-layer chromatography (dichloromethane/methanol = 10 /1), the mixture was poured into water (20 mL) and extracted with ethyl acetate (10 mL × 3); the combined organic layers were washed with saturated brine (50 mL), dried over anhydrous sodium sulfate and concentrated to obtain a crude product; and the crude product was purified by preparative high-performance liquid chromatography to obtain a white solid compound, N-methyl-2-({3-[(E)-2-(pyridin-2-yl)vinyl]-1H-pyrazolo[4, 3-b]pyridin-6-yl}thio)benzamide I-21 (15.8 mg, 40.78 μmol, yield: 16.73).

This product was verified by LCMS, HNMR and CNMR.

For MS-ESI, the calculated value was $[M+H]^+$388.1, and the measurement was 388.1.

$^1H$ NMR (400MHz, DMSO-d6): δ (ppm) 13.55 (s, 1H), 8.62 (d, J = 4.4 Hz, 1H), 8.55 (s, 1H), 8.46 (d, J = 4.4 Hz, 1H), 8.20-8.07 (m, 2H), 7.93 (d, J = 16.4 Hz, 1H), 7.81 (t, J = 7.6 Hz, 1H), 7.65 (d, J = 7.6 Hz, 1H), 7.53 (d, J = 8.4 Hz, 1H), 7.39-7.26 (m, 3H), 7.07 (d, J = 7.6 Hz, 1H), 2.78 (d, J = 4.4 Hz, 3H).

$^{13}C$ NMR (100MHz, DMSO-d6): δ (ppm) 168.26, 155.29, 150.13, 149.16, 142.32, 138.48, 137.50, 137.42, 135.41, 134.52, 132.05, 131.06, 130.67, 129.41, 128.39, 127.05, 123.41, 123.27, 123.12, 122.84, 26.58.

I-26

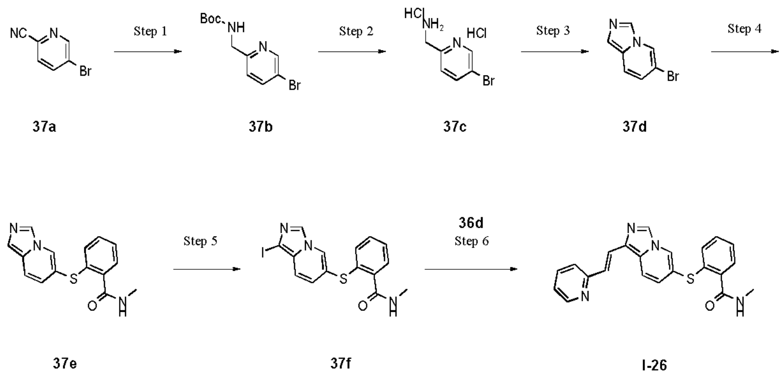


37a 37b 37c 37d

37e 37f I-26

Step 1

At 0°C, di-tert butyl dicarbonate (2.39 g, 10.93 mmol) and nickel dichloride (129.88 mg, 546.43 μmol) were added to the solution of 5-bromopyridin-2-nitrile 37a (1 g, 5.46 mmol) in methanol (10 mL); sodium borohydride (1.45 g, 38.25 mmol) was slowly added in portions within 2.0 h; and the resulting mixture was stirred at 25°C to react for 16 h. After the reaction of the starting materials completed, as indicated by thin-layer chromatography (petroleum ether/ethyl acetate = 2 /1), the mixture was poured into water (50 mL) and extracted with ethyl acetate (10 mL × 3); the combined organic layers were washed with saturated brine (20 mL), dried over anhydrous sodium sulfate and concentrated to obtain a crude product; and the crude product was purified by column chromatography (petroleum ether/ethyl acetate = 10 /1~5/1) to obtain a white solid compound, N-[(5-bromo-2-pyridyl)methyl]aminotert-butyl formate 37b (700 mg, 2.44 mmol, yield: 44.61 %).

This product was verified by LCMS and HNMR.

For MS-ESI, the calculated value was $[M+H]^+$287.0, and the measurement was 286.9.

$^1H$ NMR (400 MHz, $CDCl_3$) δ 8.60 (d, J = 2.0 Hz, 1H), 7.79 (dd, J = 8.4, 2.4Hz, 1H), 7.21 (d, J = 8.4 Hz, 1H), 5.51 (s, 1H), 4.40 (d, J = 5.4 Hz, 2H), 1.47 (s, 9H).

Step 2

The compound N-[(5-bromo-2-pyridyl)methyl]aminotert-butyl formate 37b (500 mg, 1.74 mmol) was dissolved in a hydrochloric acid/methanol solution (15 mL); the solution was stirred at 25°C to react for 2.0 h. After LCMS showed the complete reaction of the starting materials, the reaction mixture was concentrated under reduced pressure, to obtain a white solid compound (5-bromo-2-pyridyl)methylamine dihydrochloride 37c (290 mg, 1.55 mmol, yield: 89.05 %).

This product was verified by LCMS and HNMR.

For MS-ESI, the calculated value was $[M+H]^+$188.9, and the measurement was 189.0.

$^1H$ NMR (400MHz, DMSO-d6): δ 8.74 (d, J = 2.0 Hz, 1H), 8.66 (s, 3H), 8.14 (dd, J = 8.4, 2.4 Hz, 1H), 7.55 (d, J = 8.4 Hz, 1H), 4.13-4.17 (m, 2H).

Step 3

The compound (5-bromo-2-pyridyl)methylamine dihydrochloride 37c (400 mg, 1.79 mmol) was dissolved in acetic anhydride (2 mL) and formic acid (1.6 mL); the solution was stirred at 100°C to react for 16 h. After the thin-layer chromatography (petroleum ether/ethyl acetate = 2/1) showed the complete reaction of the starting materials, the reaction mixture was concentrated under reduced pressure, added to the saturated aqueous solution of sodium bicarbonate (10 mL) and extracted with ethyl acetate (10 mL × 3), and the organic layers were combined, washed with saturated brine (20

mL), dried over anhydrous sodium sulfate and concentrated to obtain a yellow solid compound 6-bromoimidazo[1,5-a] pyridine 37d (300 mg, 1.52 mmol, yield: 85.07 %).

This product was verified by LCMS and HNMR.

For MS-ESI, the calculated value was $[M+H]^+$197.0, and the measurement was 197.0.

$^1$H NMR (400 MHz, DMSO-d6) δ 9.40 (s, 1H), 8.96 (s, 1H), 8.05 (s, 1H), 7.80 (d, J = 9.6 Hz, 1H), 7.26 (dd, J =10.4Hz ,9.6 Hz, 1H).

Step 4

At room temperature, the compound *N*-methyl-2-mercaptobenzamide 1e (254.62 mg, 1.52 mmol), cesium carbonate (992.18 mg, 3.05 mmol) and 1,1'-bis(diphenylphosphino)ferrocene]dichloropalladium (222.82 mg, 304.52 μmol) were added to the solution of the compound 6-bromoimidazo[1,5-a]pyridine 37d (0.3 g, 1.52 mmol) in N,N-dimethylformamide (10 mL); the solution was 80 °C and stirred to react for 2 h; after the reaction of the starting materials completed, as indicated by thin-layer chromatography (dichloromethane/methanol =15/1), the mixture was poured into water (40 mL) and extracted with ethyl acetate (20 mL × 3); the combined organic layers were washed with saturated brine (20 mL), dried over anhydrous sodium sulfate and concentrated to obtain a crude product; and the crude product was purified by preparative high-performance liquid chromatography to obtain a yellow solid compound, 2-imidazo[1,5-a] pyridin-6-ylthio-*N*-methylbenzamide 37e (87 mg, 307.04 μmol, yield: 20.17 %).

This product was verified by LCMS and HNMR.

For MS-ESI, the calculated value was $[M+H]^+$284.1, and the measurement was 284.1.

$^1$H NMR (400 MHz, $CDCl_3$) δ 8.20 (d, J = 0.8 Hz, 1H), 8.13 (s, 1H), 7.54 (dd, J = 7.6, 1.6 Hz, 1H), 7.42 - 7.45 (m, 2H), 7.29-7.32 (m, 1H), 7.22-7.26 (m, 1H), 7.14 (dd, J = 7.6, 1.2 Hz, 1H), 6.66 (dd, J = 9.2, 1.2 Hz, 1H), 6.21 (s, 1H), 3.04 (d, J = 5.2 Hz, 3H).

Step 5

At room temperature, iodine (167.50 mg, 659.97 μmol) and potassium carbonate (95.60 mg, 776.43 μmol) were added to the solution of the compound 2-imidazo[1,5-a]pyridin-6-ylthio-*N*-methylbenzamide 37e (110 mg, 388.22 μmol) in N,N-dimethylformamide (5 mL); the solution was stirred at 25 °C to react for 3.0 h. After the reaction of the starting materials completed, as indicated by thin-layer chromatography (dichloromethane/methanol = 20/1), the mixture was poured into water (10 mL) and extracted with ethyl acetate (10 mL × 3); the combined organic layers were washed with saturated brine (20 mL), dried over anhydrous sodium sulfate and concentrated to obtain a crude product; and the crude product was purified by preparative thin-layer chromatography to obtain a yellow solid compound, 2-(1-iodoimidazol[1,5-a]pyridin-6-yl) thio-*N*-methylbenzamide 37f (89 mg, 217.47 μmol, yield: 56.02).

This product was verified by LCMS and HNMR.

For MS-ESI, the calculated value was $[M+H]^+$410.0, and the measurement was 410.0.

$^1$H NMR (400 MHz, $CDCl_3$) δ 8.15 (t, J = 10.8 Hz 2H), 7.53-7.55 (m, 1H), 7.27 - 7.33 (m, 3H), 7.13-7.15 (m, 1H), 6.72-6.75 (m, 1H), 6.10 (s, 1H), 3.05 (d, J = 5.2 Hz, 3H).

Step 6

Under the protection of nitrogen at room temperature, 2-vinylpyridine 36d (77.07 mg, 733.06 μmol), diisopropylethylamine (126.32 mg, 977.41 μmol), palladium acetate (43.89 mg, 195.48 μmol) and 1,1'-bis(diphenylphosphine) ferrocene (33.22 mg, 390.96 μmol) were added to the solution of the compound 2-(1-iodoimidazol[1,5-a]pyridin-6-yl)thio-*N*-methylbenzamide 37f (200 mg, 488.71 μmol) in N,N-dimethylformamide (10 mL); the reaction liquid was heated to 100 °C and stirred to react for 16 h. After the reaction of the starting materials completed, as indicated by thin-layer chromatography (dichloromethane/methanol = 20 /1), the mixture was poured into water (10 mL) and extracted with ethyl acetate (10 mL × 3); the combined organic layers were washed with water (10 mL × 2) and saturated brine (20 mL), dried over anhydrous sodium sulfate and concentrated to obtain a crude product; the crude product was purified by preparative high-performance liquid chromatography ; and the purified product was pulped with methanol (2 mL) and filtered. The filter cake was dried to obtain a yellow solid compound N-methyl-2-({1-[(E)-2-(pyridin-2-yl)vinyl]imidazo[1, 5-a]pyridin-6-yl}thio)benzamide I-26 (6.3 mg, 16.30 μmol, yield: 3.34 %).

This product was verified by LCMS, HNMR and CNMR.

For MS-ESI, the calculated value was $[M+H]^+$387.1, and the measurement was 387.1.

$^1$H NMR (400 MHz, CD3OD+CD3Cl) δ 8.47 (d, J = 3.6 Hz, 1H), 8.20 (d, J = 16.0 Hz, 2H), 7.77 (d, J = 15.6 Hz, 1H) 7.64-7.70 (m, 2H), 7.47 (d, J = 7.2 Hz, 1H), 7.34-7.40 (m, 2H), 7.20-7.30 (m, 2H), 7.15 (d, J =7.2 Hz, 1H), 6.74 (d, J = 9.6 Hz, 1H), 2.92 (s, 3H).

$^{13}$C NMR (100 MHz, CD3OD+CD3Cl) δ 169.50, 155.69, 148.95, 137.23, 135.69, 135.15, 130.76, 129.82,

128.99, 127.91, 127.83, 126.63, 126.43, 125.64, 124.85, 122.80, 122.20, 121.82, 120.66, 118.12, 26.36.

I-28

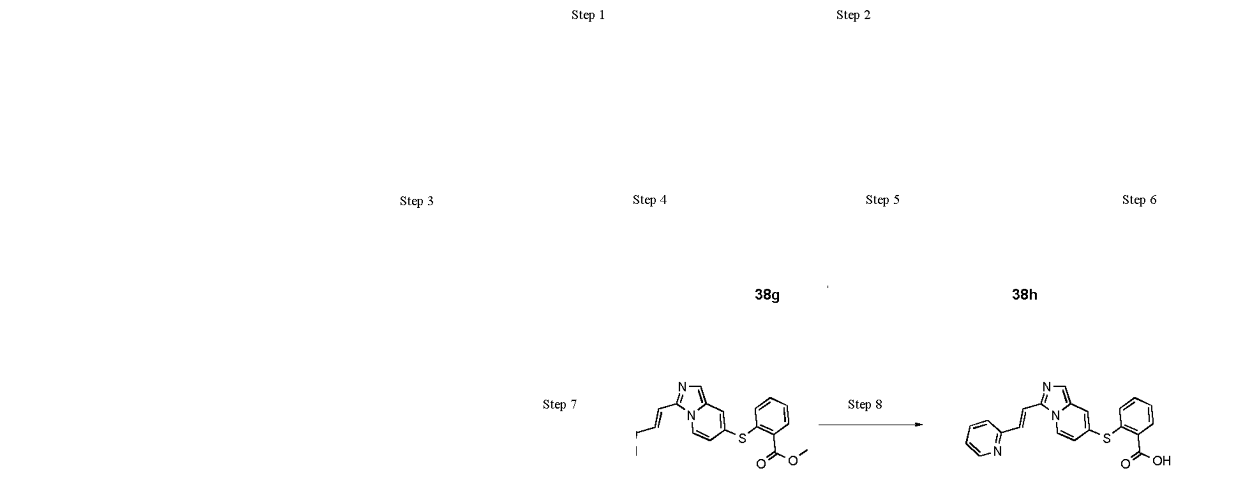


38g 38h

38j I-28

Step 1

Under the protection of nitrogen at 0°C, sodium hydride (89.6 mg, 3.73 mmol) was added to the solution of 2-diethoxyphosphorylethyl acetate 38b (460.5 mg, 2.05 mmol) in tetrahydrofuran (10 mL), stirred at 0°C for 1.0 h, followed by the slow addition of pyridin-2-formaldehyde 38a (0.2 g, 1.87 mmol). The reaction mixture was stirred at 25°C for 2 h. After the thin-layer chromatography (petroleum ether/ethyl acetate = 3/1) showed the complete reaction of the starting materials, the mixture was poured into water (10 mL) and extracted with ethyl acetate (10 mL × 3), and the organic layers were combined, washed with saturated brine (20 mL), dried over anhydrous sodium sulfate and concentrated to obtain a yellow oily crude product (*E*)-3-(2-pyridyl)prop-2-enoate38c (0.15 g, 846.50 μmol, yield: 45.33 %).

This product was verified by LCMS and HNMR.

For MS-ESI, the calculated value was $[M+H]^+$178.1, and the measurement was 178.0.

$^1$H NMR (400MHz, $CDCl_3$): δ (ppm) 8.65 (d, J=4.0Hz, 1H), 7.67-7.74 (m, 2H), 7.42 (d, J=8.0Hz, 1H), 7.25-7.29 (m, 1H), 6.90-6.94 (m, 1H), 1.17-1.31 (m, 3H).

Step 2

At room temperature, lithium hydroxide (81.1 mg, 3.39 mmol) was added to the solution of the compound (*E*)-3-(2-pyridyl)prop-2-enoate 38c (0.2 g, 1.13 mmol) in tetrahydrofuran (8 mL) and water (2 mL), and stirred at 30°C for 16 h. After the reaction of the starting materials completed, as indicated by thin-layer chromatography (dichloromethane/-methanol = 10/1), the reaction liquid was concentrated, followed by the addition of water (10 mL); the mixture was adjusted with the aqueous solution of citric acid to pH 5-6. The mixture was concentrated under reduced pressure, followed by the addition of ethanol (20 mL), and the mixture was stirred for 30 min and then filtered. The filtrate was concentrated under reduced pressure, to obtain a yellow solid crude product (*E*)-3-(2-pyridyl)prop-2-enoic acid 38d (0.15 g, 1.01 mmol, yield: 89.11 %).

This product was verified by LCMS and HNMR.

For MS-ESI, the calculated value was $[M+H]^+$150.0, and the measurement was 150.0.

$^1$H NMR (400MHz, DMSO-d6): δ (ppm) 8.62 (d, J=4.4Hz, 1H), 7.82-7.86 (m, 1H), 7.68-7.70 (m, 1H), 7.50-7.54 (m, 1H), 7.36-7.39 (m, 1H), 6.83 (d, J=15.6Hz, 1H).

Step 3

At room temperature, potassium carbonate (302.1 mg, 2.19 mmol) and cuprous iodide (41.6 mg, 218.57 μmol), and under the atmosphere of nitrogen were added to the solution of 4-bromopyridin-2-nitrile 38e (0.2 g, 1.09 mmol) and methyl 2-mercaptobenzoate (202.2 mg, 1.20 mmol) in N-methylpyrrolidone (10 mL); the solution was heated to 100 °C and stirred to react for about 16 h; after the reaction of the starting materials completed, as indicated by thin-layer chromatography (petroleum ether/ethyl acetate =5/1), the mixture was poured into water (30 mL) and extracted with

ethyl acetate (10 mL × 3); the combined organic layers were washed with saturated brine (20 mL), dried over anhydrous sodium sulfate and concentrated to obtain a crude product; and the crude product was purified by thin-layer preparative chromatography (petroleum ether/ethyl acetate =5/1) to obtain a white solid, 2-[(2-cyano-4-pyridyl)thio]methyl benzoate 38f (0.19 g, 702.91 μmol , yield: 64.32 %).

This product was verified by LCMS and HNMR.

For MS-ESI, the calculated value was $[M+H]^+$271.0, and the measurement was 271.0.

$^1$H NMR (400MHz, $CDCl_3$): δ (ppm) 8.50 (d, J=8Hz, 1H), 8.00-8.02 (m, 1H), 7.51-7.59 (m, 3 H), 7.36 (s, 1H), 7.26-7.28 (m, 1H), 3.87-3.89 (m, 3H).

Step 4

At room temperature, di-tert butyl dicarbonate (193.8 mg, 887.88 μmol) and raney nickel (100 mg) were added to the solution of the compound 2-[(2-cyano-4-pyridyl)thio]methyl benzoate 38f (200 mg, 739.90 μmol) in methanol (5 mL); under the atmosphere of hydrogen, the solution was stirred at 25°C to react for 16 h; after the reaction of the starting materials completed, as indicated by thin-layer chromatography (ethyl acetate/petroleum ether=3/1), the mixture was filtered; the filtrate was concentrated to obtain a crude product; and the crude product was purified by thin-layer preparative chromatography (ethyl acetate/petroleum ether=5/1-3/1) to obtain a yellow oily product, 2-[[2-[(tert-butoxycarbonylamino) methyl]-4-pyridyl]thio]methyl benzoate 38 g (110 mg, 293.76 μmol, yield: 39.70 %).

This product was verified by LCMS and HNMR.

For MS-ESI, the calculated value was $[M+H]^+$375.1, and the measurement was 375.2.

$^1$H NMR (400 MHz, $CDCl_3$) δ 8.34 (d, J = 5.6 Hz, 1H), 7.90 (dd, J = 6.0, 1.6 Hz, 1H), 7.26 - 7.40 (m, 3H), 7.14 (s, 1H), 7.00 (d, J = 4.8 Hz, 1H), 5.68 (s, 1H), 4.35 (d, J = 5.4 Hz, 2H), 3.84 (s, 3H), 1.40 (d, J = 2.8 Hz, 9H).

Step 5

The compound 2-[[2-[(tert-butoxycarbonylamino)methyl]-4-pyridyl]thio]methyl benzoate 38 g (140 mg, 373.88 μmol) was dissolved in the solution of hydrochloric acid in dioxane (3 mL, 9 mol/L); the solution was stirred at 25°C to react for 16 h. After LCMS showed the complete reaction of the starting materials, the reaction mixture was concentrated under reduced pressure, to obtain a compound 2-[[2-(aminomethyl)-4-pyridyl]mercapto]methyl benzoate dihydrochloride 38h crude product.

This product was verified by LCMS.

For MS-ESI, the calculated value was $[M+H]^+$275.1, and the measurement was 275.0.

Step 6

The compound 2-[[2-(aminomethyl)-4-pyridyl]mercapto]methyl benzoate hydrochloride 38h (50.0 mg, 160.88 μmol), 1-(3-dimethylaminopropyl)-3-ethylcarbodiimide hydrochloride (45.9 mg, 241.31 μmol), 1-hydroxybenzotriazole (32.6 mg, 241.31 μmol) and triethylamine (81.4 mg, 804.38 μmol) were added to the solution of the compound (*E*)-3-(2-pyridyl)prop-2-olefine acid 38d (24.0 mg, 160.88 μmol) in N,N-dimethylformamide (5 mL); the solution was stirred at 25°C to react for 3.0 h. After the thin-layer chromatography (petroleum ether/ethyl acetate = 3/1) showed the complete reaction of the starting materials, the mixture was poured into water (20 mL) and extracted with ethyl acetate (10 mL × 3), and the organic layers were combined, washed with saturated brine (20 mL), dried over anhydrous sodium sulfate and concentrated to obtain a crude product, which was purified by the thin-layer preparative chromatography (ethyl acetate/petroleum ether = 3/1), to obtain a yellow oily compound 2-[3-[(E)-2-(2-pyridyl)vinyl]imidazo[1,5-a]pyridin-7-yl] thiomethyl benzoate 38i (37 mg, 91.25 μmol, yield: 56.72 %).

This product was verified by LCMS and HNMR.

For MS-ESI, the calculated value was $[M+H]^+$406.1, and the measurement was 406.1.

$^1$H NMR (400 MHz, $CDCl_3$) δ 8.61 (d, J = 4.0 Hz, 1H), 8.38 (d, J = 5.6 Hz, 1H), 7.93 (dd, J = 8.0, 1.6 Hz, 1H), 7.62-7.71 (m, 2H), 7.34 - 7.44 (m, 4H), 7.08 - 7.25 (m, 4H), 7.05 (dd, J = 5.2, 1.6 Hz, 1H), 4.63 (s, 2H), 3.87 (s, 3H).

Step 7

At room temperature, phosphorus oxychloride (14.8 mg, 96.18 μmol) was added to the solution of the compound 2-[3-[(*E*)-2-(2-pyridyl)vinyl]imidazo[1,5-a]pyridin-7-yl]thiomethyl benzoate 38i (13 mg, 32.06 μmol) in 1,2-dichloroethane (1.5 mL); the mixture was stirred at 80°C to react for 16 h. After LCMS showed the complete reaction of the starting materials, the mixture was poured into water (10 mL) and extracted with ethyl acetate (10 mL × 3), and the organic layers were combined, washed with saturated brine (20 mL), dried over anhydrous sodium sulfate and concentrated to obtain a crude product, which was purified by the thin-layer preparative chromatography (ethyl acetate/petroleum ether = 3/1), to

obtain a yellow solid compound 2-[[2-[[(*E*)-3-(2-pyridyl)prop-2-enyl]amino]methyl]-4-pyridyl]thio]methyl benzoate 38j (5 mg, 12.90 μmol, yield: 40.25 %).

This product was verified by LCMS.

For MS-ESI, the calculated value was $[M+H]^+$388.1, and the measurement was 388.1.

Step 8

At room temperature, sodium hydroxide (16.5 mg, 412.95 μmol) was added to the solution of the compound 2-[[2-[[(*E*)-3-(2-pyridyl)prop-2-enyl]amino]methyl]-4-pyridyl]thio]methyl benzoate 38j (80 mg, 206.48 μmol) in methanol (5 mL); the mixture was stirred at 25°C to react for 1.0 h. After the thin-layer chromatography (petroleum ether/ethyl acetate = 1/1) showed the complete reaction of the starting materials, the mixture was poured into water (10 mL), and adjusted with 1N aqueous hydrochloric acid solution to the pH of 4-5. The mixture was filtered, and the filter cake was washed with water and dried, to obtain a red solid compound 2-({3-[(E)-2-(pyridin-2-ylvinyl]imidazo[1, 5-a]pyridin-7-yl}thio)benzoic acid I-28 (48 mg, 128.54 μmol, yield: 62.25 %).

This product was verified by LCMS, HNMR and CNMR.

For MS-ESI, the calculated value was $[M+H]^+$374.1, and the measurement was 374.1.

$^1$H NMR (400 MHz, $CDCl_3$&CD3OD) δ 8.59-8.71 (m, 3H), 8.13 (t, J = 7.2 Hz, 1H), 8.04 (d, J = 7.6 Hz, 1H), 7.83 (d, J = 7.6 Hz, 1H), 7.76 (s, 1H), 7.66 (s, 1H), 7.51-7.55 (m, 2H), 7.34 (t, J = 7.2 Hz, 1H), 7.23 (t, J = 7.2 Hz, 1H), 7.06 (d, J = 8.0 Hz, 1H), 6.82 (d, J = 6.8 Hz, 1H).

$^{13}$C NMR (100 MHz, $CDCl_3$&CD3OD) δ 168.38, 151.61, 144.03, 142.61, 139.59, 135.70, 132.80, 132.52, 131.63, 128.56, 125.73, 123.67, 123.29, 123.05 (s, 3H), 120.92, 119.86.

I-27

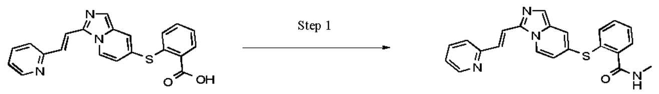


I-28 I-27

Step 1

At room temperature, methylamine (8.3 mg, 123.18 μmol), benzotriazol-1-yl-oxytripyrrolidylphosphine hexafluorophosphate (48.1 mg, 92.39 μmol) and 1,8-diazabicyclo[5.4.0]undecylcarbonyl-7-ene (18.8 mg, 123.18 μmol) were added to the solution of the compound 2-({3-[(E)-2-(pyridin-2-yl)vinyl]imidazo[1, 5-a]pyridin-7-yl}thio)benzoic acid I-28 (23 mg, 61.59 μmol) in N,N-dimethylformamide (2 mL). The mixture was stirred at 25°C for 16 hours. After the reaction of the starting materials completed, as indicated by thin-layer chromatography (dichloromethane/methanol = 15/1), the mixture was poured into water (10 mL), and extracted with ethyl acetate (10 mL × 3). The organic layers were combined, washed with saturated brine (20 mL), dried over anhydrous sodium sulfate and concentrated to obtain a crude product. The crude product was purified by preparative high-performance liquid chromatography to obtain a white solid, N-methyl-2-({3-[(E)-2-(pyridin-2-yl)vinyl]imidazo[1, 5-a]pyridin-7-yl}thio)benzamide I-27 (8 mg, 20.70 μmol, yield: 33.61 %).

This product was verified by LCMS and HNMR.

For MS-ESI, the calculated value was $[M+H]^+$387.1, and the measurement was 387.0.

$^1$H NMR (400 MHz, CD3OD) δ 8.57 (d, J = 4.4 Hz, 1H), 8.42 (d, J = 7.2 Hz, 1H), 7.96 (d, J = 15.6 Hz, 1H), 7.86 - 7.90 (m, 1H), 7.68 (d, J = 7.6 Hz, 1H), 7.63 (s, 1H), 7.51-7.56 (m, 3H), 7.92-7.44 (m, 3H), 7.32-7.35 (m, 1H), 6.67 (dd, J = 7.6, 1.6 Hz, 1H), 2.88 (s, 3H).

I-42

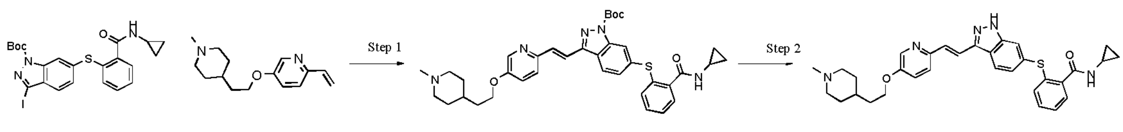


27f 39a 39b I-42

Step 1

At room temperature, 6-[2-(cyclopropylcarbamoyl)phenyl]thioalkyl-3-iodoindazol-1-tert-butyl carboxylate 27f (500 mg, 933.89 μmol) was dissolved in dioxane (20 mL), followed by the addition of 5-[2-(1-methyl-4-piperidyl)ethoxy]-2-vinyl-pyridine 39a (230 mg, 933.89 μmol), palladium chloride (83 mg, 466.94 μmol), tris(o-methylphenyl)phosphine (284 mg, 933.89 μmol) and triethylamine (473 mg, 4.67 mmol). The reaction system was stirred at 90°C under the protection of nitrogen to react for 1.5 h. The reaction mixture was poured into water (20 mL) and extracted with ethyl acetate (20 mL × 3), and the organic phases were combined, washed with saturated brine (20 mL), dried over anhydrous sodium sulfate, and concentrated under reduced pressure to obtain a crude product. The crude product was purified by the medium-pressure preparative chromatography to obtain a brown solid 6-[2-(cyclopropylaminoformyl)phenyl]thioalkyl-3-[(trans)-2-[5-[2-(1-methyl-4-piperidyl)ethoxy]-2-pyridyl]vinyl]indazol-1-tert-butyl formate 39b (200 mg, 305.89 μmol, yield: 32.75 %).

The product was verified by LCMS.

For MS-ESI, the calculated value was $[M+H]^+$654.3, and the measurement was 654.4.

Step 2

At room temperature, 6-[2-(cyclopropylcarbamoyl)phenyl]thioalkyl-3-[(trans)-2-[5-[2-(1-methyl-4-piperidyl)ethoxy]-2-pyridyl]vinyl]indazol-1-tert-butyl formate 39b (800 mg, 1.22 mmol) was dissolved in dichloromethane (9 mL) and trifluoroacetic acid (3 mL); the reaction system was stirred at 25°C to react for 2 h. The reaction mixture was poured into water (30 mL), adjusted with the saturated sodium carbonate solution to the pH of 8-9 and extracted with dichloromethane (20 mL × 3), and the organic phases were combined, washed with saturated brine (3 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{5-[2-(1-methylpiperidin-4-yl)ethoxy]pyridin-2-yl}vinyl]-1H-indazol-6-yl}thio)benzamide I-42 (69 mg, 124.61 μmol, yield: 10.18 %).

The product was verified by LCMS, H-NMR and C-NMR.

For MS-ESI, the calculated value was $[M+H]^+$554.3, and the measurement was 554.1.

$^1$H NMR (400MHz, $CD_3OD$): δ (ppm) 8.30 (s, 2H), 8.23 (d, *J* = 2.8 Hz, 1H), 8.03 (d, *J* = 8.4 Hz, 1H), 7.66 (dd, *J* = 23.2, 12.6 Hz, 2H), 7.58-7.47 (m, 2H), 7.44-7.42(m, 2H), 7.38-7.29 (m, 2H), 7.28-7.15 (m, 2H), 4.18 (t, *J* = 6.0 Hz, 2H), 3.49 (d, *J* = 11.4 Hz, 2H), 3.02 (s, 2H), 2.85 (s, 3H), 2.81-2.75 (m, 1H), 2.07 (d, *J* = 14.6 Hz, 2H), 1.91 (s, 1H), 1.88-1.81 (m, 2H), 1.56 (s, 2H), 0.74 (dt, *J* = 6.8, 3.4 Hz, 2H), 0.61-0.48 (m, 2H).

$^{13}$C NMR (100 MHz, $CD_3OD$) δ (ppm) 172.62, 167.55, 155.95, 149.33, 144.07, 143.61, 139.08, 138.44, 136.00, 135.50, 133.05, 131.75, 130.41, 129.17, 128.19, 126.32, 123.89, 123.19, 122.97, 122.63, 121.51, 115.02, 67.15, 55.53, 31.57, 30.67, 23.88, 6.59.

I-43

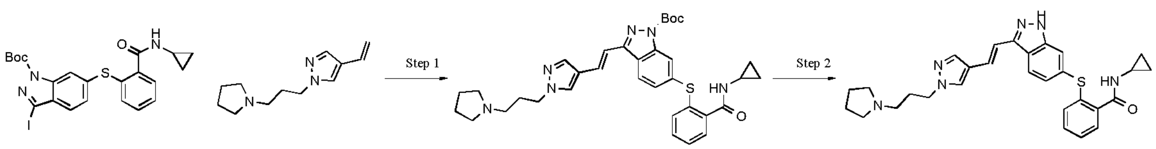


27f 16c 40a I-43

Step 1

At room temperature, 6-[2-(cyclopropylcarbamoyl)phenyl]thioalkyl-3-iodoindazol-1-tert-butyl carboxylate 27f (500 mg, 933.9 μmol) was dissolved in dioxane (5 mL), followed by the addition of 1-(3-pyrrolidin-1-ylpropyl)-4-vinylpyrazole 16c (230 mg, 1.12 mmol), palladium acetate (63 mg, 280.2 μmol), tris(o-methylphenyl)phosphine (85 mg, 280.2 μmol) and triethylamine (284 mg, 2.80 mmol). The reaction system was stirred at 95°C under the protection of nitrogen to react for 1.5 h. The reaction mixture was poured into water (20 mL) and extracted with ethyl acetate (20 mL × 3), and the organic phases were combined, washed with saturated brine (50 mL), dried over anhydrous sodium sulfate, and concentrated under reduced pressure to obtain a crude product. The crude product was purified by the preparative thin-layer chromatography (dichloromethane: methanol = 10/1) to obtain a yellow solid 6-[2-(cyclopropylaminoformyl)phenyl]thioalkyl-3-[(trans)-2-[1-(3-pyrrolidin-1-ylpropyl)pyrazol-4-yl]vinyl]indazol-1-tert-butyl formate 40a (300 mg, 489.6 μmol, yield: 52.42 %).

The product was verified by LCMS.

For MS-ESI, the calculated value was $[M+H]^+$613.3, and the measurement was 613.3.

Step 2

At room temperature, 6-[2-(cyclopropylcarbamoyl)phenyl]thioalkyl-3-[(trans)-2-[1-(3-pyrrolidin-1-ylpropyl)pyrazole-4-yl]vinyl]indazol-1-tert-butyl formate 40a (600 mg, 979.1 μmol) was dissolved in dichloromethane (5 mL) and

trifluoroacetic acid (5 mL); the reaction system was stirred at 25°C to react for 16 h. The reaction mixture was poured into water (20 mL), adjusted with the saturated sodium carbonate solution to the pH of 8-9 and extracted with dichloromethane (20 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{1-[3-(pyrrolidin-1-yl)propyl]-1H-pyrazol-4-yl}vinyl]-1H-indazol-6-yl}thio)benzamide I-43 (29 mg, 56.6 μmol, yield: 5.78 %).

The product was verified by LCMS, H-NMR and C-NMR.

For MS-ESI, the calculated value was $[M+H]^+$513.2, and the measurement was 513.1.

$^1$H NMR (400MHz, $CD_3OD$): δ (ppm) 8.54 (s, 1H), 7.98 *(d, J*= 8.6 Hz, 1H), 7.91 (s, 1H), 7.82 (s, 1H), 7.51 (s, 1H), 7.47-7.42 (m, 1H), 7.37-7.30 (m, 3H), 7.26-7.22 (m, 1H), 7.15 (s, 2H), 4.26 (t, *J* = 6.4 Hz, 2H), 3.07 (s, 4H), 2.99-2.92 (m, 2H), 2.82-2.73 (m, 1H), 2.27-2.18 (m, 2H), 1.98 (dd, *J* = 8.4, 5.4 Hz, 4H), 0.75 (td, *J* = 7.2, 5.2 Hz, 2H), 0.58-0.48 (m, 2H).

$^{13}$C NMR (100 MHz, $CD_3OD$) δ (ppm) 172.70, 143.55, 139.22, 138.79, 135.94, 135.61, 133.18, 131.67, 129.84, 129.14, 128.19, 125.90, 122.64, 122.38, 121.15, 119.14, 55.17, 53.95, 50.34, 28.74, 24.06, 23.79, 6.48.

I-44

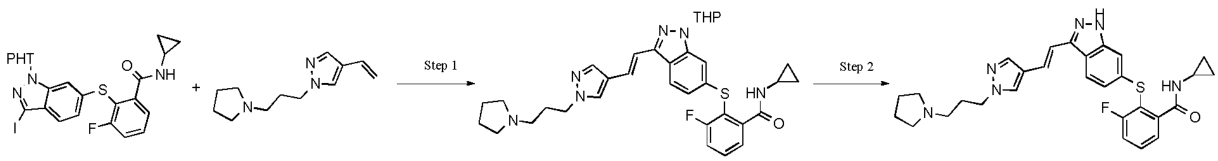


41a 16c 41b I-44

Step 1

At room temperature, N-cyclopropyl-3-fluoro-2-(3-iodo-1-tetrahydropyran-2-yl-indazol-6-yl)thioalkyl benzamide 41a (2 g, 3.72 mmol) was dissolved in dioxane (20 mL), followed by the addition of 1-(3-pyrrolidin-1-ylpropyl)-4-vinylpyrazole 16c (458 mg, 2.23 mmol), dichloropalladium (II) (198 mg, 1.12 mmol), tris(o-methylphenyl)phosphine (679 mg, 2.23 mmol) and triethylamine (1.1 g, 11.17 mmol). The reaction system was stirred at 90°C to react for 16 h under the protection of nitrogen. The reaction mixture was poured into water (50 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was purified by medium-pressure preparative chromatography to obtain a yellow solid product, N-cyclopropyl-3-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 41b (700 mg, 1.14 mmol, yield: 50.99 %).

The product was verified by LCMS.

For MS-ESI, the calculated value was $[M+H]^+$615.3, and the measurement was 615.1

Step 2

At room temperature, N-cyclopropyl-3-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 41b (700 mg, 1.14 mmol) was dissolved in dichloromethane (16 mL), followed by the addition of trifluoroacetic acid (4 mL). The reaction system was stirred at 25°C to react for 16 h. The reaction mixture was poured into water (50 mL), adjusted with the saturated aqueous solution of sodium carbonate to the pH of 8-9, and then extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-3-fluoro-2-({3-[(E)-2-{1-[3-(pyrrolidin-1-yl)propyl]-1H-pyrazol-4-yl}vinyl]-1H-indazol-6-yl}thio)benzamide I-44 (90.15 mg, 169.88 μmol, yield: 14.92 %). The product was verified by LCMS, H-NMR and F-NMR.

For MS-ESI, the calculated value was $[M+H]^+$531.2, and the measurement was 531.1

$^1$H NMR (400MHz,$CD_3OD$): δ (ppm) 7.92-7.89 (m, 2H), 7.78 (s, 1H), 7.54-7.49 (m, 1H), 7.33-7.26 (m, 4H), 7.16-7.05 (m, 2H), 4.11 (t, *J* = 6.8 Hz, 2H), 2.78-2.73 (m, 1H), 2.75 (s, 4H), 2.49 (t, *J* = 7.6 Hz, 2H), 2.13-2.05 (m, 2H), 1.82-1.80 (m, 4H), 0.75-0.70 (m, 2H), 0.52-0.48 (m, 2H).

$^{19}$F NMR (400MHz,$CD_3OD$): δ (ppm)-105.90.

I-46

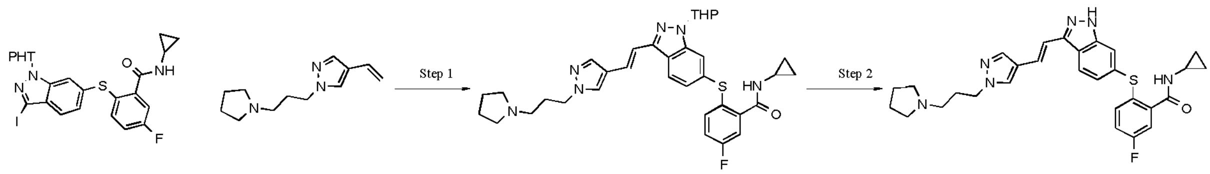


42a 16c 42b I-46

Step 1

At room temperature, N-cyclopropyl-3-fluoro-2-(3-iodo-1-tetrahydropyran-2-yl-indazol-6-yl)thioalkyl benzamide 42a (2 g, 3.7 mmol) was dissolved in dioxane (20 mL), followed by the addition of 1-(3-pyrrolidin-1-ylpropyl)-4-vinylpyrazole 16c (764 mg, 3.7 mmol), tris(o-methylphenyl)phosphine (1.1 g, 3.7 mmol), triethylamine (1.9 g, 18.6 mmol) and palladium acetate (417 mg, 1.9 mmol). The reaction system was stirred at 90°C under the protection of nitrogen to react for 16 h. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a crude product. The crude product was purified by the column chromatography (dichloromethane/methanol = 20/1-10/1) to obtain a yellow oily product N-cyclopropyl-5-fluoro-2-[3-[(trans)-2-[1-(3-pyrrolidin-1-ylpropyl)pyrazol-4-yl]vinyl]-1-tetrahydropyran-2-yl-indazol-6-yl]thioalkylbenzamide 42b (0.6 g, 975.97 μmol, yield: 26.22 %). The product was verified by LCMS.

For MS-ESI, the calculated value was $[M+H]^+$615.3, and the measurement was 615.1.

Step 2

At room temperature, N-cyclopropyl-5-fluoro-2-[3-[(trans)-2-[1-(3-pyrrolidin-1-ylpropyl)pyrazole -4-yl]vinyl]-1-tetrahydropyran-2-yl-indazol-6-yl]thioalkyl benzamide 42b (600 mg, 976.0 μmol) was dissolved in dichloromethane (12 mL) and trifluoroacetic acid (4 mL). The reaction system was stirred at 25°C to react for 16 h. The reaction mixture was poured into water (30 mL), adjusted with the saturated aqueous solution of sodium carbonate to the pH of 8-9 and extracted with dichloromethane (30 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a white solid product N-cyclopropyl-5-fluoro-2-({3-[(E)-2-{1-[3-(pyrrolidin-1-yl)propyl]-1H-pyrazol-4-yl}vinyl]-1H-indazol-6-yl}thio)benzamide I-46 (55 mg, 103.6 μmol, yield: 10.62 %).

The product was verified by LCMS, F-NMR, H-NMR and C-NMR.

For MS-ESI, the calculated value was $[M+H]^+$531.2, and the measurement was 531.0.

$^1$H NMR (400 MHz $CD_3OD$): δ (ppm) 7.98 (d, J = 8.4 Hz, 1H), 7.90 (s, 1H), 7.79 (s, 1H), 7.45 (s, 1H), 7.37-7.32 (m, 2H), 7.24 (dd, J = 8.4, 2.8 Hz, 1H), 7.18-7.13 (m, 2H), 7.11 (dd, J = 8.4, 1.2 Hz, 1H), 4.23 (t, J = 6.8 Hz, 2H), 2.78-2.75 (m, 1H), 2.61 (s, 4H), 2.53 (t, J = 7.6 Hz, 2H), 2.14-2.07 (m, 2H), 1.84-1.81 (m, 4H), 0.76-0.71 (m, 2H), 0.53-0.49 (m, 2H).

$^{13}$C NMR (100 MHz, $CD_3OD$) δ (ppm) 171.20, 164.43, 141.96, 138.40, 136.55, 136.47, 136.10, 130.65, 129.70, 125.15,122.73, 121.04, 118.57, 116.12, 113.86, 55.03, 54.32, 30.77, 30.26, 24.19, 24.19, 23.78, 6.44.

$^{19}$F NMR (400MHz, $CD_3OD$): δ (ppm) -115.68.

I-51

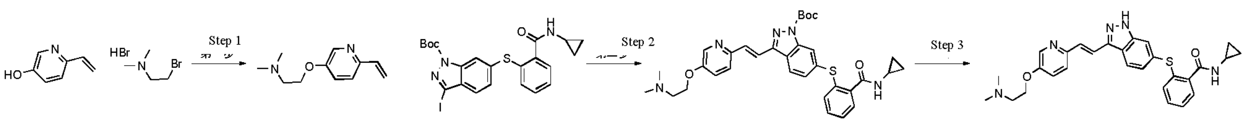


1c 43a 43b 27f 43c I-51

Step 1

At room temperature, 6-vinylpyridin-3-ol 1c (1 g, 8.26 mmol) was dissolved in anhydrous tetrahydrofuran (10 mL), the mixture was cooled to 0°C, followed by the adition of sodium hydride (991 mg, 24.77 mmol, 60 %); the reaction system was stirred at 0°C to react for 0.5 h. At room temperature, 2-bromo-N,N-dimethyl ethanolamine hydrobromide 43a (2.9 g, 12.38 mmol) was added. The reaction system was stirred at 50°C to react for 2 h. The reaction mixture was poured into water (30 mL) and extracted with ethyl acetate (20 mL × 3), and the organic phases were combined, washed with saturated

brine (20 mL), dried over anhydrous sodium sulfate, and concentrated under reduced pressure to obtain a crude product. The crude product was purified by the column chromatography (petroleum ether:ethyl acetate = 10/1-3/1) to obtain a white solid product N,N-dimethyl-2-[(6-vinyl-3-pyridyl)oxy]ethylamine 43b (900 mg, 4.68 mmol, yield: 56.71 %).

The product was verified by LCMS.

For MS-ESI, the calculated value was $[M+H]^+$193.1, and the measurement was 193.1.

Step 2

At room temperature, N,N-dimethyl-2-[(6-vinyl-3-pyridyl)oxy]ethylamine 43b (216 mg, 1.12 mmol) was dissolved in dioxane (10 mL), followed by the addition of 6-[2-(cyclopropylcarbamoyl)phenyl]thioalkyl-3-iodoindazol-1-tert-butyl carboxylate 27f (500 mg, 933.89 μmol), tris(o-methylphenyl)phosphine (500 mg, 1.64 mmol), tris(dibenzalaceton) dipalladium (500 mg, 546.02 μmol) and triethylamine (284 mg, 2.80 mmol). The reaction system was stirred at 80°C under the protection of nitrogen to react for 1.5 h. The reaction mixture was poured into water (30 mL) and extracted with ethyl acetate (20 mL × 3), and the organic phases were combined, washed with saturated brine (30 mL), dried over anhydrous sodium sulfate, and concentrated under reduced pressure to obtain a yellow solid crude product 6-[2-(cyclopropylaminoformyl)phenyl]thioalkyl-3-[(trans)-2-[5-[2-(dimethylamino)ethoxy]-2-pyridyl]vinyl]indazol-1-tert-butyl formate 43c (300 mg, 500.22 μmol, yield: 53.56 %). The product was verified by LCMS.

For MS-ESI, the calculated value was $[M+H]^+$600.3, and the measurement was 600.4.

Step 3

At room temperature, 6-[2-(cyclopropylcarbamoyl)phenyl]thioalkyl-3-[(trans)-2-[5-[2-(dimethylamino) ethoxy]-2-pyridyl]vinyl]indazol-1-tert-butyl formate 43c (300 mg, 500.22 μmol) was dissolved in dichloromethane (4 mL), followed by the addition of trifluoroacetic acid (4 mL); the reaction liquid was stirred at 25°C to react for 16 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{5-[2-(dimethylamino)ethoxy]pyridin-2-yl}vinyl]-1H-indazol-6-yl}thio)benzamide I-51 (2.8 mg, 5.60 μmol, yield: 1.12 %).

The product was verified by LCMS, H-NMR and C-NMR.

For MS-ESI, the calculated value was $[M+H]^+$500.2, and the measurement was 500.2

$^1H$ NMR (400MHz, $CDCl_3$): δ (ppm) 8.36 *(d, J* = 2.8 Hz, 1H), 7.99 (d, *J* = 8.6 Hz, 1H), 7.75-7.63 (m, 2H), 7.73 (d, *J* = 16.0 Hz, 1H), 7.68-7.65 (m, 1H), 7.50-7.46 (m, 1H), 7.43-7.41 (m, 2H), 7.35-7.31 (m, 3H), 7.17 (d, *J* = 8.0 Hz, 1H), 6.51 (s, 1H), 4.25 (s, 2H), 2.92 (s, 2H), 2.85-2.78 (m, 1H), 2.48 (s, 6H), 0.80-0.78 (m, 2H), 0.49-0.45 (m, 2H). $^{13}C$ NMR (100 MHz, $CDCl_3$): δ (ppm) 169.23, 148.94, 148.78, 142.07, 138.03, 136.79, 133.94, 133.59, 132.75, 132.60, 131.00, 130.53, 129.95, 129.11, 127.54, 124.27, 122.43, 121.69, 121.62, 121.52, 120.80, 57.68, 45.21, 31.93, 29.70, 23.04, 22.69, 14.12, 6.72.

I-52

Step 1
Step 2
Step 3

Step 1

At room temperature, 6-vinylpyridin-3-ol 1c (1 g, 8.26 mmol) was dissolved in anhydrous tetrahydrofuran (20 mL); the mixture was cooled to 0°C, followed by the addition of sodium hydride (991 mg, 24.77 mmol, 60 %); the reaction system was stirred at 0°C to react for 0.5 h. At the same temperature, 2-bromo-N, N-diethyl ethanolamine hydrobromide 44a (3.2 g, 12.38 mmol) was added. The reaction system was stirred at 50°C to react for 3 h. The reaction mixture was poured into water (40 mL) and extracted with ethyl acetate (40 mL × 3), and the organic phases were combined, washed with saturated brine (40 mL), dried over anhydrous sodium sulfate, and concentrated under reduced pressure to obtain a crude product. The crude product was purified by medium-pressure preparative chromatography to obtain a brown liquid product, N,N-diethyl-2-[(6-vinyl-3-pyridyl)oxy]ethylamine 44b (850 mg, 3.86 mmol, yield: 46.74 %).

The product was verified by LCMS and HNMR.

For MS-ESI, the calculated value was $[M+H]^+$221.2, and the measurement was 221.1.

$^1H$ NMR (400MHz, $CDCl_3$): δ (ppm) 8.27 (d, *J* = 2.8 Hz, 1H), 7.27 (s, 1H), 7.17 (dd, *J* = 8.6, 2.8 Hz, 1H), 6.77 (dd, *J* = 17.6, 10.8 Hz, 1H), 6.02 (dd, *J* = 17.6, 1.2 Hz, 1H), 5.35 (dd, *J* = 10.8, 1.2 Hz, 1H), 4.10 (dd, *J* = 11.8, 5.8 Hz, 2H), 2.90 (dd, *J* =

8.2, 4.0 Hz, 2H), 2.65 (dd, *J* = 8.2, 6.2 Hz, 4H), 1.10-1.07 (m, 6H).

Step 2

At room temperature, N,N-diethyl-2-[(6-vinyl-3-pyridyl)oxy]ethylamine 44b (150 mg, 680.86 μmol) was dissolved in dioxane (8 mL), followed by the addition of 6-[2-(cyclopropylcarbamoyl)phenyl]thioalkyl-3-iodoindazol-1-tert-butyl carboxylate 27f (304 mg, 567.38 μmol), tris(o-methylphenyl)phosphine (173 mg, 567.38 μmol), palladium chloride (50 mg, 283.69 μmol) and triethylamine (287 mg, 2.84 mmol). The reaction system was stirred at 95 °C under the protection of nitrogen to react for 1.5 h. The reaction mixture was poured into water (10 mL) and extracted with ethyl acetate (10 mL × 3), and the organic phases were combined, washed with saturated brine (20 mL), dried over anhydrous sodium sulfate, and concentrated under reduced pressure to obtain a brown solid crude product 6-[2-(cyclopropylaminoformyl) phenyl]aminothioalkyl-3-[(trans)-2-[5-[2-(diethylamino)ethoxy]-2-pyridyl]vinyl]indazol-1-n-butyl formate 44c (200 mg, 318.58 μmol, yield: 56.15 %).
The product was verified by LCMS.
For MS-ESI, the calculated value was $[M+H]^+$628.3, and the measurement was 628.2.

Step 3

At room temperature, 6-[2-(cyclopropylcarbamoyl)phenyl]aminothioalkyl-3-[(trans)-2-[5-[2-(diethylamino) ethoxy]-2-pyridyl]vinyl]indazol-1-n-butyl formate 44c (200 mg, 318.58 μmol) was dissolved in dichloromethane (9 mL), followed by the addition of trifluoroacetic acid (3 mL); the reaction liquid was stirred at 25°C to react for 2 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography to obtain a yellow solid product N-cyclopropyl-2-({3-[(E)-2-{5-[2-(diethylamino)ethoxy]pyridin-2-yl}vinyl]-1H-indazol-6-yl}thio)benzoyl I-52 (15 mg, 28.43 μmol, yield: 8.92 %).
The product was verified by LCMS, H-NMR and C-NMR.
For MS-ESI, the calculated value was $[M+H]^+$528.2, and the measurement was 528.2.
$^1$H NMR (400MHz, $CD_3OD$): δ (ppm) 8.28 (d, *J* = 2.8 Hz, 1H), 8.04 (d, *J* = 8.4 Hz, 1H), 7.68 (dd, *J* = 24.6, 12.6 Hz, 2H), 7.57-7.42 (m, 4H), 7.34-7.31 (m, 2H), 7.25 (dd, *J* = 7.4, 1.8 Hz, 1H), 7.18 (dd, *J* = 8.4, 1.4 Hz, 1H), 4.29 (t, *J*= 5.4 Hz, 2H), 3.18 (t, *J*= 5.2 Hz, 2H), 2.92 (q, *J*= 7.2 Hz, 4H), 2.78 (d, *J*= 3.8 Hz, 1H), 1.20 (t, *J* = 7.2 Hz, 6H), 0.75 (dd, *J*= 7.2, 1.8 Hz, 2H), 0.54 (dd, *J* = 3.8, 2.2 Hz, 2H).
$^{13}$C NMR (100 MHz, $CD_3OD$) δ (ppm) 172.70, 155.61, 149.97, 139.29, 138.57, 135.73, 133.24, 131.68, 130.37, 129.14, 128.22, 126.27, 123.87, 123.47, 122.54, 121.49, 66.46, 52.43, 30.75, 23.79, 14.45, 10.69, 6.47.

I-53

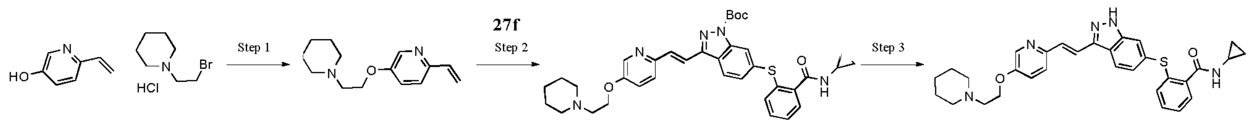


1c 45a 45b 45c I-53

Step 1

At room temperature, 6-vinylpyridin-3-ol 1c (1 g, 8.26 mmol) was dissolved in anhydrous tetrahydrofuran (10 mL); the mixture was cooled to 0°C, followed by the addition of sodium hydride (660 mg, 16.51 mmol, 60 %); the reaction system was stirred at 0°C to react for 0.5 h. At the same temperature, 1-(2-chloroethyl)piperidine hydrochloride 45a (2.3 g, 9.91 mmol) was added. The reaction system was stirred at 25°C to react for 2 h. The reaction mixture was poured into water (20 mL) and extracted with ethyl acetate (20 mL × 3), and the organic phases were combined, washed with saturated brine (50 mL), dried over anhydrous sodium sulfate, and concentrated under reduced pressure to obtain a crude product. The crude product was purified by the column chromatography (dichloromethane:methanol = 1-10/1) to obtain a yellow liquid product 5-[2-(1-piperidyl)ethoxy]-2-vinylpyridine 45b (800 mg, 3.44 mmol, yield: 41.71 %).
The product was verified by LCMS and HNMR.
For MS-ESI, the calculated value was $[M+H]^+$233.2, and the measurement was 233.2.
$^1$H NMR (400MHz, $CDCl_3$): δ (ppm) 8.27 (d, *J* = 2.8 Hz, 1H), 8.07 (d, *J* = 3.2 Hz, 1H), 7.36 (d, *J* = 8.8 Hz, 1H), 7.21-7.08 (m, 1H), 6.77 (dd, *J* = 17.6, 11.2 Hz, 1H), 6.02 (dd, *J* = 17.6, 1.2 Hz, 1H), 5.35 (dd, *J* = 10.8, 1.2 Hz, 1H), 4.18-4.08

(m, 2H), 2.80-2.75 (m, 2H), 2.51-2.43 (m, 4H), 1.62-1.58 (m, 4H), 1.49-1.38 (m, 2H)

Step 2

At room temperature, 5-[2-(1-piperidyl)ethoxy]-2-vinylpyridine 45b (260 mg, 1.12 mmol) was dissolved in dioxane (5 mL), followed by the addition of 6-[2-(cyclopropylcarbamoyl)phenyl]thioalkyl-3-iodoindazol-1-tert-butyl carboxylate 27f (500 mg, 933.89 μmol), tris(o-methylphenyl)phosphine (853 mg, 2.80 mmol), tris(dibenzalaceton)dipalladium (500 mg, 564.4 mmol) and triethylamine (284 mg, 2.80 mmol). The reaction system was stirred at 85°C under the protection of nitrogen to react for 1.5 h. The reaction mixture was poured into water (20 mL) and extracted with ethyl acetate (20 mL × 3), and the organic phases were combined, washed with saturated brine (50 mL), dried over anhydrous sodium sulfate, and concentrated under reduced pressure to obtain a yellow oily crude product 6-[2-(cyclopropylaminoformyl) phenyl]thioalkyl-3-[(trans)-2-[5-[2-(1-piperidyl)ethoxy]-2-pyridyl]vinyl]indazol-1-tert-butyl formate 45c (190 mg, 296.97 μmol, yield: 31.80 %).

The product was verified by LCMS.

For MS-ESI, the calculated value was $[M+H]^+$640.3, and the measurement was 640.1.

Step 3

At room temperature, 6-[2-(cyclopropylcarbamoyl)phenyl]thioalkyl-3-[(trans)-2-[5-[2-(1-piperidyl)ethoxy]-2-pyridyl]vinyl]indazol-1-tert-butyl formate 45c (60 mg, 93.78 μmol) was dissolved in dichloromethane (5 mL), followed by the addition of trifluoroacetic acid (2 mL); the reaction liquid was stirred at 25°C to react for 16 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography to obtain a yellow solid product N-cyclopropyl-2-({3-[(E)-2-{5-[2-(piperidin-1-yl) ethoxy]pyridin-2-yl}vinyl]-1H-indazol-6-yl}thio)benzamide I-53 (2.4 mg, 4.45 μmol, yield: 4.74 %).

The product was verified by LCMS and H-NMR.

For MS-ESI, the calculated value was $[M+H]^+$540.2, and the measurement was 540.2.

$^1$H NMR (400MHz, $CD_3OD$): δ (ppm) 8.28 (d, *J*= 2.8 Hz, 1H), 8.05 (d, *J* = 8.6 Hz, 1H), 7.71 (d, *J* = 16.8 Hz, 1H), 7.65 (d, *J*= 8.6 Hz, 1H), 7.55 (d, *J*= 3.4 Hz, 1H), 7.44 (dd, *J* = 4.6, 1.8 Hz, 1H), 7.36-7.30 (m, 1H), 7.27-7.24 (m, 1H), 7.18 (dd, *J* = 8.6, 1.4 Hz, 1H), 4.29 (t, *J* = 5.4 Hz, 2H), 2.95 (s, 2H), 2.81-2.66 (m, 5H), 1.74-1.64 (m, 2H), 1.53 (s, 2H), 0.77-0.69 (m, 2H), 0.56-0.49 (m, 2H).

I-55

Step 1

Step 2

Step 3

Step 4

Step 1

At room temperature, 2-(6-vinyl-3-pyridyl)ethanol 46a (1 g, 6.70 mmol) was dissolved in dichloromethane (10 mL) and acetonitrile (5 mL), followed by the addition of paratoluensulfonyl chloride (1.7 g, 8.71 mmol) and triethylamine (1.7 g, 16.76 mmol); the reaction system was stirred at 25°C to react for 15 h. The reaction mixture was poured into water (30 mL) and extracted with dichloromethane (30 mL × 3), and the organic phases were combined, washed with saturated brine (40 mL), dried over anhydrous sodium sulfate, and concentrated under reduced pressure to obtain a crude product. The crude product was purified by the preparative plate (petroleum ether:ethyl acetate = 3/1) to obtain a yellow oily product 2-(6-vinylpyridin-3-yl)ethyl-4-methylbenzene sulfonate 46b (945 mg, 3.11 mmol, yield: 46.47 %).

The product was verified by LCMS and HNMR.

For MS-ESI, the calculated value was $[M+H]^+$304.1, and the measurement was 304.1.

$^1$H NMR (400MHz, $CDCl_3$): δ (ppm) 8.30 (d, *J* = 2.0 Hz, 1H), 7.66 (d, *J* = 8.0 Hz, 2H), 7.42 (dd, *J* = 8.0, 2.4 Hz, 1H), 7.28 (s, 1H), 7.26-7.22 (m, 2H), 6.78 (dd, *J* = 17.2, 10.8 Hz, 1H), 6.16 (dd, *J* = 17.6, 1.2 Hz, 1H), 5.47 (dd, *J* = 10.8, 0.8 Hz, 1H), 4.22 (t, *J* = 6.8 Hz, 2H), 2.94 (t, *J* = 6.8 Hz, 2H), 2.43 (s, 3H).

Step 2

At room temperature, 2-(6-vinylpyridin-3-yl)ethyl-4-methylbenzene sulfonate 46b (940 mg, 3.10 mmol) was dissolved in acetonitrile (15 mL), followed by the addition of tetrahydropyrrole (1.1 g, 15.49 mmol) and triethylamine (784 mg, 7.75 mmol); the reaction system was stirred at 6°C to react for 1.5 h. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane:methanol = 10/1) to obtain a yellow oily product 5-(2-pyrrolidin-1-yl)-2-vinylpyridine 46c (396.7 mg, 1.96 mmol, yield: 63.29 %).

The product was verified by LCMS and HNMR.

For MS-ESI, the calculated value was $[M+H]^+$203.2, and the measurement was 203.2.

$^1$H NMR (400MHz, $CDCl_3$): δ (ppm) 8.43 (d, *J*= 2.0 Hz, 1H), 7.51 (dd, *J* = 8.0, 2.4 Hz, 1H), 7.28 (d, *J* = 8.0 Hz, 1H), 6.80 (dd, *J* = 17.6, 10.8 Hz, 1H), 6.14 (dd, *J* = 17.6, 1.2Hz, 1H), 5.44 (dd, *J* = 10.8, 1.2 Hz, 1H), 2.87-2.83 (m, 2H), 2.74-2.70 (m, 2H), 2.62 (s, 4H), 1.84-1.81 (m, 4H).

Step 3

At room temperature, 5-(2-pyrrolidin-1-yl)-2-vinylpyridine 46c (189 mg, 933.89 μmol) was dissolved in dioxane (10 mL), followed by the addition of 6-[2-(cyclopropylcarbamoyl)phenyl]thioalkyl-3-iodoindazol-1-tert-butyl carboxylate 27f (500 mg, 933.89 μmol), tris(o-methylphenyl)phosphine (284 mg, 933.89 μmol), palladium chloride (83 mg, 466.94 μmol) and triethylamine (473 mg, 4.67 mmol). The reaction system was stirred at 95 °C under the protection of nitrogen to react for 1.5 h. The reaction mixture was poured into water (20 mL) and extracted with ethyl acetate (20 mL × 3), and the organic phases were combined, washed with saturated brine (20 mL), dried over anhydrous sodium sulfate, and concentrated under reduced pressure to obtain a brown solid crude product 6-[2-(cyclopropylaminoformyl)phenyl] thioalkyl-3-[(trans)-2-[5-(2-pyrrolidin-1-yl)-2-pyridyl]vinyl]indazol-1-tert-butyl formate 46d (300 mg, 491.98 μmol, yield: 52.68 %).

The product was verified by LCMS.

For MS-ESI, the calculated value was $[M+H]^+$610.3, and the measurement was 610.3.

Step 4

At room temperature, 6-[2-(cyclopropylcarbamoyl)phenyl]thioalkyl-3-[(trans)-2-[5-(2-pyrrolidin-1-yl)-2-pyridyl] vinyl]indazol-1-tert-butyl formate 46d (300 mg, 491.98 μmol) was dissolved in dichloromethane (12 mL), followed by the addition of trifluoroacetic acid (4 mL); the reaction liquid was stirred at 25°C to react for 2 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography to obtain a white solid product N-cyclopropyl-2-({3-[(E)-2-{5-[2-(pyrrolidin-1-yl)ethyl] pyridin-2-yl}vinyl]-1H-indazol-6-yl}thio)benzamide I-55 (17 mg, 33.36 μmol, yield: 6.78 %).

The product was verified by LCMS, H-NMR and C-NMR.

For MS-ESI, the calculated value was $[M+H]^+$512.2, and the measurement was 512.2.

$^1$H NMR (400MHz, $CD_3OD$): δ (ppm) 8.53 (s, 1H), 8.49 (s, 1H), 8.03 (d, *J* = 8.4 Hz, 1H), 7.81 (dd, *J* = 26.2, 12.4 Hz, 2H), 7.64 (d, *J* = 8.0 Hz, 1H), 7.57-7.42 (m, 3H), 7.39-7.28 (m, 2H), 7.24 (d, *J* = 7.2 Hz, 1H), 7.18 (d, *J* = 8.4 Hz, 1H), 3.48-3.32 (m, 6H), 3.15-3.04 (m, 2H), 2.84 -2.73 (m, 1H), 2.09 (s, 4H), 0.82-0.66 (m, 2H), 0.61-0.47 (m, 2H).

$^{13}$C NMR (100 MHz, $CD_3OD$) δ (ppm) 172.67, 155.80, 150.59, 143.76, 143.61, 139.29, 138.94, 135.82, 133.25, 131.72, 130.27, 129.17, 128.27, 126.41, 125.36, 123.26, 122.50, 121.58, 114.86, 56.57, 55.26, 30.25, 24.12, 23.82, 6.51.

I-59

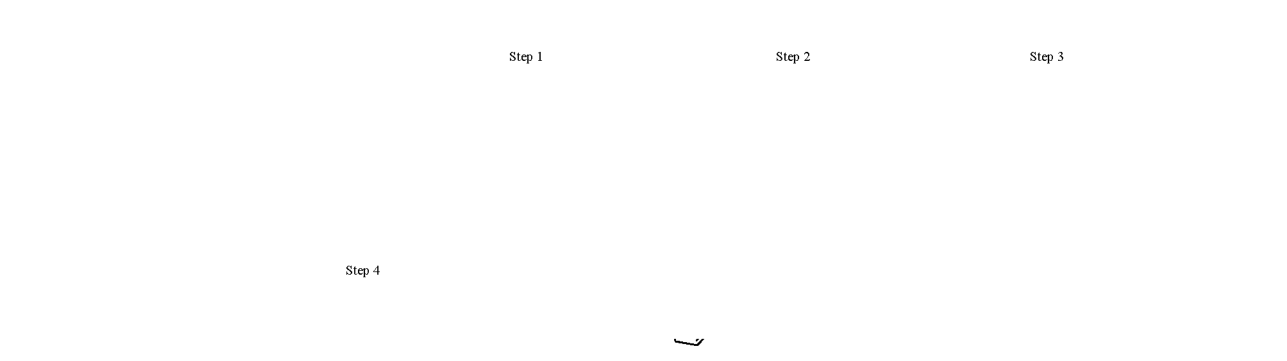


Step 1

At room temperature, 3-(6-vinyl-3-pyridyl)propan-1-ol 47a (1.5 g, 9.19 mmol) was dissolved in dichloromethane (20 mL), followed by the addition of paratoluensulfonyl chloride (972 mg, 13.79 mmol) and triethylamine (2.8 g, 27.57 mmol); the reaction system was stirred at 25°C to react for 16 h. The reaction mixture was poured into water (50 mL) and extracted with dichloromethane (50 mL × 3), and the organic phases were combined, washed with saturated brine (100 mL), dried over anhydrous sodium sulfate, and concentrated under reduced pressure to obtain a crude product. The crude product was purified by the column chromatography (petroleum ether:ethyl acetate = 1-10/1) to obtain a yellow oily product 3-(6-vinyl-3-pyridyl)propyl-4-methylbenzene sulfonate 47b (1.5 g, 4.73 mmol, yield: 51.42 %).

The product was verified by LCMS and HNMR.

For MS-ESI, the calculated value was $[M+H]^+$318.1, and the measurement was 318.0.

$^1$H NMR (400MHz, $CDCl_3$): δ (ppm) 8.32 *(d, J=* 2.0 Hz, 1H), 7.79 (d, *J* = 8.4 Hz, 2H), 7.38-7.34 (m, 3H), 7.24 (d, *J*= 8.0 Hz, 1H), 6.78 (dd, *J* = 17.6, 10.8 Hz, 1H), 6.14 (dd, *J* = 17.6, 1.2 Hz, 1H), 5.45 (dd, *J* = 10.8, 1.2 Hz, 1H), 4.04 (t, *J*= 6.0 Hz, 2H), 2.67 (t, *J* = 7.6 Hz, 2H), 2.46 (s, 3H), 1.98-1.93 (m, 2H).

Step 2

At room temperature, 3-(6-vinyl-3-pyridyl)propyl-4-methylbenzene sulfonate 47b (800 mg, 2.52 mmol) was dissolved in acetonitrile (10 mL), followed by the addition of pyrrolidine (215 mg, 3.02 mmol) and triethylamine (765 mg, 7.56 mmol); the reaction system was stirred at 60 °C to react for 16 h. The reaction mixture was poured into water (40 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the preparative thin-layer chromatography (dichloromethane:methanol = 20/1) to obtain a yellow oily product 5-(3-pyrrolidin-1-ylpropyl)-2-vinylpyridine 47c (1.5 g, 4.73 mmol, yield: 51.42 %).

The product was verified by LCMS and HNMR.

For MS-ESI, the calculated value was $[M+H]^+$217.2, and the measurement was 217.1.

$^1$H NMR (400MHz, $CDCl_3$): δ (ppm) 8.41 (d, *J* = 2.0 Hz, 1H), 7.48 (dd, *J* = 8.0, 2.4 Hz, 1H), 7.29 (s, 1H), 6.80 (dd, *J* = 17.6, 10.8 Hz, 1H), 6.14 (dd, *J* = 17.6, 1.6 Hz, 1H), 5.43 (dd, *J* = 10.8, 1.2 Hz, 1H), 2.66(t, *J* = 7.6 Hz, 2H), 2.50-2.44 (m, 6H), 1.88-1.82 (m, 2H), 1.80-1.74 (m, 4H).

Step 3

At room temperature, 6-[2-(cyclopropylcarbamoyl)phenyl]thioalkyl-3-iodoindazol-1-tert-butyl carboxylate 27f (900 mg, 1.68 mmol) was dissolved in dioxane (10 mL), followed by the addition of 5-(3-pyrrolidin-1-ylpropyl)-2-vinylpyridine 47c (364 mg, 1.68 mmol), tris(o-methylphenyl)phosphine (256 mg, 840.50 μmol), palladium chloride (149 mg, 840.50 μmol) and triethylamine (170 mg, 1.68 mmol). The reaction system was stirred at 95 °C under the protection of nitrogen to react for 1.5 h. The reaction mixture was poured into water (50 mL) and extracted with ethyl acetate (40 mL × 3), and the organic phases were combined, washed with saturated brine (40 mL), dried over anhydrous sodium sulfate, and concentrated under reduced pressure to obtain a crude product. The crude product was purified by the preparative thin-layer chromatography (dichloromethane:methanol = 20/1) to obtain a yellow solid product 6-[2-(cyclopropylaminoformyl)phenyl]thioalkyl-3-[(trans)-2-[5-(3-pyrrolidin-1-ylpropyl)-2-pyridyl]vinyl]indazol-1-tert-butyl formate 47d (380 mg, 609.16 μmol, yield: 36.24 %).

The product was verified by LCMS.

For MS-ESI, the calculated value was $[M+H]^+$624.3, and the measurement was 624.2.

Step 4

At room temperature, 6-[2-(cyclopropylcarbamoyl)phenyl]thioalkyl-3-[(trans)-2-[5-(3-pyrrolidin-1-ylpropyl)-2-pyridyl]vinyl]indazol-1-tert-butyl formate 47d (330 mg, 529.01 μmol) was dissolved in dichloromethane (10 mL), followed by the addition of trifluoroacetic acid (603 mg, 5.29 mmol); the reaction liquid was stirred at 25°C to react for 4 h. The reaction mixture was poured into water (20 mL), adjusted with the saturated sodium carbonate solution to the pH of 9, and then extracted with dichloromethane (10 mL × 3), and the organic phases were combined, washed with saturated brine (20 mL), dried over anhydrous sodium sulfate, and concentrated under reduced pressure to obtain a crude product, which was purified by the high-pressure liquid chromatography to obtain a yellow solid product N-cyclopropyl-2-({3-[(E)-2-{5-[3-(pyrrolidin-1-yl)propyl]pyridin-2-yl}vinyl]-1H-indazol-6-yl}thio)benzamide I-59 (17 mg, 32.46 μmol, yield: 6.14 %).

The product was verified by LCMS, H-NMR and C-NMR.

For MS-ESI, the calculated value was $[M+H]^+$540.2, and the measurement was 540.2.

$^1$H NMR (400MHz, $CD_3OD$): δ (ppm) 8.42 (d, *J* = 1.6 Hz, 1H), 8.05 (d, *J* = 8.8 Hz, 1H), 7.82 (d, *J* = 16.8 Hz, 1H), 7.73 (dd, *J* = 8.4, 2.4 Hz, 1H), 7.65 (d, *J* = 8.0 Hz, 1H), 7.57-7.53 (m, 2H), 7.45 (dd, *J* = 6.8, 1.6 Hz, 1H), 7.34-7.30 (m, 2H), 7.27-7.25 (m, 1H), 7.19 (dd, *J* = 8.8, 1.6 Hz, 1H), 2.80-2.68 (m, 8H), 2.03-1.88 (m, 7H), 0.90-0.88 (m, 2H), 0.55-0.51 (m, 2H).

$^{13}$CNMR (100MHz, $CD_3OD$) δ (ppm) 172.68, 154.71, 150.24, 143.88, 143.62, 139.24, 138.56, 137.90, 135.70, 133.19, 130.59, 128.20, 126.39, 124.68, 122.98, 122.54, 121.58, 114.92, 56.68, 55.04, 36.56, 33.09, 30.70, 30.47, 30.35, 28.13, 26.94, 24.16, 23.81, 14.47, 6.51.

I-60

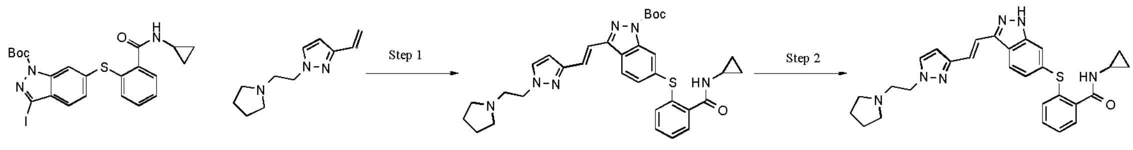


27f 17c 48a I-60

Step 1

At room temperature, 6-[2-(cyclopropylcarbamoyl)phenyl]thioalkyl-3-iodoindazol-1-tert-butyl carboxylate 27f (1.0 g, 1.87 mmol) was dissolved in dioxane (10 mL), followed by the addition of 1-(2-pyrrolidin-1-yl)-3-vinylpyrazole 17c (357 mg, 1.87 mmol), palladium chloride (166 mg, 933.89 umol), tris(o-methylphenyl)phosphine (284 mg, 933.89 μmol) and triethylamine (567 mg, 5.60 mmol). The reaction system was stirred at 95°C to react for 2 h under the protection of nitrogen. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a yellow solid product 2-[3-[(trans)-2-[5-(diethylaminomethyl)-2-pyridyl]vinyl]-1-tetrahydropyran-2-yl-indazol-6-yl]thioalkyl-3-fluoro-N-methyl-benzamide 48a (1.0 g, 1.67 mmol, yield: 90.91 %).

The product was verified by LCMS.

For MS-ESI, the calculated value was $[M+H]^+$ 559.3, and the measurement was 559.1.

Step 2

At room temperature, 2-[3-[(trans)-2-[5-(diethylaminomethyl)-2-pyridyl]vinyl]-1-tetrahydropyran-2-yl-indazol-6-yl]thioalkyl-3-fluoro-N-methyl-benzamide 48a (300 mg, 501.04 μmol) was dissolved in dichloromethane (10 mL), followed by the addition of trifluoroacetic acid (571 mg, 5.01 mmol). The reaction system was stirred at 25°C to react for 2 h. The reaction mixture was poured into water (10 mL), adjusted with the saturated aqueous solution of sodium carbonate to the pH of 8-9, extracted with dichloromethane (10 mL × 3), and the organic phases were combined, washed with saturated brine (10 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{1-[2-(pyrrolidin-1-yl)ethyl]-1H-pyrazol-3-yl}vinyl]-1H-indazol-6-yl}thio)benzamide I-60 (11.0 mg, 22.06 μmol, yield: 4.40 %).

The product was verified by LCMS, H-NMR and C-NMR.

For MS-ESI, the calculated value was $[M+H]^+$ 499.2, and the measurement was 499.1.

$^1$H NMR (400 MHz, $CD_3OD$) δ (ppm) 7.99 (d, *J* = 8.6 Hz, 1H), 7.63 (d, *J* = 2.4 Hz, 1H), 7.56 (s, 1H), 7.40 (d, *J* = 5.4

Hz, 3H), 7.30 (d, *J* = 7.4, 3.6 Hz, 2H), 7.24-7.19 (m, 1H), 7.17 (dd, *J* = 8.6, 1.4 Hz, 1H), 6.61 (d, *J* = 2.4 Hz, 1H), 4.31 (s, 2H), 3.02 (d, *J* = 6.9 Hz, 2H), 2.85-2.76 (m, 1H), 2.60 (s, 4H), 1.85-1.78 (m, 4H), 0.80-0.72 (m, 2H), 0.60-0.53 (m, 2H).

$^{13}C$ NMR (100 MHz, $CD_3OD$) δ (ppm) 172.40, 151.80, 135.97, 135.10, 132.76, 132.69, 131.44, 128.84, 127.73, 126.04, 123.81, 122.37, 121.03, 103.72, 79.18, 78.86, 78.53, 56.61, 49.19, 48.98, 48.77, 48.55, 48.34, 24.09, 23.59, 6.42.

I-61

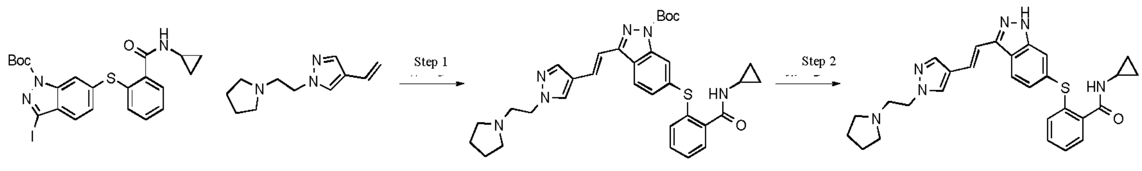


27f 17c' 49a I-61

Step 1

At room temperature, 6-[2-(cyclopropylcarbamoyl)phenyl]thioalkyl-3-iodoindazol-1-tert-butyl carboxylate 27f (2 g, 3.74 mmol) was dissolved in dioxane (40 mL), followed by the addition of 1-(2-pyrrolidin-1-yl)-4-vinylpyrazole 17c' (715 mg, 3.74 mmol), palladium chloride (199 mg, 1.12 mmol), tris(o-methylphenyl)phosphine (1 g, 1.12 mmol) and triethylamine (1.1 g, 11.21 mmol). The reaction system was stirred at 95°C under the protection of nitrogen to react for 1.5 h. The reaction mixture was poured into water (100 mL) and extracted with ethyl acetate (50 mL × 3), and the organic phases were combined, washed with saturated brine (100 mL), dried over anhydrous sodium sulfate, and concentrated under reduced pressure to obtain a crude product. The crude product was purified by the preparative thin-layer chromatography (dichloromethane:methanol = 10/1) to obtain a yellow solid 6-[2-(cyclopropylaminoformyl)phenyl]thioalkyl-3-[(trans)-2-[1-(2-pyrrolidin-1-yl)pyrazol-4-yl]vinyl[indazol-1-tert-butyl formate 49a (180 mg, 300.62 μmol, yield: 8.05 %).

The product was verified by LCMS.

For MS-ESI, the calculated value was $[M+H]^+$ 599.3, and the measurement was 599.1.

Step 2

At room temperature, 6-[2-(cyclopropylcarbamoyl)phenyl]thioalkyl-3-[(trans)-2-[1-(2-pyrrolidin-1-yl)pyrazole-4-yl]vinyl]indazol-1-tert-butyl formate 49a (180 mg, 300.62 μmol) was dissolved in dichloromethane (5 mL) and trifluoroacetic acid (171 mg, 1.50 mmol); the reaction system was stirred at 25°C to react for 4 h. The reaction mixture was poured into water (20 mL), adjusted with the saturated sodium carbonate solution to the pH of 8-9 and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{1-[2-(pyrrolidin-1-yl)ethyl]-1H-pyrazol-4-yl}vinyl]-1H-indazol-6-yl}thio)benzamide I-61 (32 mg, 64.17 μmol, yield: 21.35 %).

The product was verified by LCMS, H-NMR and C-NMR.

For MS-ESI, the calculated value was $[M+H]^+$ 499.2, and the measurement was 499.1.

$^1H$ NMR (400MHz, $CD_3OD$): δ (ppm) 7.99 (d, *J* = 8.4 Hz, 1H), 7.93 (s, 1H), 7.81 (s, 1H), 7.52 (s, 1H), 7.45-7.43 (m, 1H), 7.37-7.29 (m, 3H), 7.25-7.23 (m, 1H), 7.19-7.13 (m, 2H), 4.33 (t, *J* = 6.8 Hz, 2H), 3.04 (t, *J* = 6.8 Hz, 2H), 2.79-2.75 (m, 1H), 2.65 (s, 4H), 1.83-1.82 (m, 4H), 0.76-0.72 (m, 2H), 0.56-0.52 (m, 2H).

$^{13}C$ NMR (100 MHz, $CDCl_3$&$CD_3OD$) δ (ppm) 172.66, 143.53, 138.59, 135.21, 131.77, 129.18, 127.96, 126.17, 122.77, 122.23, 121.30, 119.17, 115.28, 56.87, 55.33, 52.12, 24.44, 23.96, 6.82.

I-67

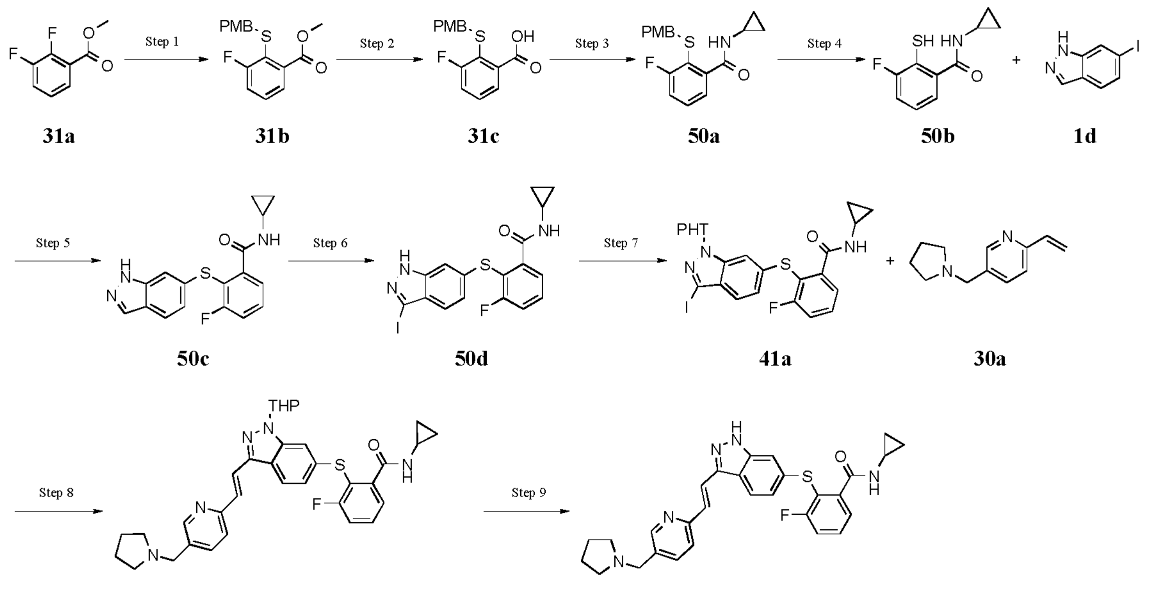


31a 31b 31c 50a 50b 1d

50c 50d 41a 30a

50e I-67

Step 1

At room temperature, 2,3-difluoromethyl benzoate 31a (50 g, 290.48 mmol) was dissolved in N,N-dimethylformamide (500 mL), followed by the addition of (4-methoxyphenyl)methanethiol (44.8 g, 290.48 mmol) and cesium carbonate (189.3 g, 580.96 mmol); the reaction system was stirred at 25°C to react for 2 h. The reaction mixture was filtered; the filtrate was poured into an aqueous solution (1000 mL) and extracted with ethyl acetate (500 mL × 3); and the organic phases were combined, washed with saturated brine (500 mL), dried over anhydrous sodium sulfate, and concentrated under reduced pressure to obtain a crude product. The crude product was purified by the medium-pressure preparative chromatography to obtain a white solid product 3-fluoro-2-[(4-methoxyphenyl)methylthioalkyl]methyl benzoate 31b (50 g, 163.21 mmol, yield: 56.19 %).

The product was verified by LCMS and H-NMR.

For MS-ESI, the calculated value was $[M+Na]^+$ 329.1, and the measurement was 328.9.

$^1H$ NMR (400MHz, $CDCl_3$): δ (ppm) 7.37 (d, *J*= 7.6 Hz, 1H), 7.30-7.25 (m, 1H), 7.18-7.13 (m, 3H), 6.76 (d, *J*= 8.8 Hz, 2H), 4.08 (s, 2H), 3.89 (s, 3H), 3.76 (s, 3H)

Step 2

At room temperature, 3-fluoro-2-[(4-methoxyphenyl)methylthioalkyl]methyl benzoate 31b (50 g, 163.21 mmol) was dissolved in tetrahydrofuran (360 mL) and water (102 mL), followed by the addition of lithium hydroxide (11.7 g, 489.63 mmol). The reaction system was stirred at 50°C to react for 16 h. Tetrahydrofuran was spun away, and the remaining mixture was poured into the aqueous solution (1000 mL), adjusted with the dilute hydrochloric acid (1N) to the pH of 5, and filtered to collect the filter cake. The filter cake was dried to obtain a white solid product 3-fluoro-2-[(4-methoxyphenyl)methylthioalkyl]benzoic acid 31c (41 g, 140.25 mmol, yield: 85.93 %).

The product was verified by LCMS and H-NMR.

For MS-ESI, the calculated value was $[M+ H_2O]^+$ 310.1, and the measurement was 310.0.

$^1H$ NMR (400MHz, $CDCl_3$): δ (ppm) 7.86 (d, *J*= 7.6 Hz, 1H), 7.42-7.37 (m, 1H), 7.29 (d, *J*= 8.4 Hz, 1H), 7.09 (d, *J*= 8.4 Hz, 2H), 6.76 (d, *J*= 8.0 Hz, 2H), 4.12 (s, 2H), 3.76 (s, 3H).

Step 3

At room temperature, 3-fluoro-2-[(4-methoxyphenyl)methylthioalkyl]benzoic acid 31c (20 g, 68.42 mmol) was dissolved in N,N-dimethylformamide (200 mL), followed by the addition of cyclopropylamine (9.6 g, 102.63 mmol), 1-ethyl-(3-dimethylaminopropyl)carbonyldiimine hydrochloride (19.7 g, 102.63 mmol), 1-hydroxybenzotriazole (13.9 g, 102.63 mmol) and N,N-diisopropylethylamine (26.5 g, 205.25 mmol). The reaction system was stirred at 25°C to react for 2 h. The reaction mixture was poured into water (1000 mL) and extracted with ethyl acetate (500 mL × 3), and the organic

phases were combined, washed with saturated brine (300 mL), dried over anhydrous sodium sulfate, and concentrated under reduced pressure to obtain a crude product. The crude product was purified by the medium-pressure preparative chromatography to obtain a white solid product N-cyclopropyl-3-fluoro-2-[(4-methoxyphenyl)methylthioalkyl]benzamide 50a (15 g, 45.26 mmol, yield: 66.16 %).

The product was verified by LCMS and H-NMR.

For MS-ESI, the calculated value was $[M+H]^+$332.1, and the measurement was 332.0.

$^1$H NMR (400 MHz, $CDCl_3$) δ (ppm) 7.34-7.31 (m, 2H), 7.17-7.12 (m, 1H), 6.97 (d, *J*= 8.4 Hz, 2H), 6.74 (d, *J*= 8.4 Hz, 2H), 4.00 (s, 2H), 3.76 (s, 3H), 2.77-2.73 (m, 1H), 0.81-0.76 (m, 2H), 0.48-0.44 (m, 2H).

Step 4

At room temperature, N-cyclopropyl-3-fluoro-2-[(4-methoxyphenyl)methylthioalkyl]benzamide 50a (24 g, 72.42 mmol) was dissolved in trifluoroacetic acid (240 mL). The reaction system was stirred at 70°C under the protection of nitrogen to react for 5 h. The reaction mixture was concentrated under reduced pressure to obtain a crude product. The crude product was purified by the medium-pressure preparative chromatography to obtain a yellow solid product N-cyclopropyl-3-fluoro-2-mercapto-benzamide 50b (7 g, 33.14 mmol, yield: 45.75 %).

The product was verified by LCMS and HNMR.

For MS-ESI, the calculated value was $[M+H]^+$212.1, and the measurement was 212.1.

$^1$H NMR (400 MHz, $CDCl_3$) δ (ppm) 7.24-7.18 (m, 1H), 7.15-7.06 (m, 2H), 6.24 (s, 1H), 4.73 (s, 1H), 2.91-2.86 (m, 1H), 0.91-0.82 (m, 2H), 0.70-0.63 (m, 2H).

Step 5

At room temperature, N-cyclopropyl-3-fluoro-2-mercapto-benzamide 50b (7 g, 33.14 mmol) was dissolved in N,N-dimethylformamide (100 mL), followed by the addition of 6-iodo-1H-indazole 1d (8.1 g, 33.14 mmol), tris(dibenzalaceton)dipalladium (3 g, 3.31 mmol), 4,5-bisdiphenylphosphino-9,9-dimethylxanthene (3.8 g, 6.62 mmol) and cesium carbonate (32.4 g, 99.41 mmol). The reaction system was stirred at 90°C under the protection of nitrogen to react for 16 h. The reaction mixture was poured into water (500 mL), and then extracted with ethyl acetate (100 mL × 3), and the organic phases were combined, washed with saturated brine (100 mL), dried over anhydrous sodium sulfate, and concentrated under reduced pressure to obtain a crude product. The crude product was purified by the medium-pressure preparative chromatography to obtain a yellow solid product N-cyclopropyl-3-fluoro-2-(1H-indazol-6-ylthioalkyl)benzamide 50c (5.6 g, 17.11 mmol, yield: 51.62 %).

The product was verified by LCMS, H-NMR.

For MS-ESI, the calculated value was $[M+H]^+$328.1, and the measurement was 328.0

$^1$H NMR (400MHz,$CD_3OD$): δ (ppm) 7.97 (s, 1H), 7.62 (d, *J* = 8.8 Hz, 1H), 7.54-7.46 (m, 1H), 7.38 (s, 1H), 7.28-7.25 (m, 2H), 7.00 (dd, *J* = 9.6, 1.2 Hz, 1H), 2.78-2.73 (m, 1H), 0.74-0.69 (m, 2H), 0.51-0.47 (m, 2H).

Step 6

At room temperature, N-cyclopropyl-3-fluoro-2-(1H-indazol-6-ylthioalkyl)benzamide 50c (2.5 g, 7.64 mmol) was dissolved in N,N-dimethylformamide (30 mL), followed by the addition of potassium carbonate (3.2 g, 22.91 mmol) and iodine (3.9 g, 15.27 mmol). The reaction system was stirred at 40°C to react for 2 h. The reaction mixture was poured into water (200 mL), and then extracted with ethyl acetate (100 mL × 3), and the organic phases were combined, washed with saturated brine (100 mL), dried over anhydrous sodium sulfate, and concentrated under reduced pressure to obtain a yellow solid product N-cyclopropyl-3-fluoro-2-[(3-iodo-1H-indazol-6-yl)thioalkyl]benzamide 50d (2.5 g, 5.52 mmol, yield: 72.23 %).

The product was verified by LCMS and H-NMR.

For MS-ESI, the calculated value was $[M+H]^+$454.0, and the measurement was 453.9

$^1$H NMR (400MHz, DMSO_*d*6): δ (ppm) 13.38 (s, 1H), 8.53 (d, *J*= 4.0 Hz, 1H), 7.62-7.54 (m, 1H), 7.41-7.30 (m, 4H), 7.04 (d, *J*= 8.4 Hz, 1H), 2.79-2.74 (m, 1H), 0.68-0.63 (m, 2H), 0.48-0.45 (m, 2H).

Step 7

At room temperature, N-cyclopropyl-3-fluoro-2-[(3-iodo-1H-indazol-6-yl)thioalkyl]benzamide 50d (6 g, 13.24 mmol) was dissolved in ethyl acetate (80 mL), followed by the addition of p-toluenesulfonic acid (1.1 g, 6.62 mmol) and 3.4dyhydro-2H-pyran (3.3 g, 39.71 mmol). The reaction system was stirred at 70°C to react for 3 h. The reaction mixture was concentrated under reduced pressure to obtain a crude product. The crude product was purified by the medium-pressure preparative chromatography to obtain a yellow solid product N-cyclopropyl-3-fluoro-2-(3-iodo-1-tetrahydropyran-2-yl-indazol-6-yl)thioalkylbenzamide 41a (4.2 g, 7.82 mmol, yield: 59.04 %).

The product was verified by LCMS and H-NMR.

For MS-ESI, the calculated value was $[M+H]^+$538.0, and the measurement was 537.9

$^1$H NMR (400 MHz, DMSO_*d*6) δ (ppm) 8.53 (d, J = 4.0 Hz, 1H), 7.69 (s, 1H), 7.60-7.52 (m, 1H), 7.41-7.28 (m, 3H), 6.95 (d, *J* = 8.8 Hz, 1H), 5.85-5.73 (m, 1H), 3.85 (d, *J* = 11.6 Hz, 1H), 3.70 (t, *J* = 12.8 Hz, 1H), 2.79-2.73 (m, 1H), 2.31 (dd, *J* = 21.2, 8.8 Hz, 2H), 1.99-1.91 (m, 2H), 1.72 (s, 1H), 1.55 (s, 2H), 0.65 (dd, *J* = 6.8, 2.0 Hz, 2H), 0.51 - 0.42 (m, 2H).

Step 8

At room temperature, N-cyclopropyl-3-fluoro-2-(3-iodo-1-tetrahydropyran-2-yl-indazol-6-yl)thioalkyl benzamide 41a (2 g, 3.72 mmol) was dissolved in dioxane (20 mL), followed by the addition of 5-(pyrrolidin-1-ylmethyl)-2-vinylpyridine 30a (841 mg, 4.47 mmol), dichloropalladium (II) (330 mg, 1.86 mmol), tris(o-methylphenyl)phosphine (1.1 g, 3.72 mmol) and triethylamine (1.9 g, 18.61 mmol). The reaction system was stirred at 90°C to react for 16 h under the protection of nitrogen. The reaction mixture was poured into water (100 mL) and extracted with ethyl acetate (100 mL × 3), and the organic phases were combined, washed with saturated brine (50 mL), dried over anhydrous sodium sulfate, and concentrated under reduced pressure to obtain a crude product. The crude product was purified by medium-pressure preparative chromatography to obtain a yellow solid product, N-cyclopropyl-3-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 50e (1.4 g, 2.34 mmol, yield: 62.93 %).

The product was verified by LCMS.

For MS-ESI, the calculated value was $[M+H]^+$598.3, and the measurement was 598.1

Step 9

At room temperature, N-cyclopropyl-3-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 50e (1.4 g, 2.34 mmol) was dissolved in dichloromethane (18 mL), followed by the addition of trifluoroacetic acid (6 mL). The reaction system was stirred at 25°C to react for 16 h. The reaction mixture was poured into water (100 mL), adjusted with the saturated aqueous solution of sodium carbonate to the pH of 8-9, and then extracted with dichloromethane (100 mL × 3), and the organic phases were combined, washed with saturated brine (50 mL), dried over anhydrous sodium sulfate, and concentrated under reduced pressure to obtain a crude product. The crude product was purified by the high-pressure preparative chromatography to obtain a yellow solid product N-cyclopropyl-3-fluoro-2-(3-[(E)-2-{5-[(pyrrolidin-1-yl)methyl]pyridin-2-yl}vinyl]-1H-indazol-6-yl}thio)benzamide I-67 (214.47 mg, 417.56 μmol, yield: 17.83 %).

The product was verified by LCMS, F-NMR and H-NMR.

For MS-ESI, the calculated value was $[M+H]^+$514.2, and the measurement was 514.2

$^1$H NMR (400MHz,$CD_3OD$): δ (ppm) 8.51 (d, *J* = 1.6 Hz, 1H), 7.98 (d, *J* = 8.8 Hz, 1H), 7.88-7.79 (m, 2H), 7.67 (d, *J* = 8.0 Hz, 1H), 7.58-7.47 (m, 2H), 7.39 (s, 1H), 7.30-7.26 (m, 2H), 7.13-7.10 (m, 1H), 3.70 (s, 2H), 2.78-2.74 (m, 1H), 2.59 (s, 4H), 1.84 (s, 4H), 0.78-0.68 (m, 2H), 0.52-0.48 (m, 2H).

$^{19}$F NMR (400MHz,$CD_3OD$): δ (ppm)-105.88.

I-68

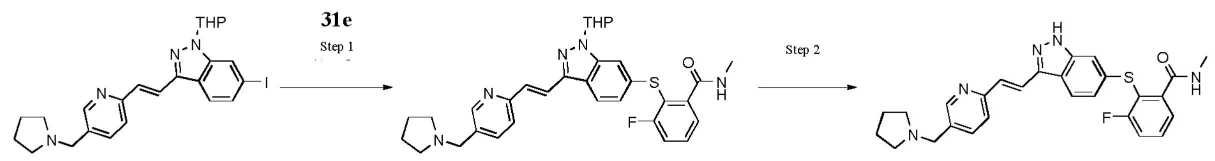


51a 51b I-68

Step 1

At room temperature, 6-iodo-3-[(trans)-2-[5-(pyrrolidin-1-ylmethyl)-2-pyridyl]vinyl]-1-tetrahydropyran-2-ylindazole 51a (190.0 mg, 369.36 μmol) was dissolved in N.N-dimethylformamide (5 mL), followed by the addition of 3-fluoro-N-methyl-2-mercaptobenzamide 31e (68.4 mg, 369.36 μmol), 1,1-bis(diphenylphosphino)ferrocene dichloropalladium (II) (107.2 mg, 147.74 μmol) and cesium carbonate (240.7 mg, 738.72 μmol). The reaction system was stirred at 80°C under the protection of nitrogen to react for 16 h. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the preparative plate (DCM/MeOH = 10/1)to obtain a brown solid product 3-fluoro-N-methyl-2-[3-[(trans)-2-[5-(pyrrolidin-1-ylmethyl)-2-pyridyl]vinyl]-1-tetrahydropyran-2-ylindol-6-yl]thioalkylbenzamide 51b (70 mg, 122.44 μmol, yield: 33.15 %).

The product was verified by LCMS.

For MS-ESI, the calculated value was $[M+H]^+$ 572.2, and the measurement was 572.1.

Step 2

At room temperature, 3-fluoro-N-methyl-2-[3-[(trans)-2-[5-(pyrrolidin-1-ylmethyl)-2-pyridyl]vinyl]-1-tetrahydropyran-2-ylindol-6-yl]thioalkyl benzamide 51b (70.0 mg, 122.44 μmol) was dissolved in dichloromethane (5 mL), followed by the addition of trifluoroacetic acid (139.6 mg, 1.22 mmol); the reaction system was stirred at 25°C to react for 4 h. The reaction mixture was poured into water (20 mL), adjusted with the saturated sodium carbonate solution to the pH of 8-9 and extracted with dichloromethane (20 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a yellow solid product 3-fluoro-N-methyl-2-(3-[(E)-2-{5-[(pyrrolidin-1-yl)methyl]pyridin-2-yl}vinyl]-1H-indazol-6-yl}thio)benzamide I-68 (10.02 mg, 20.55 μmol, yield: 16.78 %).

The product was verified by LCMS, F-NMR, H-NMR and C-NMR.

For MS-ESI, the calculated value was $[M+H]^+$ 488.2, and the measurement was 488.3.

$^1$H NMR (400MHz, $CD_3OD$): δ (ppm) 8.49 (d, J = 1.2 Hz, 1H), 7.97 (d, J= 8.8 Hz, 1H), 7.84-7.79 (m, 2H), 7.64 (d, J= 8.0 Hz, 1H), 7.54-7.50 (m, 2H), 7.39 (s, 1H), 7.32-7.26 (m, 2H), 7.12 (d, J=8.4 Hz, 1H), 3.69 (s, 2H), 2.83 (s, 3H), 2.59 (s, 4H), 1.83 (s, 4H).

$^{13}$C NMR (100MHz, $CD_3OD$): δ (ppm) 170.76, 170.74, 165.40, 162.92, 155.98, 151.01, 145.43, 143.73, 143.47, 139.57, 136.56, 134.17, 132.62, 132.53, 130.32, 125.35, 124.63, 124.79, 124.04, 122.10, 121.12, 119.98, 118.56, 118.32, 111.71, 58.15, 54.92, 26.69, 24.19.

$^{19}$F NMR (400MHz, $CD_3OD$): δ (ppm) -105.58.

I-69

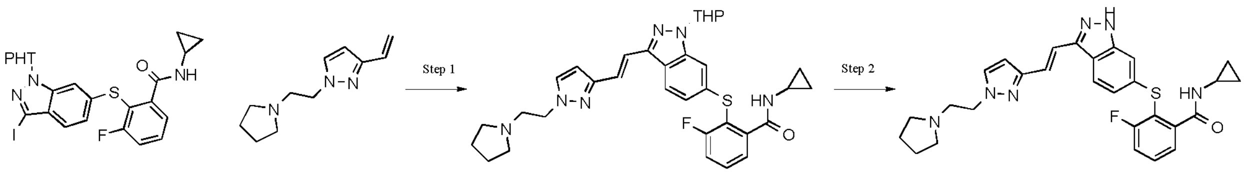


41a 17c 52a I-69

Step 1

At room temperature, N-cyclopropyl-3-fluoro-2-(3-iodo-1-tetrahydropyran-2-yl-indazol-6-yl)thioalkyl benzamide 41a (2 g, 3.72 mmol) was dissolved in 1,4-dioxane (20 mL), followed by the addition of 1-(2-pyrrolidin-1-yl)-3-vinylpyrazole 17c (712 mg, 3.72 mmol), triethylamine (1.1 g, 11.17 mmol), tris(o-methylphenyl)phosphine (1.1 g, 3.72 mmol) and palladium chloride (330 mg, 1.86 mmol). The reaction system was stirred at 90°C to react for 16 h under the protection of nitrogen. The reaction mixture was poured into water (50 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 50/1-10/1) to obtain a yellow solid product N-cyclopropyl-3-fluoro-2-[3-[(trans)-2-[1-(2-pyrrolidin-1-yl)pyrazol-3-yl]vinyl]-1-tetrahydropyran-2-yl-indazol-6-yl]thioalkylbenzamide 52a (1 g, 1.66 mmol, yield: 44.73 %).

The product was verified by LCMS.

For MS-ESI, the calculated value was $[M+H]^+$ 601.3, and the measurement was 601.1.

Step 2

At room temperature, N-cyclopropyl-3-fluoro-2-[3-[(trans)-2-[1-(2-pyrrolidin-1-yl)pyrazole -3-yl]vinyl]-1-tetrahydropyran-2-yl-indazol-6-yl]thioalkyl benzamide 52a (1 g, 1.66 mmol) was dissolved in dichloromethane (24 mL), followed by the addition of trifluoroacetic acid (8 mL). The reaction system was stirred at 25°C to react for 16 h. The reaction mixture was poured into water (100 mL), adjusted with the saturated sodium carbonate solution to the pH of 8 and extracted with dichloromethane (100 mL × 3), and the organic phases were combined, washed with saturated brine (100 mL), dried over anhydrous sodium sulfate, and concentrated under reduced pressure to obtain a crude product. The crude product was

purified by the high-pressure liquid chromatography to obtain a yellow solid product N-cyclopropyl-3-fluoro-2-(3-[(E)-2-{1-[2-(pyrrolidin-1-yl)ethyl]-1H-pyrazol-3-yl}vinyl]-1H-indazol-6-yl}thio)benzamide I-69 (68.04 mg, 131.70 μmol, yield: 7.91 %).

The product was verified by LCMS, F-NMR, H-NMR and C-NMR.

For MS-ESI, the calculated value was $[M+H]^+$517.2, and the measurement was 517.1.

$^1$H NMR (400MHz, $CD_3OD$): δ (ppm) 7.90 (d, *J*= 8.8 Hz, 1H), 7.63 (d, *J* = 2.4 Hz, 1H), 7.53-7.48 (m, 1H), 7.42-7.32 (m, 3H), 7.29-7.24 (m, 2H), 7.08 (dd, *J* = 8.4, 1.6 Hz, 1H), 6.60 (d, *J* = 2.4 Hz, 1H), 4.29 (t, *J* = 6.8 Hz, 2H), 2.99 (t, *J* = 6.8 Hz, 2H), 2.78-2.74 (m, 1H), 2.58-2.56 (m, 4H), 1.81-1.78 (m, 4H), 0.75-0.70 (m, 2H), 0.52-0.48 (m, 2H).

$^{13}$C NMR (100 MHz, $CD_3OD$&$CDCl_3$) δ (ppm) 171.63, 165.42, 162.95, 152.06, 145.24, 136.49, 133.08, 132.60, 132.52, 124.80, 124.76, 123.98, 123.69, 122.19, 120.77, 120.10, 119.91, 118.59, 118.35, 111.63, 103.94, 56.87, 55.22, 30.80, 24.33, 23.81, 14.59, 6.54.

$^{19}$F NMR (400MHz, $CD_3OD$): δ (ppm) -105.52.

I-73

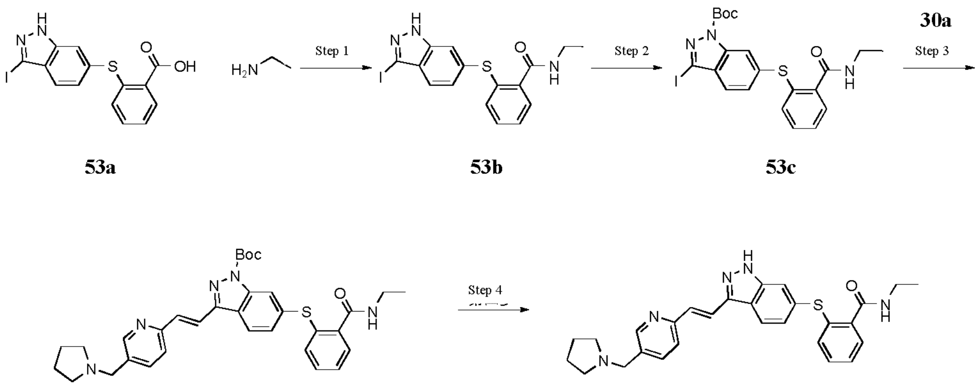


53a 53b 53c

53d I-73

Step 1

At room temperature, 2-[(3-iodo-1H-indazol-6-yl)thioalkyl]benzoic acid 53a (1.5 g, 3.79 mmol) was dissolved in N,N-dimethylformamide (20 mL), followed by the addition of ethylamine (617 mg, 7.57 mmol), HATU (2.2 g, 5. 68 mmol) and N,N-diisopropylethylamine (2.4 g, 18.93 mmol); the reaction system was stirred at 25°C to react for 2 h. The reaction mixture was poured into water (50 mL) and extracted with dichloromethane (50 mL × 3), and the organic phases were combined, washed with saturated brine (40 mL), dried over anhydrous sodium sulfate, and concentrated under reduced pressure to obtain a crude product. The crude product was purified by the medium-pressure preparative chromatography to obtain a brown solid product N-ethyl-2-[(3-iodo-1H-indazol-6-yl)thioalkyl]benzamide 53b (1.3 g, 3.07 mmol, yield: 81.12 %).

The product was verified by LCMS and HNMR.

For MS-ESI, the calculated value was $[M+H]^+$424.0, and the measurement was 424.0.

$^1$H NMR (400MHz, $CDCl_3$): δ (ppm) 12.93 (s, 1H), 7.99 (d, *J* = 8.2 Hz, 1H), 7.55 (d, *J* = 9.0 Hz, 2H), 7.44-7.38 (m, 1H), 7.27-7.07 (m, 4H), 4.19-4.00 (m, 2H), 1.34 (d, *J* = 9.0 Hz, 3H).

Step 2

At room temperature, N-ethyl-2-[(3-iodo-1H-indazol-6-yl)thioalkyl]benzamide 53b (1.3 g, 3.07 mmol) was dissolved in tetrahydrofuran (10 mL), followed by the addition of triethylamine (1.2 g, 12.29 mmol) and di-tert butyl dicarbonate (1.3 g, 6.14 mmol); the reaction system was stirred at 60°C to react for 16 h. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a crude product. The crude product was purified by the medium-pressure preparative chromatography to obtain a white solid product 6-[2-(ethylaminoformyl)phenyl]thioalkyl-3-iodoindazol-1-tert-butyl carboxylate 53c (1.5 g, 2.87 mmol, yield: 93.31 %).

The product was verified by LCMS and HNMR.

For MS-ESI, the calculated value was $[M+H]^+$524.0, and the measurement was $[M-100]^+$424.0.

$^1$H NMR (400MHz, $CDCl_3$): δ (ppm) 8.05 (s, 1H), 7.66 (dd, *J* = 4.8, 1.6 Hz, 1H), 7.42-7.31 (m, 4H), 7.27 (d, *J* = 1.2

Hz, 1H), 6.29 (s, 1H), 2.80 (s, 2H), 1.63 (s, 9H), 1.14 (t, *J* = 7.4 Hz, 3H).

Step 3

At room temperature, 6-[2-(ethylcarbamoyl)phenyl]thioalkyl-3-iodoindazol-1-tert-butyl carboxylate 53c (500 mg, 955.32 μmol) was dissolved in dioxane (10 mL), followed by the addition of 5-(pyrrolidin-1-ylmethyl)-2-vinylpyridine 30a (180 mg, 955.32 μmol), tris(o-methylphenyl)phosphine (290 mg, 955.32 μmol), palladium chloride (85 mg, 477.66 μmol) and triethylamine (483 mg, 4.78 mmol). The reaction system was stirred at 95 °C under the protection of nitrogen to react for 1.5 h. The reaction mixture was poured into water (10 mL) and extracted with ethyl acetate (10 mL × 3), and the organic phases were combined, washed with saturated brine (20 mL), dried over anhydrous sodium sulfate, and concentrated under reduced pressure to obtain a brown solid crude product 6-[2-(ethylaminoformyl)phenyl]thioalkyl-3-[(trans)-2-[5-(pyrrolidin-1-ylmethyl)-2-pyridyl]vinyl]indazol-1-tert-butyl formate 53d (200 mg, 342.62 μmol, yield: 35.86 %).

The product was verified by LCMS.

For MS-ESI, the calculated value was $[M+H]^+$584.3, and the measurement was 584.3.

Step 4

At room temperature, 6-[2-(ethylcarbamoyl)phenyl]thioalkyl-3-[(trans)-2-[5-(pyrrolidin-1-ylmethyl)-2-pyridyl]vinyl]indazol-1-tert-butyl formate 53d (350 mg, 599.58 μmol) was dissolved in dichloromethane (9 mL), followed by the addition of trifluoroacetic acid (3 mL); the reaction liquid was stirred at 25°C to react for 2 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure preparative chromatography to obtain a yellow solid product N-ethyl-2-({3-[(E)-2-{5-[(pyrrolidin-1-yl) methyl]pyridin-2-yl}vinyl]-1H-indazol-6-yl}thio)benzamide I-73 (53 mg, 109.59 μmol, yield: 18.28 %).

The product was verified by LCMS, H-NMR and C-NMR.

For MS-ESI, the calculated value was $[M+H]^+$484.2, and the measurement was 484.2.

$^1$H NMR (400MHz, $CD_3OD$): δ (ppm) 8.66 (d, *J* = 1.6 Hz, 1H), 8.48 (s, 1H), 8.05 (d, *J* = 8.6 Hz, 1H), 7.99-7.88 (m, 2H), 7.72 (d, *J* = 8.2 Hz, 1H), 7.61-7.52 (m, 2H), 7.51-7.44 (m, 1H), 7.39-7.28 (m, 2H), 7.28-7.17 (m, 2H), 4.32 (s, 2H), 3.34 (dd, *J* = 14.2, 6.8 Hz, 2H), 3.25 (s, 4H), 2.06 (s, 4H), 1.16 (t, *J* = 7.4 Hz, 3H).

$^{13}$C NMR (100 MHz, $CD_3OD$) δ (ppm) 171.00, 169.53, 157.82, 151.96, 143.62, 140.34, 139.56, 135.84, 133.17, 131.62, 129.10, 128.13, 126.62, 126.54, 123.38, 122.45, 121.65, 114.98, 56.51, 54.85, 35.80, 23.94, 14.70.

I-74

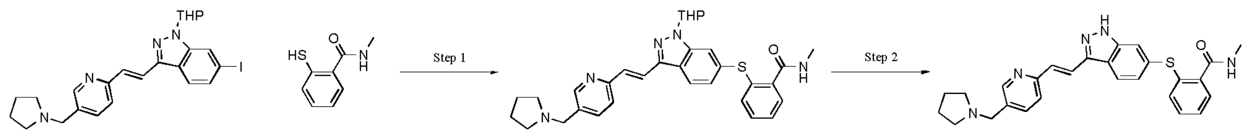


51a 1e 54a I-74

Step 1

At room temperature, 6-iodo-3-[(trans)-2-[5-(pyrrolidin-1-ylmethyl)-2-pyridyl]vinyl]-1-tetrahydropyran-2-ylindazole 51a (200 mg, 388.80 μmol) was dissolved in N,N-dimethylformamide (6 mL), followed by the addition of N-methyl-2-mercapto-benzamide 1e (65 mg, 388.80 μmol), [1,1'-bis(diphenylphosphino)ferrocene]dichloropalladium (II) (100 mg, 136.80 μmol) and cesium carbonate (253 mg, 777.60 μmol). The reaction system was stirred at 80°C under the protection of nitrogen to react for 6 h. The reaction mixture was poured into water (30 mL), and then extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a brown solid product N-methyl-2-[3-[(trans)-2-[5-(pyrrolidin-1-ylmethyl)-2-pyridyl]vinyl]-1-tetrahydropyran-2-ylindazol-6-yl]thioalkylbenzamide 54a (100 mg, 180.60 μmol, yield: 46.45 %).

The product was verified by LCMS.

For MS-ESI, the calculated value was $[M+H]^+$554.3, and the measurement was 554.4.

Step 2

At room temperature, N-methyl-2-[3-[(trans)-2-[5-(pyrrolidin-1-ylmethyl)-2-pyridyl]vinyl]-1-tetrahydropyran-2-ylindazol-6-yl]thioalkyl benzamide 54a (150 mg, 270.90 μmol) was dissolved in dichloromethane (10 mL), followed by the addition of trifluoroacetic acid (2 mL). The reaction system was stirred at 25°C to react for 3 h. The reaction mixture was poured into water (50 mL), adjusted with the saturated aqueous solution of sodium carbonate to the pH of 8-9, and then extracted with dichloromethane (50 mL × 3), and the organic phases were combined, washed with saturated brine (50 mL), dried over anhydrous sodium sulfate, and concentrated under reduced pressure to obtain a crude product. The crude product was purified by the high-pressure preparative chromatography to obtain a white solid product N-methyl-2-({3-[(E)-2-{5-[(pyrrolidin-1-yl)methyl]pyridin-2-yl}vinyl]-1H-indazol-6-yl}thio)benzamide I-74 (29.2 mg, 62.07 μmol, yield: 22.91 %).

The product was verified by LCMS, H-NMR and C-NMR.

For MS-ESI, the calculated value was $[M+H]^+$470.2, and the measurement was 470.1

$^1H$ NMR (400 MHz, $CD_3OD$): δ (ppm) 8.53 (s, 1H), 8.07 (d, *J* = 8.6 Hz, 1H), 7.85 (dd, *J* = 18.2, 9.4 Hz, 2H), 7.69 (d, *J*= 8.2 Hz, 1H), 7.57 (d, *J* = 16.8 Hz, 2H), 7.49-7.45 (m, 1H), 7.34-7.31 (m, 2H), 7.23 (dd, *J* = 9.0, 4.6 Hz, 1H), 3.74 (s, 2H), 2.86 (s, 2H), 2.63 (s, 4H), 1.85 (s, 4H).

$^{13}C$ NMR (100MHz, $CD_3OD$): δ (ppm) 171.73, 156.09, 151.05, 139.64, 132.60, 131.78, 130.52, 129.11, 127.88, 127.04, 122.94, 122.64, 79.44, 79.11, 78.79, 58.37, 55.10, 27.14, 24.32

I-75

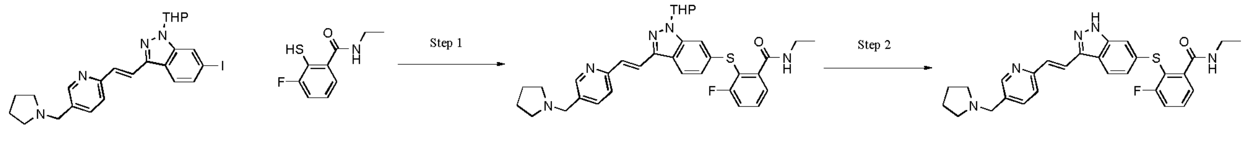


51a 55a 55b I-75

Step 1

Under the protection of nitrogen at room temperature, 6-iodo-3-[(trans)-2-[5-(pyrrolidin-1-ylmethyl)-2-pyridyl]vinyl]-1-tetrahydropyran-2-ylindole 51a (180 mg, 349.92 μmol) was dissolved in DMF (6 mL), followed by the addition of N-ethyl-3-fluoro-2-thioalkyl-benzamide 55a (69.7 mg, 349.92 μmol), cesium carbonate (228.2 mg, 700.23 μmol) and $Pd(dppf)Cl_2$ (90 mg, 124.03 μmol). The reaction system was stirred at 80°C to react for 5.0 h. The reaction mixture was poured into water (30 mL), extracted with EA (30 mL × 3), and the organic phases were combined, washed with saturated brine (30 mL), dried over anhydrous sodium sulfate, and concentrated under reduced pressure to obtain a crude product. The crude product was purified by the preparative thin-layer chromatography (DCM/MeOH = 10/1) to obtain a brown solid crude product N-ethyl-3-fluoro-2-[3-[(trans)-2-[5-(pyrrolidin-1-ylmethyl)-2-pyridyl]vinyl]-1-tetrahydropyran-2-ylindol-6-yl]thioalkylbenzamide 55b (400 mg, crude).

The product was verified by LCMS.

For MS-ESI, the calculated value was $[M+H]^+$ 586.3, and the measurement was 586.3.

Step 2

N-ethyl-3-fluoro-2-[3-[(trans)-2-[5-(pyrrolidin-1-ylmethyl)-2-pyridyl]vinyl]-1-tetrahydropyran-2-ylindol-6-yl] thioalkyl benzamide 55b (80 mg, 136.58 μmol) was dissolved in DCM (10 mL), followed by the addition of trifluoroacetic acid (155.7 mg, 1.37 mmol). The reaction system was stirred at 25°C to react for 2 h. The reaction mixture was poured into water (20 mL), adjusted with the saturated aqueous solution of sodium bicarbonate to the pH of 8-9, and extracted with DCM (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-ethyl-3-fluoro-2-({3-[(E)-2-{5-[(pyrrolidin-1-yl)methyl]pyridin-2-yl}vinyl]-1H-indazol-6-yl}thio)benzamide I-75 (21.51 mg, 42.88 μmol, yield: 31.40 %).

The product was verified by LCMS, F-NMR, H-NMR and C-NMR.

For MS-ESI, the calculated value was $[M+H]^+$ 502.2, and the measurement was 502.2.

$^1H$ NMR (400 MHz, $CD_3OD$): δ (ppm) 8.63 (s, 1H), 7.98-7.83 (m, 3H), 7.70 (d, J=8.0, 1H), 7.56-7.51 (m, 2H), 7.38-7.27 (m, 3H), 7.13 (d, J=8.4, 1H), 4.26 (s, 2H), 3.35-3.29 (m, 2H), 3.19 (s, 4H), 2.03 (s, 4H), 1.13 (t, J=7.2, 3H).

$^{13}C$ NMR (100 MHz, $CD_3OD$): δ (ppm) 169.99, 169.97, 165.45, 162.97, 157.67, 151.86, 145.65, 143.56, 143.49,140.29, 136.77, 132.75, 132.67, 128.52, 126.49, 123.31, 122.06, 121.10, 119.85, 119.65, 118.50, 118.26,

111.52, 56.58, 54.82, 35.82, 23.95, 14.62

$^{19}F$ NMR (400 MHz, $CD_3OD$): δ (ppm) -105.69.

I-76

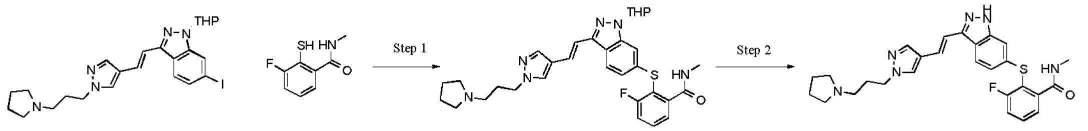


56a 31e 56b I-76

Step 1

Under the protection of nitrogen at room temperature, 6-iodo-3-[(trans)-2-[1-(3-pyrrolidin-1-ylpropyl)pyrazole -4-yl]vinyl]-1-tetrahydropyran-2-ylindazole 56a (200 mg, 376.34 μmol) was dissolved in DMF (6 mL), followed by the addition of 3-fluoro-N-methyl-2-thioalkyl benzamide 31e (69.7 mg, 376.34 μmol), cesium carbonate (245.2 mg, 752.69 μmol) and Pd(dppf)$Cl_2$ (136.6 mg, 188.17 μmol). The reaction system was stirred at 80°C to react for 5.0 h. The reaction mixture was poured into water (30 mL), extracted with EA (10 mL × 3), and the organic phases were combined, washed with saturated brine (30 mL), dried over anhydrous sodium sulfate, and concentrated under reduced pressure to obtain a crude product. The crude product was purified by the preparative thin-layer chromatography (DCM/MeOH = 10/1) to obtain a yellow solid product 3-fluoro-N-methyl-2-[3-[(E)-2-[1-(3-pyrrolidin-1-ylpropyl)pyrazol-4-yl]vinyl]-1-tetrahydropyran-2-ylindazol-6-yl] thioalkylbenzamide 56b (110 mg, 186.84 μmol, yield: 49.65 %).

The product was verified by LCMS.

For MS-ESI, the calculated value was $[M+H]^+$ 589.3, and the measurement was 589.3.

Step 2

3-fluoro-N-methyl-2-[[3-[(E)-2-[1-(3-pyrrolidin-1-ylpropyl)pyrazole -4-yl]vinyl]-1H-indazol-6-yl]sulfanilamido] benzamide 56b (60 mg, 101.91 μmol) was dissolved in DCM (5 mL), followed by the addition of trifluoroacetic acid (11.62 mg, 101.91 μmol). The reaction system was stirred at 25°C to react for 2.0 h. The reaction mixture was poured into water (30 mL), adjusted with the saturated aqueous solution of sodium bicarbonate to the pH of 8-9, and extracted with DCM (20 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product 3-fluoro-N-methyl-2-({3-[(E)-2-{1-[3-(pyrrolidin-1-yl) propyl]-1H-pyrazol-4-yl}vinyl]-1H-indazol-6-yl}thio)benzamide I-76 (6.91 mg, 13.96 μmol, yield: 13.44 %).

The product was verified by LCMS, F-NMR, H-NMR and C-NMR.

For MS-ESI, the calculated value was $[M+H]^+$505.2, and the measurement was 505.2.

$^1H$ NMR (400MHz, $CD_3OD$): δ (ppm) 7.90-7.89 (m, 2H), 7.82 (s, 1H), 7.55-7.50 (m, 1H), 7.34-7.26 (m, 4H), 7.17-7.06 (m, 2H), 4.27 (t, J=6.4 Hz, 2H), 3.21 (s, 4H), 3.07 (t, J=4.6 Hz, 2H), 2.83 (s, 3H), 2.29-2.22 (m, 2H), 2.04 (s, 4H).

$^{13}C$ NMR (100MHz, $CD_3OD$) δ (ppm) 170.78, 162.93, 145.43, 138.87, 136.49, 132.62, 132.53, 129.85, 124.83, 122.46, 122.19, 120.64, 120.17, 119.26, 118.53, 118.29, 111.52, 55.21, 53.84, 50.11, 28.33, 26.66, 24.02.

$^{19}F$ NMR (400MHz, $CD_3OD$): δ (ppm) -105.67.

I-77

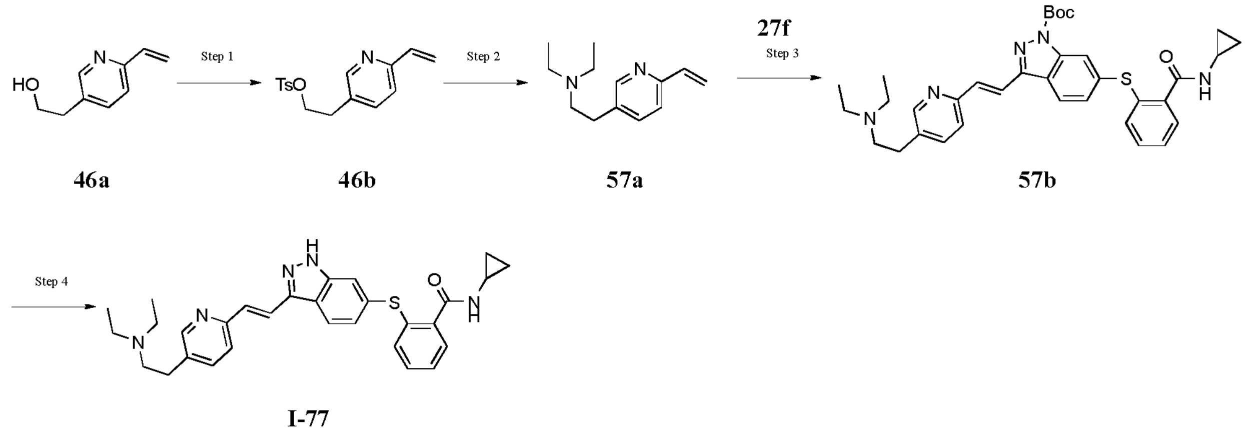


46a 46b 57a 57b

I-77

Step 1

At room temperature, 2-(6-vinyl-3-pyridyl)ethanol 46a (1 g, 6.70 mmol) was dissolved in acetonitrile (10 mL), followed by the addition of paratoluensulfonyl chloride (1.7 g, 8.71 mmol) and triethylamine (1.7 g, 16.76 mmol); the reaction system was stirred at 25°C to react for 16 h. The reaction mixture was poured into water (30 mL) and extracted with dichloromethane (30 mL × 3), and the organic phases were combined, washed with saturated brine (40 mL), dried over anhydrous sodium sulfate, and concentrated under reduced pressure to obtain a crude product. The crude product was purified by the preparative plate chromatography (petroleum ether:ethyl acetate = 3/1) to obtain a brown oily product 2-(6-vinylpyridin-3-yl)ethyl-4-methylbenzene sulfonate 46b ( 500 mg, 1.65 mmol, yield: 24.59 %).

The product was verified by LCMS and HNMR.

For MS-ESI, the calculated value was $[M+H]^+$304.1, and the measurement was 304.1.

$^1$H NMR (400MHz, $CDCl_3$): δ (ppm) 8.30 (d, *J* = 2.0 Hz, 1H), 7.66 (d, *J* = 8.4 Hz, 2H), 7.42 (dd, *J* = 8.0, 2.4 Hz, 1H), 7.26 (dd, J= 9.4, 4.6 Hz, 3H), 6.78 (dd, J= 17.6, 10.8 Hz, 1H), 6.16 (dd, *J* = 17.6, 1.2 Hz, 1H), 5.48 (dd,*J* = 10.8, 1.2 Hz, 1H), 4.22 (t, *J* = 6.6 Hz, 2H), 2.96 (s, 2H), 2.42 (s, 3H)..

Step 2

At room temperature, 2-(6-vinylpyridin-3-yl)ethyl-4-methylbenzene sulfonate 46b (400 mg, 1.32 mmol) was dissolved in acetonitrile (10 mL), followed by the addition of diethylamine (482 mg, 6.59 mmol); the reaction system was stirred at 55°C to react for 16 h. The reaction mixture was poured into water (20 mL) and extracted with ethyl acetate (20 mL × 3), and the organic phases were combined, washed with saturated brine (20 mL), dried over anhydrous sodium sulfate, and concentrated under reduced pressure to obtain a brown oily product N,N-diethyl-2-(6-vinyl-3-pyridyl)ethylamine 57a (200 mg, 978.90 μmol, yield: 74.24 %).

The product was verified by LCMS and HNMR.

For MS-ESI, the calculated value was $[M+H]^+$205.2, and the measurement was 205.1.

$^1$H NMR (400MHz, $CDCl_3$): δ (ppm) 8.40 (d, *J* = 2.2 Hz, 1H), 7.77 (d, *J* = 8.2 Hz, 2H), 6.79 (dd, *J* = 17.6, 10.8 Hz, 1H), 6.16 (dd, *J* = 17.6, 1.2 Hz, 1H), 5.47 (dd, *J* = 10.8, 1.2 Hz, 1H), 3.06-2.98 (m, 1H), 1.28-1.23 (m, 6H).

Step 3

At room temperature, N,N-diethyl-2-(6-vinyl-3-pyridyl)ethylamine 57a (191 mg, 933.89 μmo l) was dissolved in dioxane (10 mL), followed by the addition of 6-[2-(cyclopropylcarbamoyl)phenyl]thioalkyl-3-iodoindazol-1-tert-butyl carboxylate 27f (500 mg, 933.89 μmol), tris(o-methylphenyl)phosphine (284 mg, 933.89 μmol), palladium chloride (83 mg, 466.94 μmol) and triethylamine (473 mg, 4.67 mmol). The reaction system was stirred at 95°C under the protection of nitrogen to react for 2 h. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a brown solid crude product 6-[2-(cyclopropylaminoformyl) phenyl]thioalkyl-3-[(trans)-2-[5-[2-(diethylamino)ethyl]-2-pyridyl]vinyl]indazol-1-tert-butyl formate 57b (200 mg, 326.91 μmol, yield: 35.00 %).

The product was verified by LCMS.

For MS-ESI, the calculated value was $[M+H]^+$612.3, and the measurement was 612.2.

Step 4

At room temperature, 6-[2-(cyclopropylcarbamoyl)phenyl]thioalkyl-3-[(trans)-2-[5-[2-(diethylamino)ethyl]-2-pyridyl]vinyl]indazol-1-tert-butyl formate 57b (150 mg, 245.18 μmol) was dissolved in dichloromethane (9 mL), followed by the addition of trifluoroacetic acid (3 mL); the reaction liquid was stirred at 25 °C to react for 2 h. The reaction mixture was poured into water (30 mL), adjusted with the saturated sodium carbonate solution to the pH of 9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography to obtain a yellow solid product N-cyclopropyl-2-({3-[(E)-2-{5-[2-(diethylamino)ethyl] pyridin-2-yl}vinyl]-1H-indazol-6-yl}thio)benzamide I-77 (16 mg, 31.27 μmol, yield: 12.75 %).

The product was verified by LCMS, H-NMR and C-NMR.

For MS-ESI, the calculated value was $[M+H]^+$512.2, and the measurement was 512.2.

$^1$H NMR (400MHz, $CD_3OD$): δ (ppm) 8.52 (s, 1H), 8.34 (s, 2H), 8.05 (d, *J* = 8.4 Hz, 1H), 7.84 (dd, *J* = 19.6, 12.4 Hz, 2H), 7.67 (d, *J* = 8.2 Hz, 1H), 7.60-7.45 (m, 3H), 7.39-7.17 (m, 4H), 3.41 (dd, *J* = 10.4, 6.6 Hz, 2H), 3.35 (d, J = 7.4 Hz, 2H), 3.32-3.25 (m, 2H), 3.12 (dd, *J* = 10.4, 6.4 Hz, 2H), 2.85-2.74 (m, 1H), 1.38 *(t, J*= 7.2 Hz, 6H), 0.83-0.69 (m, 2H), 0.61-0.49 (m, 2H).

$^{13}$C NMR (100 MHz, $CD_3OD$) δ (ppm) 172.68, 167.46, 155.86, 150.62, 143.75, 143.62, 139.29, 139.02, 135.80, 133.27, 132.57, 131.72, 130.22, 129.17, 128.28, 126.40, 125.43, 123.28, 122.49, 121.57, 114.85, 53.42, 28.11, 23.82, 9.16, 6.50.

I-78

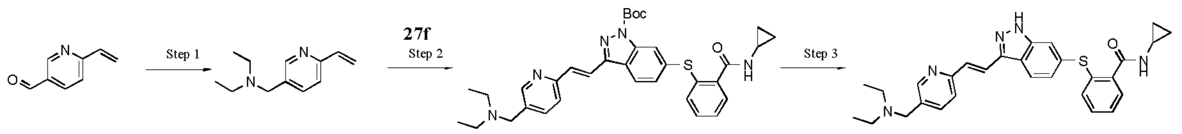


29b 58a 58b I-78

Step 1

At room temperature, 6-vinylpyridin-3-formaldehyde 29a (1 g, 7.51 mmol) was dissolved in anhydrous tetrahydrofuran (10 mL), followed by the addition of diethylamine hydrochloride (1.2 g, 11.27 mmol) and acetic acidsodium borohydride (4.8 g, 22.53 mmol); the reaction system was stirred at 25°C to react for 16 h. The reaction mixture was poured into water (50 mL) and extracted with ethyl acetate (40 mL × 3), and the organic phases were combined, washed with saturated brine (20 mL), dried over anhydrous sodium sulfate, and concentrated under reduced pressure to obtain a crude product. The crude product was purified by the preparative plate chromatography (dichloromethane:methanol = 10/1) to obtain a yellow oily product N-ethyl-N-[(6-vinyl-3-pyridyl)methyl]ethylamine 58a (700 mg, 3.68 mmol, yield: 48.98 %).

The product was verified by LCMS and HNMR.

For MS-ESI, the calculated value was $[M+H]^+$190.1, and the measurement was 190.1.

$^1$H NMR (400MHz, $CDCl_3$): δ (ppm) 8.50 (d, *J* = 1.6 Hz, 1H), 7.75 (dd, *J* = 8.0, 2.4 Hz, 1H), 7.36 (d, *J* = 8.0 Hz, 1H), 6.83 (dd, *J* = 17.6, 10.8 Hz, 1H), 6.18 (dd, *J* = 17.6, 1.2 Hz, 1H), 5.49 (dd, *J* = 10.8, 1.2 Hz, 1H), 3.71 (s, 2H), 2.64 (q, *J* = 7.2 Hz, 4H), 1.10 (t, *J* = 7.2 Hz, 6H).

Step 2

At room temperature, 6-[2-(cyclopropylcarbamoyl)phenyl]thioalkyl-3-iodoindazol-1-tert-butyl carboxylate 27f (500 mg, 933.89 μmol) was dissolved in dioxane (10 mL), followed by the addition of N-ethyl-N-[(6-vinyl-3-pyridyl)methyl] ethylamine 58a (178 mg, 933.89 μmol), tris(o-methylphenyl)phosphine (284 mg, 933.89 μmol), palladium chloride (83 mg, 466.94 μmol) and triethylamine (283.5 mg, 2.80 mmol). The reaction system was stirred at 95 °C under the protection of nitrogen to react for 1.5 h. The reaction mixture was poured into water (30 mL) and extracted with ethyl acetate (30 mL × 3), and the organic phases were combined, washed with saturated brine (40 mL), dried over anhydrous sodium sulfate, and concentrated under reduced pressure to obtain a crude product, which was purified by the preparative plate chromatography (dichloromethane:methanol = 10/1) to obtain a yellow solid product 6-[2-(cyclopropylaminoformyl) phenyl]thioalkyl-3-[(trans)-2-[5-(diethylaminomethyl)-2-pyridyl]vinyl]indazol-1-tert-butyl formate 58b (115 mg, 192.38 μmol, yield: 20.60 %).

The product was verified by LCMS.

For MS-ESI, the calculated value was $[M+H]^+$598.3, and the measurement was 598.2.

Step 3

At room temperature, 6-[2-(cyclopropylcarbamoyl)phenyl]thioalkyl-3-[(trans)-2-[5-(diethylaminomethyl)-2-pyridyl]vinyl]indazol-1-tert-butyl formate 58b (105 mg, 175.65 μmol) was dissolved in dichloromethane (5 mL), followed by the addition of trifluoroacetic acid (100 mg, 878.27 μmol); the reaction liquid was stirred at 25°C to react for 4 h. The reaction mixture was poured into water (10 mL), adjusted with the saturated sodium carbonate solution to the pH of 9, and then extracted with dichloromethane (20 mL × 3), and the organic phases were combined, washed with saturated brine (20 mL), dried over anhydrous sodium sulfate, and concentrated under reduced pressure to obtain a crude product, which was purified by the high-pressure liquid chromatography to obtain a white solid product N-cyclopropyl-2-({3-[(E)-2-{5-[(diethylamino)methy]pyridin-2-yl}vinyl]-1H-indazol-6-yl}thio)benzamide I-78 (15 mg, 30.14 μmol, yield: 17.16 %).

The product was verified by LCMS, H-NMR and C-NMR.

For MS-ESI, the calculated value was $[M+H]^+$498.2, and the measurement was 498.2.

$^1$H NMR (400MHz, $CD_3OD$): δ (ppm) 8.54 (s, 1H), 8.07 (d, *J* = 8.4 Hz, 1H), 7.90-7.84 (m, 2H), 7.70 *(d, J* = 7.6 Hz, 1H), 7.59-7.55 (m, 2H), 7.45 (dd, *J* = 6.8, 1.6 Hz, 1H), 7.37-7.32 (m, 2H), 7.27 -7.25 (m, 1H), 7.20 (d, *J* = 8.4 Hz, 1H), 3.79 (s, 2H), 2.80-2.75 (m, 1H), 2.70 (s, 4H), 1.15 (t, *J* = 7.2 Hz, 6H), 0.77-0.72 (m, 2H), 0.55-0.51 (m, 2H). $^{13}$C NMR (100 MHz, $CDCl_3$&$CD_3OD$) δ (ppm) 172.51, 155.83, 151.15, 139.66, 138.34, 132.98, 131.94, 130.74, 129.32, 128.07, 126.73, 125.37, 122.83, 122.74, 121.84, 115.48, 55.63, 24.07, 12.16, 7.13.

I-79

Step 1

Step 2

Step 1

At room temperature, 6-iodo-3-[(trans)-2-[5-(pyrrolidin-1-ylmethyl)-2-pyridyl]vinyl]-1-tetrahydropyran-2-ylindazole 51a (200 mg, 388.80 μmol) was dissolved in N, N-dimethylformamide (6 mL), followed by the addition of N-cyclopropyl-4-fluoro-2-mercaptobenzamide 59a (82.1 mg, 388.80 μmol), 1,1-bis(diphenylphosphino)ferrocene dichloropalladium (II) (100.0 mg, 136.80 μmol) and cesium carbonate (253.5 mg, 778.04 μmol). The reaction system was stirred at 80°C under the protection of nitrogen to react for 6 h. The reaction mixture was poured into water (20 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was purified by the preparative plate (DCM/MeOH = 10/1)to obtain a yellow solid product N-cyclopropyl-4-fluoro-2-[3-[(trans)-2-[5-(pyrrolidin-1-ylmethyl)-2-pyridyl]vinyl]-1-tetrahydropyran-2-ylindol-6-yl]thioalkylbenzamide 59b (200 mg, 334.59 μmol, yield: 86.06 %). The product was verified by LCMS.

For MS-ESI, the calculated value was $[M+H]^+$ 598.3, and the measurement was 598.4.

Step 2

At room temperature, N-cyclopropyl -4-fluoro-2-[3-[(trans)-2-[ 5-(pyrrolidin-1-ylmethyl)-2-pyridyl]vinyl]-1-tetrahydropyran-2-ylindol-6-yl]thioalkyl benzamide 59b (350 mg, 585.54 μmol) was dissolved in dichloromethane (10 mL), followed by the addition of trifluoroacetic acid (5 mL); the reaction system was stirred at 25°C to react for 2 h. The reaction mixture was poured into water (20 mL), adjusted with sodium carbonate to the pH of 8-9 and extracted with dichloromethane (20 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a white solid product N-cyclopropyl-4-fluoro-2-({3-[(E)-2-{5-[(pyrrolidin-1-yl)methyl]pyridin-2-yl}vinyl]-1H-indazol-6-yl}thio)benzamide I-79 (11.1 mg, 21.63 μmol, yield: 3.69 %).

The product was verified by LCMS, F-NMR, H-NMR and C-NMR.

For MS-ESI, the calculated value was $[M+H]^+$ 514.2, and the measurement was 514.2.

$^1$H NMR (400 MHz, $CD_3OD$) δ (ppm) 8.68 (s, 1H), 8.44 (s, 1H), 8.13 (d, J = 8.56 Hz, 1H), 7.99-7.95 (m, 2H), 7.75-7.71 (m, 2H), 7.59 (d, J = 16.4 Hz, 1H), 7.49 (dd, J = 8.6, 5.8 Hz, 1H), 7.26 (d, J = 8.4 Hz, 1H), 7.01-6.96 (m, 1H), 6.75 (dd, J = 9.8, 2.4 Hz, 1H), 4.39 (s, 2H), 3.31 (s, 4H), 2.92-2.72 (m, 1H), 2.09 (s, 4H), 0.81-0.76 (m, 2H), 0.61-0.57 (m, 2H).

$^{13}$C NMR (100 MHz, $CD_3OD$): δ (ppm) 171.64, 157.97, 152.07, 143.68, 143.62, 133.59, 131.23, 129.94, 127.60, 126.54, 123.49, 122.95, 122.24, 117.84, 117.59, 116.94, 114.33, 114.11, 56.34, 54.85, 23.92, 6.54.

$^{19}$F NMR (400MHz, $CD_3OD$): δ (ppm) -111.34.

I-80

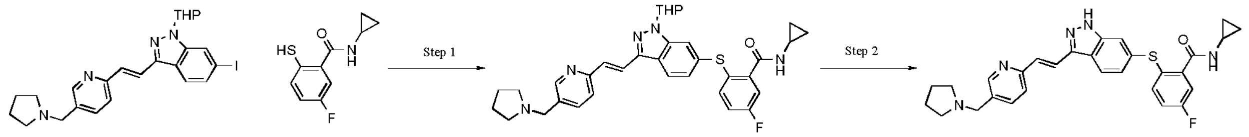


51a 60a 60b I-80

Step 1

At room temperature, 6-iodo-3-[(trans)-2-[5-(pyrrolidin-1-ylmethyl)-2-pyridyl]vinyl]-1-tetrahydropyran-2-ylindazole 51a (1 g, 1.9 mmol) was dissolved in N,N-dimethylformamide (10 mL), followed by the addition of N-cyclopropyl-5-fluoro-2-mercapto-benzamide 60a (493 mg, 2.3 mmol), [1,1'-bis(diphenylphosphino)ferrocene]dichloropalladium (II) (500 mg, 689.0 $\mu$mol) and cesium carbonate (529 mg, 3.9 mmol). The reaction system was stirred at 80°C under the protection of nitrogen to react for 6 h. The reaction mixture was poured into water (50 mL), and then extracted with ethyl acetate (50 mL $\times$ 3), and the organic phases were combined, washed with saturated brine (100 mL), dried over anhydrous sodium sulfate, and concentrated under reduced pressure to obtain a crude product. The crude product was purified by the preparative plate (dichloromethane/methanol = 10/1) to obtain a brown solid product N-cyclopropyl-5-fluoro-2-[3-[(trans)-2-[5-(pyrrolidin-1-ylmethyl)-2-pyridyl]vinyl]-1-tetrahydropyran-2-yl-indazol-6-yl]thioalkylbenzamide 60b (300 mg, 501.9 $\mu$mol, yield: 25.82 %).

The product was verified by LCMS.

For MS-ESI, the calculated value was $[M+H]^+$598.3, and the measurement was 598.1.

Step 2

At room temperature, N-cyclopropyl-5-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 60b (250 mg, 418.2 $\mu$mol) was dissolved in dichloromethane (5 mL), followed by the addition of trifluoroacetic acid (95 mg, 836.5 $\mu$mol). The reaction system was stirred at 25°C to react for 16 h. The reaction mixture was poured into water (20 mL), adjusted with the saturated aqueous solution of sodium carbonate to the pH of 8-9, and then extracted with dichloromethane (20 mL $\times$ 3), and the organic phases were combined, washed with saturated brine (50 mL), dried over anhydrous sodium sulfate, and concentrated under reduced pressure to obtain a crude product. The crude product was purified by the high-pressure preparative chromatography to obtain a yellow solid product N-cyclopropyl-5-fluoro-2-({3-[(E)-2-{5-[(pyrrolidin-1-yl)methyl]pyridin-2-yl}vinyl]-1H-indazol-6-yl}thio)benzamide I-80 (52 mg, 101.2 $\mu$mol, yield: 24.21 %).

The product was verified by LCMS, F-NMR, H-NMR and C-NMR.

For MS-ESI, the calculated value was $[M+H]^+$514.2, and the measurement was 514.3 $^1$H NMR (400MHz, $CD_3OD$): $\delta$ (ppm) 8.51 (d, *J*= 1.6 Hz, 1H), 8.04 (d, *J* = 8.6 Hz, 1H), 7.83 (dd, *J* = 15.0, 9.4 Hz, 2H), 7.67 (d, *J* = 8.0 Hz, 1H), 7.60-7.45 (m, 2H), 7.37 (dd, *J* = 8.8, 5.2 Hz, 1H), 7.24 (dd, *J* = 8.6, 2.8 Hz, 1H), 7.20-7.04 (m, 2H), 3.71 (s, 2H), 2.79-2.74 (m, 1H), 2.60 (s, 4H), 1.84 (s, 4H), 0.73 (dd, *J* = 7.0, 1.8 Hz, 2H), 0.51 (dd, *J* = 3.8, 2.0 Hz, 2H).

$^{13}$C NMR (100MHz, $CD_3OD$): $\delta$ (ppm) 171.19, 164.49, 162.02, 155.98, 151.04, 143.59, 142.20, 142.13, 139.60, 136.72, 136.64, 136.36, 134.24, 130.40, 125.63, 125.28, 122.92, 122.52, 121.47, 118.81, 118.59, 116.39, 116.15, 113.91, 58.16, 54.93. 24.19. 23.78. 6.44.

$^{19}$F NMR (400MHz, $CD_3OD$): $\delta$ (ppm) -115.48.

I-81

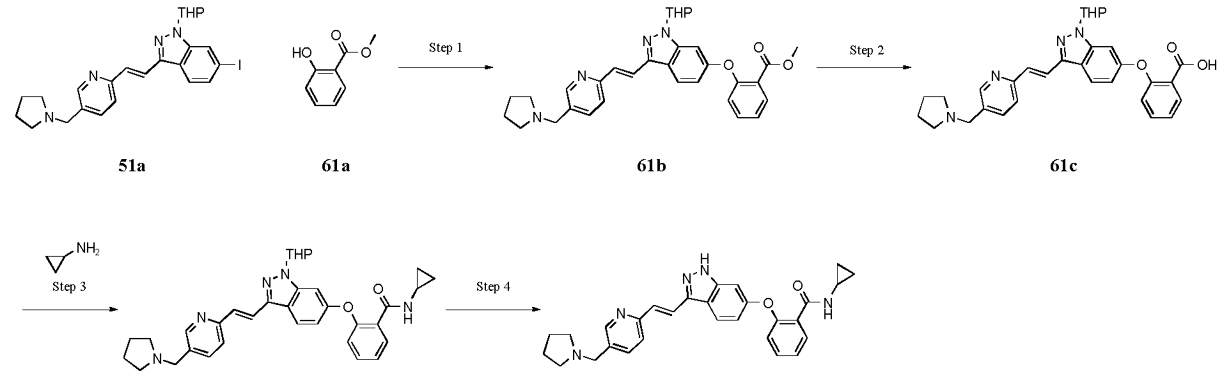


51a 61a 61b 61c

61d I-81

Step 1

At room temperature, 6-iodo-3-[(trans)-2-[5-(pyrrolidin-1-ylmethyl)-2-pyridyl]vinyl]-1-tetrahydropyran-2-ylindazole 51a (200 mg, 388.80 μmol) was dissolved in dimethyl sulfoxide (5 mL), followed by the addition of 2-hydroxymethyl benzoate 61a (88.7 mg, 583.20 μmol), pyridine carboxylic acid (47.9 mg, 388.80 μmol), potassium phosphate (247.6 mg, 1.17 mmol) and cuprous iodide (37.0 mg, 194.40 μmol). The reaction system was stirred at 100°C to react for 16 h under the protection of nitrogen. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the preparative thin-layer chromatography (DCM/MeOH = 10/1) to obtain a yellow solid product 2-[3-[(trans)-2-[5-(pyrrolidin-1-ylmethyl)-2-pyridyl]vinyl]-1-tetrahydropyran-2-ylindazol-6-yl]oxymethyl benzoate 61b (200 mg, 371.31 μmol, yield: 95.50 %).

The product was verified by LCMS.

For MS-ESI, the calculated value was $[M+H]^+$ 539.3, and the measurement was 539.1.

Step 2

2-[3-[(trans)-2-[5-(pyrrolidin-1-ylmethyl)-2-pyridyl]vinyl]-1-tetrahydropyran-2-ylindazol-6-yl]oxymethyl benzoate 61b (200 mg, 371.31 μmol) was dissolved in tetrahydrofuran (5 mL) and water (1 mL); sodium hydroxide (74.3 mg, 1.86 mmol) was added to the resulting mixture, which was then stirred at 60°C for 3 h. Water was added to the reaction mixture (20 mL), and the mixture was adjusted with the dilute hydrochloric acid (3N) to the pH of 6-7, and then extracted with ethyl acetate (20 mL × 3). The organic phases were combined, washed with saturated brine (20 mL), dried over anhydrous sodium sulfate, and concentrated under reduced pressure to obtain a crude product brown solid crude product. The crude product was purified by the preparative thin-layer chromatography (DCM/MeOH = 10/1) to obtain a yellow solid product 2-[3-[(trans)-2-[5-(pyrrolidin-1-ylmethyl)-2-pyridyl]vinyl]-1-tetrahydropyran-2-ylindazol-6-yl]oxybenzoic acid 61c (30 mg, 57.19 μmol, yield: 12.32 %).

The product was verified by LCMS.

For MS-ESI, the calculated value was $[M+H]^+$ 525.2, and the measurement was 525.2.

Step 3

At room temperature, 2-[3-[(trans)-2-[5-(pyrrolidin-1-ylmethyl)-2-pyridyl]vinyl]-1-tetrahydropyran-2-ylindazol-6-yl]oxybenzoic acid 61c (20 mg, 38.12 μmol) was dissolved in N,N-dimethylformamide (2 mL), followed by the addition of cyclopropylamine (2.2 mg, 38.12 μmol), HATU (21.7 mg, 57.19 μmol) and N,N-diisopropylethylamine (14.8 mg, 114.37 μmol). The reaction system was stirred at 25°C to react for 2 h. The reaction mixture was poured into water (10 mL) and extracted with ethyl acetate (10 mL × 3), and the organic phases were combined, washed with saturated brine (20 mL), dried over anhydrous sodium sulfate, and concentrated under reduced pressure to obtain a crude product N-cyclopropyl-2-[3-[(trans)-2-[5-(pyrrolidin-1-ylmethyl)-2-pyridyl]vinyl]-1-tetrahydropyran-2-ylindazol-6-yl]oxybenzamide 61d (20 mg, 35.48 μmol, yield: 93.07 %).

The product was verified by LCMS.

For MS-ESI, the calculated value was $[M+H]^+$ 564.3, and the measurement was 564.2.

Step 4

N-cyclopropyl-2-[3-[(trans)-2-[5-(pyrrolidin-1-ylmethyl)-2-pyridyl]vinyl]-1-tetrahydropyran-2-ylindazol-6-yl]oxybenzamide 61d (20 mg, 35.48 μmol) was dissolved in trifluoroacetic acid (2 mL) and dichloromethane (6 mL). The reaction system was stirred at 25°C to react for 16 h. The reaction mixture was poured into water (10 mL), adjusted with the saturated sodium carbonate solution to the pH of 8 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a white solid product N-cyclopropyl-2-({3-[(E)-2-{5-[(pyrrolidin-1-yl)methyl]pyridin-2-yl}vinyl]-1H-indazol-6-yl}oxy)benzamide I-81 (8.73 mg, 18.20 μmol, yield: 51.31 %).
The product was verified by LCMS, H-NMR and C-NMR.
For MS-ESI, the calculated value was $[M+H]^+$ 479.2, and the measurement was 480.3.
$^1$H NMR (400MHz, $CD_3OD$): δ (ppm) 8.52 (s, 1H), 8.10 (d, J= 9.2 Hz, 1H), 7.88-7.84 (m, 2H), 7.76 (d, J= 7.6 Hz, 1H), 7.69 (d, J= 7.6 Hz, 1H), 7.59-7.49 (m, 2H), 7.30 (t, J= 7.6 Hz, 1H), 7.07 (d, J= 8.4 Hz, 1H), 7.01 (d, J= 8.8 Hz, 1H), 6.93 (s, 1H), 3.71 (s, 2H), 2.73-2.70 (m, 1H), 2.60 (s, 4H), 1.84 (s, 4H), 0.71-0.66 (m, 2H), 0.39-0.35 (m, 2H). $^{13}$C NMR (100 MHz, $CD_3OD$) δ (ppm) 170.02, 158.30, 156.01, 155.08, 151.03, 143.86, 139.56, 134.34, 133.58, 131.42, 130.43, 128.86, 125.61, 123.25, 122.88, 121.57, 118.89, 115.61, 99.01, 58.19, 54.93, 24.21, 23.75, 6.69.

I-82

Step 1

Step 2

Step 3

Step 4

Step 1

At room temperature, 3-[(trans)-2-[5-(pyrrolidin-1-ylmethyl)-2-pyridyl]vinyl]-1-tetrahydropyran-2-ylindazol-6-amine 62a (400 mg, 991.28 μmol) was dissolved in toluene (8 mL), followed by the addition of 2-iodomethyl benzoate 62b (260 mg, 991.28 μmol), tris(dibenzalaceton)dipalladium (91 mg, 99.13 μmol), 4,5-bisdiphenylphosphino-9,9-dimethylxanthene (573 mg, 991.28 μmol) and cesium carbonate (970 mg, 2.97 mmol). The reaction system was stirred at 100°C to react for 6 h under the protection of nitrogen. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a crude product. The crude product was purified by the medium-pressure preparative chromatography to obtain a yellow solid product 2-[[3-[(trans)-2-[5-(pyrrolidin-1-ylmethyl)-2-pyridyl]vinyl]-1-tetrahydropyran-2-ylindazol-6-yl]amino]methyl benzoate 62c (500 mg, 929.97 μmol, yield: 93.82 %).
The product was verified by LCMS and H-NMR.
For MS-ESI, the calculated value was $[M+H]^+$ 538.3, and the measurement was 538.4.
$^1$H NMR (400 MHz, $CDCl_3$): δ (ppm) 9.66 (s, 1H), 8.55 (d, *J*= 1.8 Hz, 1H), 7.99 (dd, *J* = 8.4, 3.6 Hz, 2H), 7.85 (d, *J* = 16.5 Hz, 1H), 7.74-7.66 (m, 1H), 7.57 (d, *J* = 16.6 Hz, 1H), 7.48 (d, *J*= 8.0 Hz, 1H), 7.41 (d, *J* = 1.4 Hz, 1H), 7.36 (d, *J*= 3.4 Hz, 2H), 6.77-6.81 (m, 1H), 5.65 (dd, *J*= 9.4, 2.6 Hz, 1H), 4.06 (d, *J* = 10.4 Hz, 1H), 3.93 (s, 3H), 3.77-3.70 (m, 1H), 3.66 (s, 2H), 2.56 (s, 4H), 2.21-2.05 (m, 2H), 1.82 (s, 4H), 1.68 (s, 5H).

Step 2

At room temperature, 2-[[3-[(trans)-2-[5-(pyrrolidin-1-ylmethyl)-2-pyridyl]vinyl]-1-tetrahydropyran-2-ylindazol-6-yl]amino]methyl benzoate 62c (470 mg, 874.17 μmol) was dissolved in tetrahydrofuran (5 mL) and water (1 mL), followed by the addition of sodium hydroxide (210 mg, 5.25 mmol). The reaction system was stirred at 60 °C to react for 16 h. The reaction mixture was poured into water (20 mL), adjusted with the dilute hydrochloric acid (1N) to the pH

of 6-7 and extracted with ethyl acetate (20 mL × 3), and the organic phases were combined, washed with saturated brine (50 mL), dried over anhydrous sodium sulfate, and concentrated under reduced pressure to obtain a brown solid product 2-[[3-[(trans)-2-[5-(pyrrolidin-1-ylmethyl)-2-pyridyl]vinyl]-1-tetrahydropyran-2-ylindazol-6-yl]amino]benzoic acid 62d (400 mg, 763.91 μmol, yield: 87.39 %).

The product was verified by LCMS.

For MS-ESI, the calculated value was $[M+H]^+$ 524.3, and the measurement was 524.4.

Step 3

At room temperature, 2-[[3-[(trans)-2-[5-(pyrrolidin-1-ylmethyl)-2-pyridyl]vinyl]-1-tetrahydropyran-2-ylindazol-6-yl]amino]benzoic acid 62d (180 mg, 343.76 μmol) was dissolved in N,N-dimethylformamide (6 mL), followed by the addition of cyclopropylamine (20 mg, 343.76 μmol), N,N,N,N-tetramethyl-O-(7-azabenzotriazol-1-yl)urea hexafluorophosphate (196 mg, 515.64 μmol) and N,N-diisopropylethylamine (88.7 mg, 687.52 μmol). The reaction system was stirred at 25 °C to react for 2 h. The reaction mixture was poured into water (30 mL) and extracted with ethyl acetate (30 mL × 3), and the organic phases were combined, washed with saturated brine (20 mL), dried over anhydrous sodium sulfate, and concentrated under reduced pressure to obtain a crude product. The crude product was purified by the preparative plate (dichloromethane/methanol = 10/1) to obtain a brown solid product N-cyclopropyl-2-[[3-[(trans)-2-[5-(pyrrolidin-1-ylmethyl)-2-pyridyl]vinyl]-1-tetrahydropyran-2-yl-indazol-6-yl]amino]benzamide 62e (160 mg, 284.34 μmol, yield: 82.72 %).

The product was verified by LCMS and H-NMR.

For MS-ESI, the calculated value was $[M+H]^+$ 563.3, and the measurement was 563.5.

$^1$H NMR (400MHz, $CDCl_3$): δ (ppm) 9.68 (s, 1H), 8.55 (d, *J* = 1.8 Hz, 1H), 7.96-7.71 (m, 3H), 7.64-7.28 (m, 6H), 7.15 (dd, *J*= 8.8, 1.8 Hz, 1H), 6.80 (s, 1H), 6.30 (s, 1H), 5.62 (d, *J* = 6.8 Hz, 1H), 4.06 (d, *J*= 9.6 Hz, 1H), 3.76 (s, 2H), 2.88 (s, 1H), 2.69 (s, 5H), 1.80-1.55 (m, 10H), 0.89 (d, *J* = 5.6 Hz, 2H), 0.64 (d, *J* = 2.4 Hz, 2H).

Step 4

At room temperature, N-cyclopropyl-2-[[3-[(trans)-2-[5-(pyrrolidin-1-ylmethyl)-2-pyridyl]vinyl]-1-tetrahydropyran-2-yl-indazol-6-yl]amino]benzamide 62e (150 mg, 266.57 μmol) was dissolved in dichloromethane (6 mL), followed by the addition of trifluoroacetic acid (2 mL). The reaction system was stirred at 25°C to react for 2 h. The reaction mixture was poured into water (30 mL), adjusted with the saturated aqueous solution of sodium carbonate to the pH of 8-9, and then extracted with dichloromethane (30 mL × 3), and the organic phases were combined, washed with saturated brine (20 mL), dried over anhydrous sodium sulfate, and concentrated under reduced pressure to obtain a crude product. The crude product was purified by the high-pressure preparative chromatography to obtain a white solid product N-cyclopropyl-2-({3-[(E)-2-{5-[(pyrrolidin-1-yl)methyl]pyridin-2-yl}vinyl]-1H-indazol-6-yl}amino)benzamide I-82 (40 mg, 83.58 μmol, yield: 31.35 %).

The product was verified by LCMS, H-NMR and C-NMR.

For MS-ESI, the calculated value was $[M+H]^+$479.3, and the measurement was 479.1.

$^1$H NMR (400MHz, $CD_3OD$): δ (ppm) 8.68 (d, *J* = 1.6 Hz, 1H), 8.03-7.89 (m, 3H), 7.74 (d, *J* = 8.2 Hz, 1H), 7.61-7.52 (m, 2H), 7.45 (d, *J* = 8.2 Hz, 1H), 7.40-7.33 (m, 1H), 7.21 (d, *J* = 1.6 Hz, 1H), 7.05 (dd, *J* = 8.8, 1.6 Hz, 1H), 6.92 (t, *J* = 7.6 Hz, 1H), 4.41 (s, 2H), 3.33 (dd, *J* = 11.0, 4.2 Hz, 4H), 2.84-2.75 (m, 1H), 2.10 (t, *J* = 6.6 Hz, 4H), 0.80-0.72 (m, 2H), 0.59-0.51 (m, 2H).

$^{13}$C NMR (100MHz, $CD_3OD$): δ (ppm) 173.19, 168.09, 158.28, 152.05, 144.85, 144.51, 143.57, 143.51, 140.42, 133.18, 130.25, 129.44, 127.24, 127.15, 123.32, 122.59, 122.22, 120.84, 118.36, 117.88, 117.83, 98.12, 56.29, 54.85, 23.89, 23.79, 6.59

I-83

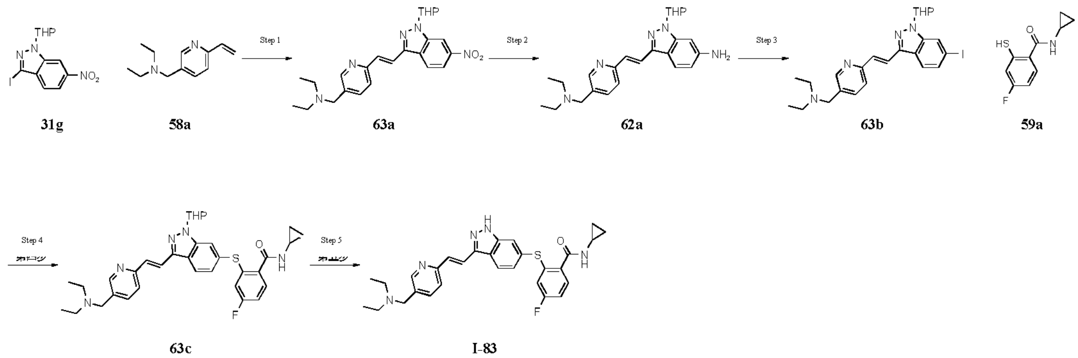


31g 58a 63a 62a 63b 59a

63c I-83

Step 1

At room temperature, 3-iodo-6-nitro-1-tetrahydropyran-2-ylindazole 31 g (5 g, 13.40 mmol) was dissolved in dioxane (100 mL), followed by the addition of N-ethyl-N-[(6-vinyl-3-pyridyl)methyl]ethylamine 58a (2.6 g, 13.40 mmol), tris(dibenzalaceton)dipalladium (5 g, 5.46 mmol), tris(o-methylphenyl)phosphine (5 g, 16.45 mmol) and triethylamine (6.8 g, 67.00 mmol). The reaction system was stirred at 95°C under the protection of nitrogen to react for 2 h. The reaction mixture was poured into water (100 mL) and extracted with ethyl acetate (100 mL × 3), and the organic phases were combined, washed with saturated brine (100 mL), dried over anhydrous sodium sulfate, and concentrated under reduced pressure to obtain a crude product, which was purified by the medium-pressure preparative chromatography to obtain a brown solid product N-ethyl-N-[[6-[(trans)-2-(6-nitro-1-tetrahydropyran-2-yl-indazol-3-yl)vinyl]-3-pyridyl]methyl]ethylamine 63a (2.0 g, 4.59 mmol, yield: 34.27 %).

The product was verified by LCMS and H-NMR.

For MS-ESI, the calculated value was $[M+H]^+$ 436.2, and the measurement was 436.3.

$^1$H NMR (400 MHz,$CDCl_3$): δ (ppm)8.67 (d, *J* = 2.2 Hz, 1H), 8.62-8.55 (m, 2H), 8.15-8.05 (m, 2H), 7.98 (d, *J* = 16.2 Hz, 1H), 7.64-7.51 (m, 2H), 5.84 (dd, *J* = 9.2, 2.6 Hz, 1H), 4.26 (d, *J* = 16.8 Hz, 2H), 4.08 (d, *J* = 11.6 Hz, 1H), 3.90-3.78 (m, 1H), 3.28-3.14 (m, 4H), 2.57 (dt, *J* = 13.2, 6.8 Hz, 1H), 2.19 (dd, *J* = 16.4, 8.6 Hz, 2H), 1.87-1.75 (m, 3H), 1.55 (t, *J* = 7.4 Hz, 6H)

Step 2

At room temperature, N-ethyl-N-[[6-[(trans)-2-(6-nitro-1-tetrahydropyran-2-yl-indazol-3-yl)vinyl]-3-pyridyl] methyl]ethylamine 63a (2 g, 4.59 mmol) was dissolved in methanol (10 mL), tetrahydrofuran (10 mL) and water (10 mL), followed by the addition of sodium sulfide nonahydrate (3.3 g, 13.78 mmol). The reaction system was stirred at 60°C to react for 4 h. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a yellow solid crude product 3-[(trans)-2-[5-(diethylaminomethyl)-2-pyridyl]vinyl]-1-tetrahydropyran-2-ylindazol-6-amine 62a (1.6 g, 3.95 mmol, yield: 85.91 %).

The product was verified by LCMS and H-NMR.

For MS-ESI, the calculated value was $[M+H]^+$ 406.3, and the measurement was 406.3.

$^1$H NMR (400MHz, $CDCl_3$):δ(ppm) 8.52 (s, 1H), 7.91-7.60 (m, 4H), 7.49 (dd, *J* = 17.2, 12.4 Hz, 2H), 6.75 (t, *J* = 6.6 Hz, 1H), 6.73-6.63 (m, 1H), 5.58 (dd, *J* = 9.4, 2.6 Hz, 1H), 3.83-3.63 (m, 2H), 3.60 (s, 2H), 2.59-2.54 (m, 4H), 2.13 (dd, *J* = 27.8, 22.2 Hz, 4H), 1.74 (dd, *J* = 21.2, 10.8 Hz, 3H), 1.07 (t, *J* = 7.2 Hz, 6H).

Step 3

At room temperature, 3-[(trans)-2-[5-(diethylaminomethyl)-2-pyridyl]vinyl]-1-tetrahydropyran-2-ylindazol-6-amine 62a (1.9 g, 4.69 mmol) was dissolved in acetic acid (10 mL) and acetonitrile (10 mL); the mixture was cooled to 0~5°C, followed by the addition of sodium nitrite (485 mg, 7.03 mmol) in water (5 mL). The reaction system was stirred at 0~5°C to react for 1 h. The aqueous solution (5 mL) of sodium iodide (1.4 g, 9.37 mmol) and iodine (595 mg, 2.34 mmol) was added. The reaction system was continuously stirred at 0~5°C to react for 1 h. The reaction mixture was poured into water (50 mL), adjusted with the saturated sodium carbonate solution to the pH of 8-9, extracted with dichloromethane (50 mL × 3), dried over anhydrous sodium sulfate, and concentrated under reduced pressure to obtain a crude product. The crude product was purified by the medium-pressure preparative chromatography to obtain a brown solid product N-ethyl-N-[[6-[(trans)-2-(6-iodo-1-tetrahydropyran-2-yl-indazol-3-yl)vinyl]-3-pyridyl]methyl]ethylamine 63b (1 g, 1.94 mmol, yield:

41.33 %).

The product was verified by LCMS and H-NMR.

For MS-ESI, the calculated value was $[M+H]^+$ 517.1, and the measurement was 517.2.

$^1$H NMR (400MHz, $CDCl_3$): δ (ppm) 8.56 (d, *J* = 16.0 Hz, 1H), 8.05-7.93 (m, 1H), 7.88-7.64 (m, 3H), 7.57-7.44(m, 3H), 5.68 (dd, *J* = 9.4, 2.6 Hz, 1H), 4.10-3.73 (m, 3H), 3.62 (d, *J* = 7.2 Hz, 2H), 2.60-2.53 (m, 4H), 2.27-2.00 (m, 3H), 1.68 (s, 2H), 1.08 (dd, *J* = 8.8, 5.4 Hz, 6H).

Step 4

At room temperature, N-ethyl-N-[[6-[(trans)-2-(6-iodo-1-tetrahydropyran-2-yl-indazol-3-yl)vinyl]-3-pyridyl] methyl]ethylamine 63b (200 mg, 387.28 μmol) was dissolved in N,N-dimethylformamide (6 mL), followed by the addition of N-cyclopropyl-4-fluoro-2-mercapto-benzamide 59a (82 mg, 387.28 μmol), DPPF dichloropalladium (II) (57 mg, 77.46 μmol) and cesium carbonate (253 mg, 774.57 μmol). The reaction system was stirred at 80°C under the protection of nitrogen to react for 6 h. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a brown solid crude product N-cyclopropyl-2-[3-[(trans)-2-[5-(diethylaminomethyl)-2-pyridyl]vinyl]-1-tetrahydropyran-2-ylindazol-6-yl]thioalkyl-4-fluoro-benzamide 63c (120 mg, 200.08 μmol, yield: 51.66 %).

The product was verified by LCMS.

For MS-ESI, the calculated value was $[M+H]^+$600.3, and the measurement was 600.1.

Step 5

At room temperature, N-cyclopropyl-2-[3-[(trans)-2-[5-(diethylaminomethyl)-2-pyridyl]vinyl]-1-tetrahydropyran-2-ylindazol-6-yl]thioalkyl-4-fluoro-benzamide 63c (120 mg, 200.08 μmol) was dissolved in dichloromethane (9 mL), followed by the addition of trifluoroacetic acid (3 mL); the reaction liquid was stirred at 25°C to react for 2 h. The reaction mixture was poured into water (30 mL), adjusted with the saturated sodium carbonate solution to the pH of 8-9, and then extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography to obtain a yellow solid product N-cyclopropyl-2-({3-[(E)-2-{5-[(diethylamino)methyl]pyridin-2-yl}vinyl]-1H-indazol-6-yl}thio)-4-fluorobenzamide I-83 (12 mg, 23.27 μmol, yield: 11.63 %).

The product was verified by LCMS, F-NMR, H-NMR and C-NMR.

For MS-ESI, the calculated value was $[M+H]^+$516.2, and the measurement was 516.3.

$^1$H NMR (400MHz, $CD_3OD$): δ (ppm) 8.68 (s, 1H), 8.43 (s, 1H), 8.15 (d, *J*= 8.6 Hz, 1H), 7.99 (t, *J* = 10.2 Hz, 2H), 7.80-7.69 (m, 2H), 7.61 *(d, J*= 16.4 Hz, 1H), 7.50 (dd, *J* = 8.6, 5.8 Hz, 1H), 7.27 (dd, *J* = 8.6, 1.2 Hz, 1H), 6.99 (d, *J*= 2.4 Hz, 1H), 6.75 (dd, *J*= 9.8, 2.4 Hz, 1H), 4.35 (s, 2H), 3.18 (q, *J*= 7.2 Hz, 4H), 2.81 (s, 1H), 1.35 (t, *J*= 7.2 Hz, 6H), 0.83-0.73 (m, 2H), 0.59 (dd, *J* = 3.8, 2.2 Hz, 2H).

$^{13}$C NMR (100 MHz, $CD_3OD$) δ (ppm) 171.66, 166.17, 163.68, 157.97, 152.50, 143.62, 140.92, 133.64, 129.93, 126.60, 123.48, 122.23, 117.84, 117.60, 116.93, 114.34, 114.12, 54.38, 23.89, 9.31, 6.53

$^{19}$F NMR (400 MHz, $CD_3OD$) δ (ppm) -111.39

I-84

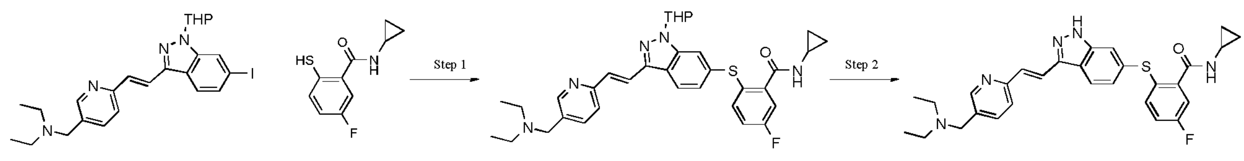


63b 60a 64a I-84

Step 1

At room temperature, N-ethyl-N-[[6-[(trans)-2-(6-iodo-1-tetrahydropyran-2-yl-indazol-3-yl)vinyl]-3-pyridyl] methyl]ethylamine 63b (200 mg, 387.28 μmol) was dissolved in N,N-dimethylformamide (8 mL), followed by the addition of N-cyclopropyl-5-fluoro-2-mercapto-benzamide 60a (82 mg, 387.28 μmol), DPPF dichloropalladium (II) (100 mg, 136.80 μmol) and cesium carbonate (126 mg, 387.28 μmol). The reaction system was stirred at 80°C under the protection of nitrogen to react for 6 h. The reaction mixture was poured into water (30 mL) and extracted with ethyl acetate (30 mL × 3),

and the organic phases were combined, washed with saturated brine (30 mL), dried over anhydrous sodium sulfate, and concentrated under reduced pressure to obtain a brown solid crude product N-cyclopropyl-2-[3-[(trans)-2-[5-(diethylaminomethyl)-2-pyridyl]vinyl]-1-tetrahydropyran-2-ylindazol-6-yl]thioalkyl-5-fluoro-benzamide 64a (100 mg, 166.73 μmol, yield: 43.05 %).

The product was verified by LCMS.

For MS-ESI, the calculated value was $[M+H]^+$ 600.3, and the measurement was 600.3.

Step 2

At room temperature, N-cyclopropyl-2-[3-[(trans)-2-[5-(diethylaminomethyl)-2-pyridyl]vinyl]-1-tetrahydropyran-2-ylindazol-6-yl]thioalkyl-5-fluoro-benzamide 64a (100 mg, 166.73 μmol) was dissolved in dichloromethane (9 mL) and trifluoroacetic acid (3 mL); the reaction system was stirred at 25°C to react for 2 h. The reaction mixture was poured into water (30 mL), adjusted with the saturated sodium carbonate solution to the pH of 8-9 and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a brown solid product N-cyclopropyl-2-({3-[(E)-2-{5-[(diethylamino)methyl] pyridin-2-yl}vinyl]-1H-indazol-6-yl}thio)-5-fluorobenzamide I-84 (18 mg, 34.91 μmol, yield: 20.94 %).

The product was verified by LCMS, F-NMR, H-NMR and C-NMR.

For MS-ESI, the calculated value was $[M+H]^+$ 516.2, and the measurement was 516.0.

$^1$H NMR (400MHz, $CD_3OD$): δ (ppm) 8.63 (s, 1H), 8.51 (s, 1H), 8.04 (d, *J* = 8.6 Hz, 1H), 7.97-7.90 (m, 2H), 7.72 *(d, J*= 8.0 Hz, 1H), 7.60-7.46 (m, 2H), 7.38 (dd, *J* = 8.8, 5.2 Hz, 1H), 7.25 (dd, *J* = 8.6, 2.8 Hz, 1H), 7.18-7.14 (m, 2H), 4.18 (s, 2H), 3.04 (q, *J* = 7.2 Hz, 4H), 2.85-2.70 (m, 1H), 1.29 (t, *J* = 7.2 Hz, 6H), 0.74-0.71 (m, 2H), 0.58-0.47 (m, 2H).

$^{13}$C NMR (100 MHz, $CD_3OD$) δ (ppm) 171.18, 162.04, 157.48, 152.17, 142.18, 140.62, 136.78, 136.70, 136.48, 129.94, 126.35, 125.69, 123.29, 122.47, 121.48, 118.84, 118.62, 116.42, 116.18, 113.86, 54.60, 23.79, 9.84, 6.45 $^{19}$F NMR (400 MHz, $CD_3OD$) δ (ppm) -115.35.

I-85

Step 1

Step 2

Step 1

At room temperature, N-cyclopropyl-3-fluoro-2-(3-iodo-1-tetrahydropyran-2-yl-indazol-6-yl)thioalkyl benzamide 42a (500 mg, 930.43 μmol) was dissolved in dioxane (10 mL), followed by the addition of N-ethyl-N-[(6-vinyl-3-pyridyl)methyl]ethylamine 58a (177 mg, 930.43 μmol), palladium chloride (83 mg, 465.21 μmol), tris(o-methylphenyl) phosphine (283 mg, 930.43 μmol) and triethylamine (283 mg, 2.79 mmol). The reaction system was stirred at 90°C under the protection of nitrogen to react for 16 h. The reaction mixture was poured into water (50 mL) and extracted with ethyl acetate (50 mL × 3), and the organic phases were combined, washed with saturated brine (20 mL), dried over anhydrous sodium sulfate, and concentrated under reduced pressure to obtain a crude product. The crude product was purified by the medium-pressure preparative chromatography to obtain a yellow solid product N-cyclopropyl-2-[3-[(trans)-2-[5-(diethylaminomethyl)-2-pyridyl]vinyl]-1-tetrahydropyran-2-yl-indazol-6-yl]thioalkyl-3-fluoro-benzamide 65a (500 mg, 833.67 μmol, yield: 89.60 %).

The product was verified by LCMS.

For MS-ESI, the calculated value was $[M+H]^+$ 600.3, and the measurement was 600.2.

Step 2

At room temperature, N-cyclopropyl-2-[3-[(trans)-2-[5-(diethylaminomethyl)-2-pyridyl]vinyl]-1-tetrahydropyran-2-yl-indazol-6-yl]thioalkyl-3-fluoro-benzamide 65a (450 mg, 750.30 μmol) was dissolved in dichloromethane (16 mL) and trifluoroacetic acid (4 mL); the reaction system was stirred at 25°C to react for 16 h. The reaction mixture was poured into water (30 mL), adjusted with saturated sodium carbonate to the pH of 8 and extracted with dichloromethane (30 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a white solid product N-cyclopropyl-2-({3-[(E)-2-{5-[(diethylamino)methyl] pyridin-2-yl}vinyl]-1H-indazol-6-yl}thio)-3-fluorobenzamide I-85 (53.59 mg, 103.93 μmol, yield: 13.85 %).

The product was verified by LCMS, F-NMR, H-NMR and C-NMR.

For MS-ESI, the calculated value was $[M+H]^+$ 516.2, and the measurement was 516.3.

$^1H$ NMR (400MHz, $CD_3OD$): δ (ppm) 8.44 (s, 1H), 7.95 (d, J = 8.8 Hz, 1H), 7.81-7.72 (m, 2H), 7.59 (d, J = 8.0 Hz, 1H), 7.52-7.47 (m, 2H), 7.38 (s, 1H), 7.29-7.24 (m, 2H), 7.10 (d, J = 8.8 Hz, 1H), 3.58 (s, 2H), 2.79-2.75 (m, 1H), 2.52 (q, J = 7.2 Hz, 4H), 1.05 (t, J = 7.2 Hz, 6H), 0.74-0.70 (m, 2H), 0.53-0.49 (m, 2H).

$^{19}F$ NMR (400MHz, $CD_3OD$): δ (ppm) -105.58.

$^{13}C$ NMR (100 MHz, $CD_3OD$) δ (ppm) 171.58, 171.55, 165.42, 162.95, 155.68, 151.04, 145.34, 143.77, 143.47, 139.56, 136.56, 134.79, 132.66, 132.58, 130.41, 125.19, 124.83, 124.79, 123.90, 122.77, 122.15, 121.11, 120.00, 119.80, 118.55, 118.31, 111.62, 55.37, 23.81, 11.70, 6.51.

I-86

Step 1 Step 2

Step 1

Under the protection of nitrogen at room temperature, N-ethyl-N-[(6-vinyl-3-pyridyl)methyl]ethylamine 58a (145.4 mg, 764.25 μmol), $PdCl_2$ (67.6 mg, 382.13 μmol), $P(o\text{-}tol)_3$ (232.3 mg, 764.25 μmol) and triethylamine (386.7 mg, 3.82 mmol) were added to the solution of tert-butyl 6-[2-(ethylcarbamoyl)phenyl]sulfonyl-3-iodoindazol-1-carboxylate 53c (400 mg, 764.25 μmol) in 1,4-dioxane (5 mL); the solution was heated to 95 °C and stirred to react for about 2 h. After the thin-layer chromatography (dichloromethane/methanol = 10/1) showed the complete reaction of the starting materials, the mixture was poured into water (10 mL) and extracted with ethyl acetate (10 mL × 3), and the organic layers were combined, washed with saturated brine (20 mL), dried over anhydrous sodium sulfate and concentrated to obtain a brownish-yellow solid crude product 3-[(E)-2-[5-(diethylaminomethyl)-2-pyridyl]vinyl]-6-[2-(ethylcarbamoyl)phenyl]sulfanilamido-indazol-1-carboxylate 66a (200 mg, 341.44 μmol, yield: 44.68 %).

This product was verified by LCMS.

Step 2

At room temperature, trifluoroacetic acid (3 mL) was added to the solution of 3-[(E)-2-[5-(diethylaminomethyl)-2-pyridyl]vinyl]-6-[2-(ethylcarbamoyl)phenyl]sulfanilamido-indazol-1-carboxylate 66a (150 mg, 256.08 μmol) in dichloromethane (9 mL), and then stirred to react for about 2 h. After LC-MS showed the complete reaction of the starting materials, the mixture was poured into water (30 mL), adjusted with the sodium carbonate solution to the pH of 8-9 and extracted with dichloromethane (20 mL × 3), and the organic layers were combined, washed with saturated brine (30 mL), dried over anhydrous sodium sulfate and concentrated to obtain a crude product. The crude product was purified by preparative thin-layer chromatography to obtain a yellow solid, 2-({3-[(E)-2-{5-[(diethylamino)methyl]pyridin-2-yl}vinyl]-1H-indazol-6-yl}thio)-N-ethylbenzamide I-86 (15 mg, 30.89 μmol, yield: 12.06 %).

This product was verified by LCMS, HNMR and CNMR.

$^1H$ NMR (400MHz, $CD_3OD$): δ (ppm) 8.50 (d, J = 1.8 Hz, 1H), 8.06 (d, J = 8.4 Hz, 1H), 7.90 -7.77 (m, 2H), 7.67 (d, J = 8.2 Hz, 1H), 7.62-7.46 (m, 3H), 7.39-7.30 (m, 2H), 7.28-7.17 (m, 2H), 3.65 (s, 2H), 3.40-3.34 (m, 2H), 2.61-2.55 (m, 4H), 1.18 (t, J = 7.4 Hz, 3H), 1.10 (t, J = 7.2 Hz, 6H).

$^{13}C$ NMR (100 MHz, $CD_3OD$) δ (ppm) 171.03, 155.69, 151.06, 143.82, 143.63, 139.64, 139.48, 135.95, 135.72, 134.93, 133.11, 130.48, 128.18, 126.54, 125.11, 122.83, 122.52, 121.61, 115.03, 55.37, 35.80, 14.70, 11.67.

I-87

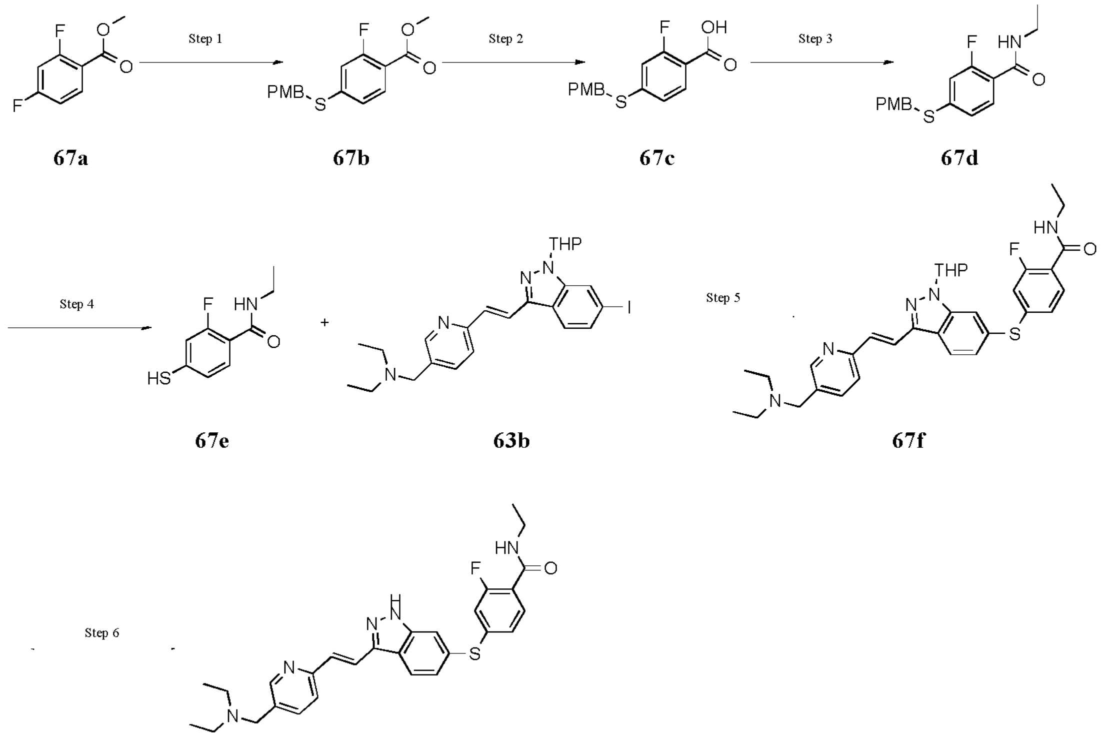


67a 67b 67c 67d

67e 63b 67f

I-87

Step 1

At room temperature, 2,4-difluoromethyl benzoate 67a (10.0 g, 58.10 mmol) was dissolved in N,N-dimethylformamide (100 mL), followed by the addition of (4-methoxyphenyl)methanethiol (9.0 g, 58.10 mmol) and cesium carbonate (37.9 g, 116.19 mmol). The reaction system was stirred at 25°C to react for 2 h. The reaction mixture was poured into water (200 mL) and extracted with ethyl acetate (200 mL × 3), and the organic phases were combined, washed with saturated brine (200 mL), dried over anhydrous sodium sulfate, and concentrated under reduced pressure to obtain a yellow solid crude product 2-fluoro-4-[(4-methoxyphenyl)methylthioalkyl]methyl benzoate 67b (15.0 g, 48.96 mmol, yield: 84.28 %).

The product was verified by LCMS and H-NMR.

For MS-ESI, the calculated value was $[M+H]^+$307.1, and the measurement was 307.0.

$^1$H NMR (400 MHz,$CDCl_3$): δ (ppm)7.80 (t, J = 8.0 Hz, 1H), 7.28 (s, 1H), 7.03 (dd, J = 18.8, 8.4 Hz, 2H), 6.84 (d, J = 8.4 Hz, 3H), 4.14 (s, 2H), 3.89 (s, 3H), 3.78 (s, 3H).

Step 2

At room temperature, 2-fluoro-4-[(4-methoxyphenyl)methylthioalkyl]methyl benzoate 67b (18.0 g, 58.76 mmol) was dissolved in tetrahydrofuran (200 mL) and water (50 mL), followed by the addition of lithium hydroxide (4.2 g, 176.28 mmol). The reaction system was stirred at 50°C to react for 5 h. The reaction mixture was concentrated under reduced pressure to obtain a crude product, to which water (300 mL) was added and then the dilute hydrochloric acid solution (2N) was slowly dropwise added to adjust to the pH of3, and the mixture was filtered to collect the filter cake, obtaining a white solid product 2-fluoro-4-[(4-methoxyphenyl)methylthioalkyl]benzoic acid 67c (15.0 g, 51.31 mmol, yield: 87.33 %).

The product was verified by LCMS and H-NMR.

For MS-ESI, the calculated value was $[M+H]^+$ 293.1, and the measurement was 293.0.

$^1$H NMR (400MHz, $CDCl_3$): δ (ppm)7.88 (t, J = 8.0 Hz, 1H), 7.29 (d, J = 8.4 Hz, 1H), 7.17 (d, J = 8.0 Hz, 1H), 7.07 (dd, J = 20.0, 8.4 Hz, 2H), 6.87-6.84 (m, 3H), 4.18 (s, 2H), 3.80 (s, 3H).

Step 3

At room temperature, 2-fluoro-4-[(4-methoxyphenyl)methylthioalkyl]benzoic acid 67c (10.0 g, 34.21 mmol) was

dissolved in N,N-dimethylformamide (100 mL), followed by the addition of EDCI (13.1 g, 68.42 mmol), HOBT (9.2 g, 68.42 mmol), N,N-diisopropylethylamine (22.1 g, 171.04 mmol) and ethylamine hydrochloride (4.2 g, 51.31 mmol). The reaction system was stirred at 25°C to react for 3 h. The reaction mixture was poured into water (200 mL) and extracted with ethyl acetate (200 mL × 3), and the organic phases were combined, washed with saturated brine (300 mL), dried over anhydrous sodium sulfate, and concentrated under reduced pressure to obtain a crude product. The crude product was purified by medium-pressure preparative chromatography to obtain a yellow solid product, N-ethyl-2-fluoro-4-[(4-methoxyphenyl)methylthioalkyl ]benzamide 67d (7 g, 21.92 mmol, yield: 64.07 %). The product was verified by LCMS and H-NMR.

For MS-ESI, the calculated value was $[M+H]^+$320.1, and the measurement was 320.1.

$^1$H NMR (400MHz, $CDCl_3$): δ (ppm) 7.99 (t, J = 8.4 Hz, 1H), 7.28 (s, 1H), 7.12 (d, J = 8.4 Hz, 1H), 6.97 (d, J = 12.8 Hz, 1H), 6.91-6.77 (m, 3H), 6.63 (s, 1H), 4.15 (s, 2H), 3.79 (s, 3H), 3.53-3.46 (m, 2H), 1.24 (t, J = 7.2 Hz, 3H).

Step 4

At room temperature, N-ethyl-2-fluoro-4-[(4-methoxyphenyl)methylthioalkyl]benzamide 67d (7.0 g, 21.92 mmol) was dissolved in trifluoroacetic acid (40 mL). The reaction system was stirred at 70°C to react for 6 h. The reaction mixture was concentrated under reduced pressure to obtain a crude product. The crude product was purified by the medium-pressure preparative chromatography to obtain a yellow solid product N-ethyl-2-fluoro-4-aminobenzamide 67e (3 g, 15.06 mmol, yield: 68.70 %).

The product was verified by LCMS and H-NMR.

For MS-ESI, the calculated value was $[M+H]^+$200.1, and the measurement was 200.0.

$^1$H NMR (400MHz, $CDCl_3$): δ (ppm) 7.97 (t, J = 8.4 Hz, 1H), 7.10 (d, J = 8.4 Hz, 1H), 7.00 (d, J = 12.4 Hz, 1H), 6.62 (s, 1H), 3.64 (s, 1H), 3.54-3.47 (m, 2H), 1.25 (t, J = 7.2 Hz, 3H).

Step 5

At room temperature, N-ethyl-N-[[6-[(trans)-2-(6-iodo-1-tetrahydropyran-2-yl-indazol-3-yl)vinyl]-3-pyridyl] methyl]ethylamine 63b (130 mg, 251.73 μmol) was dissolved in N,N-dimethylformamide (6 mL), followed by the addition of N-ethyl-2-fluoro-4-aminobenzamide 67e (50 mg, 251.73 μmol), $Pd(dppf)Cl_2$ (65 mg, 89.62 μmol), and cesium carbonate (164 mg, 503.47 μmol). The reaction system was stirred at 80°C under the protection of nitrogen to react for 6 h. The reaction mixture was poured into water (50 mL) and extracted with ethyl acetate (50 mL × 3), and the organic phases were combined, washed with saturated brine (50 mL), dried over anhydrous sodium sulfate, and concentrated under reduced pressure to obtain a crude product. The crude product was purified by the preparative thin-layer chromatography (DCM/MeOH = 10/1) to obtain a brown solid product 4-[3-[(trans)-2-[5-(diethylaminomethyl)-2-pyridyl]vinyl]-1-tetrahydropyran-2-yl-indazol-6-yl]thioalkyl-N-ethyl-2-fluorobenzamide 67f (110 mg, 187.15 μmol, yield: 74.35 %).

The product was verified by LCMS.

For MS-ESI, the calculated value was $[M+H]^+$588.3, and the measurement was 588.2

Step 6

At room temperature, 4-[3-[(trans)-2-[5-(diethylaminomethyl)-2-pyridyl]vinyl]-1-tetrahydropyran-2-yl-indazol-6-yl]thioalkyl-N-ethyl-2-fluorobenzamide 67f (90 mg, 153.13 μmol) was dissolved in dichloromethane (9 mL), followed by the addition of trifluoroacetic acid (3 mL); the reaction liquid was stirred at 25°C to react for 2 h. The reaction mixture was poured into water (30 mL), adjusted with sodium carbonate to the pH of 9, and then extracted with dichloromethane (50 mL × 3), and the organic phases were combined, washed with saturated brine (30 mL), dried over anhydrous sodium sulfate, and concentrated under reduced pressure to obtain a crude product, which was purified by the high-pressure preparative chromatography to obtain a yellow solid product 4-({3-[(E)-2-{5-[(diethylamino)methyl]pyridin-2-yl}vinyl]-1H-indazol-6-yl}thio)-N-ethyl-2-fluorobenzamide I-87 (26.52 mg, 52.66 μmol, yield: 34.39 %).

The product was verified by LCMS, F-NMR, H-NMR and C-NMR.

For MS-ESI, the calculated value was $[M+H]^+$504.2, and the measurement was 504.3

$^1$H NMR (400MHz, $CDCl_3$): δ (ppm) 8.58 (s, 1H), 8.02 (d, J = 8.4 Hz, 1H), 7.95-7.88 (m, 2H), 7.71 (d, J = 8.0 Hz, 1H), 7.65-7.60 (m, 2H), 7.44 (d, J = 8.0 Hz, 1H), 7.23 (d, J = 8.4 Hz, 1H), 6.96 (d, J = 8.4 Hz, 1H), 6.80 (d, J = 12.4 Hz, 1H), 6.70-6.67 (m, 1H), 3.62 (s, 2H), 3.52-3.45 (m, 2H), 2.57 (q, J = 7.2 Hz, 4H), 1.22 (t, J = 7.2 Hz, 3H), 1.07 (t, J = 7.2 Hz, 6H).

$^{13}$C NMR (100 MHz, $CDCl_3$) δ (ppm) 162.90, 162.87, 161.84, 159.36, 154.20, 150.04, 144.96, 144.87, 143.39, 142.12, 137.44, 133.99, 132.21, 130.26, 129.94, 126.50, 122.05, 121.73, 121.65, 118.47, 118.35, 116.08, 114.79, 114.51, 54.64, 46.72, 34.98, 14.73, 11.66

$^{19}$F (400MHz, $CDCl_3$): δ (ppm) -112.83

I-88

Step 1 Step 2

Step 1

At room temperature, N-ethyl-N-[[6-[(trans)-2-(6-iodo-1-tetrahydropyran-2-ylindazol-3-yl)vinyl]-3-pyridyl] methyl]ethylamine 63b (500.0 mg, 968.21 μmol) was dissolved in N,N-dimethylformamide (10 mL), followed by the addition of N-ethyl-5-fluoro-2-thiamino-benzamide 68a (250.8 mg, 1.26 mmol), cesium carbonate (630.9 mg, 1.94 mmol) and Pd(pddf)$Cl_2$ (250.0 mg, 344.52 μmol). The reaction system was stirred at 80°C under the protection of nitrogen to react for 6 h. The reaction mixture was poured into an aqueous solution (30 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was purified by the thin-layer chromatography (DCM/MeOH = 10/1) to obtain a brown solid product 2-[3-[(trans)-2-[5-(diethylatninomethyl)-2-pyridyl] vinyl]-1-tetrahydropyran-2-ylindazol-6-yl]thioalkyl-N-ethyl-5-fluorobenzamide 68b (300.0 mg, 510.42 μmol, yield: 52.72 %).

The product was verified by LCMS.

For MS-ESI, the calculated value was $[M+H]^+$ 588.3, and the measurement was 588.2.

Step 2

At room temperature, 2-[3-[(trans)-2-[5-(diethylaminomethyl)-2-pyridyl]vinyl]-1-tetrahydropyran-2-ylindazol-6-yl]thioalkyl-N-ethyl-5-fluorobenzamide 68b (300.0 mg, 510.42 μmol) was dissolved in dichloromethane (10 mL) and trifluoroacetic acid (5 mL). The reaction system was stirred at 25°C to react for 2 h. The reaction mixture was poured into water (20 mL), adjusted with the saturated aqueous solution of sodium carbonate to the pH of 8-9 and extracted with dichloromethane (20 mL × 3), and the organic phases were combined, washed with saturated brine (30 mL), dried over anhydrous sodium sulfate, and concentrated under reduced pressure to obtain a crude product, which was purified by the high-pressure liquid chromatography (mobile phase: trifluoroacetic acid/water/acetonitrile) to obtain a final product, which was freed with the saturated aqueous solution of sodium carbonate to obtain a yellow solid product 2-({3-[(E)-2-{5-[(diethylamino)methyl]pyridin-2-yl}vinyl]-1H-indazol-6-yl}thio)-N-ethyl-5-fluorobenzamide I-88 (57.98 mg, 115.12 μmol, yield: 57.98 %).

The product was verified by LCMS, F-NMR, H-NMR and C-NMR.

For MS-ESI, the calculated value was $[M+H]^+$ 504.2, and the measurement was 504.1.

1H NMR (400MHz, CD3OD): δ (ppm) 8.50 (s, 1H), 8.04 (d, J = 8.4 Hz, 1H), 7.83 (t, J = 11.7 Hz, 2H), 7.66 (d, J = 8.2 Hz, 1H), 7.59 - 7.45 (m, 2H), 7.37 (dd, J = 8.4, 5.2 Hz, 1H), 7.29 - 7.09 (m, 3H), 3.66 (s, 2H), 3.34 (d, J = 7.2 Hz, 2H), 2.58 (q, J = 7.2 Hz, 4H), 1.15 - 1.08 (m, 9H).

$^{13}C$ NMR (100 MHz, $CD_3OD$) δ (ppm) 169.55, 164.55, 162.08, 158.16, 152.27, 143.60, 143.49, 142.58, 141.41, 136.76, 136.68, 136.62, 130.30, 129.17, 127.41, 125.68, 123.59, 121.54, 118.75, 118.53, 116.36, 116.12, 113.95, 54.81, 54.30, 35.82, 14.57, 9.02.

$^{19}F$ NMR (400 MHz, $CD_3OD$): δ (ppm) -115.67

I-90

Step 1 Step 2

Step 1

At room temperature, 3-iodo-6-[2-(methylcarbamoyl)phenyl]thioalkyl-indazol-1-tert-butyl carboxylate 69a (500 mg, 981.63 μmol) was dissolved in dioxane (10 mL), followed by the addition of N-ethyl-N-[(6-vinyl-3-pyridyl)methyl] ethylamine 68a (187 mg, 981.63 μmol), palladium chloride (87 mg, 490.81 μmol), tris(o-methylphenyl)phosphine (298 mg, 981.63 μmol) and triethylamine (497 mg, 4.91 mmol). The reaction system was stirred at 95°C under the protection of

nitrogen to react for 2 h. The reaction mixture was poured into water (20 mL) and extracted with ethyl acetate (20 mL × 3), and the organic phases were combined, washed with saturated brine (20 mL), dried over anhydrous sodium sulfate, and concentrated under reduced pressure to obtain a brown solid crude product 6-[2-(cyclopropylaminoformyl)phenyl] thioalkyl-3-[(trans)-2-[5-[2-(1-methyl-4-piperidyl)ethoxy]-2-pyridyl]vinyl]indazol-1-tert-butyl formate 69b (200 mg, 305.89 μmol, yield: 32.75 %).

The product was verified by LCMS.

For MS-ESI, the calculated value was $[M+H]^+$ 572.3, and the measurement was 572.2.

Step 2

At room temperature, 6-[2-(cyclopropylcarbamoyl)phenyl]thioalkyl-3-[(trans)-2-[5-[2-(1-methyl-4-piperidyl) ethoxy]-2-pyridyl]vinyl]indazol-1-tert-butyl formate 69b (150 mg, 262.36 μmol) was dissolved in dichloromethane (9 mL) and trifluoroacetic acid (3 mL); the reaction system was stirred at 25°C to react for 2 h. The reaction mixture was poured into water (30 mL), adjusted with the saturated sodium carbonate solution to the pH of 8 and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product 2-({3-[(E)-2-{5-[(diethylamino)methyl] pyridin-2-yl}vinyl]-1H-indazol-6-yl}thio)-N-methylbenzamide I-90 (69 mg, 124.61 μmol, yield: 10.18 %).

The product was verified by LCMS, H-NMR and C-NMR.

For MS-ESI, the calculated value was $[M+H]^+$ 472.2, and the measurement was 472.0.

$^1$H NMR (400MHz, $CD_3OD$): δ (ppm) 8.52 (d, *J* = 1.6 Hz, 1H), 8.09 (d, *J* = 8.6 Hz, 1H), 7.85 (dd, *J* = 18.0, 9.4 Hz, 2H), 7.70 (d, *J*= 8.2 Hz, 1H), 7.63-7.55 (m, 2H), 7.52-7.46 (m, 1H), 7.38-7.30 (m, 2H), 7.27-7.21 (m, 2H), 3.69 (s, 2H), 2.88 (s, 3H), 2.60 (d, *J*= 7.2 Hz, 4H), 1.12 (t, *J* = 7.2 Hz, 6H).

$^{13}$C NMR (100 MHz, $CD_3OD$) δ (ppm) 171.78, 155.74, 151.10, 139.69, 136.37, 135.57, 134.84, 132.87, 131.69, 130.48, 129.07, 128.04, 126.75, 125.18, 122.86, 122.54, 121.67, 55.35, 47.69, 26.74, 11.62.

I-91

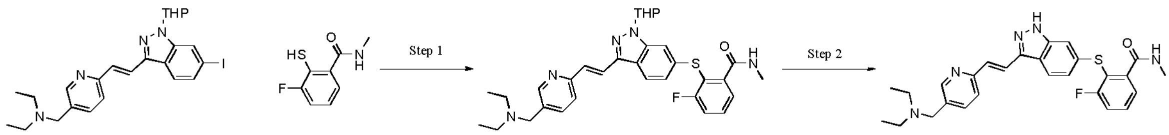


63b 31e 70a I-91

Step 1

At room temperature, N-ethyl-N-[[6-[(trans)-2-(6-iodo-1-tetrahydropyran-2-yl-indazol-3-yl)vinyl]-3-pyridyl] methyl]ethylamine 63b (300 mg, 580.93 μmol) was dissolved in N,N-dimethylformamide (8 mL), followed by the addition of 3-fluoro-N-methyl-2-mercaptobenzamide 31e (108 mg, 580.93 μmol), [1,1'-bis(diphenylphosphino)ferrocene]dichloropalladium (II) (150 mg, 205.20 μmol) and cesium carbonate (379 mg, 1.16 mmol). The reaction system was stirred at 80°C to react for 6 h under the protection of nitrogen. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a brown solid product 2-[3-[(trans)-2-[5-(diethylaminomethyl)-2-pyridyl]vinyl]-1-tetrahydropyran-2-yl-indazol-6-yl]thioalkyl-3-fluoro-N-methyl-benzamide 70a (150 mg, 261.45 μmol, yield: 45.01 %).

The product was verified by LCMS.

For MS-ESI, the calculated value was $[M+H]^+$ 574.3, and the measurement was 574.3.

Step 2

At room temperature, 2-[3-[(trans)-2-[5-(diethylaminomethyl)-2-pyridyl]vinyl]-1-tetrahydropyran-2-yl-indazol-6-yl]thioalkyl-3-fluoro-N-methyl-benzamide 70a (150 mg, 261.45 μmol) was dissolved in dichloromethane (9 mL), followed by the addition of trifluoroacetic acid (3 mL). The reaction system was stirred at 25°C to react for 2 h. The reaction mixture was poured into water (30 mL), adjusted with the saturated aqueous solution of sodium carbonate to the pH of 8-9, extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a brown solid product 2-({3-[(E)-2-{5-[(diethylamino)methyl]pyridin-2-yl}vinyl]-1H-indazol-6-yl}thio)-3-fluoro-N-methylbenzamide I-91 (29 mg, 59.23 μmol, yield: 22.65 %).

The product was verified by LCMS, F-NMR, H-NMR and C-NMR.

For MS-ESI, the calculated value was $[M+H]^+$ 490.2, and the measurement was 490.2.

$^1$HNMR (400 MHz, $CD_3OD$) δ (ppm) 8.56 (d, *J* = 1.2 Hz, 1H), 8.54 (s, 1H), 7.97 (d, *J* = 8.6 Hz, 1H), 7.91-7.82 (m, 2H), 7.67 (d, *J* = 8.2 Hz, 1H), 7.56-7.48 (m, 2H), 7.39-7.25 (m, 3H), 7.12 (dd, *J* = 8.6, 1.4 Hz, 1H), 4.00 (s, 2H), 2.89 (q, *J* = 7.2 Hz, 4H), 2.83 (s, 3H), 1.22 (t, *J* = 7.2 Hz, 6H).

$^{13}$C NMR (100MHz, $CD_3OD$): δ (ppm) 169.31, 163.96, 161.49, 155.56, 150.41, 144.02, 142.21, 142.04, 138.87, 135.21, 131.24, 131.15, 128.57, 124.63, 123.38, 121.68, 119.69, 118.46, 117.13, 116.90, 110.23, 53.36, 25.28, 8.90.

$^{19}$F NMR (400MHz, $CD_3OD$): δ (ppm) -105.56.

I-92

Step 1

Step 2

Step 1

At room temperature, N-ethyl-N-[[6-[(trans)-2-(6-iodo-1-tetrahydropyran-2-yl-indazol-3-yl)vinyl]-3-pyridyl]methyl]ethylamine 63b (200 mg, 387.28 μmol) was dissolved in N,N-dimethylformamide (6 mL). 4-fluoro-N-methyl-2-mercapto-benzamide 33e (72 mg, 387.28 μmol), [1,1'-bis(diphenylphosphino)ferrocene]palladium dichloride (100 mg, 387.28 μmol) and cesium carbonate (252 mg, 774.57 μmol) were added. The reaction system was stirred at 80°C under the protection of nitrogen to react for 6 h. The reaction mixture was poured into water (30 mL), and then extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a brown solid product 2-[3-[(trans)-2-[5-(diethylaminomethyl)-2-pyridyl]vinyl]-1-tetrahydropyran-2-yl-indazol-6-yl]thioalkyl-4-fluoro-N-methyl-benzamide 71a (100.0 mg, 174.30 μmol, yield: 45.01 %).

The product was verified by LCMS.

For MS-ESI, the calculated value was $[M+H]^+$574.3, and the measurement was 574.3.

Step 2

At room temperature, 2-[3-[(trans)-2-[5-(diethylaminomethyl)-2-pyridyl]vinyl]-1-tetrahydropyran-2-yl-indazol-6-yl]thioalkyl-4-fluoro-N-methyl-benzamide 71a (100 mg, 174.30 μmol) was dissolved in dichloromethane (9 mL), followed by the addition of trifluoroacetic acid (3 mL). The reaction system was stirred at 25°C to react for 16 h. The reaction mixture was poured into water (10 mL), adjusted with the saturated aqueous solution of sodium carbonate to the pH of 8-9, and then extracted with dichloromethane (10 mL × 3), and the organic phases were combined, washed with saturated brine (10 mL), dried over anhydrous sodium sulfate, and concentrated under reduced pressure to obtain a crude product. The crude product was purified by the high-pressure preparative chromatography to obtain a yellow solid product 2-({3-[(E)-2-{5-[(diethylamino)methyl]pyridin-2-yl}vinyl]-1H-indazol-6-yl}thio)-4-fluoro-N-methylbenzamide I-92 (6.0 mg, 12.25 μmol, yield: 7.03 %).

The product was verified by LCMS, F-NMR, H-NMR and C-NMR.

For MS-ESI, the calculated value was $[M+H]^+$490.2, and the measurement was 490.3

$^1$H NMR (400MHz, $CD_3OD$): δ (ppm) 8.55 (s, 1H), 8.16 (d, *J* = 8.4 Hz, 1H), 7.98-7.83 (m, 2H), 7.80-7.66 (m, 2H), 7.61 (d, *J* = 16.4 Hz, 1H), 7.52 (dd, *J* = 8.6, 5.8 Hz, 1H), 7.29 (dd, *J* = 8.6, 1.4 Hz, 1H), 7.01-6.96 (m, 1H), 6.73 (dd, *J*= 9.8, 2.4 Hz, 1H), 3.83 (s, 2H), 2.88 (s, 3H), 2.72 (d, *J* = 6.8 Hz, 4H), 1.26-1.01 (m, 6H).

$^{13}$C NMR (100 MHz, $CD_3OD$): δ (ppm) 170.69, 163.70, 157.43, 152.15, 141.35, 140.59, 131.14, 126.22, 122.28, 117.58, 117.33, 117.23, 114.20, 113.98, 54.66, 49.45, 48.81, 47.88, 27.22, 26.82, 9.90.

$^{19}$F NMR (400MHz, $CD_3OD$): δ (ppm) -111.45.

I-93

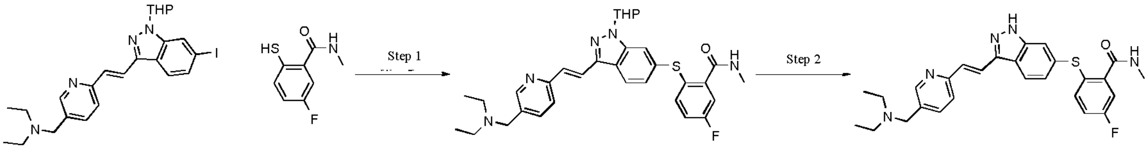


63b 32e 72a I-93

Step 1

At room temperature, N-ethyl-N-[[6-[(trans)-2-(6-iodo-1-tetrahydropyran-2-yl-indazol-3-yl)vinyl]-3-pyridyl]methyl]ethylamine 63b (150 mg, 290.46 μmol) was dissolved in N,N-dimethylformamide (5 mL). 5-fluoro-N-methyl-2-mercapto-benzamide 32e (54 mg, 290.46 μmol), [1,1'-bis(diphenylphosphino)ferrocene]palladium dichloride (70 mg, 95.76 μmol) and cesium carbonate (189 mg, 580.08 μmol) were added. The reaction system was stirred at 80°C under the protection of nitrogen to react for 6 h. The reaction mixture was poured into water (20 mL), and then extracted with ethyl acetate (20 mL × 3), and the organic phases were combined, washed with saturated brine (20 mL), dried over anhydrous sodium sulfate, and concentrated under reduced pressure to obtain a crude product. The crude product was purified by the preparative plate (dichloromethane/methanol = 10/1) to obtain a yellow solid product 2-[3-[(trans)-2-[5-(diethylaminomethyl)-2-pyridyl]vinyl]-1-tetrahydropyran-2-yl-indazol-6-yl]thioalkyl-5-fluoro-N-methyl-benzamide 72a (110.0 mg, 191.73 μmol, yield: 66.01 %).

The product was verified by LCMS.

For MS-ESI, the calculated value was $[M+H]^+$574.3, and the measurement was 574.2.

Step 2

At room temperature, 2-[3-[(trans)-2-[5-(diethylaminomethyl)-2-pyridyl]vinyl]-1-tetrahydropyran-2-yl-indazol-6-yl]thioalkyl-5-fluoro-N-methyl-benzamide 72a (100 mg, 174.30 μmol) was dissolved in dichloromethane (9 mL), followed by the addition of trifluoroacetic acid (3 mL). The reaction system was stirred at 25°C to react for 2 h. The reaction mixture was poured into water (10 mL), adjusted with the saturated aqueous solution of sodium carbonate to the pH of 8-9, and then extracted with dichloromethane (10 mL × 3), and the organic phases were combined, washed with saturated brine (10 mL), dried over anhydrous sodium sulfate, and concentrated under reduced pressure to obtain a crude product. The crude product was purified by the high-pressure preparative chromatography to obtain a yellow solid product 2-({3-[(E)-2-{5-[(diethylamino)methyl]pyridin-2-yl}vinyl]-1H-indazol-6-yl}thio)-5-fluoro-N-methylbenzamide I-93 (3.5 mg, 7.15 μmol, yield: 4.10 %).

The product was verified by LCMS, F-NMR, H-NMR and C-NMR.

For MS-ESI, the calculated value was $[M+H]^+$490.2, and the measurement was 490.3

$^1$H NMR (400 MHz, $CD_3OD$): δ (ppm) 8.63 (s, 1H), 8.05 (d, *J* = 8.4 Hz, 1H), 7.98-7.88 (m, 2H), 7.73 (d, *J* = 8.2 Hz, 1H), 7.60-7.51 (m, 2H), 7.35 (dd, *J* = 8.8, 5.2 Hz, 1H), 7.26 (dd, *J* = 8.8, 2.8 Hz, 1H), 7.21-7.11 (m, 2H), 4.18 (s, 2H), 3.03 (q, *J* = 7.2 Hz, 4H), 2.84 (s, 3H), 1.29 (t, *J* = 7.2 Hz, 6H).

$^{13}$C NMR (100 MHz, $CD_3OD$): δ (ppm) 170.28, 167.39, 164.41, 162.16, 161.94, 158.61, 157.47, 156.30, 152.15, 148.15, 143.60, 130.83, 128.67, 123.59, 121.57, 116.52, 114.34, 107.82, 68.88, 62.90, 49.44, 49.02, 48.38, 43.90, 26.73, 26.56, 26.50, 20.06, 9.88, 1.43.

$^{19}$F NMR (400MHz, $CD_3OD$): δ (ppm) -115.71.

I-94

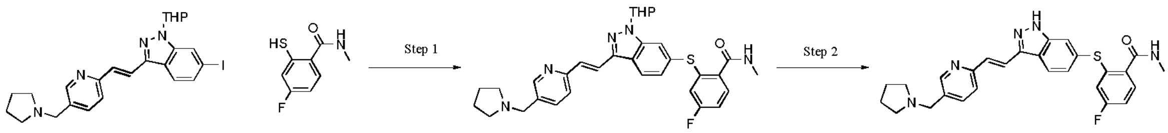


51a 33e 73a I-94

Step 1

At room temperature, 6-iodo-3-[(trans)-2-[5-(pyrrolidin-1-ylmethyl)-2-pyridyl]vinyl]-1-tetrahydropyran-2-ylindazole 51a (200 mg, 388.8 μmol) was dissolved in N,N-dimethylformamide (6 mL), followed by the addition of 4-fluoro-2-mercapto-benzamide 33e (72 mg, 388.8 μmol), [1,1'-bis(diphenylphosphino)ferrocene]dichloropalladium (II) (100 mg,

136.8 μmol) and cesium carbonate (254 mg, 777.6 μmol). The reaction system was stirred at 80°C to react for 6 h under the protection of nitrogen. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a brown solid product 4-fluoro-N-methyl-2-[3-[(trans)-2-[5-(pyrrolidin-1-ylmethyl)-2-pyridyl]vinyl]-1-tetrahydropyran-2-yl-indazol-6-yl]thioalkylbenzamide 73a (120 mg, 209.9 μmol, yield: 54.0 %).

The product was verified by LCMS.

For MS-ESI, the calculated value was $[M+H]^+$ 572.2, and the measurement was 572.3.

Step 2

At room temperature, 4-fluoro-N-methyl-2-[3-[(trans)-2-[5-(pyrrolidin-1-ylmethyl)-2-pyridyl]vinyl]-1-tetrahydropyran-2-yl-indazol-6-yl]thioalkyl benzamide 73a (120 mg, 209.90 μmol) was dissolved in dichloromethane (10 mL), followed by the addition of trifluoroacetic acid (3 mL). The reaction system was stirred at 25°C to react for 2 h. The reaction mixture was poured into water (30 mL), adjusted with the saturated aqueous solution of sodium carbonate to the pH of 8-9, extracted with dichloromethane (30 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product 4-fluoro-N-methyl-2-({3-[(E)-2-{5-[(pyrrolidin-1-yl)methyl]pyridin-2-yl}vinyl]-1H-indazol-6-yl}thio)benzamide I-94 (19.0 mg, 39.0 μmol, yield: 18.6 %).

The product was verified by LCMS, F-NMR, H-NMR and C-NMR.

For MS-ESI, the calculated value was $[M+H]^+$ 488.2, and the measurement was 488.1.

$^1$H NMR (400MHz, $CD_3OD$): δ (ppm) 8.69 (s, 1H), 8.45 (s, 1H), 8.14 (d, *J* = 8.8 Hz, 1H), 8.04-7.94 (m, 2H), 7.74 (d, *J*= 9.6 Hz, 2H), 7.61-7.51 (m, 2H), 7.27 (dd, *J* = 8.6, 1.2 Hz, 1H), 6.98 (d, *J*= 2.6 Hz, 1H), 6.72 (dd, *J* = 9.8, 2.6 Hz, 1H), 4.41 (s, 2H), 3.43-3.32 (m, 4H), 2.89 (s, 3H), 2.10 (s, 4H).

$^{13}$C NMR (100 MHz, $CD_3OD$): δ (ppm) 170.70, 166.19, 163.70, 158.05, 152.10, 143.67, 143.62, 141.42, 141.34, 140.52, 133.45, 131.24, 131.15, 129.93, 127.86, 127.27, 126.58, 123.52, 122.98, 122.29, 117.57, 117.32, 117.27, 114.22, 114.00, 56.26, 54.86, 26.85, 23.90.

$^{19}$F NMR (400MHz, $CD_3OD$): δ (ppm) -111.35.

I-95

Step 1

Step 2

Step 1

At room temperature, 6-iodo-3-[(trans)-2-[5-(pyrrolidin-1-ylmethyl)-2-pyridyl]vinyl]-1-tetrahydropyran-2-ylindazole 51a (200 mg, 388.80 μmol) was dissolved in N,N-dimethylformamide (6 mL). 5-fluoro-N-methyl-2-mercaptobenzamide 32e (72 mg, 388.80 μmol), [1,1'-bis(diphenylphosphino)ferrocene]palladium dichloride (100 mg, 136.80 μmol) and cesium carbonate (254 mg, 778.04 μmol) were added. The reaction system was stirred at 80°C under the protection of nitrogen to react for 6 h. The reaction mixture was poured into water (30 mL), and then extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a brown solid product 5-fluoro-N-methyl-2-[3-[(trans)-2-[5-(pyrrolidin-1-ylmethyl)-2-pyridyl]vinyl]-1-tetrahydropyran-2-yl-indazol-6-yl]thioalkylbenzamide 74a (150 mg, 262.37 μmol, yield: 67.48 %).

The product was verified by LCMS.

For MS-ESI, the calculated value was $[M+H]^+$572.2, and the measurement was 572.1.

Step 2

At room temperature, 2-[3-[(trans)-2-[5-(diethylaminomethyl)-2-pyridyl]vinyl]-1-tetrahydropyran-2-yl-indazol-6-yl]thioalkyl-5-fluoro-N-methyl-benzamide 74a (200 mg, 349.83 μmol) was dissolved in dichloromethane (10 mL), followed by the addition of trifluoroacetic acid (3 mL). The reaction system was stirred at 25°C to react for 2 h. The reaction mixture

was poured into water (10 mL), adjusted with the saturated aqueous solution of sodium carbonate to the pH of 8-9, and then extracted with dichloromethane (10 mL × 3), and the organic phases were combined, washed with saturated brine (10 mL), dried over anhydrous sodium sulfate, and concentrated under reduced pressure to obtain a crude product. The crude product was purified by the high-pressure preparative chromatography to obtain a yellow solid product 5-fluoro-N-methyl-2-({3-[(E)-2-{5-[(pyrrolidin-1-yl)methyl]pyridin-2-yl}vinyl]-1H-indazol-6-yl}thio)benzamide I-95 (11 mg, 22.56 μmol, yield: 6.45 %).

The product was verified by LCMS, F-NMR, H-NMR and C-NMR.

For MS-ESI, the calculated value was $[M+H]^+$488.2, and the measurement was 488.1

$^1H$ NMR (400 MHz, $CD_3OD$): δ (ppm) 8.67 (s, 1H), 8.03 (d, *J* = 8.6 Hz, 1H), 7.90-7.97 (m, 2H), 7.70 (d, *J* = 8.0 Hz, 1H), 7.54 (d, *J* = 17.2 Hz, 2H), 7.34 (dd, *J* = 8.8, 5.2 Hz, 1H), 7.27 (dd, *J* = 8.8, 2.8 Hz, 1H), 7.20-7.07 (m, 2H), 4.36 (s, 2H), 3.32-3.23 (m, 4H), 2.86 (s, 3H), 2.08 (s, 4H).

$^{13}C$ NMR (100 MHz, $CD_3OD$): δ (ppm) 170.27, 161.89, 157.85, 152.02, 143.58, 141.88, 140.40,136.41, 136.05, 130.87, 129.83, 127.77, 126.55, 126.07, 123.39, 122.5, 121.58, 118.85,118.63, 116.35, 116.11, 114.41, 56.36, 54.79, 49.68, 49.47, 49.26, 49.04, 48.83, 48.62, 48.41, 26.79, 23.94.

$^{19}F$ NMR (400MHz, $CD_3OD$): δ (ppm) -115.639

I-97

Step 1

Step 2

Step 3

Step 1

At room temperature, N-ethyl-5-fluoro-2-[(3-iodo-1H-indazol-6-yl)thioalkyl]benzamide 75a (7 g, 15.86 mmol) was dissolved in ethyl acetate (70 mL), followed by the addition of p-toluenesulfonic acid (546 mg, 3.17 mmol) and 3,4-dyhydro-2H-pyran (2.7 g, 31.73 mmol). The reaction system was stirred at 70°C to react for 16 h. The reaction mixture was concentrated under reduced pressure to obtain a crude product. The crude product was purified by the column chromatography (petroleum ether/ethyl acetate = 10/1-1/1) to obtain a yellow solid product N-ethyl-5-fluoro-2-(3-iodo-1-tetrahydropyran-2-yl-indazol-6-yl)thioalkylbenzamide 75b (7 g, 13.32 mmol, yield: 83.99 %). The product was verified by LCMS and H-NMR.

For MS-ESI, the calculated value was $[M+H]^+$ 526.0, and the measurement was 525.9.

$^1H$ NMR (400MHz, $CDCl_3$): δ (ppm) δ 7.49 (s, 1H), 7.43 (dd, *J* = 8.8, 2.8 Hz, 1H), 7.38 (d, *J* = 8.4 Hz, 1H), 7.32 (dd, *J* = 8.4, 5.2 Hz, 1H), 7.09-7.04 (m, 2H), 6.39 (s, 1H), 5.60 (dd, *J* = 9.6, 2.8 Hz, 1H), 3.98 *(d, J* = 12.0 Hz, 1H), 3.71-3.65 (m, 1H), 3.42-3.35 (m, 2H), 2.50-2.42 (m, 1H), 2.10-2.04 (m, 2H), 1.75-1.65 (m, 3H), 1.11 (t, *J* = 7.2 Hz, 3H).

Step 2

At room temperature, N-ethyl-5-fluoro-2-(3-iodo-1-tetrahydropyran-2-yl-indazol-6-yl)thioalkyl benzamide 75b (3.5 g, 6.66 mmol) was dissolved in 1,4-dioxane (40 mL), followed by the addition of 5-(pyrrolidin-1-ylmethyl)-2-vinylpyridine 30a (1.3 g, 6.66 mmol), Xphos Pd G3 (564 mg, 666.19 μmol) and triethylamine (2 g, 19.99 mmol). The reaction system was stirred at 90°C to react for 16 h under the protection of nitrogen. The reaction mixture was poured into water (100 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the column chromatography (dichloromethane/methanol = 100/1-10/1) to obtain a yellow solid product N-ethyl-5-fluoro-2-[3-[(trans)-2-[5-(pyrrolidin-1-ylmethyl)-2-pyridyl]vinyl]-1-tetrahydropyran-2-yl-indazol-6-yl]thioalkylbenzamide 75c (2.1 g, 3.59 mmol, yield: 53.82 %).

The product was verified by LCMS.

For MS-ESI, the calculated value was $[M+H]^+$ 586.3, and the measurement was 586.2.

Step 3

At room temperature, N-ethyl-5-fluoro-2-[3-[(trans)-2-[5-(pyrrolidin-1-ylmethyl)-2-pyridyl]vinyl]-1-tetrahydropyran-2-yl-indazol-6-yl]thioalkyl benzamide 75c (2.1 g, 3.59 mmol) was dissolved in dichloromethane (30 mL), followed by the addition of trifluoroacetic acid (10 mL). The reaction system was stirred at 25°C to react for 16 h. The reaction mixture was poured into water (50 mL), adjusted with saturated sodium carbonate to the pH of 8 and extracted with dichloromethane(20 mL × 3), and the organic phases were combined, washed with saturated brine (50 mL), dried over anhydrous sodium

sulfate, and concentrated under reduced pressure to obtain a crude product. The crude product was purified by the high-pressure liquid chromatography to obtain a yellow solid product N-ethyl-5-fluoro-2-({3-[(E)-2-{5-[(pyrrolidin-1-yl)methyl]pyridin-2-yl}vinyl]-1H-indazol-6-yl}thio)benzamide I-97 (157 mg, 312.99 μmol, yield: 8.73 %).

The product was verified by LCMS, F-NMR and H-NMR.

For MS-ESI, the calculated value was $[M+H]^+$ 502.2, and the measurement was 502.0.

$^1$H NMR (400MHz, $CD_3OD$): δ (ppm) 8.51 (d, *J* = 1.6 Hz, 1H), 8.04 (d, *J* =8.4 Hz, 1H), 7.87-7.80 (m, 2H), 7.66 (d, *J* =8.0 Hz, 1H), 7.57-7.50 (m, 2H), 7.38-7.35 (m, 1H), 7.25 (dd, *J* =8.8, 2.8 Hz, 1H), 7.18-7.13 (m, 2H), 3.69 (s, 2H), 3.33-3.29 (m, 2H), 2.58 (s, 4H), 1.85-1.81 (m, 4H), 1.14 (t, *J* = 7.2Hz, 3H).

$^{19}$F NMR (400MHz, $CD_3OD$): δ (ppm) -115.65

I-98

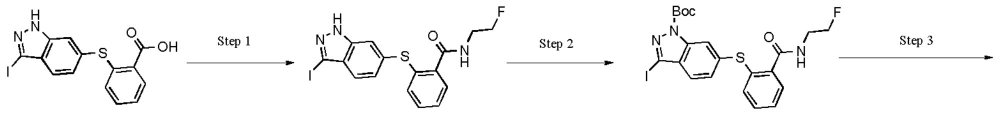


53a 76a 76b

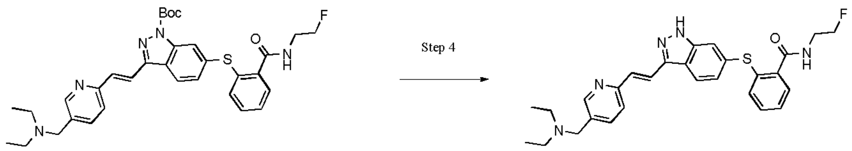


76c I-98

Step 1

At room temperature, 2-[(3-iodo-1H-indazol-6-yl)thioalkyl]benzoic acid 53a (1.0 g, 2.52 mmol) was dissolved in N,N-dimethylformamide (10 mL), followed by the addition of 2-fluoroethylamine hydrochloride (502.4 mg, 5.05 mmol), 1-hydroxybenzotriazole (511.6 mg, 3.79 mmol), 1-(3-dimethylaminopropyl)-3-ethylcarbodiimide hydrochloride (725.8 mg, 3.79 mmol) and N,N-diisopropylethylamine (1.3 g, 10.10 mmol). The reaction system was stirred to react for 2 h. The system was poured into water (30 mL) and extracted with ethyl acetate (30 mL × 3), and the organic phases were combined, washed with brine (50 mL), dried over anhydrous sodium sulfate, and concentrated to obtain a crude product. The crude product was purified by hight-pressure liquid chromatography to obtain a white solid product, N-(2-fluoroethyl)-2-( (3-iodo-1H-indazol-6-yl)thioalkyl )benzamide 76a (750.0 mg, 1.70 mmol, yield: 67.34 %).

The product was verified by LCMS and H-NMR.

For MS-ESI, the calculated value was $[M+H]^+$ 442.0, and the measurement was 442.1.

$^1$H NMR (400 MHz, $CD_3OD$) δ (ppm) 8.48 (s, 0H), 7.63-7.53 (m, 1H), 7.41 (dd, J = 8.4, 3.8 Hz, 1H), 7.34-7.12 (m, 1H), 7.10-6.93 (m, 0H), 4.76-4.46 (m, 2H), 3.80-3.45 (m, 2H).

Step 2

At room temperature, N-(2-fluoroethyl)-2-( (3-iodo-1H-indazol-6-yl)thioalkyl )benzamide 76a (700 mg, 1.59 mmol) was dissolved in tetrahydrofuran (7 mL), followed by the addition of triethylamine (642.1 mg, 6.35 mmol) and di-tert butyl dicarbonate (692.4 mg, 3.17 mmol). The system was stirred at 60°C to react for 16 h, then poured into water (20 mL) and extracted with ethyl acetate (30 mL × 3), and the organic phases were combined, washed with brine (20 mL), dried over anhydrous sodium sulfate and concentrated to obtain a crude product. The crude product was purified by preparative plate (PE/EA=3 /1) to obtain a white solid product, 6-( (2-( (2-fluoroethyl)carbamoyl)phenyl)thioalkyl)-3-iodo-1-H-indazol-1-tert-butylformate 76b (655.0 mg, 1.21 mmol, yield: 76.27 %).

The product was verified by LCMS and H-NMR.

For MS-ESI, the calculated value was $[M+H]^+$ 542.0, and the measurement was 442.1.

$^1$H NMR (400MHz, $CDCl_3$): δ (ppm) 8.06 (s, 1H), 7.69 (dd, J = 4.8, 2.0 Hz, 1H), 7.61-7.47 (m, 1H), 7.42-7.35 (m, 4H), 6.72 (s, 1H), 4.57 (t, J = 4.8 Hz, 1H), 4.45 (t, J = 4.8 Hz, 1H), 3.74 (dd, J = 10.4, 5.2 Hz, 1H), 3.67 (dd, J = 10.4, 5.2 Hz, 1H), 1.63 (s, 9H).

Step 3

At room temperature, 6-((2-((2-fluoroethyl)carbamoyl)phenyl)thioalkyl)-3-iodo-1-H-indazol-1-tert-butyl formate 76b (500 mg, 923.57 μmol) was dissolved in dioxane (10 mL), followed by the addition of N-ethyl-N-((6-vinylpyridin-3-yl) methyl)ethylamine (175.8 mg, 923.57 μmol), tris(dibenzalaceton)dipalladium (422.9 mg, 461.79 μmol), tris(o-methylphenyl)phosphine (421.6 mg, 1.39 mmol) and triethylamine (467.3 mg, 4.62 mmol). The system was stirred at 95°C under the protection of nitrogen to react for 2 h. The reaction system was poured into water (10 mL) and extracted with ethyl acetate (10 mL × 3), and the organic phases were combined, washed with brine (20 mL), dried over anhydrous sodium sulfate, and concentrated to obtain a yellow solid crude product (trans)-3-(2-(5-( (diethylamino)methyl)pyridine-2-yl) vinyl)-6-((2-((2-fluoroethyl)carbamoyl)phenyl)thioalkyl)-1H-indol-1-methyl tert-butyl ester 76c (70 mg, 115.94 μmol, yield: 12.55 %).
The product was verified by LCMS.
For MS-ESI, the calculated value was $[M+H]^+$ 604.3, and the measurement was 604.4.

Step 4

At room temperature, (trans)-3-(2-(5-( (diethylamino)methyl)pyridin-2-yl)vinyl)-6-( (2-( (2-fluoroethyl)carbamoyl) phenyl)thioalkyl )-1H-indol-1-methyl tert-butyl ester 76c (300 mg, 496.90 μmol) was dissolved in dichloromethane (10 mL), followed by the addition of trifluoroacetic acid (56.7 mg, 496.90 μmol). The system was stirred at room temperature to react for 2 h. The system was poured into water (50 mL), adjusted with the saturated sodium carbonate solution to the pH of 8-9 and extracted with dichloromethane (10 mL × 3), and the organic phases were combined, washed with brine (20 mL), dried over anhydrous sodium sulfate, and concentrated to obtain a crude product, which was purified by the high-pressure liquid chromatography to obtain a yellow solid product 2-({3-[(E)-2-{5-(diethylamino)methyl]pyridin-2-ylvinyl]-1H-indazol-6-yl}thio)-N-(2-fluoroethyl)benzamide I-98 (16.57 mg, 32.90 μmol, yield: 6.62 %).
The product was verified by LCMS, F-NMR, H-NMR and C-NMR.
For MS-ESI, the calculated value was $[M+H]^+$ 504.2, and the measurement was 504.3.
$^1$H NMR (400 MHz, $CD_3OD$) δ (ppm) 8.50 (d, J = 1.8 Hz, 1H), 8.07 (d, J = 8.4 Hz, 1H), 7.86 (d, J = 16.6 Hz, 2H), 7.69 (s, 1H), 7.58 (dd, J = 11.4, 8.6 Hz, 2H), 7.52-7.49 (m, 1H), 7.34 (d, J = 1.0 Hz, 2H), 7.27-7.19 (m, 2H), 4.57 (s, 1H), 4.45 (s, 1H), 3.69-3.54 (m, 4H), 2.57 (q, J = 7.2 Hz, 4H), 1.10 (m, J = 7.2 Hz, 6H).
$^{13}$C NMR (100 MHz, $CD_3OD$): δ (ppm) 171.41, 161.443, 155.85, 151.04, 139.59, 131.81, 129.14. 126.66, 122.75, 121.84, 116.03, 83.94, 82.27, 79.66, 79.33, 79.01, 55.44, 39.28, 30.79, 11.81.
$^{19}$F NMR (400MHz, $CD_3OD$): δ (ppm) -123.62.

I-99

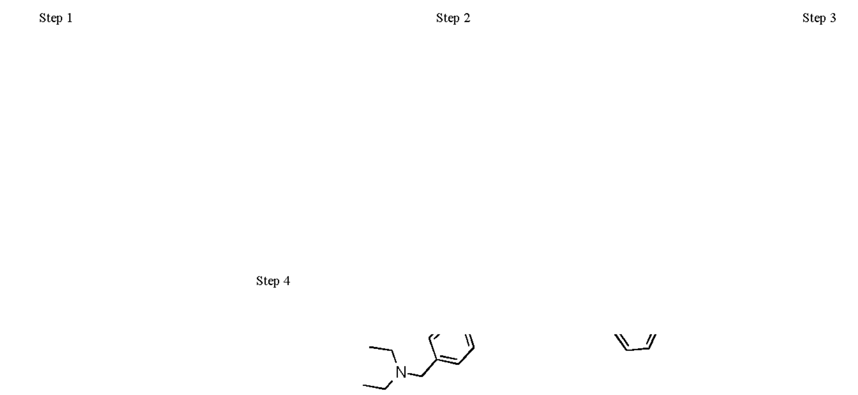


I-99

Step 1

At room temperature, 2-[(3-iodo-1H-indazol-6-yl)thioalkyl]benzoic acid 53a (1.0 g, 2.52 mmol) was dissolved in N,N-dimethylformamide (10 mL), followed by the addition of 2,2-difluoroethylamine (306.9 mg, 3.79 mmol), 1-hydroxybenzotriazole (511.6 mg, 3.79 mmol), 1-(3-dimethylaminopropyl)-3-ethylcarbodiimide hydrochloride (725.8 mg, 3.79 mmol) and N,N-diisopropylethylamine (978.6 mg, 7.57 mmol). The reaction system was stirred to react for 2 h. The system was poured into water (50 mL) and extracted with ethyl acetate (20 mL × 3), and the organic phases were combined, washed with brine (50 mL), dried over anhydrous sodium sulfate, and concentrated to obtain a crude product. The crude

product was purified by column chromatography (DCM/MeOH= 1/0-20/1)to obtain a yellow solid product, N-(2,2-difluoroethyl)-2-[(3-iodo-1H-indazol-6-yl)thioalkyl ]benzamide 77a (1.0 g, 2.18 mmol, yield: 86.27 %).

The product was verified by LCMS and H-NMR.

For MS-ESI, the calculated value was $[M+H]^+$ 460.0, and the measurement was 459.8.

$^1$H NMR (400MHz, $CDCl_3$): δ (ppm) 13.10 (s, 1H), 8.30 (s, 1H), 7.61-7.35 (m, 4H), 7.27-7.08 (m, 3H), 3.73 (s, 2H), 1.18 (s, 1H).

Step 2

At room temperature, N-(2,2-difluoroethyl)-2-[(3-iodo-1H-indazol-6-yl)thioalkyl]benzamide 77a (1.0 g, 2.18 mmol) was dissolved in tetrahydrofuran (10 mL), followed by the addition of triethylamine (881.4 mg, 8.71 mmol) and di-tert butyl dicarbonate (950.4 mg, 4.35 mmol). The reaction system was stirred at 60°C to react for 16 h, then poured into water (100 mL), and filtered to collect the filter cake, which was then dissolved in ethyl acetate (20 mL), dried over anhydrous sodium sulfate, and concentrated to obtain a yellow solid product, 6-[2-(2,2-difluoroethylcarbamoyl)phenyl] thioalkyl-3-iodoindazol-1-tert-butyl formate 77b (600.0 mg, 1.07 mmol, yield: 49.26 %).

The product was verified by LCMS and H-NMR.

For MS-ESI, the calculated value was $[M+H]^+$ 560.0, and the measurement was 459.9.

$^1$H NMR (400MHz, $CD_3OD$): δ (ppm) 8.05 (s, 1H), 7.61-7.45 (m, 1H), 7.42-7.35 (m, 4H), 8.66 (s, 1H), 6.02-5.72 (m, 1H), 3.82-3.71 (m, 2H), 1.63 (s, 9H).

Step 3

At room temperature, 6-[2-(2,2-difluoroethylcarbamoyl)phenyl]thioalkyl-3-iodoindazol-1-tert-butyl formate 77b (300.0 mg, 536.32 μmol) was dissolved in dioxane (10 mL), followed by the addition of N-ethyl-N-( (6-vinylpyridin-3-yl) methyl)ethylamine (102.1 mg, 536.32 μmol), tris(dibenzalaceton)dipalladium (245.6 mg, 268.16 μmol), tris(o-methylphenyl)phosphine (244.9 mg, 804.48 μmol) and triethylamine (271.4 mg, 2.68 mmol). The system was stirred at 95°C under the protection of nitrogen to react for 1.5 h. The reaction system was poured into water (20 mL) and extracted with ethyl acetate (10 mL × 3), and the organic phases were combined, washed with brine (50 mL), dried over anhydrous sodium sulfate, and concentrated to obtain a crude product, which was then purified by the preparative plate (DCM/MeOH = 10/1) to obtain a yellow solid product 3-[(trans)-2-[5-(diethylaminomethyl)-2-pyridyl]vinyl]-6-[2-(2,2-difluoroethylcarbamoyl)phenyl]thioalkylindol-1-tert-butyl formate 77c (300.0 mg, 482.52 μmol, yield: 89.97 %).

The product was verified by LCMS.

For MS-ESI, the calculated value was $[M+H]^+$ 622.3, and the measurement was 622.1.

Step 4

At room temperature, 3-[(trans)-2-[5-(diethylaminomethyl)-2-pyridyl]vinyl]-6-[2-(2,2-difluoroethylcarbamoyl) phenyl]thioalkyl indol-1-tert-butyl formate 77c (500.0 mg, 804.20 μmol) was dissolved in dichloromethane (5 mL), followed by the addition of trifluoroacetic acid (916.9 mg, 8.04 mmol). The system was stirred at room temperature to react for 2 h. The system was poured into water (10 mL), adjusted with the saturated sodium carbonate solution to the pH of 8-9 and extracted with dichloromethane (20 mL × 3), and the organic phases were combined, washed with brine (20 mL), dried over anhydrous sodium sulfate, and concentrated to obtain a crude product, which was purified by the high-pressure liquid chromatography to obtain a yellow solid product 2-({3-[(E)-2-{5-[(diethylamino)methyl]pyridin-2-yl}vinyl]-1H-indazol-6-yl}thio)-N-(2, 2-difluoroethyl)benzamide I-99 (18.47 mg, 35.41 μmol, yield: 4.40 %).

The product was verified by LCMS, F-NMR, H-NMR and C-NMR.

For MS-ESI, the calculated value was $[M+H]^+$ 522.2, and the measurement was 522.1.

$^1$H NMR (400MHz, $CD_3OD$): δ (ppm) 8.50 (s, 1H), 8.07 (d, J=8.4 Hz, 1H), 7.88-7.61 (m, 2H), 7.68 (d, J=8.0 Hz, 1H), 7.60-7.50 (m, 3H), 7.36-7.32 (m, 2H), 7.25-7.20 (m, 2H), 6.09-5.81 (m, 1H), 3.69-3.64 (m, 4H), 2.60-2.55 (m, 4H), 1.13-1.07 (m, 6H).

$^{13}$C NMR (100MHz, $CD_3OD$): δ (ppm) 171.68, 155.77, 151.05, 143.94, 143.80, 139.63, 138.26, 136.67, 135.14, 134.94, 132.87, 131.97, 130.30, 129.13, 128.00, 126.68, 125.20, 122.78, 122.56, 121.75, 115.60, 115.36, 55.39, 43.18, 30.75, 11.68.s

$^{19}$F NMR (400MHz, $CD_3OD$): δ (ppm) -123.90.

I-100

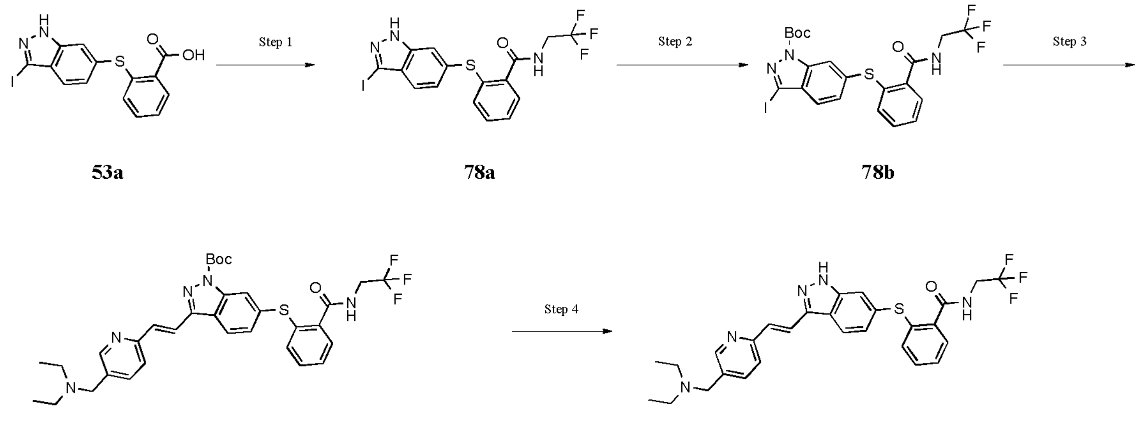


53a 78a 78b

78c I-100

Step 1

At room temperature, 2-[(3-iodo-1H-indazol-6-yl)thioalkyl]benzoic acid 53a (798.3 mg, 2.01 mmol) was dissolved in N,N-dimethylformamide (10 mL), followed by the addition of 2,2,2-trifluoroethylamine (399.2 mg, 4.03 mmol), 1-hydroxybenzotriazole (408.4 mg, 3.02 mmol), 1-(3-dimethylaminopropyl)-3-ethylcarbodiimide hydrochloride (579.4 mg, 3.02 mmol) and N,N-diisopropylethylamine (781.2 mg, 6.04 mmol). The reaction system was stirred to react for 4 h. The system was poured into water (50 mL) and extracted with ethyl acetate (10 mL × 3), and the organic phases were combined, washed with brine (30 mL), dried over anhydrous sodium sulfate, and concentrated to obtain a crude product. The crude product was purified by preparative plate (DCM/MeOH= 10/1)to obtain a yellow solid product, 2-[(3-iodo-1H-indazol-6-yl)thioalkyl ]-N-(2,2,2-trifluoroethyl)benzamide 78a (300.0 mg, 628.61 μmol, yield: 31.20 %).

The product was verified by LCMS and H-NMR.

For MS-ESI, the calculated value was $[M+H]^+$ 478.0, and the measurement was 477.8.

$^1H$ NMR (400MHz, $CDCl_3$): δ (ppm) 10.34 (s, 1H), 8.02 (s, 1H), 7.73 (dd, J = 5.9, 3.4 Hz, 1H), 7.60-7.52 (m, 1H), 7.46 (d, J = 8.1 Hz, 1H), 7.39 (d, J = 3.8 Hz, 2H), 7.17 (dd, J = 8.5, 1.4 Hz, 1H), 4.11-4.00 (m, 2H).

Step 2

At room temperature, 2-[(3-iodo-1H-indazol-6-yl)thioalkyl]-N-(2,2,2-trifluoroethyl)benzamide 78a (250.0 mg, 523.8 μmol) was dissolved in tetrahydrofuran (6 mL), followed by the addition of triethylamine (159.0 mg, 1.57 mmol) and di-tert butyl dicarbonate (228.6 mg, 1.05 mmol). The system was stirred at 60°C to react for 16 h, then poured into water (50 mL) and extracted with ethyl acetate(30 mL × 3), and the organic phases were combined, washed with brine (30 mL), dried over anhydrous sodium sulfate and concentrated to obtain a crude product, which was purified by the preparative plate (PE/EA = 1/1) to obtain a white solid product 3-iodo-6-[2-(2, 2, 2-trifluoroethylcarbamoyl)phenyl]thioalkylindazol-1-tert-butyl carboxylate 78b (200.0 mg, 346.41 μmol, yield: 66.13 %).

The product was verified by LCMS and H-NMR.

For MS-ESI, the calculated value was $[M+H]^+$ 578.0, and the measurement was 477.8.

$^1H$ NMR (400MHz, $CDCl_3$): δ (ppm) 8.04 (s, 1H), 7.75-7.71 (m, 1H), 7.44-7.35 (m, 5H), 7.26 -7.23 (m, 1H), 4.05 (dd, J = 9.2, 6.4 Hz, 2H), 1.62 (s, 9H).

Step 3

At room temperature, 3-iodo-6-[2-(2, 2, 2-trifluoroethylcarbamoyl)phenyl]thioalkyl indazol-1-tert-butyl carboxylate 78b (213.3 mg, 369.43 μmol) was dissolved in dioxane (5 mL), followed by the addition of N-ethyl-N-[(6-vinyl-3-pyridyl)methyl]ethylamine (70.3 mg, 369.43 μmol), dichloropalladium (II) (100.0 mg, 563.92 μmol), tris(o-methylphenyl) phosphine (100.0 mg, 328.55 μmol) and triethylamine (186.9 mg, 1.85 mmol). The system was stirred at 95°C under the protection of nitrogen to react for 1.5 h. The reaction system was poured into water (50 mL) and extracted with ethyl acetate (30 mL × 3), and the organic phases were combined, washed with brine (50 mL), dried over anhydrous sodium sulfate, and concentrated to obtain a crude product, which was then purified by the preparative plate (DCM/MeOH = 10/1) to obtain a brown solid product 3-[(trans)-2-[5-(diethylaminomethyl)-2-pyridyl]vinyl]-6-[2-(2,2,2-trifluoroethylaminoformyl)phenyl] thioalkylindazol-1-tert-butyl carboxylate 78c (90 mg, 140.68 μmol, yield: 38.08 %).

The product was verified by LCMS.

For MS-ESI, the calculated value was $[M+H]^+$ 640.2/641.3, and the measurement was 639.9/640.9.

Step 4

At room temperature, 3-[(trans)-2-[5-(diethylaminomethyl)-2-pyridyl]vinyl]-6-[2-(2,2,2-trifluoroethylcarbamoyl) phenyl]thioalkyl indazol-1-tert-butyl carboxylate 78c (90 mg, 140.68 μmol) was dissolved in dichloromethane (10 mL), followed by the addition of trifluoroacetic acid (2 mL). The system was stirred at room temperature to react for 2 h. The system was poured into water (20 mL), adjusted with sodium carbonate to the pH of 8-9 and extracted with dichloromethane (50 mL × 3), and the organic phases were combined, washed with brine (100 mL), dried over anhydrous sodium sulfate, and concentrated to obtain a crude product, which was then purified by the high-pressure liquid chromatography to obtain a yellow solid product 2-({3-[(E)-2-{5-[(diethylamino)methyl]pyridin-2-yl}vinyl]-1H-indazol-6-yl}thio)-N-(2,2,2-trifluoroethyl)benzamide I-100 (9.93 mg, 18.40 μmol, yield: 13.08 %).

The product was verified by LCMS, F-NMR, H-NMR and C-NMR.

For MS-ESI, the calculated value was $[M+H]^+$ 540.2, and the measurement was 540.0.

$^1H$ NMR (400MHz, $CD_3OD$): δ (ppm) 8.74 (s, 1H), 8.13-8.04 (m, 3H), 7.91 (d, *J* = 8.0 Hz, 1H), 7.62 (d, *J* = 16.0 Hz, 2H), 7.54-7.49 (m, 1H), 7.40-7.32 (m, 2H), 7.27-7.22 (m, 1H), 4.47 (s, 2H), 4.09-4.02 (m, 2H), 3.30-3.21 (m, 4H), 1.39 (t, *J* = 7.3 Hz, 6H).

$^{13}C$ NMR (100 MHz, $CD_3OD$) δ (ppm) 171.40, 151.50, 151.47, 143.65, 143.37, 142.21, 142.17, 138.16, 136.50, 135.62, 133.04, 132.14, 129.14, 128.15, 127.00, 125.97, 123.78, 122.41, 121.83, 115.37, 54.18, 41.82, 41.47, 17.57, 9.01.

$^{19}F$ NMR (400MHz, $CD_3OD$): δ (ppm) -73.46, -77.25

I-101

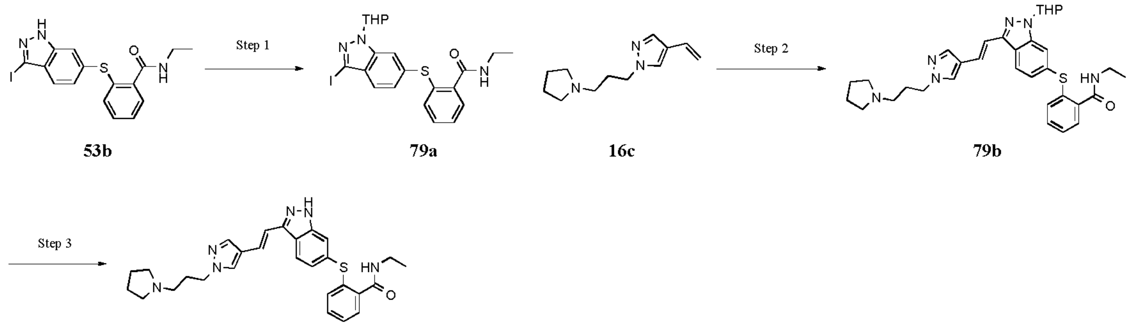


53b 79a 16c 79b I-101

Step 1

At room temperature, N-ethyl-2-[(3-iodo-1H-indazol-6-yl)thioalkyl]benzamide 53b (2.4 g, 5.67 mmol) was dissolved in ethyl acetate (30 mL), followed by the addition of 3,4dyhydro-2H-pyran (715.4 mg, 8.51 mmol) and p-toluenesulfonic acid (117.2 mg, 680.42 μmol). The reaction system was stirred at 60°C to react for 16 h. The reaction mixture was poured into water (50 mL) and extracted with ethyl acetate (20 mL × 3), and the organic phases were combined, washed with saturated brine (50 mL), dried over anhydrous sodium sulfate, and concentrated under reduced pressure to obtain a crude product. The crude product was purified by the column chromatography (PE/EA = 1/0-1/1) to obtain a yellow solid product N-ethyl-2-( (3-iodo-1-(tetrahydro-2H-pyran-2-yl)-1H-indazol-6-yl)thioalkyl)benzamide 79a (1.5 g, 2.96 mmol, yield: 52.14 %).

The product was verified by LCMS and H-NMR.

For MS-ESI, the calculated value was $[M-THP]^+$ 424.0, and the measurement was 423.9.

$^1H$ NMR (400MHz, $CDCl_3$): δ (ppm) 7.61 (s, 2H), 7.39 (d, J= 8.4 Hz, 1H), 7.31-7.28 (m, 2H), 7.21-7.15 (m, 2H), 6.21 (s, 1H), 5.61 (d, J= 9.2 Hz, 1H), 4.03-3.97 (m, 1H), 3.68 (t, J= 8.8 Hz, 1H), 3.46-3.39 (m, 2H), 2.49-2.46 (m, 1H), 2.10-2.04 (m, 2H), 1.72 (s, 3H), 1.15 (t, J=7.2 Hz, 3H).

Step 2

At room temperature, N-ethyl-2-( (3-iodo-1-(tetrahydro-2H-pyran-2-yl)-1H-indazol-6-yl)thioalkyl)benzamide 79a (1 g, 1.97 mmol) was dissolved in 1,4-dioxane (10 mL), followed by the addition of 1-(3-pyrrolidin-1-ylpropyl)-4-vinylpyrazole 16c (404.6 mg, 1.97 mmol), tris(o-methylphenyl)phosphine (299.9 mg, 985.44 μmol), palladium chloride

(174.7 mg, 985.44 μmol) and triethylamine (997.2 mg, 9.85 mmol). The reaction system was stirred at 95°C to react for 16 h under the protection of nitrogen. The reaction mixture was poured into water (50 mL) and extracted with ethyl acetate (20 mL × 3), and the organic phases were combined, washed with saturated brine (50 mL), dried over anhydrous sodium sulfate, and concentrated under reduced pressure to obtain a crude product. The crude product was purified by the column chromatography (DCM/MeOH = 1/0-10/ 1) to obtain a yellow solid product (trans)-N-ethyl-2-( (3-(2-(1-(3-(pyrrolidin-1-yl) propyl)-1H-pyrazol-4-yl)vinyl)-1-(tetrahydro-2H-pyran-2-yl)-1H-indazol-6-yl)thioalkyl)benzamide 79b (500 mg, 855.03 μmol, yield: 43.38 %).

The product was verified by LCMS.

For MS-ESI, the calculated value was $[M+H]^+$ 585.3, and the measurement was 585.1.

Step 3

At room temperature, (trans)-N-ethyl-2-((3-(2-(1-(3-(pyrrolidin-1-yl)propyl)-1H-pyrazole -4-yl)vinyl)-1-(tetrahydro-2H-pyran-2-yl)-1H-indazol-6-yl)thioalkyl )benzamide 79b (650 mg, 1.11 mmol) was dissolved in trifluoroacetic acid (6 mL) and dichloromethane (20 mL). The reaction system was stirred at 25°C to react for 16 h. The reaction mixture was poured into water (50 mL), adjusted with saturated sodium carbonate to the pH of 8 and extracted with dichloromethane (20 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-ethyl-2-({3-[(E)-2-{1-[3-(pyrrolidin-1-yl)propyl]-1H-pyrazol-4-yl}vinyl]-1H-indazol-6-yl}thio)benzamide I-101 (69.48 mg, 138.78 μmol, yield: 12.49 %).

The product was verified by LCMS, H-NMR and C-NMR.

For MS-ESI, the calculated value was $[M+H]^+$ 501.2, and the measurement was 501.2.

$^1$H NMR (400MHz, $CD_3OD$): δ (ppm) 7.99 (d, J=8.4 Hz, 1H), 7.89 (s, 1H), 7.78 (s, 1H), 7.53 (s, 1H), 7.46 (d, J=7.2 Hz, 1H), 7.36-7.32 (m, 3H), 7.24-7.14 (m, 3H), 4.21 (t, J=6.8 Hz, 2H), 3.36-3.33 (m, 2H), 2.56 (s, 4H), 2.48 (t, J=7.6 Hz, 2H), 2.13-2.06 (m, 2H), 1.81 (s, 4H), 1.16 (t, J=7.2 Hz, 3H).

$^{13}$C NMR (100 MHz, $CD_3OD$) δ (ppm) 170.98, 144.82, 143.57, 139.34, 138.37, 136.16, 135.42, 132.94, 131.57, 129.66, 129.06, 128.05, 126.08, 122.83, 122.72, 122.10, 121.23, 118.88, 115.01, 55.00, 54.34, 51.19, 35.80, 30.39, 24.23, 14.73.

I-102

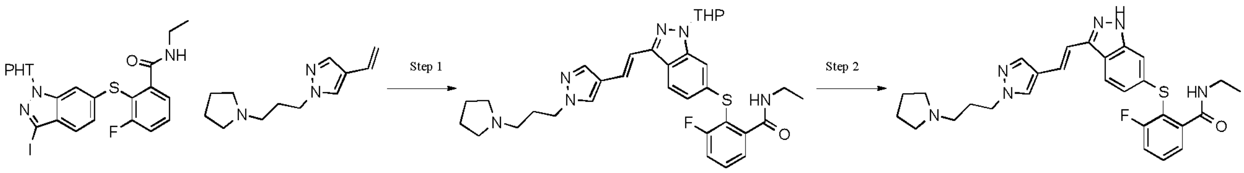


80a 16c 80b I-102

Step 1

At room temperature, N-ethyl-3-fluoro-2-(3-iodo-1-tetrahydropyran-2-yl-indazol-6-yl)thioalkyl benzamide 80a (2 g, 3.81 mmol) was dissolved in 1,4-dioxane (20 mL), followed by the addition of 1-(3-pyrrolidin-1-ylpropyl)-4-vinylpyrazole 16c (782 mg, 3.81 mmol), tris(o-methylphenyl)phosphine (1.2 g, 3.81 mmol), palladium chloride (338 mg, 1.90 mmol) and triethylamine (1.2 g, 11.42 mmol). The reaction system was stirred at 90°C to react for 16 h under the protection of nitrogen. The reaction mixture was poured into water (50 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 50/1-10/1) to obtain a yellow solid product N-ethyl-3-fluoro-2-[3-[(trans)-2-[1-(3-pyrrolidin-1-ylpropyl)pyrazol-4-yl]vinyl]-1-tetrahydropyran-2-yl-indazol-6-yl]thioalkylbenzamide 80b (1.3 g, 2.16 mmol, yield: 56.65 %).

The product was verified by LCMS.

For MS-ESI, the calculated value was $[M+H]^+$ 603.3, and the measurement was 603.1.

Step 2

At room temperature, N-ethyl-3-fluoro-2-[3-[(trans)-2-[1-(3-pyrrolidin-1-ylpropyl)pyrazole -4-yl]vinyl]-1-tetrahydropyran-2-yl-indazol-6-yl]thioalkyl benzamide 80b (1.3 g, 2.16 mmol) was dissolved in dichloroethane (24 mL), followed

by the addition of trifluoroacetic acid (8 mL). The reaction system was stirred at 25°C to react for 16 h. The reaction mixture was poured into water (50 mL), adjusted with the saturated sodium carbonate solution to the pH of 8 and extracted with dichloromethane (20 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-ethyl-3-fluoro-2-({3-(E)-2-{1-[3-(pyrrolidin-1-yl)propyl]-1H-pyrazol-4-yl}vinyl]-1H-indazol-6-yl}thio)benzamide I-102 (94.94 mg, 183.05 μmol, yield: 8.49 %).

The product was verified by LCMS, F-NMR, H-NMR and C-NMR.

For MS-ESI, the calculated value was $[M+H]^+$ 519.2, and the measurement was 519.1.

$^1H$ NMR (400MHz, $CD_3OD$): δ (ppm) 7.92-7.89 (m, 2H), 7.78 (s, 1H), 7.55-7.50 (m, 1H), 7.34-7.25 (m, 4H), 7.13 (d, *J* = 16.8 Hz, 1H), 7.07 (dd, *J* = 8.4, 1.6 Hz, 1H), 4.20 (t, *J* = 6.8 Hz, 2H), 3.33-3.31 (m, 2H), 2.58-2.55 (m, 4H), 2.50-2.46 (m, 2H), 2.12-2.05 (m, 2H), 1.83-1.79 (m, 4H), 1.12 (t, *J* = 7.2 Hz, 3H).

$^{13}C$ NMR (100 MHz, $CD_3OD$) δ (ppm) 169.98, 165.45, 162.97, 145.61, 144.74, 143.40, 138.34, 136.48, 132.62, 132.53, 129.65, 124.77, 124.74, 123.44, 122.72, 122.29, 122.10, 120.65, 120.06, 119.86, 118.91, 118.45, 118.22, 111.44, 55.00, 54.34, 51.17, 35.81, 30.39, 24.20, 14.61.

$^{19}F$ NMR (400MHz, $CD_3OD$): δ (ppm) -105.79.

I-104

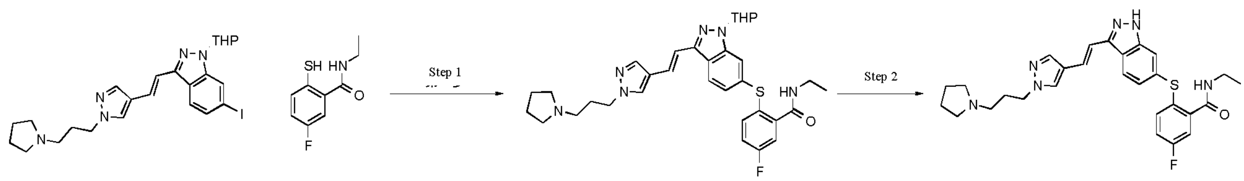


56a 68a 81a I-104

Step 1

Under the protection of nitrogen at room temperature, 6-iodo-3-[(trans)-2-[1-(3-pyrrolidin-1-ylpropyl)pyrazole -4-yl]vinyl]-1-tetrahydropyran-2-ylindazole 56a (500 mg, 940.86 μmol) was dissolved in DMF (8 mL), followed by the addition of N-ethyl-5-fluoro-2-thioalkyl-benzamide 68a (187.5 mg, 940.86 μmol), cesium carbonate (613.1 mg, 1.88 mmol) and Pd(dppf)$Cl_2$ (341.4 mg, 470.43 μmol). The reaction system was stirred at 80°C to react for 5.0 h. The reaction mixture was poured into water (50 mL), extracted with EA (50 mL × 3), and the organic phases were combined, washed with saturated brine (30 mL), dried over anhydrous sodium sulfate, and concentrated under reduced pressure to obtain a crude product. The crude product was purified by the preparative thin-layer chromatography (DCM/MeOH = 10/1) to obtain a brown solid crude product ethyl-5-fluoro-2-[3-[(trans)-2-[1-(3-pyrrolidin-1-ylpropyl)pyrazol-4-yl]vinyl]-1-tetrahydropyran-2-ylindol-6-yl]thioalkylbenzamide 81a (400 mg, crude).

The product was verified by LCMS.

For MS-ESI, the calculated value was $[M+H]^+$ 603.3, and the measurement was 603.2.

Step 2

Ethyl-5-fluoro-2-[3-[(trans)-2-[1-(3-pyrrolidin-1-ylpropyl)pyrazole -4-yl]vinyl]-1-tetrahydropyran-2-ylindol-6-yl] thioalkyl benzamide 81a (350 mg, 580.66 μmol) was dissolved in DCM (12 mL), followed by the addition of trifluoroacetic acid (4 mL). The reaction system was stirred at 25°C to react for 2 h. The reaction mixture was poured into water (50 mL), adjusted with the saturated aqueous solution of sodium bicarbonate to the pH of 8-9, and extracted with DCM (50 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a brown solid product N-ethyl-5-fluoro-2-({3-[(E)-2-{1-[3-(pyrrolidin-1-yl)propyl]-1H-pyrazol-4-yl}vinyl]-1H-indazol-6-yl}thio)benzamide I-104 (52.07 mg, 100.40 μmol, yield: 17.3 %).

The product was verified by LCMS, F-NMR, H-NMR and C-NMR.

For MS-ESI, the calculated value was $[M+H]^+$519.2, and the measurement was 519.1.

$^1H$ NMR (400MHz, $CD_3OD$): δ (ppm) 7.95 (d, J=8.8 Hz, 1H), 7.91 (s, 1H), 7.84 (s, 1H), 7.46 (s, 1H), 7.36-7.24 (m, 3H), 7.18-7.10 (m, 3H), 4.29 (t, J=6.4, 2H), 3.66 (s, 2H), 3.35-3.10 (m, 2H), 3.23 -3.19 (m, 2H), 3.06-3.03 (m, 2H), 2.33-2.26 (m, 2H), 2.13 (s, 2H), 2.02-1.99 (m, 2H), 1.14 (t, J=7.2, 3H).

$^{13}C$ NMR (100MHz, $CD_3OD$) δ (ppm) 169.55, 164.43, 161.96, 161.88, 1616.51, 144.65, 143.49, 142.26, 142.19, 138.96, 136.48, 136.40, 136.20, 130.60, 130.56, 129.96, 125.31, 122.66, 122.62, 122.45, 121.00, 119.14, 118.74, 118.52, 116.32, 116.09, 113.98, 55.27, 53.72, 35.84, 27.86, 23.98, 14.60.

$^{19}F$ NMR (400MHz, $CD_3OD$): δ (ppm) -77.23, -115.62.

I-106

Step 1

Step 2

Step 1

At room temperature, 6-iodo-3-[(trans)-2-[1-(3-pyrrolidin-1-ylpropyl)pyrazole -4-yl]vinyl]-1-tetrahydropyran-2-ylindazole 56a (200 mg, 376.34 μmol) was dissolved in N,N-dimethylformamide (6 mL), followed by the addition of 5-fluoro-N-methyl-2-mercaptobenzamide 32e (69.7 mg, 376.34 μmol), 1,1-bis(diphenylphosphino)ferrocene dichloropalladium (II) (100.0 mg, 136.80 μmol) and cesium carbonate (245.2 mg, 752.69 μmol). The reaction system was stirred at 80°C under the protection of nitrogen to react for 6 h. The reaction mixture was poured into water (30 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (DCM/MeOH = 10/1)to obtain a brown solid product 5-fluoro-N-methyl-2-[3-[(trans)-2-[1-(3-pyrrolidin-1-ylpropyl)pyrazol-4-yl]vinyl]-1-tetrahydropyran-2-ylindazol-6-yl]thioalkylbenzamide 82a (130 mg, 220.81 μmol, yield: 58.67 %).

The product was verified by LCMS.

For MS-ESI, the calculated value was $[M+H]^+$ 589.3, and the measurement was 589.4.

Step 2

At room temperature, 5-fluoro-N-methyl-2-[3-[(trans)-2-[1-(3-pyrrolidin-1-ylpropyl)pyrazole -4-yl]vinyl]-1-tetrahydropyran-2-ylindazol-6-yl]thioalkyl benzamide 82a (130 mg, 220.81 μmol) was dissolved in dichloromethane (10 mL) and trifluoroacetic acid (3 mL); the reaction system was stirred at 25°C to react for 2 h. The reaction mixture was poured into water (20 mL), adjusted with the saturated sodium carbonate solution to the pH of 8 and extracted with ethyl acetate (20 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a pale-yellow solid product 5-fluoro-N-methyl-2-({3-[(E)-2-{1-[3-(pyrrolidin-1-yl)propyl]-1H-pyrazol-4-ylvinyl]-1H-indazol-6-yl}thio)benzamide I-106 (8.5 mg, 16.84 μmol, yield: 7.63 %).

The product was verified by LCMS, F-NMR, H-NMR and C-NMR.

For MS-ESI, the calculated value was $[M+H]^+$ 505.2, and the measurement was 505.1.

$^1H$ NMR (400 MHz, $CD_3OD$) δ (ppm) 8.54 (s, 1H), 7.96 (d, J = 8.6 Hz, 1H), 7.91 (s, 1H), 7.83 (s, 1H), 7.48 (d, J = 0.8 Hz, 1H), 7.37-7.29 (m, 2H), 7.26 (dd, J = 8.8, 2.8 Hz, 1H), 7.19 -7.10 (m, 3H), 4.28 (t, J = 6.6 Hz, 2H), 3.25 (s, 4H), 3.14-3.07 (m, 2H), 2.84 (s, 3H), 2.31-2.22 (m, 2H), 2.03 (dd, J = 8.4, 5.4 Hz, 4H).

$^{13}C$ NMR (100MHz, $CD_3OD$): δ (ppm) 170.29, 164.33, 161.87, 144.71, 143.50, 141.84, 141.77, 138.88, 136.21, 136.13, 135.95, 131.01, 129.88, 125.51, 122.57, 122.43, 121.10, 118.58, 116.07, 114.31, 55.15, 53.75, 28.18, 26.74, 24.03.

$^{19}F$ NMR (400MHz, $CD_3OD$): δ (ppm) -115.879.

I-107

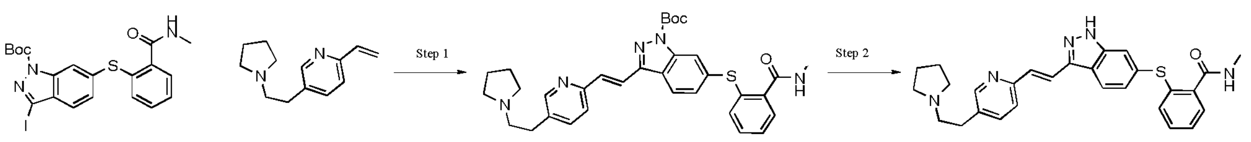


69a 57a 83a I-107

Step 1

At room temperature, 5-(2-pyrrolidin-1-yl)-2-vinylpyridine 57a (218 mg, 1.08 mmol) was dissolved in dioxane (10 mL), followed by the addition of 3-iodo-6-[2-(methylcarbamoyl)phenyl]thioalkyl-indazol-1-tert-butyl carboxylate 69a (500 mg, 981.63 μmol), palladium chloride (52 mg, 294.49 μmol), tris(o-methylphenyl)phosphine (449 mg, 490.81 μmol) and triethylamine (298 mg, 2.94 mmol). The reaction system was stirred at 90°C under the protection of nitrogen to react for 1.5 h. The reaction mixture was poured into water (30 mL) and extracted with ethyl acetate (30 mL × 3), and the organic phases

were combined, washed with saturated brine (40 mL), dried over anhydrous sodium sulfate, and concentrated under reduced pressure to obtain a crude product. The crude product was purified by the preparative thin-layer chromatography (dichloromethane:methanol = 10/1) to obtain a yellow solid 6-[2-(methylaminoformyl)phenyl]thioalkyl-3-[(trans)-2-[5-(2-pyrrolidin-1-yl)-2-pyridyl]vinyl]indazol-1-tert-butyl formate 83a (300 mg, 513.93 μmol, yield: 52.35 %).

The product was verified by LCMS.

For MS-ESI, the calculated value was $[M+H]^+$ 584.3, and the measurement was 584.3.

Step 2

At room temperature, 6-[2-(methylcarbamoyl)phenyl]thioalkyl-3-[(trans)-2-[5-(2-pyrrolidin-1-yl)-2-pyridyl]vinyl] indazol-1-tert-butyl formate 83a (300 mg, 513.93 μmol) was dissolved in dichloromethane (10 mL) and trifluoroacetic acid (293 mg, 2.57 mmol); the reaction system was stirred at 25°C to react for 3 h. The reaction mixture was poured into water (20 mL), adjusted with the saturated sodium carbonate solution to the pH of 8 and extracted with dichloromethane (10 mL × 3), and the organic phases were combined, washed with saturated brine (20 mL), dried over anhydrous sodium sulfate, and concentrated under reduced pressure to obtain a crude product. The crude product was purified by the high-pressure liquid chromatography to obtain a yellow solid product N-methyl-2-({3-[(E)-2-{5-[2-(pyrrolidin-1-yl)ethyl]pyridin-2-yl} vinyl]-1H-indazol-6-yl}thio)benzamide I-107 (22 mg, 45.49 μmol, yield: 8.85 %).

The product was verified by LCMS, H-NMR and C-NMR.

For MS-ESI, the calculated value was $[M+H]^+$ 484.2, and the measurement was 484.1.

$^1$H NMR (400MHz, $CD_3OD$): δ (ppm) 8.45 (d, *J* = 1.6 Hz, 1H), 8.06 (d, *J* = 8.4 Hz, 1H), 7.83 (d, *J* = 16.8 Hz, 1H), 7.76-7.33 (m, 1H), 7.65 (d, *J* = 8.0 Hz, 1H), 7.59-7.53 (m, 2H), 7.48-7.46 (m, 1H), 7.34-7.31 (m, 2H), 7.24-7.20 (m, 2H), 2.91-2.86 (m, 7H), 2.76 (s, 4H), 1.88 (s, 4H).

$^{13}$C NMR (100 MHz, $CDCl_3$&$CD_3OD$) δ (ppm) 171.69, 155.00, 150.41, 143.92, 138.84, 135.96, 135.17, 132.56, 130.73, 127.84, 127.06, 124.94, 122.95, 122.69, 121.85, 115.88, 58.74, 55.26, 33.20, 30.87, 27.25, 24.44.

I-108

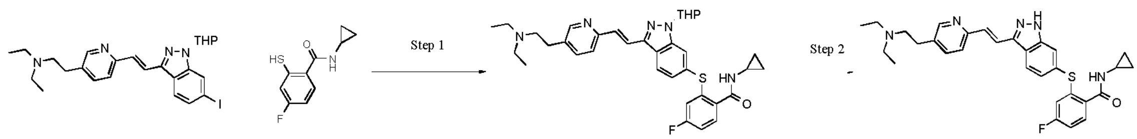


84a 59a 84b I-108

Step 1

At room temperature, N, N-diethyl-2-[6-[(trans)-2-(6-iodo-1-tetrahydropyran-2-ylindazol-3-yl)vinyl]-3-pyridyl] ethylamine 84a (500 mg, 942.61 μmol) was dissolved in N,N-dimethylformamide (10 mL), followed by the addition of N-cyclopropyl-4-fluoro-2-thioalkyl-benzamide 59a (239.0 mg, 1.13 mmol), 1,1'-bis(diphenylphosphino)ferrocene dichloropalladium (II) (136.8 mg, 188.52 μmol) and cesium carbonate (921.4 mg, 2.83 mmol). The reaction system was stirred at 80°C under the protection of nitrogen to react for 16 h. The reaction mixture was poured into water (30 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was purified by the column chromatography (DCM/MeOH = 10/ 1) to obtain a yellow solid product N-cyclopropyl-2-[3-[(trans)-2-[5-[2-(diethylamino)ethyl]-2-pyridyl]vinyl]-1-tetrahydropyran-2-yl-indazol-6-yl]thioalkyl-4-fluoro-benzamide 84b (130 mg, 211.80 μmol, yield: 22.47 %).

The product was verified by LCMS.

For MS-ESI, the calculated value was $[M+H]^+$ 614.3, and the measurement was 614.1.

Step 2

At room temperature, N-cyclopropyl-2-[3-[(trans)-2-[5-[2-(diethylamino)ethyl]-2-pyridyl]vinyl]-1-tetrahydropyran-2-yl-indazol-6-yl]thioalkyl-4-fluoro-benzamide 84b (130 mg, 211.80 μmol) was dissolved in dichloromethane (3 mL), followed by the addition of trifluoroacetic acid (1 mL). The reaction system was stirred at 25°C to react for 2 h. The reaction mixture was poured into water (30 mL), adjusted with the saturated aqueous solution of sodium carbonate to the pH of 8-9 and extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a pale-yellow solid product N-cyclopropyl-2-({3-[(E)-2-{5-[2-(diethylamino)ethyl]pyridin-2-yl}vinyl]-1H-indazol-6-yl}thio)-4-fluorobenzamide I-108 (32.62 mg,

61.59 μmol, yield: 29.08 %).

The product was verified by LCMS, F-NMR, H-NMR and C-NMR.

For MS-ESI, the calculated value was $[M+H]^+$ 530.2, and the measurement was 530.2.

$^1$H NMR (400 MHz, $CD_3OD$): δ (ppm) 8.43 (s, 1H), 8.13 (d, J = 8.4 Hz, 1H), 7.84 (d, J = 16.8 Hz, 1H), 7.73-7.71 (m, 2H), 7.64 (d, J = 8.4 Hz, 1H), 7.57 (d, J = 16.4 Hz, 1H), 7.51-7.47 (m, 1H), 7.26 (d, J = 8.8 Hz, 1H), 7.01-6.96 (m, 1H), 6.75 (dd, J = 9.6, 2.0 Hz, 1H), 2.85-2.75 (m, 5H), 2.68 (q, J = 7.2 Hz, 4H), 1.10 (t, J = 6.8 Hz, 6H), 0.80-0.76 (m, 2H), 0.63-0.57 (m, 2H).

$^{13}$C NMR (100 MHz, $CD_3OD$) δ (ppm) 171.67, 154.81, 150.48, 143.97, 138.97, 136.53, 133.53, 131.19, 130.73, 127.61, 124.61, 123.02, 122.17, 117.85, 117.60, 116.90, 114.30, 114.08, 55.04, 30.75, 30.39, 23.87, 11.51, 6.51.

$^{19}$F NMR (400 MHz, $CD_3OD$): δ (ppm) -111.46.

I-109

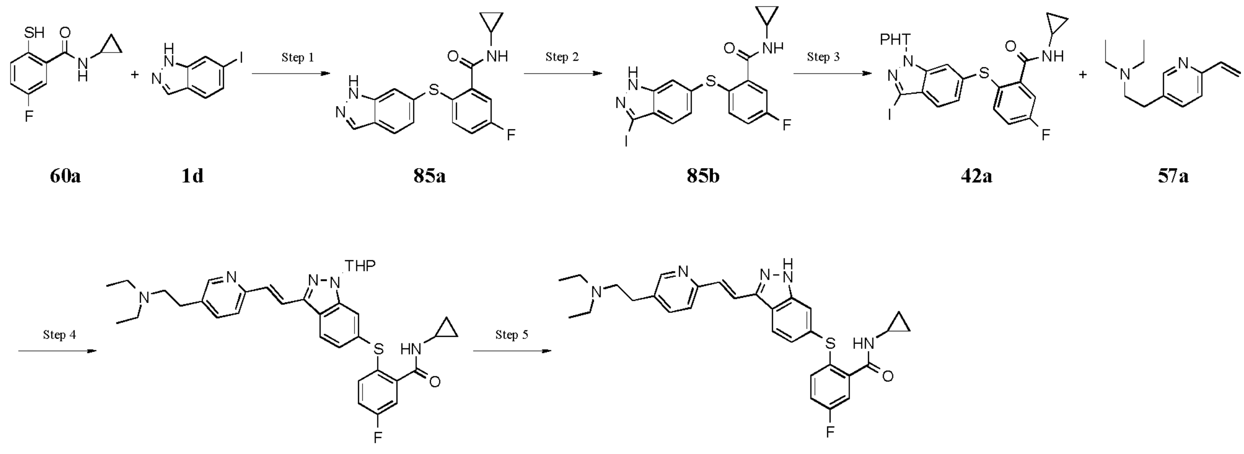


60a 1d 85a 85b 42a 57a

85c I-109

Step 1

At room temperature, N-cyclopropyl-5-fluoro-2-mercapto-benzamide 60a (1.2 g, 5.68 mmol) was dissolved in N,N-dimethylformamide (20 mL), followed by the addition of 6-iodo-1H-indazole 1d (1.4 g, 5.68 mmol), tris(dibenzalaceton)dipalladium (520 mg, 568.03 μmol), 4,5-bisdiphenylphosphino-9,9-dimethylxanthene (657 mg, 1.14 mmol) and cesium carbonate (5.6 g, 17.04 mmol). The reaction system was stirred at 90°C under the protection of nitrogen to react for 16 h. The reaction mixture was poured into an aqueous solution (100 mL) and extracted with ethyl acetate (100 mL × 3), and the organic phases were combined, washed with saturated brine (50 mL), dried over anhydrous sodium sulfate, and concentrated under reduced pressure to obtain a crude product. The crude product was purified by the medium-pressure liquid chromatography to obtain a yellow solid product N-cyclopropyl-5-fluoro-2-(1H-indazol-6-ylthioalkyl)benzamide 85a (1.4 g, 4.28 mmol, yield: 75.29 %).

The product was verified by LCMS and H-NMR.

For MS-ESI, the calculated value was $[M+H]^+$ 328.1, and the measurement was 328.0.

$^1$H NMR (400MHz, $CDCl_3$): δ (ppm) 10.55 (s, 1H), 8.03 (s, 1H), 7.68 (d, *J* = 8.4 Hz, 1H), 7.46 (dd, *J* = 8.8, 2.8 Hz, 1H), 7.35 (t, *J* = 8.8 Hz, 2H), 7.09-7.03 (m, 2H), 6.75 (s, 1H), 2.81-2.76 (m 1H), 0.79-0.74 (m, 2H), 0.43-0.39 (m, 2H).

Step 2

At room temperature, N-cyclopropyl-5-fluoro-2-(1H-indazol-6-ylthioalkyl)benzamide 85a (1.3 g, 3.97 mmol) was dissolved in N,N-dimethylformamide (15 mL), followed by the addition of potassium carbonate (1.7 g, 11.91 mmol) and iodine (2 g, 7.94 mmol). The reaction system was stirred at 40°C to react for 2 h. The reaction mixture was poured into an aqueous solution (100 mL) and extracted with ethyl acetate (50 mL × 3), and the organic phases were combined, washed with saturated brine (50 mL), dried over anhydrous sodium sulfate, and concentrated under reduced pressure to obtain a crude product. The crude product was purified by the medium-pressure liquid chromatography to obtain a yellow solid product N-cyclopropyl-5-fluoro-2-[(3-iodo-1H-indazol-6-yl)thioalkyl]benzamide 85b (1.4 g, 3.09 mmol, yield: 77.78 %).

The product was verified by LCMS and H-NMR.

For MS-ESI, the calculated value was $[M+H]^+$ 454.0, and the measurement was 453.8.

$^1$H NMR (400MHz, $CDCl_3$): δ (ppm) 11.59 (s, 1H), 7.44 - 7.36 (m, 3H), 7.32 (dd, J = 11.2, 8.8 Hz, 1H), 7.09 - 7.03

(m, 2H), 6.77 (s, 1H), 2.81-2.76 (m, 1H), 0.79-0.75 (m, 2H), 0.48 - 0.39 (m, 2H).

Step 3

At room temperature, N-cyclopropyl-5-fluoro-2-[(3-iodo-1H-indazol-6-yl)thioalkyl]benzamide 85b (1.5 g, 3.31 mmol) was dissolved in ethyl acetate (20 mL), followed by the addition of p-toluenesulfonic acid (314 mg, 1.65 mmol) and 3,4-dyhydro-2H-pyran (835 mg, 9.93 mmol). The reaction system was stirred at 70°C to react for 3 h. The reaction mixture was poured into water (50 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was purified by the medium-pressure liquid chromatography to obtain a yellow solid product N-cyclopropyl-5-fluoro-2-(3-iodo-1-tetrahydropyran-2-yl-indazol-6-yl)thioalkylbenzamide 42a (1.1 g, 2.05 mmol, yield: 61.85 %).

The product was verified by LCMS and H-NMR.

For MS-ESI, the calculated value was $[M+H]^+$ 538.0, and the measurement was 537.9.

$^1$H NMR (400 MHz, $CDCl_3$) δ(ppm) 7.45-7.43 (m, 2H), 7.39-7.32 (m, 2H), 7.10-7.04 (m, 2H), 6.58 (s, 1H), 5.59 (d, *J* = 9.2 Hz, 1H), 3.97 *(d, J* = 11.6 Hz, 1H), 3.68 *(t, J* = 8.8 Hz, 1H), 2.82-2.76 (m, 1H), 2.51-2.41 (m, 1H), 2.13-2.03 (m, 2H), 1.77-1.65 (m, 3H), 0.79-0.75 (m, 2H), 0.44-0.40 (m, 2H).

Step 4

At room temperature, N-cyclopropyl-5-fluoro-2-(3-iodo-1-tetrahydropyran-2-yl-indazol-6-yl)thioalkyl benzamide 42a (1 g, 1.86 mmol) was dissolved in 1,4-dioxane (20 mL), followed by the addition of N,N-diethyl-2-(6-vinyl-3-pyridyl)ethylamine 57a (760 mg, 3.72 mmol), tris(o-methylphenyl)phosphine (566 mg, 1.86 mmol), tris(dibenzalaceton) dipalladium (851 mg, 930.43 μmol) and triethylamine (942 mg, 9.30 mmol). The reaction system was stirred at 90°C under the protection of nitrogen to react for 16 h. The reaction mixture was poured into water (50 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was purified by the medium-pressure liquid chromatography to obtain a yellow solid product N-cyclopropyl-2-[3-[(trans)-2-[5-[2-(diethylamino)ethyl]-2-pyridyl]vinyl]-1-tetrahydropyran-2-yl-indazol-6-yl]thioalkyl-5-fluoro-benzamide 85c (1 g, 1.63 mmol, yield: 87.55 %).

The product was verified by LCMS.

For MS-ESI, the calculated value was $[M+H]^+$614.3, and the measurement was 614.2.

Step 5

At room temperature, N-cyclopropyl-2-[3-[(trans)-2-[5-[2-(diethylamino)ethyl]-2-pyridyl]vinyl]-1-tetrahydropyran-2-yl-indazol-6-yl]thioalkyl-5-fluoro-benzamide 85c (900 mg, 1.47 mmol) was dissolved in dichloromethane (18 mL), followed by the addition of trifluoroacetic acid (6 mL). The reaction system was stirred at 25°C to react for 16 h. The reaction mixture was poured into water (50 mL), adjusted with the saturated aqueous solution of sodium carbonate to the pH of 8-9, and then extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{5-[2-(diethylamino)ethyl]pyridin-2-yl}vinyl]-1H-indazol-6-yl}thio)-5-fluorobenzamide I-109 (265.59 mg, 501.43 μmol, yield: 34.20 %).

The product was verified by LCMS, F-NMR and H-NMR.

For MS-ESI, the calculated value was $[M+H]^+$530.2, and the measurement was 530.1

$^1$H NMR (400MHz,$CD_3OD$): δ (ppm) 8.42 (d, *J*= 1.6 Hz, 1H), 8.04 (d, *J* = 8.4 Hz, 1H), 7.81 (d, *J* = 16.4 Hz, 1H), 7.73 (dd, *J* = 8.4, 2.0 Hz, 1H), 7.64 (d, *J* = 8.0 Hz, 1H), 7.56-7.49 (m, 2H), 7.38 (dd, *J* = 8.8, 5.2 Hz, 1H), 7.24 (dd, *J* = 8.8, 2.8 Hz, 1H), 7.18-7.13 (m, 2H), 2.85-2.74 (m, 5H), 2.69 (q, *J* = 7.2 Hz, 4H), 1.10 *(t, J* = 7.2 Hz, 6H), 0.76-0.71 (m, 2H), 0.53-0.49 (m, 2H).

$^{19}$F NMR (400MHz, $CD_3OD$): δ (ppm) -115.50.

I-110

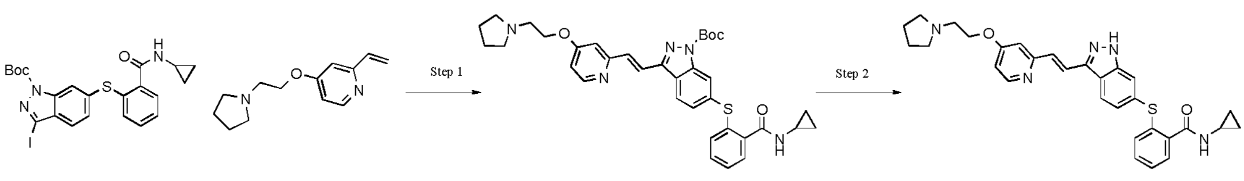


27f 23c 86a I-110

Step 1

At room temperature, 6-[2-(cyclopropylcarbamoyl)phenyl]thioalkyl-3-iodoindazol-1-tert-butyl carboxylate 27f (1.0 g, 1.87 mmol) was dissolved in 1,4-dioxane (10 mL), followed by the addition of 4-(2-pyrrolidin-1-ethoxy)-2-vinylpyridine 23c (489 mg, 2.24 mmol), tris(o-methylphenyl)phosphine (569 mg, 1.87 mmol), triethylamine (567 mg, 5.60 mmol) and palladium chloride (166 mg, 933.89 μmol). The reaction system was stirred at 90°C to react for 16 h under the protection of nitrogen. The reaction mixture was poured into water (50 mL) and extracted with ethyl acetate (20 mL × 3), and the organic phases were combined, washed with saturated brine (50 mL), dried over anhydrous sodium sulfate, and concentrated under reduced pressure to obtain a crude product. The crude product was purified by the column chromatography (DCM/MeOH = 10/1) to obtain a yellow solid product 6-[2-(cyclopropylaminoformyl)phenyl]thioalkyl-3-[(trans)-2-[4-(2-pyrrolidin-1-ethoxy)-2-pyridyl]vinyl]indazol-1-tert-butyl formate 86a (400 mg, 639.20 μmol, yield: 34.22 %).

The product was verified by LCMS.

For MS-ESI, the calculated value was $[M+H]^+$ 625.3, and the measurement was 626.2.

Step 2

At room temperature, 6-[2-(cyclopropylcarbamoyl)phenyl]thioalkyl-3-[(trans)-2-[4-(2-pyrrolidin-1-ethoxy)-2-pyridyl]vinyl]indazol-1-tert-butyl formate 86a (400 mg, 639.20 μmol) was dissolved in dichloromethane (10 mL), followed by the addition of trifluoroacetic acid (364 mg, 3.20 mmol). The reaction system was stirred at 25°C to react for 16 h. The reaction mixture was poured into water (30 mL), adjusted with the saturated sodium carbonate solution to the pH of 8 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{4-[2-(pyrrolidin-1-yl)ethoxy]pyridin-2-yl}vinyl]-1H-indazol-6-yl}thio)benzamide I-110 (67.53 mg, 128.47 μmol, yield: 20.10 %).

The product was verified by LCMS, H-NMR and C-NMR.

For MS-ESI, the calculated value was $[M+H]^+$ 526.2, and the measurement was 526.1.

$^1H$ NMR (400MHz, $CD_3OD$): δ (ppm) 8.35 (d, J=5.6 Hz, 1H), 8.06 (d, J= 8.4 Hz, 1H), 7.85 (d, J= 16.8 Hz, 1H), 7.56-7.44 (m, 3H), 7.37-7.30 (m, 2H), 7.20 (m, 2H), 7.20-7.18 (dd, J= 8.8, 1.6 Hz, 1H), 6.91 (dd, J= 5.6, 2.4 Hz, 1H), 4.29 (t, J= 5.6 Hz, 2H), 2.97 (t, J=5.6 Hz, 2H), 2.81-2.75 (m, 1H), 2.69 (s, 4H), 1.85 (s, 4H), 0.77-0.72 (m, 2H), 0.55-0.51 (m, 2H).

$^{13}C$ NMR (100 MHz, $CD_3OD$) δ (ppm) 172.67, 167.42, 158.51, 151.50, 143.64, 136.31, 135.49, 133.01, 130.76, 129.19, 128.05, 126.67, 125.63, 122.62, 121.80, 115.35, 110.60, 109.51, 68.06, 55.74, 24.40, 23.94, 6.78.

I-111

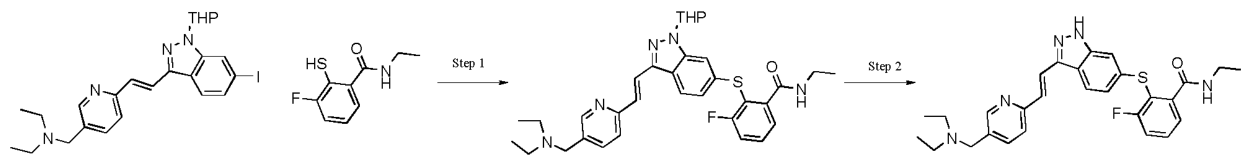


63b 55a 87a I-111

Step 1

At room temperature, N-ethyl-N-[[6-[(trans)-2-(6-iodo-1-tetrahydropyran-2-yl-indazol-3-yl)vinyl]-3-pyridyl]methyl]ethylamine 63b (450 mg, 871.39 μmol) was dissolved in N,N-dimethylformamide (10 mL), followed by the addition of N-ethyl-3-fluoro-2-mercaptobenzamide 55a (208 mg, 1.05 mmol), cesium carbonate (568 mg, 1.74 mmol) and $PdCl_2$(dppf) (190 mg, 261.42 μmol). The reaction system was stirred at 90°C under the protection of nitrogen to react for 16 h.

The reaction mixture was poured into water (30 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the preparative plate (DCM/MeOH = 10/1) to obtain a yellow solid product 2-[3-[(trans)-2-[5-(diethylaminomethyl)-2-pyridyl]vinyl]-1-tetrahydropyran-2-yl-indazol-6-yl]thioalkyl-N-ethyl-3-fluorobenzamide 87a (300 mg, 510.42 μmol, yield: 58.58 %).

The product was verified by LCMS.

For MS-ESI, the calculated value was $[M+H]^+$ 588.3, and the measurement was 588.2.

Step 2

At room temperature, 2-[3-[(trans)-2-[5-(diethylaminomethyl)-2-pyridyl]vinyl]-1-tetrahydropyran-2-yl-indazol-6-yl]thioalkyl-N-ethyl-3-fluorobenzamide 87a (370 mg, 629.52 μmol) was dissolved in dichloromethane (10 mL) and trifluoroacetic acid (5 mL); the reaction system was stirred at 25°C to react for 16 h. The reaction mixture was poured into water (30 mL), adjusted with saturated sodium carbonate to the pH of 8 and extracted with dichloromethane (30 mL × 3), and the organic phases were combined, washed with saturated brine (100 mL), dried over anhydrous sodium sulfate, and concentrated under reduced pressure to obtain a crude product. The crude product was purified by the high-pressure liquid chromatography to obtain a yellow solid product 2-({3-[(E)-2-{5-[(diethylamino)methyl]pyridin-2-yl}vinyl]-1H-indazol-6-yl}thio)-N-ethyl-3-fluorobenzamide I-111 (53.14 mg, 105.51 μmol, yield: 16.76 %).

The product was verified by LCMS, F-NMR, H-NMR and C-NMR.

For MS-ESI, the calculated value was $[M+H]^+$ 504.2, and the measurement was 504.3.

$^1H$ NMR (400MHz, $CD_3OD$): δ (ppm) 8.49 (s, 1H), 7.98 (d, J = 8.0 Hz, 1H), 7.84-7.80 (m, 2H), 7.65 (d, J = 8.0 Hz, 1H), 7.54-7.50 (m, 2H), 7.39 (s, 1H), 7.32-7.26 (m, 2H), 7.12 (d, J = 8.4 Hz, 1H), 3.65 (s, 2H), 3.31 (s, 2H), 2.57 (d, J = 6.8 Hz, 4H), 1.14-1.07 (m, 9H).

$^{19}F$ NMR (400MHz, $CD_3OD$): δ (ppm) -105.78.

$^{13}C$ NMR (100 MHz, $CD_3OD$) δ (ppm) 169.97, 165.45, 162.97, 155.71, 151.05, 145.62, 143.77, 143.47, 139.62, 136.65, 134.79, 132.57, 130.40, 124.79, 123.96, 122.10, 119.99, 119.79, 118.47, 118.24, 111.59,55.35, 35.82, 17.32, 14.61, 11.65.

I-112

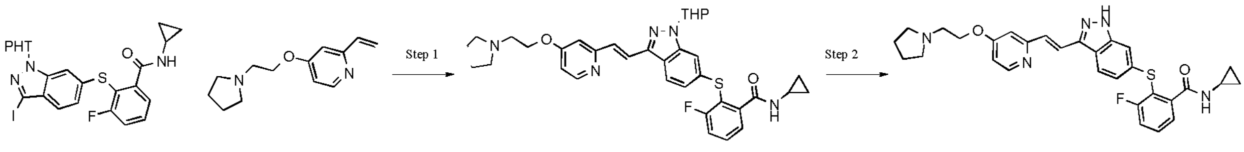


41a 23c 88a I-112

Step 1

At room temperature, N-cyclopropyl-3-fluoro-2-(3-iodo-1-tetrahydropyran-2-yl-indazol-6-yl)thioalkyl benzamide 41a (600 mg, 1.12 mmol) was dissolved in 1,4-dioxane (10 mL), followed by the addition of 4-(2-pyrrolidin-1-ethoxy)-2-vinylpyridine 23c (292 mg, 1.34 mmol), triethylamine (113 mg, 1.12 mmol) and Xphos Pd G3 (95 mg, 111.65 μmol). The reaction system was stirred at 95°C to react for 16 h under the protection of nitrogen. The reaction mixture was poured into water (50 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the column chromatography (DCM/MeOH = 1/0-20/1) to obtain a yellow solid product N-cyclopropyl-3-fluoro-2-[3-[(trans)-2-[4-(2-pyrrolidin-1-ethoxy)-2-pyridyl]vinyl]-1-tetrahydropyran-2-yl-indazol-6-yl]thioalkylbenzamide 88a (300 mg, 477.88 μmol, yield: 42.80 %).

The product was verified by LCMS.

For MS-ESI, the calculated value was $[M+H]^+$ 628.3, and the measurement was 628.2.

Step 2

At room temperature, N-cyclopropyl-3-fluoro-2-[3-[(trans)-2-[4-(2-pyrrolidin-1-ethoxy)-2-pyridyl]vinyl]-1-tetrahydropyran-2-yl-indazol-6-yl]thioalkyl benzamide 88a (400 mg, 637.18 μmol) was dissolved in dichloromethane (10 mL) and trifluoroacetic acid (3 mL). The reaction system was stirred at 25°C to react for 16 h. The reaction mixture was poured into water (20 mL), adjusted with the saturated sodium carbonate solution to the pH of 8, and extracted with dichloromethane (10 mL × 3), and the organic phases were combined, washed with saturated brine (30 mL), dried over

anhydrous sodium sulfate, and concentrated under reduced pressure to obtain a crude product. The crude product was purified by the high-pressure liquid chromatography to obtain a yellow solid product N-cyclopropyl-3-fluoro-2-({3-[(E)-2-{4-[2-(pyrrolidin-1-yl)ethoxy]pyridin-2-yl}vinyl]-1H-indazol-6-yl}thio)benzamide I-112 (55.88 mg, 102.79 μmol, yield: 16.13 %).

The product was verified by LCMS, F-NMR, H-NMR and C-NMR.

For MS-ESI, the calculated value was $[M+H]^+$ 544.2, and the measurement was 544.3

$^1$H NMR (400MHz, $CD_3OD$): δ (ppm) 8.34 (d, J=6.0 Hz, 1H), 7.95 (d, J= 8.4 Hz, 1H), 7.81-7.76 (m, 1H), 7.52-7.40 (m, 3H), 7.30-7.20 (m, 3H), 7.12 (dd, J=8.4, 1.2 Hz, 1H), 6.87 (dd, J=6.0, 2.4 Hz, 1H), 4.27 (t, J=5.6 Hz, 2H), 2.98 (t, J=5.6 Hz, 2H), 2.79-2.75 (m, 1H), 2.70 (s, 4H), 1.86 (s, 4H), 0.76-0.71 (m, 2H), 0.54-0.50 (m, 2H).

$^{13}$C NMR (100 MHz, $CD_3OD$) δ (ppm) 171.61, 167.38, 165.43, 162.95, 158.53, 151.47, 145.14, 143.46, 136.53, 132.54, 132.46, 130.67, 125.69, 124.82, 124.78, 124.07, 122.18, 121.23, 120.17, 119.98, 118.68, 118.45, 111.85, 110.58, 109.43, 67.98, 55.71, 24.40, 23.90, 17.54, 6.74.

$^{19}$F NMR (400MHz, $CD_3OD$): δ (ppm) -104.99, -105.01

I-113

Step 1

Step 2

Step 3

Step 4

Step 1

At room temperature, 6-bromopyridin-2-formaldehyde 89a (20 g, 107.52 mmol) was dissolved in N,N-dimethylformamide (200 mL), followed by the addition of $PdCl_2$(dppf) (3.9 g, 5.38 mmol), potassium vinyltrifluoroborate (21.6 g, 161.29 mmol) and triethylamine (32.6 g, 322.57 mmol). The reaction system was stirred at 90°C to react for 16 h under the protection of nitrogen. The reaction mixture was poured into water (1000 mL) and extracted with ethyl acetate (200 mL × 3), and the organic phases were combined, washed with saturated brine (500 mL), dried over anhydrous sodium sulfate, and concentrated under reduced pressure to obtain a crude product. The crude product was purified by the column chromatography (petroleum ether/ethyl acetate = 1/0-10/1) to obtain a yellow oily product 6-vinylpyridin-2-formaldehyde 89b (6 g, 45.06 mmol, yield: 41.91 %).

The product was verified by LCMS and H-NMR.

For MS-ESI, the calculated value was $[M+H]^+$ 134.1, and the measurement was 134.2.

$^1$H NMR (400MHz, $CDCl_3$): δ (ppm) 10.08 (s, 1H), 7.83 (d, *J* = 4.4 Hz, 2H), 7.58-7.54 (m, 1H), 6.90 (dd, *J* = 17.6, 10.8 Hz, 1H), 6.35 (d, *J*=17.2 Hz, 1H), 5.61 *(d, J*= 10.8 Hz, 1H).

Step 2

At room temperature, 6-vinylpyridin-2-formaldehyde 89b (6 g, 45.06 mmol) was dissolved in tetrahydrofuran (250 mL), followed by the addition of pyrrolidine (3.9 g, 54.08 mmol) and sodium triacetoxyborohydride (28.7 g, 135.19 mmol). The reaction system was stirred at 25°C to react for 16 h. The reaction mixture was poured into water (500 mL), adjusted with the aqueous solution of sodium carbonate to the pH of 8-9 and extracted with ethyl acetate (100 mL × 3), and the organic phases were combined, washed with saturated brine (300 mL), dried over anhydrous sodium sulfate, and concentrated under reduced pressure to obtain a crude product. The crude product was purified by the column chromatography (dichloromethane/methanol = 1/0-20/1) to obtain a yellow oily product 2-(pyrrolidin-1-ylmethyl)-6-vinylpyridine 89c (4 g, 21.25 mmol, yield: 47.15 %).

The product was verified by LCMS and H-NMR.

For MS-ESI, the calculated value was $[M+H]^+$ 189.1, and the measurement was 189.1.

$^1$H NMR (400MHz, $CDCl_3$): δ (ppm) 7.61 (t, *J* = 7.8 Hz, 1H), 7.31-7.24 (m, 2H), 6.83 (dd, *J* = 17.6, 10.8Hz, 1H), 6.16 *(d, J*= 17.6 Hz, 1H), 5.46 *(d, J*= 10.8 Hz, 1H), 3.81 (s, 2H), 2.63 (s, 4H), 1.85-1.78 (m, 4H).

Step 3

At room temperature, N-ethyl-3-fluoro-2-(3-iodo-1-tetrahydropyran-2-yl-indazol-6-yl)thioalkyl benzamide 80a (600 mg, 1.14 mmol) was dissolved in 1,4-dioxane (10 mL), followed by the addition of 2-(pyrrolidin-1-ylmethyl)-6-vinylpyridine 89c (258 mg, 1.37 mmol), tris(o-methylphenyl)phosphine (348 mg, 1.14 mmol), $Pd_2(dba)_3$ (523 mg, 571.02 μmol) and triethylamine (231 mg, 2.28 mmol). The reaction system was stirred at 90°C to react for 16 h under the protection of nitrogen. The reaction mixture was poured into water (30 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 1/0-20/1) to obtain a yellow solid product N-ethyl-3-fluoro-2-[3-[(trans)-2-[6-(pyrrolidin-1-ylmethyl)-2-pyridyl]vinyl]-1-tetrahydropyran-2-yl-indazol-6-yl]thioalkylbenzamide 89d (400 mg, 682.90 μmol, yield: 59.80 %).

The product was verified by LCMS.

For MS-ESI, the calculated value was $[M+H]^+$ 586.3, and the measurement was 586.1.

Step 4

At room temperature, N-ethyl-3-fluoro-2-[3-[(trans)-2-[6-(pyrrolidin-1-ylmethyl)-2-pyridyl]vinyl]-1-tetrahydropyran-2-yl-indazol-6-yl]thioalkyl benzamide 89d (400 mg, 682.90 μmol) was dissolved in dichloromethane (15 mL) and trifluoroacetic acid (5 mL). The reaction system was stirred at 25°C to react for 16 h. The reaction mixture was poured into water (30 mL), adjusted with the saturated aqueous solution of sodium carbonate to the pH of 8 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-ethyl-3-fluoro-2-({3-[(E)-2-{6-[(pyrrolidin-1-yl)methyl]pyridin-2-yl}vinyl]-1H-indazol-6-yl}thio)benzamide I-113 (99.99 mg, 199.34 μmol, yield: 30.03 %).

The product was verified by LCMS, F-NMR, H-NMR and C-NMR.

For MS-ESI, the calculated value was $[M+H]^+$ 502.2, and the measurement was 502.1.

$^1$H NMR (400MHz, $CD_3OD$): δ (ppm) 7.89 *(d, J=* 8.4 Hz, 1H), 7.78 (d, *J* = 16.4 Hz, 1H), 7.66 *(t, J=* 7.6 Hz, 1H), 7.46-7.40 (m, 3H), 7.30 (s, 1H), 7.23-7.16 (m, 3H), 7.03 (d, *J* = 8.4 Hz, 1H), 3.71 (s, 2H), 3.25-3.20 (m, 2H), 2.56 (s, 4H), 1.73 (s, 4H), 1.04 (t, *J* = 7.6 Hz, 3H).

$^{19}$F NMR (400MHz, $CD_3OD$): δ (ppm) -105.64

$^{13}$C NMR (100 MHz, $CD_3OD$) δ (ppm) 170.00, 165.45, 162.98, 159.48, 156.46, 145.61, 143.91, 143.50, 138.74, 136.63, 132.65, 132.57, 131.05, 125.28, 124.79, 124.75, 123.93, 123.29, 122,20, 121.48, 121.05, 120.00, 119.81, 118.48, 118.24, 111.62, 62.73, 55.17, 35.82, 24.30, 14.62.

I-114

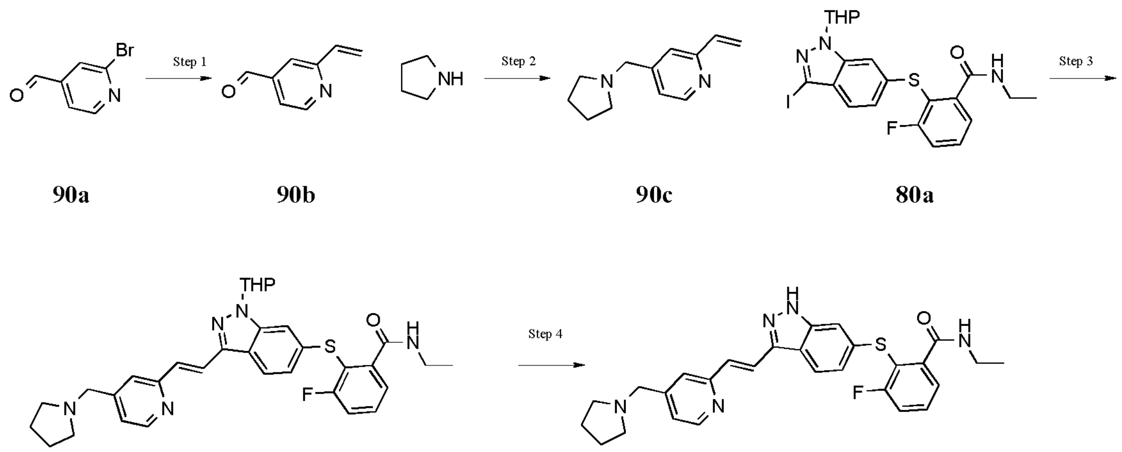


90a 90b 90c 80a

90d I-114

Step 1

At room temperature, 2-bromopyridin-4-formaldehyde 90a (20 g, 107.52 mmol) was dissolved in N,N-dimethylformamide (200 mL), followed by the addition of $PdCl_2(dppf)$ (3.9 g, 5.38 mmol), potassium vinyltrifluoroborate (21.6 g, 161.29 mmol) and triethylamine (32.6 g, 322.57 mmol). The reaction system was stirred at 90°C to react for 16 h under the

protection of nitrogen. The reaction mixture was poured into water (1000 mL) and extracted with ethyl acetate (200 mL × 3), and the organic phases were combined, washed with saturated brine (500 mL), dried over anhydrous sodium sulfate, and concentrated under reduced pressure to obtain a crude product. The crude product was purified by the column chromatography (petroleum ether/ethyl acetate = 1/0-10/1) to obtain a yellow oily product 2-vinylpyridin-4-formaldehyde 90b (300 mg, 2.25 mmol, yield: 41.91 %).

The product was verified by LCMS and H-NMR.

For MS-ESI, the calculated value was $[M+H]^+$134.1, and the measurement was 134.2.

$^1$H NMR (400MHz, $CDCl_3$): δ (ppm) 10.09 (s, 1H), 8.83 (d, *J*= 5.2 Hz, 1H), 7.74 (s, 1H), 7.57 (dd, *J* = 4.8, 1.2 Hz, 1H), 6.92 (dd, *J*= 17.6, 10.8 Hz, 1H), 6.33 (d, *J*= 17.2 Hz, 1H), 5.61 (d, *J* = 10.8 Hz, 1H).

Step 2

At room temperature, 2-vinylpyridin-4-formaldehyde 90b (6 g, 45.06 mmol) was dissolved in tetrahydrofuran (250 mL), followed by the addition of pyrrolidine (3.9 g, 54.08 mmol) and sodium triacetoxyborohydride (28.7 g, 135.19 mmol). The reaction system was stirred at 25°C to react for 16 h. The reaction mixture was poured into water (500 mL), adjusted with the saturated aqueous solution of sodium carbonate to the pH of 8-9 and extracted with ethyl acetate (100 mL × 3), and the organic phases were combined, washed with saturated brine (300 mL), dried over anhydrous sodium sulfate, and concentrated under reduced pressure to obtain a crude product. The crude product was purified by the column chromatography (dichloromethane/methanol = 1/0-20/1) to obtain a yellow oily product 4-(pyrrolidin-1-ylmethyl)-2-vinylpyridine 90c (4 g, 21.25 mmol, yield: 47.15 %).

The product was verified by LCMS and H-NMR.

For MS-ESI, the calculated value was $[M+H]^+$189.2, and the measurement was 189.1.

$^1$H NMR (400MHz, $CDCl_3$): δ (ppm) 8.49 (d, *J* = 5.0 Hz, 1H), 7.33 (s, 1H), 7.14 *(d, J*= 4.4 Hz, 1H), 6.81 (dd, *J* = 17.6, 10.8 Hz, 1H), 6.21 (d, *J*= 17.6 Hz, 1H), 5.47 (d, *J*= 10.8 Hz, 1H), 3.61 (s, 2H), 2.52 (s, 4H), 1.84-1.79 (m, 4H).

Step 3

At room temperature, N-ethyl-3-fluoro-2-(3-iodo-1-tetrahydropyran-2-yl-indazol-6-yl)thioalkyl benzamide 90c (500 mg, 951.70 μmol) was dissolved in 1,4-dioxane (10 mL), followed by the addition of 4-(pyrrolidin-1-ylmethyl)-2-vinylpyridine (215 mg, 1.14 mmol), Xphos Pd G3 (81 mg, 95.17 μmol) and triethylamine (289 mg, 2.86 mmol). The reaction system was stirred at 90°C to react for 16 h under the protection of nitrogen. The reaction mixture was poured into water (30 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 100/1-20/1) to obtain a yellow solid product N-ethyl-3-fluoro-2-[3-[(trans)-2-[4-(pyrrolidin-1-ylmethyl)-2-pyridyl]vinyl]-1-tetrahydropyran-2-yl-indazol-6-yl] thioalkylbenzamide 90d (400 mg, 682.90 μmol, yield: 71.76 %).

The product was verified by LCMS.

For MS-ESI, the calculated value was $[M+H]^+$586.3, and the measurement was 586.2.

Step 4

At room temperature, N-ethyl-3-fluoro-2-[3-[(trans)-2-[4-(pyrrolidin-1-ylmethyl)-2-pyridyl]vinyl]-1-tetrahydropyran-2-yl-indazol-6-yl]thioalkyl benzamide 90d (400 mg, 682.90 μmol) was dissolved in dichloromethane (15 mL) and trifluoroacetic acid (3 mL). The reaction system was stirred at 25°C to react for 16 h. The reaction mixture was poured into water (30 mL), adjusted with the saturated aqueous solution of sodium carbonate to the pH of 8-9 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-ethyl-3-fluoro-2-({3-[(E)-2-{4-[(pyrrolidin-1-yl)methyl]pyridin-2-yl}vinyl]-1H-indazol-6-yl}thio)benzamide I-114 (102.86 mg, 205.06 μmol, yield: 30.03 %).

The product was verified by LCMS, F-NMR, H-NMR and C-NMR.

For MS-ESI, the calculated value was $[M+H]^+$ 502.2, and the measurement was 502.1.

$^1$H NMR (400MHz, $CD_3OD$): δ (ppm) 8.48 (d, *J*= 5.2 Hz, 1H), 7.98 (d, *J*= 8.4 Hz, 1H), 7.84 (d, *J* = 16.8 Hz, 1H), 7.68 (s, 1H), 7.55-7.51 (m, 2H), 7.39 (s, 1H), 7.32-7.26 (m, 3H), 7.13 (d, *J*= 8.4 Hz, 1H), 3.72 (s, 2H), 3.31 (s, 2H), 2.61 (s, 4H), 1.85 (s, 4H), 1.13 (t, *J* = 7.2 Hz, 3H).

$^{13}$C NMR (100 MHz, $CD_3OD$) δ (ppm) 170.00, 165.45, 162.98, 156.98, 150.58, 150.28, 145.62, 136.68, 132.67, 132.58, 130.54, 125.57, 124.75, 124.17, 123.99, 123.43, 122.08, 121.11, 119.79, 118.48, 118.24, 111.61, 60.26, 55.16, 35.81, 24.30, 14.60.

$^{19}$F NMR (400MHz, $CD_3OD$): δ (ppm) -105.77

I-115

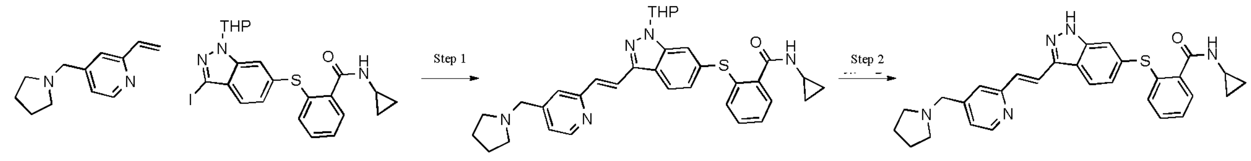


90c 91a 91b I-115

Step 1

At room temperature, N-cyclopropyl-2-(3-iodo-1-tetrahydropyran-2-yl-indazol-6-yl)thioalkyl benzamide 91a (500 mg, 962.65 μmol) was dissolved in 1,4-dioxane (5 mL), followed by the addition of 4-(pyrrolidin-1-ylmethyl)-2-vinylpyridine 90c (181 mg, 962.65 μmol), Xphos Pd G3 (81 mg, 96.27 μmol) and triethylamine (292 mg, 2.89 mmol). The reaction system was stirred at 90°C to react for 16 h under the protection of nitrogen. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (DCM/MeOH = 1/0-20/1) to obtain a yellow solid product N-cyclopropyl-2-[3-[(trans)-2-[4-(pyrrolidin-1-ylmethyl)-2-pyridyl]vinyl]-1-tetrahydropyran-2-ylindazol-6-yl]thioalkylbenzamide 91b (400 mg, 689.95 μmol, yield: 71.67 %).

The product was verified by LCMS.

For MS-ESI, the calculated value was $[M+H]^+$580.3, and the measurement was 580.2.

Step 2

At room temperature, N-cyclopropyl-2-[3-[(trans)-2-[4-(pyrrolidin-1-ylmethyl)-2-pyridyl]vinyl]-1-tetrahydropyran-2-ylindazol-6-yl]thioalkyl benzamide 91b (400 mg, 682.90 μmol) was dissolved in dichloromethane (15 mL) and trifluoroacetic acid (3 mL). The reaction system was stirred at 25°C to react for 16 h. The reaction mixture was poured into water (30 mL), adjusted with the saturated aqueous solution of sodium carbonate to the pH of 8-9 and extracted with dichloromethane(10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{4-[(pyrrolidin-1-yl)methyl]pyridin-2-yl}vinyl]-1H-indazol-6-yl}thio)benzamide I-115 (38.70 mg, 78.08 μmol, yield: 11.32 %).

The product was verified by LCMS, H-NMR and C-NMR.

For MS-ESI, the calculated value was $[M+H]^+$496.2, and the measurement was 496.1.

$^1$H NMR (400MHz, $CDCl_3$): δ (ppm) 8.56 (d, *J* = 4.8 Hz, 1H), 8.00 (d, *J*= 8.4 Hz, 1H), 7.92 (d, *J* = 16.4 Hz, 1H), 7.65-7.62 (m, 1H), 7.57 (d, *J* = 16.4 Hz, 1H), 7.51 (s, 2H), 7.31-7.29 (m, 2H), 7.25-7.22 (m, 1H), 7.18-7.16 (m, 2H), 6.53 (s, 1H), 3.67 (s, 2H), 2.85-2.82 (m, 1H), 2.57 (s, 4H), 1.83 (s, 4H), 0.83-0.78 (m, 2H), 0.51-0.47 (m, 2H). $^{13}$C NMR (100 MHz, $CD_3OD$& $CDCl_3$) δ (ppm) 172.59, 157.02, 150.52, 150.38, 138.45, 136.40, 135.35, 132.95, 131.88, 130.82, 129.26, 128.04, 126.76, 124.22, 123.42, 122.68, 121.84, 60.70, 55.45, 24.52, 24.03, 7.01, 0.65.

I-116

Step 1 Step 2 Step 3 Step 4

Step 5 Step 6

Step 1

At room temperature, 2-chloro-4-methylpyridine 92a (20 g, 156.78 mmol) was dissolved in tetrahydrofuran (200 mL); the mixture was cooled to -70°C, followed by the addition of lithium diisopropylamide (2 mol/L, 156.78 mL, 313.55 mmol). The reaction system was stirred at -70°C to react for 1 h. At the same temperature, ethylene oxide (3 mol/L, 104.52 mL, 313.55 mmol) was added, and the reaction system was stirred at -70°C to react for 3 h. The reaction mixture was poured into the saturated aqueous solution of ammonium chloride (300 mL) and extracted with ethyl acetate (300 mL × 3), and the organic phases were combined, washed with saturated brine (500 mL), dried over anhydrous sodium sulfate, and

concentrated under reduced pressure to obtain a crude product. The crude product was purified by the column chromatography (petroleum ether/ethyl acetate-petroleum ether/ethyl acetate = 1/0-1/1) to obtain a yellow oily product 3-(2-chloro-4-pyridyl)prop-1-ol 92b (14 g, 81.57 mmol, yield: 52.03 %).

The product was verified by LCMS and H-NMR.

For MS-ESI, the calculated value was $[M+H]^+$ 172.1, and the measurement was $[M+H]^+$172.0.

$^1$H NMR (400 MHz, $CDCl_3$): $\delta$ (ppm) 8.26 (d, *J* = 5.2 Hz, 1H), 7.19 (s, 1H), 7.08 (d, *J* = 4.8 Hz, 1H), 3.69 (t, *J* = 6.0 Hz, 2H), 2.73 (t, *J* = 7.6 Hz, 2H), 1.93-1.86 (m, 2H).

Step 2

At room temperature, 3-(2-chloro-4-pyridyl)prop-1-ol 92b (14 g, 81.57 mmol) was dissolved in dichloromethane (140 mL), followed by the addition of Dess-Martin periodinane (51.9 g, 122.36 mmol). The reaction system was stirred at 25°C to react for 16 h. The reaction mixture was poured into the aqueous solution of sodium bicarbonate (200 mL) and extracted with dichloromethane (100 mL × 3), and the organic phases were combined, washed with saturated brine (300 mL), dried over anhydrous sodium sulfate, and concentrated under reduced pressure to obtain a crude product. The crude product was purified by the column chromatography (petroleum ether/ethyl acetate-petroleum ether/ethyl acetate = 1/0-1/1) to obtain a yellow oily product 3-(2-chloro-4-pyridyl)propionaldehyde 92c (8 g, 47.17 mmol, yield: 57.82 %).

The product was verified by LCMS and H-NMR.

For MS-ESI, the calculated value was $[M+H]^+$ 170.0, and the measurement was $[M+H]^+$170.0.

$^1$H NMR (400MHz, $CDCl_3$): $\delta$ (ppm) 9.82 (s, 1H), 8.29 (d, *J* = 5.2 Hz, 1H), 7.19 (s, 1H), 7.08- 7.05 (m, 1H), 2.96-2.93 (m, 2H), 2.87-2.83 (m, 2H).

Step 3

At room temperature, 3-(2-chloro-4-pyridyl)propionaldehyde 92c (7.5 g, 44.22 mmol) was dissolved in tetrahydrofuran (75 mL), followed by the addition of pyrrolidine (4.7 g, 66.33 mmol) and acetic acidsodium borohydride (28.1 g, 132.66 mmol). The reaction system was stirred at 25°C to react for 16 h. The reaction mixture was poured into water (300 mL) and extracted with ethyl acetate (200 mL × 3), and the organic phases were combined, washed with saturated brine (300 mL), dried over anhydrous sodium sulfate, and concentrated under reduced pressure to obtain a crude product. The crude product was purified by the column chromatography (petroleum ether/ethyl acetate-petroleum ether/ethyl acetate = 1/0-1/1) to obtain a yellow oily liquid product 2-chloro-4-(3-pyrrolidin-1-yl propyl)pyridine 92d (6.9 g, 30.70 mmol, yield: 69.43 %).

The product was verified by LCMS and H-NMR.

For MS-ESI, the calculated value was $[M+H]^+$225.1, and the measurement was $[M+H]^+$225.1.

$^1$H NMR (400 MHz, $CDCl_3$) $\delta$(ppm) 8.23 *(d, J*= 8.0 Hz, 1H), 7.17 (s, 1H), 7.03 (d, *J* = 4.8 Hz, 1H), 2.67 (t, *J*= 7.6 Hz, 2H), 2.50-2.44 (m, 6H), 1.88-1.83 (m, 2H), 1.80-1.77 (m, 4H).

Step 4

At room temperature, 2-chloro-4-(3-pyrrolidin-1-ylpropyl)pyridine 92d (5.9 g, 26.25 mmol) was dissolved in 1,4-dioxane (60 mL), followed by the addition of 4,4,5,5-tetramethyl-2-vinyl-1,3,2-dioxa borane (6.1 g, 39.38 mmol), tetra(triphenylphosphino)palladium (738 mg, 638.22 μmol), and cesium carbonate (25.7 g, 78.76 mmol). The reaction system was stirred at 100°C under the protection of nitrogen to react for 16 h. The reaction mixture was poured into water (300 mL) and extracted with ethyl acetate (100 mL × 3), and the organic phases were combined, washed with saturated brine (300 mL), dried over anhydrous sodium sulfate, and concentrated under reduced pressure to obtain a crude product. The crude product was purified by the medium-pressure preparative chromatography to obtain a brown oily product 4-(3-pyrrolidin-1-ylpropyl)-2-vinylpyridine 92e (2 g, 9.25 mmol, yield: 35.22 %).

The product was verified by LCMS and H-NMR.

For MS-ESI, the calculated value was $[M+H]^+$217.2, and the measurement was $[M+H]^+$217.1.

$^1$H NMR (400 MHz, $CDCl_3$) $\delta$(ppm) 8.45 (d, *J* = 4.8 Hz, 1H), 7.05 (d, *J* = 4.4 Hz, 1H), 7.01-7.00 (m, 1H), 6.79 (dd, *J*= 17.2, 10.8 Hz, 1H), 6.19 (dd, *J*= 17.2, 1.2 Hz, 1H), 5.46 (dd, *J* = 10.8, 0.8 Hz, 1H), 2.68-2.64 (m, 2H), 2.49-2.43 (m, 6H), 1.90-1.82(m, 2H), 1.80-1.76 (m, 4H).

Step 5

At room temperature, 4-(3-pyrrolidin-1-ylpropyl)-2-vinylpyridine 92e (247 mg, 1.14 mmol) was dissolved in 1,4-dioxane (10 mL), followed by the addition of N-ethyl-3-fluoro-2-(3-iodo-1-tetrahydropyran-2-yl-indazol-6-yl)thioalkyl benzamide 80a (500 mg, 951.70 μmol), tris(o-methylphenyl)phosphine (290 mg, 951.70 μmol), palladium chloride

(85 mg, 475.85 μmol) and triethylamine (289 mg, 2.86 mmol). The reaction system was stirred at 90°C to react for 16 h. The reaction mixture was poured into water (100 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a yellow solid product N-ethyl-3-fluoro-2-[3-[(trans)-2-[4-(3-pyrrolidin-1-ylpropyl)-2-pyridyl]vinyl]-1-tetrahydropyran-2-yl-indazol-6-yl]thioalkylbenzamide 92f (170 mg, 276.97 μmol, yield: 29.10 %).

The product was verified by LCMS.

For MS-ESI, the calculated value was $[M+H]^+$614.3, and the measurement was 614.2

Step 6

At room temperature, N-ethyl-3-fluoro-2-[3-[(E)-2-[4-(3-pyrrolidin-1-ylpropyl)-2-pyridyl]vinyl]-1-tetrahydropyran-2-yl-indazol-6-yl]thioalkyl benzamide 92f (160 mg, 260.68 μmol) was dissolved in dichloromethane (3 mL), followed by the addition of trifluoroacetic acid (0.6 mL). The reaction system was stirred at 25°C to react for 16 h. The reaction mixture was poured into water (30 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the high-pressure preparative chromatography to obtain a white solid product N-ethyl-3-fluoro-2-({3-[(E)-2-{4-[3-(pyrrolidin-1-yl)propyl]pyridin-2-yl}vinyl]-1H-indazol-6-yl}thio)benzamide I-116.

The product was verified by LCMS, F-NMR, H-NMR and C-NMR.

For MS-ESI, the calculated value was $[M+H]^+$530.2, and the measurement was 530.1

$^1$H NMR (400MHz,$CD_3OD$): δ (ppm) 8.41 (d, *J*= 5.2 Hz, 1H), 7.98 (d, *J* = 8.8 Hz, 1H), 7.83 (d, *J* = 16.8 Hz, 1H), 7.57-7.49 (m, 3H), 7.39 (s, 1H), 7.32-7.26 (m, 2H), 7.18 (d, *J* = 5.2 Hz, 1H), 7.13 (dd, *J*= 8.8, 1.2 Hz, 1H), 3.34-3.31 (m, 2H), 2.73 (t, *J* = 7.6 Hz, 2H), 2.60-2.54 (m, 6H), 1.97-1.89 (m, 2H), 1.84-1.80 (m, 4H), 1.13 (t, *J* = 7.2 Hz, 3H).

$^{19}$F NMR (400MHz,$CD_3OD$): δ (ppm) -105.78

$^{13}$C NMR (400MHz,$CD_3OD$): δ (ppm) 169.95, 165.45, 162.97, 156.77, 154.21, 150.09, 145.64, 143.75, 143.48, 136.65, 132.66, 132.58, 130.66, 125.35, 124.75, 123.96, 123.36, 122.11, 121.11, 119.99, 119.79, 118.47, 118.23, 111.60, 56.82, 55.00, 35.81, 34.04, 30.17, 24.18, 14.62

I-117

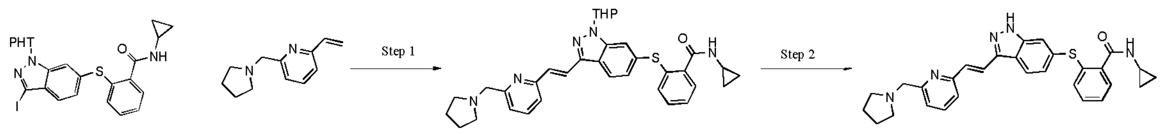


91a 89c 93a I-117

Step 1

At room temperature, 2-(pyrrolidin-1-ylmethyl)-6-vinylpyridine 89c(217 mg, 1.16 mmol) was dissolved in 1,4-dioxane (10 mL), followed by the addition of N-cyclopropyl-2-(3-iodo-1-tetrahydropyran-2-yl-indazol-6-yl)thioalkyl benzamide 91a (500 mg, 962.65 μmol), Xphos Pd G3 (81 mg, 96.27 μmol) and triethylamine (292 mg, 2.89 mmol). The reaction system was stirred at 90°C to react for 16 h under the protection of nitrogen. The reaction mixture was poured into water (100 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the preparative plate (dichloromethane/methanol = 10/1) to obtain a yellow solid product N-cyclopropyl-2-[3-[(trans)-2-[6-(pyrrolidin-1-ylmethyl)-2-pyridyl]vinyl]-1-tetrahydropyran-2-ylindazol-6-yl] thioalkylbenzamide 93a (350 mg, 603.70 μmol, yield: 62.71 %).

The product was verified by LCMS.

For MS-ESI, the calculated value was $[M+H]^+$ 580.3, and the measurement was 580.1.

Step 2

At room temperature, N-cyclopropyl-2-[3-[(trans)-2-[6-(pyrrolidin-1-ylmethyl)-2-pyridyl]vinyl]-1-tetrahydropyran-2-ylindazol-6-yl]thioalkyl benzamide 93a (330 mg, 569.21 μmol) was dissolved in dichloromethane (8 mL), followed by the addition of trifluoroacetic acid (2 mL). The reaction system was stirred at 25°C to react for 16 h. The reaction mixture was poured into water (50 mL), adjusted with solid sodium carbonate to the pH value of 10 and extracted with dichloromethane (50 mL × 3), and the organic phases were combined, washed with saturated brine (30 mL), dried over

anhydrous sodium sulfate, and concentrated under reduced pressure to obtain a crude product. The crude product was purified by the high-pressure liquid chromatography to obtain a yellow solid product N-cyclopropyl-2-({3-[(E)-2-{6-[(pyrrolidin-1-yl)methyl]pyridin-2-yl}vinyl]-1H-indazol-6-yl}thio)benzamide I-117 (92.73 mg, 187.09 μmol, yield: 32.87 %).

The product was verified by LCMS, H-NMR and C-NMR.

For MS-ESI, the calculated value was $[M+H]^+$ 496.2, and the measurement was 496.1.

$^1H$ NMR (400MHz, $CD_3OD$): δ (ppm) 8.08 (d, *J*= 8.4 Hz, 1H), 7.93 (d, *J* = 16.8 Hz, 1H), 7.79 (t, *J* =7.6Hz, 1H), 7.60-7.56 (m, 3H), 7.46-7.44 (m, 1H), 7.37-7.30 (m, 3H), 7.27-7.25 (m, 1H), 7.19 (d, J = 8.4 Hz, 1H), 3.85 (s, 2H), 2.80-2.75 (m, 1H), 2.70 (s, 4H), 1.85 (s, 4H), 0.77-0.72 (m, 2H), 0.55-0.51 (m, 2H).

$^{13}C$ NMR (400MHz, $CD_3OD$): δ (ppm) 172.56, 159.27, 156.54, 138.87, 138.38, 136.40, 135.31, 132.94, 131.90, 131.35, 129.28, 128.05, 126.71, 125.52, 123.36, 122.86, 121.75, 121.47, 115.51, 62.94, 55.44, 24.53, 24.05, 7.07

I-118

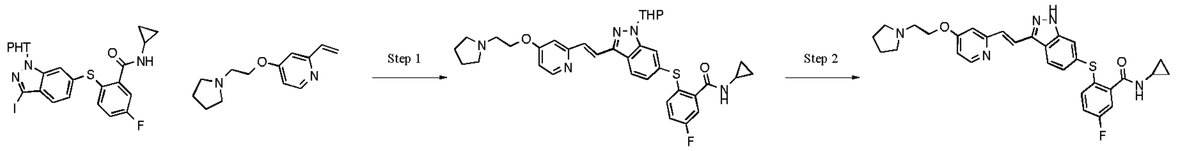


42a 23c 94a I-118

Step 1

At room temperature, N-cyclopropyl-5-fluoro-2-(3-iodo-1-tetrahydropyran-2-yl-indazol-6-yl)thioalkyl benzamide 42a (500 mg, 930.43 μmol) was dissolved in 1,4-dioxane (3 mL), followed by the addition of 4-(2-pyrrolidin-1-ethoxy)-2-vinylpyridine 23c (244 mg, 1.12 mmol), Xphos Pd G3 (79 mg, 93.04 μmol) and triethylamine (283 mg, 2.79 mmol). The reaction system was stirred at 90°C to react for 16 h. The reaction mixture was poured into water (50 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the preparative thin-layer chromatography (dichloroethane /methanol = 10/1) to obtain a yellow oily product N-cyclopropyl-5-fluoro-2-[3-[(trans)-2-[4-(2-pyrrolidin-1-ethoxy)-2-pyridyl]vinyl]-1-tetrahydropyran-2-yl-indazol-6-yl]thioalkylbenzamide 94a (300 mg, 477.88 μmol, yield: 51.36 %). The product was verified by LCMS.

For MS-ESI, the calculated value was $[M+H]^+$628.3, and the measurement was 628.3.

Step 2

At room temperature, N-cyclopropyl-5-fluoro-2-[3-[(trans)-2-[4-(2-pyrrolidin-1-ethoxy)-2-pyridyl]vinyl]-1-tetrahydropyran-2-yl-indazol-6-yl]thioalkyl benzamide 94a (300 mg, 477.88 μmol) was dissolved in dichloromethane (12 mL), followed by the addition of trifluoroacetic acid (3 mL). The reaction system was stirred at 25°C to react for 16 h. The reaction mixture was poured into water (50 mL), adjusted with solid sodium carbonate to the pH value of 10 and extracted with dichloromethane (10 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-5-fluoro-2-({3-[(E)-2-{4-[2-(pyrrolidin-1-yl)ethoxy]pyridin-2-yl}vinyl]-1H-indazol-6-yl}thio)benzamide I-118 (50.89 mg, 93.61 μmol, yield: 19.59 %).

The product was verified by LCMS, F-NMR, H-NMR and C-NMR.

For MS-ESI, the calculated value was $[M+H]^+$ 544.2, and the measurement was 544.2.

$^1H$ NMR (400MHz, $CD_3OD$): δ (ppm) 8.34 (d, *J*= 5.6 Hz, 1H), 8.03 (d, *J* = 8.4 Hz, 1H), 7.83 (d, *J* = 16.4 Hz, 1H), 7.53-7.45 (m, 2H), 7.37 (dd, *J* = 8.8, 5.6 Hz, 1H), 7.27-7.21 (m, 2H), 7.18-7.13 (m, 2H), 6.89 (dd, *J* = 6.0, 2.4 Hz, 1H), 4.28 (t, *J* = 5.4 Hz, 1H), 2.96 (t, *J* = 5.6 Hz, 1H), 2.79-2.73 (m, 1H), 2.72-2.65(m, 4H), 1.87-1.80(m, 4H), 0.76-0.70(m, 2H), 0.54-0.48 (m, 2H).

$^{19}F$ NMR (400MHz,$CD_3OD$): δ (ppm)-115.49

$^{13}C$ NMR (100MHz, $CD_3OD$): δ (ppm) 171.20, 167.52, 164.48, 162.01, 158.51, 151.45, 143.72, 143.57, 142.19, 136.70, 136.62, 136.31, 130.65, 130.47, 125.62, 125.52, 118.81, 118.59, 116.15, 113.93, 110.58, 109.48, 68.05, 55.66, 55.56, 24.27, 23.78, 6.44.

I-119

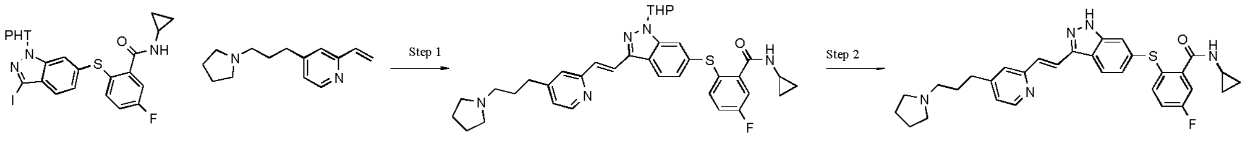


42a 92e 95a I-119

Step 1

At room temperature, N-cyclopropyl-5-fluoro-2-(3-iodo-1-tetrahydropyran-2-yl-indazol-6-yl)thioalkyl benzamide 42a (500 mg, 930.43 μmol) was dissolved in 1,4-dioxane (10 mL), followed by the addition of 4-(3-pyrrolidin-1-ylpropyl)-2-vinylpyridine 92e (201 mg, 930.43 μmol), triethylamine (282 mg, 2.79 mmol) and Xphos Pd G3 (79 mg, 93.04 μmol). The reaction system was stirred at 90°C to react for 16 h under the protection of nitrogen. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 100/1-10/1) to obtain a yellow solid product N-cyclopropyl-5-fluoro-2-[3-[(trans)-2-[4-(3-pyrrolidin-1-ylpropyl)-2-pyridyl]vinyl]-1-tetrahydropyran-2-yl-indazol-6-yl]thioalkylbenzamide 95a (300 mg, 479.39 μmol, yield: 51.52 %).

The product was verified by LCMS.

For MS-ESI, the calculated value was $[M+H]^+$ 626.3, and the measurement was 626.3.

Step 2

At room temperature, N-cyclopropyl-5-fluoro-2-[3-[(trans)-2-[4-(3-pyrrolidin-1-ylpropyl)-2-pyridyl]vinyl]-1-tetrahydropyran-2-yl-indazol-6-yl]thioalkyl benzamide 95a (300 mg, 479.39 μmol) was dissolved in dichloromethane (10 mL), followed by the addition of trifluoroacetic acid (3 mL). The reaction system was stirred at 25°C to react for 16 h. The reaction mixture was poured into water (30 mL), adjusted with the saturated sodium carbonate solution to the pH of 8 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-5-fluoro-2-({3-[(E)-2-{4-[3-(pyrrolidin-1-yl)propyl]pyridin-2-yl}vinyl]-1H-indazol-6-yl}thio)benzamide I-119 (52.65 mg, 97.20 μmol, yield: 20.28 %).

The product was verified by LCMS, F-NMR, H-NMR and C-NMR.

For MS-ESI, the calculated value was $[M+H]^+$ 542.2, and the measurement was 542.1.

$^1H$ NMR (400MHz, $CD_3OD$): δ (ppm) 8.42 (d, *J*= 5.2 Hz, 1H), 8.05 (d, *J* = 8.8 Hz, 1H), 7.85 (d, *J* = 16.4 Hz, 1H), 7.58-7.49 (m, 3H), 7.38 (dd, *J* = 8.8, 5.2 Hz, 1H), 7.26-7.14 (m, 4H), 2.79-2.72 (m, 3H), 2.58-2.53 (m, 6H), 1.97-1.89 (m, 2H), 1.84-1.80 (m, 4H), 0.76-0.71 (m, 2H), 0.53-0.49 (m, 2H).

$^{13}C$ NMR (100 MHz, $CD_3OD$) δ (ppm) 171.17, 164.47, 162.00, 156.78, 154.30, 150.11, 143.82, 143.58, 142.16, 136.67, 136.59, 136.30, 130.73, 130.51, 125.63, 125.29, 124.10, 123.41, 122.53, 121.49, 118.80, 118.58, 116.38, 116.15, 113.97, 56.87, 55.01, 34.08, 30.26, 24.19, 23.79, 6.45.

$^{19}F$ NMR (400MHz, $CD_3OD$): δ (ppm) -115.50

I-120

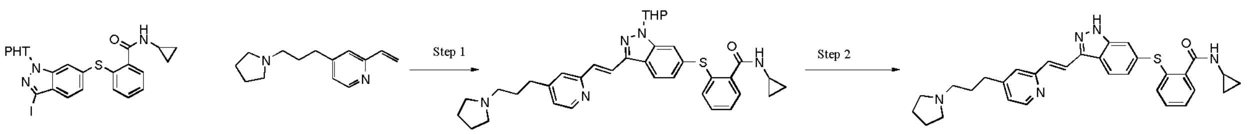


93a 92e 96a I-120

Step 1

At room temperature, N-cyclopropyl-2-(3-iodo-1-tetrahydropyran-2-yl-indazol-6-yl)thioalkyl benzamide 93a (400 mg, 770.12 μmol) was dissolved in 1,4-dioxane (6 mL), followed by the addition of 4-(3-pyrrolidin-1-ylpropyl)-2-vinylpyridine 92e (200 mg, 924.15 μmol), tris(o-methylphenyl)phosphine (234 mg, 770.12 μmol), dichloropalladium (II) (68 mg, 385.06 μmol) and triethylamine (234 mg, 2.31 mmol). The reaction system was stirred at 90°C under the protection of nitrogen to react for 16 h. The reaction mixture was poured into water (100 mL) and extracted with ethyl acetate (30 mL ×

3), and the organic phases were combined, washed with saturated brine (100 mL), dried over anhydrous sodium sulfate, and concentrated under reduced pressure to obtain a crude product. The crude product was purified by the preparative thin-layer chromatography (dichloromethane/methanol = 10/1) to obtain a yellow oily product N-cyclopropyl-2-[3-[(trans)-2-[4-(3-pyrrolidin-1-ylpropyl)-2-pyridyl]vinyl]-1-tetrahydropyran-2-yl-indazol-6-yl]thioalkylbenzamide 96a (160 mg, 263.24 μmol, yield: 34.18 %).

The product was verified by LCMS.

For MS-ESI, the calculated value was $[M+H]^+$ 608.3, and the measurement was 608.2.

Step 2

At room temperature, N-cyclopropyl-2-[3-[(trans)-2-[4-(3-pyrrolidin-1-ylpropyl)-2-pyridyl]vinyl]-1-tetrahydropyran-2-yl-indazol-6-yl]thioalkyl benzamide 96a (140 mg, 230.34 μmol) was dissolved in dichloromethane (12 mL), followed by the addition of trifluoroacetic acid (3 mL). The reaction system was stirred at 25°C to react for 16 h. The reaction mixture was poured into water (50 mL), adjusted with the saturated aqueous solution of sodium carbonate to a pH value of 10 and extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure preparative chromatography to obtain a yellow solid product N-cyclopropyl-2-({3-[(E)-2-{4-[3-(pyrrolidin-1-yl)propyl]pyridin-2-yl}vinyl]-1H-indazol-6-yl}thio)benzamide I-120 (53.95 mg, 103.02 μmol, yield: 44.73 %).

The product was verified by LCMS and H-NMR.

For MS-ESI, the calculated value was $[M+H]^+$ 524.2, and the measurement was 524.2.

The product was verified by LCMS, H-NMR and C-NMR.

$^1H$ NMR (400MHz, $CD_3OD$): δ (ppm) 8.41 (d, *J*= 4.8 Hz, 1H), 8.05 (d, *J* = 8.4 Hz, 1H), 7.85 (d, *J* = 16.4 Hz, 1H), 7.56-7.51 (m, 3H), 7.46-7.44 (m, 1H), 7.35-7.29 (m, 2H), 7.25-7.23 (m, 1H), 7.19-7.16 (m, 1H), 2.80-2.75 (m, 1H), 2.72 (t, *J*= 7.6 Hz, 2H), 2.59-2.53 (m, 6H), 1.96-1.88 (m, 2H), 1.81 (s, 4H), 0.77-0.72 (m, 2H), 0.56-0.52 (m, 2H). $^{13}C$ NMR (100MHz, $CD_3OD$): δ (ppm) 172.68, 156.78, 154.21, 150.13, 143.82, 143.61, 139.26, 135.90, 135.73, 133.22, 131.69, 130.73, 129.14, 128.21, 126.41, 125.31, 124.09, 123.42, 122.53, 121.62, 114.90, 56.81, 55.02, 34.03, 30.13, 24.18, 23.81, 6.50

I-121

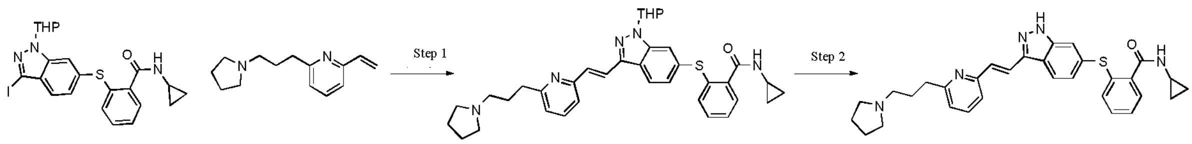


91a 97a 97b I-121

Step 1

At room temperature, N-cyclopropyl-2-(3-iodo-1-tetrahydropyran-2-yl-indazol-6-yl)thioalkyl benzamide 91a (400 mg, 770.12 μmol) was dissolved in 1,4-dioxane (5 mL), followed by the addition of 2-(3-pyrrolidin-1-ylpropyl)-6-vinylpyridine 97a (250 mg, 1.16 mmol), triethylamine (234 mg, 2.31 mmol) and Xphos Pd G3 (65 mg, 77.01 μmol). The reaction system was stirred at 90°C to react for 16 h under the protection of nitrogen. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 100/1-10/1) to obtain a yellow solid product N-cyclopropyl-2-[3-[(trans)-2-[6-(3-pyrrolidin-1-ylpropyl)-2-pyridyl]vinyl]-1-tetrahydropyran-2-yl-indazol-6-yl]thioalkylbenzamide 97b (300 mg, 493.58 μmol, yield: 64.09 %).

The product was verified by LCMS.

For MS-ESI, the calculated value was $[M+H]^+$ 608.3, and the measurement was 608.3.

Step 2

At room temperature, N-cyclopropyl-2-[3-[(trans)-2-[6-(3-pyrrolidin-1-ylpropyl)-2-pyridyl]vinyl]-1-tetrahydropyran-2-yl-indazol-6-yl]thioalkyl benzamide 97b (300 mg, 493.58 μmol) was dissolved in dichloromethane (12 mL), followed by the addition of trifluoroacetic acid (4 mL). The reaction system was stirred at 25°C to react for 16 h. The reaction mixture was poured into water (30 mL), adjusted with saturated sodium carbonate to the pH of 8 and extracted with dichloromethane (10 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{6-[3-(pyrrolidin-1-yl)propyl]pyridin-2-yl}vinyl]-1H-indazol-6-yl}thio)benzamide I-121 (50.86 mg, 97.12 μmol, yield: 19.68 %).

The product was verified by LCMS, H-NMR and C-NMR.

For MS-ESI, the calculated value was $[M+H]^+$ 524.2, and the measurement was 524.0.

$^1H$ NMR (400MHz, $CD_3OD$): δ (ppm) 8.08 (d, *J*= 8.8 Hz, 1H), 7.86 (d, *J* = 16.4 Hz, 1H), 7.73 *(t, J*= 7.6 Hz, 1H), 7.59-7.51 (m, 3H), 7.46-7.44 (m, 1H), 7.37-7.30 (m, 2H), 7.26 (d, *J* = 7.2 Hz, 1H), 7.21-7.17 (m, 2H), 2.86 (t, *J*= 7.6 Hz, 2H), 2.64 (s, 6H), 2.07-1.99 (m, 2H), 1.83 (s, 4H), 0.77-0.72 (m, 2H), 0.55-0.51 (m, 2H).

$^{13}C$ NMR (100 MHz, $CD_3OD$) δ (ppm) 172.70, 162.71, 156.48, 143.98, 143.65, 139.27, 138.80, 135.89, 135.74, 133.24, 131.69, 131.26, 129.14, 128.22, 126.37, 125.14, 122.97, 122.61, 121.51, 120.42, 114.89, 57.07, 55.03, 36.84, 29.77, 24.17, 23.81, 6.50.

I-122

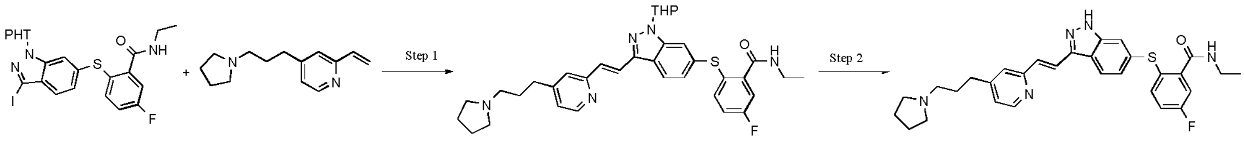


75b 92e 98a I-122

Step 1

At room temperature, N-ethyl-5-fluoro-2-(3-iodo-1-tetrahydropyran-2-yl-indazol-6-yl)thioalkyl benzamide 75b (300 mg, 571.02 μmol) was dissolved in dioxane (10 mL), followed by the addition of 4-(3-pyrrolidin-1-ylpropyl)-2-vinylpyridine 92e (148 mg, 685.22 μmol), (2-dicyclohexylphosphino-2',4',6'-triisopropyl-1,1'-biphenyl)(2'-amino-1,1'-biphen-2-yl)palladium(II) methanesulfonate (48 mg, 57.10 μmol) and triethylamine (289 mg, 2.86 mmol). The reaction system was stirred at 90°C under the protection of nitrogen to react for 16 h. The reaction mixture was poured into water (50 mL) and extracted with ethyl acetate (50 mL × 3), and the organic phases were combined, washed with saturated brine (30 mL), dried over anhydrous sodium sulfate, and concentrated under reduced pressure to obtain a crude product. The crude product was subjected to the medium-pressure chromatography to obtain a yellow solid product N-ethyl-5-fluoro-2-[3-[(trans)-2-[4-(3-pyrrolidin-1-ylpropyl)-2-pyridyl]vinyl]-1-tetrahydropyran-2-yl-indazol-6-yl]thioalkylbenzamide 98a (300 mg, 488.77 μmol, yield: 85.60 %). The product was verified by LCMS.

For MS-ESI, the calculated value was $[M+H]^+$ 614.3, and the measurement was 614.1.

Step 2

At room temperature, N-ethyl-5-fluoro-2-[3-[(trans)-2-[4-(3-pyrrolidin-1-ylpropyl)-2-pyridyl]vinyl]-1-tetrahydropyran-2-yl-indazol-6-yl]thioalkyl benzamide 98a (250 mg, 407.31 μmol) was dissolved in dichloromethane (12 mL) and trifluoroacetic acid (4 mL); the reaction system was stirred at 25°C to react for 16 h. The reaction mixture was poured into water (50 mL), adjusted with the saturated sodium carbonate solution to the pH of 8 and extracted with dichloromethane (50 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-ethyl-5-fluoro-2-({3-[(E)-2-{4-[3-(pyrrolidin-1-yl)propyl]pyridin-2-yl}vinyl]-1H-indazol-6-yl}thio)benzamide I-122 (48.76 mg, 92.06 μmol, yield: 22.60 %).

The product was verified by LCMS, F-NMR, H-NMR and C-NMR.

For MS-ESI, the calculated value was $[M+H]^+$ 530.2, and the measurement was 530.3.

$^1H$ NMR (400MHz, $CD_3OD$): δ (ppm) 8.40 (d, *J* = 4.8 Hz, 1H), 8.03 (d, *J*= 8.8 Hz, 1H), 7.84 (d, *J* = 16.4 Hz, 1H), 7.55-7.50 (m, 3H), 7.35 (dd, *J* = 8.8, 5.6 Hz, 1H), 7.25 (dd, *J* = 8.8, 3.2 Hz, 1H), 7.17-7.12 (m, 3H), 3.35-3.29 (m, 2H), 2.71 (t, *J* = 7.6 Hz, 2H), 2.57-2.50 (m, 6H), 1.95-1.87 (m, 2H), 1.84-1.76 (m, 4H), 1.14 *(t, J*= 7.2 Hz, 3H).

$^{13}C$ NMR (100 MHz, $CD_3OD$) δ (ppm) 169.54, 164.44, 161.97, 156.77, 154.27, 150.11, 143.81, 143.59, 142.36, 142.29, 136.54, 136.46, 136.26, 130.73, 130.60, 130.56, 125.78, 125.29, 123.41, 122.53, 121.51, 118.73, 118.51, 116.31, 116.08, 114.11, 56.86, 55.01, 35.83, 34.07, 30.24, 24.19, 14.60.

$^{19}F$ NMR (400 MHz, $CD_3OD$) δ (ppm) -115.62.

I-123

Step 1
Step 2

Step 1

At room temperature, N-cyclopropyl-3-fluoro-2-(3-iodo-1-tetrahydropyran-2-yl-indazol-6-yl)thioalkyl benzamide 41a (600 mg, 1.12 mmol) was dissolved in 1,4-dioxane (10 mL), followed by the addition of 4-(3-pyrrolidin-1-ylpropyl)-2-vinylpyridine 92e (290 mg, 1.34 mmol), dichloropalladium (II) (99 mg, 558.26 μmol), triethylamine (339 mg, 3.35 mmol) and tris(o-methylphenyl)phosphine (340 mg, 1.12 mmol). The reaction system was stirred at 90°C to react for 16 h. The reaction mixture was poured into water (50 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the medium-pressure preparative chromatography to obtain a yellow solid product N-cyclopropyl-3-fluoro-2-[3-[(trans)-2-[4-(3-pyrrolidin-1-ylpropyl)-2-pyridyl]vinyl]-1-tetrahydropyran-2-yl-indazol-6-yl]thioalkylbenzamide 99a (400 mg, 639.19 μmol, yield: 57.25 %).

The product was verified by LCMS.

For MS-ESI, the calculated value was $[M+H]^+$ 626.3, and the measurement was 626.1.

Step 2

At room temperature, N-cyclopropyl-3-fluoro-2-[3-[(trans)-2-[4-(3-pyrrolidin-1-ylpropyl)-2-pyridyl]vinyl]-1-tetrahydropyran-2-yl-indazol-6-yl]thioalkyl benzamide 99a (400 mg, 639.19 μmol) was dissolved in dichloromethane (10 mL) and trifluoroacetic acid (3 mL). The reaction system was stirred at 25°C to react for 16 h. The reaction mixture was poured into water (50 mL), adjusted with the saturated aqueous solution of sodium carbonate to the pH of 8-9 and extracted with dichloromethane (30 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-3-fluoro-2-({3-(E)-2-{4-[3-(pyrrolidin-1-yl)propyl]pyridin-2-yl}vinyl]-1H-indazol-6-yl}thio)benzamide I-123 (35.08 mg, 64.76 μmol, yield: 10.13 %).

The product was verified by LCMS, F-NMR, H-NMR and C-NMR.

For MS-ESI, the calculated value was $[M+H]^+$ 542.2, and the measurement was 542.1.

$^1$HNMR(400MHz, $CD_3OD$): δ (ppm) 8.42 (d, *J* = 4.8 Hz, 1H), 7.98 (d, *J* = 8.4 Hz, 1H), 7.83 (d, *J* = 16.4 Hz, 1H), 7.58 (s, 1H), 7.55-7.50 (m, 2H), 7.39 (s, 1H), 7.31-7.27 (m, 2H), 7.19 (d, *J* = 5.2 Hz, 1H), 7.12 (dd, *J* = 8.8, 1.6 Hz, 1H), 2.79-2.63 (m, 9H), 2.00-1.92 (m, 2H), 1.87-1.84 (m, 4H), 0.75-0.70 (m, 2H), 0.52-0.48 (m, 2H).

$^{13}$C NMR(100MHz, $CD_3OD$): δ (ppm) 171.63, 165.43, 162.95, 156.85, 153.86, 150.18, 145.15, 143.77, 143.48, 136.56, 132.56, 132.48, 130.72, 125.49, 124.06, 123.32, 122.19, 121.20, 119.96, 118.68, 118.45, 111.84, 56.82, 55.18, 34.11, 29.98, 24.28, 23.89, 6.73.

$^{19}$F NMR(400MHz, $CD_3OD$): δ (ppm) -105.03.

I-124

Step 1
Step 2
Step 3
Step 4
Step 5
Step 6

Step 1

Under the protection of nitrogen at room temperature, 2-chloro-6-methylpyridine 100a (50 g, 391.94 mmol) was dissolved in tetrahydrofuran (500 mL); the mixture was cooled to -78°C, followed by the addition of lithium diisopropylamide (2 M, 392 mL, 783.88 mmol) The reaction system was stirred at -78°C to react for 2 h. Then, ethylene oxide (3 M, 261 mL, 783.88 mmol) was slowly added at -78°C, and the reaction system was stirred at - 78°C to react for 2 h. The reaction mixture was poured into the saturated ammonium chloride solution (2000 mL) and extracted with ethyl acetate (1000 mL × 3), and the organic phases were combined, washed with saturated brine (1000 mL), dried over anhydrous sodium sulfate,

and concentrated under reduced pressure to obtain a crude product. The crude product was purified by the column chromatography (petroleum ether:ethyl acetate = 1/0-0/1) to obtain a yellow solid product 3-(6-chloro-2-pyridyl)prop-1-ol 100b (16 g, 93.23 mmol, yield: 23.79 %).

The product was verified by LCMS and H-NMR.

For MS-ESI, the calculated value was $[M+H]^+$ 172.1, and the measurement was 172.2.

$^1$H NMR (400 MHz,$CDCl_3$): δ (ppm) 7.57 (t, *J* = 7.6 Hz, 1H), 7.17 (d, *J* = 8.0 Hz, 1H), 7.11 (d, *J* = 7.6 Hz, 1H), 3.70 (s, 2H), 2.90 (t, *J* = 7.6 Hz, 2H), 2.71 (s, 1H), 2.02-1.95 (m, 2H).

Step 2

At room temperature, 3-(6-chloro-2-pyridyl)prop-1-ol 100b (11 g, 64.09 mmol) was dissolved in dichloromethane (200 mL), followed by the addition of Dess-Martin periodinane (40.8 g, 96.14 mmol). The reaction system was stirred at 25°C to react for 16 h. The reaction mixture was poured into the saturated aqueous solution of sodium carbonate (300 mL) and extracted with dichloromethane (100 mL × 3), and the organic phases were combined, dried over anhydrous sodium sulfate, and concentrated under reduced pressure to obtain a crude product, which was purified by the column chromatography (petroleum ether:ethyl acetate = 1/0-10/1) to obtain a yellow solid product 3-(6-chloro-2-pyridyl) propionaldehyde 100c (2.6 g, 15.33 mmol, yield: 23.92 %).

The product was verified by LCMS and H-NMR.

For MS-ESI, the calculated value was $[M+H]^+$ 170.0, and the measurement was 170.0.

$^1$H NMR (400MHz, $CDCl_3$): δ(ppm) 9.85 (s, 1H), 7.58-7.55 (m, 1H), 7.18-7.13 (m, 2H), 3.10 (t, *J* = 7.2 Hz, 2H), 2.98 (t, *J*= 6.8 Hz, 2H).

Step 3

At room temperature, 3-(6-chloro-2-pyridyl)propionaldehyde 100c (2.7 g, 15.92 mmol) was dissolved in tetrahydrofuran (30 mL), followed by the addition of pyrrolidine (1.7 g, 23.88 mmol) and sodium triacetoxyborohydride (10.1 g, 47.76 mmol). The reaction system was stirred at 25°C to react for 16 h. The reaction mixture was poured into water (300 mL) and extracted with ethyl acetate (100 mL × 3), and the organic phases were combined, washed with saturated brine (300 mL), dried over anhydrous sodium sulfate, and concentrated under reduced pressure to obtain a crude product. The crude product was purified by the column chromatography (petroleum ether:ethyl acetate = 20/1-1/1) to obtain a yellow solid product 2-chloro-6-(3-pyrrolidin-1-ylpropyl)pyridine 100d (2 g, 8.90 mmol, yield: 55.91 %).

The product was verified by LCMS and H-NMR.

For MS-ESI, the calculated value was $[M+H]^+$225.1, and the measurement was 225.0.

$^1$H NMR (400MHz, $CDCl_3$): δ (ppm) 7.57-7.53 (m, 1H), 7.16-7.12 (m, 1H), 7.09 (d, J = 7.6 Hz, 1H), 2.81 (t, J = 7.6 Hz, 2H), 2.61-2.48 (m, 6H), 1.99-1.90 (m, 2H), 1.80-1.70 (m, 4H).

Step 4

At room temperature, 2-chloro-6-(3-pyrrolidin-1-ylpropyl)pyridine 100d (2.7 g, 12.01 mmol) was dissolved in dioxane (30 mL), followed by the addition of pinacol vinylboronate (2.8 g, 18.02 mmol), tetra(triphenylphosphino)palladium (0.3 g, 259.61 μmol) and cesium carbonate (11.7 g, 36.04 mmol). The reaction system was stirred at 100°C under the protection of nitrogen to react for 16 h. The reaction mixture was poured into water (300 mL) and extracted with ethyl acetate (100 mL × 3), and the organic phases were combined, washed with saturated brine (300 mL), dried over anhydrous sodium sulfate, and concentrated under reduced pressure to obtain a brown oily crude product 2-(3-pyrrolidin-1-ylpropyl)-6-vinylpyridine 97a (1.2 g, 5.55 mmol, yield: 46.17 %).

The product was verified by LCMS.

For MS-ESI, the calculated value was $[M+H]^+$217.2, and the measurement was 217.1

Step 5

At room temperature, N-cyclopropyl-3-fluoro-2-(3-iodo-1-tetrahydropyran-2-yl-indazol-6-yl)thioalkyl benzamide 41a (600 mg, 1.12 mmol) was dissolved in dioxane (10 mL), followed by the addition of 2-(3-pyrrolidin-1-ylpropyl)-6-vinylpyridine 97a (362 mg, 1.67 mmol), tris(o-methylphenyl)phosphine (339 mg, 1.12 mmol), triethylamine (565 mg, 5.58 mmol) and palladium chloride (99 mg, 558.26 μmol). The reaction system was stirred at 90°C under the protection of nitrogen to react for 16 h. The reaction mixture was poured into water (20 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was subjected to the medium-pressure chromatography to obtain a yellow solid N-cyclopropyl-3-fluoro-2-[3-[(trans)-2-[6-(3-pyrrolidin-1-ylpropyl)-2-pyridyl]vinyl]-1-tetrahydropyran-2-yl-indazol-6-yl]thioalkylbenzamide 100e (500 mg, 798.98 μmol, yield: 71.56 %).

The product was verified by LCMS.

For MS-ESI, the calculated value was $[M+H]^+$626.3, and the measurement was 626.1

Step 6

At room temperature, N-cyclopropyl-3-fluoro-2-[3-[(trans)-2-[6-(3-pyrrolidin-1-ylpropyl)-2-pyridyl]vinyl]-1-tetrahydropyran-2-yl-indazol-6-yl]thioalkyl benzamide 100e (500 mg, 798.98 μmol) was dissolved in dichloromethane (12 mL) and trifluoroacetic acid (4 mL); the reaction system was stirred at 25°C to react for 16 h. The reaction mixture was poured into water (30 mL), adjusted with the saturated sodium carbonate solution to the pH of 8-9 and extracted with dichloromethane (30 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-3-fluoro-2-({3 [(E)-2-{6-[3-(pyrrolidin-1-yl)propyl]pyridin-2-ylvinyl]-1H-indazol-6-yl}thio)benzamide I-124 (51.74 mg, 95.52 μmol, yield: 11.95 %).

The product was verified by LCMS, F-NMR, H-NMR and C-NMR.

For MS-ESI, the calculated value was $[M+H]^+$542.2, and the measurement was 542.3.

$^1$H NMR (400MHz, $CD_3OD$): δ (ppm) 8.00 (d, *J* = 8.4 Hz, 1H), 7.83 (d, *J* = 16.8 Hz, 1H), 7.72 (t, *J* = 8.0 Hz, 1H), 7.56-7.50 (m, 3H), 7.39 (s, 1H), 7.30-7.26 (m, 2H), 7.17 (d, *J*= 7.6 Hz, 1H), 7.12 (dd, *J* = 8.4, 1.2 Hz, 1H), 2.85 (t, *J* = 7.6 Hz, 2H), 2.79-2.74 (m, 1H), 2.63-2.57 (m, 6H), 2.05-1.98 (m, 2H), 1.84-1.80 (m, 4H), 0.75-0.70 (m, 2H), 0.52-0.48 (m, 2H).

$^{13}$C NMR (100 MHz, $CD_3OD$): δ (ppm) 171.64, 165.43, 162.96, 162.76, 156.50, 145.34, 143.92, 143.51, 138.78, 136.57, 132.67, 132.58, 131.18, 123.90, 122.93, 122.22, 121.00, 120.34, 120.01, 119.82, 118.54, 118.31, 111.63, 57.11, 55.02, 36.88, 33.10, 30.78, 30.50, 29.90, 24.18, 23.76, 14.47, 6.44.

$^{19}$F NMR (400MHz, $CD_3OD$): δ (ppm) -105.86.

I-132

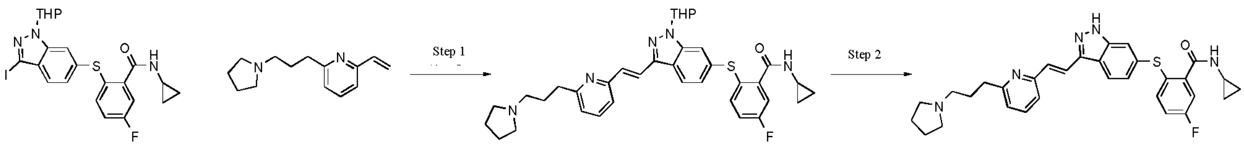


42a 97a 101a I-132

Step 1

At room temperature, N-cyclopropyl-5-fluoro-2-(3-iodo-1-tetrahydropyran-2-yl-indazol-6-yl)thioalkyl benzamide 42a (600 mg, 1.12 mmol) was dissolved in 1,4-dioxane (8 mL), followed by the addition of 2-(3-pyrrolidin-1-ylpropyl)-6-vinylpyridine 97a (290 mg, 1.34 mmol) and Xphos Pd G3 (90 mg, 106.33 μmol). The reaction system was stirred at 90°C to react for 16 h. The reaction mixture was poured into water (30 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was subjected to the medium-pressure chromatography to obtain a brown solid product N-cyclopropyl-5-fluoro-2-[3-[(trans)-2-[6-(3-pyrrolidin-1-ylpropyl)-2-pyridyl]vinyl]-1-tetrahydropyran-2-yl-indazol-6-yl]thioalkylbenzamide 101a (400 mg, 639.19 μmol, yield: 57.25 %).

The product was verified by LCMS.

For MS-ESI, the calculated value was $[M+H]^+$ 626.3, and the measurement was $[M+H]^+$626.1.

Step 2

At room temperature, N-cyclopropyl-5-fluoro-2-[3-[(trans)-2-[6-(3-pyrrolidin-1-ylpropyl)-2-pyridyl]vinyl]-1-tetrahydropyran-2-yl-indazol-6-yl]thioalkyl benzamide 101a (400 mg, 639.19 μmol) was dissolved in dichloromethane (12 mL) and trifluoroacetic acid (3 mL). The reaction system was stirred at 25°C to react for 16 h; the reaction mixture was poured into water (50 mL), adjusted with the saturated sodium carbonate solution to the pH of 10 and extracted with dichloromethane (50 mL × 3); and the organic phases were combined, washed with saturated brine (30 mL), dried over anhydrous sodium sulfate, and concentrated under reduced pressure to obtain a crude product. The crude product was purified by the high-pressure liquid chromatography to obtain a white solid product N-cyclopropyl-5-fluoro-2-({3-[(E)-2-{6-[3-(pyrrolidin-1-yl)propyl]pyridin-2-yl}vinyl]-1H-indazol-6-yl}thio)benzamide I-132 (47.24 mg, 87.21 μmol, yield: 13.64 %).

The product was verified by LCMS, F-NMR, H-NMR and C-NMR.

For MS-ESI, the calculated value was $[M+H]^+$ 542.2, and the measurement was $[M+H]^+$ 542.2.

$^1H$ NMR (400MHz, $CD_3OD$): δ (ppm) 8.05 (d, *J* = 8.8 Hz, 1H), 7.85 (d, *J* = 16.4 Hz, 1H), 7.72 *(t, J*= 7.6 Hz, 1H), 7.57-7.49 (m, 3H), 7.37 (dd, *J* = 8.8, 5.4 Hz, 1H), 7.24 (dd, *J* = 8.8, 2.8 Hz, 1H), 7.18-7.13 (m, 3H), 2.85 *(t, J*= 7.6 Hz, 2H), 2.79-2.74 (m, 1H), 2.72-2.64 (m, 6H), 2.07-2.00 (m, 2H), 1.88-1.80 (m, 4H), 0.76-0.71 (m, 2H), 0.53-0.50 (m, 2H).

$^{19}F$ NMR (400MHz, $CD_3OD$): δ (ppm) -115.44.

$^{13}C$ NMR (100MHz, $CD_3OD$): δ (ppm) 171.19, 171.18, 164.48, 162.50, 162.01, 156.47, 143.96, 143.60, 142.23, 142.10, 138.85, 136.70, 136.62, 136.33, 131.21, 130.47, 130.43, 125.60, 125.16, 123.00, 125.16, 123.00, 122.61, 121.36, 120.53, 118.82, 118.80, 116.440, 116.16, 113.94, 56.95, 55.05, 36.66, 29.40, 24.15 ,23.79, 6.46.

I-134

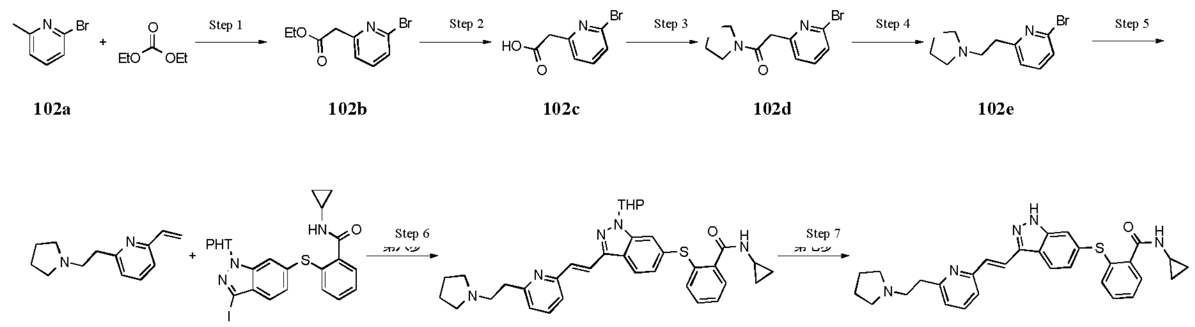


102a 102b 102c 102d 102e

102f 93a 102g I-134

Step 1

Under the protection of nitrogen at room temperature, 2-bromo-6-methylpyridine 102a (50 g, 290.66 mmol) was dissolved in tetrahydrofuran (500 mL); the mixture was cooled to -70°C, followed by the addition of lithium diisopropylamide (2 M, 291 mL, 581.32 mmol). The reaction system was stirred at -70°C to react for 1 h. Then, diethyl carbonate (41.2 g, 348.79 mmol) was slowly added at -70°C, and the reaction system was stirred at -70°C to react for 4 h. The reaction mixture was poured into water (1000 mL) and extracted with ethyl acetate (500 mL × 3), and the organic phases were combined, washed with saturated brine (300 mL), dried over anhydrous sodium sulfate, and concentrated under reduced pressure to obtain a crude product. The crude product was subjected to the medium-pressure chromatography to obtain a yellow oily product 2-(6-bromo-2-pyridyl)ethyl acetate 102b (38 g, 155.68 mmol, yield: 53.56 %).

The product was verified by LCMS and H-NMR.

For MS-ESI, the calculated value was $[M+H]^+$244.0, and the measurement was 243.9.

$^1H$ NMR (400 MHz,$CDCl_3$): δ (ppm) 7.53 (t, *J* = 7.8 Hz, 1H), 7.40 (d, *J* = 8.0 Hz, 1H), 7.31-7.26 (m, 1H), 4.19 (q, *J*= 7.2 Hz, 2H), 3.83 (s, 2H), 1.30-1.23 (m, 3H).

Step 2

At room temperature, 2-(6-bromo-2-pyridyl)ethyl acetate102b (40 g, 163.88 mmol) was dissolved in water (100 mL), tetrahydrofuran (100 mL) and methanol (200 mL), followed by the addition of lithium hydroxide (11.8 g, 491.63 mmol). The reaction system was stirred at 25°C to react for 2 h. Tetrahydrofuran and methanol were spun away; the mixture was poured into water (500 mL), adjusted with 1N dilute hydrochloric acid to the pH of 3, and then extracted with ethyl acetate (500 mL × 3); and the organic phases were combined, washed with saturated brine (300 mL), dried over anhydrous sodium sulfate, and concentrated under reduced pressure to obtain a yellow solid product 2-(6-bromo-2-pyridyl)acetic acid 102c (33 g, 152.76 mmol, yield: 93.21 %).

The product was verified by LCMS and H-NMR.

For MS-ESI, the calculated value was $[M+H]^+$ 216.0, and the measurement was 215.9.

$^1H$ NMR (400MHz, $CDCl_3$):δ(ppm) 7.58 *(t, J*= 7.6 Hz, 1H), 7.44 *(d, J*= 8.0 Hz, 1H), 7.27 *(d, J*= 7.2 Hz, 1H), 3.89 (s, 2H).

Step 3

At room temperature, 2-(6-bromo-2-pyridyl) acetic acid 102c (26 g, 120.35 mmol) was dissolved in N,N-dimethylformamide (400 mL), followed by the addition of pyrrolidine (12.8 g, 180.53 mmol), **1**-hydroxybenzotriazole (24.4 g, 180.53 mmol), N,N-diisopropylethylamine (46.6 g, 361.06 mmol) and 1-(3-dimethylaminopropyl)-3-ethylcarbodiimide hydrochloride (34.7 g, 180.53 mmol). The reaction system was stirred at 25°C to react for 16 h. The reaction mixture was poured into water (500 mL) and extracted with ethyl acetate (500 mL × 3), and the organic phases were combined, washed with saturated brine (300 mL), dried over anhydrous sodium sulfate, and concentrated under reduced pressure to obtain a crude product. The crude product was purified by the medium-pressure preparative chromatography to obtain a yellow solid product 2-(6-bromo-2-pyridyl)-1-pyrrolidin-1-ylethanone 102d (28 g, 104.04 mmol, yield: 86.44 %).

The product was verified by LCMS and H-NMR.

For MS-ESI, the calculated value was $[M+H]^+$269.0, and the measurement was 268.9.

$^1$H NMR (400MHz, $CDCl_3$): δ (ppm) 7.51 (t, *J* = 7.6 Hz, 1H), 7.39-7.36 (m, 2H), 3.83 (s, 2H), 3.56 (t, *J* = 6.8 Hz, 2H), 3.48 *(t, J*= 6.8 Hz, 2H), 2.00-1.93 (m, 2H), 1.90-1.84 (m, 2H).

Step 4

At room temperature, 2-(6-bromo-2-pyridyl)-1-pyrrolidin-1-ylethanone 102d (26 g, 96.61 mmol) was dissolved in tetrahydrofuran (250 mL), followed by the addition of borane dimethyl sulfide complex (10 M, 19 mL). The reaction system was stirred at 70°C to react for 3 h. The reaction mixture was cooled to room temperature, followed by the slow addition of methanol (50 mL), and the mixture was refluxed and stirred for 4 h and concentrated under reduced pressure to obtain a crude product. The crude product was subjected to the medium-pressure chromatography to obtain a yellow oily product 2-bromo-6-(2-pyrrolidin-1-yl)pyridine 102e (20 g, 78.38 mmol, yield: 81.14 %).

The product was verified by LCMS and H-NMR.

For MS-ESI, the calculated value was $[M+H]^+$255.0, and the measurement was 255.0.

$^1$H NMR (400MHz, $CDCl_3$): δ (ppm) 7.46 (t, *J* = 7.6 Hz, 1H), 7.32 (d, *J* = 7.6 Hz, 1H), 7.17 (d, *J* = 7.2 Hz, 1H), 3.08-3.01 (m, 2H), 2.94-2.88 (m, 2H), 2.67-2.60 (m, 4H), 1.84-1.81 (m, 4H).

Step 5

At room temperature, 2-bromo-6-(2-pyrrolidin-1-yl)pyridine 102e (20 g, 78.38 mmol) was dissolved in N,N-dimethylformamide (200 mL), followed by the addition of DPPF dichloropalladium (II) (2.0 g, 2.74 mmol), triethylamine (23.8 g, 235.15 mmol) and potassium trifluoro(vinyl)borate (15.8 g, 117.58 mmol). The reaction system was stirred at 85°C under the protection of nitrogen to react for 16 h. The reaction mixture was poured into water (500 mL) and extracted with ethyl acetate (500 mL × 3), and the organic phases were combined, washed with saturated brine (300 mL), dried over anhydrous sodium sulfate, and concentrated under reduced pressure to obtain a crude product. The crude product was subjected to the medium-pressure chromatography to obtain a brown oily product 2-(2-pyrrolidin-1-yl)-6-vinylpyridine 102f (9.5 g, 46.96 mmol, yield: 59.91 %).

The product was verified by LCMS and H-NMR.

For MS-ESI, the calculated value was $[M+H]^+$203.1, and the measurement was 203.1

$^1$H NMR (400MHz, $CDCl_3$): 7.55 *(t, J*= 8 Hz, 1H), 7.18 *(d, J*= 8.0 Hz, 1H), 7.07 (d, *J* = 7.6 Hz, 1H), 6.84-6.75 (m, 1H), 6.21-6.14 (m, 1H), 5.45 (dd, *J* = 10.8, 1.6 Hz, 1H), 3.06-3.02 (m, 2H), 2.94-2.88 (m, 2H), 2.65-2.58 (m, 4H), 1.85-1.76 (m, 4H).

Step 6

At room temperature, N-cyclopropyl-2-(3-iodo-1-tetrahydropyran-2-yl-indazol-6-yl)thioalkyl benzamide 93a (600 mg, 1.16 mmol) was dissolved in dioxane (10 mL), followed by the addition of 2-(2-pyrrolidin-1-yl)-6-vinylpyridine 102f (234 mg, 1.16 mmol), dichloropalladium (II) (102 mg, 577.59 μmol), triethylamine (585 mg, 5.78 mmol) and tris(o-methylphenyl)phosphine (351 mg, 1.16 mmol). The reaction system was stirred at 90°C under the protection of nitrogen to react for 16 h. The reaction mixture was poured into water (40 mL) and extracted with ethyl acetate (40 mL × 3), and the organic phases were combined, washed with saturated brine (40 mL), dried over anhydrous sodium sulfate, and concentrated under reduced pressure to obtain a crude product. The crude product was subjected to the medium-pressure chromatography to obtain N-cyclopropyl-2-[3-[(trans)-2-[6-(2-pyrrolidin-1-yl)-2-pyridyl]vinyl]-1-tetrahydropyran-2-yl-indazol-6-yl]thioalkylbenzamide 102 g (300 mg, 505.24 μmol, yield: 43.74 %).

The product was verified by LCMS.

For MS-ESI, the calculated value was $[M+H]^+$594.3, and the measurement was 594.1

Step 7

At room temperature, N-cyclopropyl-2-[3-[(trans)-2-[6-(2-pyrrolidin-1-yl)-2-pyridyl]vinyl]-1-tetrahydropyran-2-yl-indazol-6-yl]thioalkyl benzamide 102 g (300 mg, 505.24 μmol) was dissolved in dichloromethane (12 mL) and trifluoroacetic acid (4 mL); the reaction system was stirred at 25 °C to react for 16 h. The reaction mixture was poured

into water (50 mL), adjusted with the saturated sodium carbonate solution to the pH of 8 and extracted with dichloromethane (50 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{4-[2-(pyrrolidin-1-yl)ethyl]pyridin-2-yl}vinyl]-1H-indazol-6-yl}thio)benzamide I-134 (72.04 mg, 141.35 μmol, yield: 27.98 %).

The product was verified by LCMS, H-NMR and C-NMR.

For MS-ESI, the calculated value was $[M+H]^+$510.2, and the measurement was 510.3

$^1$H NMR (400MHz, $CD_3OD$): 8.09 (d, *J* = 8.8 Hz, 1H), 7.87 (d, *J* = 16.8 Hz, 1H), 7.73 (t, *J* = 8.0 Hz, 1H), 7.61-7.41 (m, 4H), 7.39-7.15 (m, 5H), 3.08-3.04 (m, 2H), 2.96-2.93 (m, 2H), 2.82-2.74 (m, 1H), 2.68 (s, 4H), 1.93-1.78 (m, 4H), 0.81-0.66 (m, 2H), 0.58-0.49 (m, 2H).

$^{13}$C NMR (100 MHz, $CD_3OD$) δ (ppm) 172.69, 160.99, 156.50, 144.00, 143.65, 139.29, 138.81, 135.76, 133.25, 131.25, 128.22, 125.16, 123.22, 122.63, 121.47, 120.70, 114.86, 57.26, 55.05, 38.04, 24.25, 23.78, 6.47.

I-136

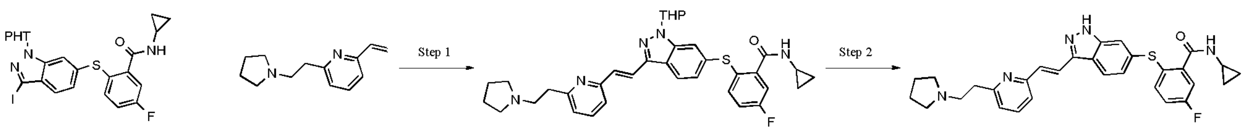


42a 102f 103a I-136

Step 1

At room temperature, N-cyclopropyl-5-fluoro-2-(3-iodo-1-tetrahydropyran-2-yl-indazol-6-yl)thioalkyl benzamide 42a (300 mg, 558.26 μmol) was dissolved in 1,4-dioxane (5 mL), followed by the addition of 2-(2-pyrrolidin-1-yl)-6-vinylpyridine 102f (113 mg, 558.26 μmol), triethylamine (282 mg, 2.79 mmol) and Xphos Pd G3 (47 mg, 55.83 μmol). The reaction system was stirred at 90°C to react for 16 h under the protection of nitrogen. The reaction mixture was poured into water (20 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 20/1-10/1) to obtain a yellow solid product N-cyclopropyl-5-fluoro-2-[3-[(trans)-2-[6-(2-pyrrolidin-1-yl)-2-pyridyl]vinyl]-1-tetrahydropyran-2-yl-indazol-6-yl]thioalkylbenzamide 103a (80 mg, 130.77 μmol, yield: 23.42 %).

The product was verified by LCMS.

For MS-ESI, the calculated value was $[M+H]^+$ 612.3, and the measurement was 612.3.

Step 2

At room temperature, N-cyclopropyl-5-fluoro-2-[3-[(trans)-2-[6-(2-pyrrolidin-1-yl)-2-pyridyl]vinyl]-1-tetrahydropyran-2-yl-indazol-6-yl]thioalkyl benzamide 103a (340 mg, 555.76 μmol) was dissolved in dichloromethane (12 mL), followed by the addition of trifluoroacetic acid (3 mL). The reaction system was stirred at 25°C to react for 16 h. The reaction mixture was poured into water (30 mL), adjusted with the saturated sodium carbonate solution to the pH of 10 and extracted with dichloromethane (30 mL × 3), and the organic phases were combined, washed with saturated brine (50 mL), dried over anhydrous sodium sulfate, and concentrated under reduced pressure to obtain a crude product. The crude product was purified by the high-pressure liquid chromatography to obtain a white solid product N-cyclopropyl-5-fluoro-2-({3-[(E)-2-{6-[2-(pyrrolidin-1-yl)ethyl]pyridin-2-yl}vinyl]-1H-indazol-6-yl}thio)benzamide I-136 (10.59 mg, 20.07 μmol, yield: 3.61 %).

The product was verified by LCMS, F-NMR and H-NMR.

For MS-ESI, the calculated value was $[M+H]^+$ 528.2, and the measurement was 528.2.

$^1$H NMR (400MHz, $CD_3OD$): δ (ppm) 8.07 (d, *J* = 8.4 Hz, 1H), 7.90 (d, *J* = 16.4Hz, 1H), 7.78 (t, *J* = 8.0 Hz, 1H), 7.58-7.52 (m, 2H), 7.48 (s, 1H), 7.40 (dd, *J* = 8.8, 5.25 Hz, 1H), 7.27-7.23(m, 2H), 7.20-7.14 (m, 2H), 3.50 (t, *J* = 7.6 Hz, 2H), 3.25-3.20 (m, 6H), 2.80-2.73 (m, 1H), 2.07-2.03 (m, 4H), 0.76-0.72 (m, 2H), 0.54-0.50 (m, 2H).

$^{19}$F NMR (400MHz, $CD_3OD$): δ (ppm) -115.37

I-137

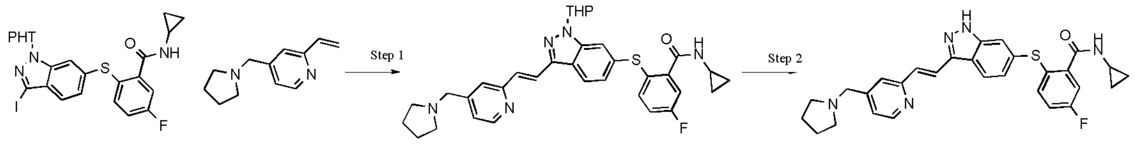


42a 90c 104a I-137

Step 1

At room temperature, N-cyclopropyl-3-fluoro-2-(3-iodo-1-tetrahydropyran-2-yl-indazol-6-yl)thioalkyl benzamide 42a (300 mg, 558.26 μmol) was dissolved in 1,4-dioxane (5 mL), followed by the addition of 4-(pyrrolidin-1-ylmethyl)-2-vinylpyridine 90c (105 mg, 558.26 μmol), triethylamine (283 mg, 2.79 mmol) and Xphos Pd G3 (47 mg, 55.83 μmol). The reaction system was stirred at 90°C to react for 16 h under the protection of nitrogen. The reaction mixture was poured into water (30 mL) and extracted with ethyl acetate (30 mL × 3), and the organic phases were combined, washed with saturated brine (50 mL), dried over anhydrous sodium sulfate, and concentrated under reduced pressure to obtain a crude product. The crude product was purified by the column chromatography (dichloromethane/methanol = 50/1-10/1) to obtain a brown solid product N-cyclopropyl-5-fluoro-2-[3-[(trans)-2-[4-(pyrrolidin-1-ylmethyl)-2-pyridyl]vinyl]-1-tetrahydropyran-2-yl-indazol-6-yl]thioalkylbenzamide 104a (190 mg, 317.86 μmol, yield: 56.94 %).

The product was verified by LCMS.

For MS-ESI, the calculated value was $[M+H]^+$ 598.3, and the measurement was 598.2.

Step 2

N-cyclopropyl-5-fluoro-2-[3-[(trans)-2-[4-(pyrrolidin-1-ylmethyl)-2-pyridyl]vinyl]-1-tetrahydropyran-2-yl-indazol-6-yl]thioalkyl benzamide 104a (190 mg, 317.86 μmol) was dissolved in dichloromethane (3 mL), followed by the addition of trifluoroacetic acid (1 mL). The reaction system was stirred at 25°C to react for 16 h. The reaction mixture was poured into water (10 mL), adjusted with the saturated sodium carbonate solution to the pH of 8 and extracted with dichloromethane (10 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a white solid product N-cyclopropyl-5-fluoro-2-({3-[(E)-2-{4-[(pyrrolidin-1-yl)methyl]pyridin-2-yl}vinyl]-1H-indazol-6-yl}thio)benzamide I-137 (21.71 mg, 42.27 μmol, yield: 13.30 %).

The product was verified by LCMS, F-NMR and H-NMR.

For MS-ESI, the calculated value was $[M+H]^+$514.2, and the measurement was 514.1.

$^1$H NMR (400MHz, $CD_3OD$): δ (ppm) 8.48 (d, $J$= 5.2 Hz, 1H), 8.05 (d, $J$= 8.8 Hz, 1H), 7.86 (d, $J$ = 16.8 Hz, 1H), 7.68 (s, 1H), 7.57-7.49 (m, 2H), 7.39-7.36 (m, 1H), 7.30 (d, $J$= 4.8 Hz, 1H), 7.25 (dd, $J$ = 8.8, 2.8 Hz, 1H), 7.18-7.13 (m, 2H), 3.72 (s, 2H), 2.78-2.74 (m, 1H), 2.60-2.58 (m, 4H), 1.86-1.83 (m, 4H), 0.76-0.71 (m, 2H), 0.53-0.49 (m, 2H).

$^{19}$F NMR (400MHz, $CD_3OD$): δ (ppm) -115.51.

I-138

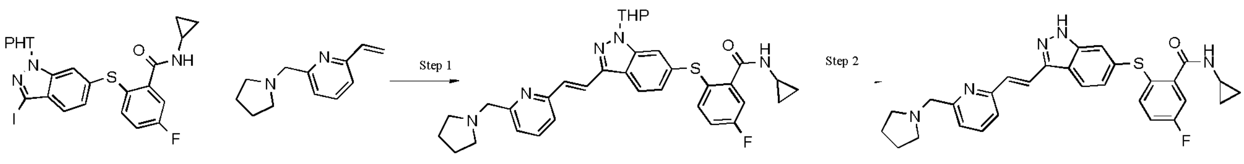


42a 89c 105a I-138

Step 1

At room temperature, N-cyclopropyl-5-fluoro-2-(3-iodo-1-tetrahydropyran-2-yl-indazol-6-yl)thioalkyl benzamide 42a (300 mg, 558.26 μmol) was dissolved in 1,4-dioxane (5 mL), followed by the addition of 2-(pyrrolidin-1-ylmethyl)-6-vinylpyridine 89c (105 mg, 558.26 μmol), Xphos Pd $G_3$ (47 mg, 55.83 μmol) and triethylamine (169 mg, 1.67 mmol). The reaction system was stirred at 90°C to react for 16 h under the protection of nitrogen. The reaction mixture was poured into water (20 mL) and extracted with ethyl acetate (10 mL × 3), and the organic phases were combined, washed with saturated brine (30 mL), dried over anhydrous sodium sulfate, and concentrated under reduced pressure to obtain a

crude product. The crude product was purified by the column chromatography (dichloromethane/methanol = 50/1-10/1) to obtain a yellow solid product N-cyclopropyl-5-fluoro-2-[3-[(trans)-2-[6-(pyrrolidin-1-ylmethyl)-2-pyridyl]vinyl]-1-tetrahydropyran-2-yl-indazol-6-yl]thioalkylbenzamide 105a (300 mg, 501.89 μmol, yield: 89.90 %).

The product was verified by LCMS.

For MS-ESI, the calculated value was $[M+H]^+$ 598.3, and the measurement was 598.0.

Step 2

At room temperature, N-cyclopropyl-5-fluoro-2-[3-[(trans)-2-[6-(pyrrolidin-1-ylmethyl)-2-pyridyl]vinyl]-1-tetrahydropyran-2-yl-indazol-6-yl]thioalkyl benzamide 105a (300 mg, 501.89 μmol) was dissolved in dichloromethane (3 mL), followed by the addition of trifluoroacetic acid (1 mL). The reaction system was stirred at 25°C to react for 16 h. The reaction mixture was poured into water (10 mL), adjusted with the saturated sodium carbonate solution to the pH of 8 and extracted with dichloromethane (10 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a white solid product N-cyclopropyl-5-fluoro-2-({3-[(E)-2-{6-[(pyrrolidin-1-yl)methyl]pyridin-2-yl}vinyl]-1H-indazol-6-yl}thio)benzamide I-138 (13 mg, 25.31 μmol, yield: 5.04 %).

The product was verified by LCMS, F-NMR and H-NMR.

For MS-ESI, the calculated value was $[M+H]^+$ 514.2, and the measurement was 514.1.

$^1$H NMR (400MHz, $CD_3OD$): δ (ppm) 8.06 (d, *J* = 8.4 Hz, 1H), 7.91 (d, *J* = 16.4 Hz, 1H), 7.78 (t, *J* = 8.4 Hz, 1H), 7.59-7.54 (m, 2H), 7.49 (d, *J* = 0.8 Hz, 1H), 7.39-7.33 (m, 2H), 7.24 (dd, *J*= 8.8, 3.2 Hz, 1H), 7.18-7.13 (m, 2H), 3.83 (s, 2H), 2.79-2.74 (m, 1H), 2.69-2.65 (m, 4H), 1.86-1.82 (m, 4H), 0.76-0.69 (m, 2H), 0.53-0.49 (m, 2H).

$^{19}$F NMR (400MHz, $CD_3OD$): δ (ppm) -115.52.

I-103

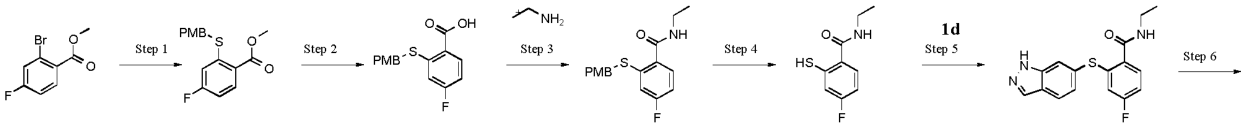


33a 33b 33c 106a 106b 106c

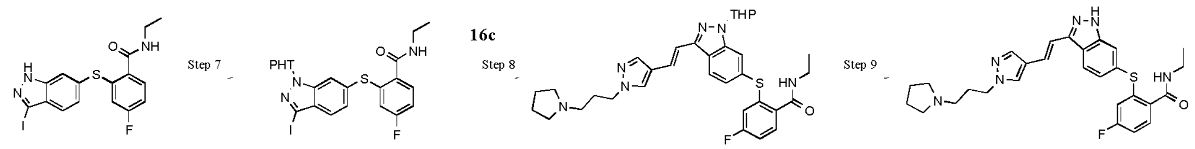


106d 106e 106f I-103

Step 1

At room temperature, 2-bromo-4-fluoro-methyl benzoate 33a (25 g, 107.28 mmol) was dissolved in N,N-dimethylformamide (250 mL), followed by the addition of (4-methoxyphenyl)methanethiol (16.6 g, 107.28 mmol), triethylamine (32.5 g, 321.84 mmol) and [1,1'-bis(diphenylphosphino)ferrocene]dichloropalladium (II) (2.5 g, 107.28 mmol). The reaction system was stirred at 120°C to react for 16 h under the protection of nitrogen. The reaction mixture was poured into water (1000 mL) and extracted with ethyl acetate(500 mL × 3), and the organic phases were combined, washed with saturated brine (500 mL), dried over anhydrous sodium sulfate, and concentrated under reduced pressure to obtain a crude product. The crude product was purified by the column chromatography (petroleum ether/ethyl acetate = 50/1-20/1) to obtain a yellow solid product 4-fluoro-2-[(4-methoxyphenyl)methylthioalkyl]methyl benzoate 33b (13 g, 42.43 mmol, yield: 39.56 %).

The product was verified by LCMS and H-NMR.

For MS-ESI, the calculated value was $[M+Na]^+$329.1, and the measurement was 328.9.

$^1$HNMR (400MHz, $CDCl_3$): δ (ppm) 8.02 (dd, *J=8.8,* 6.0 Hz, 1H), 7.36-7.32 (m, 2H), 7.05 (dd, *J* = 10.4, 2.4 Hz, 1H), 6.88-6.84 (m, 2H), 6.82-6.80 (m, 1H), 4.08 (s, 2H), 3.88 (s, 3H), 3.79 (s, 3H).

Step 2

At room temperature, 4-fluoro-2-[(4-methoxyphenyl)methylthioalkyl]methyl benzoate 33b (13 g, 42.43 mmol) was dissolved in tetrahydrofuran (120 mL) and water (40 mL), followed by the addition of lithium hydroxide (3.05 g, 127.30 mmol). The reaction system was stirred at 50°C to react for 5 h. The reaction mixture was poured into water (300 mL), adjusted with 2N dilute hydrochloric acid to the pH of 5 and extracted with ethyl acetate (300 mL × 3); and the organic phases were combined, washed with saturated brine (300 mL), dried over anhydrous sodium sulfate, and concentrated under reduced pressure to obtain a yellow solid product 4-fluoro-2-[(4-methoxyphenyl)methylthioalkyl]benzoic acid 33c (11 g, 37.63 mmol, yield: 88.68 %).
The product was verified by LCMS and H-NMR.
For MS-ESI, the calculated value was $[M+Na]^+$315.1, and the measurement was 314.9.
$^1$HNMR (400 MHz, $CD_3OD$): δ(ppm) 8.14 (dd, *J=8.8,* 6.0 Hz, 1H), 7.34 (d, *J=8.4* Hz, 2H), 7.08 (dd, *J*=10.4, 2.4 Hz, 1H), 6.89-6.84 (m, 3H), 4.10 (s, 2H), 3.81 (s, 3H).

Step 3

At room temperature, 4-fluoro-2-[(4-methoxyphenyl)methylthioalkyl]benzoic acid 33c (15 g, 51.31 mmol) was dissolved in N,N-dimethylformamide (150 mL), followed by the addition of ethylamine (5 g, 61.58 mmol, 6.25 mL), N,N-diisopropylethylamine (19.9 g, 153.94 mmol), 1-hydroxybenzotriazole (10.4 g, 76.97 mmol) and 1-(3-dimethylaminopropyl)-3-ethylcarbodiimide hydrochloride (14.8 g, 76.97 mmol). The reaction system was stirred at 25°C to react for 2 h. The reaction liquid was poured into water (500 mL) and filtered; the filter cake was dissolved in ethyl acetate, dried over anhydrous sodium sulfate, and concentrated under reduced pressure to obtain a white solid crude product, N-ethyl-4-fluoro-2-[(4-methoxyphenyl)methylthioalkyl]benzamide 106a (15 g, 46.96 mmol, yield: 91.53 %).
The product was verified by LCMS and H-NMR.
For MS-ESI, the calculated value was $[M+H]^+$320.1, and the measurement was 320.1.
$^1$HNMR (400MHz, $CDCl_3$): δ (ppm) 7.60 (dd, *J=8.8,* 6.0 Hz, 1H), 7.16 (d, *J=8.8* Hz, 2H), 7.07 (dd, *J*=9.6, 2.8 Hz, 1H), 6.94-6.89 (m, 1H), 6.83-6.79 (m, 2H), 6.32 (s, 1H), 4.06 (s, 2H), 3.78 (s, 3H), 3.47-3.40 (m, 2H), 1.21 (t, *J*=7.2 Hz, 3H).

Step 4

At room temperature, N-ethyl-4-fluoro-2-[(4-methoxyphenyl)methylthioalkyl]benzamide 106a (6 g, 18.79 mmol) was dissolved in trifluoroacetic acid (60 mL). The reaction system was stirred at 70°C to react for 5 h. The reaction mixture was concentrated under reduced pressure to obtain a crude product. The crude product was purified by medium-pressure preparative chromatography to obtain a yellow solid product, N-ethyl-4-fluoro-2-mercaptobenzamide 106b (3 g, 15.06 mmol, yield: 80.15 %).
The product was verified by LCMS and H-NMR.
For MS-ESI, the calculated value was $[M+H]^+$200.0, and the measurement was 200.0.
$^1$HNMR (400MHz, $CDCl_3$): δ (ppm) 7.43 (dd, *J*=8.4, 5.6 Hz, 1H), 7.04 (dd, *J*=10.0, 3.2 Hz, 1H), 6.85-6.80 (m, 2H), 6.00 (s, 1H), 5.08 (s, 1H), 3.52-3.44 (m, 2H), 1.26 (t, *J*=7.2 Hz, 3H).

Step 5

At room temperature, N-ethyl-4-fluoro-2-mercaptobenzamide 106b (4.5 g, 22.59 mmol) was dissolved in N,N-dimethylformamide (50 mL), followed by the addition of 6-iodo-1H-indazole 1d (5.5 g, 22.59 mmol), cesium carbonate (22.1 g, 67.76 mmol), tris(dibenzalaceton)dipalladium (2.1 g, 2.26 mmol) and 4,5-bisdiphenylphosphino-9,9-dimethylxanthene (2.6 g, 4.52 mmol). The reaction system was stirred at 80°C to react for 16 h under the protection of nitrogen. The reaction mixture was poured into water (300 mL) and extracted with ethyl acetate(100 mL × 3), and the organic phases were combined, washed with saturated brine (300 mL), dried over anhydrous sodium sulfate, and concentrated under reduced pressure to obtain a crude product. The crude product was purified by the column chromatography (petroleum ether/ethyl acetate = 10/1-1/1) to obtain a yellow solid product N-ethyl-4-fluoro-2-(1H-indazol-6-ylthioalkyl)benzamide 106c (5.6 g, 17.76 mmol, yield: 78.62 %).
The product was verified by LCMS and H-NMR.
For MS-ESI, the calculated value was $[M+H]^+$316.1, and the measurement was 316.0.
$^1$HNMR (400MHz, $CDCl_3$): δ (ppm) 8.13 (s,1H), 7.80-7.77 (m, 2H), 7.60 (dd, *J=8.8,* 6.0 Hz, 1H), 7.22 (d, *J=8.4* Hz, 1H), 6.93-6.88 (m, 1H), 6.72 (dd, *J*=9.6, 2.4 Hz, 1H), 6.26 (s, 1H), 3.55-3.48 (m, 2H), 1.25 (t, *J*=7.2 Hz, 3H).

Step 6

At room temperature, N-ethyl-4-fluoro-2-(1H-indazol-6-ylthioalkyl )benzamide 106c (6 g, 19.03 mmol) was dissolved in N,N-dimethylformamide (60 mL), followed by the addition of potassium carbonate (7.9 g, 57.08 mmol) and iodine (7.2 g, 28.54 mmol). The reaction system was stirred at 40°C to react for 1 h. The reaction liquid was poured into water (300 mL) and filtered; the filter cake was dissolved in ethyl acetate, dried over anhydrous sodium sulfate, and concentrated under reduced pressure to obtain a yellow solid crude product, N-ethyl-4-fluoro-2-[(3-iodo-1H-indazol-6-yl) thioalkyl]benzamide 106d (7 g, 15.86 mmol, yield: 83.38 %).

The product was verified by LCMS and H-NMR.

For MS-ESI, the calculated value was $[M+H]^+$442.0, and the measurement was 441.9.

$^1$HNMR (400 MHz, DMSO-d6): δ (ppm) 8.51 (t, *J*=5.2 Hz, 1H), 7.74 (s, 1H), 7.58 (dd, *J=8.8,* 6.0Hz, 1H), 7.49 (d, *J=8.4* Hz, 1H), 7.17 (d, *J=8.4* Hz, 1H), 7.12-7.07 (m, 1H), 6.59 (dd, *J*=10.0, 2.4 Hz, 1H), 3.30-3.23 (m, 2H), 1.13 (t, *J*=7.2 Hz, 3H).

Step 7

At room temperature, N-ethyl-4-fluoro-2-[(3-iodo-1H-indazol-6-yl)thioalkyl]benzamide 106d (7 g, 15.86 mmol) was dissolved in ethyl acetate (70 mL), followed by the addition of 3,4-dyhydro-2H-pyran (4.0 g, 47.59 mmol) and p-toluenesulfonic acid (1.4 g, 7.93 mmol). The reaction system was stirred at 70°C to react for 16 h. The reaction mixture was filtered, and the filter cake was dried in vacuum to obtain a yellow solid crude product N-ethyl-4-fluoro-2-(3-iodo-1-tetrahydropyran-2-yl-indazol-6-yl)thioalkylbenzamide 106e (7 g, 13.32 mmol, yield: 83.99 %). The product was verified by LCMS and H-NMR.

For MS-ESI, the calculated value was $[M+H]^+$526.0, and the measurement was 525.9.

$^1$HNMR (400MHz, DMSO-d6): δ (ppm) 8.51 (t, *J*=5.6 Hz, 1H), 8.04 (s, 1H), 7.61 (dd, *J*=8.4, 6.0 Hz, 1H), 7.52 (d, J=7.6 Hz, 1H), 7.26 (d, *J=8.4* Hz, 1H), 7.13-7.08 (m, 1H), 6.57 (dd, *J*=10.0, 2.4 Hz, 1H), 5.92 (dd, *J*=9.6, 1.6 Hz, 1H), 3.87 (d, *J*=11.2 Hz, 1H), 3.78-3.71 (m, 1H), 3.31-3.24 (m, 2H), 2.41-2.31 (m, 1H), 2.02-1.97 (m, 2H), 1.77-1.66 (m, 1H), 1.57 (s, 2H), 1.13 (t, *J*=7.2 Hz, 3H).

Step 8

At room temperature, N-ethyl-4-fluoro-2-(3-iodo-1-tetrahydropyran-2-yl-indazol-6-yl)thioalkyl benzamide 106e (1 g, 1.90 mmol) was dissolved in 1,4-dioxane (10 mL), followed by the addition of 1-(3-pyrrolidin-1-ylpropyl)-4-vinylpyrazole 16c (391 mg, 1.90 mmol), triethylamine (578 mg, 5.71 mmol) and (2-dicyclohexylphosphino-2',4',6'-triisopropyl-1,1'-biphenyl)(2'-amino-1,1'-biphen-2-yl)palladium(II) methanesulfonate (161 mg, 190.34 μmol). The reaction system was stirred at 90°C to react for 16 h under the protection of nitrogen. The reaction mixture was poured into water (30 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 50/1-10/1) to obtain a yellow solid product N-ethyl-4-fluoro-2-[3-[(trans)-2-[1-(3-pyrrolidin-1-ylpropyl)pyrazol-4-yl]vinyl]-1-tetrahydropyran-2-yl-indazol-6-yl]thioalkylbenzamide 106f (500 mg, 829.51 μmol, yield: 43.58 %).

The product was verified by LCMS.

For MS-ESI, the calculated value was $[M+H]^+$603.3, and the measurement was 603.1.

Step 9

At room temperature, N-ethyl-4-fluoro-2-[3-[(trans)-2-[1-(3-pyrrolidin-1-ylpropyl)pyrazole -4-yl]vinyl]-1-tetrahydropyran-2-yl-indazol-6-yl]thioalkyl benzamide 106f (1 g, 1.66 mmol) was dissolved in dichloromethane (24 mL), followed by the addition of trifluoroacetic acid (8 mL). The reaction system was stirred at 25°C to react for 16 h. The reaction mixture was poured into water (100 mL), adjusted with the saturated aqueous solution of sodium carbonate to the pH of 10 and extracted with dichloromethane (100 mL × 3); and the organic phases were combined, washed with saturated brine (100 mL), dried over anhydrous sodium sulfate, and concentrated under reduced pressure to obtain a crude product, which was purified by the high-pressure liquid chromatography to obtain a yellow solid product N-ethyl-4-fluoro-2-({3-[(E)-2-{1-[3-(pyrrolidin-1-yl)propyl]-1H-pyrazol-4-yl}vinyl]-1H-indazol-6-yl}thio)benzamide I-103 (124.46 mg, 239.97 μmol, yield: 14.46 %).

The product was verified by LCMS, F-NMR, H-NMR and C-NMR.

For MS-ESI, the calculated value was $[M+H]^+$519.2, and the measurement was 519.1.

$^1$HNMR (400MHz, $CD_3OD$): δ (ppm) 8.08 (d, *J=8.8* Hz, 1H), 7.91 (s, 1H), 7.80 (s, 1H), 7.68 (s, 1H), 7.50 (dd, *J=8.8,* 6.0 Hz, 1H), 7.37 (d, *J*=16.8 Hz, 1H), 7.23-7.21 (m, 2H), 7.01-7.96 (m, 1H), 6.73 (dd, *J*=10.0, 2.4 Hz, 1H), 4.22 (t,

$J$=6.8 Hz, 2H), 3.39-3.34 (m, 2H), 2.57 (s, 4H), 2.51-2.47 (m, 2H), 2.12-2.08 (m, 2H), 1.83-1.80 (m, 4H), 1.20 (t, $J$=7.2 Hz, 3H).

$^{13}$CNMR (100MHz, $CD_3OD$&$CDCl_3$) δ (ppm) 169.96, 166.19, 163.69, 144.89, 143.59, 141.79, 141.71, 138.48, 133.04, 132.93, 131.13, 131.05, 129.63, 127.37, 123.30, 122.97, 122.08, 121.91, 118.99, 117.43, 117.18, 114.02, 113.80, 55.17, 54.44, 51.36, 36.04, 33.16, 30.85, 30.57, 30.49, 24.35, 23.86, 15.08, 14.82.

$^{19}$FNMR (400MHz, $CD_3OD$): δ (ppm) -111.69.

I-128

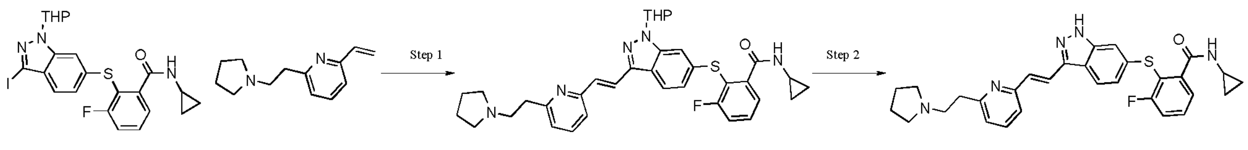


41a 102f 107a I-128

Step 1

At room temperature, N-cyclopropyl-3-fluoro-2-(3-iodo-1-tetrahydropyran-2-yl-indazol-6-yl)thioalkyl benzamide 41a (500 mg, 930.43 μmol) was dissolved in 1,4-dioxane (5 mL), followed by the addition of 2-(2-pyrrolidin-1-ylethyl)-6-vinylpyridine 102f (226 mg, 1.12 mmol), tris(o-methylphenyl)phosphine (1.1 g, 930.43 μmol), triethylamine (471 mg, 4.65 mmol) and dichloropalladium (II) (75.00 mg, 422.95 μmol). The reaction system was stirred at 90°Cunder the protection of nitrogen to react for 16 h. The reaction mixture was poured into water (30 mL) and extracted with ethyl acetate (20 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/absolute methanol = 10/1) to obtain a yellow solid product N-cyclopropyl-3-fluoro-2-[3-[(trans)-2-[6-(2-pyrrolidin-1-ylethyl)-2-pyridyl]vinyl]-1-tetrahydropyran-2-yl-indazol-6-yl]thioalkylbenzamide 107a (250 mg, 408.65 μmol, yield: 43.92 %).

The product was verified by LCMS.

For MS-ESI, the calculated value was $[M+H]^+$ 612.3, and the measurement was 612.1.

Step 2

N-cyclopropyl-3-fluoro-2-[3-[(trans)-2-[6-(2-pyrrolidin-1-ylethyl)-2-pyridyl]vinyl]-1-tetrahydropyran-2-yl-indazol-6-yl]thioalkyl benzamide 107a (250 mg, 408.65 μmol) was dissolved in dichloromethane (12 mL) and trifluoroacetic acid (3 mL); the mixture was stirred at 25°C to react for 16 h. The reaction mixture was poured into water (50 mL), adjusted with the saturated aqueous solution of sodium carbonate to the pH of 10 and extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-3-fluoro-2-({3-[(E)-2-{6-[2-(pyrrolidin-1-yl)ethyl]pyridin-2-yl}vinyl]-1H-indazol-6-yl}thio)benzamide I-128 (19.34 mg, 36.65 μmol, yield: 8.97 %).

The product was verified by LCMS, F-NMR and H-NMR.

For MS-ESI, the calculated value was $[M+H]^+$ 528.2, and the measurement was 528.1.

$^1$H NMR (400MHz,$CD_3OD$): δ (ppm) 8.01 (d, $J$ = 8.4 Hz, 1H), 7.85 (d, $J$ = 16.8 Hz, 1H), 7.73 (t, $J$ = 8.0 Hz, 1H), 7.56-7.50 (m, 3H), 7.39 (s, 1H), 7.32-7.27 (m, 2H), 7.20 (d, $J$ = 7.6 Hz, 1H), 7.13 (dd, $J$ = 8.8, 1.6 Hz, 1H), 3.34-3.31 (m, 2H), 3.08-3.05 (m, 2H), 3.01-2.96 (m, 2H), 2.77-2.66 (m, 4H), 1.90-1.82 (m, 4H), 1.13 (t, $J$ = 7.2 Hz, 3H). $^{19}$F NMR (400MHz, $CD_3OD$): δ (ppm) -105.89.

I-129

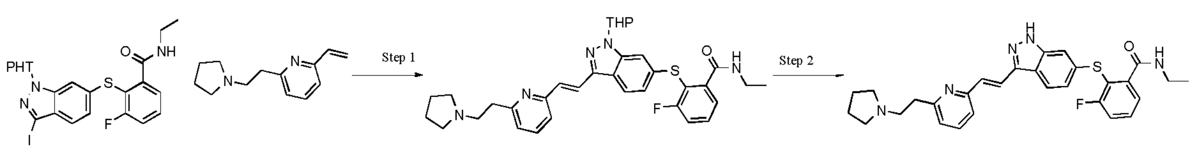


80a 102f 108a I129

Step 1

At room temperature, N-ethyl-3-fluoro-2-(3-iodo-1-tetrahydropyran-2-yl-indazol-6-yl)thioalkyl benzamide 80a (500 mg, 951.70 μmol) was dissolved in 1,2-dioxane (4 mL), followed by the addition of 2-(2-pyrrolidin-1-ylethyl)-6-vinylpyridine 102f (231 mg, 1.14 mmol), tris(o-methylphenyl)phosphine (1.3 g, 1.14 mmol), palladium dichloride (75 mg, 422.95 μmol) and triethylamine (289 mg, 2.86 mmol). The reaction system was stirred at 90°C under the protection of nitrogen to react for 16 h. The reaction mixture was poured into water (50 mL) and extracted with ethyl acetate (30 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/absolute methanol = 10/1) to obtain a yellow solid product N-ethyl-3-fluoro-2-[3-[(trans)-2-[6-(2-pyrrolidin-1-ylethyl)-2-pyridyl]vinyl]-1-tetrahydropyran-2-yl-indazol-6-yl]thioalkylbenzamide 108a (300 mg, 500.20 μmol, yield: 52.56 %).

The product was verified by LCMS.

For MS-ESI, the calculated value was $[M+H]^+$ 600.3, and the measurement was 600.1.

Step 2

N-ethyl-3-fluoro-2-[3-[(trans)-2-[6-(2-pyrrolidin-1-ylethyl)-2-pyridyl]vinyl]-1-tetrahydropyran-2-yl-indazol-6-yl] thioalkyl benzamide 108a (300 mg, 500.20 μmol) was dissolved in dichloromethane (12 mL) and trifluoroacetic acid (3 mL); the mixture was stirred at 25°C to react for 16 h. The reaction mixture was poured into water (50 mL), adjusted with 2 mol/L aqueous solution of sodium hydroxide to the pH of 10 and extracted with dichloromethane (50 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-ethyl-3-fluoro-2-({3-[(E)-2-{6-[2-(pyrrolidin-1-yl)ethyl]pyridin-2-yl}vinyl]-1H-indazol-6-yl}thio)benzamide I-129 (62.68 mg, 121.56 μmol, yield: 24.30 %)

The product was verified by LCMS, F-NMR and H-NMR.

For MS-ESI, the calculated value was $[M+H]^+$ 516.2, and the measurement was 516.1.

$^1$H NMR (400MHz,$CD_3OD$): δ (ppm) 8.01 (d, *J* = 8.4 Hz, 1H), 7.85 (d, *J* = 16.8 Hz, 1H), 7.73 (t, *J* = 8.0 Hz, 1H), 7.56-7.50 (m, 3H), 7.39 (s, 1H), 7.32-7.29 (m, 2H), 7.20 (d, *J* = 7.6 Hz, 1H), 7.13 (dd, *J* = 8.8, 1.6 Hz, 1H), 3.30-3.28 (m, 2H), 3.09-3.05 (m, 2H), 3.01-2.96 (m, 2H), 2.72 (s, 4H), 1.86 (t, *J* = 3.6 Hz, 4H), 1.13 (t, *J* = 7.2 Hz, 3H).

$^{19}$F NMR (400MHz, $CD_3OD$): δ (ppm) -104.67.

I-133

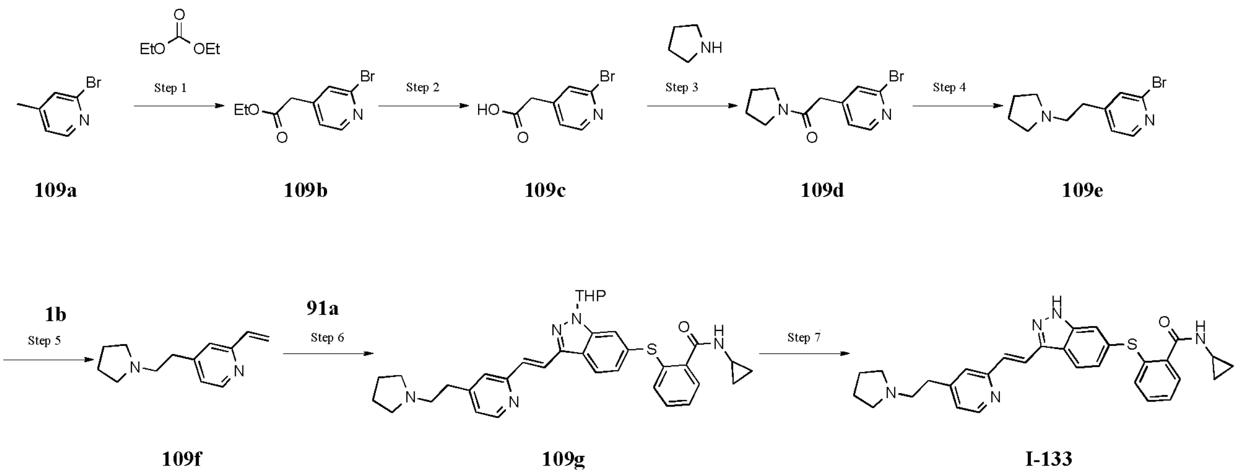


109a 109b 109c 109d 109e

109f 109g I-133

Step 1

Under the protection of nitrogen at room temperature, 2-bromo-4-methylpyridine 109a (40 g, 232.53 mmol) was dissolved in tetrahydrofuran (1.2 L); the mixture was cooled to -70°C, followed by the addition of lithium diisopropylamide (2 mol/L, 174.40 mL, 348.80 mmol). The reaction system was stirred at -70°C to react for 1 h, diethyl carbonate (33 g, 279.03 mmol) was slowly added at -70°C, and the reaction system was stirred at -70°C to react for 4 h. The reaction mixture was poured into saturated aqueous solution of ammonium chloride (1000 mL) and extracted with ethyl acetate (500 mL × 3), and the organic phases were combined, washed with saturated brine (1000 mL), dried over anhydrous sodium sulfate, and

concentrated under reduced pressure to obtain a crude product. The crude product was purified by the column chromatography (petroleum ether/ethyl acetate = 50/1-10/1) to obtain a yellow oily product 2-(2-bromo-4-pyridyl)ethyl acetate109b (35 g, 143.39 mmol, yield: 61.67 %).

The product was verified by LCMS and H-NMR.

For MS-ESI, the calculated value was $[M+H]^+$ 244.0/246.0, and the measurement was 243.9/245.9.

$^1$H NMR (400MHz, $CDCl_3$): δ (ppm) 8.32 (d, *J*= 5.2 Hz, 1H), 7.45 (d, *J*= 0.8 Hz, 1H), 7.21 (dd, *J*= 5.2, 1.2 Hz, 1H), 4.19 (q, *J*= 7.2 Hz, 2H), 3.59 (s, 2H), 1.28 (t, *J*= 7.2 Hz, 3H).

Step 2

At room temperature, 2-(2-bromo-4-pyridyl)ethyl acetate 109b (35 g, 143.39 mmol) was dissolved in tetrahydrofuran (150 mL), methanol (350 mL) and water (150 mL), followed by the addition of lithium hydroxide (10 g, 430.18 mmol). The reaction system was stirred at 25 °C to react for 2 h. Tetrahydrofuran was removed by rotary evaporation; the mixture was poured into water (500 mL), adjusted with 1N dilute hydrochloric acid to the pH of 3, and then extracted with ethyl acetate (300 mL × 3); and the organic phases were combined, dried over anhydrous sodium sulfate, and concentrated under reduced pressure to obtain a crude product 2-(2-bromo-4-pyridyl)acetic acid 109c (25 g, 115.72 mmol, yield: 80.70 %).

The product was verified by LCMS and H-NMR.

For MS-ESI, the calculated value was $[M+H]^+$ 216.0/218.0, and the measurement was 215.9/217.9.

$^1$H NMR (400MHz, $CDCl_3$): δ (ppm) 8.30 (d, *J*= 4.8 Hz, 1H), 7.47 (s, 1H), 7.23 (d, *J*= 4.8 Hz, 1H), 3.59 (s, 2H).

Step 3

At room temperature, 2-(2-bromo-4-pyridyl) acetic acid 109c (25 g, 115.72 mmol) was dissolved in N,N-dimethylformamide (250 mL), followed by the addition of tetrahydropyrrole (10 g, 138.87 mmol),N,N-diisopropylethylamine (45 g, 347.17 mmol),1-hydroxybenzotriazole (23 g, 173.58 mmol) and 1-(3-dimethylaminopropyl)-3-ethylcarbodiimide hydrochloride (33 g, 173.59 mmol). The reaction system was stirred at 25°C to react for 16 h. The reaction mixture was poured into water (800 mL) and extracted with ethyl acetate (300 mL × 3), and the organic phases were combined, washed with saturated brine (500 mL), dried over anhydrous sodium sulfate, and concentrated under reduced pressure to obtain a yellow oily crude product 2-(2-bromo-4-pyridyl)-1-pyrrolidin-1-ylethanone 109d (16 g, 59.45 mmol, yield: 51.37 %).

The product was verified by LCMS and H-NMR.

For MS-ESI, the calculated value was $[M+H]^+$ 269.0/271.0, and the measurement was 269.0/271.0.

$^1$H NMR (400MHz, $CDCl_3$): δ (ppm) 8.30 (d, *J*= 5.2 Hz, 1H), 7.43 (s, 1H), 7.21 (d, *J*= 5.2 Hz, 1H), 3.61 (s, 2H), 3.50 (t, *J*= 6.8 Hz, 2H), 3.45 (t, *J*= 6.8 Hz, 2H), 2.02-1.96 (m, 2H), 1.92-1.85 (m, 2H).

Step 4

At room temperature, 2-(2-bromo-4-pyridyl)-1-pyrrolidin-1-ylethanone 109d (8 g, 29.72 mmol) was dissolved in tetrahydrofuran (80 mL), followed by the addition of borane dimethyl sulfide complex (10 mol/L, 5.94 mL, 59.44 mmol). The reaction system was stirred at 70 °C to react for 3 h. Then, methanol (50 mL) was added, and the mixture was refluxed for 6 h. The reaction mixture was spin-dried, poured into water (200 mL) and extracted with ethyl acetate (50 mL × 3), and the organic phases were combined, washed with saturated brine (30 mL), dried over anhydrous sodium sulfate, and concentrated under reduced pressure to obtain a yellow oily crude product 2-bromo-4-(2-pyrrolidin-1-ylethyl)pyridine 109e (1.9 g, 7.45 mmol, yield: 25.05 %).

The product was verified by LCMS and H-NMR.

For MS-ESI, the calculated value was $[M+H]^+$ 255.0/257.0, and the measurement was 255.0/257.0.

$^1$H NMR (400MHz, $CDCl_3$): δ (ppm) 8.33 (d, *J*= 5.2 Hz, 1H), 7.42 (s, 1H), 7.22 (dd, *J*= 4.8, 1.2 Hz, 1H), 3.30-3.19 (m, 8H), 2.18-2.11 (m, 4H).

Step 5

At room temperature, 2-bromo-4-(2-pyrrolidin-1-yl)pyridine 109e (2 g, 7.45 mmol) was dissolved in N,N-dimethylformamide (20 mL), followed by the addition of potassium vinyltrifluoroborate (1.2 g, 8.94 mmol), triethylamine (2.3 g, 22.34 mmol) and [1,1'-bis(diphenylphosphino)ferrocene]dichloropalladium (II) (200 mg, 7.45 mmol). The reaction system was stirred at 90 °C to react for 16 h under the protection of nitrogen. The reaction mixture was poured into water (30 mL) and extracted with ethyl acetate (30 mL × 3), and the organic phases were combined, washed with saturated brine (50 mL), dried over anhydrous sodium sulfate, and concentrated under reduced pressure to obtain a crude product. The crude

product was purified by the silica gel preparative plate (dichloromethane/methanol = 10/1) to obtain a brown oily product 4-(2-pyrrolidin-1-ylethyl)-2-vinylpyridine 109f (0.3 g, 1.48 mmol, yield: 19.92 %).

The product was verified by LCMS and H-NMR.

For MS-ESI, the calculated value was $[M+H]^+$ 203.1, and the measurement was 203.2.

$^1$H NMR (400MHz, $CDCl_3$): δ (ppm) 8.45 *(d, J*= 5.2 Hz, 1H), 7.19 (s, 1H), 7.02 (d, *J* = 5.2 Hz, 1H), 6.80 (dd,*J* = 17.6, 10.8 Hz, 1H), 6.20 (dd, *J* = 17.6, 1.2 Hz, 1H), 5.47 (dd, *J*= 10.8,1.2 Hz, 1H), 2.87-2.82 (m, 2H), 2.78-2.74 (m, 2H), 2.63 (s, 4H), 1.85-1.82 (m, 4H).

Step 6

At room temperature, 4-(2-pyrrolidin-1-ylethyl)-2-vinylpyridine 109f (290 mg, 1.43 mmol) was dissolved in 1,4-dioxane (5 mL), followed by the addition of N-cyclopropyl-2-(3-iodo-1-tetrahydropyran-2-yl-indazol-6-yl)thioalkyl benzamide 91a (745 mg, 1.43 mmol), triethylamine (725 mg, 7.17 mmol), tris(o-methylphenyl)phosphine (436 mg, 1.43 mmol) and palladium chloride (127 mg, 716.78 μmol). The reaction system was stirred at 90 °C to react for 16 h under the protection of nitrogen. The reaction mixture was poured into water (10 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the silica gel preparative plate (dichloromethane/methanol = 10/1) to obtain a yellow solid product N-cyclopropyl-2-[3-[(trans)-2-[4-(2-pyrrolidin-1-ylethyl)-2-pyridyl]vinyl]-1-tetrahydropyran-2-yl-indazol-6-yl]thioalkylbenzamide 109 g (150 mg, 252.62 μmol, yield: 17.62 %).

The product was verified by LCMS.

For MS-ESI, the calculated value was $[M+H]^+$ 594.3, and the measurement was 594.2.

Step 7

At room temperature, N-cyclopropyl-2-[3-[(trans)-2-[4-(2-pyrrolidin-1-ylethyl)-2-pyridyl]vinyl]-1-tetrahydropyran-2-yl-indazol-6-yl]thioalkyl benzamide 109 g (150 mg, 252.62 μmol) was dissolved in dichloromethane (12 mL), followed by the addition of trifluoroacetic acid (3 mL). The reaction system was stirred at 25 °C to react for 2 h. The reaction mixture was poured into water (20 mL), adjusted with 2 mol/L aqueous solution of sodium hydroxide to the pH of 9-10 and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-2-({3-[(E)-2-{4-[2-(pyrrolidin-1-yl)ethyl]pyridin-2-yl}vinyl]-1H-indazol-6-yl}thio)benzamide I-133 (34.4 mg, 67.57 μmol, yield: 26.75 %).

The product was verified by LCMS and H-NMR.

For MS-ESI, the calculated value was $[M+H]^+$ 510.2, and the measurement was 510.3.

$^1$H NMR (400MHz, $CD_3OD$): δ (ppm) 8.43 (d, *J*= 5.2 Hz, 1H), 8.07 (d, *J* = 8.4 Hz, 1H), 7.87 (d, *J* = 16.4 Hz, 1H), 7.59 (dd, *J* = 18.0, 16.0 Hz, 3H), 7.46-7.44 (m, 1H), 7.35-7.32 (m, 2H), 7.27- 7.25 (m, 1H), 7.22-7.18 (m, 2H), 2.95-2.91 (m, 2H), 2.87-2.80 (m, 2H), 2.80-2.77 (m, 1H), 2.67 (s, 4H), 1.86-1.85 (m, 4H), 0.77 -0.72 (m, 2H), 0.55-0.51 (m, 2H).

I-135

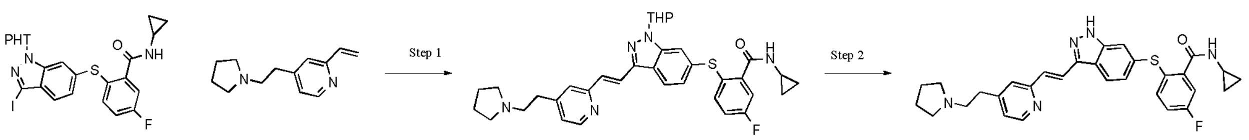


42a 109f 110a I-135

Step 1

At room temperature, 4-(2-pyrrolidin-1-ylethyl)-2-vinylpyridine 109f (130 mg, 642.63 μmol) was dissolved in 1,4-dioxane (5 mL), followed by the addition of N-cyclopropyl-5-fluoro-2-(3-iodo-1-tetrahydropyran-2-yl-indazol-6-yl)thioalkyl benzamide 42a (345 mg, 642.63 μmol), triethylamine (325 mg, 3.21 mmol) and c(2-dicyclohexylphosphino-2',4',6'-tri-isopropyl-1,1'-biphenyl)(2'-amino-1,1'-biphen-2-yl)palladium(II) methanesulfonate (54 mg, 64.26 μmol). The reaction system was stirred at 90 °C to react for 16 h under the protection of nitrogen. The reaction mixture was poured into water (10 mL) and extracted with ethyl acetate (10 mL × 3), and the organic phases were combined, washed with saturated brine (30 mL), dried over anhydrous sodium sulfate, and concentrated under reduced pressure to obtain a crude product. The crude product was purified by the silica gel preparative plate (dichloromethane/methanol = 10/1) to obtain a yellow solid product

N-cyclopropyl-5-fluoro-2-[3-[(trans)-2-[4-(2-pyrrolidin-1-ylethyl)-2-pyridyl]vinyl]-1-tetrahydropyran-2-yl-indazol-6-yl]thioalkylbenzamide 110a (200 mg, 326.92 μmol, yield: 50.87 %).

The product was verified by LCMS.

For MS-ESI, the calculated value was $[M+H]^+$ 612.3, and the measurement was 612.1.

Step 2

At room temperature, N-cyclopropyl-5-fluoro-2-[3-[(trans)-2-[4-(2-pyrrolidin-1-ylethyl)-2-pyridyl]vinyl]-1-tetrahydropyran-2-yl-indazol-6-yl]thioalkyl benzamide 110a (200 mg, 326.92 μmol) was dissolved in dichloromethane (12 mL), followed by the addition of trifluoroacetic acid (3 mL). The reaction system was stirred at 25° C to react for 2 h. The reaction mixture was poured into water (20 mL), adjusted with 2 mol/L aqueous solution of sodium hydroxide to the pH of 9-10 and extracted with dichloromethane (30 mL × 3), and the organic phases were combined, washed with saturated brine (30 mL), dried over anhydrous sodium sulfate, and concentrated under reduced pressure to obtain a crude product. The crude product was purified by the high-pressure preparative chromatography to obtain a yellow solid product N-cyclopropyl-5-fluoro-2-({3-[(E)-2-{4-[2-(pyrrolidin-1-yl)ethyl]pyridin-2-yl}vinyl]-1H-indazol-6-yl}thio)benzamide I-135 (17.31 mg, 32.81 μmol, yield: 10.03 %).

The product was verified by LCMS, F-NMR and H-NMR.

For MS-ESI, the calculated value was $[M+H]^+$ 528.2, and the measurement was 528.3.

$^1H$ NMR (400MHz, $CD_3OD$): δ (ppm) 8.47 (d, *J* = 4.8 Hz, 1H), 8.04 (d, *J* = 8.8 Hz, 1H), 7.88 (d, *J* = 16.4 Hz, 1H), 7.63 (s, 1H), 7.57-7.49 (m, 1H), 7.40 (dd, *J* = 8.8, 5.2 Hz, 1H), 7.26-7.23 (m, 1H), 7.19-7.14 (m,1H), 3.30 (s, 1H), 3.16-3.14 (m, 1H), 3.04-3.02 (m, 2H), 2.79-2.74 (m, 1H), 2.01-1.94 (m, 2H), 0.76- 0.71 (m, 1H), 0.53 -0.49 (m, 2H).

$^{19}F$ NMR (400MHz, $CD_3OD$): δ (ppm) -115.43.

I-139

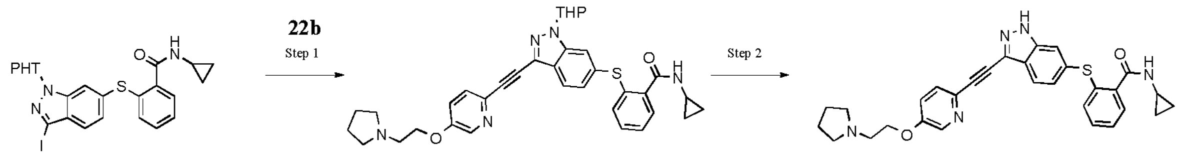


91a 111a I-139

Step 1

At room temperature, N-cyclopropyl-2-(3-iodo-1-tetrahydropyran-2-yl-indazol-6-yl)thioalkyl benzamide 91a (1.2 g, 2.31 mmol) was dissolved in N,N-dimethylformamide (10 mL), followed by the addition of 2-ethyl-5-(2-pyrrolidin-1-ylethoxy)pyridine 22b (500 mg, 2.31 mmol), triethylamine (2.3 g, 23.12 mmol), bis(triphenylphosphine)palladium(II) chloride (42 mg, 59.36 μmol) and cuprous iodide (88 mg, 462.37 μmol). The reaction system was stirred at 100°C under the protection of nitrogen to react for 2 h. The reaction mixture was poured into water (30 mL) and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the column chromatography (dichloromethane/methanol = 10/1) to obtain a brown solid product N-cyclopropyl-2-[3-[2-[5-(2-pyrrolidin-1-ylethoxy)-2-pyridyl]ethynyl]-1-tetrahydropyran-2-ylindazol-6-yl]thioalkylbenzamide 111a (290 mg, 477.16 μmol, yield: 20.64 %).

The product was verified by LCMS.

For MS-ESI, the calculated value was $[M+H]^+$ 608.3, and the measurement was 608.2.

Step 2

At room temperature, N-cyclopropyl-2-[3-[2-[5-(2-pyrrolidin-1-ylethoxy)-2-pyridyl]ethynyl]-1-tetrahydropyran-2-ylindazol-6-yl]thioalkyl benzamide 111a (340 mg, 559.43 μmol) was dissolved in dichloromethane (12 mL) and trifluoroacetic acid (3 mL); the reaction system was stirred at 25°C to react for 16 h. The reaction mixture was poured into water (50 mL), adjusted with the saturated sodium carbonate solution to the pH of 8 and extracted with dichloromethane (20 mL × 3), and the organic phases were combined, washed with saturated brine (30 mL), dried over anhydrous sodium sulfate, and concentrated under reduced pressure to obtain a crude product. The crude product was purified by the high-pressure liquid chromatography to obtain a yellow solid product N-cyclopropyl-2-{[3-(2-{5-[2-(pyrrolidin-1-yl)ethoxy]pyridin-2-yl}ethynyl)-1H-indazol-6-yl]thio}benzamide I-139 (12.13 mg, 23.16 μmol, yield: 4.14 %).

The product was verified by LCMS and H-NMR.

For MS-ESI, the calculated value was $[M+H]^+$ 524.2, and the measurement was 524.2.

$^1H$ NMR (400MHz, $CD_3OD$): δ (ppm) 8.32 (d, *J*= 3.2 Hz, 1H), 7.84 (d, *J* = 8.4 Hz, 1H), 7.70 (d, *J* = 8.4 Hz, 1H), 7.57 (s, 1H), 7.51 (dd, *J* = 8.8, 3.2 Hz, 1H), 7.47-7.44 (m, 1H), 7.37-7.33 (m, 2H), 7.29-7.27 (m, 1H), 7.19 (dd,*J* = 8.4, 1.2 Hz, 1H), 4.31 (t, *J* = 5.6 Hz, 2H), 3.15-3.13 (m, 2H), 2.87 (s, 4H), 2.79-2.74 (m, 1H), 1.92 (s, 4H), 0.77-0.72 (m, 2H), 0.54-0.50 (m, 2H).

EMBODIMENT 2

Testing of the Ability of Compounds to Inhibit VEGFR and EGFR Kinases

This assay directly monitored the ability of compounds to inhibit kinase phosphorylation by measuring non-phosphorylated and phosphorylated substrates in kinase reactions by using Mobility Shift Assays/IMAP™ analysis.

Table 2. Reagents and Instruments

| Reagent | Supplier | Catalog No. |
|---|---|---|
| FLT1 (VEGFR1) | Invitrogen | PR6731B |
| KDR (VEGFR2) | Carna | 08-191 |
| FLT4 (VEGFR3) | Invitrogen | PV4129 |
| EGFR | Carna | 08-115 |
| ATP | Sigma | 2383-5G |
| MgCl2 | Sigma | M2670-500 g |
| DTT | Sigma | D0632-10G |
| EGTA | Sigma | E3889-25G |
| DMSO | Sigma | D2650 |
| Staurosporine | MCE | HY-15141 |
| Plate Reader | Caliper LifeSciences | CaliperEZ Reader II |

Test Protocol and Procedures:

Add the kinase and DTT to 1x kinase buffer to form a 2.5x kinase solution; transfer 10 μL of 2.5x kinase solution to a 384-well reaction plate and add 1x kinase buffer to negative control wells. Incubate the plate for 10 min at room temperature; add FAM-labeled peptides, $MgCl_2$, ATP and other reagents to 1x kinase buffer to form a 2.5x substrate solution; transfer 10 μL of the above 2.5x substrate solution to the 384-well reaction plate; incubate the plate at 28°C for different times, and add 25 μL of stop solution to the 384-well reaction plate to stop the reaction. Read the conversion ratio data on CaliperEZ Reader II. Convert conversion rates into inhibition ratio data.

$$\%Inhibition = \frac{Max - Conversion}{Max - Min} \times 100\ \%$$

"Min" indicates the readout of the control well without the addition of kinase; and "Max" indicates the readout of the control well with the addition of DMSO. Fit $IC_{50}$ values with XLFit excel add-in version 5.4.0.8.

$$Y = Bottom + \frac{Top - Bottom}{1 + \frac{IC_{50}}{X}^{(LogIC_{50} - X)Hill\ Slope}}$$

X indicates the log value of compound concentration, and Y indicates the inhibition ratio ( %inhibition). The $IC_{50}$ results of the compounds of the present invention for VEGFR2 are shown in the table below:

Table 3. $IC_{50}$ results of the compounds for VEGFR2

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-1 | A | I-137 | B | I-38 | A | I-81 | C |
| I-10 | B | I-138 | B | I-39 | A | I-82 | A |
| I-100 | B | I-14 | B | I-4 | A | I-83 | A |
| I-101 | A | I-15 | B | I-40 | A | I-84 | B |
| I-102 | C | I-16 | B | I-41 | A | I-85 | B |
| I-104 | A | I-17 | B | I-42 | A | I-86 | A |
| I-106 | B | I-18 | B | I-43 | A | I-87 | D |
| I-107 | B | I-19 | B | I-44 | B | I-88 | B |
| I-108 | A | I-2 | A | I-46 | A | I-9 | B |
| I-109 | A | I-20 | C | I-5 | A | I-90 | B |
| I-11 | B | I-21 | C | I-55 | A | I-91 | B |
| I-110 | B | I-22 | C | I-59 | A | I-92 | B |
| I-111 | B | I-23 | C | I-6 | B | I-93 | B |
| I-112 | C | I-24 | C | I-60 | B | I-94 | B |
| I-113 | D | I-25 | D | I-61 | B | I-95 | B |
| I-114 | D | I-26 | D | I-67 | B | I-97 | B |
| I-115 | B | I-27 | D | I-68 | B | I-98 | B |
| I-116 | C | I-28 | D | I-69 | C | I-99 | B |
| I-117 | B | I-29 | D | I-7 | B | I-136 | B |
| I-118 | B | I-3 | A | I-73 | B | I-132 | B |
| I-119 | A | I-30 | D | I-74 | B | I-103 | A |
| I-120 | A | I-31 | D | I-75 | B | I-133 | B |
| I-121 | A | I-32 | B | I-76 | B | I-129 | C |
| I-122 | B | I-33 | B | I-77 | A | I-128 | C |
| I-123 | C | I-34 | B | I-78 | A | I-139 | C |
| I-124 | C | I-35 | B | I-79 | A | I-135 | C |
| I-13 | B | I-36 | B | I-8 | B | | |
| I-134 | B | I-37 | A | I-80 | A | | |

Here, for $IC_{50}$: A indicates 1 nM or below, B indicates from 1 nM to 10 nM or below, C indicates from 10 nM to 100 nM or below, and D indicates above 100 nM.

The $IC_{50}$ results of the compounds of the present invention for EGFR are shown in the table below:

Table 4. $IC_{50}$ results of the compounds for EGFR

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-1 | C | I-137 | D | I-38 | B | I-81 | C |
| I-10 | C | I-138 | D | I-39 | C | I-82 | C |
| I-100 | C | I-14 | C | I-4 | C | I-83 | C |
| I-101 | C | I-15 | C | I-40 | C | I-84 | C |
| I-102 | C | I-16 | C | I-41 | C | I-85 | C |
| I-104 | D | I-17 | C | I-42 | D | I-86 | C |

(continued)

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-106 | C | I-18 | C | I-43 | C | I-87 | D |
| I-107 | C | I-19 | D | I-44 | C | I-88 | C |
| I-108 | C | I-2 | C | I-46 | C | I-9 | C |
| I-109 | C | I-20 | C | I-5 | C | I-90 | C |
| I-11 | C | I-21 | C | I-55 | C | I-91 | C |
| I-110 | C | I-22 | D | I-59 | C | I-92 | C |
| I-111 | C | I-23 | C | I-6 | C | I-93 | C |
| I-112 | C | I-24 | C | I-60 | B | I-94 | C |
| I-113 | C | I-25 | C | I-61 | C | I-95 | C |
| I-114 | D | I-26 | D | I-67 | C | I-97 | C |
| I-115 | C | I-27 | C | I-68 | C | I-98 | C |
| I-116 | D | I-28 | A | I-69 | C | I-99 | C |
| I-117 | D | I-29 | D | I-7 | C | I-136 | D |
| I-118 | D | I-3 | C | I-73 | C | I-132 | C |
| I-119 | D | I-30 | C | I-74 | C | I-103 | C |
| I-120 | D | I-31 | D | I-75 | C | I-133 | D |
| I-121 | C | I-32 | C | I-76 | B | I-129 | D |
| I-122 | D | I-33 | C | I-77 | C | I-128 | D |
| I-123 | D | I-34 | C | I-78 | C | I-139 | D |
| I-124 | C | I-35 | C | I-79 | C | I-135 | D |
| I-13 | C | I-36 | C | I-8 | C | | |
| I-134 | D | I-37 | C | I-80 | C | | |

Here, for $IC_{50}$: A indicates 600 nM or below, B indicates from 600 nM to 6,000 nM or below, C indicates from 6,000 nM to 60,000 nM or below, and D indicates above 60,000 nM.

The $IC_{50}$ results of the compounds of the present invention for VEGFR1 are shown in the table below:

Table 5. $IC_{50}$ results of the compounds for VEGFR1

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-1 | A | I-15 | B | I-26 | D | I-37 | A |
| I-10 | B | I-16 | B | I-27 | D | I-38 | A |
| I-11 | B | I-17 | B | I-28 | D | I-39 | A |
| I-115 | B | I-18 | B | I-29 | D | I-4 | A |
| I-116 | C | I-19 | B | I-3 | A | I-40 | A |
| I-117 | B | I-2 | A | I-30 | D | I-44 | B |
| I-118 | C | I-20 | C | I-31 | D | I-5 | A |
| I-119 | B | I-21 | C | I-32 | B | I-6 | A |
| I-120 | B | I-22 | C | I-33 | B | I-7 | A |
| I-121 | B | I-23 | C | I-34 | B | I-8 | B |
| I-13 | B | I-24 | C | I-35 | B | I-9 | B |

(continued)

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-14 | B | I-25 | C | I-36 | D | I-97 | B |

Here, for $IC_{50}$: A indicates 1 nM or below, B indicates from 1 nM to 10 nM or below, C indicates from 10 nM to 100 nM or below, and D indicates above 100 nM.

The $IC_{50}$ results of the compounds of the present invention for VEGFR3 are shown in the table below:

Table 6. $IC_{50}$ results of the compounds for VEGFR3

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-1 | A | I-15 | B | I-26 | D | 1-37 | B |
| I-10 | A | I-16 | B | I-27 | D | I-38 | A |
| I-11 | B | I-17 | B | I-28 | D | I-39 | B |
| I-115 | C | I-18 | B | I-29 | D | I-4 | A |
| I-116 | C | I-19 | B | I-3 | A | I-40 | B |
| I-117 | B | I-2 | B | I-30 | D | I-44 | C |
| I-118 | C | I-20 | C | I-31 | D | I-5 | A |
| I-119 | C | I-21 | C | I-32 | B | I-6 | B |
| I-120 | B | I-22 | C | I-33 | B | I-7 | B |
| I-121 | A | I-23 | D | I-34 | B | I-8 | B |
| I-13 | A | I-24 | C | I-35 | B | I-9 | B |
| I-14 | B | I-25 | C | I-36 | B | I-97 | B |

Here, for $IC_{50}$: A indicates 1 nM or below, B indicates from 1 nM to 10 nM or below, C indicates from 10 nM to 100 nM or below, and D indicates above 100 nM.

The results show that the compounds provided by the present invention can have a kinase inhibition activity, for example, with the $IC_{50}$ (nM) of 100 or below for VEGFR1/2/3, or the $IC_{50}$ (nM) of 10 or below for VEGFR1/2/3, or the $IC_{50}$ (nM) of 1 or below for VEGFR1/2/3. Moreover, the compounds provided by the present invention are highly selective for EGFR inhibition.

EMBODIMENT 3

Testing of the Ability of Compounds to Inhibit VEGFR2 Phosphorylation in HUVEC Cells

VEGF is a highly potent growth factor capable of promoting angiogenesis and vascular permeability. Vascular endothelial growth factor receptor 2 (VEGFR2) is a primary receptor for VEGF-induced endothelial cell signaling. After ligand binding, VEGFR2 is autophosphorylated and activated. The main function of signaling from VEGFR2 is to induce vascularization during development and after tissue injury, and to bypass blocked blood vessels. Pathologically, VEGF and VEGFR2 are involved in tumor angiogenesis and vascular leakage. They are considered to be main participants in many different cancers and ocular diseases that accompany pathological neovascularization. This assay measured the ability of the compounds to inhibit VEGF-induced VEGFR2 autophosphorylation (pVEGFR2) in HUVEC cells by using ELISA.

Table 7. Reagents and Instruments

| Reagent or Instrument | Supplier | Item number or model number |
|---|---|---|
| Endothelial Cell Growth Base Media | R&D | 390598 |
| Endothelial Cell Growth Supplement (50X) | R&D | 390599 |
| Foetal bovine serum | Invitrogen | 10099141C |
| DPBS | Corning | 21-031-CV |

(continued)

| Reagent or Instrument | Supplier | Item number or model number |
|---|---|---|
| Trypsin-EDTA (0.05 %), phenol red | Invitrogen | 25300062 |
| Human VEGF-165 Recombinant Protein | CST | 48143 |
| Phosphatase Inhibitor Cocktail 3 | Sigma | P0044 |
| Phosphatase Inhibitor Cocktail 2 | Sigma | P5726 |
| PathScan® Phospho-VEGFR-2 (Tyr1175) Sandwich ELISA Antibody Pair | CST | 7824S |
| Protease Inhibitor | Roche | 4693159001 |
| 10X Cell Lysis Buffer | CST | 9803S |
| Tween® 20 | Sigma | P1379 |
| Bovine Serum Albumin (BSA) | Sigma | B2064-100G |
| TMB Substrate | CST | 7004P4 |
| STOP Solution | CST | 7002P4 |
| Microplate Reader | Molecular Devices | SpectraMax® 340 PC 384 |

Experimental Procedures:

Culture cells in a cell culture flask to reach a fusion degree of 60 %-80 % before cell inoculation. Dilute the cell suspension to $4.5\times10^6$ cells/mL with a pre-warmed complete medium. Add 80 μL of the cell suspension to all wells of a 96-well cell plate (Corning-3599); and incubate the plate overnight at 37 °C in the presence of 5 % $CO_2$. Dissolve the compound powder to 10 mM with DMSO and store it in a nitrogen cabinet. Compound dilution plate: Add a reference compound Ponatinib or a compound to be tested to a 96 V-well plate (Axygen-WIPP02280) at concentrations achieved as follows: performing 3-fold gradient dilution starting from 5 mM (5 μL of 10 mM compound solution plus 5 μL of DMSO) for a total of 8 concentration points. For the 100 % inhibition control group, the concentration of the reference compound Ponatinib in the V-well plate was 2.5 mM (3 μL of 10 mM compound solution plus 9 μL of DMSO). Transfer 20 μL of the diluted compound from the intermediate plate to a cell plate. Then, incubate the cell plate for 1 h at 37 °C in the presence of 5 % $CO_2$. After the compound was treated for 1 h, add 25 μL of 250 ng/mL human VEGF at a final concentration of 50 ng/mL to a 96-well plate; and incubate the plate for 5 min at 37 °C in the presence of 5 % $CO_2$, centrifuge the plate for 10 min at 3,000 rpm, remove the medium and wash the cells once with 300 μL of pre-chilled DPBS per well. Then, centrifuge the plate for 10 min at 4,000 rpm, remove DPBS, and add 110 μL of pre-chilled 1X cell lysis buffer per well to the 96-well plate. Shake the plate for 40 min at 4 °C. Then, freeze the plate in a refrigerator at -80°C. On the next day, remove the plate from the refrigerator at -80 °C, thaw the plate, centrifuge the plate for 10 min at 3500 rpm, and add 100 μL of the supernatant per well to an ELISA plate. Seal the plate and incubate it for 2 h at 37 °C. Wash the wells 4 times with 1x wash buffer. Add 100 μL of recombinant detection antibody per well. Seal the plate, incubate the plate for 1 h at 37 °C, and repeat the step of plate washing. Add 100 μL of secondary antibody per well. Seal the plate, incubate the plate for 30 min at 37 °C, and repeat the step of plate washing. Add 100 μL of TMB (protected from light) per well. Seal the plate, incubate the plate for 30 min at 37 °C, and add 100 μL of the stop solution per well. Gently shake for 30 sec, measure the absorbance with a SpectraMax® 340 PC 384 Absorbance Microplate Reader within 30 min, and read an optical density value at 450 nm. Conduct data analysis using XL Fit software:

$$\text{Inhibition ratio}(\ \%\text{Inhibition}) = \frac{\text{Mean value of negative control group} - \text{Sample value}}{\text{Mean value of negative control group} - \text{Mean value of positive control group}}$$

"Human VEGF-stimulated cells with DMSO" was taken as the negative control group (0% inhibition ratio), "Human VEGF-stimulated cells with 5 μM Ponatinib" was taken as the positive control group (100 % inhibition ratio), and all other values were expressed as percentages (inhibition ratio) of this control.

Conduct curve fitting using the 4 Parameter Logistic Model or Sigmoidal Dose-Response Model:

$$Y = Bottom + \frac{Top - Bottom}{1 + 10^{(LogIC_{50} - X)\times Hill\ Slope}}$$

X indicating the log value of compound concentration, Y indicating the inhibition ratio (%inhibition), Top indicating the readout of the negative control wells, and bottom indicating the readout of the positive control well.
The $IC_{50}$ results of the compounds of the present invention for pVEGFR2 are shown in the table below:

Table 8. $IC_{50}$ results of the compounds for pVEGFR2

| No. | $IC_{50}$ (nM) | No. | $IC_{50}$ (nM) | No. | $IC_{50}$ (nM) | No. | $IC_{50}$ (nM) |
|---|---|---|---|---|---|---|---|
| I-100 | A | I-34 | A | I-6 | B | I-86 | A |
| I-104 | A | I-40 | A | I-61 | A | I-88 | A |
| I-107 | A | I-41 | A | I-67 | A | I-9 | A |
| I-109 | B | I-42 | A | I-77 | A | I-90 | A |
| I-110 | B | I-43 | A | I-78 | A | I-97 | A |
| I-113 | C | I-44 | B | I-80 | A | | |
| I-20 | B | I-55 | A | I-83 | A | | |
| I-3 | A | I-59 | A | I-84 | A | | |

Here, for $IC_{50}$ A indicates 1 nM or below, B indicates from 1 nM to 5 nM or below, and C indicates above 5nM.

EMBODIMENT 4

Solubility Assay for Compounds

Table 9. Reagents and Instruments

| Reagent or Instrument | Supplier | Catalog or model number |
|---|---|---|
| Trisodium citrate dihydrate | Sinopharm Chemical Reagent Co., Ltd. | 10019418 |
| Citric acid monohydrate | Sinopharm Chemical Reagent Co., Ltd. | 10007118 |
| Methanol | ASTOON | AT-3292 |
| Acetonitrile | ASTOON | AT-3022 |
| Verapamil hydrochloride | Aladdin | V111249 |
| Glibenclamide | Aladdin | G127198 |
| Water | Self-prepared deionized water | - |

Experiment Protocol and Procedures:

Thermodynamic solubility: accurately weigh 3 portions of about 2 mg of the compound, and add appropriate volumes of buffers with the pHs of 2.0, 6.5, and 7.4 respectively to obtain a solution with a concentration of 2 mg/mL. After sonication for 10 min, fix them on a shaker and shaking for 8 h at room temperature. After shaking, sonicate for 10 min and centrifuge for 15 min at 13,000 rpm. Pipette 0.1 mL of supernatant into a new tube, shake and rinse it with 5 mL, and discard the liquid. Transfer 0.5 mL of the supernatant again to a new tube, centrifuge it for 15 min at 13,000 rpm, then collect the supernatant (if necessary, dilute with water) and analyze it by LC-MS. The sample concentrations at 3~5 points are subjected to fitting and reticle for quantification.
The solubility of the compound of the present invention is shown in the table below:

Table 10. Solubility of Compounds

| No. | Solubility (μg/mL) | No. | Solubility (μg/mL) | No. | Solubility (μg/mL) | No. | Solubility (μg/mL) |
|---|---|---|---|---|---|---|---|
| I-1 | 14.2 | I-21 | 0.9 | I-43 | 71.6 | I-83 | 79.6 |
| I-100 | 209.0 | I-22 | 57.4 | I-44 | 563.5 | I-84 | 599.0 |
| I-104 | 2178.0 | I-23 | 859.0 | I-5 | 177.0 | I-86 | 1125.5 |
| I-106 | 1580.0 | I-25 | 216.0 | I-6 | 226.0 | I-88 | 546.0 |

(continued)

| No. | Solubility (μg/mL) | No. | Solubility (μg/mL) | No. | Solubility (μg/mL) | No. | Solubility (μg/mL) |
|---|---|---|---|---|---|---|---|
| I-109 | 1948.0 | I-26 | 142.0 | I-67 | 118.0 | I-9 | 363.0 |
| I-11 | 866.0 | I-3 | 0.5 | I-7 | 294.0 | I-90 | 31.9 |
| I-12 | 2.0 | I-31 | 542.0 | I-76 | 1774.0 | I-91 | 1648.0 |
| I-14 | 46.8 | I-32 | 327.0 | I-77 | 1940.0 | I-94 | 31.8 |
| I-15 | 78.8 | I-33 | 57.7 | I-78 | 27.6 | I-97 | 318.7 |
| I-16 | 27.2 | I-34 | 19.3 | I-79 | 80.4 | I-98 | 456.0 |
| I-17 | 278.0 | I-35 | 26.6 | I-8 | 1870.0 | I-99 | 341.0 |
| I-2 | 28.8 | I-36 | 16.2 | I-80 | 199.0 | | |
| I-20 | 128.0 | I-4 | 413.0 | I-82 | 294.0 | | |

The results showed that the compounds provided by the present invention may have the advantage of improved solubility, for example, with the thermodynamic solubility of 10 μg/mL or above, or the thermodynamic solubility of 100 μg/mL or above, or the thermodynamic solubility of 1000 μg/mL or above. The thermodynamic solubility of the positive compound Axitinib was less than 2 μg/mL, which was in line with what has been reported in the literature.

EMBODIMENT 5

Detection of Binding Capacity of Compounds to Melanin

Pigmented eye tissues contain melanin in intracellular melanosomes. Drugs bind to melanin to varying degrees, ranging from non-binding to extensive binding. Therefore, melanin binding is an important feature that affects drug delivery and biodistribution in the eye. The binding capacity of a compound to the melanin must be considered in the development of ophthalmic drugs.

Reagent:

Melanin was derived from the melanin of cuttlefish (Sepia officinalis) and was commercially available from Sigma Aldrich (St Louis, MO).

Experimental Procedures:

First, prepare 1 mg/mL melanin suspension with PBS (pH 7.4). As a washing step for the matrix, centrifuge the suspended particles for 5 min at 3000 rpm. Resuspend the resulting particles in a fresh PBS buffer, heat to 37 °C over continuous shaking and incubate it for 1 h. Accurately pipette 2 μL of the test stock solution (0.1 mM) into 98 μL of melanin suspension (1 mg/mL) to achieve a final incubation concentration of 2 μM. Similarly, accurately pipette 2 μL of the stock solution (0.1 mM) to 98 μL of PBS buffer (containing 0.1 % BSA) as a blank control sample. Incubate the samples in a shaker (80-90 rpm) for 2 h at 37°C. Then, centrifuge the samples at room temperature. Pipette 50 μL of the supernatant and add 50 μL of precipitant (containing an internal standard). After protein precipitation of all the above samples, centrifuge the samples to collect the supernatants, dilute the supernatants with water (if necessary) and inject them for analysis. At the same time, measure the positive controls, including the high-binding compounds Sunitinib and chloroquine and the low-binding compound celecoxib. Establish an LC-MS/MS method for determining compound concentrations, and calibrate the peak area ratio of analyte/internal standard (Analyte/AIS) in the measured sample by the dilution factor to take it as an alternative concentration for the sample for data processing. The binding rate ($F_b$) of a compound in melanin was calculated as:

$$Fb = 100 - \frac{C_{test}}{C_{control}} \times 100\ \%$$

$C_{test}$ was the concentration of the compound in the melanin-incubated sample or the peak area ratio of the test compound to the internal standard. $C_{control}$ was the concentration of the compound in the blank control sample or the peak area ratio of the test compound to the internal standard. Percentage of free drug ($F_u$ %) = 100 - $F_b$

The binding rates of the compounds of the present invention to melanin are shown in the table below:

Table 11. Binding rate of compounds to melanin

| No. | Fu % | No. | Fu % | No. | Fu % | No. | Fu % |
|---|---|---|---|---|---|---|---|
| I-1 | C | I-20 | B | I-40 | B | I-79 | C |
| I-100 | C | I-21 | B | I-41 | C | I-8 | B |
| I-104 | C | I-22 | B | I-42 | B | I-80 | B |
| I-106 | C | I-23 | B | I-43 | B | I-82 | B |
| I-107 | B | I-25 | C | I-44 | B | I-83 | C |
| I-109 | C | I-26 | B | I-5 | B | I-84 | C |
| I-11 | B | I-27 | B | I-51 | B | I-86 | B |
| I-115 | B | I-28 | C | I-52 | C | I-88 | C |
| I-116 | B | I-3 | B | I-53 | C | I-9 | B |
| I-117 | B | I-31 | B | I-55 | B | I-90 | B |
| I-118 | A | I-32 | B | I-59 | B | I-91 | B |
| I-13 | B | I-33 | C | I-6 | B | I-93 | B |
| I-14 | B | I-34 | C | I-61 | B | I-94 | B |
| I-15 | B | I-35 | B | I-67 | C | I-97 | B |
| I-16 | A | I-36 | C | I-7 | C | I-98 | C |
| I-17 | B | I-37 | B | I-73 | B | I-99 | C |
| I-18 | B | I-38 | B | I-76 | C | | |
| I-19 | B | I-39 | B | I-77 | B | | |
| I-2 | B | I-4 | C | I-78 | B | | |

Here, for Fu %: A indicates below or equal to 1 %, B indicates from 1 % to 5 %, and C indicates above 5 %.

The test results provide the percentage of the free drug of the compound of the present invention (which does not bind to the melanin and is able to interact with receptors in the eye tissues).

EMBODIMENT 6

Testing of Compound Distribution in Plasma and Ocular Tissues after Single Ocular Dose in Dutch Belted Rabbits Age-related macular degeneration and diabetic retinopathy are associated with neovascularization in the posterior eye segment Due to the corneal and conjunctival barrier, one of the main problems with topical eye drops used to treat the posterior eye segment disorders is the low-efficiency delivery of the drug to the posterior eye segment. Therefore, it is important to measure the distribution of the compound in an ophthalmic formulation in several major ocular tissues. Increasing the concentration of the compound in the posterior eye segment may significantly increase the efficacy of the drug to improve clinical outcomes in patients with age-related macular degeneration and diabetic retinopathy. Dutch belted rabbits were used in this experiment. After the ocular administration of the compound-containing eye drops, the plasma and the main tissues of the eye were collected to determinate the concentration of the analyte, and the distribution and pharmacokinetic characteristics of the compound in the rabbit eye tissues were studied.

Experimental Protocol:

In this experiment, male Dutch belted rabbits weighed 1.5-2.5 kg were purchased from Pizhou Dongfang Breeding Co., Ltd. Any operation on the laboratory animals in this experiment was in compliance with the relevant SOP requirements of the experimental operation of 3D BioOptima, Suzhou, China, and had been approved by the Institutional Animal Care and Use Committee (IACUC) of 3D BioOptima. Preparation of formulation: Weigh about 1 mg of the test sample into a glass bottle, add an eye drop formulation vehicle (Tris-HCl buffer (0.05 mol/L): Tween 80: 0.5 % CMC-Na = 49:1:50), Tris-HCl buffer and Tween 80 in order based on converted values, then conduct vortexing and sonication, then add 0.5 % CMC-Na for vortexing and sonication or cutting if necessary, to prepare a 1 mg/mL dose formulation in each case. The prepared dose formulations were freshly prepared on the day of administration, samples were retained and the

actual concentration of the dose formulation was determined.

On the day of the experiment, animals were administrated at 1 mg/mL (0.05 mg/eye, 50 μL/eye). Each group included 2 animals. At 0.5 h, 4 h, or 8 h post-dose, the animals were sacrificed in an appropriate manner to collect blood samples and ocular tissues (i.e., cornea, retina, choroid, and sclera). One (1) mL of the collected whole blood was placed in an EDTA-$K_2$ anticoagulant tube, reversed several times to mix well, and centrifuged (at 1500~1600 g) for 10 min within 30 minutes, and the plasma was separated to obtain plasma samples, which were then stored in a -80°C environment for biological sample analysis. After collection, ocular tissues were washed with pre-chilled saline, dried up and stored in a refrigerator at -80°C or cryopreserved under equivalent conditions for subsequent tissue homogenation for sample analysis. For the ocular tissues to be homogenated, all or some of the tissues were selected, weighed accurately, and homogenated by adding 20 % methanol-water at a homogenation ratio of 1:10, and the tissue homogenate was stored in a refrigerator at -80°C for sample analysis. The concentration of each analyte in the rabbit ocular tissues and plasma obtained in this experiment was determined by LC-MS/MS. The matrix was Dutch rabbit plasma (EDTA-$K_2$ anticoagulated) and rabbit ocular tissues (1:10, 20 % methanol-water). The left and right eyes were tested separately. BLOQ (lower limit of quantification) was the lowest concentration of analyte quantified with acceptable precision and accuracy. The compounds were each analyzed after being diluted at 1:10 with a homogenate prior to analysis, and if the concentration was less than 1 ng/mL, the BLOQ was labeled.

Experimental results:

The test results showed that the compounds of the present invention (with pVEGFR $IC_{50}$ < = 5 nM, as shown in Table 8) were able to maintain sufficient drug concentration in the posterior eye segment (choroid: 27.2 ng/g to 137 ng/g; retina: 14.5 ng/g to 71.9 ng/g) to bind to relevant receptors targeting posterior eye segment tissues, which increased the bioavaialbility in the posterior eye segment. Moreover, they were rapidly degraded in plasma (2.4 ng/mL to BLOQ) with a low systemic exposure level. Comparatively, axitinib had a low exposure level in the posterior eye segment (choroid: 17.2 ng/g to 24 ng/g; retina: below BLOQ).

The foregoing detailed description is provided by way of explanation and examples, and is not intended to limit the scope of the appended claims. Various changes of the embodiments listed in the present invention until now would be obvious to those of ordinary skills in the art, and should be kept within the scope of the appended claims and the equivalents thereof.

## Claims

1. A compound of formula (I),

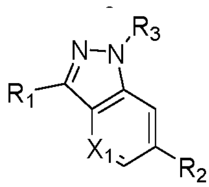 formula (I),

wherein, said $R_1$ contains a substituted cyclic structure;
$R_2$ contains an optionally substituted substituent;
$R_3$ contains an optionally substituted substituent;
$X_1$ is an optionally substituted atom;
or a pharmaceutically acceptable prodrug, a pharmaceutically active metabolite, and/or a pharmaceutically acceptable salt of said compound.

2. The compound according to claim 1, wherein said cyclic structure of said $R_1$ is substituted by a substituent containing basic nitrogen atom.

3. The compound according to any one of claims 1-2, wherein said cyclic structure of said $R_1$ is substituted by a substituent selected from the group consisting of: optionally substituted amino, and optionally substituted aliphatic heterocyclyl containing nitrogen atom.

4. The compound according to any one of claims 1-3, wherein said $R_1$ contains

$(R_{1-0})_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein $R_{1-0}$, $R_{1-1}$, $R_{1-2}$, $R_{1-3}$, $R_{1-4}$, and $R_{1-5}$ each independently contain an optionally substituted substituent, and nr0, nr1, nr2, nr3, nr4 and nr5 are each independently selected from 0 or a larger number.

**5.** The compound according to claim 4, wherein said $R_{1-0}$ is a chemical bond or selected from the group consisting of: optionally substituted alkenyl, optionally substituted alkynyl, and optionally substituted amino.

**6.** The compound according to any one of claims 4-5, wherein said $R_{1-0}$ is a chemical bond or selected from the group consisting of: optionally substituted ethenyl, optionally substituted ethynyl, and optionally substituted amino.

**7.** The compound according to any one of claims 4-6, wherein said nr0 is 0 or 1.

**8.** The compound according to any one of claims 4-7, wherein said $R_{1-1}$ is a chemical bond or selected from the group consisting of: optionally substituted acylamino, optionally substituted aryl, and optionally substituted heteroaryl.

**9.** The compound according to any one of claims 4-8, wherein said $R_{1-1}$ is a chemical bond or selected from the group consisting of: optionally substituted acylamino, optionally substituted phenyl, and optionally substituted heteroaryl.

**10.** The compound according to any one of claims 4-9, wherein said $R_{1-1}$ is a chemical bond or selected from the group consisting of: optionally substituted acylamino, optionally substituted phenyl, optionally substituted pyridyl and optionally substituted pyrazolyl.

**11.** The compound according to any one of claims 4-10, wherein said nr1 is 0 or 1.

**12.** The compound according to any one of claims 4-11, wherein said $R_{1-2}$ is a chemical bond or selected from the group consisting of: hydrogen, protium, deuterium, tritium, and optionally substituted hydroxyl.

**13.** The compound according to any one of claims 4-12, wherein said nr2 is 0 or 1.

**14.** The compound according to any one of claims 4-13, wherein said $R_{1-3}$ is a chemical bond or selected from the group consisting of: hydrogen, protium, deuterium, tritium, and optionally substituted alkyl.

**15.** The compound according to any one of claims 4-14, wherein said $R_{1-3}$ is a chemical bond or selected from the group consisting of: hydrogen, protium, deuterium, tritium, optionally substituted methyl, optionally substituted ethyl, and optionally substituted propyl.

**16.** The compound according to any one of claims 4-15, wherein said nr3 is 0 or 1.

**17.** The compound according to any one of claims 4-16, wherein said $R_{1-4}$ is a chemical bond or selected from the group consisting of: hydrogen, protium, deuterium, tritium, optionally substituted amino, and optionally substituted aliphatic heterocyclyl.

**18.** The compound according to any one of claims 4-17, wherein said $R_{1-4}$ is a chemical bond or selected from the group consisting of: hydrogen, protium, deuterium, tritium, optionally substituted amino, optionally substituted pyrryl, optionally substituted piperidyl, optionally substituted piperazinyl, and optionally substituted morpholinyl.

**19.** The compound according to any one of claims 4-18, wherein said nr4 is 0 or 1.

**20.** The compound according to any one of claims 4-19, wherein said $R_{1-5}$ is a chemical bond or selected from the group consisting of: hydrogen, protium, deuterium, tritium, and optionally substituted alkyl.

**21.** The compound according to any one of claims 4-20, wherein said $R_{1-5}$ is a chemical bond or selected from the group consisting of: hydrogen, protium, deuterium, tritium, optionally substituted methyl, and optionally substituted ethyl.

**22.** The compound according to any one of claims 4-21, wherein said nr5 is 0, 1 or 2.

**23.** The compound according to any one of claims 1-22, wherein said $R_2$ is optionally substituted sulfhydryl.

**24.** The compound according to any one of claims 1-23, wherein said $R_2$ is substituted by one or more $R_{2-1}$, and each said $R_{2-1}$ is independently an optionally substituted substituent.

**25.** The compound according to claim 24, wherein said $R_{2-1}$ is optionally substituted aryl.

**26.** The compound according to any one of claims 24-25, wherein said $R_{2-1}$ is optionally substituted phenyl.

**27.** The compound according to any one of claims 24-26, wherein said $R_{2-1}$ is substituted by one or more $R_{2-2}$, and each said $R_{2-2}$ is independently an optionally substituted substituent.

**28.** The compound according to claim 27, wherein said $R_{2-2}$ is selected from the group consisting of: hydrogen, protium, deuterium, tritium, halogen, optionally substituted carbonyl, optionally substituted carboxyl, optionally substituted acylamino, and optionally substituted alkyl.

**29.** The compound according to any one of claims 27-28, wherein said $R_{2-2}$ is selected from the group consisting of: hydrogen, protium, deuterium, tritium, halogen, optionally substituted carbonyl, optionally substituted carboxyl, optionally substituted acylamino, and optionally substituted methyl.

**30.** The compound according to any one of claims 27-29, wherein said $R_{2-2}$ is substituted by one or more $R_{2-3}$, and each said $R_{2-3}$ is independently an optionally substituted substituent.

**31.** The compound according to claim 30, wherein said $R_{2-3}$ is selected from the group consisting of: hydrogen, protium, deuterium, tritium, halogen, optionally substituted alkyl, optionally substituted alcyl, and optionally substituted amino.

**32.** The compound according to any one of claims 30-31, wherein said $R_{2-3}$ is selected from the group consisting of: hydrogen, protium, deuterium, tritium, halogen, optionally substituted methyl, optionally substituted ethyl, optionally substituted cyclopropyl, and optionally substituted amino.

**33.** The compound according to any one of claims 30-32, wherein said $R_{2-3}$ is substituted by one or more $R_{2-4}$, and each said $R_{2-4}$ is independently an optionally substituted substituent.

**34.** The compound according to claim 33, wherein said $R_{2-4}$ is selected from the group consisting of: hydrogen, protium, deuterium, tritium, halogen, optionally substituted alkyl, and optionally substituted alcyl.

**35.** The compound according to any one of claims 33-34, wherein said $R_{2-4}$ is selected from the group consisting of: hydrogen, protium, deuterium, tritium, halogen, optionally substituted methyl, optionally substituted ethyl, and optionally substituted cyclopropyl.

**36.** The compound according to any one of claims 1-35, wherein said $R_3$ is selected from the group consisting of: hydrogen, protium, deuterium, and tritium.

**37.** The compound according to claim 36, wherein said $X_1$ is selected from the group consisting of: optionally substituted CH and N.

**38.** A compound or a pharmaceutically acceptable salt, stereoisomer, ester, prodrug, solvate, and deuterated compound thereof, wherein said compound has the following structure:

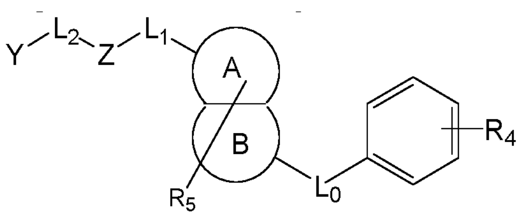

(II)

wherein,

Ring A is five- to seven-membered nitrogen-containing heterocyclyl;
Ring B is five- to seven-membered aryl or heterocyclyl;
Z is selected from: -C(O)NH-, -NHC(O)- or

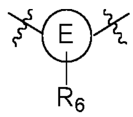

;

Ring E is aryl or heteroaryl;

$L_0$ is selected from: O, S, N($R_{La}$), $C_1$-$C_6$ alkylene, C(O); $R_{La}$ is selected from: H, $C_1$-$C_6$ alkyl;

$L_1$ is selected from: single bond, $C_1$-$C_6$ alkylene, $C_2$-$C_6$ alkenylene, $C_2$-$C_6$ alkinylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, - N($R_{Lb}$)C(O)O-, -N($R_{Lb}$)C(O)N($R_{Lb}$)-, -N($R_{Lb}$)-, -S(O)$_2$-, -S(O)$_2$N($R_{rb}$)-, -N($R_{Lb}$)S(O)$_2$-, -S(O)-, -S(O)N($R_{Lb}$)-, - N($R_{Lb}$)S(O)-; H atom(s) in said alkylene may be optionally substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxyl, $C_1$-$C_{10}$ alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, four- to ten-membered heterocyclyl, four- to ten-membered heterocyclic alkyl;

$L_2$ is selected from: single bond, $C_1$-$C_6$ alkylene, -($C_0$-$C_6$ alkylene)-$Q_2$-($C_0$-$C_6$ alkylene)-, $Q_2$ is selected from: -O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, -S(O)$_2$-, -S(O)$_2$N($R_{Lc}$)-, -N($R_{Lc}$)S(O)$_2$-, -S(O)-, -S(O)N($R_{Lc}$)-, -N($R_{Lc}$)S(O)-; H atom(s) in said alkylene may be optionally substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxyl, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, four- to ten-membered heterocyclyl, four- to ten-membered heterocyclic alkyl;

Y is selected from: H, -$NR_7R_8$, nitrogen-containing heterocyclyl, wherein, said nitrogen-containing heterocyclyl may be optionally substituted by one or more $R_9$ groups; $R_9$ is selected from: halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_7$, $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, four- to ten-membered heterocyclyl, four- to ten-membered heterocyclic alkyl; wherein, said $C_1$-$C_{10}$ alkyl, $C_2$-$C_{10}$ alkenyl, $C_2$-$C_{10}$ alkynyl, $C_3$-$C_{10}$ cycloalkyl, four- to ten-membered heterocyclyl may be optionally substituted by one or more groups selected from the group consisting of: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkyl alkyl, $C_6$-$C_{10}$ aryl, $C_7$-$C_{12}$ aralkyl, four- to ten-membered heterocyclyl, four- to ten-membered heterocyclic alkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)$OR_{401}$, -$NR_{402}$C(O)$OR_{401}$, -OC(O)$R_{401}$, -$NR_{402}SO_2R_{401}$, -$SO_2NR_{401}R_{402}$, -$NR_{402}$C(O)$R_{401}$, -C(O)$NR_{401}R_{402}$,-$NR_{401}R_{402}$, -($C_0$-$C_6$ alkylene)-$NR_{401}R_{402}$, -$SR_{401}$, -S(O)$R_{401}$, -S(O)$_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein, said $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, four- to ten-membered heterocyclyl may be optionally substituted by one or more groups selected from the group consisting of: halogen, cyano, amino, hydroxyl, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxyl substituted alkyl, $C_1$-$C_{10}$ alkoxy substituted alkyl, $C_1$-$C_{10}$ alkylamino substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), - ($C_0$-$C_6$ alkylene)-(four- to ten-membered heterocyclyl), -$SO_2$-($C_0$-$C_6$ alkylene)-(four- to ten-membered heterocyclyl), -S-($C_0$-$C_6$ alkylene)-(four- to ten-membered heterocyclyl), -O-($C_0$-$C_6$ alkylene)-(four- to ten-membered heterocyclyl), -($C_0$-$C_6$ alkylene)-($C_3$-$C_{10}$ cycloalkyl),

$-SO_2-(C_0-C_6$ alkylene)-($C_3-C_{10}$ cycloalkyl), -S-($C_0-C_6$ alkylene)-($C_3-C_{10}$ cycloalkyl), -O-($C_0-C_6$ alkylene)-($C_3-C_{10}$ cycloalkyl);

$R_{40}$, and $R_{402}$ are each independently selected from: H, $C_1-C_{10}$ alkyl, $C_3-C_{10}$ cycloalkyl, $C_3-C_{10}$ cycloalkyl alkyl, $C_6-C_{10}$ aryl, $C_6-C_{10}$ aralkyl, four- to ten-membered heterocyclyl, four- to ten-membered heterocyclic alkyl;

$R_5$ is one or more independent substituents on the

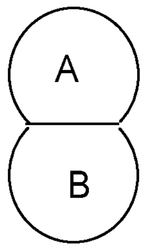

ring, each $R_5$ being independently selected from: H, halogen, cyano, amino, hydroxyl, $C_1-C_{10}$ alkyl, $C_1-C_{10}$ haloalkyl, $C_1-C_{10}$ alkoxy, $C_1-C_{10}$ haloalkoxy, $C_1-C_{10}$ alkylamino, $C_1-C_{10}$ cyanoalkyl, $C_1-C_{10}$ hydroxyl substituted alkyl, $C_1-C_{10}$ alkoxy substituted alkyl, $C_1-C_{10}$ alkylamino substituted alkyl, -($C_0-C_6$ alkylene)-($C_6-C_{10}$ aryl), $-SO_2-(C_0-C_6$ alkylene)-($C_6-C_{10}$ aryl), -S-($C_0-C_6$ alkylene)-($C_6-C_{10}$ aryl), -O-($C_0-C_6$ alkylene)-($C_6-C_{10}$ aryl), -($C_0-C_6$ alkylene)-(four- to ten-membered heterocyclyl), $-SO_2-(C_0-C_6$ alkylene)-(four- to ten-membered heterocyclyl), -S-($C_0-C_6$ alkylene)-(four- to ten-membered heterocyclyl), -O-($C_0-C_6$ alkylene)-(four- to ten-membered heterocyclyl), -($C_0-C_6$ alkylene)-($C_3-C_{10}$ cycloalkyl), $-SO_2-(C_0-C_6$ alkylene)-($C_3-C_{10}$ cycloalkyl), -S-($C_0-C_6$ alkylene)-($C_3-C_{10}$ cycloalkyl), -O-($C_0-C_6$ alkylene)-($C_3-C_{10}$ cycloalkyl); and

$R_6$ is one or more independent substituents on the E ring, each $R_6$ being independently selected from: H, halogen, cyano, amino, hydroxyl, $C_1-C_{10}$ alkyl, $C_1-C_{10}$ haloalkyl, $C_1-C_{10}$ alkoxy, $C_1-C_{10}$ haloalkoxy, $C_1-C_{10}$ alkylamino, $C_1-C_{10}$ cyanoalkyl, $C_1-C_{10}$ hydroxyl substituted alkyl, $C_1-C_{10}$ alkoxy substituted alkyl, $C_1-C_{10}$ alkylamino substituted alkyl, -($C_0-C_6$ alkylene)-($C_6-C_{10}$ aryl), $-SO_2-(C_0-C_6$ alkylene)-($C_6-C_{10}$ aryl), -S-($C_0-C_6$ alkylene)-($C_6-C_{10}$ aryl), -O-($C_0-C_6$ alkylene)-($C_6-C_{10}$ aryl), -($C_0-C_6$ alkylene)-(four- to ten-membered heterocyclyl), $-SO_2-(C_0-C_6$ alkylene)-(four- to ten-membered heterocyclyl), -S-($C_0-C_6$ alkylene)-(four- to ten-membered heterocyclyl), -O-($C_0-C_6$ alkylene)-(four- to ten-membered heterocyclyl), -($C_0-C_6$ alkylene)-($C_3-C_{10}$ cycloalkyl), $-SO_2-(C_0-C_6$ alkylene)-($C_3-C_{10}$ cycloalkyl), -S-($C_0-C_6$ alkylene)-($C_3-C_{10}$ cycloalkyl), -O-($C_0-C_6$ alkylene)-($C_3-C_{10}$ cycloalkyl).

**39.** The compound according to claim 38, wherein the

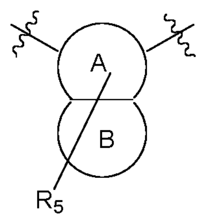

moiety is one of the following structures:

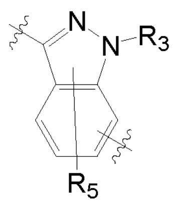 , 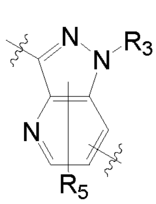 , 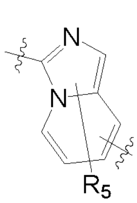 , 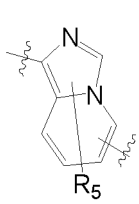 , 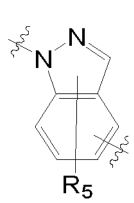 , 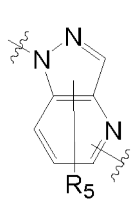 ,

preferably selected from:

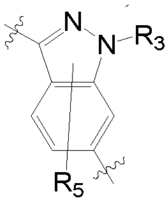 , 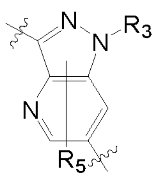 , 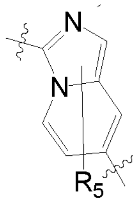 , 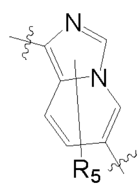 , 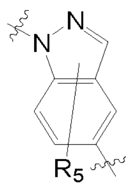 ,

more preferably is

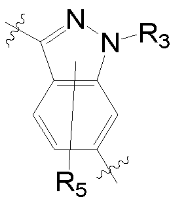 ,

wherein $R_3$ is selected from: H, $C_1$-$C_6$ alkyl.

**40.** The compound according to claim 38 or 39, wherein $L_0$ is selected from: S, O, NH, methylene, preferably S.

**41.** The compound according to any one of claims 38-40, wherein $L_1$ is selected from: $C_2$-$C_6$ alkenylene, $C_2$-$C_6$ alkinylene, -$Q_1$-($C_0$-$C_6$ alkylene)-, -($C_0$-$C_6$ alkylene)-$Q_1$-;

preferably, $Q_1$ is -N($R_{Lb}$)-, and $R_{Lb}$ is selected from: H, $C_1$-$C_3$ alkyl;
more preferably, $L_1$ is selected from:

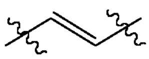 , 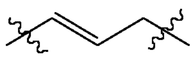 , 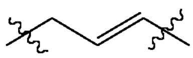 , 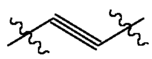 ,

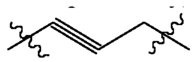 , 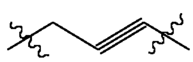 ,

-NH-.

**42.** The compound according to any one of claims 38-41, wherein Ring E is a benzene ring or a five- or six-membered nitrogen-containing heteroaryl; preferably, the

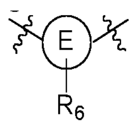

moiety is one of the following structures:

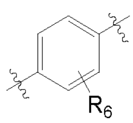 , 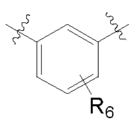 , 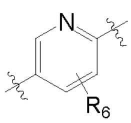 ,

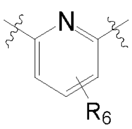 , 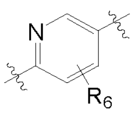 , 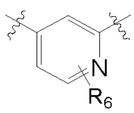 , 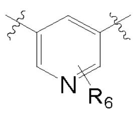 , 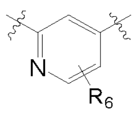 , 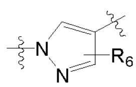 , 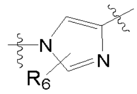 , 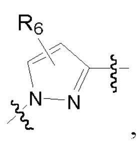 ,

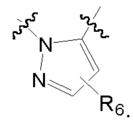

**43.** The compound according to any one of claims 38-42, wherein $L_2$ is selected from: single bond, $C_1$-$C_6$ alkylene, -$Q_2$-($C_0$-$C_6$ alkylene)-;

preferably, $Q_2$ is selected from: -O-, -S-, -N($R_{Lc}$)-, wherein $R_{Lc}$ is selected from: H, $C_1$-$C_3$ alkyl; more preferably, $L_2$ is selected from:

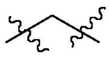, 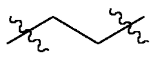, 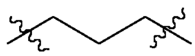, 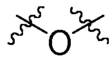, 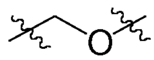,

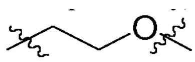, 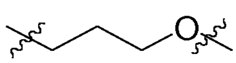.

**44.** The compound according to any one of claims 38-43, wherein Y is -$NR_7R_8$, wherein $R_7$, $R_8$ are each independently selected from: H, $C_1$-$C_6$ alkyl, -($C_0$-$C_6$ alkylene)-($C_3$-$C_6$ cycloalkyl); preferably, Y is

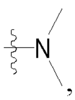, 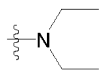.

**45.** The compound according to any one of claims 38-43, wherein Y is selected from:

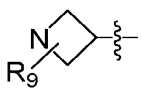,

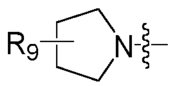, 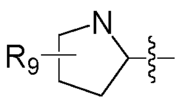, 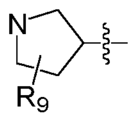, 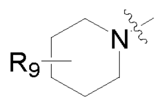, 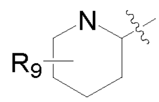, 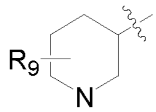, 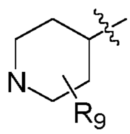,

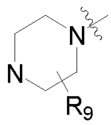, 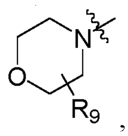, 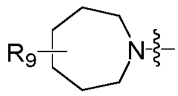, 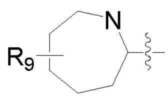, 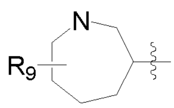, 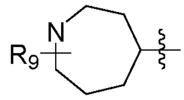, 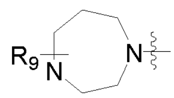;

preferably, Y is selected from:

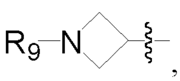, 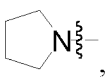, 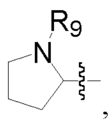, 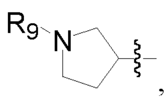, 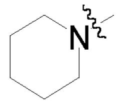, 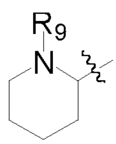,

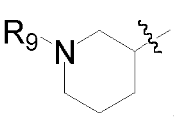, 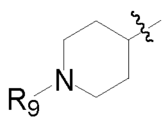, 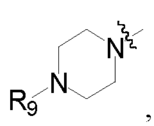, 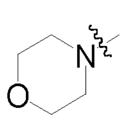, 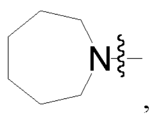, 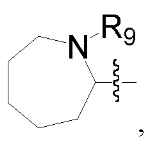, 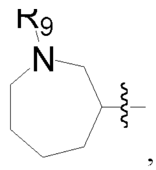,

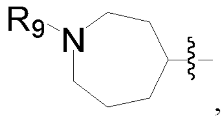, 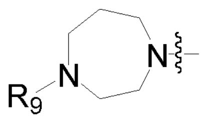;

preferably, $R_9$ is selected from: H, $C_1$-$C_6$ alkyl (for example $-CH_3$,

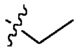, 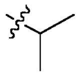, 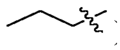

), $C_1$-$C_6$ hydroxyl substituted alkyl (for example

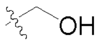, 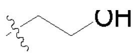),

$C_1$-$C_6$ amino substituted alkyl (for example

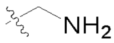,

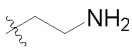),

$C_1$-$C_6$ alkoxy substituted alkyl (for example

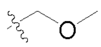, 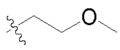),

$C_1$-$C_6$ alkylamino substituted alkyl (for example

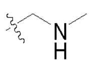, 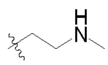, 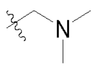, 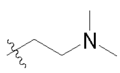),

-($C_0$-$C_6$ alkylene)-($C_3$-$C_6$ cycloalkyl) (for example

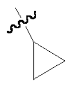, 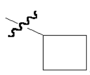, 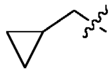, 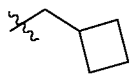);

more preferably, Y is selected from:

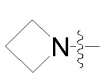, 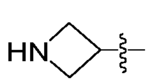, 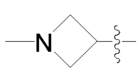, 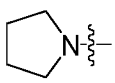, 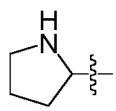, 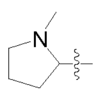,

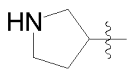, 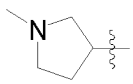, 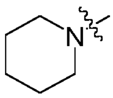, 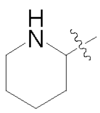, 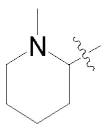, 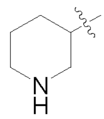, 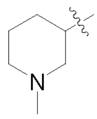, 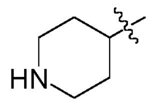, 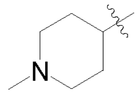,

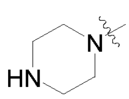, 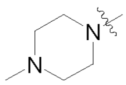, 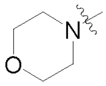, 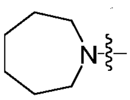, 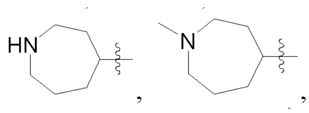, , 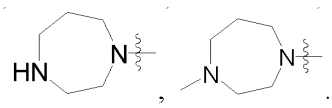, .

**46.** The compound according to any one of claims 38-45, wherein the

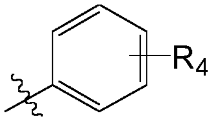

moiety is

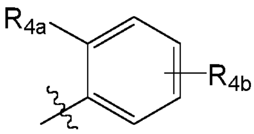, 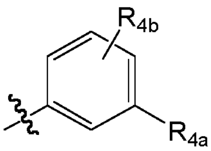 or 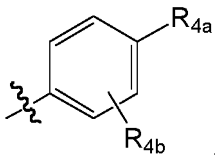,

wherein,

$R_{4a}$ is selected from: -C(O)NR$_{401}$R$_{402}$, -(C$_0$-C$_6$ alkylene)-NR$_{401}$R$_{402}$, -C(O)R$_{401}$, -C(O)OR$_{401}$, R$_{401}$ and R$_{402}$ are independently selected from: H, C$_1$-C$_6$ alkyl, -(C$_0$-C$_6$ alkylene)-(C$_3$-C$_6$ cycloalkyl); wherein, said C$_0$-C$_6$ alkylene, C$_1$-C$_6$ alkyl, C$_3$-C$_6$ cycloalkyl may be optionally substituted by one or more groups selected from the group consisting of: halogen, cyano, amino, hydroxyl, C$_1$-C$_6$ alkyl, C$_1$-C$_6$ haloalkyl;
$R_{4b}$ is one or more independent substituents on the benzene ring, which are selected from: H, halogen, cyano, amino, hydroxyl, C$_1$-C$_6$ alkyl, C$_1$-C$_6$ haloalkyl;
preferably, $R_{4a}$ is selected from:

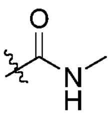, 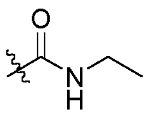, 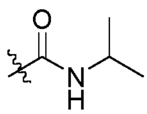, 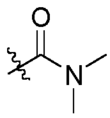, 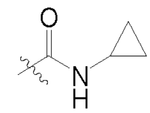,

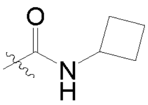, 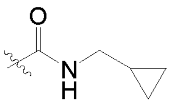, 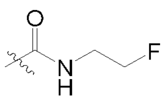, 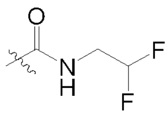, 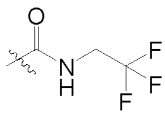, 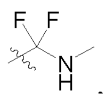, 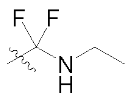,

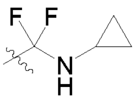, 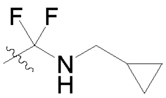, 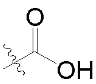, 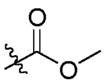, 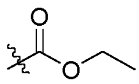, 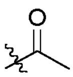, 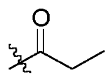, 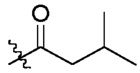,

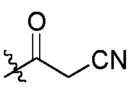, 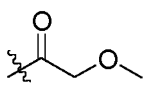, 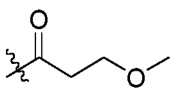, 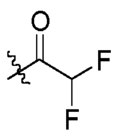, 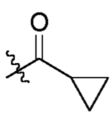, 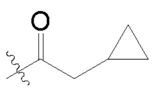;

preferably, $R_{4b}$ is H or halogen;
more preferably, the

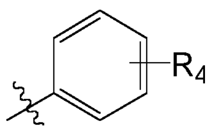

moiety is

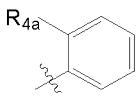, 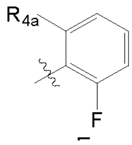, 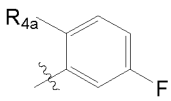, 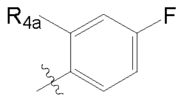,

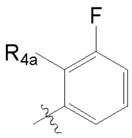, 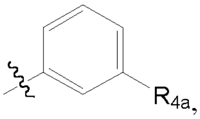, 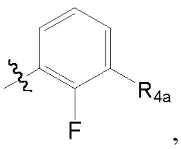, 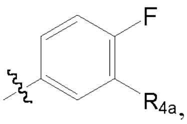, 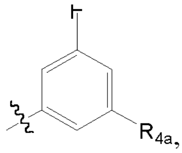, 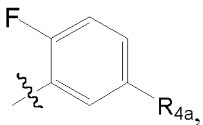,

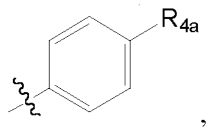, 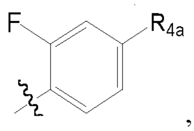, 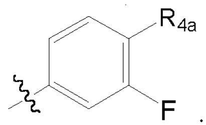.

**47.** A compound, wherein said compound has a structure selected from the group consisting of:

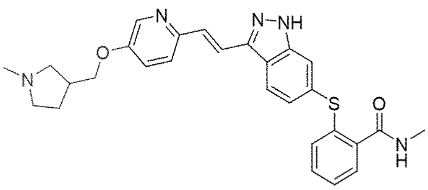, 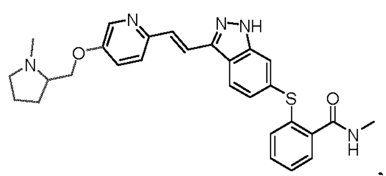, 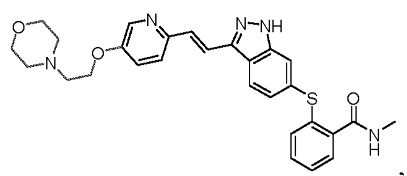,

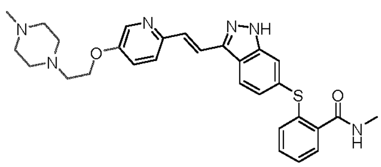, 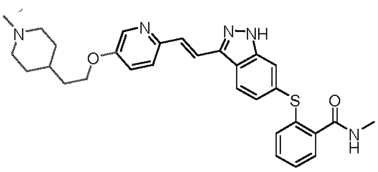, 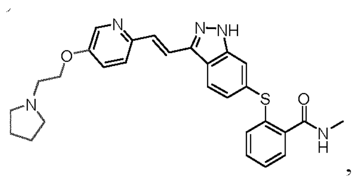,

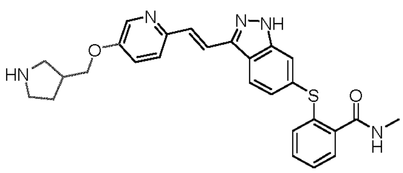, 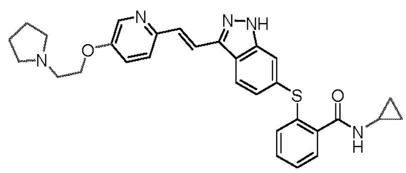, 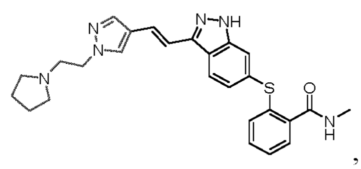,

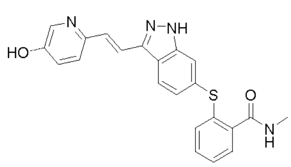, 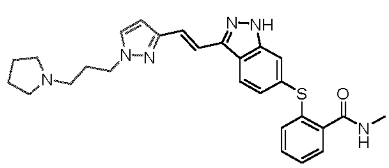, 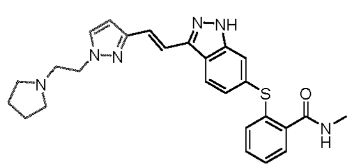,

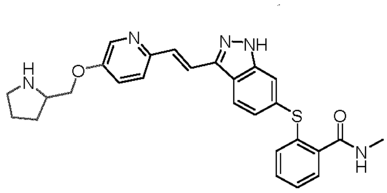, 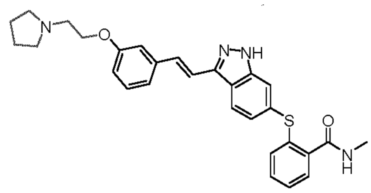, 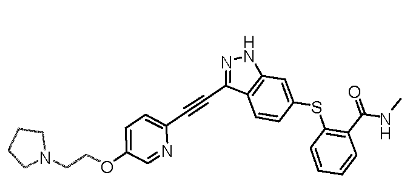,

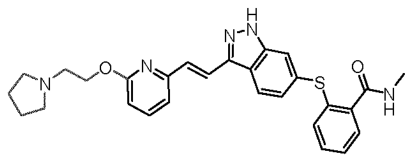,
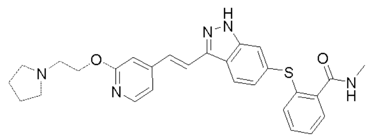,
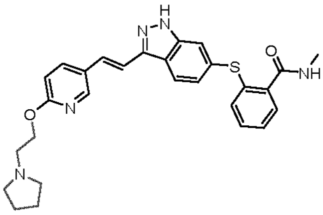,

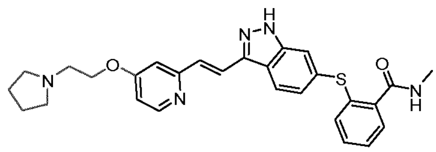,
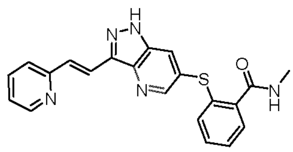,
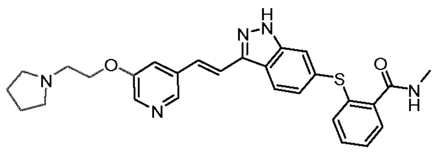,

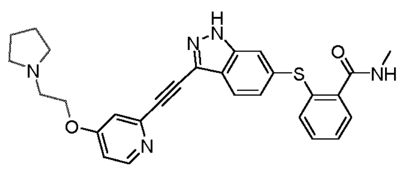,
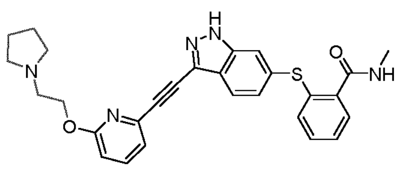,
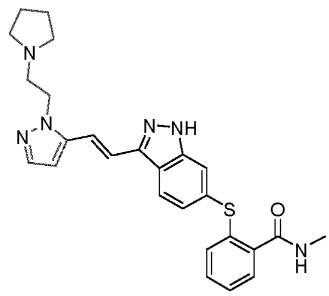,

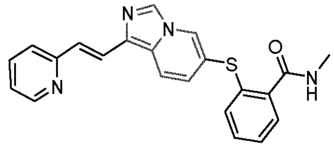,
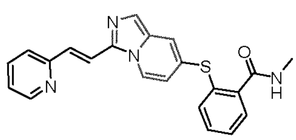,
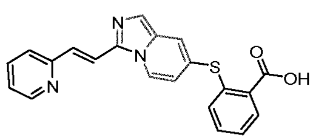,

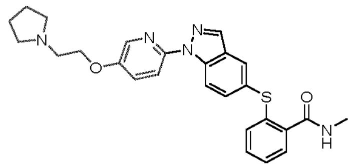,
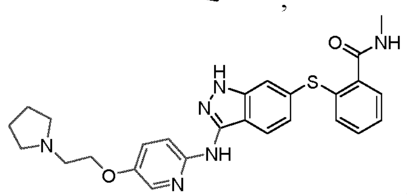,
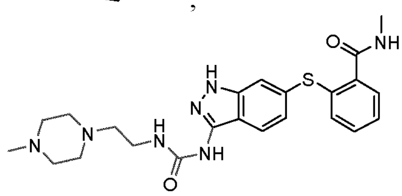,

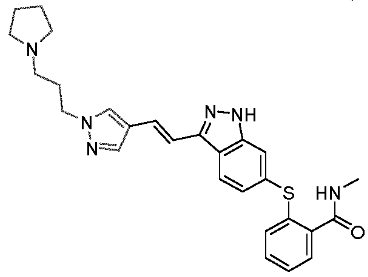,
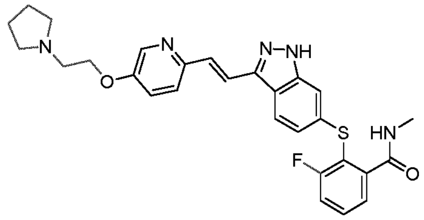,

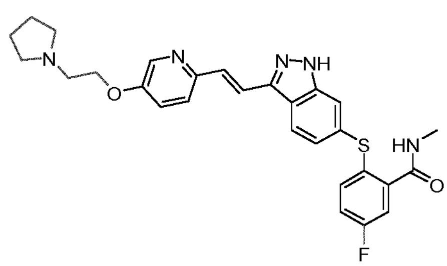,
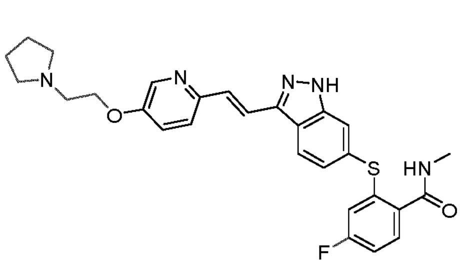,

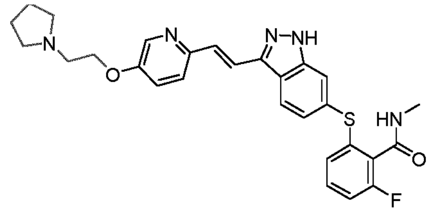,

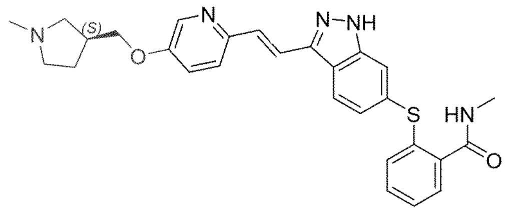,

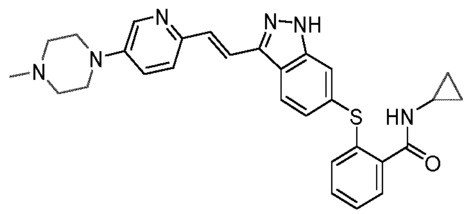,

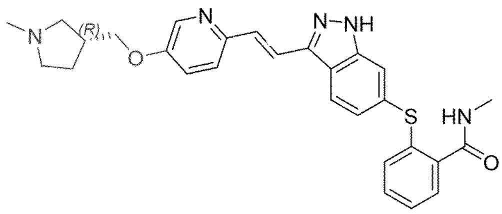,

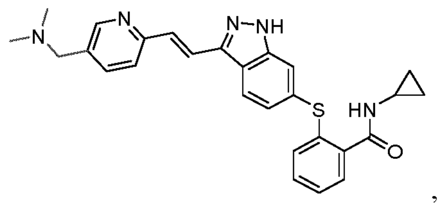,

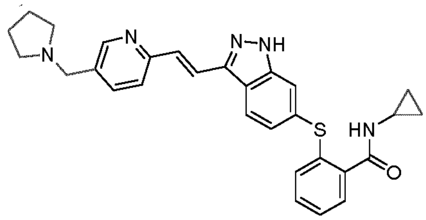,

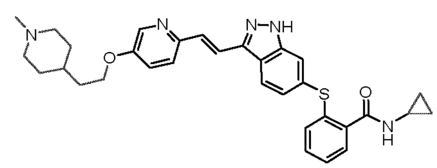,

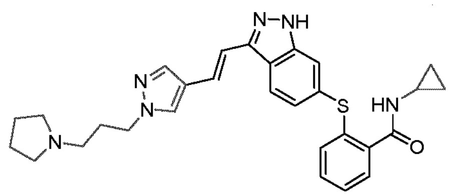,

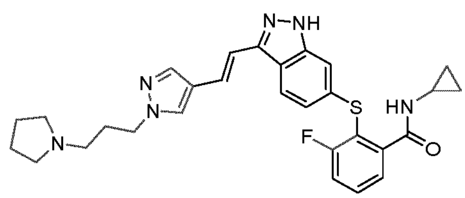,

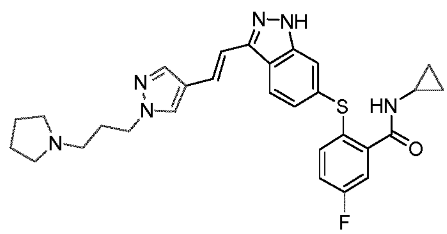,

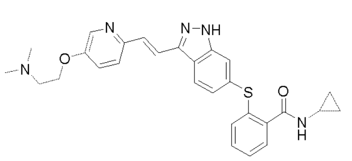,

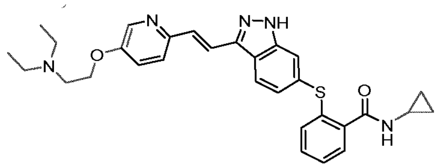,

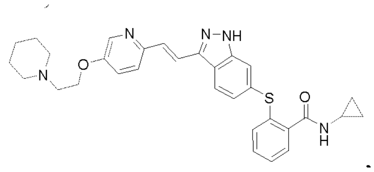,

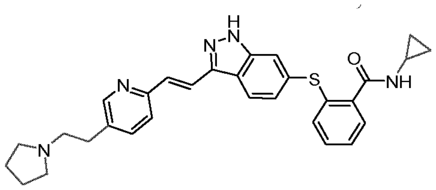,

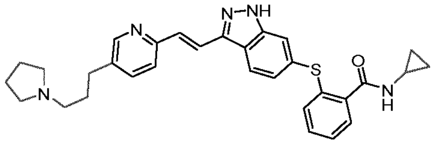,

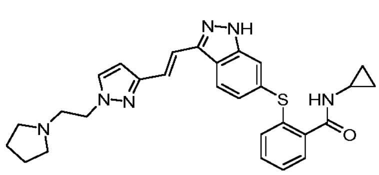,
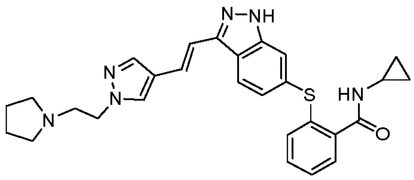,
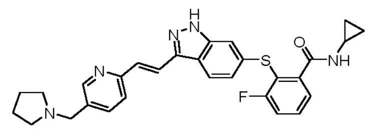,

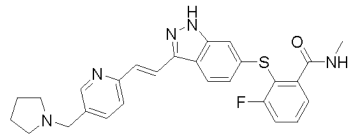,
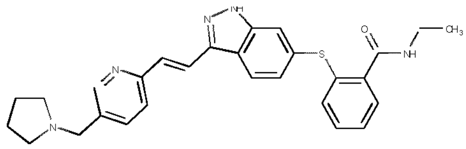,

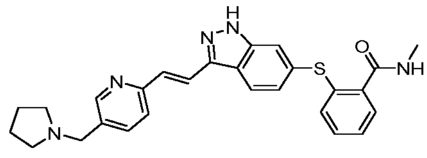,
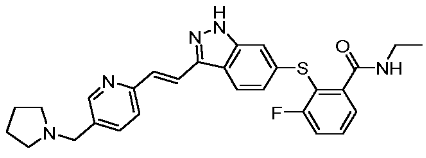,

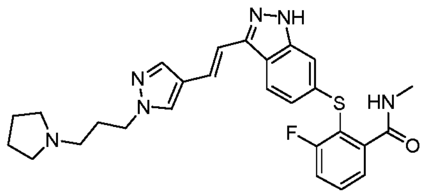,
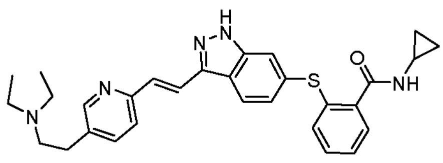,

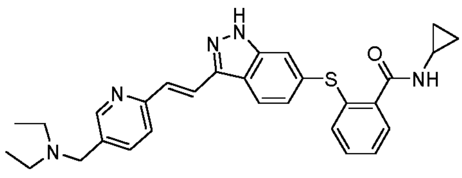,
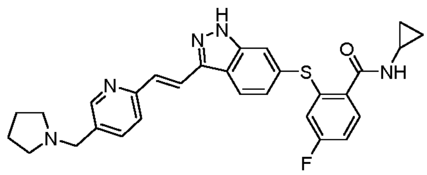,

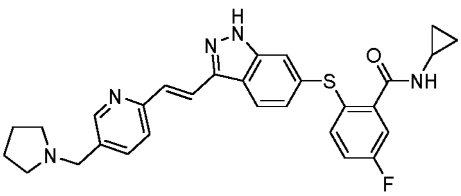,
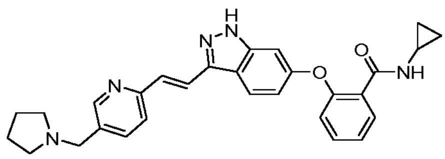,

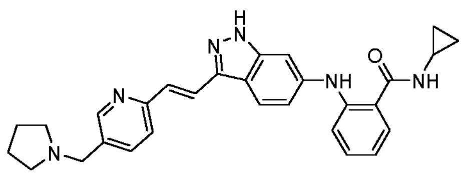,
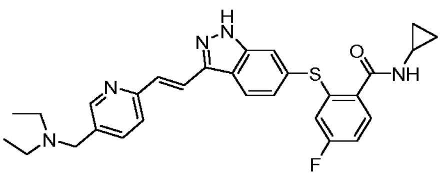,

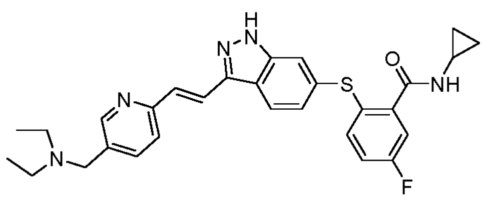,
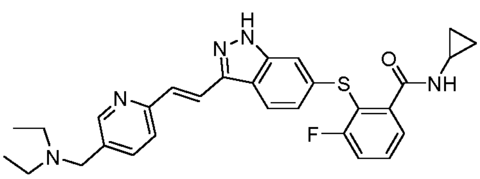,

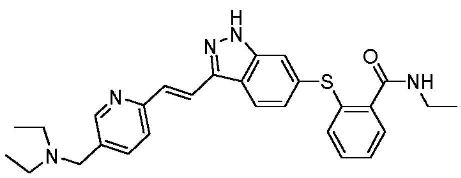,
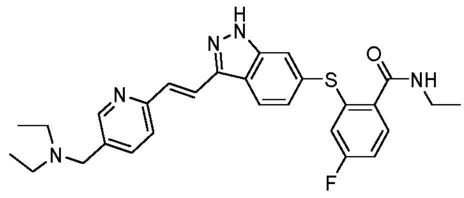,

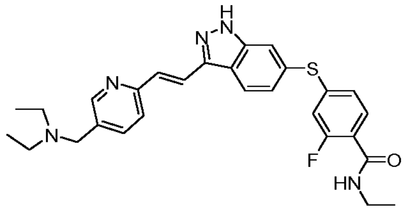,
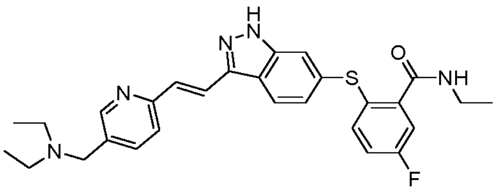,

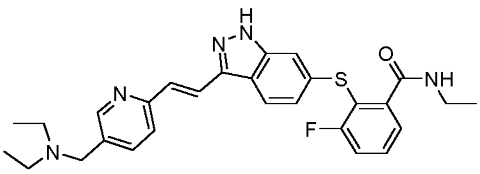,
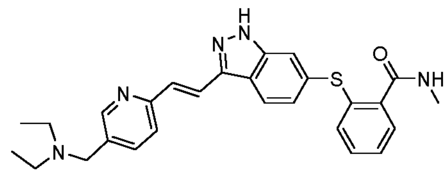,

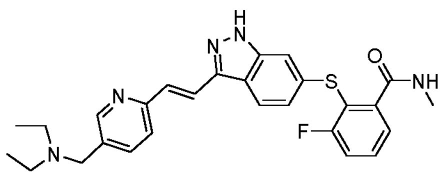,
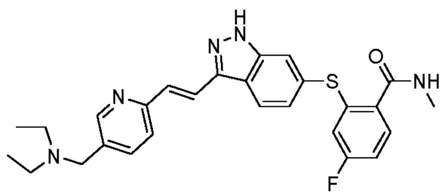,

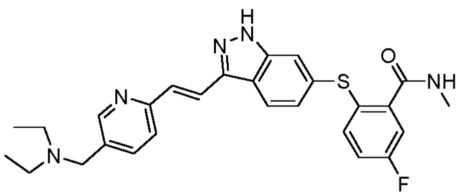,
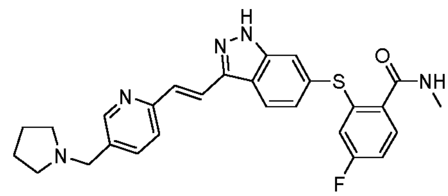,

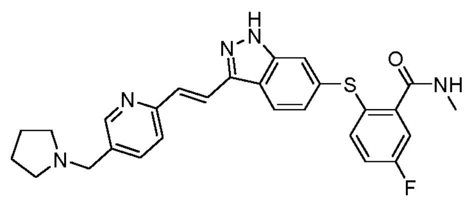,
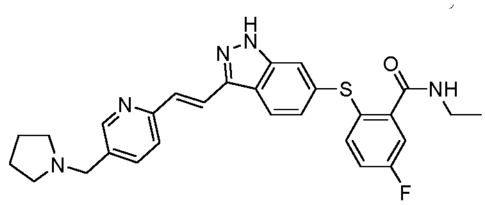,

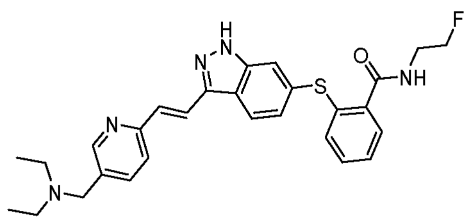,
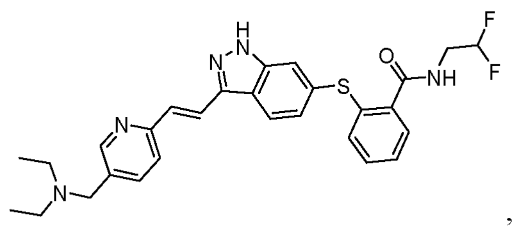,

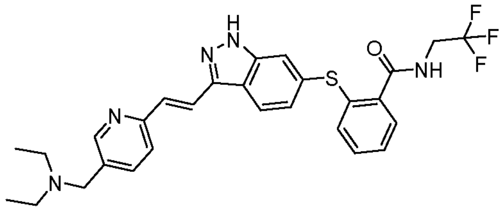
,
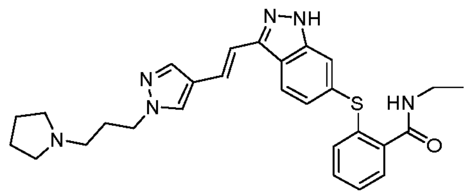
,
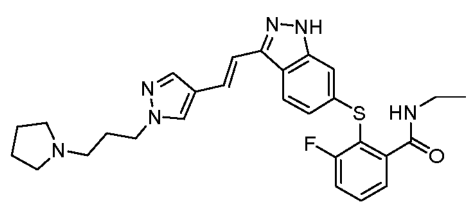
,
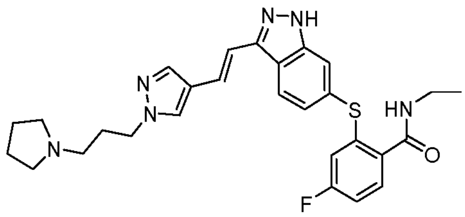
,
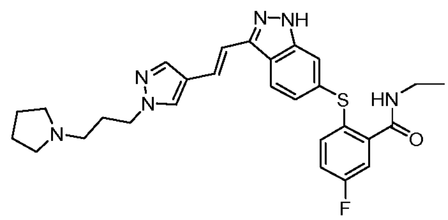
,
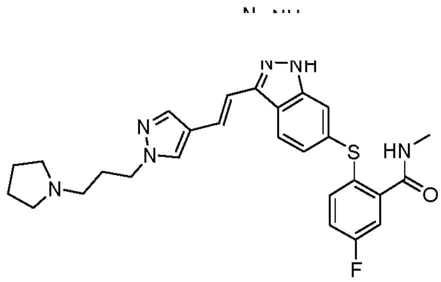
,
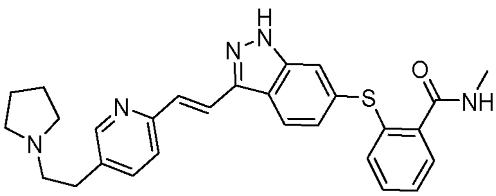
,
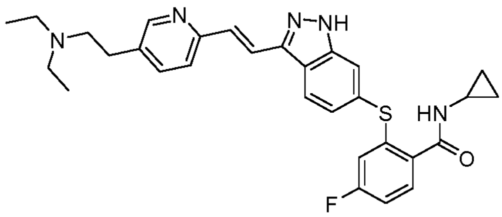
,
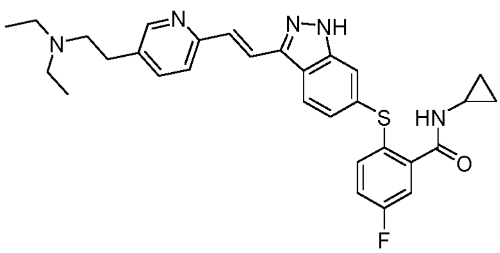
,
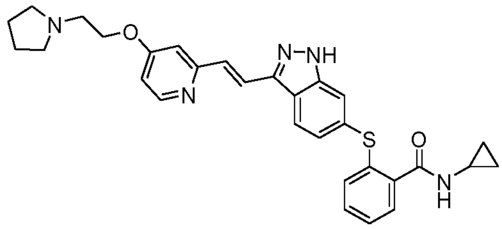
,
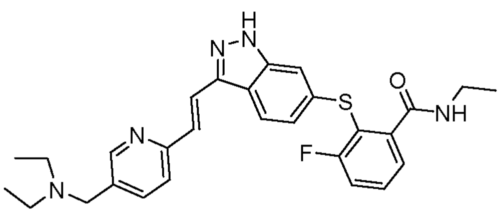
,
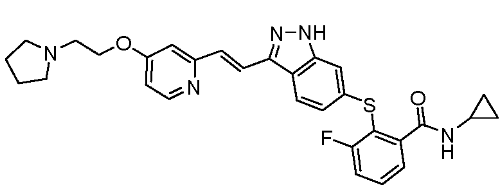
,

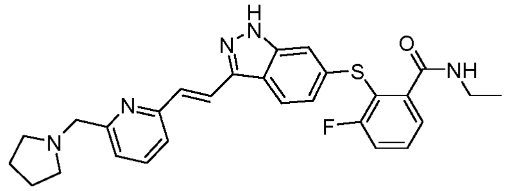 , 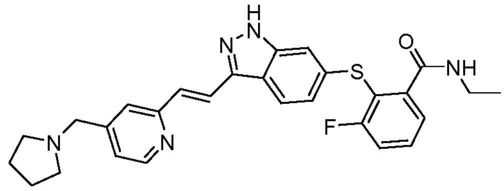 ,

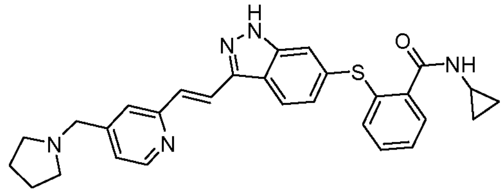 , 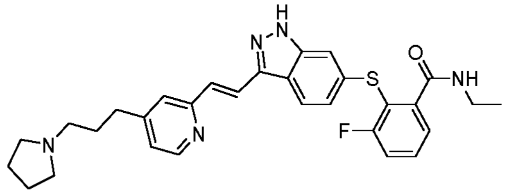 ,

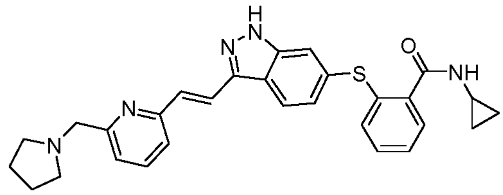 , 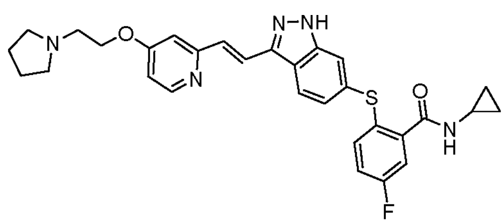 ,

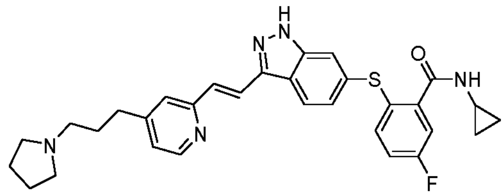 , 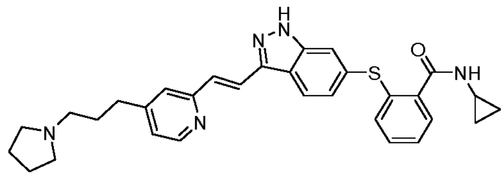 ,

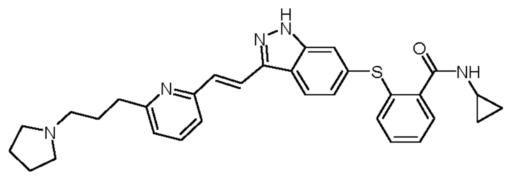 , 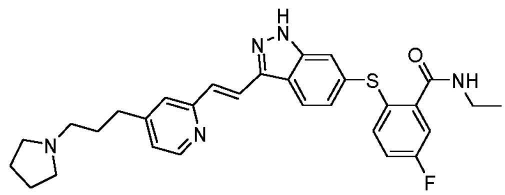 ,

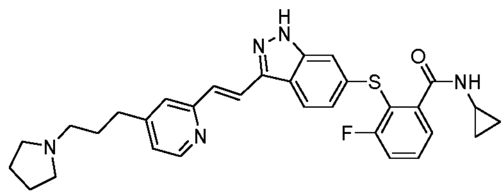 , 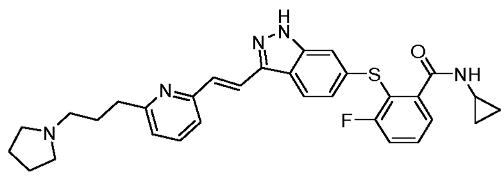 ,

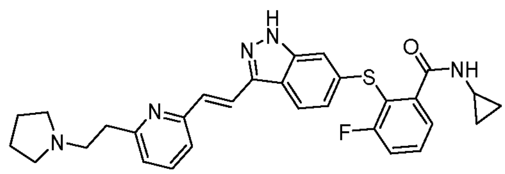 , 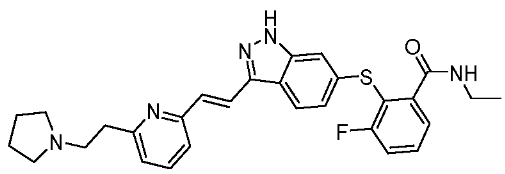 ,

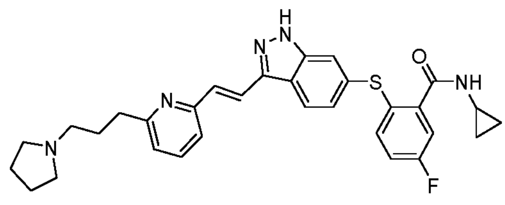, 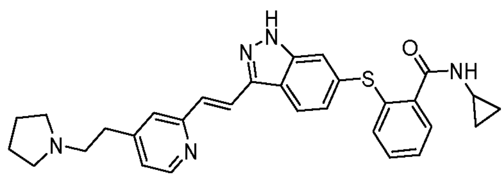,

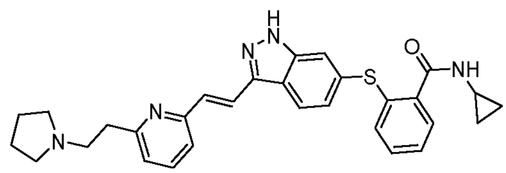, 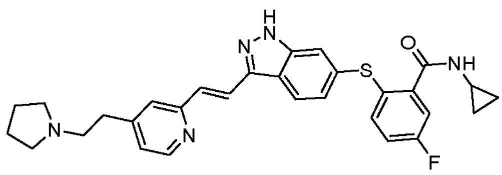,

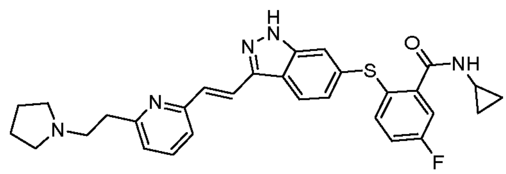, 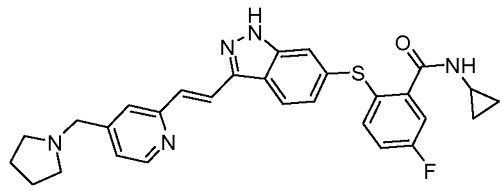,

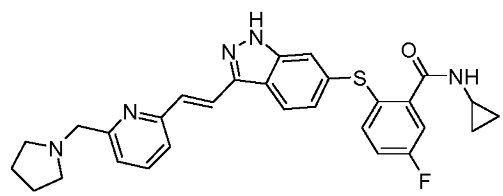, 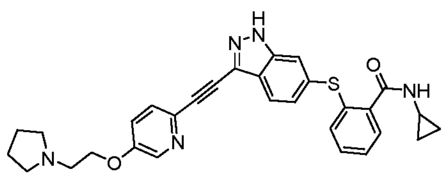,

or a pharmaceutically acceptable prodrug, a pharmaceutically active metabolite, and/or a pharmaceutically acceptable salt of said compound.

**48.** A pharmaceutical composition, comprising the compound according to any one of claims 1-47 or a pharmaceutically acceptable prodrug, a pharmaceutically active metabolite, and/or a pharmaceutically acceptable salt of said compound, as well as an optional carrier.

**49.** Use of the compound according to any one of claims 1-47 or a pharmaceutically acceptable salt, stereoisomer, ester, prodrug, solvate, and deuterated compound thereof, and/or the pharmaceutical composition according to claim 48 in the preparation of a medicament for prevention and/or treatment of a disease;
preferably, said disease is a protein tyrosine kinase-mediated proliferative disease.

**50.** The use according to claim 49, wherein said disease is an ocular disease, preferably selected from: diabetic retinopathy (including non-proliferative diabetic retinopathy, proliferative diabetic retinopathy, and diabetic macular edema); age-related macular degeneration (AMD) (including neovascular (wet/exudative) AMD, dry AMD, and geographic atrophy); pathological choroidal neovascularization (CNV) originating from any pathological mechanism (i.e., high myopia, trauma, sickle cell anemia; ocular histoplasmosis, angioid streaks, traumatic choroidal rupture, optic nerve drusen, and certain retinal dystrophies); pathological retinal neovascularization originating from any pathological mechanism (i.e., sickle cell retinopathy, Eales disease (retinal periphlebitis), ocular ischemic syndrome, carotid cavernous fistula, familial exudative vitreoretinopathy, hyperviscosity syndrome, idiopathic occlusive arteritis, birdshot retinochoroidopathy, retinal vasculitis, sarcoidosis, or toxoplasmosis); uveitis; retinal vein occlusion (central or branch); ocular trauma; postsurgical edema; postsurgical neovascularization; cystoid macular edema; ocular ischemia; retinopathy of prematurity; Coat's disease; sickle cell retinopathy and/or neovascular glaucoma;

preferably, said disease is diabetic retinopathy (including non-proliferative and proliferative diabetic retinopathy and diabetic macular edema) or retinal vein occlusion (central or branch);
or, said disease is a tumor, preferably selected from: breast cancer, lung cancer (especially non-small cell lung cancer), adenocarcinoma, colorectal adenocarcinoma, renal cancer, liver cancer, pancreatic cancer, ovarian

cancer, prostate cancer, glioma, glioblastoma, myeloma, leukemia, agnogenic myeloid metaplasia, mesothelioma, myelodysplastic syndrome;
preferably, said disease is a hematological malignancy, more preferably selected from: chronic lymphocytic leukemia (CLL), chronic myeloid leukemia (CML), acute lymphoblastic leukemia (ALL), acute myeloid leukemia (AML), acute monoblastic leukemia, diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphoma, primary mediastinal B-cell lymphoma, Burkitt's lymphoma, lymphoplasmacytic lymphoma, hairy cell leukemia (HCL), immunoblastic large cell lymphoma, precursor B lymphoblastic lymphoma and primary central nervous system (CNS) lymphoma and T-cell non-Hodgkin lymphoma (NHL), such as precursor T lymphoblastic lymphoma/leukemia, peripheral T-cell lymphoma (PTCL), angioimmunoblastic T-cell lymphoma, extranodal natural killer (NK)/T-cell lymphoma, enteropathy-type T-cell lymphoma, subcutaneous panniculitis-like T-cell lymphoma, and anaplastic large cell lymphoma, NK/T-cell lymphoma.

**51.** A method for modulating kinase receptor activity, comprising administrating the compound according to any one of claims 1-47 or a pharmaceutically acceptable salt, stereoisomer, ester, prodrug, solvate, and deuterated compound thereof, and/or the pharmaceutical composition according to claim 48;

preferably, said method selectively inhibits kinase receptor activity;
more preferably, said kinase receptor comprises VEGFR, EGFR, or their functionally active fragments;
even more preferably, said method selectively inhibits VEGFR (including VEGFR1, VEGFR2, VEGFR3) receptor activity, while avoids inhibiting EGFR receptor activity.

**INTERNATIONAL SEARCH REPORT**

International application No.
**PCT/CN2023/111668**

**A. CLASSIFICATION OF SUBJECT MATTER**

C07D231/56(2006.01)i; C07D401/04(2006.01)i; C07D401/12(2006.01)i; C07D401/14(2006.01)i; C07D403/12(2006.01)i; C07D403/14(2006.01)i; C07D405/12(2006.01)i; C07D405/14(2006.01)i; C07D413/12(2006.01)i; C07D413/14(2006.01)i; C07D417/12(2006.01)i; C07D419/12(2006.01)i; C07D417/14(2006.01)i; C07D419/14(2006.01)i; A61K31/416(2006.01)i; A61P27/06(2006.01)i; A61P27/02(2006.01)i; A61P7/00(2006.01)i

According to International Patent Classification (IPC) or to both national classification and IPC

**B. FIELDS SEARCHED**

Minimum documentation searched (classification system followed by classification symbols)

C07D231; C07D401; C07D403; C07D405; C07D413; C07D417; C07D419; A61K31; A61P27; A61P7

Documentation searched other than minimum documentation to the extent that such documents are included in the fields searched

Electronic data base consulted during the international search (name of data base and, where practicable, search terms used)

STN(Registy, Caplus): 化学structural formula search CNTXT, ENTXT, CNKI: : 蛋白酪氨酸激酶, 抑制剂, 吲唑, 苯并吡唑, 黄斑变性, 视网膜病变; Protein tyrosine kinase inhibitors, indazole, benzopyrazole, macular degeneration, retinopathy

**C. DOCUMENTS CONSIDERED TO BE RELEVANT**

| Category* | Citation of document, with indication, where appropriate, of the relevant passages | Relevant to claim No. |
|---|---|---|
| X | CN 1374950 A (AGOURON PHARMACEUTICALS INC.) 16 October 2002 (2002-10-16)<br>claims, and in particular, description, compounds of pages 11-13 | 1-51 |
| X | CN 1495171 A (AGOURON PHARMACEUTICALS INC.) 12 May 2004 (2004-05-12)<br>claims, and in particular, description, compounds of pages 11-13 | 1-51 |
| X | US 2004192735 A1 (AGOURON PHARMA) 30 September 2004 (2004-09-30)<br>description, compounds disclosed in pages 1-7 | 1-51 |
| X | WO 0102369 A2 (AGOURON PHARMA) 11 January 2001 (2001-01-11)<br>claims, and in particular, description, compounds of paragraphs 19-21 | 1-51 |
| X | WO 2022143628 A1 (ONQUALITY PHARMACEUTICALS CHINA LTD.) 07 July 2022 (2022-07-07)<br>claims, and in particular, compounds I-1-I-15 | 1-51 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 October 2023** | **30 October 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/111668**

| Patent document cited in search report | Publication date (day/month/year) | Patent family member(s) | Publication date (day/month/year) |
|---|---|---|---|
| CN 1374950 A | 16 October 2002 | JP 2006348043 A | 28 December 2006 |
| | | JP 3969669 B2 | 05 September 2007 |
| | | BE 2013C015I2 | 21 May 2019 |
| | | EP 1614683 A1 | 11 January 2006 |
| | | EP 1614683 B1 | 21 November 2007 |
| | | TNSN 00146 A1 | 10 November 2005 |
| | | DE 60036879 D1 | 06 December 2007 |
| | | DE 60036879 T2 | 14 February 2008 |
| | | DK 1614683 T3 | 25 March 2008 |
| | | CZ 20014634 A3 | 11 September 2002 |
| | | CZ 301667 B6 | 19 May 2010 |
| | | WO 0102369 A2 | 11 January 2001 |
| | | WO 0102369 A3 | 25 April 2002 |
| | | LU 92154 I2 | 22 April 2013 |
| | | DE 60037211 D1 | 03 January 2008 |
| | | DE 60037211 T2 | 11 December 2008 |
| | | NO 2013004 I1 | 18 March 2013 |
| | | NO 2013004 I2 | 02 June 2014 |
| | | HK 1085470 A1 | 25 August 2006 |
| | | PL 355757 A1 | 17 May 2004 |
| | | PL 212108 B1 | 31 August 2012 |
| | | NZ 516676 A | 26 September 2003 |
| | | BG 106380 A | 30 September 2002 |
| | | BG 66070 B1 | 31 January 2011 |
| | | UY 26231 A1 | 31 January 2001 |
| | | EE 200100717 A | 17 February 2003 |
| | | EE 05585 B1 | 15 October 2012 |
| | | SK 19252001 A3 | 06 November 2002 |
| | | SK 286936 B6 | 06 July 2009 |
| | | IL 146710 A0 | 25 July 2002 |
| | | NO 20060596 L | 01 March 2002 |
| | | KR 20020027379 A | 13 April 2002 |
| | | KR 100529639 B1 | 16 January 2006 |
| | | MEP 45108 A | 10 May 2011 |
| | | AP 200202392 A0 | 31 March 2002 |
| | | AP 2002002392 A0 | 01 November 2005 |
| | | MY 139999 A | 30 November 2009 |
| | | UA 66933 C2 | 15 June 2004 |
| | | IS 6207 A | 19 December 2001 |
| | | IS 2791 B | 15 June 2012 |
| | | NO 20015797 D0 | 28 November 2001 |
| | | NO 20015797 L | 01 March 2002 |
| | | NO 322507 B1 | 16 October 2006 |
| | | AR 035554 A1 | 16 June 2004 |
| | | HK 1048813 A1 | 17 April 2003 |
| | | CR 6517 A | 21 September 2006 |
| | | HRP 20020109 A2 | 31 December 2003 |
| | | HRP 20020109 B1 | 31 July 2008 |
| | | PT 1614683 E | 24 January 2008 |
| | | AT 376543 T | 15 November 2007 |

Form PCT/ISA/210 (patent family annex) (July 2022)

**INTERNATIONAL SEARCH REPORT**
**Information on patent family members**

International application No.
**PCT/CN2023/111668**

| Patent document cited in search report | Publication date (day/month/year) | Patent family member(s) | Publication date (day/month/year) |
|---|---|---|---|
| | | DZ 3191 A1 | 11 January 2001 |
| | | SI 1614683 T1 | 29 February 2008 |
| | | FR 13C0010 I1 | 22 March 2013 |
| | | FR 13C0010 I2 | 16 August 2013 |
| | | AU 5785200 A | 22 January 2001 |
| | | AU 777701 B2 | 28 October 2004 |
| | | CA 2383630 A1 | 11 January 2001 |
| | | CA 2383630 C | 18 November 2008 |
| | | PT 1218348 E | 14 December 2007 |
| | | MA 26803 A1 | 20 December 2004 |
| | | SI 1218348 T1 | 29 February 2008 |
| | | GT 200000107 A | 21 December 2001 |
| | | ES 2293906 T3 | 01 April 2008 |
| | | MXPA 01012795 A | 02 September 2002 |
| | | EA 200200120 A1 | 29 August 2002 |
| | | EA 004460 B1 | 29 April 2004 |
| | | CY 1107147 T1 | 24 October 2012 |
| | | EP 1218348 A2 | 03 July 2002 |
| | | EP 1218348 B1 | 24 October 2007 |
| | | SV 2002000121 A | 02 December 2002 |
| | | MY 137622 A | 27 February 2009 |
| | | HK 1065037 A1 | 08 February 2005 |
| | | CO 5190686 A1 | 29 August 2002 |
| | | OA 11980 A | 18 April 2006 |
| | | ES 2296014 T3 | 16 April 2008 |
| | | GEP 20063885 B | 10 August 2006 |
| | | AR 065937 A2 | 15 July 2009 |
| | | YU 92901 A | 03 September 2004 |
| | | RS 50339 B | 10 November 2009 |
| | | JO 2319 B | 12 September 2005 |
| | | JP 2003503481 A | 28 January 2003 |
| | | JP 3878849 B2 | 07 February 2007 |
| | | PE 20010306 A1 | 29 March 2001 |
| | | HU 0202490 A2 | 28 November 2002 |
| | | HU 0202490 A3 | 28 January 2003 |
| | | HU 228502 B1 | 28 March 2013 |
| | | BR 0012352 A | 14 May 2002 |
| | | BRPI 0012352 B1 | 16 August 2016 |
| | | BRPI 0012352 B8 | 25 May 2021 |
| | | PA 8498001 A1 | 26 August 2002 |
| | | JO 2319 B1 | 12 September 2005 |
| | | CR 10194 A | 30 September 2008 |
| | | ZA 200110061 B | 06 February 2003 |
| | | DK 1218348 T3 | 25 February 2008 |
| | | AP 200202392 D0 | 31 March 2002 |
| CN 1495171 A | 12 May 2004 | None | |
| US 2004192735 A1 | 30 September 2004 | AP 200503341 A0 | 30 June 2005 |
| | | MA 27570 A1 | 03 October 2005 |
| | | PE 20041006 A1 | 24 December 2004 |
| | | PL 377713 A1 | 06 February 2006 |

Form PCT/ISA/210 (patent family annex) (July 2022)

**INTERNATIONAL SEARCH REPORT**
**Information on patent family members**

International application No.
**PCT/CN2023/111668**

| Patent document cited in search report | | | Publication date (day/month/year) | Patent family member(s) | | | Publication date (day/month/year) |
|---|---|---|---|---|---|---|---|
| | | | | OA | 12977 | A | 13 October 2006 |
| | | | | KR | 20050084439 | A | 26 August 2005 |
| | | | | KR | 100730267 | B1 | 20 June 2007 |
| | | | | US | 7053107 | B2 | 30 May 2006 |
| | | | | AP | 200503341 | D0 | 30 June 2005 |
| | | | | NL | 1025073 | A1 | 22 June 2004 |
| | | | | NL | 1025073 | C2 | 01 February 2005 |
| | | | | PT | 1585743 | E | 12 July 2007 |
| | | | | ECSP | 055858 | A | 20 September 2005 |
| | | | | GT | 200300294 | A | 27 July 2004 |
| | | | | DK | 1585743 | T3 | 17 September 2007 |
| | | | | NZ | 540340 | A | 27 July 2007 |
| | | | | CA | 2510850 | A1 | 08 July 2004 |
| | | | | WO | 2004056806 | A1 | 08 July 2004 |
| | | | | CR | 7882 | A | 05 August 2005 |
| | | | | HRP | 20050559 | A2 | 28 February 2006 |
| | | | | EA | 200500811 | A1 | 29 December 2005 |
| | | | | EA | 008501 | B1 | 29 June 2007 |
| | | | | AU | 2003286321 | A1 | 14 July 2004 |
| | | | | DE | 60314013 | D1 | 05 July 2007 |
| | | | | DE | 60314013 | T2 | 17 January 2008 |
| | | | | PA | 8592901 | A1 | 04 February 2005 |
| | | | | UY | 28130 | A1 | 30 July 2004 |
| | | | | NO | 20053486 | L | 18 July 2005 |
| | | | | AT | 362928 | T | 15 June 2007 |
| | | | | TNSN | 05164 | A1 | 14 May 2007 |
| | | | | CO | 5580767 | A2 | 30 November 2005 |
| | | | | MXPA | 05006676 | A | 16 August 2005 |
| | | | | SI | 1585743 | T1 | 31 August 2007 |
| | | | | TW | 200424193 | A | 16 November 2004 |
| | | | | EP | 1585743 | A1 | 19 October 2005 |
| | | | | EP | 1585743 | B1 | 23 May 2007 |
| | | | | ZA | 200504339 | B | 26 July 2006 |
| | | | | IS | 7859 | A | 23 May 2005 |
| | | | | JP | 2006515589 | A | 01 June 2006 |
| | | | | JP | 3814285 | B2 | 23 August 2006 |
| | | | | RS | 20050430 | A | 03 August 2007 |
| | | | | BR | 0317548 | A | 22 November 2005 |
| | | | | AR | 042510 | A1 | 22 June 2005 |
| | | | | US | 2006160858 | A1 | 20 July 2006 |
| WO | 0102369 | A2 | 11 January 2001 | None | | | |
| WO | 2022143628 | A1 | 07 July 2022 | TW | 202241440 | A | 01 November 2022 |

Form PCT/ISA/210 (patent family annex) (July 2022)